(19) Europäisches Patentamt
European Patent Office
Office européen des brevets

(11) **EP 2 124 059 A1**

(12) **EUROPEAN PATENT APPLICATION**
published in accordance with Art. 153(4) EPC

(43) Date of publication:
**25.11.2009 Bulletin 2009/48**

(51) Int Cl.:
***G01N 33/68*** *(2006.01)* ***A61B 10/00*** *(2006.01)*

(21) Application number: **08703771.9**

(22) Date of filing: **24.01.2008**

(86) International application number:
**PCT/JP2008/050942**

(87) International publication number:
**WO 2008/090941 (31.07.2008 Gazette 2008/31)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **24.01.2007 JP 2007014050**

(71) Applicants:
• **Ajinomoto Co., Inc.**
  **Tokyo 104-8315 (JP)**
• **Keio University**
  **Tokyo 108-8345 (JP)**

(72) Inventors:
• **MURAMATSU, Takahiko**
  **Kawasaki-shi**
  **Kanagawa 210-8681 (JP)**
• **ANDOU, Ayatoshi**
  **Kawasaki-shi**
  **Kanagawa 210-8681 (JP)**
• **HIBI, Toshifumi**
  **Tokyo 160-8582 (JP)**
• **HISAMATSU, Tadakazu**
  **Tokyo 160-8582 (JP)**

• **OKAMOTO, Susumu**
  **Tokyo 160-8582 (JP)**
• **SAKAI, Ryosei**
  **Kawasaki-shi**
  **Kanagawa 210-8681 (JP)**
• **ONO, Nobukazu**
  **Kawasaki-shi**
  **Kanagawa 210-8681 (JP)**
• **SUZUKI, Manabu**
  **Kawasaki-shi**
  **Kanagawa 210-8681 (JP)**
• **ANDO, Toshihiko**
  **Kawasaki-shi**
  **Kanagawa 210-8681 (JP)**
• **HASHIMOTO, Masaki**
  **Kawasaki-shi**
  **Kanagawa 210-8681 (JP)**

(74) Representative: **Johnson, Richard Alan et al**
  **Mewburn Ellis LLP**
  **33 Gutter Lane**
  **London**
  **EC2V 8AS (GB)**

(54) **METHOD OF EVALUATING IBD, AMINO ACID DATA PROCESSOR, AMINO ACID DATA PROCESSING METHOD, AMINO ACID DATA PROCESSING SYSTEM, AMINO ACID DATA PROCESSING PROGRAM AND RECORDING MEDIUM**

(57) Provided are a method of evaluating IBD, an amino acid data processor, an amino acid data-processing method, an amino acid data-processing system, an amino acid data-processing program and a recording medium, which are capable of evaluating an IBD state accurately by utilizing the concentration of amino acids in blood. According to the method of evaluating IBD of the present invention, amino acid concentration data on the concentration value of amino acid in blood collected from a subject to be evaluated is measured, and an inflammatory bowel disease state in the subject is evaluated based on the measured amino acid concentration data of the subject.

FIG.1

**Description**

TECHNICAL FIELD

The present invention relates to a method of evaluating IBD (Inflammatory Bowel Disease), an amino acid data processor, an amino acid data-processing method, an amino acid data-processing system, an amino acid data-processing program and a recording medium, which utilize the concentration of amino acids in blood (plasma).

In the present specification, inflammatory bowel disease, Crohn disease and ulcerative colitis may be briefly indicated as IBD, CD and UC, respectively. Here, the term "CD" is an abbreviation for "Crohn disease." The term "UC" is an abbreviation for "ulcerative colitis."

In the present specification, the term "inflammatory bowel disease state is evaluated" includes, for example, "discrimination between inflammatory bowel disease and inflammatory bowel disease-free is conducted," " Crohn disease state is evaluated," "ulcerative colitis state is evaluated," and "discrimination between Crohn disease and ulcerative colitis is conducted." Furthermore, the term "Crohn disease state is evaluated" includes, for example, "discrimination between Crohn disease and Crohn disease-free is conducted," "discrimination between active phase and remission phase of Crohn disease is conducted," and "condition of Crohn disease is evaluated." The term "ulcerative colitis state is evaluated" includes, for example, "discrimination between ulcerative colitis and ulcerative colitis-free is conducted," "discrimination between active phase and remission phase of ulcerative colitis is conducted," and "condition of ulcerative colitis is evaluated." The term "discrimination between Crohn disease and ulcerative colitis is conducted" includes, for example, "discrimination between Crohn disease in active phase and ulcerative colitis in active phase is conducted."

BACKGROUND ART

Inflammatory bowel disease is a generic name for bowel diseases accompanied by inflammation, and major diseases among them include ulcerative colitis, Crohn disease and the like.

Ulcerative colitis is a diffuse non-specific inflammatory disease that mainly invades the mucosa or submucosa of large intestine, frequently forming erosion or ulcer therein. Clinical symptoms include femafecia, abdominal pain, hemafecia, watery stool, fever, anorexia, nausea, vomiting and the like. As the medicament for the treatment of ulcerative colitis, salazosulfapyridine, adrenocortical steroids, immunosuppressants, 5-aminosalicyclic acid (5-ASA), and the like are in use.

Crohn disease is idiopathic chronic enteritis of unknown origin, and is a disease showing non-specific inflammatory symptoms extended from the small intestine to the large intestine. Crohn disease includes granulomatous lesion accompanied by fibrosis or ulcer, and there is a possibility that the lesion may appear over the entire digestive tract from the oral cavity to the anus. Crohn disease often causes various digestive organ and extra-intestinal symptoms (for example, serious symptoms such as intestinal stricture, intestinal perforation, abdominal abscess and hematorrhea) simultaneously, in addition to dystrophy, and requires remedy such as intestinal surgery. Treatment of Crohn disease is achieved in Japan by high calorie fluid or enteral nutrition therapy for the purpose of an improvement of nutritional status. Since the high calorie fluid increases the risk of bacterial translocation, enteral nutrition therapy is employed particularly for long-term treatment. For the treatment of Crohn disease, a treatment based on medicament is also attempted. In drug therapy, salazosulfapyridine, metronidazole, adrenocortical steroids, immunosuppressants, 5-aminosalicylic acid (5-ASA) and the like are mainly administered. Recently, administration of anti-TNF (tumor necrosis factor) antibodies has also been put into clinical use.

The disease state classification of ulcerative colitis includes disease type classification by extent of lesion (pancolitis, left sided colitis, proctitis, right sided or segmental colitis), classification by clinical severity (severe, moderate, mild), classification by disease phase (active phase, remission phase), classification by clinical course (recurrent remissive type, chronic persistent type, acute fulminant type, initial onset type), and the like. On the other hand, in most cases of Crohn disease, lesions such as longitudinal ulcer, cobblestone appearance or aphtha occur in the small intestine or large intestine, or in both. The disease type of Crohn disease is classified into small intestinal type, small intestinal and large intestinal type, large intestinal type and the like, based on longitudinal ulcer, cobblestone appearance or the site of presence of stricture. Since both of ulcerative colitis and Crohn disease repeatedly undergo recurrence and remission, long-term medical care is necessary (see Nonpatent Literature 1).

However, differential diagnosis between inflammatory bowel disease and non-inflammatory bowel disease is performed by diagnosis by exclusion of infectious enteritis or the like, and by verification of characteristic finding based on enema X-ray examination or colonoscopic examination of the entire large intestine, or of the pathological appearance upon biopsy (see Nonpatent Literature 2 and Nonpatent Literature 3). Differential diagnosis between Crohn disease and ulcerative colitis is performed by comparison of the difference in the frequent site and of the pathological appearance upon biopsy (see Nonpatent Literature 2, Nonpatent Literature 3 and Nonpatent Literature 4).

In regard to changes in the condition of disease, the state of inflammation is figured out by checking increase in the erythrocyte sedimentation rate, elevation of CRP (C-reactive protein), increase in the leukocyte count, increase in the platelet level, or the like, and the state of undernutrition is figured out by checking decrease in the albumin level, decrease in the total serum cholesterol level, or the like. Furthermore, CDAI (Crohn's Disease Activity Index) of Crohn disease (see Nonpatent Literature 5) or CAI (Clinical Activity Index) of ulcerative colitis (see Nonpatent Literature 6) is used as an index for the diagnosis of disease condition.

As for the diagnostic marker of the current research stage, fecal calprotectin, ASCA (anti-saccharomyces cerevisiae antibody), ANCA (anti-neutrophil cytoplasmic antibody) and OmpC antibodies, I2, and the like are known (see Nonpatent Literature 7 and Nonpatent Literature 8). Also, markers that have been hitherto published in patents include polynucleotides that are portions of the base sequence of BAFF (B cell activating factor belonging to the TNF family) gene (see Patent Document 1), C8FW gene or C8FW protein (see Patent Document 2), a method for immunological assay of anti-pig amylase antibody (see Patent Document 3), and the amount of platelet-leukocyte complex (see Patent Document 4).

Nonpatent Literature 1: Official website for Japan Intractable Diseases Information Center, the Ministry of Health, Labour and Welfare (address (as of January 10, 2007): http://www.nanbyou.or.jp/top.html).

Nonpatent Literature 2: Munakata, Akihiro, Revised Diagnostic Criteria for Ulcerative Colitis (Research Report in 1997 by the Division for Investigation and Research on Intractable Inflammatory Bowel Disorders as Diseases Specified by the Ministry of Health, Labour and Welfare), p. 96, 1998.

Nonpatent Literature 3: Hiwatashi, Nobuo, Diagnostic Criteria for Crohn disease (2002) (Research Report in 2001 by the Division for "Investigation and Research on Intractable Inflammatory Bowel Disorders" as Research Project on Measures for Specified Diseases by Grants for Health Science), p.76-77, 2002.

Nonpatent Literature 4: "Simple mucosal biopsy criteria differentiating among Crohn disease, ulcerative colitis, and other forms of colitis: measurement of validity.," Scand J Gastroenterol., 2000, Mar, 35 (3), 281-6.

Nonpatent Literature 5: "Rederived values of the eight coefficients of the Crohn's Disease Activity Index (CDAI).," Gastroenterology., 1979, Oct, 77 (4 Pt 2), 843-6. Nonpatent Literature 6: Harvey RF, Bradshaw JM., "A simple index of Crohn's-disease activity.," Lancet, 1980, 1, 514. Nonpatent Literature 7: "Faecal calprotectin: a new marker for Crohn's disease?," Ann Clin Biochem., 2004, May, 41 (Pt 3), 230-2.

Nonpatent Literature 8: "The Montreal classification of inflammatory bowel disease: controversies, consensus, and implications.," Gut., 2006, Jun, 55 (6), 749-53.

Patent Document 1: JP-A-2004-135546
Patent Document 2: JP-A-2004-321179
Patent Document 3: JP-A-11-190734
Patent Document 4: JP-A-2003-232788

DISCLOSURE OF INVENTION

PROBLEM TO BE SOLVED BY THE INVENTION

However, the differential diagnosis between inflammatory bowel disease and inflammatory bowel disease-free and the differential diagnosis between Crohn disease and ulcerative colitis at present all require advanced knowledge of medicine, and require judgment by a medical specialist. Thus, there is a problem that it takes time to obtain definitive diagnosis under the current circumstances. Furthermore, the CDAI of Crohn disease or CAI of ulcerative colitis, although used as an index for the diagnosis of disease condition, is an index calculated on the basis of information including subjective terms, and has a problem that the index is poor in convenience, such as since the health status for one week should be recorded every day. The diagnostic markers that are currently proposed also have a problem that the condition of disease cannot be quantitatively evaluated. The markers disclosed in Patent Document 1 to Patent Document 4 are essentially incapable of diagnosing the condition of disease. Moreover, there has been hitherto a problem that a technology for diagnosing the presence or absence of the onset of IBD by focusing on the concentration of amino acids, has not been developed, and such technology is not in practical use.

The present invention is made in view of the problem described above, and an object of the present invention is to provide a method of evaluating IBD, an amino acid data processor, an amino acid data-processing method, an amino acid data-processing system, an amino acid data-processing program, and a recording medium, which are capable of evaluating an inflammatory bowel disease state accurately by utilizing the concentration of amino acids in blood.

MEANS FOR SOLVING PROBLEMS

The inventors of the present invention devotedly conducted an investigation to solve the problems described above, and as a result, they identified amino acids which are useful in the discrimination between 2 groups of inflammatory bowel disease and inflammatory bowel disease-free, discrimination between 2 groups of Crohn disease and Crohn disease-free, discrimination between 2 groups of active phase and remission phase of Crohn disease, discrimination between 2 groups of ulcerative colitis and ulcerative colitis-free, discrimination between 2 groups of active phase and remission phase of ulcerative colitis, discrimination between 2 groups of Crohn disease and ulcerative colitis, and discrimination between 2 groups of Crohn disease in active phase and ulcerative colitis in active phase, respectively (specifically, amino acids which vary with a statistically significant difference between 2 groups of inflammatory bowel disease and inflammatory bowel disease-free, amino acids which vary with a statistically significant difference between 2 groups of Crohn disease and Crohn disease-free, amino acids which vary with a statistically significant difference between 2 groups of active phase and remission phase of Crohn disease, amino acids which vary with a statistically significant difference between 2 groups of ulcerative colitis and ulcerative colitis-free, amino acids which vary with a statistically significant difference between 2 groups of active phase and remission phase of ulcerative colitis, amino acids which vary with a statistically significant difference between 2 groups of Crohn disease and ulcerative colitis, and amino acids which vary with a statistically significant difference between 2 groups of Crohn disease in active phase and ulcerative colitis in active phase), and found that a multivariate discriminant (index formula, correlation equation) including the concentrations of the identified amino acids as explanatory variables, correlates significantly with the state of inflammatory bowel disease (specifically, the condition of disease, discrimination between 2 groups of inflammatory bowel disease and inflammatory bowel disease-free, discrimination between 2 groups of Crohn disease and Crohn disease-free, discrimination between 2 groups of active phase and remission phase of Crohn disease, discrimination between 2 groups of ulcerative colitis and ulcerative colitis-free, discrimination between 2 groups of active phase and remission phase of ulcerative colitis, discrimination between 2 groups of Crohn disease and ulcerative colitis, and discrimination between 2 groups of Crohn disease in active phase and ulcerative colitis in active phase). Thus, the present invention was completed.

In other words, the inventors of the present invention calculated, for the purpose of performing diagnosis in consideration of clinical needs, an index with respect to the discrimination between 2 groups of a group of healthy subjects and a group of inflammatory bowel disease patients (Crohn disease and ulcerative colitis), discrimination between 2 groups of Crohn disease and ulcerative colitis, discrimination between 2 groups of Crohn disease in active phase and ulcerative colitis in active phase, discrimination between 2 groups of active phase of Crohn disease and remission phase of Crohn disease, and discrimination between 2 groups of active phase of ulcerative colitis and remission phase of ulcerative colitis. The inventors also paid attention to the difference between the concentration of amino acids in the blood plasma of a healthy subject and the concentration of amino acids in the blood plasma of an inflammatory bowel disease patient, and developed an index for evaluating inflammatory bowel disease patients conveniently and highly accurately by measuring the concentration of amino acids in blood plasma. The inventors also paid attention to the difference between the concentration of amino acids in the blood plasma in the active phase of inflammatory bowel disease and the concentration of amino acids in the blood plasma in the remission phase of inflammatory bowel disease, and developed an index for evaluating the condition of an inflammatory bowel disease patient objectively and highly accurately by measuring the concentration of amino acids in blood plasma. If the amino acids in blood plasma are used, measurement can be made inexpensively, rapidly and conveniently, the possibility of a widespread use as an index is high, and inflammatory bowel disease can be evaluated objectively.

To solve the problem and achieve the object described above, a method of evaluating IBD according to one aspect of the present invention includes a measuring step of measuring amino acid concentration data on a concentration value of amino acid in blood collected from a subject to be evaluated, and a concentration value criterion evaluating step of evaluating an inflammatory bowel disease state in the subject, based on the amino acid concentration data of the subject measured at the measuring step.

Another aspect of the present invention is the method of evaluating IBD, wherein the concentration value criterion evaluating step further includes a concentration value criterion discriminating step of discriminating between inflammatory bowel disease and inflammatory bowel disease-free in the subject, based on the concentration value of at least one of Tau, Urea, Asn, Glu, Gln, Pro, Ala, Cit, ABA, Val, Cys, Met, Ile, Leu, Tyr, Phe, His, Trp, Orn and Lys contained in the amino acid concentration data of the subject measured at the measuring step.

Still another aspect of the present invention is the method of evaluating IBD, wherein the concentration value criterion evaluating step further includes a discriminant value calculating step of calculating a discriminant value that is a value of multivariate discriminant, based on both the concentration value of at least one of Tau, Urea, Asn, Glu, Gln, Pro, Ala, Cit, Gly, ABA, Val, Cys, Leu, Tyr, Phe, His, Trp, Orn and Lys contained in the amino acid concentration data of the subject measured at the measuring step and a previously established multivariate discriminant with the concentration of the amino acid as explanatory variable, where the concentration value of at least one of Tau, Urea, Asn, Glu, Gln, Pro, Ala, Cit, Gly, ABA, Val, Cys, Leu, Tyr, Phe, His, Trp, Orn and Lys is contained as the explanatory variable, and a discriminant value criterion discriminating step of discriminating between inflammatory bowel disease and inflammatory bowel disease-free in the subject, based on the discriminant value calculated at the discriminant value calculating step.

Still another aspect of the present invention is the method of evaluating IBD, wherein the multivariate discriminant is expressed by one fractional expression or the sum of a plurality of the fractional expressions, and contains the concentration value of at least one of Tau, Glu, Pro and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contains the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau, Glu, Pro and Cys as the explanatory variable in the denominator in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Tau, Leu, Tyr, His, Ile and Thr as the explanatory variables.

Still another aspect of the present invention is the method of evaluating IBD, wherein at the concentration value criterion evaluating step, Crohn disease state or ulcerative colitis state in the subject is evaluated, based on the amino acid concentration data of the subject measured at the measuring step.

Still another aspect of the present invention is the method of evaluating IBD, wherein the concentration value criterion evaluating step further includes a concentration value criterion discriminating step of discriminating between Crohn disease and Crohn disease-free in the subject, based on the concentration value of at least one of Tau, Thr, Urea, Asn, Glu, Gln, Pro, Gly, ABA, Val, Cys, Leu, Tyr, Phe, His, Trp and Lys contained in the amino acid concentration data of the subject measured at the measuring step, or discriminating between ulcerative colitis and ulcerative colitis-free in the subject, based on the concentration value of at least one of Tau, Urea, Asn, Gln, Ala, Cit, ABA, Val, Cys, Met, Ile, Leu, Tyr, Phe, His, Trp, Orn and Lys contained in the amino acid concentration data of the subject measured at the measuring step.

Still another aspect of the present invention is the method of evaluating IBD, wherein the concentration value criterion evaluating step further includes a concentration value criterion discriminating step of discriminating between active phase and remission phase of Crohn disease or ulcerative colitis in the subject, based on the amino acid concentration data of the subject measured at the measuring step.

Still another aspect of the present invention is the method of evaluating IBD, wherein at the concentration value criterion discriminating step, discrimination between active phase and remission phase of Crohn disease in the subject is conducted based on the concentration value of at least one of Gln, Cit, Val, Leu, His, Trp, Lys and Arg contained in the amino acid concentration data of the subject measured at the measuring step, or discrimination between active phase and remission phase of ulcerative colitis in the subject is conducted based on the concentration value of at least one of Urea, Gln, Thr, Asn, Pro, Ala, ABA, Val, Met, Leu, Tyr, Phe, His, Trp, Lys, Arg and Cys contained in the amino acid concentration data of the subject measured at the measuring step.

Still another aspect of the present invention is the method of evaluating IBD, wherein the concentration value criterion evaluating step further includes a discriminant value calculating step of calculating a discriminant value that is a value of multivariate discriminant, based on both the concentration value of at least one of Tau, Thr, Urea, Asn, Glu, Gln, Pro, Gly, ABA, Val, Cys, Leu, Tyr, Phe, His, Trp and Lys contained in the amino acid concentration data of the subject measured at the measuring step and a previously established multivariate discriminant with the concentration of the amino acid as explanatory variable, where the concentration value of at least one of Tau, Thr, Urea, Asn, Glu, Gln, Pro, Gly, ABA, Val, Cys, Leu, Tyr, Phe, His, Trp and Lys is contained as the explanatory variable, or calculating a discriminant value that is a value of multivariate discriminant, based on both the concentration value of at least one of Tau, Urea, Asn, Gln, Ala, Cit, ABA, Val, Cys, Met, Ile, Leu, Tyr, Phe, His, Trp, Orn and Lys contained in the amino acid concentration data of the subject measured at the measuring step and a previously established multivariate discriminant with the concentration of the amino acid as explanatory variable, where the concentration value of at least one of Tau, Urea, Asn, Gln, Ala, Cit, ABA, Val, Cys, Met, Ile, Leu, Tyr, Phe, His, Trp, Orn and Lys is contained as the explanatory variable, and a discriminant value criterion discriminating step of discriminating between Crohn disease and Crohn disease-free in the subject, based on the discriminant value calculated at the discriminant value calculating step, or discriminating between ulcerative colitis and ulcerative colitis-free in the subject, based on the discriminant value calculated at the discriminant value calculating step.

Still another aspect of the present invention is the method of evaluating IBD, wherein when discrimination between Crohn disease and Crohn disease-free is conducted at the discriminant value criterion discriminating step, the multivariate discriminant is expressed by one fractional expression or the sum of a plurality of fractional expressions, and contains the concentration value of at least one of Tau, Glu, Pro and Gly as the explanatory variable in the numerator in the fractional expression constituting the multivariate discriminant, and the concentration value of at least one of Urea, Asn, Gln, Thr, ABA, Val, Cys, Leu, Tyr, Phe, His, Trp and Lys as the explanatory variable in the denominator in the fractional expression constituting the multivariate discriminant, or contains the concentration value of at least one of Urea, Asn, Gln, Thr, ABA, Val, Cys, Leu, Tyr, Phe, His, Trp and Lys as the explanatory variable in the numerator in the fractional expression constituting the multivariate discriminant, and the concentration value of at least one of Tau, Glu, Pro and Gly as the explanatory variable in the denominator in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Tau, Val, Leu, Tyr, Pro and Ile as the explanatory variables. When discrimination between ulcerative colitis and ulcerative colitis-free is conducted at the discriminant value criterion discriminating step, the multivariate discriminant is expressed by one fractional expression or the sum of a plurality of fractional expressions, and contains the concentration value of at least one of Tau and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contains the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau and Cys as the explanatory variable in the denominator in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Tau, Tyr, Leu, His, Ala and Ile as the explanatory variables.

Still another aspect of the present invention is the method of evaluating IBD, wherein the concentration value criterion evaluating step further includes a discriminant value calculating step of calculating a discriminant value that is a value of multivariate discriminant, based on both the amino acid concentration data of the subject measured at the measuring step and a previously established multivariate discriminant with the concentration of the amino acid as explanatory variable, and a discriminant value criterion discriminating step of discriminating between active phase and remission phase of Crohn disease or ulcerative colitis in the subject, based on the discriminant value calculated at the discriminant value calculating step.

Still another aspect of the present invention is the method of evaluating IBD, wherein at the measuring step, a plurality of the amino acid concentration data are measured from every blood sample of the plurality of blood samples collected from the subject. The concentration value criterion evaluating step further includes a discriminant value calculating step of calculating a discriminant value that is a value of multivariate discriminant, based on both the plurality of the amino acid concentration data of the subject measured at the measuring step and a previously established multivariate discriminant with the concentration of the amino acid as explanatory variable, for each of the plurality of the amino acid concentration data, and a discriminant value criterion condition evaluating step of evaluating a condition of Crohn disease or ulcerative colitis in the subject, based on the plurality of discriminant values calculated at the discriminant value calculating step.

Still another aspect of the present invention is the method of evaluating IBD, wherein when discrimination between active phase and remission phase of Crohn disease is conducted at the discriminant value criterion discriminating step, or when the condition of Crohn disease is evaluated at the discriminant value criterion condition evaluating step, at the discriminant value calculating step, one or a plurality of the discriminant values are calculated based on both the concentration value of at least one of Gln, Cit, Val, Leu, His, Trp, Lys and Arg contained in one or a plurality of the amino acid concentration data of the subject measured at the measuring step, and the multivariate discriminant containing the concentration value of at least one of Gln, Cit, Val, Leu, His, Trp, Lys and Arg as the explanatory variable. When discrimination between active phase and remission phase of ulcerative colitis is conducted at the discriminant value criterion discriminating step, or when the condition of ulcerative colitis is evaluated at the discriminant value criterion condition evaluating step, at the discriminant value calculating step, one or a plurality of the discriminant values are calculated based on both the concentration value of at least one of Tau, Urea, Thr, Asn, Pro, Gln, Ala, Cit, ABA, Val, Met, Leu, Tyr, Phe, His, Trp, Lys, Arg and Cys contained in one or a plurality of the amino acid concentration data of the subject measured at the measuring step, and the multivariate discriminant containing the concentration value of at least one of Tau, Urea, Thr, Asn, Pro, Gln, Ala, Cit, ABA, Val, Met, Leu, Tyr, Phe, His, Trp, Lys, Arg and Cys as the explanatory variable.

Still another aspect of the present invention is the method of evaluating IBD, wherein when discrimination between active phase and remission phase of Crohn disease is conducted at the discriminant value criterion discriminating step, or when the condition of Crohn disease is evaluated at the discriminant value criterion condition evaluating step, the multivariate discriminant is expressed by one fractional expression or the sum of a plurality of fractional expressions and contains the concentration value of at least one of Gln, Cit, Val, Leu, His, Trp, Lys and Arg as the explanatory variable in any one of the numerator and denominator or both in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of His, Trp, Lys, Ser, Tau and Ile as the explanatory variables. When discrimination between active phase and remission phase of ulcerative colitis is conducted at the discriminant value criterion discriminating step, or when the condition of ulcerative colitis is evaluated at the discriminant value criterion condition evaluating step, the multivariate discriminant is expressed by one fractional expression or the sum of a plurality of fractional expressions and contains the concentration value of at least one of Tau, Urea, Thr, Asn, Pro, Gln, Ala, Cit, ABA, Val, Met, Leu, Tyr, Phe, His, Trp, Lys, Arg and Cys as the explanatory variable in any one of the numerator and denominator or both in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' distance method, a discriminant prepared by canonical discriminant analysis and a discriminant prepared by a decision tree, and has the concentration value of His, Tyr, Val, Met, Leu and Arg as the explanatory variables.

Still another aspect of the present invention is the method of evaluating IBD, wherein the concentration value criterion evaluating step further includes a concentration value criterion discriminating step of discriminating between Crohn disease and ulcerative colitis in the subject, based on the amino acid concentration data of the subject measured at the measuring step.

Still another aspect of the present invention is the method of evaluating IBD, wherein at the concentration value criterion discriminating step, discrimination between Crohn disease and ulcerative colitis in the subject is conducted based on the concentration value of at least one of Cys, ABA, Thr, Pro, Gly, Met, Ile and Orn contained in the amino acid concentration data of the subject measured at the measuring step.

Still another aspect of the present invention is the method of evaluating IBD, wherein at the concentration value criterion discriminating step, discrimination between Crohn disease in active phase and ulcerative colitis in active phase in the subject is conducted based on the amino acid concentration data of the subject measured at the measuring step.

Still another aspect of the present invention is the method of evaluating IBD, wherein at the concentration value criterion discriminating step, discrimination between Crohn disease in active phase and ulcerative colitis in active phase in the subject is conducted based on the concentration value of at least one of Tau, Thr, Ser, Gly, Met and Ile contained in the amino acid concentration data of the subject measured at the measuring step.

Still another aspect of the present invention is the method of evaluating IBD, wherein the concentration value criterion evaluating step further includes a discriminant value calculating step of calculating a discriminant value that is a value of multivariate discriminant, based on both the amino acid concentration data of the subject measured at the measuring step, and a previously established multivariate discriminant with the concentration of the amino acid as explanatory variable, and a discriminant value criterion discriminating step of discriminating between Crohn disease and ulcerative colitis in the subject, based on the discriminant value calculated at the discriminant value calculating step.

Still another aspect of the present invention is the method of evaluating IBD, wherein at the discriminant value calculating step, the discriminant value is calculated based on both the concentration value of at least one of Cys, ABA, Thr, Pro, Gly, Met, Ile and Orn contained in the amino acid concentration data of the subject measured at the measuring step, and the previously established multivariate discriminant containing the concentration value of at least one of Cys, ABA, Thr, Pro, Gly, Met, Ile and Orn as the explanatory variable.

Still another aspect of the present invention is the method of evaluating IBD, wherein the multivariate discriminant is expressed by one fractional expression or the sum of a plurality of fractional expressions, and contains the concentration value of at least one of Cys and ABA as the explanatory variable in the numerator in the fractional expression constituting the multivariate discriminant and the concentration value of at least one of Thr, Pro, Gly, Met, Ile and Orn as the explanatory variable in the denominator in the fractional expression constituting the multivariate discriminant, or contains the concentration value of at least one of Thr, Pro, Gly, Met, Ile and Orn as the explanatory variable in the numerator in the fractional expression constituting the multivariate discriminant and the concentration value of at least one of Cys and ABA as the explanatory variable in the denominator in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Met, Pro, Ile, Trp, Tau and Val as the explanatory variables.

Still another aspect of the present invention is the method of evaluating IBD, wherein at the discriminant value criterion discriminating step, discrimination between Crohn disease in active phase and ulcerative colitis in active phase in the subject is conducted based on the discriminant value calculated at the discriminant value calculating step.

Still another aspect of the present invention is the method of evaluating IBD, wherein at the discriminant value calculating step, the discriminant value is calculated based on both the concentration value of at least one of Tau, Thr, Ser, Gly, Met and Ile contained in the amino acid concentration data of the subject measured at the measuring step, and the multivariate discriminant containing the concentration value of at least one of Tau, Thr, Ser, Gly, Met and Ile as the explanatory variable.

Still another aspect of the present invention is the method of evaluating IBD, wherein the multivariate discriminant is expressed by one fractional expression or the sum of a plurality of fractional expressions, and contains the concentration value of at least one of Tau, Thr, Ser, Gly, Met and Ile as the explanatory variable in any one of the numerator and denominator or both in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Met, Tau, Trp, Tyr, Orn and Phe as the explanatory variables.

The present invention also relates to an amino acid data processor, the amino acid data processor according to one aspect of the present invention includes a control unit and a memory unit to discriminate between inflammatory bowel disease and inflammatory bowel disease-free in a subject to be evaluated. The control unit includes a discriminant value-calculating unit that calculates a discriminant value that is a value of multivariate discriminant, based on both the concentration value of at least one of Tau, Urea, Asn, Glu, Gln, Pro, Ala, Cit, Gly, ABA, Val, Cys, Leu, Tyr, Phe, His, Trp, Orn and Lys contained in previously obtained amino acid concentration data on a concentration value of amino acid in the subject and a multivariate discriminant with the concentration of the amino acid as explanatory variable stored in the memory unit, where the concentration value of at least one of Tau, Urea, Asn, Glu, Gln, Pro, Ala, Cit, Gly, ABA, Val, Cys, Leu, Tyr, Phe, His, Trp, Orn and Lys is contained as the explanatory variable, and a discriminant value criterion-discriminating unit that discriminates between inflammatory bowel disease and inflammatory bowel disease-free in the subject, based on the discriminant value calculated by the discriminant value-calculating unit.

Another aspect of the present invention is the amino acid data processor, wherein the multivariate discriminant is expressed by one fractional expression or the sum of a plurality of the fractional expressions, and contains the concentration value of at least one of Tau, Glu, Pro and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contains the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau, Glu, Pro and Cys as the explanatory variable in the denominator in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Tau, Leu, Tyr, His, Ile and Thr as the explanatory variables.

An amino acid data processor according to one aspect of the present invention includes a control unit and a memory unit to discriminate between Crohn disease and Crohn disease-free or between ulcerative colitis and ulcerative colitis-free in a subject to be evaluated. The control unit includes a discriminant value-calculating unit that calculates a discriminant value that is a value of multivariate discriminant, based on both the concentration value of at least one of Tau, Thr, Urea, Asn, Glu, Gln, Pro, Gly, ABA, Val, Cys, Leu, Tyr, Phe, His, Trp and Lys contained in previously obtained amino acid concentration data on a concentration value of amino acid in the subject and a multivariate discriminant with the concentration of the amino acid as explanatory variable stored in the memory unit, where the concentration value of at least one of Tau, Thr, Urea, Asn, Glu, Gln, Pro, Gly, ABA, Val, Cys, Leu, Tyr, Phe, His, Trp and Lys is contained as the explanatory variable, or calculates a discriminant value that is a value of multivariate discriminant, based on both the concentration value of at least one of Tau, Urea, Asn, Gln, Ala, Cit, ABA, Val, Cys, Met, Ile, Leu, Tyr, Phe, His, Trp, Orn and Lys contained in previously obtained amino acid concentration data on a concentration value of amino acid in the subject and a multivariate discriminant with the concentration of the amino acid as explanatory variable stored in the memory unit, where the concentration value of at least one of Tau, Urea, Asn, Gln, Ala, Cit, ABA, Val, Cys, Met, Ile, Leu, Tyr, Phe, His, Trp, Orn and Lys is contained as the explanatory variable, and a discriminant value criterion-discriminating unit that discriminates between Crohn disease and Crohn disease-free in the subject, based on the discriminant value calculated by the discriminant value-calculating unit, or discriminates between ulcerative colitis and ulcerative colitis-free in the subject, based on the discriminant value calculated by the discriminant value-calculating unit.

Another aspect of the present invention is the amino acid data processor, wherein when the discriminant value criterion-discriminating unit discriminates between Crohn disease and Crohn disease-free, the multivariate discriminant is expressed by one fractional expression or the sum of a plurality of fractional expressions, and contains the concentration value of at least one of Tau, Glu, Pro and Gly as the explanatory variable in the numerator in the fractional expression constituting the multivariate discriminant, and the concentration value of at least one of Urea, Asn, Gln, Thr, ABA, Val, Cys, Leu, Tyr, Phe, His, Trp and Lys as the explanatory variable in the denominator in the fractional expression constituting the multivariate discriminant, or contains the concentration value of at least one of Urea, Asn, Gln, Thr, ABA, Val, Cys, Leu, Tyr, Phe, His, Trp and Lys as the explanatory variable in the numerator in the fractional expression constituting the multivariate discriminant, and the concentration value of at least one of Tau, Glu, Pro and Gly as the explanatory variable in the denominator in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Tau, Val, Leu, Tyr, Pro and Ile as the explanatory variables. When the discriminant value criterion-discriminating unit discriminates between ulcerative colitis and ulcerative colitis-free, the multivariate discriminant is expressed by one fractional expression or the sum of a plurality of fractional expressions, and contains the concentration value of at least one of Tau and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contains the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau and Cys as the explanatory variable in the denominator in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Tau, Tyr, Leu, His, Ala and Ile as the explanatory variables.

An amino acid data processor according to one aspect of the present invention includes a control unit and a memory unit to discriminate between active phase and remission phase of Crohn disease or ulcerative colitis in a subject to be evaluated. The control unit includes a discriminant value-calculating unit that calculates a discriminant value that is a value of multivariate discriminant, based on both previously obtained amino acid concentration data on a concentration value of amino acid in the subject and a multivariate discriminant with the concentration of the amino acid as explanatory variable stored in the memory unit, and a discriminant value criterion-discriminating unit that discriminates between active phase and remission phase of Crohn disease or ulcerative colitis in the subject, based on the discriminant value calculated by the discriminant value-calculating unit.

An amino acid data processor according to one aspect of the present invention includes a control unit and a memory unit to evaluate a condition of Crohn disease or ulcerative colitis in a subject to be evaluated. The control unit includes a discriminant value-calculating unit that calculates a discriminant value that is a value of multivariate discriminant, based on both a plurality of previously obtained amino acid concentration data on a concentration value of amino acid in the subject and a multivariate discriminant with the concentration of the amino acid as explanatory variable stored in the memory unit, for each of the plurality of the amino acid concentration data, and a discriminant value criterion-condition evaluating unit that evaluates the condition of Crohn disease or ulcerative colitis in the subject, based on the plurality of the discriminant values calculated by the discriminant value-calculating unit.

Another aspect of the present invention is the amino acid data processor, wherein when the discriminant value criterion-discriminating unit discriminates between active phase and remission phase of Crohn disease, or when the discriminant value criterion-condition evaluating unit evaluates the condition of Crohn disease, the discriminant value-calculating unit calculates one or a plurality of the discriminant values, based on both the concentration value of at least one of Gln, Cit, Val, Leu, His, Trp, Lys and Arg contained in one or a plurality of the amino acid concentration data, and the multivariate discriminant containing the concentration value of at least one of Gln, Cit, Val, Leu, His, Trp, Lys and Arg as the explanatory variable. When the discriminant value criterion-discriminating unit discriminates between active phase and remission phase of ulcerative colitis, or when the discriminant value criterion-condition evaluating unit evaluates the condition of ulcerative colitis, the discriminant value-calculating unit calculates one or a plurality of the discriminant values, based on both the concentration value of at least one of Tau, Urea, Thr, Asn, Pro, Gln, Ala, Cit, ABA, Val, Met, Leu, Tyr, Phe, His, Trp, Lys, Arg and Cys contained in one or a plurality of the amino acid concentration data, and the multivariate discriminant containing the concentration value of at least one of Tau, Urea, Thr, Asn, Pro, Gln, Ala, Cit, ABA, Val, Met, Leu, Tyr, Phe, His, Trp, Lys, Arg and Cys as the explanatory variable.

Still another aspect of the present invention is the amino acid data processor, wherein when the discriminant value criterion-discriminating unit discriminates between active phase and remission phase of Crohn disease, or when the discriminant value criterion-condition evaluating unit evaluates the condition of Crohn disease, the multivariate discriminant is expressed by one fractional expression or the sum of a plurality of fractional expressions and contains the concentration value of at least one of Gln, Cit, Val, Leu, His, Trp, Lys and Arg as the explanatory variable in any one of the numerator and denominator or both in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of His, Trp, Lys, Ser, Tau and Ile as the explanatory variables. When the discriminant value criterion-discriminating unit discriminates between active phase and remission phase of ulcerative colitis, or when the discriminant value criterion-condition evaluating unit evaluates the condition of ulcerative colitis, the multivariate discriminant is expressed by one fractional expression or the sum of a plurality of fractional expressions and contains the concentration value of at least one of Tau, Urea, Thr, Asn, Pro, Gln, Ala, Cit, ABA, Val, Met, Leu, Tyr, Phe, His, Trp, Lys, Arg and Cys as the explanatory variable in any one of the numerator and denominator or both in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' distance method, a discriminant prepared by canonical discriminant analysis and a discriminant prepared by a decision tree, and has the concentration value of His, Tyr, Val, Met, Leu and Arg as the explanatory variables.

An amino acid data processor according to one aspect of the present invention includes a control unit and a memory unit to discriminate between Crohn disease and ulcerative colitis in a subject to be evaluated. The control unit includes a discriminant value-calculating unit that calculates a discriminant value that is a value of multivariate discriminant, based on both previously obtained amino acid concentration data on a concentration value of amino acid in the subject and a multivariate discriminant with the concentration of the amino acid as explanatory variable stored in the memory unit, and a discriminant value criterion-discriminating unit that discriminates between Crohn disease and ulcerative colitis in the subject, based on the discriminant value calculated by the discriminant value-calculating unit.

Another aspect of the present invention is the amino acid data processor, wherein the discriminant value-calculating unit calculates the discriminant value that is a value of the multivariate discriminant, based on both the concentration value of at least one of Cys, ABA, Thr, Pro, Gly, Met, Ile and Orn contained in the amino acid concentration data, and the multivariate discriminant containing the concentration value of at least one of Cys, ABA, Thr, Pro, Gly, Met, Ile and Orn as the explanatory variable.

Still another aspect of the present invention is the amino acid data processor, wherein the multivariate discriminant is expressed by one fractional expression or the sum of a plurality of fractional expressions, and contains the concentration value of at least one of Cys and ABA as the explanatory variable in the numerator in the fractional expression constituting the multivariate discriminant and the concentration value of at least one of Thr, Pro, Gly, Met, Ile and Orn as the explanatory variable in the denominator in the fractional expression constituting the multivariate discriminant, or contains the concentration value of at least one of Thr, Pro, Gly, Met, Ile and Orn as the explanatory variable in the numerator in the fractional expression constituting the multivariate discriminant and the concentration value of at least one of Cys and ABA as the explanatory variable in the denominator in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Met, Pro, Ile, Trp, Tau and Val as the explanatory variables.

Still another aspect of the present invention is the amino acid data processor, wherein the discriminant value criterion-discriminating unit discriminates between Crohn disease in active phase and ulcerative colitis in active phase in the subject, based on the discriminant value calculated by the discriminant value-calculating unit.

Still another aspect of the present invention is the amino acid data processor, wherein the discriminant value-calculating unit calculates the discriminant value, based on both the concentration value of at least one of Tau, Thr, Ser, Gly, Met and Ile contained in the amino acid concentration data, and the multivariate discriminant containing the concentration value of at least one of Tau, Thr, Ser, Gly, Met and Ile as the explanatory variable.

Still another aspect of the present invention is the amino acid data processor, wherein the multivariate discriminant is expressed by one fractional expression or the sum of a plurality of fractional expressions, and contains the concentration value of at least one of Tau, Thr, Ser, Gly, Met and Ile as the explanatory variable in any one of the numerator and denominator or both in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Met, Tau, Trp, Tyr, Orn and Phe as the explanatory variables.

The present invention also relates to an amino acid data-processing method, one aspect of the present invention is the amino acid data-processing method of discriminating between inflammatory bowel disease and inflammatory bowel disease-free in a subject to be evaluated. The method is carried out with an information processing apparatus including a control unit and a memory unit. The method includes (i) a discriminant value calculating step of calculating a discriminant value that is a value of multivariate discriminant, based on both the concentration value of at least one of Tau, Urea, Asn, Glu, Gln, Pro, Ala, Cit, Gly, ABA, Val, Cys, Leu, Tyr, Phe, His, Trp, Orn and Lys contained in previously obtained amino acid concentration data on a concentration value of amino acid in the subject and a multivariate discriminant with the concentration of the amino acid as explanatory variable stored in the memory unit, where the concentration value of at least one of Tau, Urea, Asn, Glu, Gln, Pro, Ala, Cit, Gly, ABA, Val, Cys, Leu, Tyr, Phe, His, Trp, Orn and Lys is contained as the explanatory variable, and (ii) a discriminant value criterion discriminating step of discriminating between inflammatory bowel disease and inflammatory bowel disease-free in the subject, based on the discriminant value calculated at the discriminant value calculating step. The steps (i) and (ii) are executed by the control unit.

One aspect of the present invention is an amino acid data-processing method of discriminating between Crohn disease and Crohn disease-free or between ulcerative colitis and ulcerative colitis-free in a subject to be evaluated. The method is carried out with an information processing apparatus including a control unit and a memory unit. The method includes (i) a discriminant value calculating step of calculating a discriminant value that is a value of multivariate discriminant, based on both the concentration value of at least one of Tau, Thr, Urea, Asn, Glu, Gln, Pro, Gly, ABA, Val, Cys, Leu, Tyr, Phe, His, Trp and Lys contained in previously obtained amino acid concentration data on a concentration value of amino acid in the subject and a multivariate discriminant with the concentration of the amino acid as explanatory variable stored in the memory unit, where the concentration value of at least one of Tau, Thr, Urea, Asn, Glu, Gln, Pro, Gly, ABA, Val, Cys, Leu, Tyr, Phe, His, Trp and Lys is contained as the explanatory variable, or calculating a discriminant value that is a value of multivariate discriminant, based on both the concentration value of at least one of Tau, Urea, Asn, Gln, Ala, Cit, ABA, Val, Cys, Met, Ile, Leu, Tyr, Phe, His, Trp, Orn and Lys contained in previously obtained amino acid concentration data on a concentration value of amino acid in the subject and a multivariate discriminant with the concentration of the amino acid as explanatory variable stored in the memory unit, where the concentration value of at least one of Tau, Urea, Asn, Gln, Ala, Cit, ABA, Val, Cys, Met, Ile, Leu, Tyr, Phe, His, Trp, Orn and Lys is contained as the explanatory variable, and (ii) a discriminant value criterion discriminating step of discriminating between Crohn disease and Crohn disease-free in the subject, based on the discriminant value calculated at the discriminant value calculating step, or discriminating between ulcerative colitis and ulcerative colitis-free in the subject, based on the discriminant value calculated at the discriminant value calculating step. The steps (i) and (ii) are executed by the control unit.

One aspect of the present invention is an amino acid data-processing method of discriminating between active phase and remission phase of Crohn disease or ulcerative colitis in a subject to be evaluated. The method is carried out with an information processing apparatus including a control unit and a memory unit. The method includes (i) a discriminant value calculating step of calculating a discriminant value that is a value of multivariate discriminant, based on both previously obtained amino acid concentration data on a concentration value of amino acid in the subject and a multivariate discriminant with the concentration of the amino acid as explanatory variable stored in the memory unit, and (ii) a discriminant value criterion discriminating step of discriminating between active phase and remission phase of Crohn disease or ulcerative colitis in the subject, based on the discriminant value calculated at the discriminant value calculating step. The steps (i) and (ii) are executed by the control unit.

One aspect of the present invention is an amino acid data-processing method of evaluating a condition of Crohn disease or ulcerative colitis in a subject to be evaluated. The method is carried out with an information processing apparatus including a control unit and a memory unit. The method includes (i) a discriminant value calculating step of calculating a discriminant value that is a value of multivariate discriminant, based on both a plurality of previously obtained amino acid concentration data on a concentration value of amino acid in the subject and a multivariate discriminant with the concentration of the amino acid as explanatory variable stored in the memory unit, for each of the plurality of the amino acid concentration data, and (ii) a discriminant value criterion condition evaluating step of evaluating the condition of Crohn disease or ulcerative colitis in the subject, based on the plurality of the discriminant values calculated at the discriminant value calculating step. The steps (i) and (ii) are executed by the control unit.

One aspect of the present invention is an amino acid data-processing method of discriminating between Crohn disease and ulcerative colitis in a subject to be evaluated. The method is carried out with an information processing apparatus including a control unit and a memory unit. The method includes (i) a discriminant value calculating step of calculating a discriminant value that is a value of multivariate discriminant, based on both previously obtained amino acid concentration data on a concentration value of amino acid in the subject and a multivariate discriminant with the concentration of the amino acid as explanatory variable stored in the memory unit, and (ii) a discriminant value criterion discriminating step of discriminating between Crohn disease and ulcerative colitis in the subject, based on the discriminant value calculated at the discriminant value calculating step. The steps (i) and (ii) are executed by the control unit.

Another aspect of the present invention is the amino acid data-processing method, wherein at the discriminant value criterion discriminating step, discrimination between Crohn disease in active phase and ulcerative colitis in active phase in the subject is conducted based on the discriminant value calculated at the discriminant value calculating step.

The present invention also relates to an amino acid data-processing system, the amino acid data-processing system according to one aspect of the present invention includes an amino acid data processor including a control unit and a memory unit to discriminate between inflammatory bowel disease and inflammatory bowel disease-free in a subject to be evaluated and an information communication terminal apparatus that provides amino acid concentration data on the concentration value of amino acid in the subject connected to each other communicatively via a network. The information communication terminal apparatus includes an amino acid concentration data-sending unit that transmits the amino acid concentration data of the subject to the amino acid data processor, and a discrimination result-receiving unit that receives the discrimination result as to discrimination between inflammatory bowel disease and inflammatory bowel disease-free of the subject transmitted from the amino acid data processor. The control unit of the amino acid data processor includes an amino acid concentration data-receiving unit that receives the amino acid concentration data of the subject transmitted from the information communication terminal apparatus, a discriminant value-calculating unit that calculates a discriminant value that is a value of multivariate discriminant, based on both the concentration value of at least one of Tau, Urea, Asn, Glu, Gln, Pro, Ala, Cit, Gly, ABA, Val, Cys, Leu, Tyr, Phe, His, Trp, Orn and Lys contained in the amino acid concentration data of the subject received by the amino acid concentration data-receiving unit and a multivariate discriminant with the concentration of the amino acid as explanatory variable stored in the memory unit, where the concentration value of at least one of Tau, Urea, Asn, Glu, Gln, Pro, Ala, Cit, Gly, ABA, Val, Cys, Leu, Tyr, Phe, His, Trp, Orn and Lys is contained as the explanatory variable, a discriminant value criterion-discriminating unit that discriminates between inflammatory bowel disease and inflammatory bowel disease-free in the subject, based on the discriminant value calculated by the discriminant value-calculating unit, and a discrimination result-sending unit that transmits the discrimination result of the subject obtained by the discriminant value criterion-discriminating unit to the information communication terminal apparatus.

An amino acid data-processing system according to one aspect of the present invention includes an amino acid data processor including a control unit and a memory unit to discriminate between Crohn disease and Crohn disease-free or between ulcerative colitis and ulcerative colitis-free in a subject to be evaluated and an information communication terminal apparatus that provides amino acid concentration data on the concentration value of amino acid in the subject connected to each other communicatively via a network. The information communication terminal apparatus includes an amino acid concentration data-sending unit that transmits the amino acid concentration data of the subject to the amino acid data processor, and a discrimination result-receiving unit that receives the discrimination result as to discrimination between Crohn disease and Crohn disease-free or between ulcerative colitis and ulcerative colitis-free of the subject transmitted from the amino acid data processor. The control unit of the amino acid data processor includes an amino acid concentration data-receiving unit that receives the amino acid concentration data of the subject transmitted from the information communication terminal apparatus, a discriminant value-calculating unit that calculates a discriminant value that is a value of multivariate discriminant, based on both the concentration value of at least one of Tau, Thr, Urea, Asn, Glu, Gln, Pro, Gly, ABA, Val, Cys, Leu, Tyr, Phe, His, Trp and Lys contained in the amino acid concentration data of the subject received by the amino acid concentration data-receiving unit and a multivariate discriminant with the concentration of the amino acid as explanatory variable stored in the memory unit, where the concentration value of at least one of Tau, Thr, Urea, Asn, Glu, Gln, Pro, Gly, ABA, Val, Cys, Leu, Tyr, Phe, His, Trp and Lys is contained as the explanatory variable, or calculates a discriminant value that is a value of multivariate discriminant, based on both the concentration value of at least one of Tau, Urea, Asn, Gln, Ala, Cit, ABA, Val, Cys, Met, Ile, Leu, Tyr, Phe, His, Trp, Orn and Lys contained in the amino acid concentration data of the subject received by the amino acid concentration data-receiving unit and a multivariate discriminant with the concentration of the amino acid as explanatory variable stored in the memory unit, where the concentration value of at least one of Tau, Urea, Asn, Gln, Ala, Cit, ABA, Val, Cys, Met, Ile, Leu, Tyr, Phe, His, Trp, Orn and Lys is contained as the explanatory variable, a discriminant value criterion-discriminating unit that discriminates between Crohn disease and Crohn disease-free in the subject, based on the discriminant value calculated by the discriminant value-calculating unit, or discriminates between ulcerative colitis and ulcerative colitis-free in the subject, based on the discriminant value calculated by the discriminant value-calculating unit, and a discrimination result-sending unit that transmits the discrimination result of the subject obtained by the discriminant value criterion-discriminating unit to the information communication terminal apparatus.

An amino acid data-processing system according to one aspect of the present invention includes an amino acid data processor including a control unit and a memory unit to discriminate between active phase and remission phase of Crohn disease or ulcerative colitis in a subject to be evaluated and an information communication terminal apparatus that provides amino acid concentration data on the concentration value of amino acid in the subject connected to each other communicatively via a network. The information communication terminal apparatus includes an amino acid concentration data-sending unit that transmits the amino acid concentration data of the subject to the amino acid data processor, and a discrimination result-receiving unit that receives the discrimination result as to discrimination between active phase and remission phase of Crohn disease or ulcerative colitis of the subject transmitted from the amino acid data processor. The control unit of the amino acid data processor includes an amino acid concentration data-receiving unit that receives the amino acid concentration data of the subject transmitted from the information communication terminal apparatus, a discriminant value-calculating unit that calculates a discriminant value that is a value of multivariate discriminant, based on both the amino acid concentration data of the subject received by the amino acid concentration data-receiving unit and a multivariate discriminant with the concentration of the amino acid as explanatory variable stored in the memory unit, a discriminant value criterion-discriminating unit that discriminates between active phase and remission phase of Crohn disease or ulcerative colitis in the subject, based on the discriminant value calculated by the discriminant value-calculating unit, and a discrimination result-sending unit that transmits the discrimination result of the subject obtained by the discriminant value criterion-discriminating unit to the information communication terminal apparatus.

An amino acid data-processing system according to one aspect of the present invention includes an amino acid data processor including a control unit and a memory unit to evaluate a condition of Crohn disease or ulcerative colitis in a subject to be evaluated and an information communication terminal apparatus that provides a plurality of amino acid concentration data on the concentration value of amino acid in the subject connected to each other communicatively via a network. The information communication terminal apparatus includes an amino acid concentration data-sending unit that transmits the plurality of the amino acid concentration data of the subject to the amino acid data processor, and an evaluation result-receiving unit that receives the evaluation result as to the condition of Crohn disease or ulcerative colitis of the subject transmitted from the amino acid data processor. The control unit of the amino acid data processor includes an amino acid concentration data-receiving unit that receives the plurality of the amino acid concentration data of the subject transmitted from the information communication terminal apparatus, a discriminant value-calculating unit that calculates a discriminant value that is a value of multivariate discriminant, based on both the plurality of the amino acid concentration data of the subject received by the amino acid concentration data-receiving unit and a multivariate discriminant with the concentration of the amino acid as explanatory variable stored in the memory unit, for each of the plurality of the amino acid concentration data, a discriminant value criterion-condition evaluating unit that evaluates the condition of Crohn disease or ulcerative colitis in the subject, based on the plurality of the discriminant values calculated by the discriminant value-calculating unit, and an evaluation result-sending unit that transmits the evaluation result of the subject obtained by the discriminant value criterion-condition evaluating unit to the information communication terminal apparatus.

An amino acid data-processing system according to one aspect of the present invention includes an amino acid data processor including a control unit and a memory unit to discriminate between Crohn disease and ulcerative colitis in a subject to be evaluated and an information communication terminal apparatus that provides amino acid concentration data on the concentration value of amino acid in the subject connected to each other communicatively via a network. The information communication terminal apparatus includes an amino acid concentration data-sending unit that transmits the amino acid concentration data of the subject to the amino acid data processor, and a discrimination result-receiving unit that receives the discrimination result as to discrimination between Crohn disease and ulcerative colitis of the subject transmitted from the amino acid data processor. The control unit of the amino acid data processor includes an amino acid concentration data-receiving unit that receives the amino acid concentration data of the subject transmitted from the information communication terminal apparatus, a discriminant value-calculating unit that calculates a discriminant value that is a value of multivariate discriminant, based on both the amino acid concentration data of the subject received by the amino acid concentration data-receiving unit and a multivariate discriminant with the concentration of the amino acid as explanatory variable stored in the memory unit, a discriminant value criterion-discriminating unit that discriminates between Crohn disease and ulcerative colitis in the subject, based on the discriminant value calculated by the discriminant value-calculating unit, and a discrimination result-sending unit that transmits the discrimination result of the subject obtained by the discriminant value criterion-discriminating unit to the information communication terminal apparatus.

Another aspect of the present invention is the amino acid data-processing system, wherein the discrimination result is a discrimination result as to discrimination between Crohn disease in active phase and ulcerative colitis in active phase. The discriminant value criterion-discriminating unit discriminates between Crohn disease in active phase and ulcerative colitis in active phase in the subject, based on the discriminant value calculated by the discriminant value-calculating unit.

The present invention also relates to an amino acid data-processing program product, one aspect of the present invention is the amino acid data-processing program product that makes an information processing apparatus including a control unit and a memory unit execute a method of discriminating between inflammatory bowel disease and inflammatory bowel disease-free in a subject to be evaluated. The method includes (i) a discriminant value calculating step of calculating a discriminant value that is a value of multivariate discriminant, based on both the concentration value of at least one of Tau, Urea, Asn, Glu, Gln, Pro, Ala, Cit, Gly, ABA, Val, Cys, Leu, Tyr, Phe, His, Trp, Orn and Lys contained in previously obtained amino acid concentration data on a concentration value of amino acid in the subject and a multivariate discriminant with the concentration of the amino acid as explanatory variable stored in the memory unit, where the concentration value of at least one of Tau, Urea, Asn, Glu, Gln, Pro, Ala, Cit, Gly, ABA, Val, Cys, Leu, Tyr, Phe, His, Trp, Orn and Lys is contained as the explanatory variable, and (ii) a discriminant value criterion discriminating step of discriminating between inflammatory bowel disease and inflammatory bowel disease-free in the subject, based on the discriminant value calculated at the discriminant value calculating step. The steps (i) and (ii) are executed by the control unit.

One aspect of the present invention is an amino acid data-processing program product that makes an information processing apparatus including a control unit and a memory unit execute a method of discriminating between Crohn disease and Crohn disease-free or between ulcerative colitis and ulcerative colitis-free in a subject to be evaluated. The method includes (i) a discriminant value calculating step of calculating a discriminant value that is a value of multivariate discriminant, based on both the concentration value of at least one of Tau, Thr, Urea, Asn, Glu, Gln, Pro, Gly, ABA, Val, Cys, Leu, Tyr, Phe, His, Trp and Lys contained in previously obtained amino acid concentration data on a concentration value of amino acid in the subject and a multivariate discriminant with the concentration of the amino acid as explanatory variable stored in the memory unit, where the concentration value of at least one of Tau, Thr, Urea, Asn, Glu, Gln, Pro, Gly, ABA, Val, Cys, Leu, Tyr, Phe, His, Trp and Lys is contained as the explanatory variable, or calculating a discriminant value that is a value of multivariate discriminant, based on both the concentration value of at least one of Tau, Urea, Asn, Gln, Ala, Cit, ABA, Val, Cys, Met, Ile, Leu, Tyr, Phe, His, Trp, Orn and Lys contained in previously obtained amino acid concentration data on a concentration value of amino acid in the subject and a multivariate discriminant with the concentration of the amino acid as explanatory variable stored in the memory unit, where the concentration value of at least one of Tau, Urea, Asn, Gln, Ala, Cit, ABA, Val, Cys, Met, Ile, Leu, Tyr, Phe, His, Trp, Orn and Lys is contained as the explanatory variable, and (ii) a discriminant value criterion discriminating step of discriminating between Crohn disease and Crohn disease-free in the subject, based on the discriminant value calculated at the discriminant value calculating step, or discriminating between ulcerative colitis and ulcerative colitis-free in the subject, based on the discriminant value calculated at the discriminant value calculating step. The steps (i) and (ii) are executed by the control unit.

One aspect of the present invention is an amino acid data-processing program product that makes an information processing apparatus including a control unit and a memory unit execute a method of discriminating between active phase and remission phase of Crohn disease or ulcerative colitis in a subject to be evaluated. The method includes (i) a discriminant value calculating step of calculating a discriminant value that is a value of multivariate discriminant, based on both previously obtained amino acid concentration data on a concentration value of amino acid in the subject and a multivariate discriminant with the concentration of the amino acid as explanatory variable stored in the memory unit, and (ii) a discriminant value criterion discriminating step of discriminating between active phase and remission phase of Crohn disease or ulcerative colitis in the subject, based on the discriminant value calculated at the discriminant value calculating step. The steps (i) and (ii) are executed by the control unit.

One aspect of the present invention is an amino acid data-processing program product that makes an information processing apparatus including a control unit and a memory unit execute a method of evaluating a condition of Crohn disease or ulcerative colitis in a subject to be evaluated. The method includes (i) a discriminant value calculating step of calculating a discriminant value that is a value of multivariate discriminant, based on both a plurality of previously obtained amino acid concentration data on a concentration value of amino acid in the subject and a multivariate discriminant with the concentration of the amino acid as explanatory variable stored in the memory unit, for each of the plurality of the amino acid concentration data, and (ii) a discriminant value criterion condition evaluating step of evaluating the condition of Crohn disease or ulcerative colitis in the subject, based on the plurality of the discriminant values calculated at the discriminant value calculating step. The steps (i) and (ii) are executed by the control unit.

One aspect of the present invention is an amino acid data-processing program product that makes an information processing apparatus including a control unit and a memory unit execute a method of discriminating between Crohn disease and ulcerative colitis in a subject to be evaluated. The method includes (i) a discriminant value calculating step of calculating a discriminant value that is a value of multivariate discriminant, based on both previously obtained amino acid concentration data on a concentration value of amino acid in the subject and a multivariate discriminant with the concentration of the amino acid as explanatory variable stored in the memory unit, and (ii) a discriminant value criterion discriminating step of discriminating between Crohn disease and ulcerative colitis in the subject, based on the discriminant value calculated at the discriminant value calculating step. The steps (i) and (ii) are executed by the control unit.

Another aspect of the present invention is the amino acid data-processing program product, wherein at the discriminant value criterion discriminating step, discrimination between Crohn disease in active phase and ulcerative colitis in active phase in the subject is conducted based on the discriminant value calculated at the discriminant value calculating step.

The present invention also relates to a computer-readable recording medium, the computer-readable recording medium according to one aspect of the present invention includes the amino acid data-processing program product described above.

EFFECTS OF THE INVENTION

According to the present invention, amino acid concentration data on the concentration value of amino acid in blood collected from a subject to be evaluated is measured, and an inflammatory bowel disease state in the subject is evaluated based on the measured amino acid concentration data of the subject. Thus, the concentrations of the amino acids in blood can be utilized to bring about an effect of enabling accurate evaluation of an inflammatory bowel disease state.

According to the present invention, discrimination between inflammatory bowel disease and inflammatory bowel disease-free in the subject is conducted based on the concentration value of at least one of Tau, Urea, Asn, Glu, Gln, Pro, Ala, Cit, ABA, Val, Cys, Met, Ile, Leu, Tyr, Phe, His, Trp, Orn and Lys contained in the measured amino acid concentration data of the subject. Thus, the concentrations of the amino acids which among amino acids in blood, are useful for discriminating between the 2 groups of inflammatory bowel disease and inflammatory bowel disease-free can be utilized to bring about an effect of enabling accurate discrimination between the 2 groups of inflammatory bowel disease and inflammatory bowel disease-free.

According to the present invention, a discriminant value that is a value of multivariate discriminant is calculated based on both the concentration value of at least one of Tau, Urea, Asn, Glu, Gln, Pro, Ala, Cit, Gly, ABA, Val, Cys, Leu, Tyr, Phe, His, Trp, Orn and Lys contained in the measured amino acid concentration data of the subject and a previously established multivariate discriminant with the concentration of the amino acid as explanatory variable, where the concentration value of at least one of Tau, Urea, Asn, Glu, Gln, Pro, Ala, Cit, Gly, ABA, Val, Cys, Leu, Tyr, Phe, His, Trp, Orn and Lys is contained as the explanatory variable, and discrimination between inflammatory bowel disease and inflammatory bowel disease-free in the subject is conducted based on the calculated discriminant value. Thus, a discriminant value obtained in a multivariate discriminant useful for discriminating between the 2 groups of inflammatory bowel disease and inflammatory bowel disease-free can be utilized to bring about an effect of enabling accurate discrimination between the 2 groups of inflammatory bowel disease and inflammatory bowel disease-free.

According to the present invention, the multivariate discriminant is expressed by one fractional expression or the sum of a plurality of the fractional expressions, and contains the concentration value of at least one of Tau, Glu, Pro and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contains the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau, Glu, Pro and Cys as the explanatory variable in the denominator in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Tau, Leu, Tyr, His, Ile and Thr as the explanatory variables. Thus, a discriminant value obtained in a multivariate discriminant useful particularly for discriminating between the 2 groups of inflammatory bowel disease and inflammatory bowel disease-free can be utilized to bring about an effect of enabling more accurate discrimination between the 2 groups of inflammatory bowel disease and inflammatory bowel disease-free.

According to the present invention, Crohn disease state or ulcerative colitis state in the subject is evaluated based on the measured amino acid concentration data of the subject. Thus, the concentrations of the amino acids in blood can be utilized to bring about an effect of enabling accurate evaluation of Crohn disease state or ulcerative colitis state.

According to the present invention, discrimination between Crohn disease and Crohn disease-free in the subject is conducted based on the concentration value of at least one of Tau, Thr, Urea, Asn, Glu, Gln, Pro, Gly, ABA, Val, Cys, Leu, Tyr, Phe, His, Trp and Lys contained in the measured amino acid concentration data of the subject, or discrimination between ulcerative colitis and ulcerative colitis-free in the subject is conducted based on the concentration value of at least one of Tau, Urea, Asn, Gln, Ala, Cit, ABA, Val, Cys, Met, Ile, Leu, Tyr, Phe, His, Trp, Orn and Lys contained in the measured amino acid concentration data of the subject. Thus, the concentrations of the amino acids which among amino acids in blood, are useful for discriminating between the 2 groups of Crohn disease and Crohn disease-free or discriminating between the 2 groups of ulcerative colitis and ulcerative colitis-free can be utilized to bring about an effect of enabling accurate discrimination between the 2 groups of Crohn disease and Crohn disease-free or discrimination between the 2 groups of ulcerative colitis and ulcerative colitis-free.

According to the present invention, discrimination between active phase and remission phase of Crohn disease or ulcerative colitis in the subject is conducted based on the measured amino acid concentration data of the subject. Thus, the concentrations of the amino acids in blood can be utilized to bring about an effect of enabling accurate discrimination between the 2 groups of active phase and remission phase of Crohn disease or ulcerative colitis.

According to the present invention, discrimination between active phase and remission phase of Crohn disease in the subject is conducted based on the concentration value of at least one of Gln, Cit, Val, Leu, His, Trp, Lys and Arg contained in the measured amino acid concentration data of the subject, or discrimination between active phase and remission phase of ulcerative colitis in the subject is conducted based on the concentration value of at least one of Urea, Gln, Thr, Asn, Pro, Ala, ABA, Val, Met, Leu, Tyr, Phe, His, Trp, Lys, Arg and Cys contained in the measured amino acid concentration data of the subject. Thus, the concentrations of the amino acids which among amino acids in blood, are useful for discriminating between the 2 groups of active phase and remission phase of Crohn disease or ulcerative colitis can be utilized to bring about an effect of enabling accurate discrimination between the 2 groups of active phase and remission phase of Crohn disease or ulcerative colitis.

According to the present invention, a discriminant value that is a value of multivariate discriminant is calculated based on both the concentration value of at least one of Tau, Thr, Urea, Asn, Glu, Gln, Pro, Gly, ABA, Val, Cys, Leu, Tyr, Phe, His, Trp and Lys contained in the measured amino acid concentration data of the subject and a previously established multivariate discriminant with the concentration of the amino acid as explanatory variable, where the concentration value of at least one of Tau, Thr, Urea, Asn, Glu, Gln, Pro, Gly, ABA, Val, Cys, Leu, Tyr, Phe, His, Trp and Lys is contained as the explanatory variable, or a discriminant value that is a value of multivariate discriminant is calculated based on both the concentration value of at least one of Tau, Urea, Asn, Gln, Ala, Cit, ABA, Val, Cys, Met, Ile, Leu, Tyr, Phe, His, Trp, Orn and Lys contained in the measured amino acid concentration data of the subject and a previously established multivariate discriminant with the concentration of the amino acid as explanatory variable, where the concentration value of at least one of Tau, Urea, Asn, Gln, Ala, Cit, ABA, Val, Cys, Met, Ile, Leu, Tyr, Phe, His, Trp, Orn and Lys is contained as the explanatory variable, and discrimination between Crohn disease and Crohn disease-free in the subject is conducted based on the calculated discriminant value or discrimination between ulcerative colitis and ulcerative colitis-free in the subject is conducted based on the calculated discriminant value. Thus, a discriminant value obtained in a multivariate discriminant useful for discriminating between the 2 groups of Crohn disease and Crohn disease-free or discriminating between the 2 groups of ulcerative colitis and ulcerative colitis-free can be utilized to bring about an effect of enabling accurate discrimination between the 2 groups of Crohn disease and Crohn disease-free or discrimination between the 2 groups of ulcerative colitis and ulcerative colitis-free.

According to the present invention, when discrimination between Crohn disease and Crohn disease-free is conducted, the multivariate discriminant is expressed by one fractional expression or the sum of a plurality of fractional expressions, and contains the concentration value of at least one of Tau, Glu, Pro and Gly as the explanatory variable in the numerator in the fractional expression constituting the multivariate discriminant, and the concentration value of at least one of Urea, Asn, Gln, Thr, ABA, Val, Cys, Leu, Tyr, Phe, His, Trp and Lys as the explanatory variable in the denominator in the fractional expression constituting the multivariate discriminant, or contains the concentration value of at least one of Urea, Asn, Gln, Thr, ABA, Val, Cys, Leu, Tyr, Phe, His, Trp and Lys as the explanatory variable in the numerator in the fractional expression constituting the multivariate discriminant, and the concentration value of at least one of Tau, Glu, Pro and Gly as the explanatory variable in the denominator in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Tau, Val, Leu, Tyr, Pro and Ile as the explanatory variables. When discrimination between ulcerative colitis and ulcerative colitis-free is conducted, the multivariate discriminant is expressed by one fractional expression or the sum of a plurality of fractional expressions, and contains the concentration value of at least one of Tau and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contains the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau and Cys as the explanatory variable in the denominator in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Tau, Tyr, Leu, His, Ala and Ile as the explanatory variables. Thus, a discriminant value obtained in a multivariate discriminant useful particularly for discriminating between the 2 groups of Crohn disease and Crohn disease-free or discriminating between the 2 groups of ulcerative colitis and ulcerative colitis-free can be utilized to bring about an effect of enabling more accurate discrimination between the 2 groups of Crohn disease and Crohn disease-free or discrimination between the 2 groups of ulcerative colitis and ulcerative colitis-free.

According to the present invention, a discriminant value that is a value of multivariate discriminant is calculated based on both the measured amino acid concentration data of the subject and a previously established multivariate discriminant with the concentration of the amino acid as explanatory variable, and discrimination between active phase and remission phase of Crohn disease or ulcerative colitis in the subject is conducted based on the calculated discriminant value. Thus, a discriminant value obtained in a multivariate discriminant can be utilized to bring about an effect of enabling accurate discrimination between the 2 groups of active phase and remission phase of Crohn disease or ulcerative colitis.

According to the present invention, a discriminant value that is a value of multivariate discriminant is calculated based on both the plurality of the measured amino acid concentration data of the subject and a previously established multivariate discriminant with the concentration of the amino acid as explanatory variable, for each of the plurality of the amino acid concentration data, and a condition of Crohn disease or ulcerative colitis in the subject is evaluated based on the plurality of calculated discriminant values. Thus, a discriminant value obtained in a multivariate discriminant can be utilized to bring about an effect of enabling accurate evaluation of a condition of Crohn disease or ulcerative colitis.

According to the present invention, when discrimination between active phase and remission phase of Crohn disease is conducted, or when the condition of Crohn disease is evaluated, one or a plurality of the discriminant values are calculated based on both the concentration value of at least one of Gln, Cit, Val, Leu, His, Trp, Lys and Arg contained in one or a plurality of the measured amino acid concentration data of the subject, and the multivariate discriminant containing the concentration value of at least one of Gln, Cit, Val, Leu, His, Trp, Lys and Arg as the explanatory variable. When discrimination between active phase and remission phase of ulcerative colitis is conducted, or when the condition of ulcerative colitis is evaluated, one or a plurality of the discriminant values are calculated based on both the concentration value of at least one of Tau, Urea, Thr, Asn, Pro, Gln, Ala, Cit, ABA, Val, Met, Leu, Tyr, Phe, His, Trp, Lys, Arg and Cys contained in one or a plurality of the measured amino acid concentration data of the subject, and the multivariate discriminant containing the concentration value of at least one of Tau, Urea, Thr, Asn, Pro, Gln, Ala, Cit, ABA, Val, Met, Leu, Tyr, Phe, His, Trp, Lys, Arg and Cys as the explanatory variable. Thus, a discriminant value obtained in a multivariate discriminant useful for discriminating between the 2 groups of active phase and remission phase of Crohn disease or ulcerative colitis can be utilized to bring about an effect of enabling accurate discrimination between the 2 groups of active phase and remission phase of Crohn disease or ulcerative colitis. A discriminant value obtained in a multivariate discriminant useful for evaluating a condition of Crohn disease or ulcerative colitis can be utilized to bring about an effect of enabling accurate evaluation of a condition of Crohn disease or ulcerative colitis.

According to the present invention, when discrimination between active phase and remission phase of Crohn disease is conducted, or when the condition of Crohn disease is evaluated, the multivariate discriminant is expressed by one fractional expression or the sum of a plurality of fractional expressions and contains the concentration value of at least one of Gln, Cit, Val, Leu, His, Trp, Lys and Arg as the explanatory variable in any one of the numerator and denominator or both in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of His, Trp, Lys, Ser, Tau and Ile as the explanatory variables. When discrimination between active phase and remission phase of ulcerative colitis is conducted, or when the condition of ulcerative colitis is evaluated, the multivariate discriminant is expressed by one fractional expression or the sum of a plurality of fractional expressions and contains the concentration value of at least one of Tau, Urea, Thr, Asn, Pro, Gln, Ala, Cit, ABA, Val, Met, Leu, Tyr, Phe, His, Trp, Lys, Arg and Cys as the explanatory variable in any one of the numerator and denominator or both in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' distance method, a discriminant prepared by canonical discriminant analysis and a discriminant prepared by a decision tree, and has the concentration value of His, Tyr, Val, Met, Leu and Arg as the explanatory variables. Thus, a discriminant value obtained in a multivariate discriminant useful particularly for discriminating between the 2 groups of active phase and remission phase of Crohn disease or ulcerative colitis can be utilized to bring about an effect of enabling more accurate discrimination between the 2 groups of active phase and remission phase of Crohn disease or ulcerative colitis. A discriminant value obtained in a multivariate discriminant useful particularly for evaluating a condition of Crohn disease or ulcerative colitis can be utilized to bring about an effect of enabling more accurate evaluation of a condition of Crohn disease or ulcerative colitis.

According to the present invention, discrimination between Crohn disease and ulcerative colitis in the subject is conducted based on the measured amino acid concentration data of the subject. Thus, the concentrations of the amino acids in blood can be utilized to bring about an effect of enabling accurate discrimination between the 2 groups of Crohn disease and ulcerative colitis.

According to the present invention, discrimination between Crohn disease and ulcerative colitis in the subject is conducted based on the concentration value of at least one of Cys, ABA, Thr, Pro, Gly, Met, Ile and Orn contained in the measured amino acid concentration data of the subject. Thus, the concentrations of the amino acids which among amino acids in blood, are useful for discriminating between the 2 groups of Crohn disease and ulcerative colitis can be utilized to bring about an effect of enabling accurate discrimination between the 2 groups of Crohn disease and ulcerative colitis.

According to the present invention, discrimination between Crohn disease in active phase and ulcerative colitis in active phase in the subject is conducted based on the measured amino acid concentration data of the subject. Thus, the concentrations of the amino acids in blood can be utilized to bring about an effect of enabling accurate discrimination between the 2 groups of Crohn disease in active phase and ulcerative colitis in active phase.

According to the present invention, discrimination between Crohn disease in active phase and ulcerative colitis in active phase in the subject is conducted based on the concentration value of at least one of Tau, Thr, Ser, Gly, Met and Ile contained in the measured amino acid concentration data. Thus, the concentrations of the amino acids which among amino acids in blood, are useful for discriminating between the 2 groups of Crohn disease in active phase and ulcerative colitis in active phase can be utilized to bring about an effect of enabling accurate discrimination between the 2 groups of Crohn disease in active phase and ulcerative colitis in active phase.

According to the present invention, a discriminant value that is a value of multivariate discriminant is calculated based on both the measured amino acid concentration data of the subject, and a previously established multivariate discriminant with the concentration of the amino acid as explanatory variable, and discrimination between Crohn disease and ulcerative colitis in the subject is conducted based on the calculated discriminant value. Thus, a discriminant value obtained in a multivariate discriminant can be utilized to bring about an effect of enabling accurate discrimination between the 2 groups of Crohn disease and ulcerative colitis.

According to the present invention, the discriminant value is calculated based on both the concentration value of at least one of Cys, ABA, Thr, Pro, Gly, Met, Ile and Orn contained in the measured amino acid concentration data of the subject and the previously established multivariate discriminant containing the concentration value of at least one of Cys, ABA, Thr, Pro, Gly, Met, Ile and Orn as the explanatory variable, and discrimination between Crohn disease and ulcerative colitis in the subject is conducted based on the calculated discriminant value. Thus, a discriminant value obtained in a multivariate discriminant useful for discriminating between the 2 groups of Crohn disease and ulcerative colitis can be utilized to bring about an effect of enabling accurate discrimination between the 2 groups of Crohn disease and ulcerative colitis.

According to the present invention, the multivariate discriminant is expressed by one fractional expression or the sum of a plurality of fractional expressions, and contains the concentration value of at least one of Cys and ABA as the explanatory variable in the numerator in the fractional expression constituting the multivariate discriminant and the concentration value of at least one of Thr, Pro, Gly, Met, Ile and Orn as the explanatory variable in the denominator in the fractional expression constituting the multivariate discriminant, or contains the concentration value of at least one of Thr, Pro, Gly, Met, Ile and Orn as the explanatory variable in the numerator in the fractional expression constituting the multivariate discriminant and the concentration value of at least one of Cys and ABA as the explanatory variable in the denominator in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Met, Pro, Ile, Trp, Tau and Val as the explanatory variables. Thus, a discriminant value obtained in a multivariate discriminant useful particularly for discriminating between the 2 groups of Crohn disease and ulcerative colitis can be utilized to bring about an effect of enabling more accurate discrimination between the 2 groups of Crohn disease and ulcerative colitis.

According to the present invention, discrimination between Crohn disease in active phase and ulcerative colitis in active phase in the subject is conducted based on the calculated discriminant value. Thus, a discriminant value obtained in a multivariate discriminant can be utilized to bring about an effect of enabling accurate discrimination between the 2 groups of Crohn disease in active phase and ulcerative colitis in active phase.

According to the present invention, the discriminant value is calculated based on both the concentration value of at least one of Tau, Thr, Ser, Gly, Met and Ile contained in the measured amino acid concentration data of the subject and the multivariate discriminant containing the concentration value of at least one of Tau, Thr, Ser, Gly, Met and Ile as the explanatory variable, and discrimination between Crohn disease in active phase and ulcerative colitis in active phase in the subject is conducted based on the calculated discriminant value. Thus, a discriminant value obtained in a multivariate discriminant useful for discriminating between the 2 groups of Crohn disease in active phase and ulcerative colitis in active phase can be utilized to bring about an effect of enabling accurate discrimination between the 2 groups of Crohn disease in active phase and ulcerative colitis in active phase.

According to the present invention, the multivariate discriminant is expressed by one fractional expression or the sum of a plurality of fractional expressions, and contains the concentration value of at least one of Tau, Thr, Ser, Gly, Met and Ile as the explanatory variable in any one of the numerator and denominator or both in the fractional expression constituting the multivariate discriminant, or the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Met, Tau, Trp, Tyr, Orn and Phe as the explanatory variables. Thus, a discriminant value obtained in a multivariate discriminant useful particularly for discriminating between the 2 groups of Crohn disease in active phase and ulcerative colitis in active phase can be utilized to bring about an effect of enabling more accurate discrimination between the 2 groups of Crohn disease in active phase and ulcerative colitis in active phase.

According to the present invention, the information communication terminal apparatus first transmits the amino acid concentration data of the subject to the amino acid data processor. The amino acid data processor receives the amino acid concentration data of the subject transmitted from the information communication terminal apparatus, calculates a discriminant value that is a value of multivariate discriminant, based on both the concentration value of at least one of Tau, Urea, Asn, Glu, Gln, Pro, Ala, Cit, Gly, ABA, Val, Cys, Leu, Tyr, Phe, His, Trp, Orn and Lys contained in the received amino acid concentration data of the subject and a multivariate discriminant with the concentration of the amino acid as explanatory variable stored in the memory unit, where the concentration value of at least one of Tau, Urea, Asn, Glu, Gln, Pro, Ala, Cit, Gly, ABA, Val, Cys, Leu, Tyr, Phe, His, Trp, Orn and Lys is contained as the explanatory variable, discriminates between inflammatory bowel disease and inflammatory bowel disease-free in the subject, based on the calculated discriminant value, and transmits the obtained discrimination result of the subject to the information communication terminal apparatus. Then, the information communication terminal apparatus receives the discrimination result as to discrimination between inflammatory bowel disease and inflammatory bowel disease-free of the subject transmitted from the amino acid data processor. Thus, a discriminant value obtained in a multivariate discriminant useful for discriminating between the 2 groups of inflammatory bowel disease and inflammatory bowel disease-free can be utilized to bring about an effect of enabling accurate discrimination between the 2 groups of inflammatory bowel disease and inflammatory bowel disease-free.

According to the present invention, the information communication terminal apparatus first transmits the amino acid concentration data of the subject to the amino acid data processor. The amino acid data processor receives the amino acid concentration data of the subject transmitted from the information communication terminal apparatus, calculates a discriminant value that is a value of multivariate discriminant, based on both the concentration value of at least one of Tau, Thr, Urea, Asn, Glu, Gln, Pro, Gly, ABA, Val, Cys, Leu, Tyr, Phe, His, Trp and Lys contained in the received amino acid concentration data of the subject and a multivariate discriminant with the concentration of the amino acid as explanatory variable stored in the memory unit, where the concentration value of at least one of Tau, Thr, Urea, Asn, Glu, Gln, Pro, Gly, ABA, Val, Cys, Leu, Tyr, Phe, His, Trp and Lys is contained as the explanatory variable, or calculates a discriminant value that is a value of multivariate discriminant, based on both the concentration value of at least one of Tau, Urea, Asn, Gln, Ala, Cit, ABA, Val, Cys, Met, Ile, Leu, Tyr, Phe, His, Trp, Orn and Lys contained in the received amino acid concentration data of the subject and a multivariate discriminant with the concentration of the amino acid as explanatory variable stored in the memory unit, where the concentration value of at least one of Tau, Urea, Asn, Gln, Ala, Cit, ABA, Val, Cys, Met, Ile, Leu, Tyr, Phe, His, Trp, Orn and Lys is contained as the explanatory variable, discriminates between Crohn disease and Crohn disease-free in the subject, based on the calculated discriminant value, or discriminates between ulcerative colitis and ulcerative colitis-free in the subject, based on the calculated discriminant value, and transmits the obtained discrimination result of the subject to the information communication terminal apparatus. Then, the information communication terminal apparatus receives the discrimination result as to discrimination between Crohn disease and Crohn disease-free or between ulcerative colitis and ulcerative colitis-free of the subject transmitted from the amino acid data processor. Thus, a discriminant value obtained in a multivariate discriminant useful for discriminating between the 2 groups of Crohn disease and Crohn disease-free or discriminating between the 2 groups of ulcerative colitis and ulcerative colitis-free can be utilized to bring about an effect of enabling accurate discrimination between the 2 groups of Crohn disease and Crohn disease-free or discrimination between the 2 groups of ulcerative colitis and ulcerative colitis-free.

According to the present invention, the information communication terminal apparatus first transmits the amino acid concentration data of the subject to the amino acid data processor. The amino acid data processor receives the amino acid concentration data of the subject transmitted from the information communication terminal apparatus, calculates a discriminant value that is a value of multivariate discriminant, based on both the received amino acid concentration data of the subject and a multivariate discriminant with the concentration of the amino acid as explanatory variable stored in the memory unit, discriminates between active phase and remission phase of Crohn disease or ulcerative colitis in the subject, based on the calculated discriminant value, and transmits the obtained discrimination result of the subject to the information communication terminal apparatus. Then, the information communication terminal apparatus receives the discrimination result as to discrimination between active phase and remission phase of Crohn disease or ulcerative colitis of the subject transmitted from the amino acid data processor. Thus, a discriminant value obtained in a multivariate discriminant can be utilized to bring about an effect of enabling accurate discrimination between the 2 groups of active phase and remission phase of Crohn disease or ulcerative colitis.

According to the present invention, the information communication terminal apparatus first transmits a plurality of the amino acid concentration data of the subject to the amino acid data processor. The amino acid data processor receives the plurality of the amino acid concentration data of the subject transmitted from the information communication terminal apparatus, calculates a discriminant value that is a value of multivariate discriminant, based on both the plurality of the received amino acid concentration data of the subject and a multivariate discriminant with the concentration of the amino acid as explanatory variable stored in the memory unit, for each of the plurality of the amino acid concentration data, evaluates the condition of Crohn disease or ulcerative colitis in the subject, based on the plurality of the calculated discriminant values, and transmits the obtained evaluation result of the subject to the information communication terminal apparatus. Then, the information communication terminal apparatus receives the evaluation result as to the condition of Crohn disease or ulcerative colitis of the subject transmitted from the amino acid data processor. Thus, a discriminant value obtained in a multivariate discriminant can be utilized to bring about an effect of enabling accurate evaluation of a condition of Crohn disease or ulcerative colitis.

According to the present invention, the information communication terminal apparatus first transmits the amino acid concentration data of the subject to the amino acid data processor. The amino acid data processor receives the amino acid concentration data of the subject transmitted from the information communication terminal apparatus, calculates a discriminant value that is a value of multivariate discriminant, based on both the received amino acid concentration data of the subject and a multivariate discriminant with the concentration of the amino acid as explanatory variable stored in the memory unit, discriminates between Crohn disease and ulcerative colitis in the subject, based on the calculated discriminant value, and transmits the obtained discrimination result of the subject to the information communication terminal apparatus. Then, the information communication terminal apparatus receives the discrimination result as to discrimination between Crohn disease and ulcerative colitis of the subject transmitted from the amino acid data processor. Thus, a discriminant value obtained in a multivariate discriminant can be utilized to bring about an effect of enabling accurate discrimination between the 2 groups of Crohn disease and ulcerative colitis.

According to the present invention, the discrimination result is a discrimination result as to discrimination between Crohn disease in active phase and ulcerative colitis in active phase, and discrimination between Crohn disease in active phase and ulcerative colitis in active phase in the subject is conducted based on the calculated discriminant value. Thus, a discriminant value obtained in a multivariate discriminant can be utilized to bring about an effect of enabling accurate discrimination between the 2 groups of Crohn disease in active phase and ulcerative colitis in active phase.

According to the present invention, the amino acid data-processing program recorded on the recording medium is read and executed by the computer, thereby allowing the computer to execute the amino acid data-processing program, thus bringing about an effect of obtaining the same effect as in the amino acid data-processing program.

When inflammatory bowel disease state is evaluated (specifically, discrimination between inflammatory bowel disease and inflammatory bowel disease-free is conducted, Crohn disease state is evaluated (discrimination between Crohn disease and Crohn disease-free is conducted, discrimination between active phase and remission phase of Crohn disease is conducted, and the condition of Crohn disease is evaluated), ulcerative colitis state is evaluated (discrimination between ulcerative colitis and ulcerative colitis-free is conducted, discrimination between active phase and remission phase of ulcerative colitis is conducted, and the condition of ulcerative colitis is evaluated), and discrimination between Crohn disease and ulcerative colitis is conducted (discrimination between Crohn disease in active phase and ulcerative colitis in active phase is conducted)) in the present invention, the concentrations of other metabolites, the protein expression level, the age and sex of the subject or the like may be used in addition to the amino acid concentration. When inflammatory bowel disease state is evaluated (specifically, discrimination between inflammatory bowel disease and inflammatory bowel disease-free is conducted, Crohn disease state is evaluated (discrimination between Crohn disease and Crohn disease-free is conducted, discrimination between active phase and remission phase of Crohn disease is conducted, and the condition of Crohn disease is evaluated), ulcerative colitis state is evaluated (discrimination between ulcerative colitis and ulcerative colitis-free is conducted, discrimination between active phase and remission phase of ulcerative colitis is conducted, and the condition of ulcerative colitis is evaluated), and discrimination between Crohn disease and ulcerative colitis is conducted (discrimination between Crohn disease in active phase and ulcerative colitis in active phase is conducted)) in the present invention, the concentrations of other metabolites, the protein expression level, the age and sex of the subject or the like may be used as explanatory variables in the multivariate discriminant in addition to the amino acid concentration.

BRIEF DESCRIPTION OF THE DRAWINGS

FIG. 1 is a principle configurational diagram showing the basic principle of the present invention;
FIG. 2 is a flowchart showing one example of the method of evaluating IBD according to the first embodiment;
FIG. 3 is a principle configurational diagram showing the basic principle of the present invention;
FIG. 4 is a diagram showing an example of the entire configuration of the present system;
FIG. 5 is a diagram showing another example of the entire configuration of the present system;
FIG. 6 is a block diagram showing an example of the configuration of the IBD-evaluating apparatus 100 in the present system;
FIG. 7 is a chart showing an example of the information stored in the user information file 106a;
FIG. 8 is a chart showing an example of the information stored in the amino acid concentration data file 106b;
FIG. 9 is a chart showing an example of the information stored in the IBD state information file 106c;
FIG. 10 is a chart showing an example of the information stored in the designated IBD state information file 106d;
FIG. 11 is a chart showing an example of the information stored in the candidate multivariable discriminant file 106e1;
FIG. 12 is a chart showing an example of the information stored in the verification result file 106e2;

FIG. 13 is a chart showing an example of the information stored in the selected IBD state information file 106e3;
FIG. 14 is a chart showing an example of the information stored in the multivariable discriminant file 106e4;
FIG. 15 is a chart showing an example of the information stored in the discriminant value file 106f;
FIG. 16 is a chart showing an example of the information stored in the evaluation result file 106g;
FIG. 17 is a block diagram showing the configuration of the multivariable discriminant-preparing part 102h;
FIG. 18 is a block diagram showing the configuration of the discriminant criterion-evaluating part 102j;
FIG. 19 is a block diagram showing an example of the configuration of the client apparatus 200 in the present system;
FIG. 20 is a block diagram showing an example of the configuration of the database apparatus 400 in the present system;
FIG. 21 is a flowchart showing an example of the IBD evaluation service processing performed in the present system;
FIG. 22 is a flowchart showing an example of the multivariate discriminant-preparing processing performed in the IBD-evaluating apparatus 100 in the present system;
FIG. 23 is a boxplot showing the distribution of amino acid explanatory variables between the 2 groups of healthy subject group and inflammatory bowel disease group;
FIG. 24 is a boxplot showing the distribution of amino acid explanatory variables between the 3 groups of healthy subject group, Crohn disease group and ulcerative colitis group;
FIG. 25 is a boxplot showing the distribution of amino acid explanatory variables between the 4 groups of remission phase Crohn disease group, active phase Crohn disease group, remission phase ulcerative colitis group, and active phase ulcerative colitis group;
FIG. 26 is a graph showing an ROC curve for evaluation of diagnostic performance between 2 groups;
FIG. 27 is a chart showing a list of formulae having the same diagnostic performance as that of index formula 11;
FIG. 28 is a chart showing a list of formulae having the same diagnostic performance as that of index formula 11;
FIG. 29 is a graph showing an ROC curve for evaluation of diagnostic performance between 2 groups;
FIG. 30 is a chart showing a list of formulae having the same diagnostic performance as that of index formula 12;
FIG. 31 is a chart showing a list of formulae having the same diagnostic performance as that of index formula 12;
FIG. 32 is a graph showing an ROC curve for evaluation of diagnostic performance between 2 groups;
FIG. 33 is a chart showing a list of formulae having the same diagnostic performance as that of index formula 13;
FIG. 34 is a chart showing a list of formulae having the same diagnostic performance as that of index formula 13;
FIG. 35 is a graph showing an ROC curve for evaluation of diagnostic performance between 2 groups;
FIG. 36 is a chart showing a list of formulae having the same diagnostic performance as that of index formula 14;
FIG. 37 is a chart showing a list of formulae having the same diagnostic performance as that of index formula 14;
FIG. 38 is a graph showing an ROC curve for evaluation of diagnostic performance between 2 groups;
FIG. 39 is a chart showing a list of formulae having the same diagnostic performance as that of index formula 15;
FIG. 40 is a chart showing a list of formulae having the same diagnostic performance as that of index formula 15;
FIG. 41 is a graph showing an ROC curve for evaluation of diagnostic performance between 2 groups;
FIG. 42 is a chart showing a list of formulae having the same diagnostic performance as that of index formula 16;
FIG. 43 is a chart showing a list of formulae having the same diagnostic performance as that of index formula 16;
FIG. 44 is a graph showing an ROC curve for evaluation of diagnostic performance between 2 groups;
FIG. 45 is a chart showing a list of formulae having the same diagnostic performance as that of index formula 17;
FIG. 46 is a chart showing a list of formulae having the same diagnostic performance as that of index formula 17;
FIG. 47 is a graph showing an ROC curve for evaluation of diagnostic performance between 2 groups;
FIG. 48 is a chart showing a list of formulae having the same diagnostic performance as that of index formula 21;
FIG. 49 is a chart showing a list of formulae having the same diagnostic performance as that of index formula 21;
FIG. 50 is a graph showing an ROC curve for evaluation of diagnostic performance between 2 groups;
FIG. 51 is a chart showing a list of formulae having the same diagnostic performance as that of index formula 22;
FIG. 52 is a chart showing a list of formulae having the same diagnostic performance as that of index formula 22;
FIG. 53 is a graph showing an ROC curve for evaluation of diagnostic performance between 2 groups;
FIG. 54 is a chart showing a list of formulae having the same diagnostic performance as that of index formula 23;
FIG. 55 is a chart showing a list of formulae having the same diagnostic performance as that of index formula 23;
FIG. 56 is a graph showing an ROC curve for evaluation of diagnostic performance between 2 groups;
FIG. 57 is a chart showing a list of formulae having the same diagnostic performance as that of index formula 24;
FIG. 58 is a chart showing a list of formulae having the same diagnostic performance as that of index formula 24;
FIG. 59 is a graph showing an ROC curve for evaluation of diagnostic performance between 2 groups;
FIG. 60 is a chart showing a list of formulae having the same diagnostic performance as that of index formula 25;
FIG. 61 is a chart showing a list of formulae having the same diagnostic performance as that of index formula 25;
FIG. 62 is a graph showing an ROC curve for evaluation of diagnostic performance between 2 groups;
FIG. 63 is a chart showing a list of formulae having the same diagnostic performance as that of index formula 26;
FIG. 64 is a chart showing a list of formulae having the same diagnostic performance as that of index formula 26;

FIG. 65 is a graph showing an ROC curve for evaluation of diagnostic performance between 2 groups;
FIG. 66 is a chart showing a list of formulae having the same diagnostic performance as that of index formula 27;
FIG. 67 is a chart showing a list of formulae having the same diagnostic performance as that of index formula 27;
FIG. 68 is a graph showing an ROC curve for evaluation of diagnostic performance between 2 groups;
FIG. 69 is a chart showing a list of formulae having the same diagnostic performance as that of index formula 28;
FIG. 70 is a chart showing a list of formulae having the same diagnostic performance as that of index formula 28;
FIG. 71 is a graph showing an ROC curve for evaluation of diagnostic performance between 2 groups;
FIG. 72 is a chart showing a list of formulae having the same diagnostic performance as that of index formula 29;
FIG. 73 is a chart showing a list of formulae having the same diagnostic performance as that of index formula 29;
FIG. 74 is a graph showing an ROC curve for evaluation of diagnostic performance between 2 groups;
FIG. 75 is a chart showing a list of formulae having the same diagnostic performance as that of index formula 30;
FIG. 76 is a chart showing a list of formulae having the same diagnostic performance as that of index formula 30;
FIG. 77 is a graph showing an ROC curve for evaluation of diagnostic performance between 2 groups;
FIG. 78 is a chart showing a list of formulae having the same diagnostic performance as that of index formula 31;
FIG. 79 is a chart showing a list of formulae having the same diagnostic performance as that of index formula 31;
FIG. 80 is a graph showing an ROC curve for evaluation of diagnostic performance between 2 groups;
FIG. 81 is a chart showing a list of formulae having the same diagnostic performance as that of index formula 32;
FIG. 82 is a chart showing a list of formulae having the same diagnostic performance as that of index formula 32;
FIG. 83 is a graph showing an ROC curve for evaluation of diagnostic performance between 2 groups;
FIG. 84 is a chart showing a list of formulae having the same diagnostic performance as that of index formula 33;
FIG. 85 is a chart showing a list of formulae having the same diagnostic performance as that of index formula 33;
FIG. 86 is a graph showing an ROC curve for evaluation of diagnostic performance between 2 groups;
FIG. 87 is a chart showing a list of formulae having the same diagnostic performance as that of index formula 34;
FIG. 88 is a chart showing a list of formulae having the same diagnostic performance as that of index formula 34;
FIG. 89 is a diagram showing changes over time in the value of index formula 16 and the value of CDAI;
FIG. 90 is a diagram showing changes over time in the value of index formula 33' and the value of CAI;
FIG. 91 is a boxplot showing the distribution of amino acid explanatory variables between the 2 groups of healthy subject group and inflammatory bowel disease group;
FIG. 92 is a boxplot showing the distribution of amino acid explanatory variables between the 2 groups of healthy subject group and Crohn disease group;
FIG. 93 is a boxplot showing the distribution of amino acid explanatory variables between the 2 groups of healthy subject group and ulcerative colitis group;
FIG. 94 is a boxplot showing the distribution of amino acid explanatory variables between the 2 groups of Crohn disease group and ulcerative colitis group;
FIG. 95 is a boxplot showing the distribution of amino acid explanatory variables between the 4 groups of remission phase Crohn disease group, active phase Crohn disease group, remission phase ulcerative colitis group, and active phase ulcerative colitis group;
FIG. 96 is a graph showing an ROC curve for evaluation of diagnostic performance between 2 groups;
FIG. 97 is a chart showing a list of formulae having the same diagnostic performance as that of index formula 35;
FIG. 98 is a chart showing a list of formulae having the same diagnostic performance as that of index formula 35;
FIG. 99 is a graph showing an ROC curve for evaluation of diagnostic performance between 2 groups;
FIG. 100 is a chart showing a list of formulae having the same diagnostic performance as that of index formula 36;
FIG. 101 is a chart showing a list of formulae having the same diagnostic performance as that of index formula 36;
FIG. 102 is a chart showing a list of formulae having the same diagnostic performance as that of index formula 36;
FIG. 103 is a graph showing an ROC curve for evaluation of diagnostic performance between 2 groups;
FIG. 104 is a chart showing a list of formulae having the same diagnostic performance as that of index formula 37;
FIG. 105 is a chart showing a list of formulae having the same diagnostic performance as that of index formula 37;
FIG. 106 is a chart showing a list of formulae having the same diagnostic performance as that of index formula 37;
FIG. 107 is a graph showing an ROC curve for evaluation of diagnostic performance between 2 groups;
FIG. 108 is a chart showing a list of formulae having the same diagnostic performance as that of index formula 38;
FIG. 109 is a chart showing a list of formulae having the same diagnostic performance as that of index formula 38;
FIG. 110 is a graph showing an ROC curve for evaluation of diagnostic performance between 2 groups;
FIG. 111 is a chart showing a list of formulae having the same diagnostic performance as that of index formula 39;
FIG. 112 is a chart showing a list of formulae having the same diagnostic performance as that of index formula 39;
FIG. 113 is a chart showing a list of formulae having the same diagnostic performance as that of index formula 39;
FIG. 114 is a graph showing an ROC curve for evaluation of diagnostic performance between 2 groups;
FIG. 115 is a chart showing a list of formulae having the same diagnostic performance as that of index formula 40;
FIG. 116 is a chart showing a list of formulae having the same diagnostic performance as that of index formula 40;

FIG. 117 is a chart showing a list of formulae having the same diagnostic performance as that of index formula 40;

FIG. 118 is a graph showing an ROC curve for evaluation of diagnostic performance between 2 groups;

FIG. 119 is a chart showing a list of formulae having the same diagnostic performance as that of index formula 41;

FIG. 120 is a chart showing a list of formulae having the same diagnostic performance as that of index formula 41;

FIG. 121 is a chart showing a list of formulae having the same diagnostic performance as that of index formula 41;

FIG. 122 is a graph showing an ROC curve for evaluation of diagnostic performance between 2 groups;

FIG. 123 is a chart showing a list of formulae having the same diagnostic performance as that of index formula 42;

FIG. 124 is a chart showing a list of formulae having the same diagnostic performance as that of index formula 42;

FIG. 125 is a graph showing an ROC curve for evaluation of diagnostic performance between 2 groups;

FIG. 126 is a chart showing a list of formulae having the same diagnostic performance as that of index formula 43;

FIG. 127 is a chart showing a list of formulae having the same diagnostic performance as that of index formula 43;

FIG. 128 is a graph showing an ROC curve for evaluation of diagnostic performance between 2 groups;

FIG. 129 is a chart showing a list of formulae having the same diagnostic performance as that of index formula 44;

FIG. 130 is a chart showing a list of formulae having the same diagnostic performance as that of index formula 44;

FIG. 131 is a chart showing a list of formulae having the same diagnostic performance as that of index formula 44;

FIG. 132 is a chart showing a list of formulae having the same diagnostic performance as that of index formula 44;

FIG. 133 is a graph showing an ROC curve for evaluation of diagnostic performance between 2 groups;

FIG. 134 is a chart showing a list of formulae having the same diagnostic performance as that of index formula 45;

FIG. 135 is a chart showing a list of formulae having the same diagnostic performance as that of index formula 45;

FIG. 136 is a chart showing a list of formulae having the same diagnostic performance as that of index formula 45;

FIG. 137 is a chart showing a list of formulae having the same diagnostic performance as that of index formula 45;

FIG. 138 is a graph showing an ROC curve for evaluation of diagnostic performance between 2 groups;

FIG. 139 is a chart showing a list of formulae having the same diagnostic performance as that of index formula 46;

FIG. 140 is a chart showing a list of formulae having the same diagnostic performance as that of index formula 46;

FIG. 141 is a chart showing a list of formulae having the same diagnostic performance as that of index formula 46;

FIG. 142 is a chart showing a list of formulae having the same diagnostic performance as that of index formula 46;

FIG. 143 is a graph showing an ROC curve for evaluation of diagnostic performance between 2 groups;

FIG. 144 is a chart showing a list of formulae having the same diagnostic performance as that of index formula 47;

FIG. 145 is a chart showing a list of formulae having the same diagnostic performance as that of index formula 47;

FIG. 146 is a chart showing a list of formulae having the same diagnostic performance as that of index formula 47;

FIG. 147 is a chart showing a list of formulae having the same diagnostic performance as that of index formula 47;

FIG. 148 is a graph showing an ROC curve for evaluation of diagnostic performance between 2 groups;

FIG. 149 is a chart showing a list of formulae having the same diagnostic performance as that of index formula 48;

FIG. 150 is a chart showing a list of formulae having the same diagnostic performance as that of index formula 48;

FIG. 151 is a graph showing an ROC curve for evaluation of diagnostic performance between 2 groups;

FIG. 152 is a chart showing a list of formulae having the same diagnostic performance as that of index formula 49;

FIG. 153 is a chart showing a list of formulae having the same diagnostic performance as that of index formula 49;

FIG. 154 is a graph showing an ROC curve for evaluation of diagnostic performance between 2 groups;

FIG. 155 is a chart showing a list of formulae having the same diagnostic performance as that of index formula 50;

FIG. 156 is a chart showing a list of formulae having the same diagnostic performance as that of index formula 50;

FIG. 157 is a chart showing a list of formulae having the same diagnostic performance as that of index formula 50;

FIG. 158 is a chart showing a list of formulae having the same diagnostic performance as that of index formula 50;

FIG. 159 is a graph showing an ROC curve for evaluation of diagnostic performance between 2 groups;

FIG. 160 is a chart showing a list of formulae having the same diagnostic performance as that of index formula 51;

FIG. 161 is a chart showing a list of formulae having the same diagnostic performance as that of index formula 51;

FIG. 162 is a chart showing a list of formulae having the same diagnostic performance as that of index formula 51;

FIG. 163 is a chart showing a list of formulae having the same diagnostic performance as that of index formula 51;

FIG. 164 is a graph showing an ROC curve for evaluation of diagnostic performance between 2 groups;

FIG. 165 is a chart showing a list of formulae having the same diagnostic performance as that of index formula 52;

FIG. 166 is a chart showing a list of formulae having the same diagnostic performance as that of index formula 52;

FIG. 167 is a chart showing a list of formulae having the same diagnostic performance as that of index formula 52;

FIG. 168 is a chart showing a list of formulae having the same diagnostic performance as that of index formula 52;

FIG. 169 is a graph showing an ROC curve for evaluation of diagnostic performance between 2 groups;

FIG. 170 is a chart showing a list of formulae having the same diagnostic performance as that of index formula 53;

FIG. 171 is a chart showing a list of formulae having the same diagnostic performance as that of index formula 53;

FIG. 172 is a chart showing a list of formulae having the same diagnostic performance as that of index formula 53;

FIG. 173 is a chart showing a list of formulae having the same diagnostic performance as that of index formula 53;

FIG. 174 is a graph showing an ROC curve for evaluation of diagnostic performance between 2 groups;

EP 2 124 059 A1

FIG. 175 is a chart showing a list of formulae having the same diagnostic performance as that of index formula 54;
FIG. 176 is a chart showing a list of formulae having the same diagnostic performance as that of index formula 54;
FIG. 177 is a chart showing a list of formulae having the same diagnostic performance as that of index formula 54;
FIG. 178 is a chart showing a list of formulae having the same diagnostic performance as that of index formula 54;
FIG. 179 is a graph showing an ROC curve for evaluation of diagnostic performance between 2 groups;
FIG. 180 is a chart showing a list of formulae having the same diagnostic performance as that of index formula 55;
FIG. 181 is a chart showing a list of formulae having the same diagnostic performance as that of index formula 55;
FIG. 182 is a chart showing a list of formulae having the same diagnostic performance as that of index formula 55;
FIG. 183 is a chart showing a list of formulae having the same diagnostic performance as that of index formula 55;
FIG. 184 is a diagram showing the value of index formula 52' and the active phase and remission phase of same patient;
FIG. 185 is a diagram showing a list of formulae having the same monitoring ability as that of index formula 52';
FIG. 186 is a diagram showing a list of formulae having the same monitoring ability as that of index formula 52';
FIG. 187 is a diagram showing a list of formulae having the same monitoring ability as that of index formula 52';
FIG. 188 is a diagram showing a list of formulae having the same monitoring ability as that of index formula 52';
FIG. 189 is a diagram showing the value of index formula 53' and the active phase and remission phase of same patient;
FIG. 190 is a diagram showing a list of formulae having the same monitoring ability as that of index formula 53';
FIG. 191 is a diagram showing a list of formulae having the same monitoring ability as that of index formula 53';
FIG. 192 is a diagram showing a list of formulae having the same monitoring ability as that of index formula 53';
FIG. 193 is a diagram showing a list of formulae having the same monitoring ability as that of index formula 53';
FIG. 194 is a diagram showing the value of index formula 54' and the active phase and remission phase of same patient;
FIG. 195 is a diagram showing a list of formulae having the same monitoring ability as that of index formula 54';
FIG. 196 is a diagram showing a list of formulae having the same monitoring ability as that of index formula 54';
FIG. 197 is a diagram showing a list of formulae having the same monitoring ability as that of index formula 54';
FIG. 198 is a diagram showing a list of formulae having the same monitoring ability as that of index formula 54';
FIG. 199 is a diagram showing the value of index formula 55' and the active phase and remission phase of same patient;
FIG. 200 is a diagram showing a list of formulae having the same monitoring ability as that of index formula 55';
FIG. 201 is a diagram showing a list of formulae having the same monitoring ability as that of index formula 55';
FIG. 202 is a diagram showing a list of formulae having the same monitoring ability as that of index formula 55';
FIG. 203 is a diagram showing a list of formulae having the same monitoring ability as that of index formula 55'.

EXPLANATION OF LETTERS OR NUMERALS

100 IBD-evaluating apparatus
102 Control device

102a Request-interpreting part
102b Browsing processing part
102c Authentication-processing part
102d Electronic mail-generating part
102e Web page-generating part
102f Receiving part
102g IBD state information-designating part
102h Multivariate discriminant-preparing part

102h1 Candidate multivariate discriminant-preparing part
102h2 Candidate multivariate discriminant-verifying part
102h3 Explanatory variable-selecting part

102i Discriminant value-calculating part
102j Discriminant value criterion-evaluating part

102j1 Discriminant value criterion-discriminating part

24

102j2 Discriminant value criterion-condition evaluating part

102k Result outputting part
102m Sending part

104 Communication interface
106 Memory device

106a User information file
106b Amino acid concentration data file
106c IBD state information file
106d Designated IBD state information file
106e Multivariate discriminant-related information database

106e1 Candidate multivariate discriminant file
106e2 Verification result file
106e3 Selected IBD state information file
106e4 Multivariate discriminant file

106f Discriminant value file
106g Evaluation result file

108 Input/output interface
112 Input device
114 Output device
200 Client apparatus (information communication terminal apparatus)
300 Network
400 Database apparatus

BEST MODE(S) FOR CARRYING OUT THE INVENTION

Hereinafter, an embodiment (first embodiment) of the method of evaluating IBD of the present invention and an embodiment (second embodiment) of the amino acid data processor, the amino acid data-processing method, the amino acid data-processing system, the amino acid data-processing program and the recording medium of the present invention are described in detail with reference to the drawings. The present invention is not limited to these embodiments.

First Embodiment

1-1. Outline of the Invention

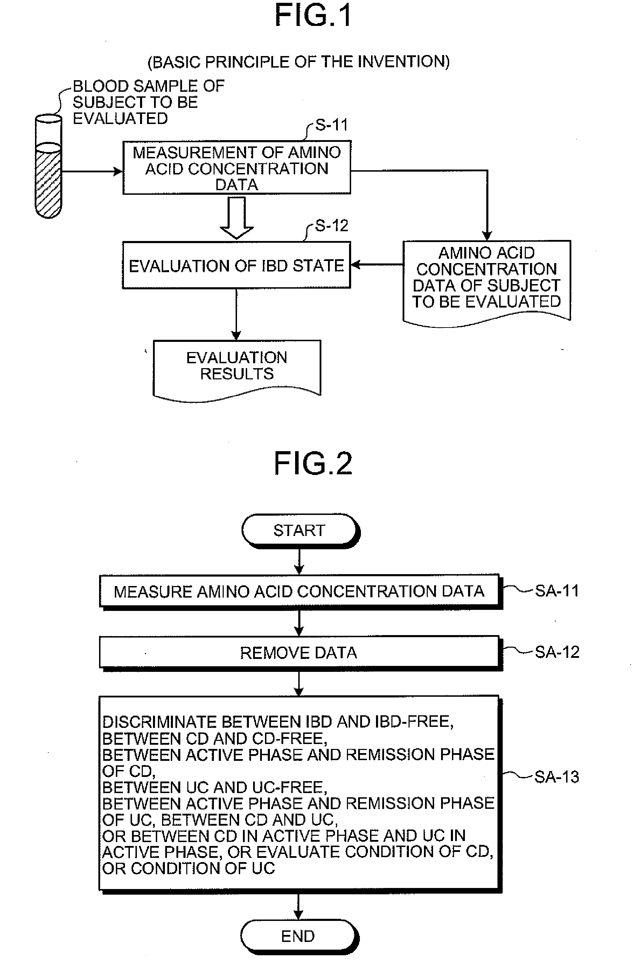

Here, an outline of the method of evaluating IBD of the present invention will be described with reference to FIG. 1. FIG. 1 is a principle configurational diagram showing the basic principle of the present invention.
In the present invention, the amino acid concentration data on concentration values of amino acids in blood collected from a subject (for example, an individual such as animal or human) to be evaluated are first measured (step S-11). The concentrations of amino acids in blood were analyzed in the following manner. A blood sample is collected in a heparin-treated tube, and then the blood plasma is separated by centrifugation of the collected blood sample. All blood plasma samples separated were frozen and stored at -70°C before measurement of amino acid concentration. Before measurement of amino acid concentration, the blood plasma sample was deproteinized by adding sulfosalicylic acid to a concentration of 3%. An amino acid analyzer by high-performance liquid chromatography (HPLC) by using ninhydrin reaction in the post column was used for measurement of amino acid concentration. The unit of amino acid concentration may be for example molar concentration, weight concentration, or these concentrations which are subjected to addition, subtraction, multiplication and division by an arbitrary constant.
In the present invention, an IBD state in the subject is evaluated based on the amino acid concentration data of the subject measured in the step S-11 (step S-12).
According to the present invention described above, amino acid concentration data on the concentration value of amino acid in blood collected from the subject is measured, and the IBD state in the subject is evaluated based on the measured amino acid concentration data of the subject. Thus, the concentrations of the amino acids in blood can be utilized to bring about an effect of enabling accurate evaluation of an IBD state.

Before step S-12 is executed, data such as defective and outliers may be removed from the amino acid concentration data of the subject measured in step S-11. Thereby, an IBD state can be more accurately evaluated.

In step S-12, discrimination between IBD and IBD-free in the subject may be conducted based on the amino acid concentration data of the subject measured in step S-11. Thus, the concentrations of the amino acids in blood can be utilized to bring about an effect of enabling accurate discrimination between the 2 groups of IBD and IBD-free. Discrimination between IBD and IBD-free in the subject may be conducted based on the concentration value of at least one of Tau, Urea, Asn, Glu, Gln, Pro, Ala, Cit, ABA, Val, Cys, Met, Ile, Leu, Tyr, Phe, His, Trp, Orn and Lys contained in the amino acid concentration data of the subject measured in step S-11. Specifically, the concentration value of at least one of Tau, Urea, Asn, Glu, Gln, Pro, Ala, Cit, ABA, Val, Cys, Met, Ile, Leu, Tyr, Phe, His, Trp, Orn and Lys may be compared with a previously established threshold (cutoff value), thereby discriminating between IBD and IBD-free in the subject. Thus, the concentrations of the amino acids which among amino acids in blood, are useful for discriminating between the 2 groups of IBD and IBD-free can be utilized to bring about an effect of enabling accurate discrimination between the 2 groups of IBD and IBD-free.

In step S-12, a discriminant value that is a value of multivariate discriminant may be calculated based on both the amino acid concentration data of the subject measured in step S-11 and a previously established multivariate discriminant with the concentration of the amino acid as explanatory variable, and the IBD state in the subject may be evaluated based on the calculated discriminant value. Thus, a discriminant value obtained in a multivariate discriminant can be utilized to bring about an effect of enabling accurate evaluation of an IBD state. A discriminant value that is a value of multivariate discriminant may be calculated based on both the amino acid concentration data of the subject measured in step S-11 and a previously established multivariate discriminant with the concentration of the amino acid as explanatory variable, and discrimination between IBD and IBD-free in the subject may be conducted based on the calculated discriminant value. Thus, a discriminant value obtained in a multivariate discriminant can be utilized to bring about an effect of enabling accurate discrimination between the 2 groups of IBD and IBD-free. A discriminant value that is a value of multivariate discriminant may be calculated based on both the concentration value of at least one of Tau, Urea, Asn, Glu, Gln, Pro, Ala, Cit, Gly, ABA, Val, Cys, Leu, Tyr, Phe, His, Trp, Orn and Lys contained in the amino acid concentration data of the subject measured in step S-11 and a previously established multivariate discriminant with the concentration of the amino acid as explanatory variable, where the concentration value of at least one of Tau, Urea, Asn, Glu, Gln, Pro, Ala, Cit, Gly, ABA, Val, Cys, Leu, Tyr, Phe, His, Trp, Orn and Lys is contained as the explanatory variable, and discrimination between IBD and IBD-free in the subject may be conducted based on the calculated discriminant value. Specifically, the discriminant value may be compared with a previously established threshold (cutoff value), thereby discriminating between IBD and IBD-free in the subject. Thus, a discriminant value obtained in a multivariate discriminant useful for discriminating between the 2 groups of IBD and IBD-free can be utilized to bring about an effect of enabling accurate discrimination between the 2 groups of IBD and IBD-free. The multivariate discriminant may be expressed by one fractional expression or the sum of a plurality of the fractional expressions, and contain the concentration value of at least one of Tau, Glu, Pro and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contain the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau, Glu, Pro and Cys as the explanatory variable in the denominator in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Tau, Leu, Tyr, His, Ile and Thr as the explanatory variables. Thus, a discriminant value obtained in a multivariate discriminant useful particularly for discriminating between the 2 groups of IBD and IBD-free can be utilized to bring about an effect of enabling more accurate discrimination between the 2 groups of IBD and IBD-free.

In step S-12, a CD state in the subject may be evaluated based on the amino acid concentration data of the subject measured in step S-11. Thus, the concentrations of the amino acids in blood can be utilized to bring about an effect of enabling accurate evaluation of a CD state. Discrimination between CD and CD-free in the subject may be conducted based on the amino acid concentration data of the subject measured in step S-11. Thus, the concentrations of the amino acids in blood can be utilized to bring about an effect of enabling accurate discrimination between the 2 groups of CD and CD-free. Discrimination between CD and CD-free in the subject may be conducted based on the concentration value of at least one of Tau, Thr, Urea, Asn, Glu, Gln, Pro, Gly, ABA, Val, Cys, Leu, Tyr, Phe, His, Trp and Lys contained in the amino acid concentration data of the subject measured in step S-11. Specifically, the concentration value of at least one of Tau, Thr, Urea, Asn, Glu, Gln, Pro, Gly, ABA, Val, Cys, Leu, Tyr, Phe, His, Trp and Lys may be compared with a previously established threshold (cutoff value), thereby discriminating between CD and CD-

26

free in the subject. Thus, the concentrations of the amino acids which among amino acids in blood, are useful for discriminating between the 2 groups of CD and CD-free can be utilized to bring about an effect of enabling accurate discrimination between the 2 groups of CD and CD-free.

In step S-12, discrimination between active phase and remission phase of CD in the subject may be conducted based on the amino acid concentration data of the subject measured in step S-11. Thus, the concentrations of the amino acids in blood can be utilized to bring about an effect of enabling accurate discrimination between the 2 groups of active phase and remission phase of CD. Discrimination between active phase and remission phase of CD in the subject may be conducted based on the concentration value of at least one of Gln, Cit, Val, Leu, His, Trp, Lys and Arg contained in the amino acid concentration data of the subject measured in step S-11. Specifically, the concentration value of at least one of Gln, Cit, Val, Leu, His, Trp, Lys and Arg may be compared with a previously established threshold (cutoff value), thereby discriminating between active phase and remission phase of CD in the subject. Thus, the concentrations of the amino acids which among amino acids in blood, are useful for discriminating between the 2 groups of active phase and remission phase of CD can be utilized to bring about an effect of enabling accurate discrimination between the 2 groups of active phase and remission phase of CD.

When the amino acid concentration data is measured from every sample of a plurality of blood samples collected from the subject in step S-11, a condition of CD in the subject may be evaluated in step S-12, based on the plurality of the amino acid concentration data of the subject measured in step S-11. Thus, the concentrations of the amino acids in blood can be utilized to bring about an effect of enabling accurate evaluation of a condition of CD. The condition of CD in the subject may be evaluated based on the concentration value of Gln, Cit, Val, Leu, His, Trp, Lys and Arg contained in the plurality of the amino acid concentration data of the subject measured in step S-11. Specifically, the concentration value of Gln, Cit, Val, Leu, His, Trp, Lys and Arg may be compared with a previously established threshold (cutoff value), thereby evaluating the condition of CD in the subject. Thus, the concentrations of the amino acids which among amino acids in blood, are useful for evaluating the condition of CD can be utilized to bring about an effect of enabling accurate evaluation of the condition of CD.

In step S-12, a discriminant value that is a value of multivariate discriminant may be calculated based on both the amino acid concentration data of the subject measured in step S-11 and a previously established multivariate discriminant with the concentration of the amino acid as explanatory variable, and a CD state in the subject may be evaluated based on the calculated discriminant value. Thus, a discriminant value obtained in a multivariate discriminant can be utilized to bring about an effect of enabling accurate evaluation of a CD state. A discriminant value that is a value of multivariate discriminant may be calculated based on both the amino acid concentration data of the subject measured in step S-11 and a previously established multivariate discriminant with the concentration of the amino acid as explanatory variable, and discrimination between CD and CD-free in the subject may be conducted based on the calculated discriminant value. Thus, a discriminant value obtained in a multivariate discriminant can be utilized to bring about an effect of enabling accurate discrimination between the 2 groups of CD and CD-free. A discriminant value that is a value of multivariate discriminant may be calculated based on both the concentration value of at least one of Tau, Thr, Urea, Asn, Glu, Gln, Pro, Gly, ABA, Val, Cys, Leu, Tyr, Phe, His, Trp and Lys contained in the amino acid concentration data of the subject measured in step S-11 and a previously established multivariate discriminant with the concentration of the amino acid as explanatory variable, where the concentration value of at least one of Tau, Thr, Urea, Asn, Glu, Gln, Pro, Gly, ABA, Val, Cys, Leu, Tyr, Phe, His, Trp and Lys is contained as the explanatory variable, and discrimination between CD and CD-free in the subject may be conducted based on the calculated discriminant value. Specifically, the discriminant value may be compared with a previously established threshold (cutoff value), thereby discriminating between CD and CD-free in the subject. Thus, a discriminant value obtained in a multivariate discriminant useful for discriminating between the 2 groups of CD and CD-free can be utilized to bring about an effect of enabling accurate discrimination between the 2 groups of CD and CD-free. The multivariate discriminant may be expressed by one fractional expression or the sum of a plurality of fractional expressions, and contain the concentration value of at least one of Tau, Glu, Pro and Gly as the explanatory variable in the numerator in the fractional expression constituting the multivariate discriminant, and the concentration value of at least one of Urea, Asn, Gln, Thr, ABA, Val, Cys, Leu, Tyr, Phe, His, Trp and Lys as the explanatory variable in the denominator in the fractional expression constituting the multivariate discriminant, or contain the concentration value of at least one of Urea, Asn, Gln, Thr, ABA, Val, Cys, Leu, Tyr, Phe, His, Trp and Lys as the explanatory variable in the numerator in the fractional expression constituting the multivariate discriminant, and the concentration value of at least one of Tau, Glu, Pro and Gly as the explanatory variable in the denominator in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Tau, Val, Leu, Tyr, Pro and Ile as the explanatory variables. Thus, a discriminant value obtained in a multivariate discriminant useful particularly for discriminating between the 2 groups of CD and CD-free can be utilized to bring about an effect of enabling more accurate discrimination between the 2 groups of CD and CD-free.

In step S-12, a discriminant value that is a value of multivariate discriminant may be calculated based on both the amino acid concentration data of the subject measured in step S-11 and a previously established multivariate discriminant with the concentration of the amino acid as explanatory variable, and discrimination between active phase and remission phase of CD in the subject may be conducted based on the calculated discriminant value. Thus, a discriminant value obtained in a multivariate discriminant can be utilized to bring about an effect of enabling accurate discrimination between the 2 groups of active phase and remission phase of CD. A discriminant value that is a value of multivariate discriminant may be calculated based on both the concentration value of at least one of Gln, Cit, Val, Leu, His, Trp, Lys and Arg contained in the amino acid concentration data of the subject measured in step S-11 and a previously established multivariate discriminant with the concentration of the amino acid as explanatory variable, where the concentration value of at least one of Gln, Cit, Val, Leu, His, Trp, Lys and Arg is contained as the explanatory variable, and discrimination between active phase and remission phase of CD in the subject may be conducted based on the calculated discriminant value. Specifically, the discriminant value may be compared with a previously established threshold (cutoff value), thereby discriminating between active phase and remission phase of CD in the subject. Thus, a discriminant value obtained in a multivariate discriminant useful for discriminating between the 2 groups of active phase and remission phase of CD can be utilized to bring about an effect of enabling accurate discrimination between the 2 groups of active phase and remission phase of CD. The multivariate discriminant may be expressed by one fractional expression or the sum of a plurality of fractional expressions and contain the concentration value of at least one of Gln, Cit, Val, Leu, His, Trp, Lys and Arg as the explanatory variable in any one of the numerator and denominator or both in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of His, Trp, Lys, Ser, Tau and Ile as the explanatory variables. Thus, a discriminant value obtained in a multivariate discriminant useful particularly for discriminating between the 2 groups of active phase and remission phase of CD can be utilized to bring about an effect of enabling more accurate discrimination between the 2 groups of active phase and remission phase of CD.

When the amino acid concentration data is measured from every sample of a plurality of blood samples collected from the subject in step S-11, a discriminant value that is a value of multivariate discriminant may be calculated based on both the plurality of the amino acid concentration data of the subject measured in step S-11 and a previously established multivariate discriminant with the concentration of the amino acid as explanatory variable, for each of the plurality of the amino acid concentration data, and the condition of CD in the subject may be evaluated based on the plurality of the calculated discriminant values. Thus, a discriminant value obtained in a multivariate discriminant can be utilized to bring about an effect of enabling accurate evaluation of a condition of CD. A discriminant values may be calculated based on both the concentration value of at least one of Gln, Cit, Val, Leu, His, Trp, Lys and Arg contained in the plurality of the amino acid concentration data of the subject measured in step S-11 and a previously established multivariate discriminant with the concentration of the amino acid as explanatory variable, where the concentration value of at least one of Gln, Cit, Val, Leu, His, Trp, Lys and Arg is contained as the explanatory variable, for each of the plurality of the amino acid concentration data, and the condition of CD in the subject may be evaluated based on the plurality of the calculated discriminant values. Specifically, the discriminant value may be compared with a previously established threshold (cutoff value), thereby evaluating the condition of CD in the subject. Thus, a discriminant value obtained in a multivariate discriminant useful for evaluating a condition of CD can be utilized to bring about an effect of enabling accurate evaluation of a condition of CD. The multivariate discriminant may be expressed by one fractional expression or the sum of a plurality of fractional expressions and contain the concentration value of at least one of Gln, Cit, Val, Leu, His, Trp, Lys and Arg as the explanatory variable in any one of the numerator and denominator or both in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of His, Trp, Lys, Ser, Tau and Ile as the explanatory variables. Thus, a discriminant value obtained in a multivariate discriminant useful particularly for evaluating a condition of CD can be utilized to bring about an effect of enabling more accurate evaluation of a condition of CD.

In step S-12, a UC state in the subject may be evaluated based on the amino acid concentration data of the subject measured in step S-11. Thus, the concentrations of the amino acids in blood can be utilized to bring about an effect of enabling accurate evaluation of a UC state. Discrimination between UC and UC-free in the subject may be conducted based on the amino acid concentration data of the subject measured in step S-11. Thus, the concentrations of the amino acids in blood can be utilized to bring about an effect of enabling accurate discrimination between the 2 groups of UC and UC-free. Discrimination between UC and UC-free in the subject may be conducted based on the concentration value of at least one of Tau, Urea, Asn, Gln, Ala, Cit, ABA, Val, Cys, Met, Ile, Leu, Tyr, Phe, His, Trp, Orn and Lys contained in the amino acid concentration data of the subject measured in step S-11. Specifically, the concentration value of at least one of Tau, Urea, Asn, Gln, Ala, Cit, ABA, Val, Cys, Met, Ile, Leu, Tyr, Phe, His, Trp, Orn and Lys may be compared with a previously established threshold (cutoff value), thereby discriminating between UC and UC-free in the subject. Thus, the concentrations of the amino acids which among amino acids in blood, are useful for discriminating between the 2 groups of UC and UC-free can be utilized to bring about an effect of enabling accurate discrimination between the 2 groups of UC and UC-free.

In step S-12, discrimination between active phase and remission phase of UC in the subject may be conducted based on the amino acid concentration data of the subject measured in step S-11. Thus, the concentrations of the amino acids in blood can be utilized to bring about an effect of enabling accurate discrimination between the 2 groups of active phase and remission phase of UC. Discrimination between active phase and remission phase of UC in the subject may be conducted based on the concentration value of at least one of Urea, Gln, Thr, Asn, Pro, Ala, ABA, Val, Met, Leu, Tyr, Phe, His, Trp, Lys, Arg and Cys contained in the amino acid concentration data of the subject measured in step S-11. Specifically, the concentration value of at least one of Urea, Gln, Thr, Asn, Pro, Ala, ABA, Val, Met, Leu, Tyr, Phe, His, Trp, Lys, Arg and Cys may be compared with a previously established threshold (cutoff value), thereby discriminating between active phase and remission phase of UC in the subject. Thus, the concentrations of the amino acids which among amino acids in blood, are useful for discriminating between the 2 groups of active phase and remission phase of UC can be utilized to bring about an effect of enabling accurate discrimination between the 2 groups of active phase and remission phase of UC.

When the amino acid concentration data is measured from every sample of a plurality of blood samples collected from the subject in step S-11, a condition of UC in the subject may be evaluated in step S-12, based on the plurality of the amino acid concentration data of the subject measured in step S-11. Thus, the concentrations of the amino acids in blood can be utilized to bring about an effect of enabling accurate evaluation of a condition of UC. The condition of UC in the subject may be evaluated based on the concentration value of at least one of Tau, Urea, Thr, Asn, Pro, Gln, Ala, Cit, ABA, Val, Met, Leu, Tyr, Phe, His, Trp, Lys, Arg and Cys contained in the plurality of the amino acid concentration data of the subject measured in step S-11. Specifically, the concentration value of at least one of Tau, Urea, Thr, Asn, Pro, Gln, Ala, Cit, ABA, Val, Met, Leu, Tyr, Phe, His, Trp, Lys, Arg and Cys may be compared with a previously established threshold (cutoff value), thereby evaluating the condition of UC in the subject. Thus, the concentrations of the amino acids which among amino acids in blood, are useful for evaluating the condition of UC can be utilized to bring about an effect of enabling accurate evaluation of the condition of UC.

In step S-12, a discriminant value that is a value of multivariate discriminant may be calculated based on both the amino acid concentration data of the subject measured in step S-11 and a previously established multivariate discriminant with the concentration of the amino acid as explanatory variable, and a UC state in the subject may be evaluated based on the calculated discriminant value. Thus, a discriminant value obtained in a multivariate discriminant can be utilized to bring about an effect of enabling accurate evaluation of a UC state. A discriminant value that is a value of multivariate discriminant may be calculated based on both the amino acid concentration data of the subject measured in step S-11 and a previously established multivariate discriminant with the concentration of the amino acid as explanatory variable, and discrimination between UC and UC-free in the subject may be conducted based on the calculated discriminant value. Thus, a discriminant value obtained in a multivariate discriminant can be utilized to bring about an effect of enabling accurate discrimination between the 2 groups of UC and UC-free. A discriminant value that is a value of multivariate discriminant may be calculated based on both the concentration value of at least one of Tau, Urea, Asn, Gln, Ala, Cit, ABA, Val, Cys, Met, Ile, Leu, Tyr, Phe, His, Trp, Orn and Lys contained in the amino acid concentration data of the subject measured in step S-11 and a previously established multivariate discriminant with the concentration of the amino acid as explanatory variable, where the concentration value of at least one of Tau, Urea, Asn, Gln, Ala, Cit, ABA, Val, Cys, Met, Ile, Leu, Tyr, Phe, His, Trp, Orn and Lys is contained as the explanatory variable, and discrimination between UC and UC-free in the subject may be conducted based on the calculated discriminant value. Specifically, the discriminant value may be compared with a previously established threshold (cutoff value), thereby discriminating between UC and UC-free in the subject. Thus, a discriminant value obtained in a multivariate discriminant useful for discriminating between the 2 groups of UC and UC-free can be utilized to bring about an effect of enabling accurate discrimination between the 2 groups of UC and UC-free. The multivariate discriminant may be expressed by one fractional expression or the sum of a plurality of fractional expressions, and contain the concentration value of at least one of Tau and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contain the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau and Cys as the explanatory variable in the denominator in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Tau, Tyr, Leu, His, Ala and Ile as the explanatory variables. Thus, a discriminant value obtained in a multivariate discriminant useful particularly for discriminating between the 2 groups of UC and UC-free can be utilized to bring about an effect of enabling more accurate discrimination between the 2 groups of UC and UC-free.

In step S-12, a discriminant value that is a value of multivariate discriminant may be calculated based on both the amino acid concentration data of the subject measured in step S-11 and a previously established multivariate discriminant with the concentration of the amino acid as explanatory variable, and discrimination between active phase and remission phase of UC in the subject may be conducted based on the calculated discriminant value. Thus, a discriminant value obtained in a multivariate discriminant can be utilized to bring about an effect of enabling accurate discrimination between the 2 groups of active phase and remission phase of UC. A discriminant value that is a value of multivariate discriminant may be calculated based on both the concentration value of at least one of Tau, Urea, Thr, Asn, Pro, Gln, Ala, Cit, ABA, Val, Met, Leu, Tyr, Phe, His, Trp, Lys, Arg and Cys contained in the amino acid concentration data of the subject measured in step S-11 and a previously established multivariate discriminant with the concentration of the amino acid as explanatory variable, where the concentration value of at least one of Tau, Urea, Thr, Asn, Pro, Gln, Ala, Cit, ABA, Val, Met, Leu, Tyr, Phe, His, Trp, Lys, Arg and Cys is contained as the explanatory variable, and discrimination between active phase and remission phase of UC in the subject may be conducted based on the calculated discriminant value. Thus, a discriminant value obtained in a multivariate discriminant useful for discriminating between the 2 groups of active phase and remission phase of UC can be utilized to bring about an effect of enabling accurate discrimination between the 2 groups of active phase and remission phase of UC. The multivariate discriminant may be expressed by one fractional expression or the sum of a plurality of fractional expressions and contain the concentration value of at least one of Tau, Urea, Thr, Asn, Pro, Gln, Ala, Cit, ABA, Val, Met, Leu, Tyr, Phe, His, Trp, Lys, Arg and Cys as the explanatory variable in any one of the numerator and denominator or both in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' distance method, a discriminant prepared by canonical discriminant analysis and a discriminant prepared by a decision tree, and have the concentration value of His, Tyr, Val, Met, Leu and Arg as the explanatory variables. Thus, a discriminant value obtained in a multivariate discriminant useful particularly for discriminating between the 2 groups of active phase and remission phase of UC can be utilized to bring about an effect of enabling more accurate discrimination between the 2 groups of active phase and remission phase of UC.

When the amino acid concentration data is measured from every sample of a plurality of blood samples collected from the subject in step S-11, a discriminant value that is a value of multivariate discriminant may be calculated based on both the plurality of the amino acid concentration data of the subject measured in step S-11 and a previously established multivariate discriminant with the concentration of the amino acid as explanatory variable, for each of the plurality of the amino acid concentration data, and the condition of UC in the subject may be evaluated based on the plurality of the calculated discriminant values. Thus, a discriminant value obtained in a multivariate discriminant can be utilized to bring about an effect of enabling accurate evaluation of a condition of UC. A discriminant values may be calculated based on both the concentration value of at least one of Tau, Urea, Thr, Asn, Pro, Gln, Ala, Cit, ABA, Val, Met, Leu, Tyr, Phe, His, Trp, Lys, Arg and Cys contained in the plurality of the amino acid concentration data of the subject measured in step S-11 and a previously established multivariate discriminant with the concentration of the amino acid as explanatory variable, where the concentration value of at least one of Tau, Urea, Thr, Asn, Pro, Gln, Ala, Cit, ABA, Val, Met, Leu, Tyr, Phe, His, Trp, Lys, Arg and Cys is contained as the explanatory variable, for each of the plurality of the amino acid concentration data, and the condition of UC in the subject may be evaluated based on the plurality of the calculated discriminant values. Thus, a discriminant value obtained in a multivariate discriminant useful for evaluating a condition of UC can be utilized to bring about an effect of enabling accurate evaluation of a condition of UC. The multivariate discriminant may be expressed by one fractional expression or the sum of a plurality of fractional expressions and contain the concentration value of at least one of Tau, Urea, Thr, Asn, Pro, Gln, Ala, Cit, ABA, Val, Met, Leu, Tyr, Phe, His, Trp, Lys, Arg and Cys as the explanatory variable in any one of the numerator and denominator or both in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' distance method, a discriminant prepared by canonical discriminant analysis and a discriminant prepared by a decision tree, and have the concentration value of His, Tyr, Val, Met, Leu and Arg as the explanatory variables. Thus, a discriminant value obtained in a multivariate discriminant useful particularly for evaluating a condition of UC can be utilized to bring about an effect of enabling more accurate evaluation of a condition of UC.

In step S-12, discrimination between CD and UC in the subject may be conducted based on the amino acid concentration data of the subject measured in step S-11. Thus, the concentrations of the amino acids in blood can be utilized to bring about an effect of enabling accurate discrimination between the 2 groups of CD and UC. Discrimination between CD and UC in the subject may be conducted based on the concentration value of at least one of Cys, ABA, Thr, Pro, Gly, Met, Ile and Orn contained in the amino acid concentration data of the subject measured in step S-11.

Specifically, the concentration value of at least one of Cys, ABA, Thr, Pro, Gly, Met, Ile and Orn may be compared with a previously established threshold (cutoff value), thereby discriminating between CD and UC in the subject. Thus, the concentrations of the amino acids which among amino acids in blood, are useful for discriminating between the 2 groups of CD and UC can be utilized to bring about an effect of enabling accurate discrimination between the 2 groups of CD and UC.

In step S-12, discrimination between CD in active phase and UC in active phase in the subject may be conducted based on the amino acid concentration data of the subject measured in step S-11. Thus, the concentrations of the amino acids in blood can be utilized to bring about an effect of enabling accurate discrimination between the 2 groups of CD in active phase and UC in active phase. Discrimination between CD in active phase and UC in active phase in the subject may be conducted based on the concentration value of at least one of Tau, Thr, Ser, Gly, Met and Ile contained in the amino acid concentration data of the subject measured in step S-11. Specifically, the concentration value of at least one of Tau, Thr, Ser, Gly, Met and Ile may be compared with a previously established threshold (cutoff value), thereby discriminating between CD in active phase and UC in active phase in the subject. Thus, the concentrations of the amino acids which among amino acids in blood, are useful for discriminating between the 2 groups of CD in active phase and UC in active phase can be utilized to bring about an effect of enabling accurate discrimination between the 2 groups of CD in active phase and UC in active phase.

In step S-12, a discriminant value that is a value of multivariate discriminant may be calculated based on both the amino acid concentration data of the subject measured in step S-11, and a previously established multivariate discriminant with the concentration of the amino acid as explanatory variable, and discrimination between CD and UC in the subject may be conducted based on the calculated discriminant value. Thus, a discriminant value obtained in a multivariate discriminant can be utilized to bring about an effect of enabling accurate discrimination between the 2 groups of CD and UC. A discriminant value that is a value of multivariate discriminant may be calculated based on both the concentration value of at least one of Cys, ABA, Thr, Pro, Gly, Met, Ile and Orn contained in the amino acid concentration data of the subject measured in step S-11 and a previously established multivariate discriminant with the concentration of the amino acid as explanatory variable, where the concentration value of at least one of Cys, ABA, Thr, Pro, Gly, Met, Ile and Orn as the explanatory variable, and discrimination between CD and UC in the subject may be conducted based on the calculated discriminant value. Specifically, the discriminant value may be compared with a previously established threshold (cutoff value), thereby discriminating between CD and UC in the subject. Thus, a discriminant value obtained in a multivariate discriminant useful for discriminating between the 2 groups of CD and UC can be utilized to bring about an effect of enabling accurate discrimination between the 2 groups of CD and UC. The multivariate discriminant may be expressed by one fractional expression or the sum of a plurality of fractional expressions, and contain the concentration value of at least one of Cys and ABA as the explanatory variable in the numerator in the fractional expression constituting the multivariate discriminant and the concentration value of at least one of Thr, Pro, Gly, Met, Ile and Orn as the explanatory variable in the denominator in the fractional expression constituting the multivariate discriminant, or contain the concentration value of at least one of Thr, Pro, Gly, Met, Ile and Orn as the explanatory variable in the numerator in the fractional expression constituting the multivariate discriminant and the concentration value of at least one of Cys and ABA as the explanatory variable in the denominator in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Met, Pro, Ile, Trp, Tau and Val as the explanatory variables. Thus, a discriminant value obtained in a multivariate discriminant useful particularly for discriminating between the 2 groups of CD and UC can be utilized to bring about an effect of enabling more accurate discrimination between the 2 groups of CD and UC.

In step S-12, a discriminant value that is a value of multivariate discriminant may be calculated based on both the amino acid concentration data of the subject measured in step S-11, and a previously established multivariate discriminant with the concentration of the amino acid as explanatory variable, and discrimination between CD in active phase and UC in active phase in the subject may be conducted based on the calculated discriminant value. Thus, a discriminant value obtained in a multivariate discriminant can be utilized to bring about an effect of enabling accurate discrimination between the 2 groups of CD in active phase and UC in active phase. A discriminant value that is a value of multivariate discriminant may be calculated based on both the concentration value of at least one of Tau, Thr, Ser, Gly, Met and Ile contained in the amino acid concentration data of the subject measured in step S-11 and a previously established multivariate discriminant with the concentration of the amino acid as explanatory variable, where the concentration value of at least one of Tau, Thr, Ser, Gly, Met and Ile is contained as the explanatory variable, and discrimination between CD in active phase and UC in active phase in the subject may be conducted based on the calculated discriminant value. Specifically, the discriminant value may be compared with a previously established threshold (cutoff value), thereby discriminating between CD in active phase and UC in active phase in the subject. Thus, a discriminant value obtained in a multivariate discriminant useful for discriminating between the 2 groups of CD in active phase and UC in active phase can be utilized to bring about an effect of enabling accurate discrimination between the 2 groups of CD in active phase and UC in active phase. The multivariate discriminant may be expressed by one fractional expression or the sum of a plurality of fractional expressions, and contain the concentration value of at least one of Tau, Thr, Ser, Gly, Met and Ile as the explanatory variable in any one of the numerator and denominator or both in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Met, Tau, Trp, Tyr, Orn and Phe as the explanatory variables. Thus, a discriminant value obtained in a multivariate discriminant useful particularly for discriminating between the 2 groups of CD in active phase and UC in active phase can be utilized to bring about an effect of enabling more accurate discrimination between the 2 groups of CD in active phase and UC in active phase.

The multivariate discriminants described above can be prepared by a method described in International Publication WO 2004/052191 that is an international application filed by the present applicant or by a method (multivariate discriminant-preparing processing described in the second embodiment described later) described in International Publication WO 2006/098192 that is an international application filed by the present applicant. Any multivariate discriminants obtained by these methods can be preferably used in evaluation of an IBD state, regardless of the unit of amino acid concentration in the amino acid concentration data as input data.

In a fractional expression, the numerator of the fractional expression is expressed by the sum of amino acids A, B, C etc. and the denominator of the fractional expression is expressed by the sum of amino acids a, b, c etc. The fractional expression also includes the sum of fractional expressions $\alpha$, $\beta$, $\gamma$ etc. (for example, $\alpha+\beta$) having such constitution. The fractional expression also includes divided fractional expressions. Amino acids used in the numerator or denominator may have suitable coefficients respectively. The amino acids used in the numerator or denominator may appear repeatedly. Each fractional expression may have a suitable coefficient. The value of a coefficient for each explanatory variable and the value for a constant term may be any real numbers. In combinations where explanatory variables in the numerator and explanatory variables in the denominator in the fractional expression are switched with each other, the positive (or negative) sign is generally reversed in correlation with objective explanatory variables, but because their correlation is maintained, such combinations can be assumed to be equivalent to one another in discrimination, and thus the fractional expression also includes combinations where explanatory variables in the numerator and explanatory variables in the denominator in the fractional expression are switched with each other.

The multivariate discriminant refers to a form of equation used generally in multivariate analysis and includes, for example, multiple regression equation, multiple logistic regression equation, linear discriminant function, Mahalanobis' generalized distance, canonical discriminant function, support vector machine, and decision tree. The multivariate discriminant also includes an equation shown by the sum of different forms of multivariate discriminants. In the multiple regression equation, multiple logistic regression equation and canonical discriminant function, a coefficient and constant term are added to each explanatory variable, and the coefficient and constant term in this case are preferably real numbers, more preferably values in the range of 99% confidence interval for the coefficient and constant term obtained from data for discrimination, more preferably in the range of 95% confidence interval for the coefficient and constant term obtained from data for discrimination. The value of each coefficient and the confidence interval thereof may be those multiplied by a real number, and the value of each constant term and the confidence interval thereof may be those having an arbitrary actual constant added or subtracted or those multiplied or divided by an arbitrary actual constant.

When IBD state is evaluated in the present invention, the concentrations of other metabolites, the protein expression level, the age and sex of the subject or the like may be used in addition to the amino acid concentration. When IBD state is evaluated in the present invention, the concentrations of other metabolites, the protein expression level, the age and sex of the subject or the like may be used as explanatory variables in the multivariate discriminant in addition to the amino acid concentration.

1-2. Method of evaluating IBD in accordance with the first embodiment

Herein, the method of evaluating IBD according to the first embodiment is described with reference to FIG. 2. FIG. 2 is a flowchart showing one example of the method of evaluating IBD according to the first embodiment.

From blood collected from an individual such as animal or human, amino acid concentration data on the concentration values of amino acids are measured (step SA-11). Measurement of the concentration values of amino acids is conducted by the method described above.

From the amino acid concentration data of the individual measured in step SA-11, data such as defective and outliers are then removed (step SA-12).

Then, either the discrimination between 2 groups or the evaluation of the condition of disease that will be described in the following 11. to 19., is conducted for the individual, based on the amino acid concentration data of the individual from which data such as defective and outliers have been removed in step SA-12, or on a previously established multivariate discriminant with the concentrations of amino acids as the explanatory variables (step SA-13).

11. Discrimination between IBD and IBD-free

The concentration value of at least one of Tau, Urea, Asn, Glu, Gln, Pro, Ala, Cit, ABA, Val, Cys, Met, Ile, Leu, Tyr, Phe, His, Trp, Orn and Lys contained in the amino acid concentration data of the individual from which data such as defective and outliers have been removed, is compared with a previously established threshold (cutoff value), thereby discriminating between IBD and IBD-free in the individual. Alternatively, a discriminant value that is a value of multivariate discriminant is calculated based on both the concentration value of at least one of Tau, Urea, Asn, Glu, Gln, Pro, Ala, Cit, Gly, ABA, Val, Cys, Leu, Tyr, Phe, His, Trp, Orn and Lys contained in the amino acid concentration data of the individual from which data such as defective and outliers have been removed and a previously established multivariate discriminant with the concentration of the amino acid as explanatory variable, where the concentration value of at least one of Tau, Urea, Asn, Glu, Gln, Pro, Ala, Cit, Gly, ABA, Val, Cys, Leu, Tyr, Phe, His, Trp, Orn and Lys is contained as the explanatory variable, and the calculated discriminant value is compared with a previously established threshold (cutoff value), thereby discriminating between IBD and IBD-free in the individual.

12. Discrimination between CD and CD-free

The concentration value of at least one of Tau, Thr, Urea, Asn, Glu, Gln, Pro, Gly, ABA, Val, Cys, Leu, Tyr, Phe, His, Trp and Lys contained in the amino acid concentration data of the individual from which data such as defective and outliers have been removed, is compared with a previously established threshold (cutoff value), thereby discriminating between CD and CD-free in the individual. Alternatively, a discriminant value that is a value of multivariate discriminant is calculated based on both the concentration value of at least one of Tau, Thr, Urea, Asn, Glu, Gln, Pro, Gly, ABA, Val, Cys, Leu, Tyr, Phe, His, Trp and Lys contained in the amino acid concentration data of the individual from which data such as defective and outliers have been removed and a previously established multivariate discriminant with the concentration of the amino acid as explanatory variable, where the concentration value of at least one of Tau, Thr, Urea, Asn, Glu, Gln, Pro, Gly, ABA, Val, Cys, Leu, Tyr, Phe, His, Trp and Lys is contained as the explanatory variable, and the calculated discriminant value is compared with a previously established threshold (cutoff value), thereby discriminating between CD and CD-free in the individual.

13. Discrimination between active phase and remission phase of CD

The concentration value of at least one of Gln, Cit, Val, Leu, His, Trp, Lys and Arg contained in the amino acid concentration data of the individual from which data such as defective and outliers have been removed, is compared with a previously established threshold (cutoff value), thereby discriminating between active phase and remission phase of CD in the individual. Alternatively, a discriminant value that is a value of multivariate discriminant is calculated based on both the concentration value of at least one of Gln, Cit, Val, Leu, His, Trp, Lys and Arg contained in the amino acid concentration data of the individual from which data such as defective and outliers have been removed and a previously established multivariate discriminant with the concentration of the amino acid as explanatory variable, where the concentration value of at least one of Gln, Cit, Val, Leu, His, Trp, Lys and Arg is contained as the explanatory variable, and the calculated discriminant value is compared with a previously established threshold (cutoff value), thereby discriminating between active phase and remission phase of CD in the individual.

14. Evaluation of condition of CD

When the amino acid concentration data is measured from every sample of a plurality of blood samples collected, for example, on a different day, from the same individual in step SA-11, the concentration value of at least one of Gln, Cit, Val, Leu, His, Trp, Lys and Arg contained in the plurality of the amino acid concentration data of the individual from which data such as defective and outliers have been removed, is compared with a previously established threshold (cutoff value) for each of the plurality of the amino acid concentration data, thereby evaluating a condition of CD in the individual. Alternatively, when the amino acid concentration data is measured from every sample of a plurality of blood samples collected, for example, on a different day, from the same individual in step SA-11, a discriminant value that is a value of multivariate discriminant is calculated based on both the concentration value of at least one of Gln, Cit, Val, Leu, His, Trp, Lys and Arg contained in the plurality of the amino acid concentration data of the individual from which data such as defective and outliers have been removed and a previously established multivariate discriminant with the concentration of the amino acid as explanatory variable, where the concentration value of at least one of Gln, Cit, Val, Leu, His, Trp, Lys and Arg is contained as the explanatory variable, and the plurality of the calculated discriminant values are compared with a previously established threshold (cutoff value) for each of the plurality of the discriminant values, thereby evaluating a condition of CD in the individual.

15. Discrimination between UC and UC-free

The concentration value of at least one of Tau, Urea, Asn, Gln, Ala, Cit, ABA, Val, Cys, Met, Ile, Leu, Tyr, Phe, His, Trp, Orn and Lys contained in the amino acid concentration data of the individual from which data such as defective and outliers have been removed, is compared with a previously established threshold (cutoff value), thereby discriminating between UC and UC-free in the individual. Alternatively, a discriminant value that is a value of multivariate discriminant is calculated based on both the concentration value of at least one of Tau, Urea, Asn, Gln, Ala, Cit, ABA, Val, Cys, Met, Ile, Leu, Tyr, Phe, His, Trp, Orn and Lys contained in the amino acid concentration data of the individual from which data such as defective and outliers have been removed and a previously established multivariate discriminant with the concentration of the amino acid as explanatory variable, where the concentration value of at least one of Tau, Urea, Asn, Gln, Ala, Cit, ABA, Val, Cys, Met, Ile, Leu, Tyr, Phe, His, Trp, Orn and Lys is contained as the explanatory variable, and the calculated discriminant value is compared with a previously established threshold (cutoff value), thereby discriminating between UC and UC-free in the individual.

16. Discrimination between active phase and remission phase of UC

The concentration value of at least one of Urea, Gln, Thr, Asn, Pro, Ala, ABA, Val, Met, Leu, Tyr, Phe, His, Trp, Lys, Arg and Cys contained in the amino acid concentration data of the individual from which data such as defective and outliers have been removed, is compared with a previously established threshold (cutoff value), thereby discriminating between active phase and remission phase of UC in the individual. Alternatively, a discriminant value that is a value of multivariate discriminant is calculated based on both the concentration value of at least one of Tau, Urea, Thr, Asn, Pro, Gln, Ala, Cit, ABA, Val, Met, Leu, Tyr, Phe, His, Trp, Lys, Arg and Cys contained in the amino acid concentration data of the individual from which data such as defective and outliers have been removed and a previously established multivariate discriminant with the concentration of the amino acid as explanatory variable, where the concentration value of at least one of Tau, Urea, Thr, Asn, Pro, Gln, Ala, Cit, ABA, Val, Met, Leu, Tyr, Phe, His, Trp, Lys, Arg and Cys is contained as the explanatory variable, and the calculated discriminant value is compared with a previously established threshold (cutoff value), thereby discriminating between active phase and remission phase of UC in the individual.

17. Evaluation of condition of UC

When the amino acid concentration data is measured from every sample of a plurality of blood samples collected, for example, on a different day, from the same individual in step SA-11, the concentration value of at least one of Tau, Urea, Thr, Asn, Pro, Gln, Ala, Cit, ABA, Val, Met, Leu, Tyr, Phe, His, Trp, Lys, Arg and Cys contained in the plurality of the amino acid concentration data of the individual from which data such as defective and outliers have been removed, is compared with a previously established threshold (cutoff value) for each of the plurality of the amino acid concentration data, thereby evaluating a condition of UC in the individual. Alternatively, when the amino acid concentration data is measured from every sample of a plurality of blood samples collected, for example, on a different day, from the same individual in step SA-11, a discriminant value that is a value of multivariate discriminant is calculated based on both the concentration value of at least one of Tau, Urea, Thr, Asn, Pro, Gln, Ala, Cit, ABA, Val, Met, Leu, Tyr, Phe, His, Trp, Lys, Arg and Cys contained in the plurality of the amino acid concentration data of the individual from which data such as defective and outliers have been removed and a previously established multivariate discriminant with the concentration of the amino acid as explanatory variable, where the concentration value of at least one of Tau, Urea, Thr, Asn, Pro, Gln, Ala, Cit, ABA, Val, Met, Leu, Tyr, Phe, His, Trp, Lys, Arg and Cys is contained as the explanatory variable, and the plurality of the calculated discriminant values are compared with a previously established threshold (cutoff value) for each of the plurality of the discriminant values, thereby evaluating a condition of UC in the individual.

18. Discrimination between CD and UC

The concentration value of at least one of Cys, ABA, Thr, Pro, Gly, Met, Ile and Orn contained in the amino acid concentration data of the individual from which data such as defective and outliers have been removed, is compared with a previously established threshold (cutoff value), thereby discriminating between CD and UC in the individual. Alternatively, a discriminant value that is a value of multivariate discriminant is calculated based on both the concentration value of at least one of Cys, ABA, Thr, Pro, Gly, Met, Ile and Orn contained in the amino acid concentration data of the individual from which data such as defective and outliers have been removed and a previously established multivariate discriminant with the concentration of the amino acid as explanatory variable, where the concentration value of at least one of Cys, ABA, Thr, Pro, Gly, Met, Ile and Orn is contained as the explanatory variable, and the calculated discriminant value is compared with a previously established threshold (cutoff value), thereby discriminating between CD and UC in the individual.

19. Discrimination between CD in active phase and UC in active phase

The concentration value of at least one of Tau, Thr, Ser, Gly, Met and Ile contained in the amino acid concentration data of the individual from which data such as defective and outliers have been removed, is compared with a previously established threshold (cutoff value), thereby discriminating between CD in active phase and UC in active phase in the individual. Alternatively, a discriminant value that is a value of multivariate discriminant is calculated based on both the concentration value of at least one of Tau, Thr, Ser, Gly, Met and Ile contained in the amino acid concentration data of the individual from which data such as defective and outliers have been removed and a previously established multivariate discriminant with the concentration of the amino acid as explanatory variable, where the concentration value of at least one of Tau, Thr, Ser, Gly, Met and Ile is contained as the explanatory variable, and the calculated discriminant value is compared with a previously established threshold (cutoff value), thereby discriminating between CD in active phase and UC in active phase in the individual.

1-3. Summary of the first embodiment and other embodiments

In the method of evaluating IBD as described above in detail, (1) the amino acid concentration data is measured from blood collected from the individual, (2) data such as defective and outliers are removed from the measured amino acid concentration data of the individual, and (3) either the discrimination between 2 groups or the evaluation of the condition of disease as described in 11. to 19. above is conducted for the individual, based on the amino acid concentration data of the individual from which data such as defective and outliers have been removed, or on a previously established multivariate discriminant with the concentration of amino acid as the explanatory variable. Thus, the concentrations of the amino acids which among amino acids in blood, are useful for discriminating between the 2 groups of IBD and IBD-free or a discriminant value obtained in a multivariate discriminant useful for discriminating between the 2 groups of IBD and IBD-free can be utilized to bring about an effect of enabling accurate discrimination between the 2 groups of IBD and IBD-free. The concentrations of the amino acids which among amino acids in blood, are useful for discriminating between the 2 groups of CD and CD-free or a discriminant value obtained in a multivariate discriminant useful for discriminating between the 2 groups of CD and CD-free can be utilized to bring about an effect of enabling accurate discrimination between the 2 groups of CD and CD-free. The concentrations of the amino acids which among amino acids in blood, are useful for discriminating between the 2 groups of active phase and remission phase of CD or a discriminant value obtained in a multivariate discriminant useful for discriminating between the 2 groups of active phase and remission phase of CD can be utilized to bring about an effect of enabling accurate discrimination between the 2 groups of active phase and remission phase of CD. The concentrations of the amino acids which among amino acids in blood, are useful for evaluating a condition of CD or a discriminant value obtained in a multivariate discriminant useful for evaluating a condition of CD can be utilized to bring about an effect of enabling accurate evaluation of a condition of CD. The concentrations of the amino acids which among amino acids in blood, are useful for discriminating between the 2 groups of UC and UC-free or a discriminant value obtained in a multivariate discriminant useful for discriminating between the 2 groups of UC and UC-free can be utilized to bring about an effect of enabling accurate discrimination between the 2 groups of UC and UC-free. The concentrations of the amino acids which among amino acids in blood, are useful for discriminating between the 2 groups of active phase and remission phase of UC or a discriminant value obtained in a multivariate discriminant useful for discriminating between the 2 groups of active phase and remission phase of UC can be utilized to bring about an effect of enabling accurate discrimination between the 2 groups of active phase and remission phase of UC. The concentrations of the amino acids which among amino acids in blood, are useful for evaluating a condition of UC or a discriminant value obtained in a multivariate discriminant useful for evaluating a condition of UC can be utilized to bring about an effect of enabling accurate evaluation of a condition of UC. The concentrations of the amino acids which among amino acids in blood, are useful for discriminating between the 2 groups of CD and UC or a discriminant value obtained in a multivariate discriminant useful for discriminating between the 2 groups of CD and UC can be utilized to bring about an effect of enabling accurate discrimination between the 2 groups of CD and UC. The concentrations of the amino acids which among amino acids in blood, are useful for discriminating between the 2 groups of CD in active phase and UC in active phase or a discriminant value obtained in a multivariate discriminant useful for discriminating between the 2 groups of CD in active phase and UC in active phase can be utilized to bring about an effect of enabling accurate discrimination between the 2 groups of CD in active phase and UC in active phase.

When the discrimination between 2 groups as described in 11. above is conducted in step SA-13, the multivariate discriminant may be expressed by one fractional expression or the sum of a plurality of the fractional expressions, and contain the concentration value of at least one of Tau, Glu, Pro and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contain the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau, Glu, Pro and Cys as the explanatory variable in the denominator in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Tau, Leu, Tyr, His, Ile and Thr as the explanatory variables. Thus, a discriminant value obtained in a multivariate discriminant useful particularly for discriminating between the 2 groups of IBD and IBD-free can be utilized to bring about an effect of enabling more accurate discrimination between the 2 groups of IBD and IBD-free.

When the discrimination between 2 groups as described in 12. above is conducted in step SA-13, the multivariate discriminant may be expressed by one fractional expression or the sum of a plurality of fractional expressions, and contain the concentration value of at least one of Tau, Glu, Pro and Gly as the explanatory variable in the numerator in the fractional expression constituting the multivariate discriminant, and the concentration value of at least one of Urea, Asn, Gln, Thr, ABA, Val, Cys, Leu, Tyr, Phe, His, Trp and Lys as the explanatory variable in the denominator in the fractional expression constituting the multivariate discriminant, or contain the concentration value of at least one of Urea, Asn, Gln, Thr, ABA, Val, Cys, Leu, Tyr, Phe, His, Trp and Lys as the explanatory variable in the numerator in the fractional expression constituting the multivariate discriminant, and the concentration value of at least one of Tau, Glu, Pro and Gly as the explanatory variable in the denominator in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Tau, Val, Leu, Tyr, Pro and Ile as the explanatory variables. Thus, a discriminant value obtained in a multivariate discriminant useful particularly for discriminating between the 2 groups of CD and CD-free can be utilized to bring about an effect of enabling more accurate discrimination between the 2 groups of CD and CD-free.

When the discrimination between 2 groups as described in 13. above or the evaluation of the condition of disease as described in 14. above is conducted in step SA-13, the multivariate discriminant may be expressed by one fractional expression or the sum of a plurality of fractional expressions and contain the concentration value of at least one of Gln, Cit, Val, Leu, His, Trp, Lys and Arg as the explanatory variable in any one of the numerator and denominator or both in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of His, Trp, Lys, Ser, Tau and Ile as the explanatory variables. Thus, a discriminant value obtained in a multivariate discriminant useful particularly for discriminating between the 2 groups of active phase and remission phase of CD can be utilized to bring about an effect of enabling more accurate discrimination between the 2 groups of active phase and remission phase of CD. A discriminant value obtained in a multivariate discriminant useful particularly for evaluating a condition of CD can be utilized to bring about an effect of enabling more accurate evaluation of a condition of CD.

When the discrimination between 2 groups as described in 15. above is conducted in step SA-13, the multivariate discriminant may be expressed by one fractional expression or the sum of a plurality of fractional expressions, and contain the concentration value of at least one of Tau and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contain the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau and Cys as the explanatory variable in the denominator in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Tau, Tyr, Leu, His, Ala and Ile as the explanatory variables. Thus, a discriminant value obtained in a multivariate discriminant useful particularly for discriminating between the 2 groups of UC and UC-free can be utilized to bring about an effect of enabling more accurate discrimination between the 2 groups of UC and UC-free.

When the discrimination between 2 groups as described in 16. above or the evaluation of the condition of disease as described in 17. above is conducted in step SA-13, the multivariate discriminant may be expressed by one fractional expression or the sum of a plurality of fractional expressions and contain the concentration value of at least one of Tau, Urea, Thr, Asn, Pro, Gln, Ala, Cit, ABA, Val, Met, Leu, Tyr, Phe, His, Trp, Lys, Arg and Cys as the explanatory variable in any one of the numerator and denominator or both in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' distance method, a discriminant prepared by canonical discriminant analysis and a discriminant prepared by a decision tree, and have the concentration value of His, Tyr, Val, Met, Leu and Arg as the explanatory variables. Thus, a discriminant value obtained in a multivariate discriminant useful particularly for discriminating between the 2 groups of active phase and remission phase of UC can be utilized to bring about an effect of enabling more accurate discrimination between the 2 groups of active phase and remission phase of UC. A discriminant value obtained in a multivariate discriminant useful particularly for evaluating a condition of UC can be utilized to bring about an effect of enabling more accurate evaluation of a condition of UC.

When the discrimination between 2 groups as described in 18. above is conducted in step SA-13, the multivariate discriminant may be expressed by one fractional expression or the sum of a plurality of fractional expressions, and contain the concentration value of at least one of Cys and ABA as the explanatory variable in the numerator in the fractional expression constituting the multivariate discriminant and the concentration value of at least one of Thr, Pro, Gly, Met, Ile and Orn as the explanatory variable in the denominator in the fractional expression constituting the multivariate discriminant, or contain the concentration value of at least one of Thr, Pro, Gly, Met, Ile and Orn as the explanatory variable in the numerator in the fractional expression constituting the multivariate discriminant and the concentration value of at least one of Cys and ABA as the explanatory variable in the denominator in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Met, Pro, Ile, Trp, Tau and Val as the explanatory variables. Thus, a discriminant value obtained in a multivariate discriminant useful particularly for discriminating between the 2 groups of CD and UC can be utilized to bring about an effect of enabling more accurate discrimination between the 2 groups of CD and UC.

When the discrimination between 2 groups as described in 19. above is conducted in step SA-13, the multivariate discriminant may be expressed by one fractional expression or the sum of a plurality of fractional expressions, and contain the concentration value of at least one of Tau, Thr, Ser, Gly, Met and Ile as the explanatory variable in any one of the numerator and denominator or both in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Met, Tau, Trp, Tyr, Orn and Phe as the explanatory variables. Thus, a discriminant value obtained in a multivariate discriminant useful particularly for discriminating between the 2 groups of CD in active phase and UC in active phase can be utilized to bring about an effect of enabling more accurate discrimination between the 2 groups of CD in active phase and UC in active phase.

The multivariate discriminants described above can be prepared by a method described in International Publication WO 2004/052191 that is an international application filed by the present applicant or by a method (multivariate discriminant-preparing processing described in the second embodiment described later) described in International Publication WO 2006/098192 that is an international application filed by the present applicant. Any multivariate discriminants obtained by these methods can be preferably used in evaluation of an IBD state, regardless of the unit of amino acid concentration in the amino acid concentration data as input data.

Second Embodiment

2-1. Outline of the Invention

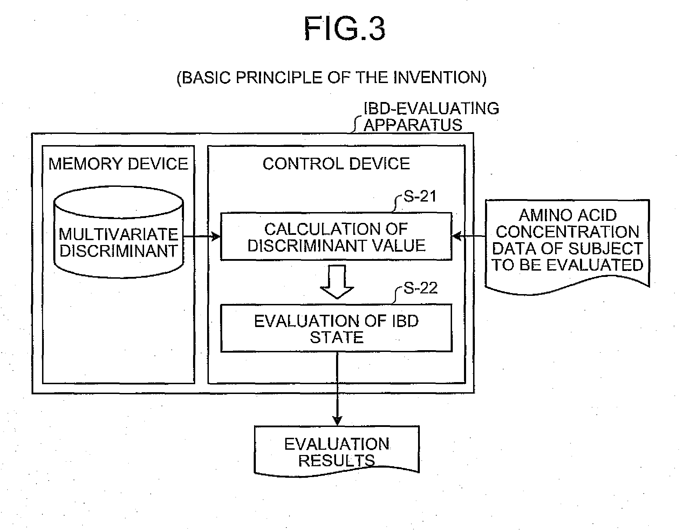

Herein, an outline of the IBD-evaluating apparatus including the amino acid data processor of the present invention, the IBD-evaluating method including the amino acid data-processing method of the present invention, the IBD-evaluating system including the amino acid data-processing system of the present invention, the IBD-evaluating program including the amino acid data-processing program of the present invention and the recording medium of the present invention are described in detail with reference to FIG. 3. FIG. 3 is a principle configurational diagram showing the basic principle of the present invention.

In the present invention, a discriminant value that is a value of a multivalent discriminant is calculated in a control device based on both previously obtained amino acid concentration data on concentration values of amino acids in a subject (for example, an individual such as animal or human) to be evaluated and a previously established multivariate discriminant with concentrations of amino acids as explanatory variables stored in a memory device (step S-21).

In the present invention, an IBD state in the subject is evaluated in the control device based on the discriminant value calculated in step S-21 (step S-22).

According to the present invention described above, the discriminant value that is the value of multivariate discriminant is calculated based on both the previously obtained amino acid concentration data on the concentration value of amino acid in the subject and the multivariate discriminant with the concentration of the amino acid as explanatory variable stored in the memory device, and the IBD state in the subject is evaluated based on the calculated discriminant value. Thus, a discriminant value obtained in a multivariate discriminant can be utilized to bring about an effect of enabling accurate evaluation of an IBD state.

In step S-22, discrimination between IBD and IBD-free in the subject may be conducted based on the discriminant value calculated in step S-21. Thus, a discriminant value obtained in a multivariate discriminant can be utilized to bring about an effect of enabling accurate discrimination between the 2 groups of IBD and IBD-free. In step S-21, a discriminant value that is a value of multivariate discriminant may be calculated based on both the concentration value of at least one of Tau, Urea, Asn, Glu, Gln, Pro, Ala, Cit, Gly, ABA, Val, Cys, Leu, Tyr, Phe, His, Trp, Orn and Lys contained in the amino acid concentration data of the subject and the multivariate discriminant with the concentration of the amino acid as explanatory variable, where the concentration value of at least one of Tau, Urea, Asn, Glu, Gln, Pro, Ala, Cit, Gly, ABA, Val, Cys, Leu, Tyr, Phe, His, Trp, Orn and Lys is contained as the explanatory variable, and in step S-22, discrimination between IBD and IBD-free in the subject may be conducted based on the discriminant value calculated in step S-21. Specifically, the discriminant value may be compared with a previously established threshold (cutoff value), thereby discriminating between IBD and IBD-free in the subject. Thus, a discriminant value obtained in a multivariate discriminant useful for discriminating between the 2 groups of IBD and IBD-free can be utilized to bring about an effect of enabling accurate discrimination between the 2 groups of IBD and IBD-free. The multivariate discriminant may be expressed by one fractional expression or the sum of a plurality of the fractional expressions, and contain the concentration value of at least one of Tau, Glu, Pro and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contain the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau, Glu, Pro and Cys as the explanatory variable in the denominator in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Tau, Leu, Tyr, His, Ile and Thr as the explanatory variables. Thus, a discriminant value obtained in a multivariate discriminant useful particularly for discriminating between the 2 groups of IBD and IBD-free can be utilized to bring about an effect of enabling more accurate discrimination between the 2 groups of IBD and IBD-free.

In step S-22, a CD state in the subject may be evaluated based on the discriminant value calculated in step S-21. Thus, a discriminant value obtained in a multivariate discriminant can be utilized to bring about an effect of enabling accurate evaluation of a CD state. In step S-22, discrimination between CD and CD-free in the subject may be conducted based on the discriminant value calculated in step S-21. Thus, a discriminant value obtained in a multivariate discriminant can be utilized to bring about an effect of enabling accurate discrimination between the 2 groups of CD and CD-free. In step S-21, a discriminant value that is a value of multivariate discriminant may be calculated based on both the concentration value of at least one of Tau, Thr, Urea, Asn, Glu, Gln, Pro, Gly, ABA, Val, Cys, Leu, Tyr, Phe, His, Trp and Lys contained in the amino acid concentration data of the subject and the multivariate discriminant with the concentration of the amino acid as explanatory variable, where the concentration value of at least one of Tau, Thr, Urea, Asn, Glu, Gln, Pro, Gly, ABA, Val, Cys, Leu, Tyr, Phe, His, Trp and Lys is contained as the explanatory variable, and in step S-22, discrimination between CD and CD-free in the subject may be conducted based on the discriminant value calculated in step S-21. Specifically, the discriminant value may be compared with a previously established threshold (cutoff value), thereby discriminating between CD and CD-free in the subject. Thus, a discriminant value obtained in a multivariate discriminant useful for discriminating between the 2 groups of CD and CD-free can be utilized to bring about an effect of enabling accurate discrimination between the 2 groups of CD and CD-free. The multivariate discriminant may be expressed by one fractional expression or the sum of a plurality of fractional expressions, and contain the concentration value of at least one of Tau, Glu, Pro and Gly as the explanatory variable in the numerator in the fractional expression constituting the multivariate discriminant, and the concentration value of at least one of Urea, Asn, Gln, Thr, ABA, Val, Cys, Leu, Tyr, Phe, His, Trp and Lys as the explanatory variable in the denominator in the fractional expression constituting the multivariate discriminant, or contain the concentration value of at least one of Urea, Asn, Gln, Thr, ABA, Val, Cys, Leu, Tyr, Phe, His, Trp and Lys as the explanatory variable in the numerator in the fractional expression constituting the multivariate discriminant, and the concentration value of at least one of Tau, Glu, Pro and Gly as the explanatory variable in the denominator in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Tau, Val, Leu, Tyr, Pro and Ile as the explanatory variables. Thus, a discriminant value obtained in a multivariate discriminant useful particularly for discriminating between the 2 groups of CD and CD-free can be utilized to bring about an effect of enabling more accurate discrimination between the 2 groups of CD and CD-free.

In step S-22, discrimination between active phase and remission phase of CD in the subject may be conducted based on the discriminant value calculated in step S-21. Thus, a discriminant value obtained in a multivariate discriminant can be utilized to bring about an effect of enabling accurate discrimination between the 2 groups of active phase and remission phase of CD. In step S-21, a discriminant value that is a value of multivariate discriminant may be calculated based on both the concentration value of at least one of Gln, Cit, Val, Leu, His, Trp, Lys and Arg contained in the amino acid concentration data of the subject and the multivariate discriminant with the concentration of the amino acid as explanatory variable, where the concentration value of at least one of Gln, Cit, Val, Leu, His, Trp, Lys and Arg is contained as the explanatory variable, and in step S-22, discrimination between active phase and remission phase of CD in the subject may be conducted based on the discriminant value calculated in step S-21. Specifically, the discriminant value may be compared with a previously established threshold (cutoff value), thereby discriminating between active phase and remission phase of CD in the subject. Thus, a discriminant value obtained in a multivariate discriminant useful for discriminating between the 2 groups of active phase and remission phase of CD can be utilized to bring about an effect of enabling accurate discrimination between the 2 groups of active phase and remission phase of CD. The multivariate discriminant may be expressed by one fractional expression or the sum of a plurality of fractional expressions and contain the concentration value of at least one of Gln, Cit, Val, Leu, His, Trp, Lys and Arg as the explanatory variable in any one of the numerator and denominator or both in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of His, Trp, Lys, Ser, Tau and Ile as the explanatory variables. Thus, a discriminant value obtained in a multivariate discriminant useful particularly for discriminating between the 2 groups of active phase and remission phase of CD can be utilized to bring about an effect of enabling more accurate discrimination between the 2 groups of active phase and remission phase of CD.

When a plurality of the amino acid concentration data of the subject are obtained previously, in step S-21, a discriminant value that is a value of multivariate discriminant may be calculated based on both the plurality of the amino acid concentration data of the subject and the multivariate discriminant with the concentration of the amino acid as explanatory variable, for each of the plurality of the amino acid concentration data, and in step S-22, the condition of CD in the subject may be evaluated based on the plurality of the discriminant values calculated in step S-21. Thus, a discriminant value obtained in a multivariate discriminant can be utilized to bring about an effect of enabling accurate evaluation of a condition of CD. In step S-21, discriminant values may be calculated based on both the concentration value of at least one of Gln, Cit, Val, Leu, His, Trp, Lys and Arg contained in the plurality of the amino acid concentration data of the subject and the multivariate discriminant with the concentration of the amino acid as explanatory variable, where the concentration value of at least one of Gln, Cit, Val, Leu, His, Trp, Lys and Arg is contained as the explanatory variable, for each of the plurality of the amino acid concentration data, and in step S-22, the condition of CD in the subject may be evaluated based on the plurality of the discriminant values calculated in step S-21. Specifically, the discriminant value may be compared with a previously established threshold (cutoff value), thereby evaluating the condition of CD in the subject.

Thus, a discriminant value obtained in a multivariate discriminant useful for evaluating a condition of CD can be utilized to bring about an effect of enabling accurate evaluation of a condition of CD. The multivariate discriminant may be expressed by one fractional expression or the sum of a plurality of fractional expressions and contain the concentration value of at least one of Gln, Cit, Val, Leu, His, Trp, Lys and Arg as the explanatory variable in any one of the numerator and denominator or both in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of His, Trp, Lys, Ser, Tau and Ile as the explanatory variables. Thus, a discriminant value obtained in a multivariate discriminant useful particularly for evaluating a condition of CD can be utilized to bring about an effect of enabling more accurate evaluation of a condition of CD.

In step S-22, a UC state in the subject may be evaluated based on the discriminant value calculated in step S-21. Thus, a discriminant value obtained in a multivariate discriminant can be utilized to bring about an effect of enabling accurate evaluation of a UC state. In step S-22, discrimination between UC and UC-free in the subject may be conducted based on the discriminant value calculated in step S-21. Thus, a discriminant value obtained in a multivariate discriminant can be utilized to bring about an effect of enabling accurate discrimination between the 2 groups of UC and UC-free. In step S-21, a discriminant value that is a value of multivariate discriminant may be calculated based on both the concentration value of at least one of Tau, Urea, Asn, Gln, Ala, Cit, ABA, Val, Cys, Met, Ile, Leu, Tyr, Phe, His, Trp, Orn and Lys contained in the amino acid concentration data of the subject and a previously established multivariate discriminant with the concentration of the amino acid as explanatory variable, where the concentration value of at least one of Tau, Urea, Asn, Gln, Ala, Cit, ABA, Val, Cys, Met, Ile, Leu, Tyr, Phe, His, Trp, Orn and Lys is contained as the explanatory variable, and in step S-22, discrimination between UC and UC-free in the subject may be conducted based on the discriminant value calculated in step S-21. Specifically, the discriminant value may be compared with a previously established threshold (cutoff value), thereby discriminating between UC and UC-free in the subject. Thus, a discriminant value obtained in a multivariate discriminant useful for discriminating between the 2 groups of UC and UC-free can be utilized to bring about an effect of enabling accurate discrimination between the 2 groups of UC and UC-free. The multivariate discriminant may be expressed by one fractional expression or the sum of a plurality of fractional expressions, and contain the concentration value of at least one of Tau and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contain the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau and Cys as the explanatory variable in the denominator in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Tau, Tyr, Leu, His, Ala and Ile as the explanatory variables. Thus, a discriminant value obtained in a multivariate discriminant useful particularly for discriminating between the 2 groups of UC and UC-free can be utilized to bring about an effect of enabling more accurate discrimination between the 2 groups of UC and UC-free.

In step S-22, discrimination between active phase and remission phase of UC in the subject may be conducted based on the discriminant value calculated in step S-21. Thus, a discriminant value obtained in a multivariate discriminant can be utilized to bring about an effect of enabling accurate discrimination between the 2 groups of active phase and remission phase of UC. In step S-21, a discriminant value that is a value of multivariate discriminant may be calculated based on both the concentration value of at least one of Tau, Urea, Thr, Asn, Pro, Gln, Ala, Cit, ABA, Val, Met, Leu, Tyr, Phe, His, Trp, Lys, Arg and Cys contained in the amino acid concentration data of the subject and the multivariate discriminant with the concentration of the amino acid as explanatory variable, where the concentration value of at least one of Tau, Urea, Thr, Asn, Pro, Gln, Ala, Cit, ABA, Val, Met, Leu, Tyr, Phe, His, Trp, Lys, Arg and Cys is contained as the explanatory variable, and in step S-22, discrimination between active phase and remission phase of UC in the subject may be conducted based on the discriminant value calculated in step S-21. Specifically, the discriminant value may be compared with a previously established threshold (cutoff value), thereby discriminating between active phase and remission phase of UC in the subject. Thus, a discriminant value obtained in a multivariate discriminant useful for discriminating between the 2 groups of active phase and remission phase of UC can be utilized to bring about an effect of enabling accurate discrimination between the 2 groups of active phase and remission phase of UC. The multivariate discriminant may be expressed by one fractional expression or the sum of a plurality of fractional expressions and contain the concentration value of at least one of Tau, Urea, Thr, Asn, Pro, Gln, Ala, Cit, ABA, Val, Met, Leu, Tyr, Phe, His, Trp, Lys, Arg and Cys as the explanatory variable in any one of the numerator and denominator or both in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' distance method, a discriminant prepared by canonical discriminant analysis and a discriminant prepared by a decision tree, and have the concentration value of His, Tyr, Val, Met, Leu and Arg as the explanatory variables. Thus, a discriminant value obtained in a multivariate discriminant useful particularly for discriminating between the 2 groups of active phase and remission phase of UC can be utilized to bring about an effect of enabling more accurate discrimination between the 2 groups of active phase and remission phase of UC.

When a plurality of the amino acid concentration data of the subject are obtained previously, in step S-21, a discriminant value that is a value of multivariate discriminant may be calculated based on both the plurality of the amino acid concentration data of the subject and the multivariate discriminant with the concentration of the amino acid as explanatory variable, for each of the plurality of the amino acid concentration data, and in step S-22, the condition of UC in the subject may be evaluated based on the plurality of the discriminant values calculated in step S-21. Thus, a discriminant value obtained in a multivariate discriminant can be utilized to bring about an effect of enabling accurate evaluation of a condition of UC. In step S-21, discriminant values may be calculated based on both the concentration value of at least one of Tau, Urea, Thr, Asn, Pro, Gln, Ala, Cit, ABA, Val, Met, Leu, Tyr, Phe, His, Trp, Lys, Arg and Cys contained in the plurality of the amino acid concentration data of the subject and the multivariate discriminant with the concentration of the amino acid as explanatory variable, where the concentration value of at least one of Tau, Urea, Thr, Asn, Pro, Gln, Ala, Cit, ABA, Val, Met, Leu, Tyr, Phe, His, Trp, Lys, Arg and Cys is contained as the explanatory variable, for each of the plurality of the amino acid concentration data, and in step S-22, the condition of UC in the subject may be evaluated based on the plurality of the discriminant values calculated in step S-21. Specifically, the discriminant value may be compared with a previously established threshold (cutoff value), thereby evaluating the condition of UC in the subject. Thus, a discriminant value obtained in a multivariate discriminant useful for evaluating a condition of UC can be utilized to bring about an effect of enabling accurate evaluation of a condition of UC. The multivariate discriminant may be expressed by one fractional expression or the sum of a plurality of fractional expressions and contain the concentration value of at least one of Tau, Urea, Thr, Asn, Pro, Gln, Ala, Cit, ABA, Val, Met, Leu, Tyr, Phe, His, Trp, Lys, Arg and Cys as the explanatory variable in any one of the numerator and denominator or both in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' distance method, a discriminant prepared by canonical discriminant analysis and a discriminant prepared by a decision tree, and have the concentration value of His, Tyr, Val, Met, Leu and Arg as the explanatory variables. Thus, a discriminant value obtained in a multivariate discriminant useful particularly for evaluating a condition of UC can be utilized to bring about an effect of enabling more accurate evaluation of a condition of UC.

In step S-22, discrimination between CD and UC in the subject may be conducted based on the discriminant value calculated in step S-21. Thus, a discriminant value obtained in a multivariate discriminant can be utilized to bring about an effect of enabling accurate discrimination between the 2 groups of CD and UC. In step S-21, a discriminant value that is a value of multivariate discriminant may be calculated based on both the concentration value of at least one of Cys, ABA, Thr, Pro, Gly, Met, Ile and Orn contained in the amino acid concentration data of the subject and the multivariate discriminant with the concentration of the amino acid as explanatory variable, where the concentration value of at least one of Cys, ABA, Thr, Pro, Gly, Met, Ile and Orn as the explanatory variable, and in step S-22, discrimination between CD and UC in the subject may be conducted based on the discriminant value calculated in step S-21. Specifically, the discriminant value may be compared with a previously established threshold (cutoff value), thereby discriminating between CD and UC in the subject. Thus, a discriminant value obtained in a multivariate discriminant useful for discriminating between the 2 groups of CD and UC can be utilized to bring about an effect of enabling accurate discrimination between the 2 groups of CD and UC. The multivariate discriminant may be expressed by one fractional expression or the sum of a plurality of fractional expressions, and contain the concentration value of at least one of Cys and ABA as the explanatory variable in the numerator in the fractional expression constituting the multivariate discriminant and the concentration value of at least one of Thr, Pro, Gly, Met, Ile and Orn as the explanatory variable in the denominator in the fractional expression constituting the multivariate discriminant, or contain the concentration value of at least one of Thr, Pro, Gly, Met, Ile and Orn as the explanatory variable in the numerator in the fractional expression constituting the multivariate discriminant and the concentration value of at least one of Cys and ABA as the explanatory variable in the denominator in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Met, Pro, Ile, Trp, Tau and Val as the explanatory variables. Thus, a discriminant value obtained in a multivariate discriminant useful particularly for discriminating between the 2 groups of CD and UC can be utilized to bring about an effect of enabling more accurate discrimination between the 2 groups of CD and UC.

In step S-22, discrimination between CD in active phase and UC in active phase in the subject may be conducted based on the discriminant value calculated in step S-21. Thus, a discriminant value obtained in a multivariate discriminant can be utilized to bring about an effect of enabling accurate discrimination between the 2 groups of CD in active phase and UC in active phase. In step S-21, a discriminant value that is a value of multivariate discriminant may be calculated based on both the concentration value of at least one of Tau, Thr, Ser, Gly, Met and Ile contained in the amino acid concentration data of the subject and the multivariate discriminant with the concentration of the amino acid as explanatory variable, where the concentration value of at least one of Tau, Thr, Ser, Gly, Met and Ile is contained as the explanatory variable, and in step S-22, discrimination between CD in active phase and UC in active phase in the subject may be conducted based on the discriminant value calculated in step S-21. Specifically, the discriminant value may be compared with a previously established threshold (cutoff value), thereby discriminating between CD in active phase and UC in active phase in the subject. Thus, a discriminant value obtained in a multivariate discriminant useful for discriminating between the 2 groups of CD in active phase and UC in active phase can be utilized to bring about an effect of enabling accurate discrimination between the 2 groups of CD in active phase and UC in active phase. The multivariate discriminant may be expressed by one fractional expression or the sum of a plurality of fractional expressions, and contain the concentration value of at least one of Tau, Thr, Ser, Gly, Met and Ile as the explanatory variable in any one of the numerator and denominator or both in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Met, Tau, Trp, Tyr, Orn and Phe as the explanatory variables. Thus, a discriminant value obtained in a multivariate discriminant useful particularly for discriminating between the 2 groups of CD in active phase and UC in active phase can be utilized to bring about an effect of enabling more accurate discrimination between the 2 groups of CD in active phase and UC in active phase.

The multivariate discriminants described above can be prepared by a method described in International Publication WO 2004/052191 that is an international application filed by the present applicant or by a method (multivariate discriminant-preparing processing described later) described in International Publication WO 2006/098192 that is an international application filed by the present applicant. Any multivariate discriminants obtained by these methods can be preferably used in evaluation of an IBD state, regardless of the unit of amino acid concentration in the amino acid concentration data as input data.

In a fractional expression, the numerator of the fractional expression is expressed by the sum of amino acids A, B, C etc. and the denominator of the fractional expression is expressed by the sum of amino acids a, b, c etc. The fractional expression also includes the sum of fractional expressions $\alpha$, $\beta$, $\gamma$ etc. (for example, $\alpha+\beta$) having such constitution. The fractional expression also includes divided fractional expressions. Amino acids used in the numerator or denominator may have suitable coefficients respectively. The amino acids used in the numerator or denominator may appear repeatedly. Each fractional expression may have a suitable coefficient. The value of a coefficient for each explanatory variable and the value for a constant term may be any real numbers. In combinations where explanatory variables in the numerator and explanatory variables in the denominator in the fractional expression are switched with each other, the positive (or negative) sign is generally reversed in correlation with objective explanatory variables, but because their correlation is maintained, such combinations can be assumed to be equivalent to one another in discrimination, and thus the fractional expression also includes combinations where explanatory variables in the numerator and explanatory variables in the denominator in the fractional expression are switched with each other.

The multivariate discriminant refers to a form of equation used generally in multivariate analysis and includes, for example, multiple regression equation, multiple logistic regression equation, linear discriminant function, Mahalanobis' generalized distance, canonical discriminant function, support vector machine, and decision tree. The multivariate discriminant also includes an equation shown by the sum of different forms of multivariate discriminants. In the multiple regression equation, multiple logistic regression equation and canonical discriminant function, a coefficient and constant term are added to each explanatory variable, and the coefficient and constant term in this case are preferably real numbers, more preferably values in the range of 99% confidence interval for the coefficient and constant term obtained from data for discrimination, more preferably in the range of 95% confidence interval for the coefficient and constant term obtained from data for discrimination. The value of each coefficient and the confidence interval thereof may be those multiplied by a real number, and the value of each constant term and the confidence interval thereof may be those having an arbitrary actual constant added or subtracted or those multiplied or divided by an arbitrary actual constant.

When IBD state is evaluated in the present invention, the concentrations of other metabolites, the protein expression level, the age and sex of the subject or the like may be used in addition to the amino acid concentration. When IBD state is evaluated in the present invention, the concentrations of other metabolites, the protein expression level, the age and sex of the subject or the like may be used as explanatory variables in the multivariate discriminant in addition to the amino acid concentration.

Here, the summary of the multivariate discriminant-preparing processing (steps 1 to 4) is described in detail.

First, from IBD state information including amino acid concentration data and IBD state index data concerning an index showing an IBD state stored in a memory device, a candidate multivariate discriminant (e.g., $y=a_1x_1+a_2x_2+...+a_nx_n$, y: IBD state index data, $x_i$: amino acid concentration data, $a_i$: constant, i = 1,2, ..., n) that is a candidate for a multivariate discriminant is prepared by a predetermined discriminant-preparing method at the control device (step 1). Data containing defective and outliers may be removed in advance from the IBD state information.

In step 1, a plurality of candidate multivariate discriminants may be prepared from the IBD state information by using a plurality of different discriminant-preparing methods (including those for multivariate analysis such as principal component analysis, discriminant analysis, support vector machine, multiple regression analysis, logistic regression analysis, k-means method, cluster analysis, and decision tree). Specifically, a plurality of candidate multivariate discriminant groups may be prepared simultaneously and concurrently by using a plurality of different algorithms with the IBD state information which is multivariate data composed of the amino acid concentration data and the IBD state index data obtained by analyzing blood samples from a large number of healthy subjects and IBD patients. For example, two different candidate multivariate discriminants may be formed by performing discriminant analysis and logistic regression analysis simultaneously with different algorithms. Alternatively, a candidate multivariate discriminant may be formed by converting the IBD state information with the candidate multivariate discriminant prepared by performing principal component analysis and then performing discriminant analysis of the converted IBD state information. In this way, it is possible to finally prepare the multivariate discriminant suitable for diagnostic condition.

The candidate multivariate discriminant prepared by principal component analysis is a linear expression consisting of amino acid explanatory variables maximizing the variance of all amino acid concentration data. The candidate multivariate discriminant prepared by discriminant analysis is a high-powered expression (including exponential and logarithmic expressions) consisting of amino acid explanatory variables minimizing the ratio of the sum of the variances in respective groups to the variance of all amino acid concentration data. The candidate multivariate discriminant prepared by using support vector machine is a high-powered expression (including kernel function) consisting of amino acid explanatory variables maximizing the boundary between groups. The candidate multivariate discriminant prepared by multiple regression analysis is a high-powered expression consisting of amino acid explanatory variables minimizing the sum of the distances from all amino acid concentration data. The candidate multivariate discriminant prepared by logistic regression analysis is a fraction expression having, as a component, the natural logarithm having a linear expression consisting of amino acid explanatory variables maximizing the likelihood as the exponent. The k-means method is a method of searching k pieces of neighboring amino acid concentration data in various groups designating the group containing the greatest number of the neighboring points as its data-belonging group, and selecting the amino acid explanatory variable that makes the group to which input amino acid concentration data belong agree well with the designated group. The cluster analysis is a method of clustering (grouping) the points closest in entire amino acid concentration data. The decision tree is a method of ordering amino acid explanatory variables and predicting the group of amino acid concentration data from the pattern possibly held by the higher-ordered amino acid explanatory variable.

Returning to the description of the multivariate discriminant-preparing processing, the candidate multivariate discriminant prepared in step 1 is verified (mutually verified) in the control device by a particular verification method (step 2). Verification of the candidate multivariate discriminant is performed on each other to each candidate multivariate discriminant prepared in step 1.

In step 2, at least one of the discrimination rate, sensitivity, specificity, information criterion, and the like of the candidate multivariate discriminant may be verified by at least one of the bootstrap method, holdout method, leave-one-out method, and the like. In this way, it is possible to prepare the candidate multivariate discriminant higher in predictability or reliability, by taking the IBD state information and the diagnostic condition into consideration.

The discrimination rate is the rate of the data wherein the IBD state evaluated according to the present invention is correct in all input data. The sensitivity is the rate of the IBD states judged correct according to the present invention in the IBD states declared IBD in the input data. The specificity is the rate of the IBD states judged correct according to the present invention in the IBD states described healthy in the input data. The information criterion is the sum of the number of the amino acid explanatory variables in the candidate multivariate discriminant prepared in step 1 and the difference in number between the IBD states evaluated according to the present invention and those described in input data. The predictability is the average of the discrimination rate, sensitivity, or specificity obtained by repeating verification of the candidate multivariate discriminant. Alternatively, the reliability is the variance of the discrimination rate, sensitivity, or specificity obtained by repeating verification of the candidate multivariate discriminant.

Returning to the description of the multivariate discriminant-preparing processing, a combination of amino acid concentration data contained in the IBD state information used in preparing the candidate multivariate discriminant is selected by selecting an explanatory variable of the candidate multivariate discriminant from the verification result in step 2 according to a predetermined explanatory variable selection method in the control device (step 3). The selection of amino acid explanatory variable is performed on each candidate multivariate discriminant prepared in step 1. In this way, it is possible to select the amino acid explanatory variable of the candidate multivariate discriminant properly. The step 1 is executed once again by using the IBD state information including the amino acid concentration data selected in step 3.

From the verification result in step 2, an amino acid explanatory variable of the candidate multivariate discriminant may be selected in step 3, based on at least one of stepwise method, best path method, local search method, and genetic algorithm.

The best path method is a method of selecting an amino acid explanatory variable by optimizing the evaluation index of the candidate multivariate discriminant while eliminating the explanatory variables contained in the candidate multivariate discriminant one by one.

Returning to the description of the multivariate discriminant-preparing processing, the steps 1, 2 and 3 are repeatedly performed in the control device, and based on verification results thus accumulated, a candidate multivariate discriminant used as the multivariate discriminant is selected from a plurality of candidate multivariate discriminants, thereby preparing the multivariate discriminant (step 4). In selection of the candidate multivariate discriminants, there are cases where the optimum multivariate discriminant is selected from candidate multivariate discriminants prepared in the same method or the optimum multivariate discriminant is selected from all candidate multivariate discriminants.

As described above, processing for preparation of candidate multivariate discriminants, verification of the candidate multivariate discriminants, and selection of explanatory variables in the candidate multivariate discriminants are performed based on the IBD state information in a series of operations in a systematized manner in the multivariate discriminant-preparing processing, whereby the optimum multivariate discriminant for evaluation of IBD state can be prepared.

2-2. System configuration

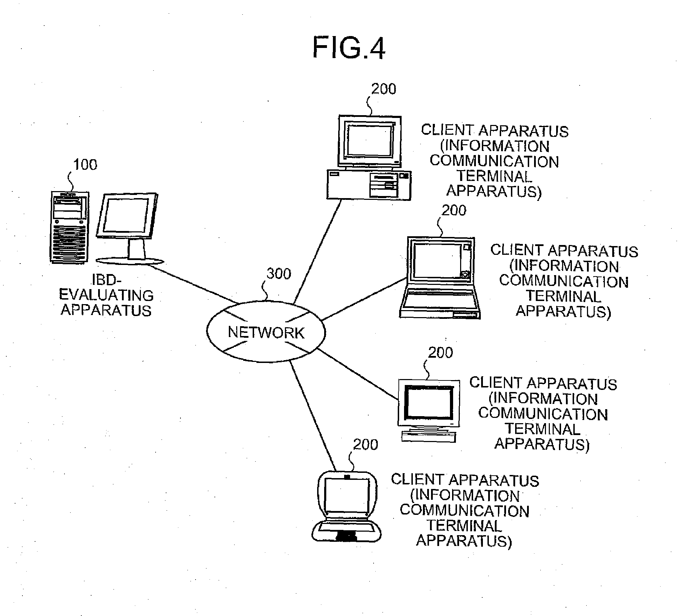

Hereinafter, the configuration of the IBD-evaluating system according to the second embodiment (hereinafter referred to sometimes as the present system) will be described with reference to FIGS. 4 to 20. This system is merely one example, and the present invention is not limited thereto.

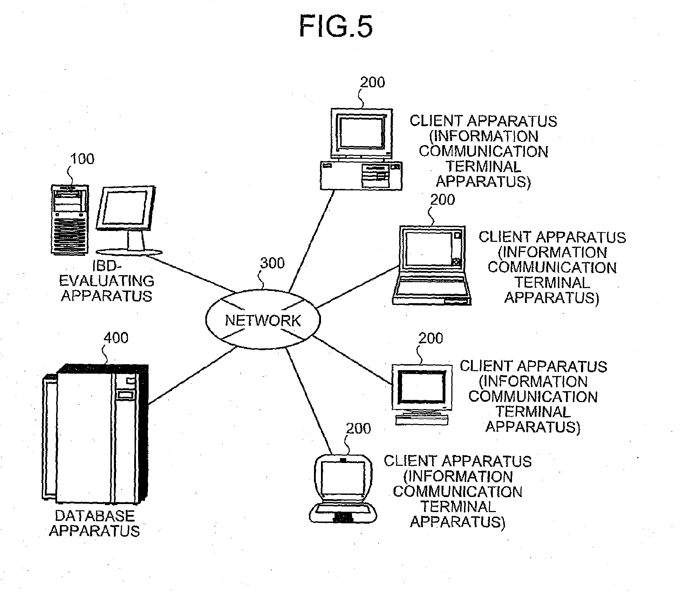

First, the entire configuration of the present system will be described with reference to FIGS. 4 and 5. FIG. 4 is a diagram showing an example of the entire configuration of the present system. FIG. 5 is a diagram showing another example of the entire configuration of the present system. As shown in FIG. 4, the present system is constituted in which an IBD-evaluating apparatus 100 that evaluates an IBD state in a subject to be evaluated, and a client apparatus 200 (corresponding to the information communication terminal apparatus of the present invention) which provides the amino acid concentration data on the concentration values of amino acids in the subject, are communicatively connected to each other via a network 300.

In the present system as shown in FIG. 5, in addition to the IBD-evaluating apparatus 100 and the client apparatus 200, a database apparatus 400 storing, for example, the IBD state information used in preparing a multivariate discriminant and the multivariate discriminant used in evaluating the IBD state in the IBD-evaluating apparatus 100, may be communicatively connected via the network 300. In this configuration, the information on an IBD state etc. are provided via the network 300 from the IBD-evaluating apparatus 100 to the client apparatuses 200 and the database apparatus 400, or from the client apparatuses 200 and the database apparatus 400 to the IBD-evaluating apparatus 100. The "information on an IBD state" is information on the measured values of particular items of the IBD state of organisms including human. The information on an IBD state is generated in the IBD-evaluating apparatus 100, client apparatus 200, and other apparatuses (e.g., various measuring apparatuses) and stored mainly in the database apparatus 400.

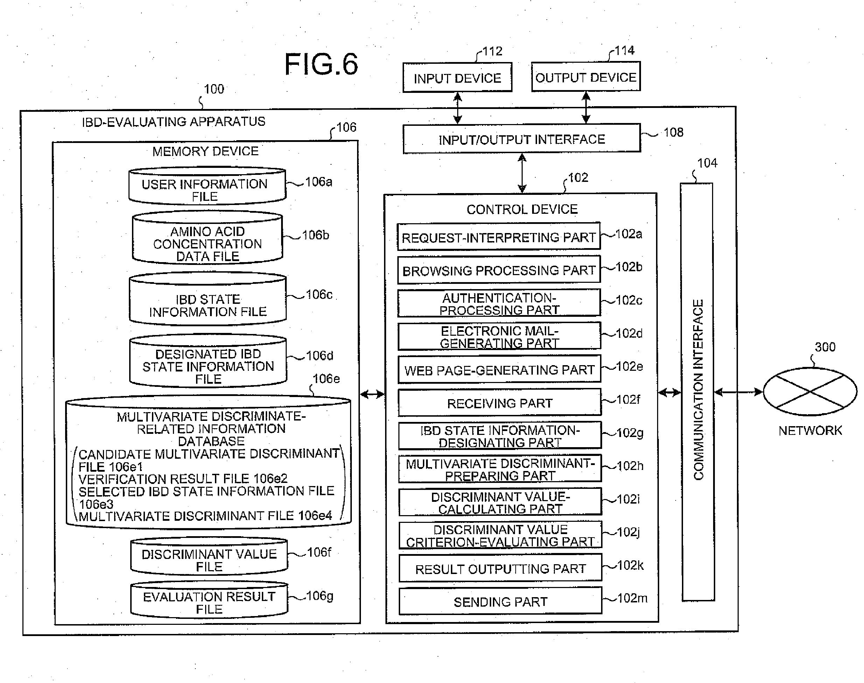

Now, the configuration of the IBD-evaluating apparatus 100 in the present system will be described with reference to FIGS. 6 to 18. FIG. 6 is a block diagram showing an example of the configuration of the IBD-evaluating apparatus 100 in the present system, showing conceptually only the region relevant to the present invention.

The IBD-evaluating apparatus 100 includes a control device 102, such as CPU (Central Processing Unit), that integrally controls the IBD-evaluating apparatus 100, a communication interface 104 that connects the IBD-evaluating apparatus 100 to the network 300 communicatively via communication apparatuses such as router and a wired or wireless communication line such as private line, a memory device 106 that stores various databases, tables, files and others, and an input/output interface 108 connected to an input device 112 and an output device 114, that are connected to each other communicatively via any communication channel. The IBD-evaluating apparatus 100 may be present together with various analyzers (e.g., amino acid analyzer) in a same housing. Typical configuration of disintegration/ integration of the IBD-evaluating apparatus 100 is not limited to that shown in the figure, and all or a part of it may be disintegrated or integrated functionally or physically in any unit, for example, according to various loads applied. For example, a part of the processing may be performed via a CGI (Common Gateway Interface).

The memory device 106 is a storage means, and examples thereof include memory apparatuses such as RAM (Random Access Memory) and ROM (Read Only Memory), fixed disk drives such as hard disk, flexible disk, optical disk, and the like. The memory device 106 stores computer programs giving instructions to CPU for various processing, together with OS (Operating System). As shown in the figure, the memory device 106 stores a user information file 106a, an amino acid concentration data file 106b, an IBD state information file 106c, a designated IBD state information file 106d, a multivariate discriminant-related information database 106e, a discriminant value file 106f and an evaluation result file 106g.

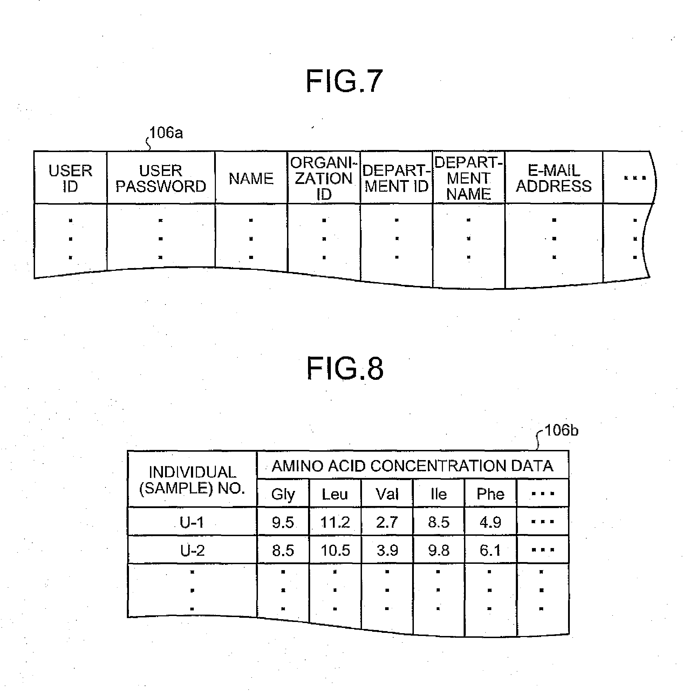

The user information file 106a stores a user information on users. FIG. 7 is a chart showing an example of the information stored in the user information file 106a. As shown in FIG. 7, the information stored in the user information file 106a includes user ID (identification) for identifying the user uniquely, user password for authentication of the user, user name, organization ID for uniquely identifying the organization of the user, department ID for uniquely identifying the department of the user organization, department name, and electronic mail address of the user that are correlated to one another.

Returning to FIG. 6, the amino acid concentration data file 106b stores amino acid concentration data on amino acid concentration values. FIG. 8 is a chart showing an example of the information stored in the amino acid concentration data file 106b. As shown in FIG. 8, the information stored in the amino acid concentration data file 106b includes individual number for uniquely identifying an individual (sample) as a subject to be evaluated and amino acid concentration data that are correlated to one another. In FIG. 8, the amino acid concentration data are assumed to be numerical values, i.e., on continuous scale, but the amino acid concentration data may be expressed on nominal scale or ordinal scale. In the case of nominal or ordinal scale, any number may be allocated to each state for analysis. The amino acid concentration data may be combined with other biological information (e.g., sex difference, age, smoking, digitalized electrocardiogram waveform, enzyme concentration, gene expression level, and the concentrations of metabolites other than amino acids).

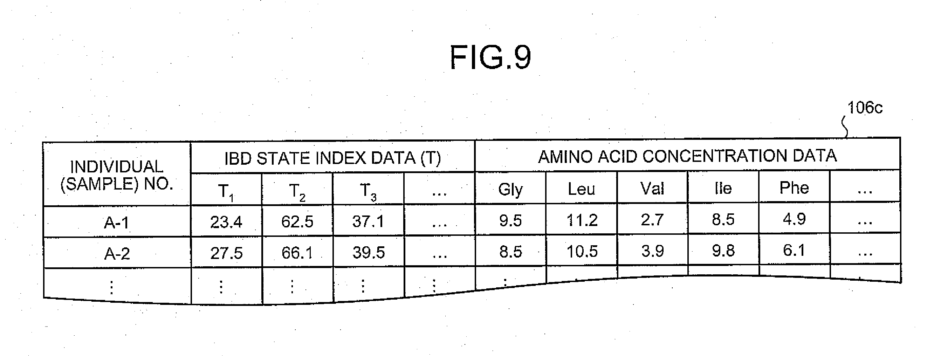

Returning to FIG. 6, the IBD state information file 106c stores the IBD state information used in preparing a multivariate discriminant. FIG. 9 is a chart showing an example of the information stored in the IBD state information file 106c. As shown in FIG. 9, the information stored in the IBD state information file 106c includes individual (sample) number, IBD state index data (T) corresponding to the IBD state index (index $T_1$, index $T_2$, index $T_3$ ...), and amino acid concentration data that are correlated to one another. In FIG. 9, the IBD state index data and the amino acid concentration data are assumed to be numerical values, i.e., on continuous scale, but the IBD state index data and the amino acid concentration data may be expressed on nominal scale or ordinal scale. In the case of nominal or ordinal scale, any number may be allocated to each state for analysis. The IBD state index data is a single known state index serving as a marker of IBD state, and numerical data may be used.

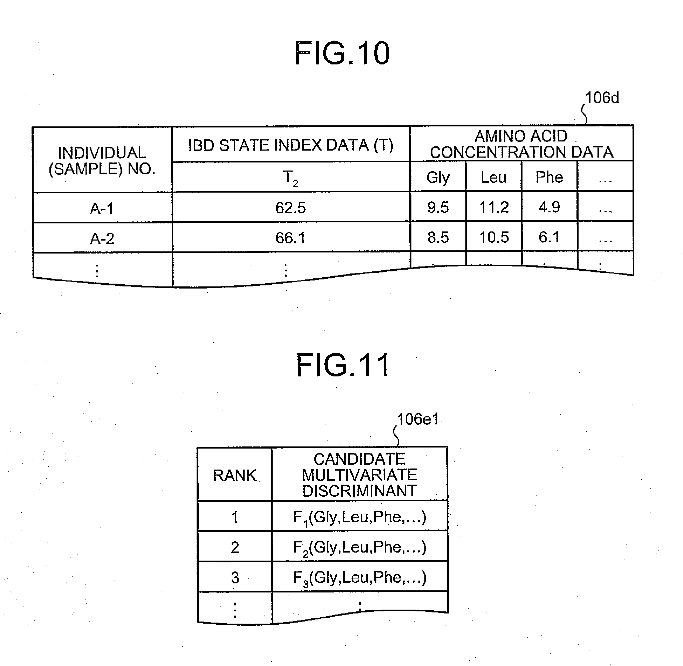

Returning to FIG. 6, the designated IBD state information file 106d stores the IBD state information designated in the IBD state information-designating part 102g described below. FIG. 10 is a chart showing an example of the information stored in the designated IBD state information file 106d. As shown in FIG. 10, the information stored in the designated IBD state information file 106d includes individual number, designated IBD state index data, and designated amino acid concentration data that are correlated to one another.

Returning to FIG. 6, the multivariate discriminant-related information database 106e is composed of a candidate multivariate discriminant file 106e1 storing the candidate multivariate discriminant prepared in the candidate multivariate discriminant-preparing part 102h1 described below; a verification result file 106e2 storing the verification results in the candidate multivariate discriminant-verifying part 102h2 described below; a selected IBD state information file 106e3 storing the IBD state information containing the combination of amino acid concentration data selected in the explanatory variable-selecting part 102h3 described below; and a multivariate discriminant file 106e4 storing the multivariate discriminant prepared in the multivariate discriminant-preparing part 102h described below.

The candidate multivariate discriminant file 106e1 stores the candidate multivariate discriminant prepared in the candidate multivariate discriminant-preparing part 102h1 described below. FIG. 11 is a chart showing an example of the information stored in the candidate multivariate discriminant file 106e1. As shown in FIG. 11, the information stored in the candidate multivariate discriminant file 106e1 includes rank, and candidate multivariate discriminant (e.g., $F_1$ (Gly, Leu, Phe, ...), $F_2$ (Gly, Leu, Phe, ...), or $F_3$ (Gly, Leu, Phe, ...) in FIG. 11) that are correlated to each other.

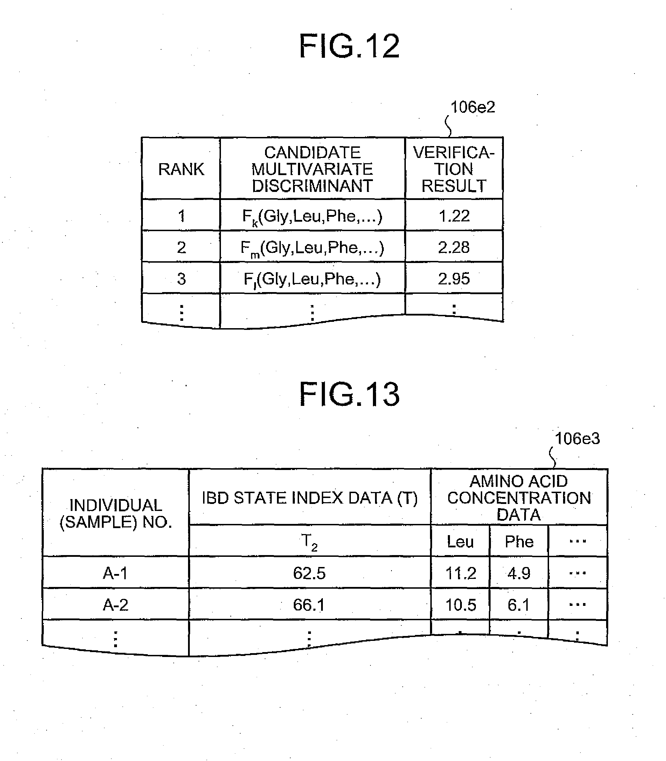

Returning to FIG. 6, the verification result file 106e2 stores the verification results verified in the candidate multivariate discriminant-verifying part 102h2 described below. FIG. 12 is a chart showing an example of the information stored in the verification result file 106e2. As shown in FIG. 12, the information stored in the verification result file 106e2 includes rank, candidate multivariate discriminant (e.g., $F_k$ (Gly, Leu, Phe, ...), $F_m$ (Gly, Leu, Phe, ...), $F_l$ (Gly, Leu, Phe, ...) in FIG. 12), and the verification results of each candidate multivariate discriminant (e.g., evaluation value of each candidate multivariate discriminant) that are correlated to one another.

Returning to FIG. 6, the selected IBD state information file 106e3 stores the IBD state information including the combination of amino acid concentration data corresponding to the explanatory variable selected in the explanatory variable-selecting part 102h3 described below. FIG. 13 is a chart showing an example of the information stored in the selected IBD state information file 106e3.

As shown in FIG. 13, the information stored in the selected IBD state information file 106e3 includes individual number, the IBD state index data designated in the IBD state information-designating part 102g described below, and the amino acid concentration data selected in the explanatory variable-selecting part 102h3 described below that are correlated to one another.

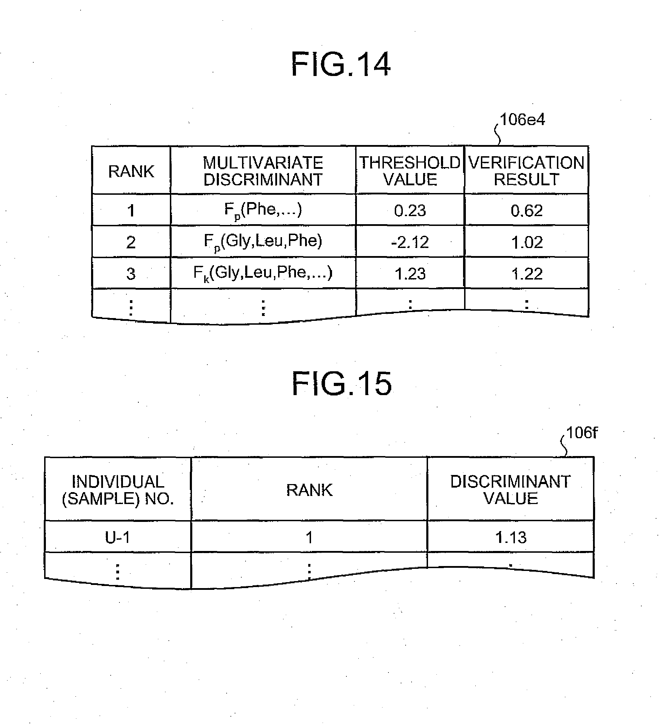

Returning to FIG. 6, the multivariate discriminant file 106e4 stores the multivariate discriminant prepared in the multivariate discriminant-preparing part 102h described below. FIG. 14 is a chart showing an example of the information stored in the multivariate discriminant file 106e4. As shown in FIG. 14, the information stored in the multivariate discriminant file 106e4 includes rank, multivariate discriminant (e.g., $F_p$ (Phe, ...), $F_p$ (Gly, Leu, Phe), $F_k$ (Gly, Leu, Phe, ...) in FIG. 14), a threshold corresponding to each discriminant-preparing method, and verification results of each multivariate discriminant (e.g., evaluation value of each multivariate discriminant) that are correlated to one another.

Returning to FIG. 6, the discriminant value file 106f stores the discriminant value calculated in the discriminant value-calculating part 102i described below. FIG. 15 is a chart showing an example of the information stored in the discriminant value file 106f. As shown in FIG. 15, the information stored in the discriminant value file 106f includes individual number for uniquely identifying an individual (sample) as a subject to be evaluated, rank (number for uniquely identifying the multivariate discriminant), and discriminant value that are correlated to one another.

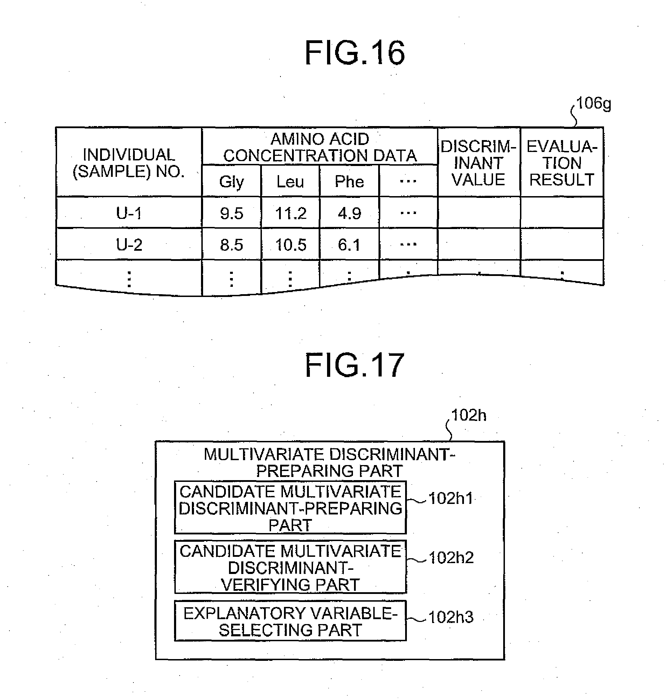

Returning to FIG. 6, the evaluation result file 106g stores the evaluation results obtained in the discriminant value criterion-evaluating part 102j described below (specifically the discrimination results obtained in the discriminant value criterion-discriminating part 102j1, or the evaluation results obtained in the discriminant value criterion-condition evaluating part 102j2). FIG. 16 is a chart showing an example of the information stored in the evaluation result file 106g.

The information stored in the evaluation result file 106g includes individual number for uniquely identifying an individual (sample) as a subject to be evaluated, previously obtained amino acid concentration data on a subject to be evaluated, discriminant value calculated in a multivariate discriminant, and evaluation results on IBD (CD or UC) state (specifically, discrimination results as to discrimination between IBD and IBD-free, discrimination results as to discrimination between CD and CD-free, discrimination results as to discrimination between active phase and remission phase of CD, evaluation results as to evaluation a condition of CD, discrimination results as to discrimination between UC and UC-free, discrimination results as to discrimination between active phase and remission phase of UC, evaluation results as to evaluation a condition of UC, discrimination results as to discrimination between CD and UC, or discrimination results as to discrimination between CD in active phase and UC in active phase) that are correlated to one another.

Returning to FIG. 6, the memory device 106 stores various Web data, CGI programs, and others for providing the client apparatuses 200 with web site information as information other than the information described above.

The Web data include various data for displaying the Web page described below and others, and the data are generated as, for example, a HTML (HyperText Markup Language) or XML (Extensible Markup Language) text file. Other temporary files such as files for the components for generation of Web data and for operation, and others are also stored in the memory device 106. In addition, it may store as needed sound files in the WAVE or AIFF (Audio Interchange File Format) format for transmission to the client apparatuses 200 and image files of still image or motion picture in the JPEG (Joint Photographic Experts Group) or MPEG2 (Moving Picture Experts Group phase 2) format.

The communication interface 104 allows communication between the IBD-evaluating apparatus 100 and the network 300 (or communication apparatus such as router). Thus, the communication interface 104 has a function to communicate data via a communication line with other terminals.

The input/output interface 108 is connected to the input device 112 and the output device 114. A monitor (including home television), a speaker, or a printer may be used as the output device 114 (hereinafter, the output device 114 may be described as monitor 114). A keyboard, a mouse, a microphone, or a monitor functioning as a pointing device together with a mouse may be used as the input device 112.

The control device 102 has an internal memory storing control programs such as OS (Operating System), programs for various processing procedures, and other needed data, and performs information processing according to these programs. As shown in the figure, the control device 102 includes mainly a request-interpreting part 102a, a browsing processing part 102b, an authentication-processing part 102c, an electronic mail-generating part 102d, a Web page-generating part 102e, a receiving part 102f, an IBD state information-designating part 102g, a multivariate discriminant-preparing part 102h, a discriminant value-calculating part 102i, a discriminant value criterion-evaluating part 102j, a result outputting part 102k and a sending part 102m. The control device 102 performs data processing such as removal of data including defective or many outliers and of explanatory variables for the defective value-including data in the IBD state information transmitted from the database apparatus 400 and in the amino acid concentration data transmitted from the client apparatus 200.

The request-interpreting part 102a interprets the request from the client apparatus 200 or the database apparatus 400 and sends the request to other parts in the control device 102 according to the analytical result.

Upon receiving browsing request for various screens from the client apparatus 200, the browsing processing part 102b generates and transmits the web data for these screens. Upon receiving authentication request from the client apparatus 200 or the database apparatus 400, the authentication-processing part 102c performs authentication. The electronic mail-generating part 102d generates an electronic mail including various kinds of information. The Web page-generating part 102e generates a Web page for a user to browse with the client apparatus 200.

The receiving part 102f receives, via the network 300, the information (specifically, the amino acid concentration data, IBD state information, multivariate discriminant etc.) transmitted from the client apparatus 200 and the database apparatus 400. The IBD state information-designating part 102g designates the objective IBD state index data and amino acid concentration data in preparing the multivariate discriminant.

The multivariate discriminant-preparing part 102h generates a multivariate discriminant based on the IBD state information received in the receiving part 102f and the IBD state information designated in the IBD state information-designating part 102g. Specifically, the multivariate discriminant-preparing part 102h generates a multivariate discriminant by selecting a candidate multivariate discriminant to be used as the multivariate discriminant from a plurality of candidate multivariate discriminants, according to the verification results accumulated by repeating the processings in the candidate multivariate discriminant-preparing part 102h1, the candidate multivariate discriminant-verifying part 102h2 and the explanatory variable-selecting part 102h3 from the IBD state information.

If a previously generated multivariate discriminant is stored in a predetermined region of the memory device 106, the multivariate discriminant-preparing part 102h may generate a multivariate discriminant by selecting a desired multivariate discriminant out of the memory device 106. Alternatively, the multivariate discriminant-preparing part 102h may generate the multivariate discriminant by selecting and downloading a desired multivariate discriminant from the multivariate discriminants previously stored in another computer apparatus (e.g., the database apparatus 400).

Hereinafter, the configuration of the multivariate discriminant-preparing part 102h will be described with reference to FIG. 17. FIG. 17 is a block diagram showing the configuration of the multivariate discriminant-preparing part 102h, and only a part in the configuration related to the present invention is shown conceptually. The multivariate discriminant-preparing part 102h has a candidate multivariate discriminant-preparing part 102h1, a candidate multivariate discriminant-verifying part 102h2, and an explanatory variable-selecting part 102h3, additionally. The candidate multivariate discriminant-preparing part 102h1 generates a candidate multivariate discriminant that is a candidate of the multivariate discriminant from the IBD state information according to a predetermined discriminant-preparing method. Specifically, the candidate multivariate discriminant-preparing part 102h1 may generate a plurality of candidate multivariate discriminants from the IBD state information, by using a plurality of different discriminant-preparing methods. The candidate multivariate discriminant-verifying part 102h2 verifies the candidate multivariate discriminants prepared in the candidate multivariate discriminant-preparing part 102h1 according to a particular verification method. Specifically, the candidate multivariate discriminant-verifying part 102h2 may verify at least one of the discrimination rate, sensitivity, specificity, and information criterion of the candidate multivariate discriminants according to at least one of bootstrap method, holdout method, and leave-one-out method. The explanatory variable-selecting part 102h3 selects the combination of the amino acid concentration data contained in the IBD state information to be used in preparing the candidate multivariate discriminant, by selecting an explanatory variable of the candidate multivariate discriminant from the verification results in the candidate multivariate discriminant-verifying part 102h2 according to a particular explanatory variable selection method. The explanatory variable-selecting part 102h3 may select the explanatory variable of the candidate multivariate discriminant from the verification results according to at least one of stepwise method, best path method, local search method, and genetic algorithm.

Returning to FIG. 6, the discriminant value-calculating part 102i calculates a discriminant value that is the value of the multivariate discriminant, based on the amino acid concentration data of the subject to be evaluated received in the receiving part 102f and the multivariate discriminant prepared in the multivariate discriminant-preparing part 102h.

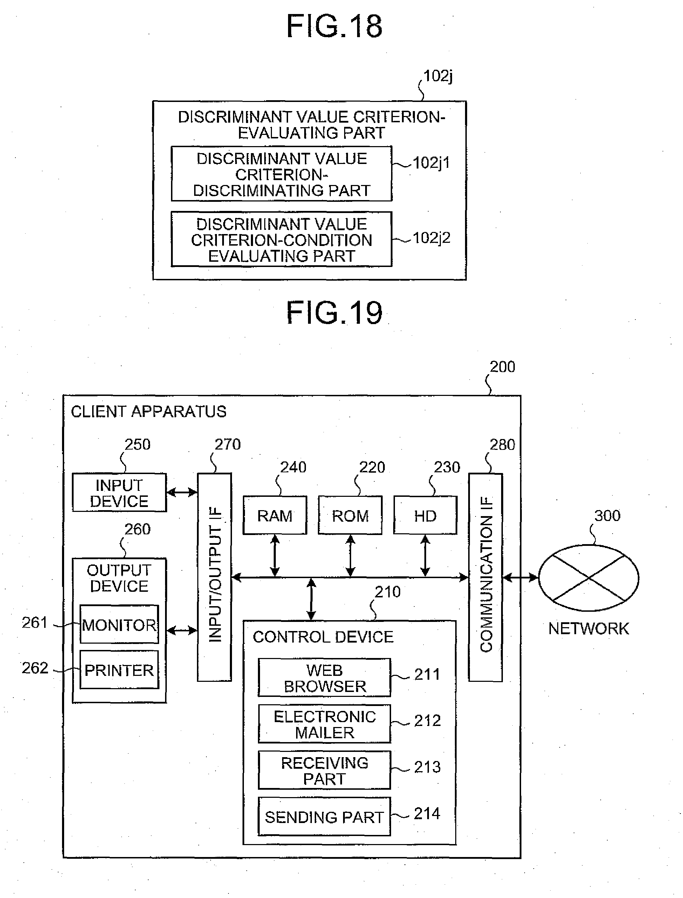

The discriminant value criterion-evaluating part 102j evaluates the IBD state in the subject to be evaluated, based on the discriminant value calculated in the discriminant value-calculating part 102i. The discriminant value criterion-evaluating part 102j further includes a discriminant value criterion-discriminating part 102j1 and a discriminant value criterion-condition evaluating part 102j2. Now, the configuration of the discriminant value criterion-evaluating part 102j will be described with reference to FIG. 18. FIG. 18 is a block diagram showing the configuration of the discriminant value criterion-evaluating part 102j, and only a part in the configuration related to the present invention is shown conceptually. Based on the discriminant value, the discriminant value criterion-discriminating part 102j1 discriminates, for example, between IBD and IBD-free, between CD and CD-free, between active phase and remission phase of CD, between UC and UC-free, between active phase and remission phase of UC, between CD and UC, or between CD in active phase and UC in active phase in the subject to be evaluated. The discriminant value criterion-condition evaluating part 102j2 evaluates, for example, the condition of CD or the condition of UC in the subject to be evaluated, based on the plurality of the discriminant values. Specifically, the discriminant value criterion-condition evaluating part 102j2 compares the discriminant value with a predetermined threshold value (cutoff value) for each of the discriminant values, thereby evaluating, for example, the condition of CD or the condition of UC in the subject to be evaluated.

Returning to FIG. 6, the result outputting part 102k outputs, into the output device 114, the processing results in each processing part in the control device 102 (the evaluation results in the discriminant value criterion-evaluating part 102j (specifically the discrimination results in the discriminant value criterion-discriminating part 102j1 or the evaluation results in the discriminant value criterion-condition evaluating part 102j2)) etc.

The sending part 102m sends the evaluation results to the client apparatus 200 that is the sender of the amino acid concentration data of the subject to be evaluated or sends the multivariate discriminant prepared in the IBD-evaluating apparatus 100, and the evaluation results, to the database apparatus 400.

Hereinafter, the configuration of the client apparatus 200 in the present system will be described with reference to FIG. 19. FIG. 19 is a block diagram showing an example of the configuration of the client apparatus 200 in the present system, and only the part in the configuration relevant to the present invention is shown conceptually.

The client apparatus 200 includes a control device 210, ROM 220, HD (Hard Disk) 230, RAM 240, an input device 250, an output device 260, an input/output IF 270, and a communication IF 280 that are connected communicatively to one another through a communication channel.

The control device 210 has a Web browser 211, an electronic mailer 212, a receiving part 213, and a sending part 214. The Web browser 211 performs browsing processing of interpreting Web data and displaying the interpreted Web data on a monitor 261 described below. The Web browser 211 may have various plug-in software, such as stream player, having functions to receive, display and feedback streaming screen image. The electronic mailer 212 sends and receives electronic mails using a particular protocol (e.g., SMTP (Simple Mail Transfer Protocol) or POP3 (Post Office Protocol version 3)). The receiving part 213 receives various information, such as the evaluation results transmitted from the IBD-evaluating apparatus 100, via the communication IF 280. The sending part 214 sends various information such as the amino acid concentration data on the subject to be evaluated, via the communication IF 280, to the IBD-evaluating apparatus 100.

The input device 250 is for example a keyboard, a mouse or a microphone. The monitor 261 described below also functions as a pointing device together with a mouse. The output device 260 is an output means for outputting the information received via the communication IF 280, and includes the monitor (including home television) 261 and a printer 262. In addition, the output device 260 may have a speaker or the like additionally. The input/output IF 270 is connected to the input device 250 and the output device 260.

The communication IF 280 connects the client apparatus 200 to the network 300 (or communication apparatus such as router) communicatively. In other words, the client apparatuses 200 are connected to the network 300 via a communication apparatus such as modem, TA (Terminal Adapter) or router, and a telephone line, or a private line. In this way, the client apparatuses 200 can access to the IBD-evaluating apparatus 100 by using a particular protocol.

The client apparatus 200 may be realized by installing software (including programs, data and others) for Web data-browsing function and electronic mail-processing function to information processing apparatus (for example, information processing terminal such as known personal computer, workstation, family computer, Internet TV (Television), PHS (Personal Handyphone System) terminal, mobile phone terminal, mobile unit communication terminal or PDA (Personal Digital Assistants)) connected as needed with peripheral devices such as printer, monitor, and image scanner.

All or a part of processings of the control device 210 in the client apparatus 200 may be performed by a CPU and programs read and executed by the CPU. Thus, computer programs for giving instructions to the CPU and executing various processings together with the OS (Operating System) are recorded in the ROM 220 or HD 230. The computer programs, which are executed as they are loaded in the RAM 240, constitute the control device 210 with the CPU. The computer programs may be stored in an application program server connected via any network to the client apparatus 200, and the client apparatus 200 may download all or a part of them as needed. All or any part of processings of the control device 210 may be realized by hardware such as wired-logic.

Hereinafter, the network 300 in the present system will be described with reference to FIGS. 4 and 5. The network 300 has a function to connect the IBD-evaluating apparatus 100, the client apparatuses 200, and the database apparatus 400 mutually, communicatively to one another, and is for example the Internet, intranet, or LAN (Local Area Network (both wired/wireless)). The network 300 may be VAN (Value Added Network), personal computer communication network, public telephone network (including both analog and digital), leased line network (including both analog and digital), CATV (Community Antenna Television) network, portable switched network or portable packet-switched network (including IMT2000 (International Mobile Telecommunication 2000) system, GSM (Global System for Mobile Communications) system, or PDC (Personal Digital Cellular)/PDC-P system), wireless calling network, local wireless network such as Bluetooth (registered trademark), PHS network, satellite communication network (including CS (Communication Satellite), BS (Broadcasting Satellite), and ISDB (Integrated Services Digital Broadcasting)), or the like.

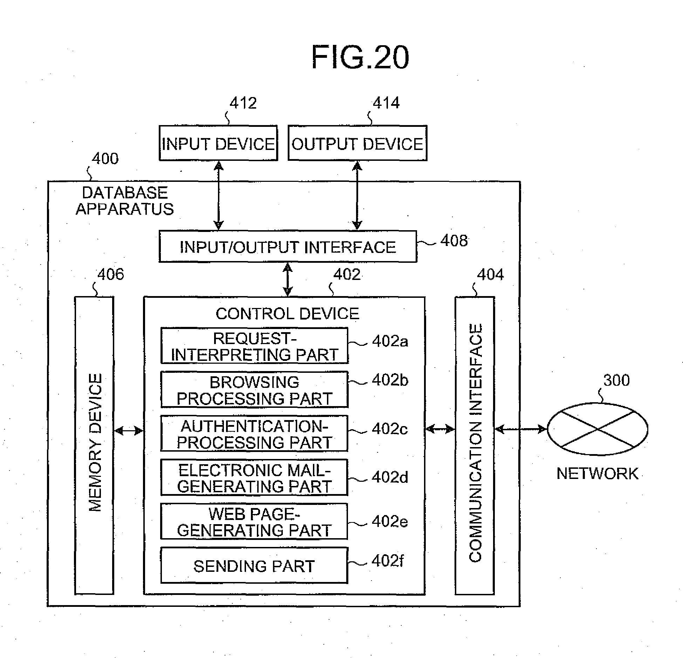

Hereinafter, the configuration of the database apparatus 400 in the present system will be described with reference to FIG. 20. FIG. 20 is a block diagram showing an example of the configuration of the database apparatus 400 in the present system, showing conceptually only the region relevant to the present invention.

The database apparatus 400 has functions to store, for example, the IBD state information used in preparing a multivariate discriminant in the IBD-evaluating apparatus 100 or in the database apparatus 400, the multivariate discriminant prepared in the IBD-evaluating apparatus 100, and the evaluation results in the IBD-evaluating apparatus 100. As shown in FIG. 20, the database apparatus 400 includes a control device 402, such as CPU, which controls the entire database apparatus 400 integrally, a communication interface 404 connecting the database apparatus to the network 300 communicatively via a communication apparatus such as router and via a wired or wireless communication circuit such as private line, a memory device 406 storing various data, tables and files (for example, file for Web page), and an input/output interface 408 connected to an input device 412 and an output device 414, and these parts are connected communicatively to each other via any communication channel.

The memory device 406 is a storage means, and may be, for example, memory apparatus such as RAM or ROM, fixed disk drive such as harddisk, flexible disk, optical disk, or the like. Various programs used in various processings are stored in the memory device 406. The communication interface 404 allows communication between the database apparatus 400 and the network 300 (or communication apparatus such as router). Thus, the communication interface 404 has a function to communicate data with other terminal via a communication line. The input/output interface 408 is connected to the input device 412 and the output device 414. A monitor (including home television), a speaker, or a printer may be used as the output device 414 (hereinafter, the output device 414 may be described as monitor 414). A keyboard, a mouse, a microphone, or a monitor functioning as a pointing device together with a mouse may be used as the input device 412.

The control device 402 has an internal memory storing control programs such as OS (Operating System), programs for various processing procedures, and other needed data, and performs various information processing according to these programs. As shown in the figure, the control device 402 includes mainly a request-interpreting part 402a, a browsing processing part 402b, an authentication-processing part 402c, an electronic mail-generating part 402d, a Web page-generating part 402e, and a sending part 402f.

The request-interpreting part 402a interprets the request from the IBD-evaluating apparatus 100 and sends the request to other parts in the control device 402 according to the analytical result. Upon receiving various screen-browsing request from the IBD-evaluating apparatus 100, the browsing processing part 402b generates and transmits web data for these screens. Upon receipt of authentication request from the IBD-evaluating apparatus 100, the authentication-processing part 402c performs authentication. The electronic mail-generating part 402d generates an electronic mail including various information. The Web page-generating part 402e generates a Web page for a user to browse with the client apparatus 200. The sending part 402f sends the information such as the IBD state information and the multivariate discriminant to the IBD-evaluating apparatus 100.

2-3. Processing in the present system

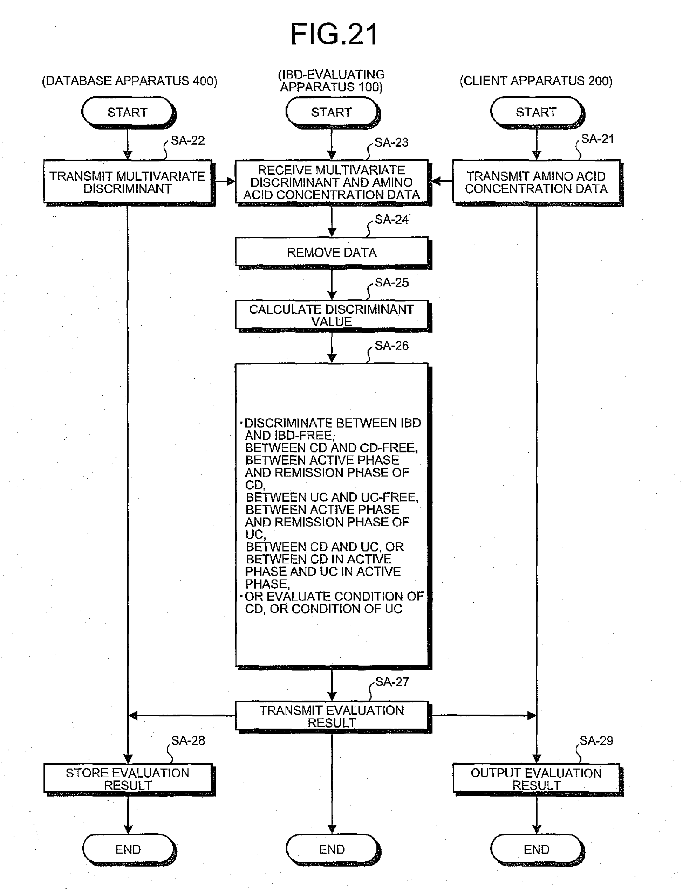

Here, an example of the IBD evaluation service processing performed in the present system constituted as described above will be described with reference to FIG. 21. FIG. 21 is a flowchart showing an example of the IBD evaluation service processing.

The amino acid concentration data used in the present processing concerns amino acid concentration value obtained by analyzing blood previously collected from an individual. Hereinafter, the method of analyzing blood amino acid will be described briefly. First, a blood sample is collected in a heparin-treated tube, and then the blood plasma is separated by centrifugation of the tube. All blood plasma samples separated are frozen and stored at -70°C before measurement of amino acid concentration. Before measurement of amino acid concentration, the blood plasma sample is deproteinized by adding sulfosalicylic acid to a concentration of 3%. An amino acid analyzer by high-performance liquid chromatography (HPLC) by using ninhydrin reaction in the post column was used for measurement of amino acid concentration.

First, the client apparatus 200 accesses the IBD-evaluating apparatus 100 when the user specifies the Web site address (such as URL) provided from the IBD-evaluating apparatus 100, via the input device 250 on the screen displaying Web browser 211. Specifically, when the user instructs update of the Web browser 211 screen on the client apparatus 200, the Web browser 211 sends the Web site's address provided from the IBD-evaluating apparatus 100 by a particular protocol, thereby transmitting a request demanding transmission of the Web page corresponding to the amino acid concentration data transmission screen to the IBD-evaluating apparatus 100 based on the routing of the address.

Then, upon receipt of the request from the client apparatus 200, the request-interpreting part 102a in the IBD-evaluating apparatus 100 analyzes the transmitted request and sends the request to other parts in the control device 102 according to the analytical result. Specifically, when the transmitted request is a request to send the Web page corresponding to the amino acid concentration data transmission screen, mainly the browsing processing part 102b in the IBD-evaluating apparatus 100 obtains the Web data for display of the Web page stored in a predetermined region of the memory device 106 and sends the obtained Web data to the client apparatus 200. More specifically, upon receiving the Web page transmission request corresponding to the amino acid concentration data transmission screen by the user, the control device 102 in the IBD-evaluating apparatus 100 demands input of user ID and user password from the user. If the user ID and password are input, the authentication-processing part 102c in the IBD-evaluating apparatus 100 examines the input user ID and password by comparing them with the user ID and user password stored in the user information file 106a for authentication. Only when the user is authenticated, the browsing processing part 102b in the IBD-evaluating apparatus 100 sends, to the client apparatus 200, the Web data for displaying the Web page corresponding to the amino acid concentration data transmission screen. The client apparatus 200 is identified with the IP (Internet Protocol) address transmitted from the client apparatus 200 together with the transmission request.

Then, the client apparatus 200 receives, in the receiving part 213, the Web data (for displaying the Web page corresponding to the amino acid concentration data transmission screen) transmitted from the IBD-evaluating apparatus 100, interprets the received Web data with the Web browser 211, and displays the amino acid concentration data transmission screen on the monitor 261.

When the user inputs and selects, via the input device 250, for example the amino acid concentration data of the individual on the amino acid concentration data transmission screen displayed on the monitor 261, the sending part 214 of the client apparatus 200 sends an identifier for identifying input information and selected items to the IBD-evaluating apparatus 100, thereby transmitting the amino acid concentration data of the individual as the subject to be evaluated to the IBD-evaluating apparatus 100 (step SA-21). In step SA-21, transmission of the amino acid concentration data may be realized for example by using an existing file transfer technology such as FTP (File Transfer Protocol).

Then, the request-interpreting part 102a of the IBD-evaluating apparatus 100 interprets the identifier transmitted from the client apparatus 200 thereby analyzing the request from the client apparatus 200, and requests the database apparatus 400 to send the multivariate discriminant for IBD evaluation (specifically, for discrimination of the 2 groups of IBD and IBD-free, for discrimination of the 2 groups of CD and CD-free, for discrimination of the 2 groups of active phase and remission phase of CD, for evaluation of the condition of CD, for discrimination of the 2 groups of UC and UC-free, for discrimination of the 2 groups of active phase and remission phase of UC, for evaluation of the condition of UC, for discrimination of the 2 groups of CD and UC, for discrimination of the 2 groups of CD in active phase and UC in active phase, or the like).

Then, the request-interpreting part 402a of the database apparatus 400 interprets the transmission request from the IBD-evaluating apparatus 100 and transmits, to the IBD-evaluating apparatus 100, the multivariate discriminant (for example, the updated newest multivariate discriminant) stored in a predetermined region of the memory device 406 (step SA-22).

When discrimination between IBD and IBD-free is conducted in step SA-26 described below, in step SA-22, the multivariate discriminant transmitted to the IBD-evaluating apparatus 100 may be expressed by one fractional expression or the sum of a plurality of the fractional expressions, and contain the concentration value of at least one of Tau, Glu, Pro and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contain the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau, Glu, Pro and Cys as the explanatory variable in the denominator in the fractional expression constituting the multivariate discriminant. The multivariate discriminant transmitted to the IBD-evaluating apparatus 100 may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Tau, Leu, Tyr, His, Ile and Thr as the explanatory variables.

When discrimination between CD and CD-free is conducted in step SA-26 described below, in step SA-22, the multivariate discriminant transmitted to the IBD-evaluating apparatus 100 may be expressed by one fractional expression or the sum of a plurality of fractional expressions, and contain the concentration value of at least one of Tau, Glu, Pro and Gly as the explanatory variable in the numerator in the fractional expression constituting the multivariate discriminant, and the concentration value of at least one of Urea, Asn, Gln, Thr, ABA, Val, Cys, Leu, Tyr, Phe, His, Trp and Lys as the explanatory variable in the denominator in the fractional expression constituting the multivariate discriminant, or contain the concentration value of at least one of Urea, Asn, Gln, Thr, ABA, Val, Cys, Leu, Tyr, Phe, His, Trp and Lys as the explanatory variable in the numerator in the fractional expression constituting the multivariate discriminant, and the concentration value of at least one of Tau, Glu, Pro and Gly as the explanatory variable in the denominator in the fractional expression constituting the multivariate discriminant. The multivariate discriminant transmitted to the IBD-evaluating apparatus 100 may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Tau, Val, Leu, Tyr, Pro and Ile as the explanatory variables.

When discrimination between active phase and remission phase of CD is conducted in step SA-26 described below, in step SA-22, the multivariate discriminant transmitted to the IBD-evaluating apparatus 100 may be expressed by one fractional expression or the sum of a plurality of fractional expressions and contain the concentration value of at least one of Gln, Cit, Val, Leu, His, Trp, Lys and Arg as the explanatory variable in any one of the numerator and denominator or both in the fractional expression constituting the multivariate discriminant. The multivariate discriminant transmitted to the IBD-evaluating apparatus 100 may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of His, Trp, Lys, Ser, Tau and Ile as the explanatory variables.

When the condition of CD is evaluated in step SA-26 described below, in step SA-22, the multivariate discriminant transmitted to the IBD-evaluating apparatus 100 may be expressed by one fractional expression or the sum of a plurality of fractional expressions and contain the concentration value of at least one of Gln, Cit, Val, Leu, His, Trp, Lys and Arg as the explanatory variable in any one of the numerator and denominator or both in the fractional expression constituting the multivariate discriminant. The multivariate discriminant transmitted to the IBD-evaluating apparatus 100 may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of His, Trp, Lys, Ser, Tau and Ile as the explanatory variables.

When discrimination between UC and UC-free is conducted in step SA-26 described below, in step SA-22, the multivariate discriminant transmitted to the IBD-evaluating apparatus 100 may be expressed by one fractional expression or the sum of a plurality of fractional expressions, and contain the concentration value of at least one of Tau and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contain the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau and Cys as the explanatory variable in the denominator in the fractional expression constituting the multivariate discriminant. The multivariate discriminant transmitted to the IBD-evaluating apparatus 100 may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Tau, Tyr, Leu, His, Ala and Ile as the explanatory variables.

When discrimination between active phase and remission phase of UC is conducted in step SA-26 described below, in step SA-22, the multivariate discriminant transmitted to the IBD-evaluating apparatus 100 may be expressed by one fractional expression or the sum of a plurality of fractional expressions and contain the concentration value of at least one of Tau, Urea, Thr, Asn, Pro, Gln, Ala, Cit, ABA, Val, Met, Leu, Tyr, Phe, His, Trp, Lys and Arg as the explanatory variable in any one of the numerator and denominator or both in the fractional expression constituting the multivariate discriminant. The multivariate discriminant transmitted to the IBD-evaluating apparatus 100 may be any one of a logistic regression equation, a linear discriminant, a multiple regression equation, a discriminant prepared by a support vector machine, a discriminant prepared by a Mahalanobis' distance method, a discriminant prepared by canonical discriminant analysis and a discriminant prepared by a decision tree, and have the concentration value of His, Tyr, Val, Met, Leu and Arg as the explanatory variables.

When the condition of UC is evaluated in step SA-26 described below, in step SA-22, the multivariate discriminant transmitted to the IBD-evaluating apparatus 100 may be expressed by one fractional expression or the sum of a plurality of fractional expressions and contain the concentration value of at least one of Tau, Urea, Thr, Asn, Pro, Gln, Ala, Cit, ABA, Val, Met, Leu, Tyr, Phe, His, Trp, Lys and Arg as the explanatory variable in any one of the numerator and denominator or both in the fractional expression constituting the multivariate discriminant. The multivariate discriminant transmitted to the IBD-evaluating apparatus 100 may be any one of a logistic regression equation, a linear discriminant, a multiple regression equation, a discriminant prepared by a support vector machine, a discriminant prepared by a Mahalanobis' distance method, a discriminant prepared by canonical discriminant analysis and a discriminant prepared by a decision tree, and have the concentration value of His, Tyr, Val, Met, Leu and Arg as the explanatory variables..

When discrimination between CD and UC is conducted in step SA-26 described below, in step SA-22, the multivariate discriminant transmitted to the IBD-evaluating apparatus 100 may be expressed by one fractional expression or the sum of a plurality of fractional expressions, and contain the concentration value of at least one of Cys and ABA as the explanatory variable in the numerator in the fractional expression constituting the multivariate discriminant and the concentration value of at least one of Thr, Pro, Gly, Met, Ile and Orn as the explanatory variable in the denominator in the fractional expression constituting the multivariate discriminant, or contain the concentration value of at least one of Thr, Pro, Gly, Met, Ile and Orn as the explanatory variable in the numerator in the fractional expression constituting the multivariate discriminant and the concentration value of at least one of Cys and ABA as the explanatory variable in the denominator in the fractional expression constituting the multivariate discriminant. The multivariate discriminant transmitted to the IBD-evaluating apparatus 100 may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Met, Pro, Ile, Trp, Tau and Val as the explanatory variables.

When discrimination between CD in active phase and UC in active phase is conducted in step SA-26 described below, in step SA-22, the multivariate discriminant transmitted to the IBD-evaluating apparatus 100 may be expressed by one fractional expression or the sum of a plurality of fractional expressions, and contain the concentration value of at least one of Tau, Thr, Ser, Gly, Met and Ile as the explanatory variable in any one of the numerator and denominator or both in the fractional expression constituting the multivariate discriminant. The multivariate discriminant transmitted to the IBD-evaluating apparatus 100 may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Met, Tau, Trp, Tyr, Orn and Phe as the explanatory variables.

Returning to FIG.21, the IBD-evaluating apparatus 100 receives, in the receiving part 102f, the amino acid concentration data of the individual transmitted from the client apparatuses 200 and the multivariate discriminant transmitted from the database apparatus 400, and stores the received amino acid concentration data in a predetermined memory region of the amino acid concentration data file 106b and the received multivariate discriminant in a predetermined memory region of the multivariate discriminant file 106e4 (step SA-23).

In the control device 102 of the IBD-evaluating apparatus 100, data such as defective and outliers are then removed from the amino acid concentration data of the individual received in step SA-23 (step SA-24).

Then, the IBD-evaluating apparatus 100 calculates the discriminant value in the discriminant value-calculating part 102i, based on the multivariate discriminant received in step SA-23 and the amino acid concentration data of the individual from which defective and outliers have been removed in step SA-24 (step SA-25). Then, the discriminant value criterion-discriminating part 102j1 or the discriminant value criterion-condition evaluating part 102j2 compares the discriminant value calculated in step SA-25 with a previously established threshold (cutoff value), thereby conducting either the discrimination between 2 groups or the evaluation of the condition of disease that will be described in the following 21. to 29, and the discrimination results or the evaluation results are stored in a predetermined memory region of the evaluation result file 106g (step SA-26).

21. Discrimination between IBD and IBD-free

A discriminant value that is a value of multivariate discriminant is calculated based on both the concentration value of at least one of Tau, Urea, Asn, Glu, Gln, Pro, Ala, Cit, Gly, ABA, Val, Cys, Leu, Tyr, Phe, His, Trp, Orn and Lys contained in the amino acid concentration data of the individual from which data such as defective and outliers have been removed and the multivariate discriminant containing the concentration value of at least one of Tau, Urea, Asn, Glu, Gln, Pro, Ala, Cit, Gly, ABA, Val, Cys, Leu, Tyr, Phe, His, Trp, Orn and Lys as the explanatory variable, and the calculated discriminant value is compared with a previously established threshold (cutoff value), thereby discriminating between IBD and IBD-free in the individual.

22. Discrimination between CD and CD-free

A discriminant value that is a value of multivariate discriminant is calculated based on both the concentration value of at least one of Tau, Thr, Urea, Asn, Glu, Gln, Pro, Gly, ABA, Val, Cys, Leu, Tyr, Phe, His, Trp and Lys contained in the amino acid concentration data of the individual from which data such as defective and outliers have been removed and the multivariate discriminant containing the concentration value of at least one of Tau, Thr, Urea, Asn, Glu, Gln, Pro, Gly, ABA, Val, Cys, Leu, Tyr, Phe, His, Trp and Lys as the explanatory variable, and the calculated discriminant value is compared with a previously established threshold (cutoff value), thereby discriminating between CD and CD-free in the individual.

23. Discrimination between active phase and remission phase of CD

A discriminant value that is a value of multivariate discriminant is calculated based on both the concentration value of at least one of Gln, Cit, Val, Leu, His, Trp, Lys and Arg contained in the amino acid concentration data of the individual from which data such as defective and outliers have been removed and the multivariate discriminant containing the concentration value of at least one of Gln, Cit, Val, Leu, His, Trp, Lys and Arg as the explanatory variable, and the calculated discriminant value is compared with a previously established threshold (cutoff value), thereby discriminating between active phase and remission phase of CD in the individual.

24. Evaluation of condition of CD

When a plurality of the amino acid concentration data of individual transmitted from the client apparatus 200 is received, a discriminant value that is a value of multivariate discriminant is calculated based on both the concentration value of at least one of Gln, Cit, Val, Leu, His, Trp, Lys and Arg contained in the plurality of the amino acid concentration data of the individual from which data such as defective and outliers have been removed and the multivariate discriminant containing the concentration value of at least one of Gln, Cit, Val, Leu, His, Trp, Lys and Arg as the explanatory variable, and the plurality of the calculated discriminant values are compared with a previously established threshold (cutoff value) for each of the plurality of the discriminant values, thereby evaluating a condition of CD in the individual.

25. Discrimination between UC and UC-free

A discriminant value that is a value of multivariate discriminant is calculated based on both the concentration value of at least one of Tau, Urea, Asn, Gln, Ala, Cit, ABA, Val, Cys, Met, Ile, Leu, Tyr, Phe, His, Trp, Orn and Lys contained in the amino acid concentration data of the individual from which data such as defective and outliers have been removed and the multivariate discriminant containing the concentration value of at least one of Tau, Urea, Asn, Gln, Ala, Cit, ABA, Val, Cys, Met, Ile, Leu, Tyr, Phe, His, Trp, Orn and Lys as the explanatory variable, and the calculated discriminant value is compared with a previously established threshold (cutoff value), thereby discriminating between UC and UC-free in the individual.

26. Discrimination between active phase and remission phase of UC

A discriminant value that is a value of multivariate discriminant is calculated based on both the concentration value of at least one of Tau, Urea, Thr, Asn, Pro, Gln, Ala, Cit, ABA, Val, Met, Leu, Tyr, Phe, His, Trp, Lys and Arg contained in the amino acid concentration data of the individual from which data such as defective and outliers have been removed and the multivariate discriminant containing the concentration value of at least one of Tau, Urea, Thr, Asn, Pro, Gln, Ala, Cit, ABA, Val, Met, Leu, Tyr, Phe, His, Trp, Lys and Arg as the explanatory variable, and the calculated discriminant value is compared with a previously established threshold (cutoff value), thereby discriminating between active phase and remission phase of UC in the individual.

27. Evaluation of condition of UC

When a plurality of the amino acid concentration data of individual transmitted from the client apparatus 200 is received, a discriminant value that is a value of multivariate discriminant is calculated based on both the concentration value of at least one of Tau, Urea, Thr, Asn, Pro, Gln, Ala, Cit, ABA, Val, Met, Leu, Tyr, Phe, His, Trp, Lys and Arg contained in the plurality of the amino acid concentration data of the individual from which data such as defective and outliers have been removed and the multivariate discriminant containing the concentration value of at least one of Tau, Urea, Thr, Asn, Pro, Gln, Ala, Cit, ABA, Val, Met, Leu, Tyr, Phe, His, Trp, Lys and Arg as the explanatory variable, and the plurality of the calculated discriminant values are compared with a previously established threshold (cutoff value) for each of the plurality of the discriminant values, thereby evaluating a condition of UC in the individual.

28. Discrimination between CD and UC

A discriminant value that is a value of multivariate discriminant is calculated based on both the concentration value of at least one of Cys, ABA, Thr, Pro, Gly, Met, Ile and Orn contained in the amino acid concentration data of the individual from which data such as defective and outliers have been removed and the multivariate discriminant containing the concentration value of at least one of Cys, ABA, Thr, Pro, Gly, Met, Ile and Orn as the explanatory variable, and the calculated discriminant value is compared with a previously established threshold (cutoff value), thereby discriminating between CD and UC in the individual.

29. Discrimination between CD in active phase and UC in active phase

A discriminant value that is a value of multivariate discriminant is calculated based on both the concentration value of at least one of Tau, Thr, Ser, Gly, Met and Ile contained in the amino acid concentration data of the individual from which data such as defective and outliers have been removed and the multivariate discriminant containing the concentration value of at least one of Tau, Thr, Ser, Gly, Met and Ile as the explanatory variable, and the calculated discriminant value is compared with a previously established threshold (cutoff value), thereby discriminating between CD in active phase and UC in active phase in the individual.

Returning to FIG.21, the sending part 102m of the IBD-evaluating apparatus 100 then sends the discrimination results (specifically, discrimination results as to discrimination between IBD and IBD-free, discrimination results as to discrimination between CD and CD-free, discrimination results as to discrimination between active phase and remission phase of CD, evaluation results as to evaluation of the condition of CD, discrimination results as to discrimination between UC and UC-free, discrimination results as to discrimination between active phase and remission phase of UC, evaluation results as to evaluation of the condition of UC, discrimination results as to discrimination between CD and UC, discrimination results as to discrimination between CD in active phase and UC in active phase, or the like) obtained in step SA-26 to the client apparatus 200 that has sent the amino acid concentration data and to the database apparatus 400 (step SA-27). Specifically, the IBD-evaluating apparatus 100 first generates a Web page for display of discrimination results in the Web page-generating part 102e and stores the Web data corresponding to the generated Web page, in a predetermined memory region of the memory device 106. Then, the user is authenticated as described above by inputting a predetermined URL (Uniform Resource Locator) into the Web browser 211 of the client apparatus 200 via the input device 250, and the client apparatus 200 sends a Web page browsing request to the IBD-evaluating apparatus 100. The IBD-evaluating apparatus 100 then examines the browsing request transmitted from the client apparatus 200 in the browsing processing part 102b and reads the Web data corresponding to the Web page for displaying the discrimination results, out of the predetermined memory region of the memory device 106. The sending part 102m of the IBD-evaluating apparatus 100 then sends the read-out Web data to the client apparatus 200 and simultaneously sends the Web data or the discrimination results to the database apparatus 400.

In step SA-27, the control device 102 of the IBD-evaluating apparatus 100 may notify the discrimination results to the user client apparatus 200 by electronic mail. Specifically, the IBD-evaluating apparatus 100 first acquires the user electronic mail address in the electronic mail-generating part 102d at the transmission timing for example based on the user ID, with reference to the user information stored in the user information file 106a. The IBD-evaluating apparatus 100 then generates electronic mail data including user name and discrimination result, with the electronic mail address obtained as its mail address in the electronic mail-generating part 102d. The sending part 102m of the IBD-evaluating apparatus 100 then sends the generated data to the user client apparatus 200.

Also in step SA-27, the IBD-evaluating apparatus 100 may send the discrimination results to the user client apparatus 200 by using an existing file transfer technology such as FTP.

Returning to FIG. 21, the control device 402 in the database apparatus 400 receives the discrimination results or the Web data transmitted from the IBD-evaluating apparatus 100 and stores (accumulates) the received discrimination results or Web data in a predetermined memory region of the memory device 406 (step SA-28).

The receiving part 213 of the client apparatus 200 receives the Web data transmitted from the IBD-evaluating apparatus 100, and the received Web data are interpreted with the Web browser 211, to display on the monitor 261 the Web page screen displaying the discrimination result of the individual (step SA-29). When the discrimination results are sent from the IBD-evaluating apparatus 100 by electronic mail, the electronic mail transmitted from the IBD-evaluating apparatus 100 is received at any timing, and the received electronic mail is displayed on the monitor 261 with the known function of the electronic mailer 212 of the client apparatus 200.

In this way, the user knows, by browsing the Web page displayed on the monitor 261, for example, the discrimination results as to the discrimination of the 2 groups of IBD and IBD-free in the individual, the discrimination results as to the discrimination of the 2 groups of CD and CD-free in the individual, the discrimination results as to the discrimination of the 2 groups of active phase and remission phase of CD in the individual, the evaluation results as to the evaluation of the condition of CD in the individual, the discrimination results as to the discrimination of the 2 groups of UC and UC-free in the individual, the discrimination results as to the discrimination of the 2 groups of active phase and remission phase of UC in the individual, the evaluation results as to the evaluation of the condition of UC in the individual, the discrimination results as to the discrimination of the 2 groups of CD and UC in the individual, or the discrimination results as to the discrimination of the 2 groups of CD in active phase and UC in active phase in the individual. The user can print out the content of the Web page displayed on the monitor 261 by the printer 262.

When the discrimination results are transmitted by electronic mail from the IBD-evaluating apparatus 100, the user reads the electronic mail displayed on the monitor 261, whereby the user can confirm the discrimination results as to the discrimination of the 2 groups of IBD and IBD-free in the individual, the discrimination results as to the discrimination of the 2 groups of CD and CD-free in the individual, the discrimination results as to the discrimination of the 2 groups of active phase and remission phase of CD in the individual, the evaluation results as to the evaluation of the condition of CD in the individual, the discrimination results as to the discrimination of the 2 groups of UC and UC-free in the individual, the discrimination results as to the discrimination of the 2 groups of active phase and remission phase of UC in the individual, the evaluation results as to the evaluation of the condition of UC in the individual, the discrimination results as to the discrimination of the 2 groups of CD and UC in the individual, or the discrimination results as to the discrimination of the 2 groups of CD in active phase and UC in active phase in the individual. The user may print out the content of the electronic mail displayed on the monitor 261 by the printer 262.

Given the foregoing description, the explanation of the IBD evaluation service processing is finished.

2-4. Summary of the second embodiment and other embodiments

According to the IBD-evaluating system described above in detail, the client apparatus 200 sends the amino acid concentration data of the individual to the IBD-evaluating apparatus 100, and upon receiving a request from the IBD-evaluating apparatus 100, the database apparatus 400 transmits the multivariate discriminant (for example, any one of the multivariate discriminant for discrimination of the 2 groups of IBD and IBD-free, the multivariate discriminant for discrimination of the 2 groups of CD and CD-free, the multivariate discriminant for discrimination of the 2 groups of active phase and remission phase of CD, the multivariate discriminant for evaluation of the condition of CD, the multivariate discriminant for discrimination of the 2 groups of UC and UC-free, the multivariate discriminant for discrimination of the 2 groups of active phase and remission phase of UC, the multivariate discriminant for evaluation of the condition of UC, the multivariate discriminant for discrimination of the 2 groups of CD and UC, and the multivariate discriminant for discrimination of the 2 groups of CD in active phase and UC in active phase) to the IBD-evaluating apparatus 100. By the IBD-evaluating apparatus 100, the amino acid concentration data are received from the client apparatus 200, and simultaneously the multivariate discriminant is received from the database apparatus 400, the discriminant value is calculated based on the received amino acid concentration data and the received multivariate discriminant, the calculated discriminant value is compared with the previously established threshold, thereby conducting either the discrimination between 2 groups or the evaluation of the condition of disease as described in 21. to 29. above, and this discrimination result or evaluation result is transmitted to the client apparatus 200 and database apparatus 400. Then, the client apparatus 200 receives and displays the discrimination result or evaluation result transmitted from the IBD-evaluating apparatus 100, and the database apparatus 400 receives and stores the discrimination result or evaluation result transmitted from the IBD-evaluating apparatus 100. Thus, a discriminant value obtained in a multivariate discriminant useful for discriminating between the 2 groups of IBD and IBD-free can be utilized to bring about an effect of enabling accurate discrimination between the 2 groups of IBD and IBD-free. A discriminant value obtained in a multivariate discriminant useful for discriminating between the 2 groups of CD and CD-free can be utilized to bring about an effect of enabling accurate discrimination between the 2 groups of CD and CD-free. A discriminant value obtained in a multivariate discriminant useful for discriminating between the 2 groups of active phase and remission phase of CD can be utilized to bring about an effect of enabling accurate discrimination between the 2 groups of active phase and remission phase of CD. A discriminant value obtained in a multivariate discriminant useful for evaluating a condition of CD can be utilized to bring about an effect of enabling accurate evaluation of a condition of CD. A discriminant value obtained in a multivariate discriminant useful for discriminating between the 2 groups of UC and UC-free can be utilized to bring about an effect of enabling accurate discrimination between the 2 groups of UC and UC-free. A discriminant value obtained in a multivariate discriminant useful for discriminating between the 2 groups of active phase and remission phase of UC can be utilized to bring about an effect of enabling accurate discrimination between the 2 groups of active phase and remission phase of UC. A discriminant value obtained in a multivariate discriminant useful for evaluating a condition of UC can be utilized to bring about an effect of enabling accurate evaluation of a condition of UC. A discriminant value obtained in a multivariate discriminant useful for discriminating between the 2 groups of CD and UC can be utilized to bring about an effect of enabling accurate discrimination between the 2 groups of CD and UC. A discriminant value obtained in a multivariate discriminant useful for discriminating between the 2 groups of CD in active phase and UC in active phase can be utilized to bring about an effect of enabling accurate discrimination between the 2 groups of CD in active phase and UC in active phase.

According to the IBD-evaluating system, when the discrimination between 2 groups as described in 21. above is conducted in step SA-26, the multivariate discriminant may be expressed by one fractional expression or the sum of a plurality of the fractional expressions, and contain the concentration value of at least one of Tau, Glu, Pro and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contain the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau, Glu, Pro and Cys as the explanatory variable in the denominator in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Tau, Leu, Tyr, His, Ile and Thr as the explanatory variables. Thus, a discriminant value obtained in a multivariate discriminant useful particularly for discriminating between the 2 groups of IBD and IBD-free can be utilized to bring about an effect of enabling more accurate discrimination between the 2 groups of IBD and IBD-free.

According to the IBD-evaluating system, when the discrimination between 2 groups as described in 22. above is conducted in step SA-26, the multivariate discriminant may be expressed by one fractional expression or the sum of a plurality of fractional expressions, and contain the concentration value of at least one of Tau, Glu, Pro and Gly as the explanatory variable in the numerator in the fractional expression constituting the multivariate discriminant, and the concentration value of at least one of Urea, Asn, Gln, Thr, ABA, Val, Cys, Leu, Tyr, Phe, His, Trp and Lys as the explanatory variable in the denominator in the fractional expression constituting the multivariate discriminant, or contain the concentration value of at least one of Urea, Asn, Gln, Thr, ABA, Val, Cys, Leu, Tyr, Phe, His, Trp and Lys as the explanatory variable in the numerator in the fractional expression constituting the multivariate discriminant, and the concentration value of at least one of Tau, Glu, Pro and Gly as the explanatory variable in the denominator in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Tau, Val, Leu, Tyr, Pro and Ile as the explanatory variables. Thus, a discriminant value obtained in a multivariate discriminant useful particularly for discriminating between the 2 groups of CD and CD-free can be utilized to bring about an effect of enabling more accurate discrimination between the 2 groups of CD and CD-free.

According to the IBD-evaluating system, when the discrimination between 2 groups as described in 23. above or the evaluation of the condition of disease as described in 24. above is conducted in step SA-26, the multivariate discriminant may be expressed by one fractional expression or the sum of a plurality of fractional expressions and contain the concentration value of at least one of Gln, Cit, Val, Leu, His, Trp, Lys and Arg as the explanatory variable in any one of the numerator and denominator or both in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of His, Trp, Lys, Ser, Tau and Ile as the explanatory variables. Thus, a discriminant value obtained in a multivariate discriminant useful particularly for discriminating between the 2 groups of active phase and remission phase of CD can be utilized to bring about an effect of enabling more accurate discrimination between the 2 groups of active phase and remission phase of CD. A discriminant value obtained in a multivariate discriminant useful particularly for evaluating a condition of CD can be utilized to bring about an effect of enabling more accurate evaluation of a condition of CD.

According to the IBD-evaluating system, when the discrimination between 2 groups as described in 25. above is conducted in step SA-26, the multivariate discriminant may be expressed by one fractional expression or the sum of a plurality of fractional expressions, and contain the concentration value of at least one of Tau and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contain the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau and Cys as the explanatory variable in the denominator in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Tau, Tyr, Leu, His, Ala and Ile as the explanatory variables. Thus, a discriminant value obtained in a multivariate discriminant useful particularly for discriminating between the 2 groups of UC and UC-free can be utilized to bring about an effect of enabling more accurate discrimination between the 2 groups of UC and UC-free.

According to the IBD-evaluating system, when the discrimination between 2 groups as described in 26. above or the evaluation of the condition of disease as described in 27. above is conducted in step SA-26, the multivariate discriminant may be expressed by one fractional expression or the sum of a plurality of fractional expressions and contain the concentration value of at least one of Tau, Urea, Thr, Asn, Pro, Gln, Ala, Cit, ABA, Val, Met, Leu, Tyr, Phe, His, Trp, Lys and Arg as the explanatory variable in any one of the numerator and denominator or both in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' distance method, a discriminant prepared by canonical discriminant analysis and a discriminant prepared by a decision tree, and have the concentration value of His, Tyr, Val, Met, Leu and Arg as the explanatory variables. Thus, a discriminant value obtained in a multivariate discriminant useful particularly for discriminating between the 2 groups of active phase and remission phase of UC can be utilized to bring about an effect of enabling more accurate discrimination between the 2 groups of active phase and remission phase of UC. A discriminant value obtained in a multivariate discriminant useful particularly for evaluating a condition of UC can be utilized to bring about an effect of enabling more accurate evaluation of a condition of UC.

According to the IBD-evaluating system, when the discrimination between 2 groups as described in 28. above is conducted in step SA-26, the multivariate discriminant may be expressed by one fractional expression or the sum of a plurality of fractional expressions, and contain the concentration value of at least one of Cys and ABA as the explanatory variable in the numerator in the fractional expression constituting the multivariate discriminant and the concentration value of at least one of Thr, Pro, Gly, Met, Ile and Orn as the explanatory variable in the denominator in the fractional expression constituting the multivariate discriminant, or contain the concentration value of at least one of Thr, Pro, Gly, Met, Ile and Orn as the explanatory variable in the numerator in the fractional expression constituting the multivariate discriminant and the concentration value of at least one of Cys and ABA as the explanatory variable in the denominator in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Met, Pro, Ile, Trp, Tau and Val as the explanatory variables. Thus, a discriminant value obtained in a multivariate discriminant useful particularly for discriminating between the 2 groups of CD and UC can be utilized to bring about an effect of enabling more accurate discrimination between the 2 groups of CD and UC.

According to the IBD-evaluating system, when the discrimination between 2 groups as described in 29. above is conducted in step SA-26, the multivariate discriminant may be expressed by one fractional expression or the sum of a plurality of fractional expressions, and contain the concentration value of at least one of Tau, Thr, Ser, Gly, Met and Ile as the explanatory variable in any one of the numerator and denominator or both in the fractional expression constituting the multivariate discriminant. The multivariate discriminant may be any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and have the concentration value of Met, Tau, Trp, Tyr, Orn and Phe as the explanatory variables. Thus, a discriminant value obtained in a multivariate discriminant useful particularly for discriminating between the 2 groups of CD in active phase and UC in active phase can be utilized to bring about an effect of enabling more accurate discrimination between the 2 groups of CD in active phase and UC in active phase.

The multivariate discriminants described above can be prepared by a method described in International Publication WO 2004/052191 that is an international application filed by the present applicant or by a method (multivariate discriminant-preparing processing described later) described in International Publication WO 2006/098192 that is an international application filed by the present applicant. Any multivariate discriminants obtained by these methods can be preferably used in evaluation of an IBD state, regardless of the unit of amino acid concentration in the amino acid concentration data as input data.

In addition to the second embodiment described above, the present invention can be practiced in various different embodiments within the technological scope of the claims. For example, among the processings described in the second embodiment above, all or a part of the processings described above as performed automatically may be performed manually, and all or a part of the manually conducted processings may be performed automatically by known methods. In addition, the processing procedure, control procedure, specific name, various registered data, information including parameters such as retrieval condition, screen, and database configuration shown in the description above or drawings may be modified arbitrarily, unless specified otherwise. For example, the components of the IBD-evaluating apparatus 100 shown in the figures are conceptual and functional and may not be the same physically as those shown in the figure. In addition, all or a part of the operational function of each component and each device in the IBD-evaluating apparatus 100 (in particular, processings in the control device 102) may be executed by the CPU (Central Processing Unit) or the programs executed by the CPU, and may be realized as wired-logic hardware.

The "program" is a data processing method written in any language or by any description method and may be of any format such as source code or binary code. The "program" may not be configured singly, and may be operated together with plurality of modules and libraries or with a different program such as OS (Operating System) to achieve the function. The program is stored on a recording medium and read mechanically as needed by the IBD-evaluating apparatus 100. Any well-known configuration or procedure may be used for reading the programs recorded on the recording medium in each apparatus and for reading procedure and installation of the procedure after reading.

The "recording media" includes any "portable physical media", "fixed physical media", and "communication media". Examples of the "portable physical media" include flexible disk, magnetic optical disk, ROM, EPROM (Erasable Programmable Read Only Memory), EEPROM (Electronically Erasable and Programmable Read Only Memory), CD-ROM (Compact Disk Read Only Memory), MO (Magneto-Optical disk), DVD (Digital Versatile Disk), and the like. Examples of the "fixed physical media" include various media installed in a computer system such as ROM, RAM, and HD. The "communication media" for example stores the program for a short period of time such as communication line and carrier wave when the program is transmitted via a network such as LAN (Local Area Network), WAN (Wide Area Network), or the Internet.

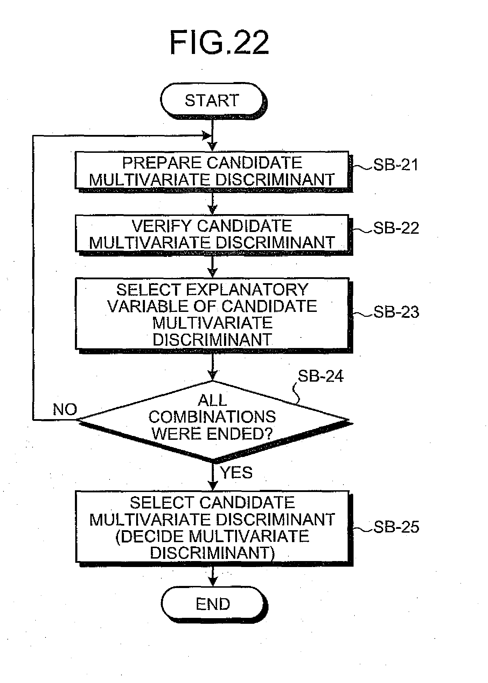

Finally, an example of the multivariate discriminant-preparing processing performed in the IBD-evaluating apparatus 100 is described in detail with reference to FIG. 22. FIG. 22 is a flowchart showing an example of the multivariate discriminant-preparing processing. The multivariate discriminant-preparing processing may be performed in the database apparatus 400 handling the IBD state information.

In the present description, the IBD-evaluating apparatus 100 stores the IBD state information previously obtained from the database apparatus 400 in a predetermined memory region of the IBD state information file 106c. The IBD-evaluating apparatus 100 shall store, in a predetermined memory region of the designated IBD state information file 106d, the IBD state information including the IBD state index data and amino acid concentration data designated previously in the IBD state information-designating part 102g.

According to a predetermined discriminant-preparing method, the candidate multivariate discriminant-preparing part 102h1 in the multivariate discriminant-preparing part 102h first prepares a candidate multivariate discriminant from the IBD state information stored in a predetermine memory region of the designated IBD state information file 106d, and the prepared candidate multivariate discriminate is stored in a predetermined memory region of the candidate multivariate discriminant file 106e1 (step SB-21). Specifically, the candidate multivariate discriminant-preparing part 102h1 in the multivariate discriminant-preparing part 102h first selects a desired method out of a plurality of different discriminant-preparing methods (including multivariate analysis methods such as principal component analysis, discriminant analysis, support vector machine, multiple regression analysis, logistic regression analysis, k-means method, cluster analysis, and decision tree and the like) and determines the form of the candidate multivariate discriminant to be prepared based on the selected discriminant-preparing method. The candidate multivariate discriminant-preparing part 102h1 in the multivariate discriminant-preparing part 102h then performs various calculation corresponding to the selected function-selecting method (e.g., average or variance), based on the IBD state information. The candidate multivariate discriminant-preparing part 102h1 in the multivariate discriminant-preparing part 102h then determines the parameters for the calculation result and the determined candidate multivariate discriminant. In this way, a candidate multivariate discriminant is generated based on the selected discriminant-preparing method. When candidate multivariate discriminants are generated simultaneously and concurrently (in parallel) by using a plurality of different discriminant-preparing methods in combination, the processings described above may be executed concurrently for each selected discriminant-preparing method. Alternatively when candidate multivariate discriminants are to be generated in series by using a plurality of different discriminant-preparing methods in combination, for example, candidate multivariate discriminants may be generated by converting IBD state information with a candidate multivariate discriminant prepared by performing principal component analysis and performing discriminant analysis of the converted IBD state information.

The candidate multivariate discriminant-verifying part 102h2 in the multivariate discriminant-preparing part 102h verifies (mutually verifies) the candidate multivariate discriminant prepared in step SB-21 according to a particular verification method and stores the verification result in a predetermined memory region of the verification result file 106e2 (step SB-22). Specifically, the candidate multivariate discriminant-verifying part 102h2 in the multivariate discriminant-preparing part 102h first generates the verification data to be used in verification of the candidate multivariate discriminant, based on the IBD state information stored in a predetermined memory region of the designated IBD state information file 106d, and verifies the candidate multivariate discriminant according to the generated verification data. If a plurality of candidate multivariate discriminants are generated by using a plurality of different discriminant-preparing methods in step SB-21, the candidate multivariate discriminant-verifying part 102h2 in the multivariate discriminant-preparing part 102h verifies each candidate multivariate discriminant corresponding to each discriminant-preparing method according to a particular verification method. Here in step SB-22, at least one of the discrimination rate, sensitivity, specificity, information criterion, and the like of the candidate multivariate discriminant may be verified based on at least one method of the bootstrap, holdout, leave-one-out, and other methods. Thus, it is possible to select a candidate multivariate discriminant higher in predictability or reliability, based on the IBD state information and diagnostic condition.

Then, the explanatory variable-selecting part 102h3 in the multivariate discriminant-preparing part 102h selects the combination of amino acid concentration data contained in the IBD state information to be used in preparing the candidate multivariate discriminant by selecting an explanatory variable of the candidate multivariate discriminant from the verification results in step SB-22 according to a particular explanatory variable selection method, and stores the IBD state information including the selected combination of amino acid concentration data in a predetermined memory region of the selected IBD state information file 106e3 (step SB-23). When a plurality of candidate multivariate discriminants are generated by using a plurality of different discriminant-preparing methods in step SB-21 and each candidate multivariate discriminant corresponding to each discriminant-preparing method is verified according to a particular verification method in step SB-22, the explanatory variable-selecting part 102h3 in the multivariate discriminant-preparing part 102h selects the explanatory variable of the candidate multivariate discriminant for each candidate multivariate discriminant corresponding to the verification result obtained in step SB-22, according to a particular explanatory variable selection method in step SB-23. Here in step SB-23, the explanatory variable of the candidate multivariate discriminant may be selected from the verification results according to at least one of stepwise method, best path method, local search method, and genetic algorithm. The best path method is a method of selecting an explanatory variable by optimizing the evaluation index of the candidate multivariate discriminant while eliminating the explanatory variables contained in the candidate multivariate discriminant one by one. In step SB-23, the explanatory variable-selecting part 102h3 in the multivariate discriminant-preparing part 102h may select the combination of amino acid concentration data based on the IBD state information stored in a predetermined memory region of the designated IBD state information file 106d.

The multivariate discriminant-preparing part 102h then judges whether all combinations of the amino acid concentration data contained in the IBD state information stored in a predetermined memory region of the designated IBD state information file 106d are processed, and if the judgment result is "End" (Yes in step SB-24), the processing advances to the next step (step SB-25), and if the judgment result is not "End" (No in step SB-24), it returns to step SB-21. The multivariate discriminant-preparing part 102h judges whether the processing is performed a predetermined number of times, and if the judgment result is "End" (Yes in step SB-24), the processing may advance to the next step (step SB-25), and if the judgment result is not "End" (No in step SB-24), it returns to step SB-21. The multivariate discriminant-preparing part 102h may judge whether the combination of the amino acid concentration data selected in step SB-23 is the same as the combination of the amino acid concentration data contained in the IBD state information stored in a predetermined memory region of the designated IBD state information file 106d or the combination of the amino acid concentration data selected in the previous step SB-23, and if the judgment result is "the same" (Yes in step SB-24), the processing may advance to the next step (step SB-25) and if the judgment result is not "the same" (No in step SB-24), it may return to step SB-21. If the verification result is specifically the evaluation value for each multivariate discriminant, the multivariate discriminant-preparing part 102h may advance to step SB-25 or return to step SB-21, based on the comparison of the evaluation value with a particular threshold corresponding to each discriminant-preparing method.

Then, the multivariate discriminant-preparing part 102h determines the multivariate discriminant based on the verification results by selecting a candidate multivariate discriminant to be used as the multivariate discriminant among the candidate multivariate discriminants, and stores the determined multivariate discriminant (selected candidate multivariate discriminant) in particular memory region of the multivariate discriminant file 106e4 (step SB-25). Here, in step SB-25, for example, the optimal multivariate discriminant may be selected from the candidate multivariate discriminants prepared by the same discriminant-preparing method or from all candidate multivariate discriminants.

These are description of the multivariate discriminant-preparing processing.

Example 1

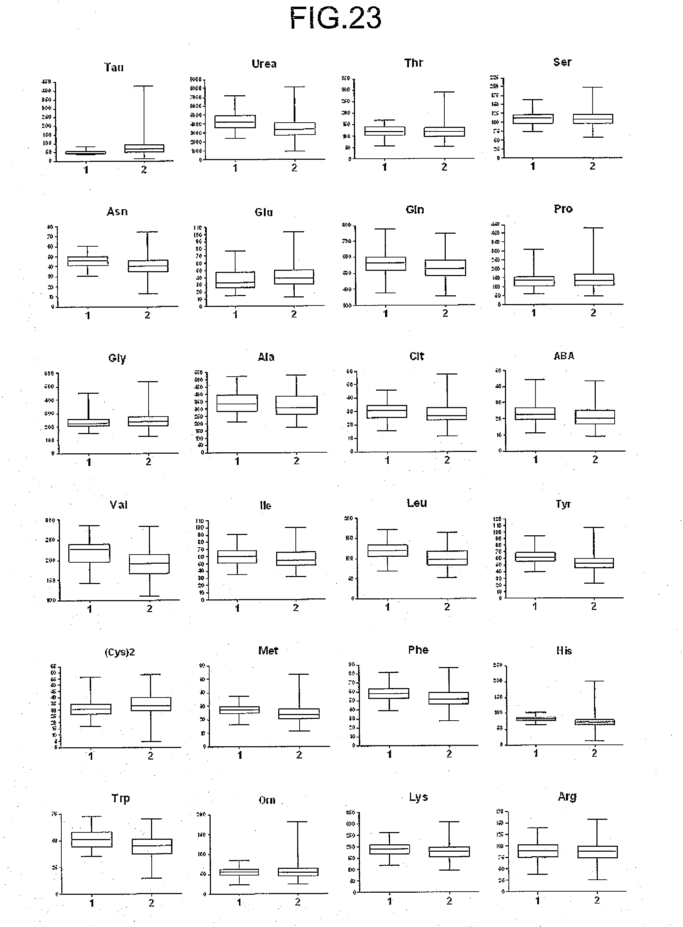

Blood samples of a group of 95 healthy subjects, and blood samples of a group of 190 inflammatory bowel disease patients determined as having inflammatory bowel disease, were subjected to measurement of the amino acid concentration in blood by the amino acid analysis method. FIG. 23 is a boxplot showing the distributions of amino acid explanatory variables in the respective groups (1: healthy subject group, 2: inflammatory bowel disease group). In FIG. 23, the vertical axis indicates amino acid concentration, with the unit of the concentration being nmol/ml, and ABA and Cys in the figure represent $\alpha$-ABA ($\alpha$-aminobutyric acid) and Cystine, respectively.

A t-test between 2 groups was performed for the purpose of discriminating between the 2 groups of the healthy subject group and the inflammatory bowel disease group. In the inflammatory bowel disease group as compared with the healthy subject group, Tau, Glu and Cys significantly increased (probability of significant difference $P<0.05$), and Urea, Asn, Gln, Ala, ABA, Val, Met, Ile, Leu, Tyr, Phe, His, Trp and Lys significantly decreased (probability of significant difference $P<0.05$). Thus, it was made clear that the amino acid explanatory variables Tau, Glu, Cys, Urea, Asn, Gln, Ala, ABA, Val, Met, Ile, Leu, Tyr, Phe, His, Trp and Lys have an ability to discriminate between the 2 groups of the healthy subject group and the inflammatory bowel disease group.

Example 2

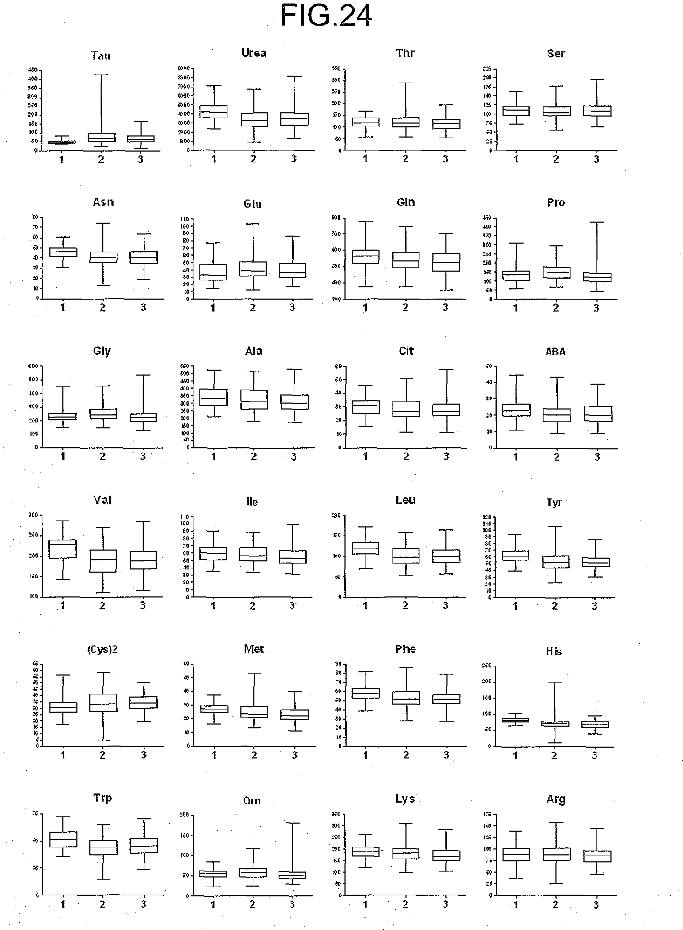

Based on the blood samples used in Example 1, the inflammatory bowel disease group of Example 1 was further divided into a group of 95 Crohn disease patients determined as having Crohn disease and a group of 95 ulcerative colitis patients determined as having ulcerative colitis. FIG. 24 is a boxplot showing the distributions of amino acid explanatory variables in the respective groups (1: healthy subject group, 2: Crohn disease group, 3: ulcerative colitis group). In FIG. 24, the vertical axis indicates amino acid concentration, with the unit of the concentration being nmol/ml, and ABA and Cys in the figure represent $\alpha$-ABA ($\alpha$-aminobutyric acid) and Cystine, respectively.

First, a t-test between 2 groups was performed for the purpose of discriminating between the 2 groups of the healthy subject group and the Crohn disease group. In the Crohn disease group as compared with the healthy subject group, Tau, Glu, Pro and Gly significantly increased (probability of significant difference $P<0.05$), and Urea, Asn, Gln, ABA, Val, Cys, Leu, Tyr, Phe, His, Trp and Lys significantly decreased (probability of significant difference $P<0.05$). Thus, it was made clear that the amino acid explanatory variables Tau, Urea, Asn, Glu, Gln, Pro, Gly, ABA, Val, Cys, Leu, Tyr, Phe, His, Trp and Lys have an ability to discriminate between the 2 groups of the healthy subject group and the Crohn disease group.

Subsequently, a t-test between 2 groups was performed for the purpose of discriminating between the 2 groups of the healthy subject group and the ulcerative colitis group. In the ulcerative colitis group as compared with the healthy subject group, Urea, Asn, Gln, Ala, Cit, Val, Met, Ile, Leu, Tyr, Phe, His, Trp and Lys significantly decreased (probability of significant difference $P<0.05$). Thus, it was made clear that the amino acid explanatory variables Urea, Asn, Gln, Ala, Cit, Val, Met, Ile, Leu, Tyr, Phe, His, Trp and Lys have an ability to discriminate between the 2 groups of the healthy subject group and the ulcerative colitis group.

Subsequently, a t-test between 2 groups was performed for the purpose of discriminating between the 2 groups of the Crohn disease group and the ulcerative colitis group. In the ulcerative colitis group as compared with the Crohn disease group, Pro, Gly, Met and Orn significantly decreased (probability of significant difference $P<0.05$). Thus, it was made clear that the amino acid explanatory variables Pro, Gly, Met and Orn have an ability to discriminate between the 2 groups of the Crohn disease group and the ulcerative colitis group.

Example 3

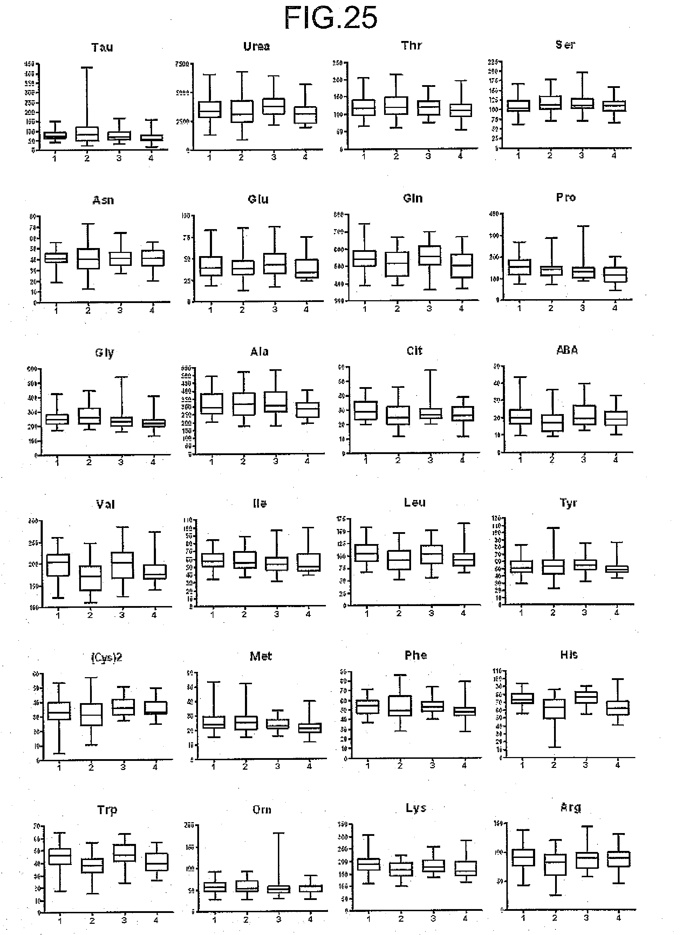

Based on the blood samples used in Example 1, the Crohn disease group of Example 2 was further divided into a group of 50 Crohn disease patients in the remission phase and a group of 26 Crohn disease patients in the active phase, and the ulcerative colitis group of Example 2 was further divided into a group of 46 ulcerative colitis patients in the remission phase and a group of 24 ulcerative colitis patients in the active phase. FIG. 25 is a boxplot showing the distributions of amino acid explanatory variables in the respective groups (1: remission phase Crohn disease group, 2:

active phase Crohn disease group, 3: remission phase ulcerative colitis group, 4: active phase ulcerative colitis group). In FIG. 25, the vertical axis indicates amino acid concentration, with the unit of the concentration being nmol/ml, and ABA and Cys in the figure represent α-ABA (α-aminobutyric acid) and Cystine, respectively.

First, a t-test between 2 groups was performed for the purpose of discriminating between the 2 groups of the active phase Crohn disease group and the active phase ulcerative colitis group. In the active phase ulcerative colitis group as compared with the active phase Crohn disease group, Gly and Met significantly decreased (probability of significant difference P<0.05). Thus, it was made clear that the amino acid explanatory variables Gly and Met have an ability to discriminate between the 2 groups of the active phase Crohn disease group and the active phase ulcerative colitis group.

Subsequently, a t-test between 2 groups was performed for the purpose of discriminating between the 2 groups of the active phase Crohn disease group and the remission phase Crohn disease group. In the active phase Crohn disease group as compared with the remission phase Crohn disease group, Cit, Val, Leu, His, Trp, Lys and Arg significantly decreased (probability of significant difference P<0.05). Thus, it was made clear that the amino acid explanatory variables Cit, Val, Leu, His, Trp, Lys and Arg have an ability to discriminate between the 2 groups of the active phase Crohn disease group and the remission phase Crohn disease group.

Subsequently, a t-test between 2 groups was performed for the purpose of discriminating between the 2 groups of the active phase ulcerative colitis group and the remission phase ulcerative colitis group. In the active phase ulcerative colitis group as compared with the remission phase ulcerative colitis group, Urea, Gln, Ala, Val, His and Trp significantly decreased (probability of significant difference P<0.05). Thus, it was made clear that the amino acid explanatory variables Urea, Gln, Ala, Val, His and Trp have an ability to discriminate between the 2 groups of the active phase ulcerative colitis group and the remission phase ulcerative colitis group.

Example 4

The sample data used in Example 1 were used. Using a method described in International Publication WO 2004/052191 that is an international application filed by the present applicant, first, an index by which the performance of discriminating between the 2 groups of the healthy subject group and the inflammatory bowel disease group is maximized was eagerly searched, and an index formula 11 was obtained among a plurality of indices having an equivalent performance.

```
Index formula 11: (Orn)/(Val)+(Tau+Cys)/(Trp+His)
```

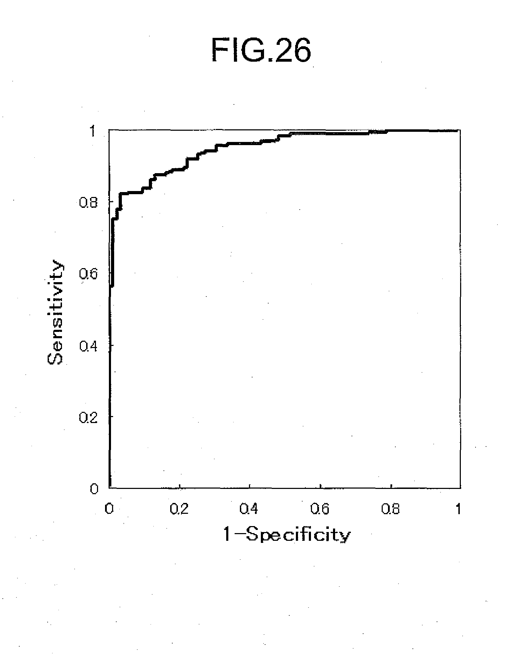

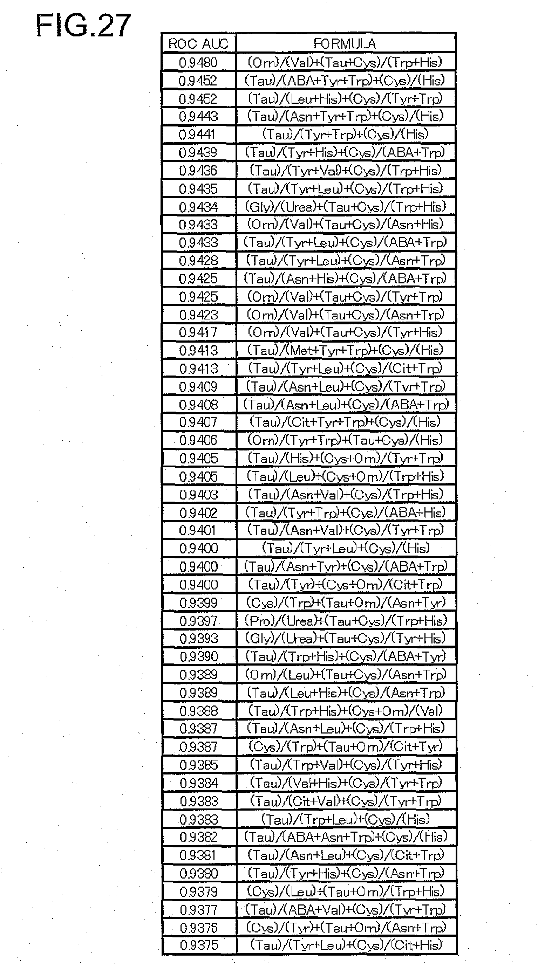

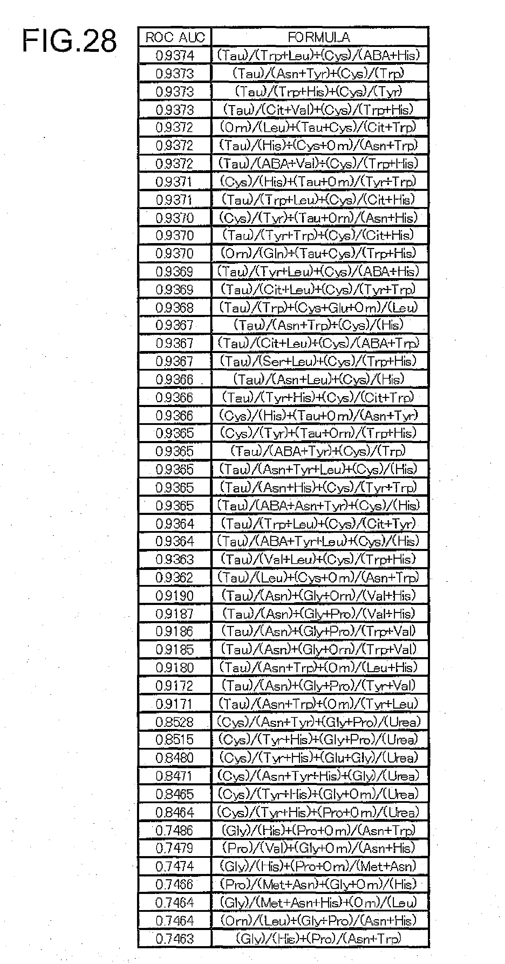

An evaluation was performed on the discrimination between the 2 groups of the healthy subject group and the inflammatory bowel disease group based on the index formula 11, on the basis of the AUC (area under the curve) of the ROC (receiver operating characteristic) curve (FIG. 26), and an AUC of $0.948\pm0.012$ (95% confidence interval: 0.924 to 0.972) was obtained. When the optimum cutoff value for the discrimination between the 2 groups of the healthy subject group and the inflammatory bowel disease group by the index formula 11 was determined, the cutoff value was 1.75, and a sensitivity of 82%, a specificity of 96%, and a correct diagnostic rate of 87% were obtained. Thus, the index formula 11 was found to be a useful index with high diagnostic performance. In addition to that, a plurality of fractional expressions having a discrimination performance equivalent to that of the index formula 11 was obtained. Those fractional expressions are presented in FIG. 27 and FIG. 28.

Subsequently, an index by which the performance of discriminating between the 2 groups of the healthy subject group and the Crohn disease group is maximized was eagerly searched, and an index formula 12 was obtained among a plurality of indices having an equivalent performance.

```
Index formula 12: (Orn)/(Val)+(Tau+Cys)/(His+Trp)
```

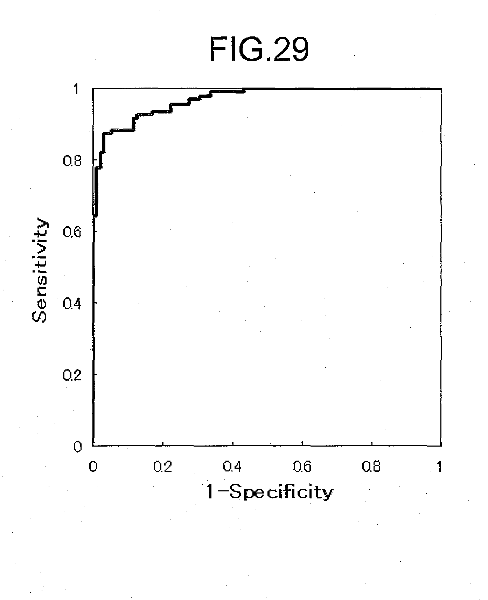

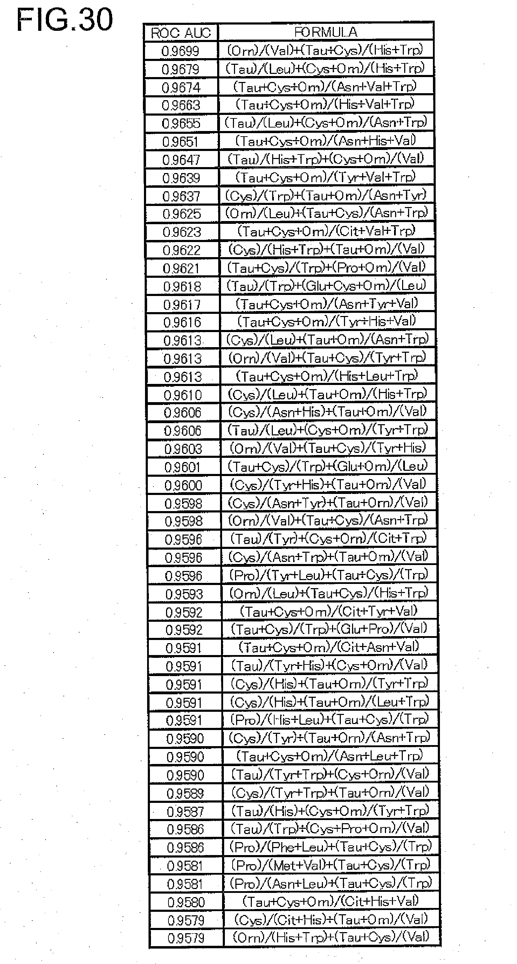

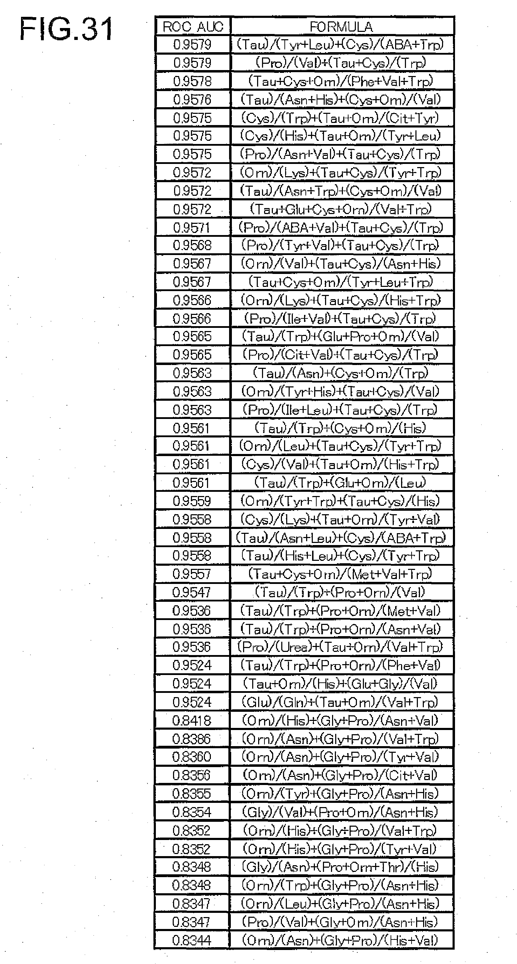

An evaluation was performed on the discrimination between the 2 groups of the healthy subject group and the Crohn disease group based on the index formula 12, on the basis of the AUC of the ROC curve (FIG. 29), and an AUC of $0.970\pm0.010$ (95% confidence interval: 0.950 to 0.989) was obtained. When the optimum cutoff value for the discrimination between the 2 groups of the healthy subject group and the Crohn disease group by the index formula 12 was determined, the cutoff value was 1.02, and a sensitivity of 87%, a specificity of 97%, and a correct diagnostic rate of 92% were obtained. Thus, the index formula 12 was found to be a useful index with high diagnostic performance. In addition to that, a plurality of fractional expressions having a discrimination performance equivalent to that of the index formula 12 was obtained. Those fractional expressions are presented in FIG. 30 and FIG. 31.

Subsequently, an index by which the performance of discriminating between the 2 groups of the healthy subject group and the ulcerative colitis group is maximized was eagerly searched, and an index formula 13 was obtained among a plurality of indices having an equivalent performance.

```
Index formula 13: (Tau)/(ABA+Asn+Tyr)+(Cys)/(His)
```

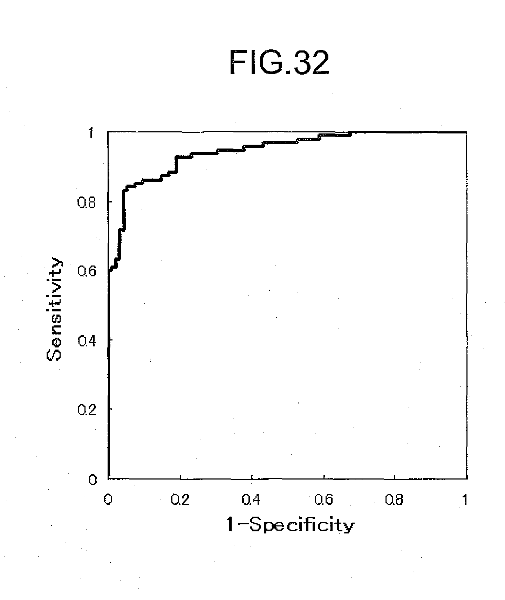

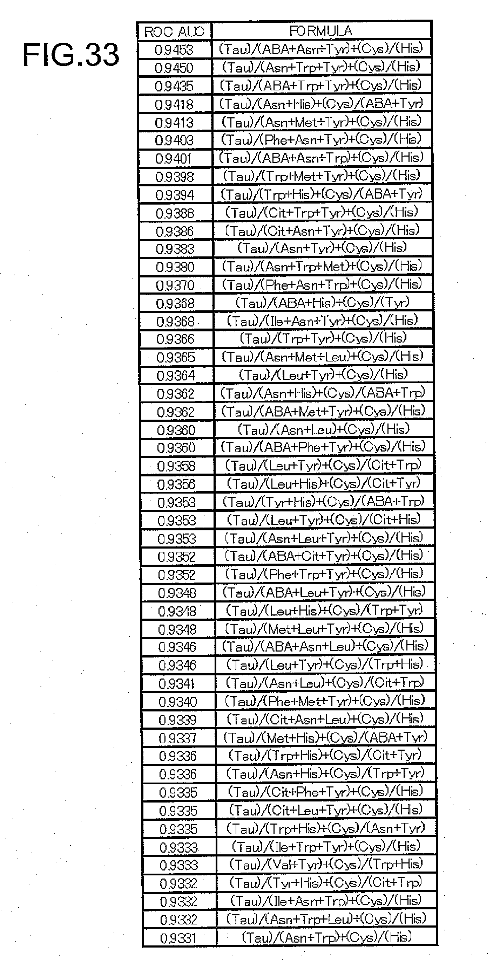

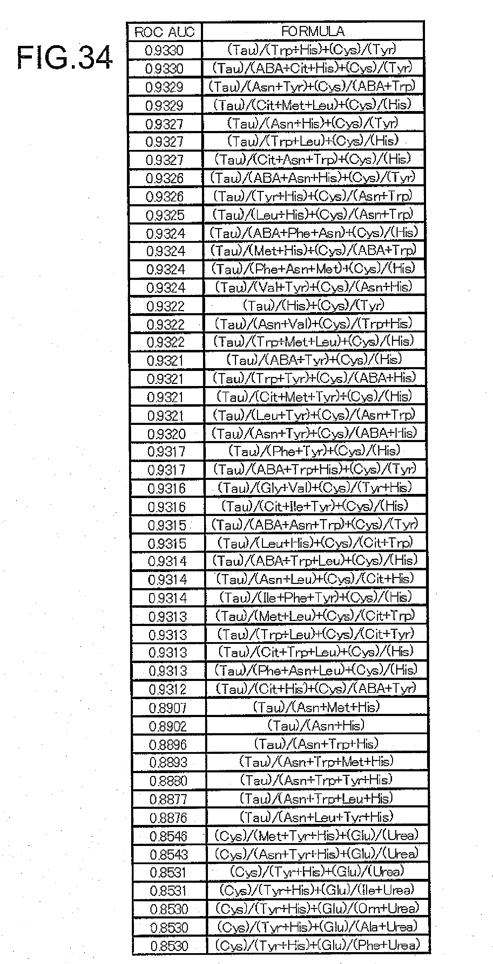

An evaluation was performed on the discrimination between the 2 groups of the healthy subject group and the ulcerative colitis group based on the index formula 13, on the basis of the AUC of the ROC curve (FIG. 32), and an AUC of $0.945\pm0.015$ (95% confidence interval: 0.915 to 0.976) was obtained. When the optimum cutoff value for the discrimination between the 2 groups of the healthy subject group and the ulcerative colitis group by the index formula 13 was determined, the cutoff value was 0.794, and a sensitivity of 83%, a specificity of 96%, and a correct diagnostic rate of 89% were obtained. Thus, the index formula 13 was found to be a useful index with high diagnostic performance. In addition to that, a plurality of fractional expressions having a discrimination performance equivalent to that of the index formula 13 was obtained. Those fractional expressions are presented in FIG. 33 and FIG. 34.

Subsequently, an index by which the performance of discriminating between the 2 groups of the Crohn disease group and the ulcerative colitis group is maximized was eagerly searched, and an index formula 14 was obtained among a plurality of indices having an equivalent performance.

```
Index formula 14: (Tau)/(Ser)+(Pro+Orn)/(Cys+Trp)
```

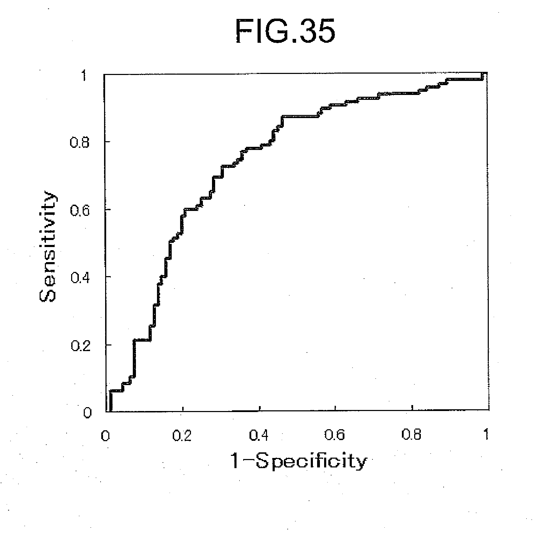

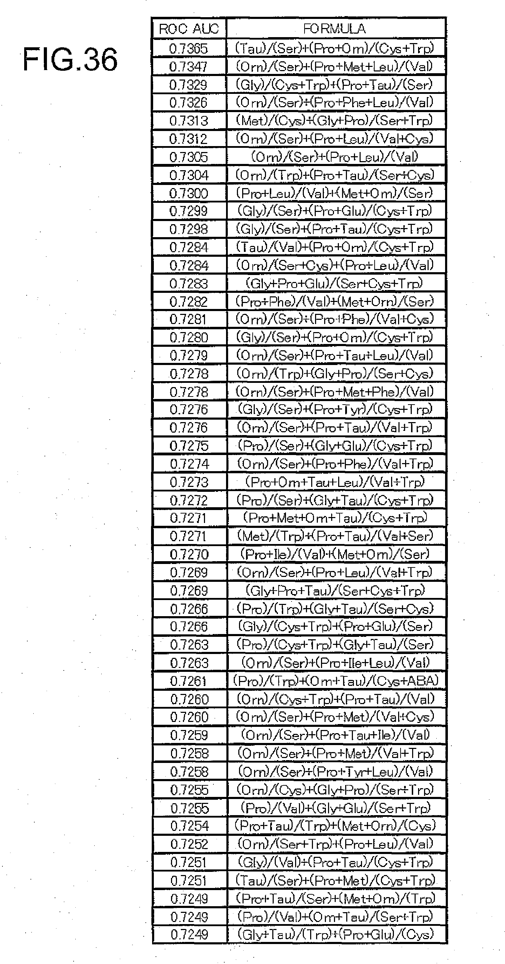

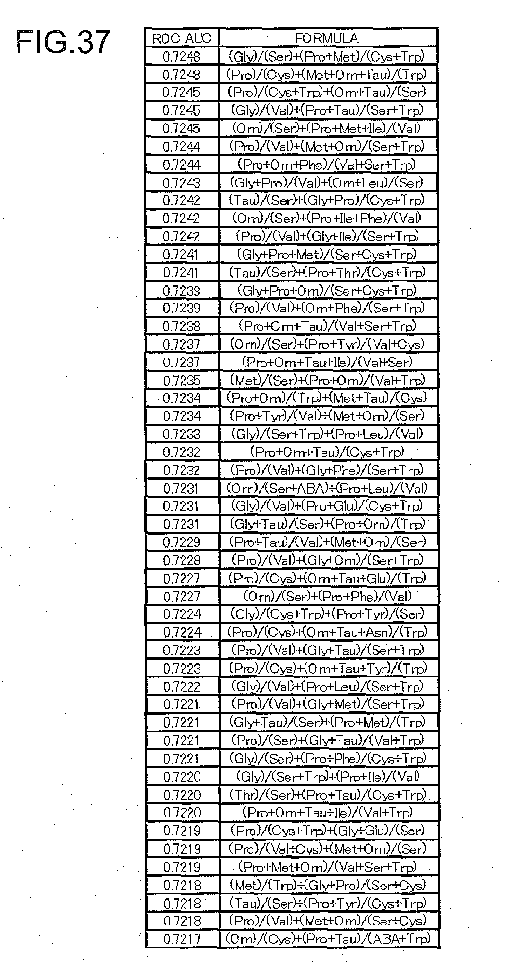

An evaluation was performed on the discrimination between the 2 groups of the Crohn disease group and the ulcerative colitis group based on the index formula 14, on the basis of the AUC of the ROC curve (FIG. 35), and an AUC of $0.737\pm0.037$ (95% confidence interval: 0.664 to 0.809) was obtained. When the optimum cutoff value for the discrimination between the 2 groups of the Crohn disease group and the ulcerative colitis group by the index formula 14 was determined, the cutoff value was 3.171, and a sensitivity of 73%, a specificity of 69%, and a correct diagnostic rate of 71% were obtained. Thus, the index formula 14 was found to be a useful index with high diagnostic performance. In addition to that, a plurality of fractional expressions having a discrimination performance equivalent to that of the index formula 14 was obtained. Those fractional expressions are presented in FIG. 36 and FIG. 37.

Subsequently, an index by which the performance of discriminating between the 2 groups of the active phase Crohn disease group and the active phase ulcerative colitis group is maximized was eagerly searched, and an index formula 15 was obtained among a plurality of indices having an equivalent performance.

```
Index formula 15: (Met+Orn)/(Trp)+(Tau+Ser)/(Arg)
```

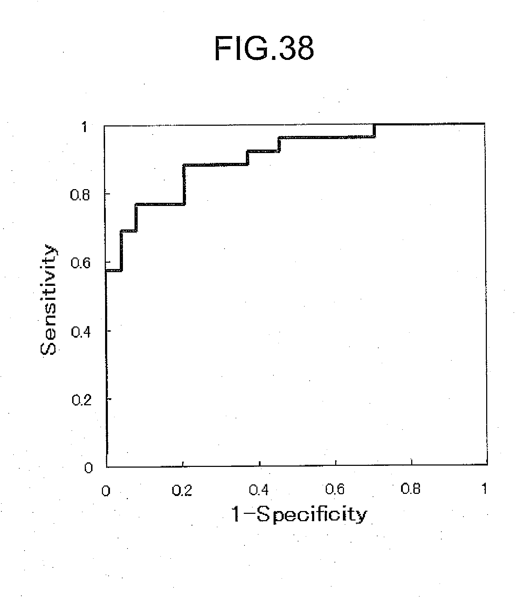

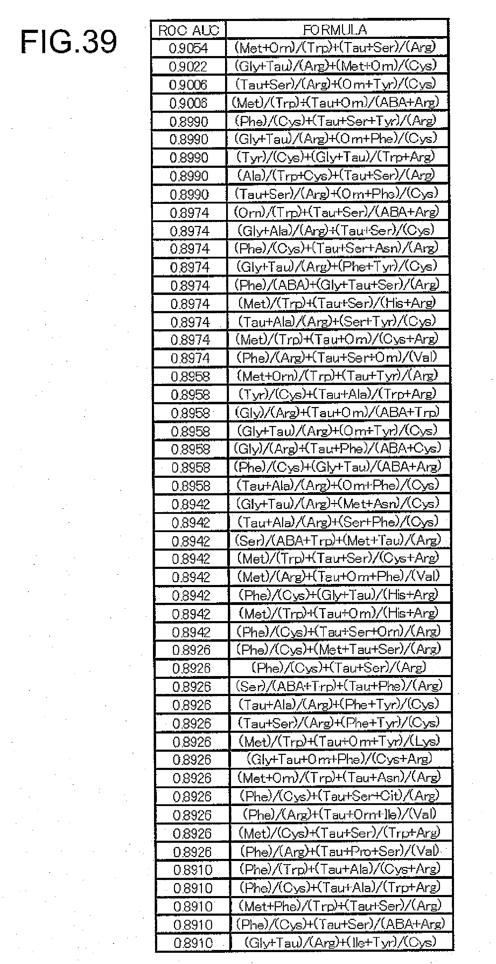

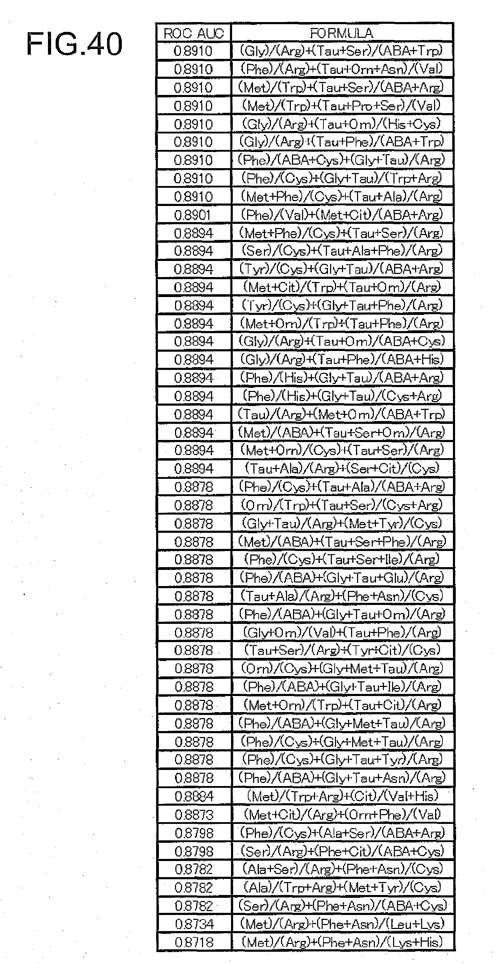

An evaluation was performed on the discrimination between the 2 groups of the active phase Crohn disease group and the active phase ulcerative colitis group based on the index formula 15, on the basis of the AUC of the ROC curve (FIG. 38), and an AUC of $0.905\pm0.041$ (95% confidence interval: 0.824 to 0.987) was obtained. When the optimum cutoff value for the discrimination between the 2 groups of the active phase Crohn disease group and the active phase ulcerative colitis group by the index formula 15 was determined, the cutoff value was 4.669, and a sensitivity of 92%, a specificity of 77%, and a correct diagnostic rate of 84% were obtained. Thus, the index formula 15 was found to be a useful index with high diagnostic performance. In addition to that, a plurality of fractional expressions having a discrimination performance equivalent to that of the index formula 15 was obtained. Those fractional expressions are presented in FIG. 39 and FIG. 40.

Subsequently, an index by which the performance of discriminating between the 2 groups of the active phase Crohn disease group and the remission phase Crohn disease group is maximized was eagerly searched, and an index formula 16 was obtained among a plurality of indices having an equivalent performance.

```
Index formula 16: (Gly)/(Lys)+(Ser+Tau+Met)/(His)
```

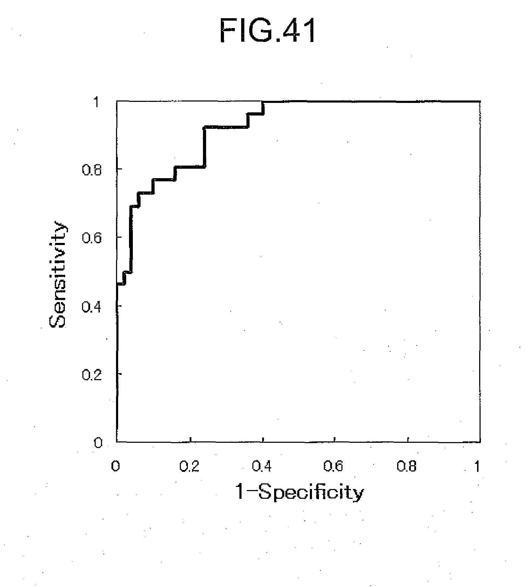

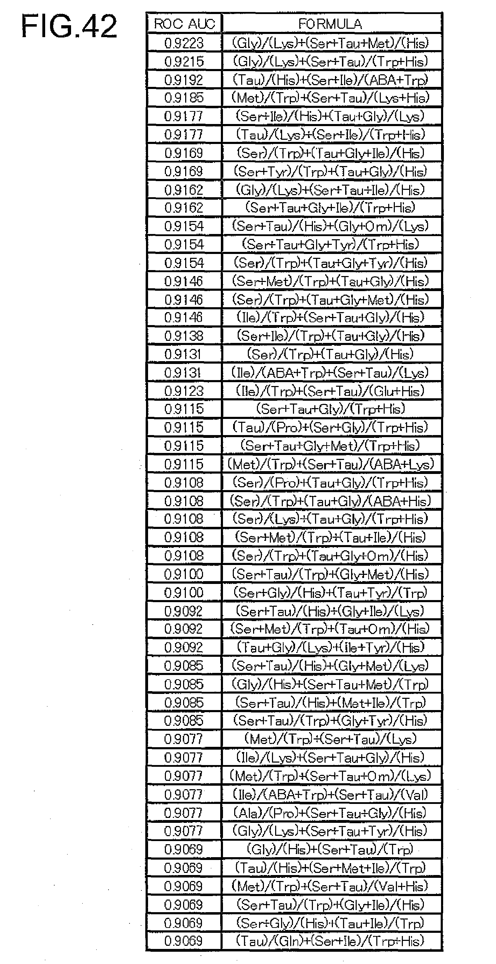

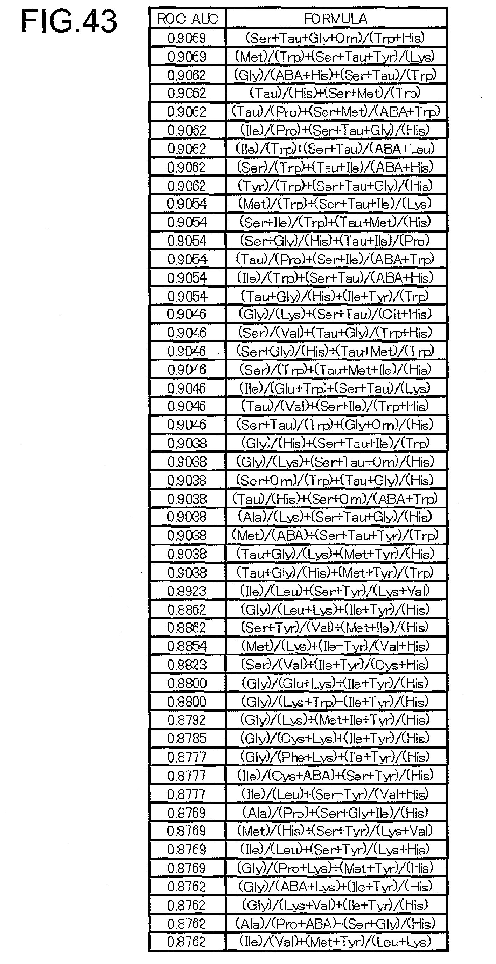

An evaluation was performed on the discrimination between the 2 groups of the active phase Crohn disease group and the remission phase Crohn disease group based on the index formula 16, on the basis of the AUC of the ROC curve (FIG. 41), and an AUC of $0.922\pm0.030$ (95% confidence interval: 0.864 to 0.981) was obtained. When the optimum cutoff value for the discrimination between the 2 groups of the active phase Crohn disease group and the remission phase Crohn disease group by the index formula 16 was determined, the cutoff value was 5.102, and a sensitivity of 96%, a specificity of 69%, and a correct diagnostic rate of 87% were obtained. Thus, the index formula 16 was found to be a useful index with high diagnostic performance. In addition to that, a plurality of fractional expressions having a discrimination performance equivalent to that of the index formula 16 was obtained. Those fractional expressions are presented in FIG. 42 and FIG. 43.

Subsequently, an index by which the performance of discriminating between the 2 groups of the active phase ulcerative colitis group and the remission phase ulcerative colitis group is maximized was eagerly searched, and an index formula 17 was obtained among a plurality of indices having an equivalent performance.

```
Index formula 17: (Orn)/(Glu+Trp+His)
```

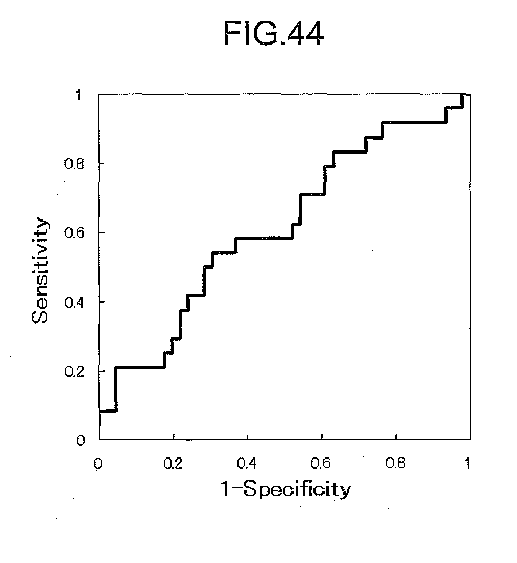

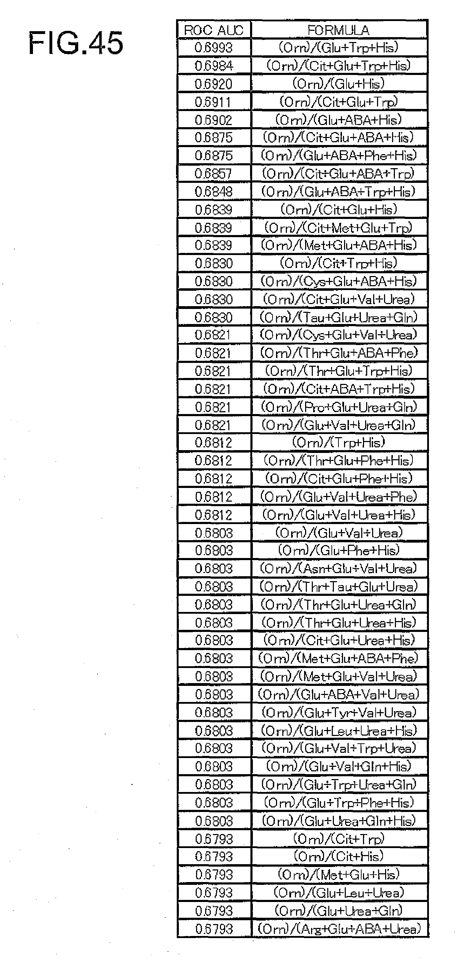

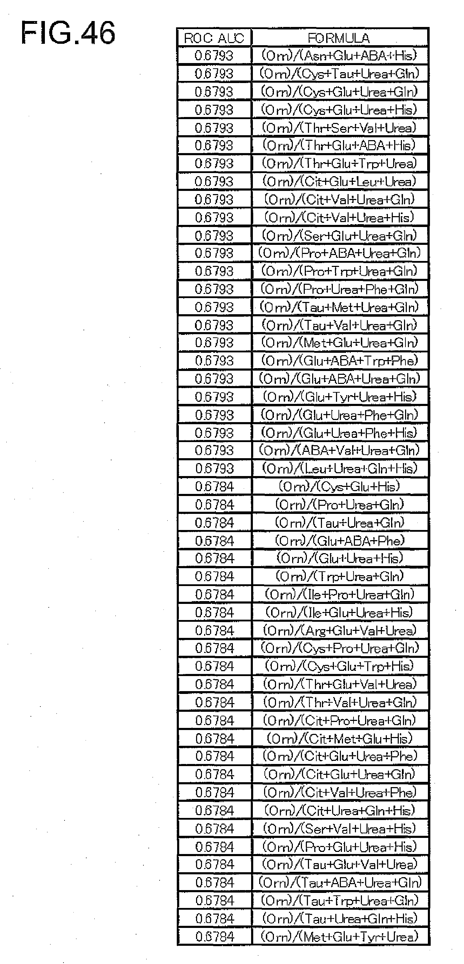

An evaluation was performed on the discrimination between the 2 groups of the active phase ulcerative colitis group and the remission phase ulcerative colitis group based on the index formula 17, on the basis of the AUC of the ROC curve (FIG. 44), and an AUC of $0.699\pm0.067$ (95% confidence interval: 0.568 to 0.830) was obtained. When the optimum cutoff value for the discrimination between the 2 groups of the active phase ulcerative colitis group and the remission phase ulcerative colitis group by the index formula 17 was determined, the cutoff value was 0.351, and a sensitivity of 78%, a specificity of 50%, and a correct diagnostic rate of 67% were obtained. Thus, the index formula 17 was found to be a useful index with high diagnostic performance. In addition to that, a plurality of fractional expressions having a discrimination performance equivalent to that of the index formula 17 was obtained. Those fractional expressions are presented in FIG. 45 and FIG. 46.

Example 5

The sample data used in Example 1 were used. Using a method (method for searching a multivariate discriminant) described in International Publication WO 2006/098192 that is an international application filed by the present applicant, first, an index by which the performance of discriminating between the 2 groups of the healthy subject group and the inflammatory bowel disease group is maximized was eagerly searched by linear discriminant analysis, and an index formula 21 was obtained among a plurality of indices having an equivalent performance.

```
Index formula 21: 1.29777+0.00433×Tau-
0.00007×Urea+0.01967×Cys-0.01055×Tyr-0.00479×His-
0.00736×Trp
```

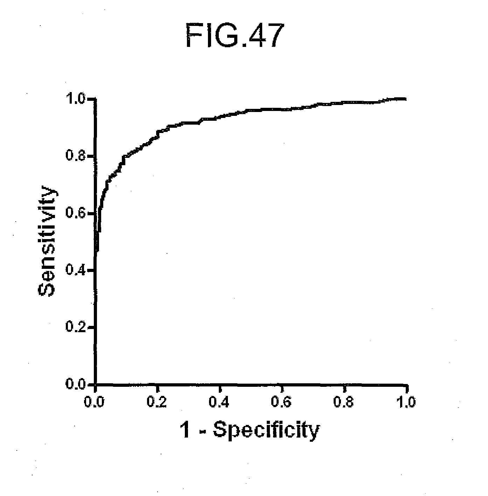

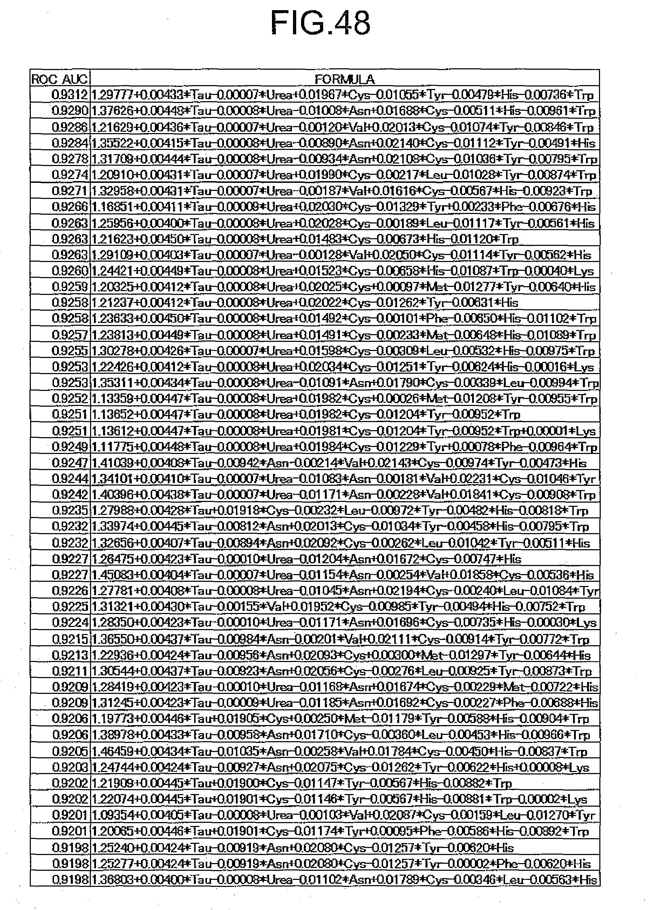

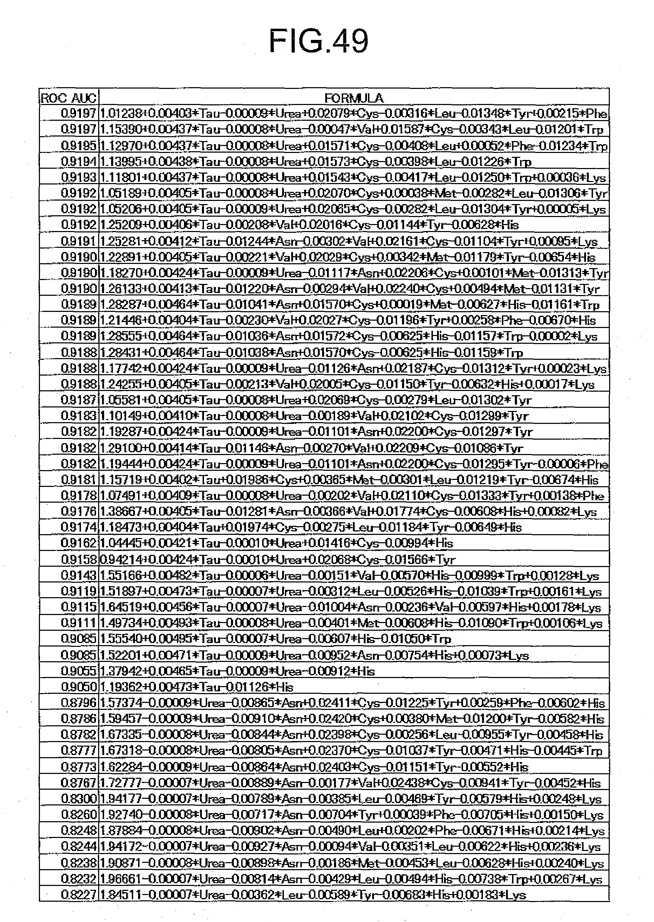

An evaluation was performed on the discrimination between the 2 groups of the healthy subject group and the inflammatory bowel disease group based on the index formula 21, on the basis of the AUC of the ROC curve (FIG. 47), and an AUC of $0.931\pm0.019$ (95% confidence interval: 0.894 to 0.968) was obtained. Thus, the index formula 21 was found to be a useful index with high diagnostic performance. In addition to that, a plurality of linear discriminants having a discrimination performance equivalent to that of the index formula 21 was obtained. Those linear discriminants are presented in FIG. 48 and FIG. 49. The discriminants presented in FIG. 48 and FIG. 49 may be discriminants obtained by transformation of monotonic increase or monotonic decrease, such as linear transformation such as addition of a constant number or multiplication of a constant number, and logit transformation.

Subsequently, an index by which the performance of discriminating between the 2 groups of the healthy subject group and the inflammatory bowel disease group is maximized was eagerly searched by logistic regression analysis, and an index formula 22 was obtained among a plurality of indices having an equivalent performance.

```
Index formula 22: 1/(1+exp(-x))
```

```
provided that
```

```
x=0.81765+0.11415×Tau+0.06468×Glu+0.01166×Gly-0.04213×Leu-
```

```
0.03070×His-0.12380×Trp
```

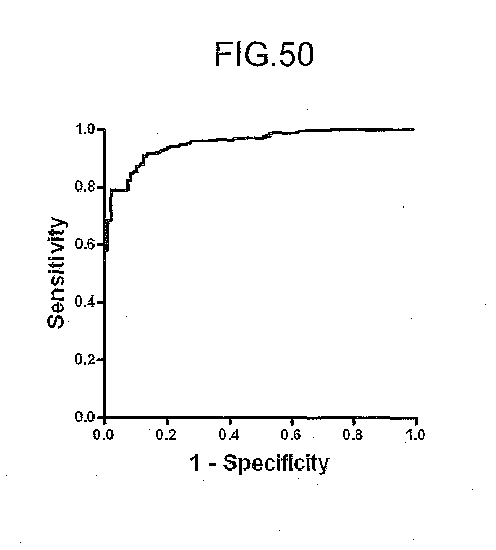

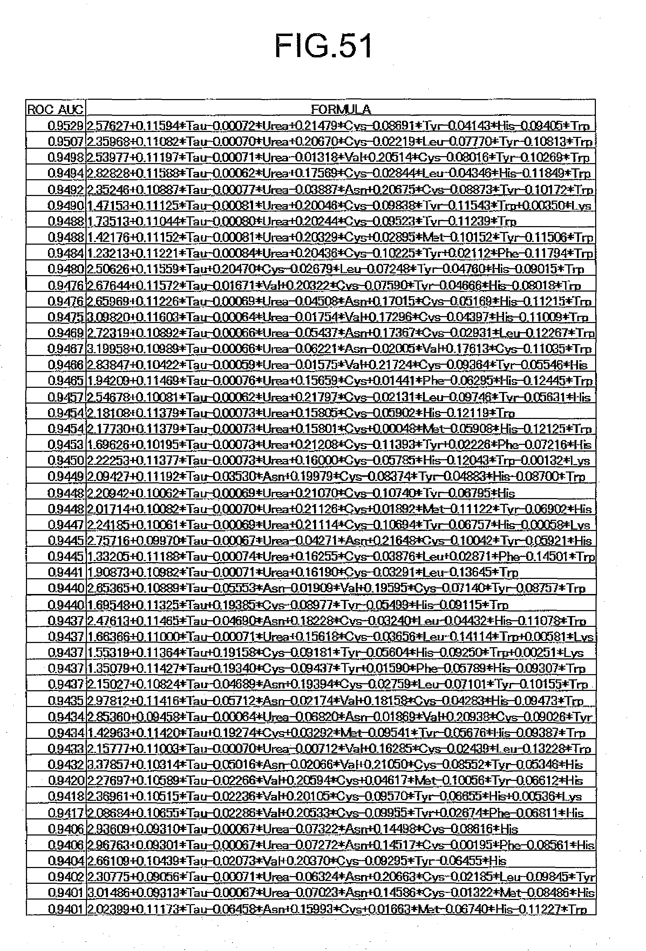

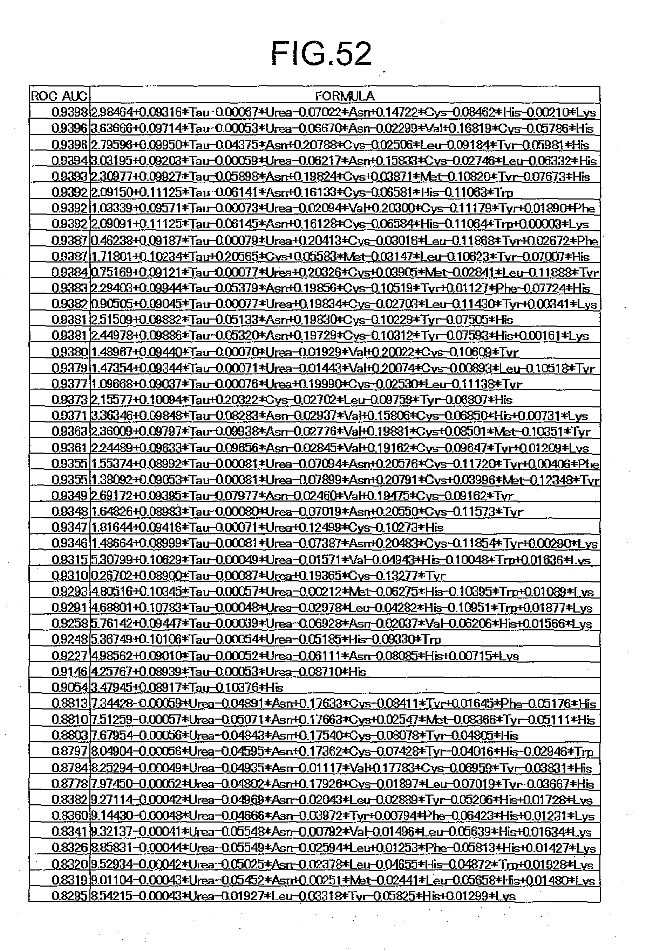

An evaluation was performed on the discrimination between the 2 groups of the healthy subject group and the inflammatory bowel disease group based on the index formula 22, on the basis of the AUC of the ROC curve (FIG. 50), and an AUC of $0.953\pm0.016$ (95% confidence interval: 0.923 to 0.983) was obtained. Thus, the index formula 22 was found to be a useful index with high diagnostic performance. In addition to that, a plurality of logistic regression equations having a discrimination performance equivalent to that of the index formula 22 was obtained. Those x's are presented in FIG. 51 and FIG. 52. The equations presented in FIG. 51 and FIG. 52 may be equations obtained by transformation of monotonic increase or monotonic decrease, such as linear transformation such as addition of a constant number or multiplication of a constant number, and logit transformation.

Subsequently, an index by which the performance of discriminating between the 2 groups of the healthy subject group and the Crohn disease group is maximized was eagerly searched by linear discriminant analysis, and an index formula 23 was obtained among a plurality of indices having an equivalent performance.

```
Index formula 23:
```

```
1.10711+0.00426×Tau+0.01052×Glu+0.00120×Gly-0.00247×Val-
```

```
0.00955×Tyr-0.01140×Trp
```

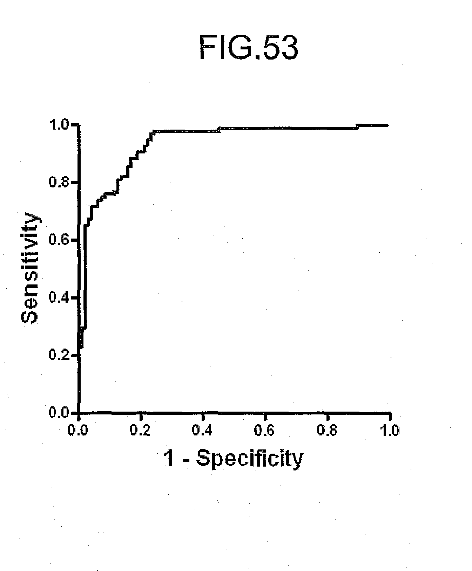

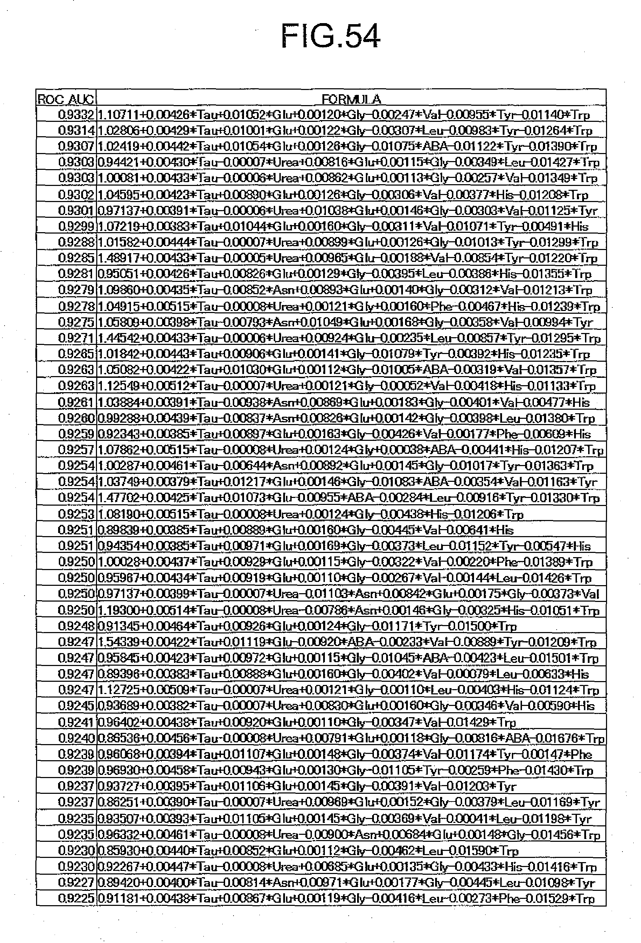

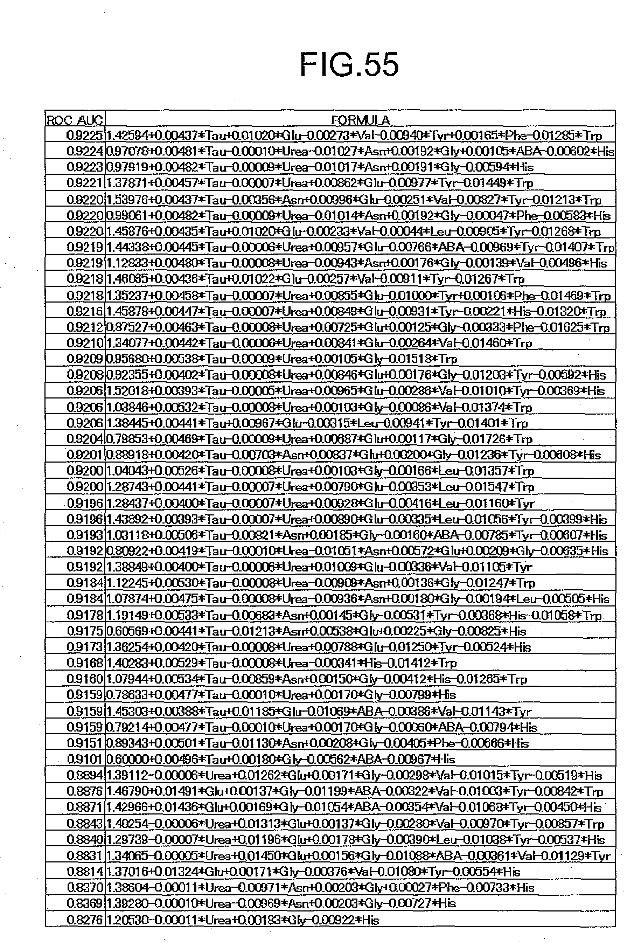

An evaluation was performed on the discrimination between the 2 groups of the healthy subject group and the Crohn disease group based on the index formula 23, on the basis of the AUC of the ROC curve (FIG. 53), and an AUC of $0.933\pm0.019$ (95% confidence interval: 0.896 to 0.970) was obtained. Thus, the index formula 23 was found to be a useful index with high diagnostic performance. In addition to that, a plurality of linear discriminants having a discrimination performance equivalent to that of the index formula 23 was obtained. Those linear discriminants are presented in FIG. 54 and FIG. 55. The discriminants presented in FIG. 54 and FIG. 55 may be discriminants obtained by transformation of monotonic increase or monotonic decrease, such as linear transformation such as addition of a constant number or multiplication of a constant number, and logit transformation.

Subsequently, an index by which the performance of discriminating between the 2 groups of the healthy subject group and the Crohn disease group is maximized was eagerly searched by logistic regression analysis, and an index formula 24 was obtained among a plurality of indices having an equivalent performance.

```
Index formula 24: 1/(1+exp(-x))
```

provided that

```
x=0.81765+0.11415×Tau+0.06468×Glu+0.01166×Gly-0.04213×Leu-
```

```
0.03070×His-0.12380×Trp
```

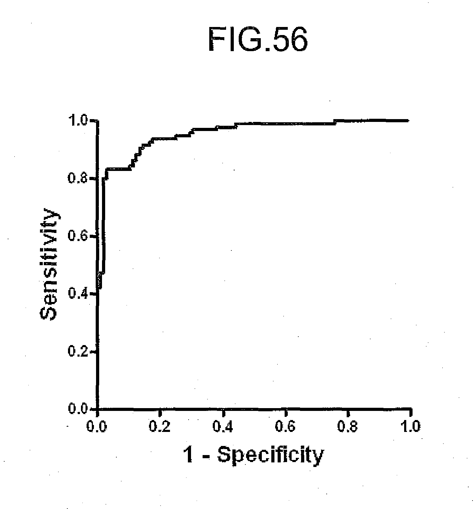

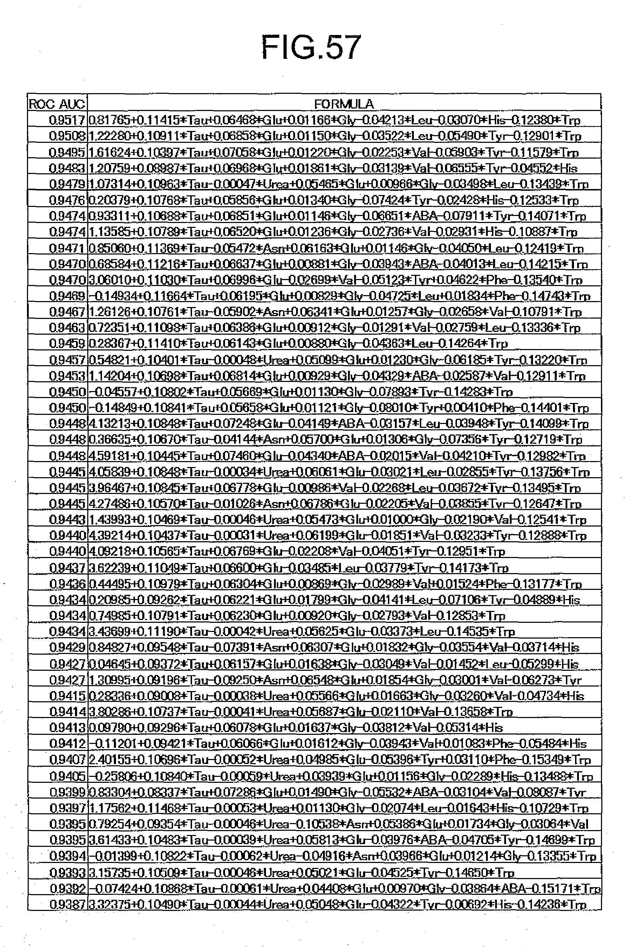

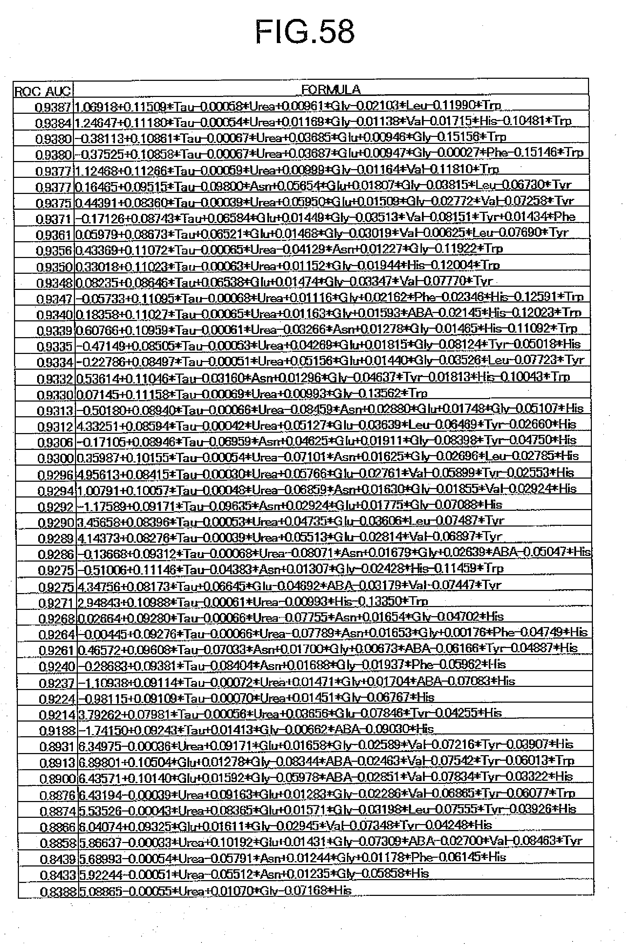

An evaluation was performed on the discrimination between the 2 groups of the healthy subject group and the Crohn disease group based on the index formula 24, on the basis of the AUC of the ROC curve (FIG. 56), and an AUC of $0.952\pm0.016$ (95% confidence interval: 0.920 to 0.984) was obtained. Thus, the index formula 24 was found to be a useful index with high diagnostic performance. In addition to that, a plurality of logistic regression equations having a discrimination performance equivalent to that of the index formula 24 was obtained. Those x's are presented in FIG. 57 and FIG. 58. The equations presented in FIG. 57 and FIG. 58 may be equations obtained by transformation of monotonic increase or monotonic decrease, such as linear transformation such as addition of a constant number or multiplication of a constant number, and logit transformation.

Subsequently, an index by which the performance of discriminating between the 2 groups of the healthy subject group and the ulcerative colitis group is maximized was eagerly searched by linear discriminant analysis, and an index formula 25 was obtained among a plurality of indices having an equivalent performance.

```
Index formula 25: 1.13409+0.00968×Tau-
0.01219×Asn+0.01651×Cys-0.01445×His-0.00759×Trp+0.00134×Lys
```

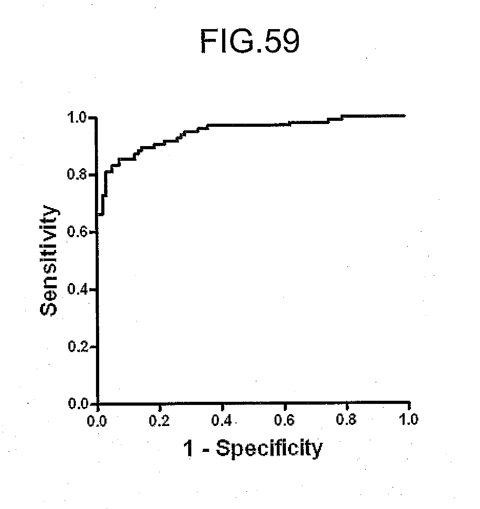

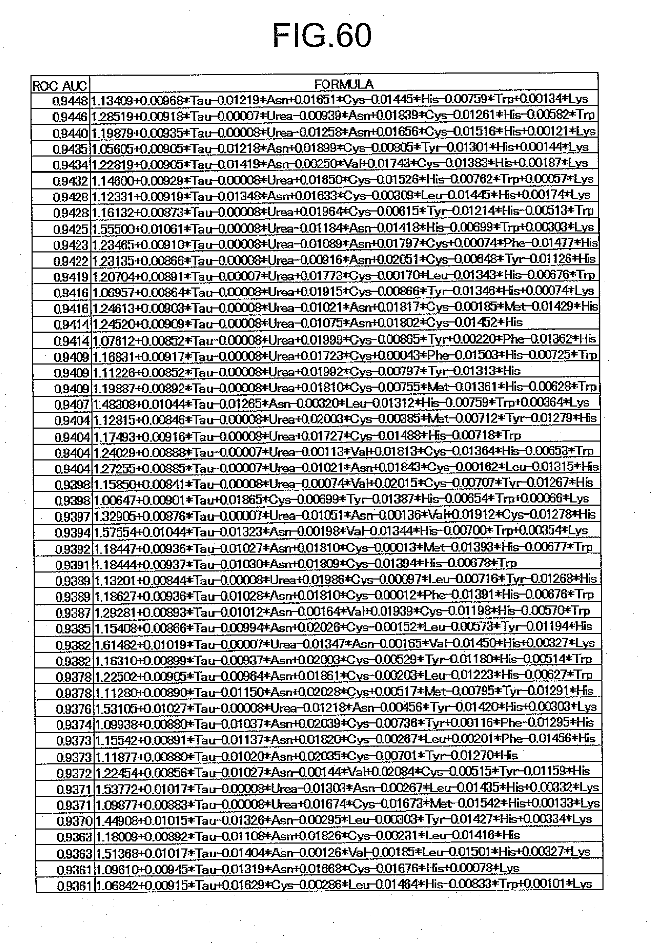

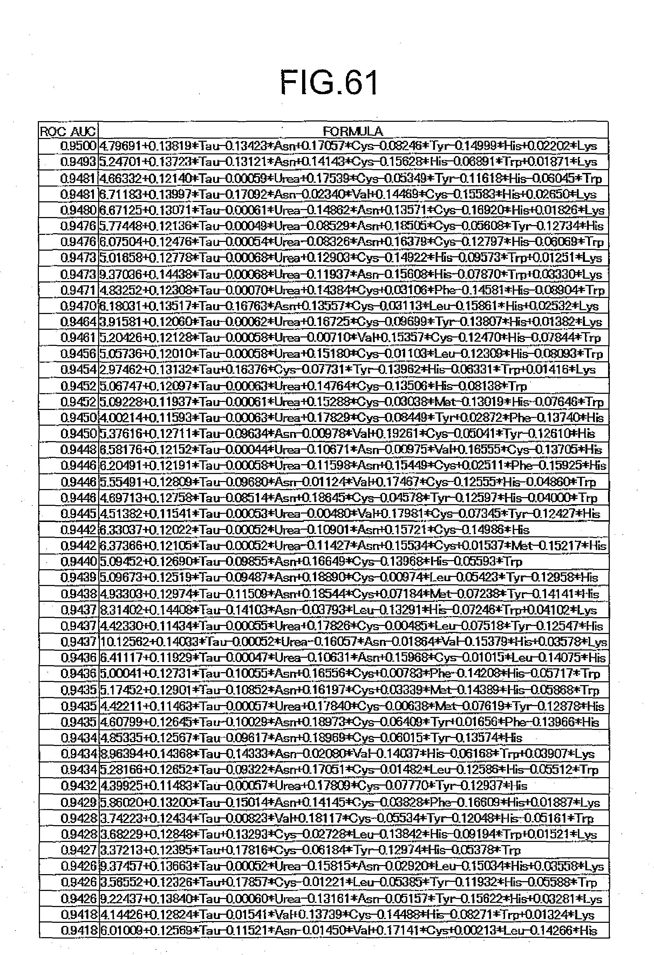

An evaluation was performed on the discrimination between the 2 groups of the healthy subject group and the ulcerative colitis group based on the index formula 25, on the basis of the AUC of the ROC curve (FIG. 59), and an AUC of $0.945\pm0.017$ (95% confidence interval: 0.911 to 0.979) was obtained. Thus, the index formula 25 was found to be a useful index with high diagnostic performance. In addition to that, a plurality of linear discriminants having a discrimination performance equivalent to that of the index formula 25 was obtained. Those linear discriminants are presented in FIG. 60 and FIG. 61. The discriminants presented in FIG. 60 and FIG. 61 may be discriminants obtained by transformation of monotonic increase or monotonic decrease, such as linear transformation such as addition of a constant number or multiplication of a constant number, and logit transformation.

Subsequently, an index by which the performance of discriminating between the 2 groups of the healthy subject group and the ulcerative colitis group is maximized was eagerly searched by logistic regression analysis, and an index formula 26 was obtained among a plurality of indices having an equivalent performance.

```
Index formula 26: 1/(1+exp(-x))
provided that x=4.79691+0.13819×Tau-
0.13423×Asn+0.17057×Cys-0.08246×Tyr-0.14999×His+0.02202×Lys
```

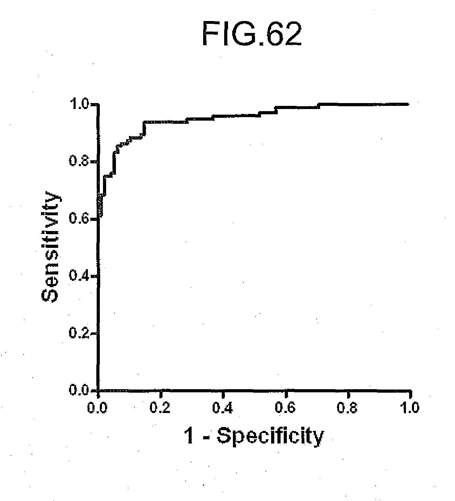

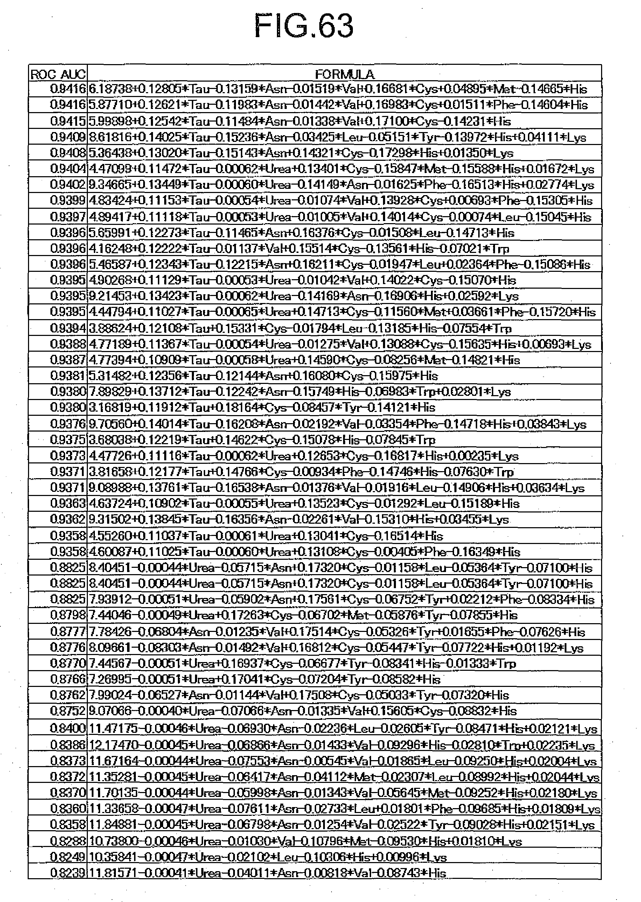

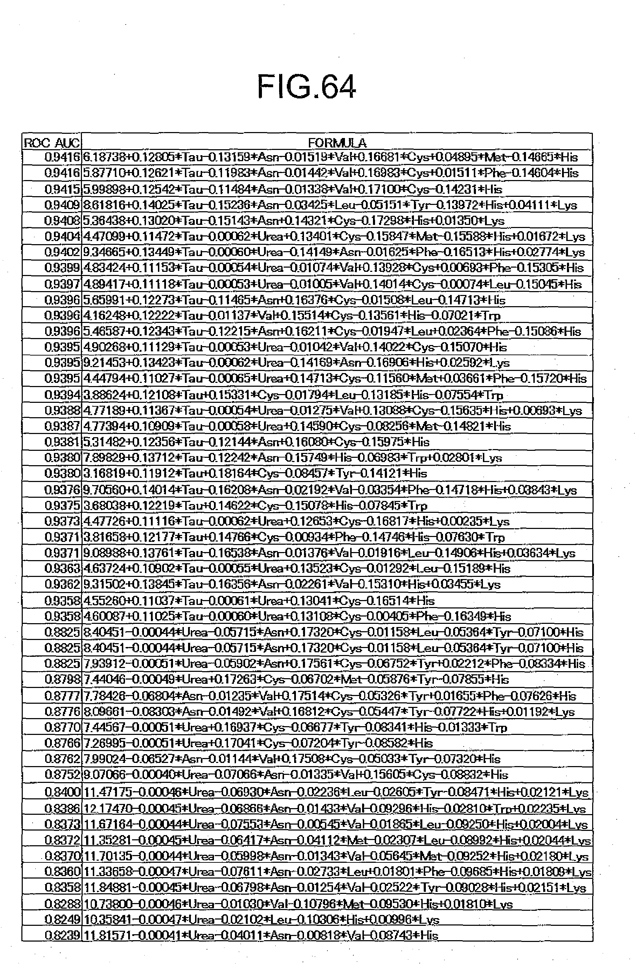

An evaluation was performed on the discrimination between the 2 groups of the healthy subject group and the ulcerative colitis group based on the index formula 26, on the basis of the AUC of the ROC curve (FIG. 62), and an AUC of $0.942\pm0.018$ (95% confidence interval: 0.907 to 0.977) was obtained. Thus, the index formula 26 was found to be a useful index with high diagnostic performance. In addition to that, a plurality of logistic regression equations having a discrimination performance equivalent to that of the index formula 26 was obtained. Those x's are presented in FIG. 63 and FIG. 64. The equations presented in FIG. 63 and FIG. 64 may be equations obtained by transformation of monotonic increase or monotonic decrease, such as linear transformation such as addition of a constant number or multiplication of a constant number, and logit transformation.

Subsequently, an index by which the performance of discriminating between the 2 groups of the Crohn disease group and the ulcerative colitis group is maximized was eagerly searched by linear discriminant analysis, and an index formula 27 was obtained among a plurality of indices having an equivalent performance.

```
Index formula 27: -0.01486+0.00202×Tau-
0.00062×Thr+0.00149×Pro+0.00135×Gly-0.00704×Trp+0.00366×Orn
```

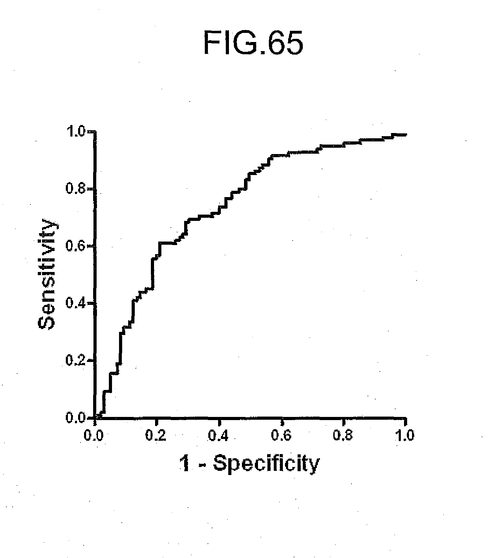

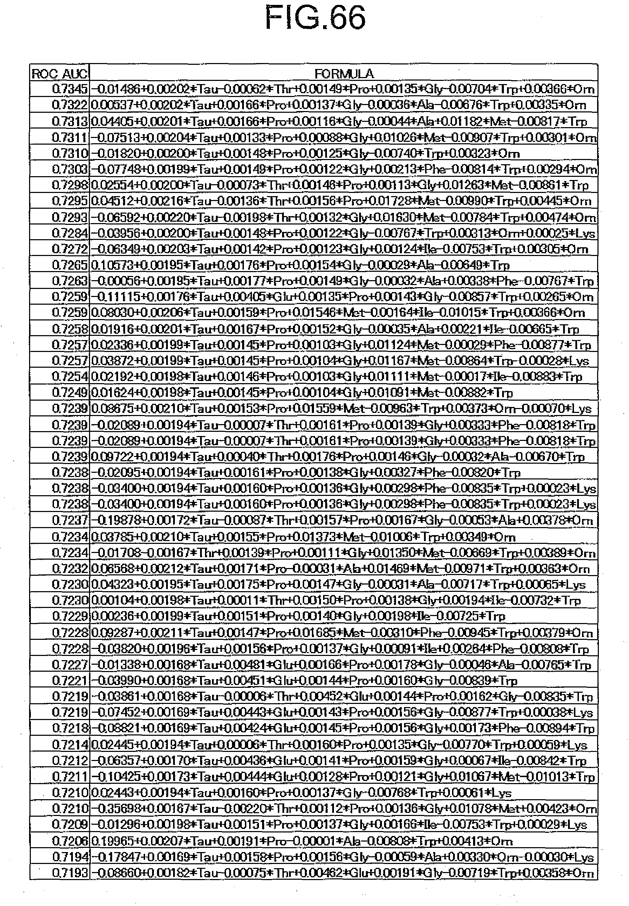

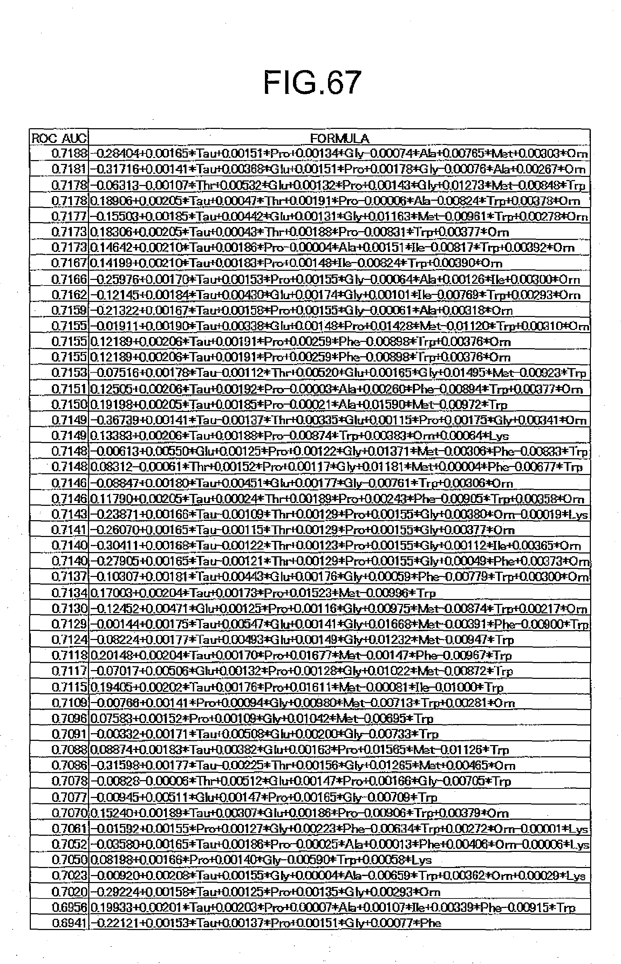

An evaluation was performed on the discrimination between the 2 groups of the Crohn disease group and the ulcerative colitis group based on the index formula 27, on the basis of the AUC of the ROC curve (FIG. 65), and an AUC of $0.735\pm0.036$ (95% confidence interval: 0.664 to 0.806) was obtained. Thus, the index formula 27 was found to be a useful index with high diagnostic performance. In addition to that, a plurality of linear discriminants having a discrimination performance equivalent to that of the index formula 27 was obtained. Those linear discriminants are presented in FIG. 66 and FIG. 67. The discriminants presented in FIG. 66 and FIG. 67 may be discriminants obtained by transformation of monotonic increase or monotonic decrease, such as linear transformation such as addition of a constant number or multiplication of a constant number, and logit transformation.

Subsequently, an index by which the performance of discriminating between the 2 groups of the Crohn disease group and the ulcerative colitis group is maximized was eagerly searched by logistic regression analysis, and an index formula 28 was obtained among a plurality of indices having an equivalent performance.

```
Index formula 28: 1/(1+exp(-x))

provided that x=-2.46344+0.01083×Tau-

0.00327×Thr+0.00661×Pro+0.00657×Gly-0.03267×Trp+0.01720×Orn
```

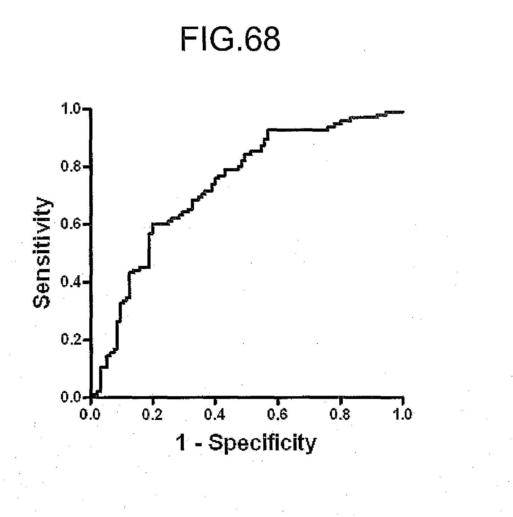

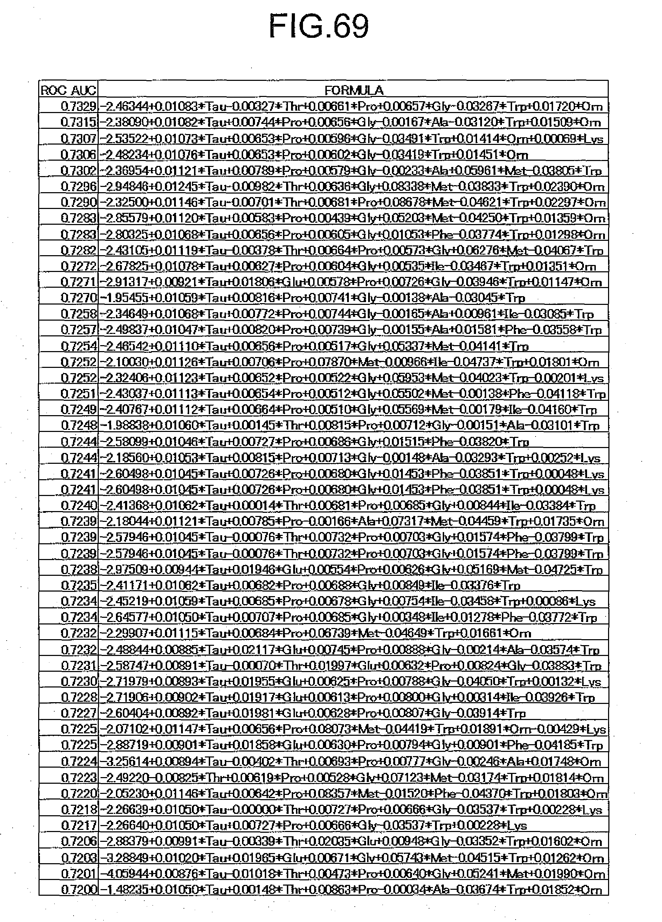

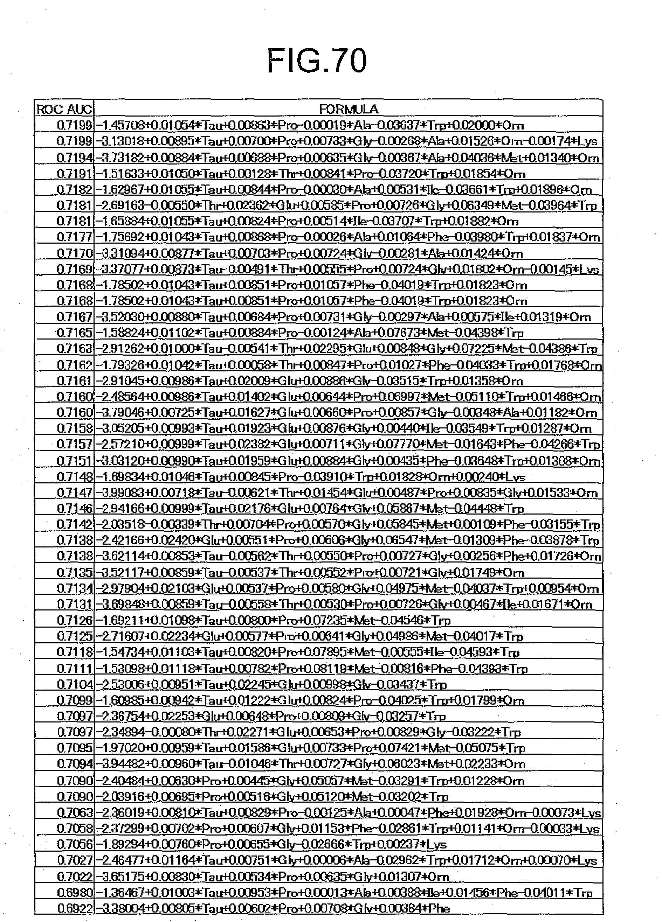

An evaluation was performed on the discrimination between the 2 groups of the Crohn disease group and the ulcerative colitis group based on the index formula 28, on the basis of the AUC of the ROC curve (FIG. 68), and an AUC of $0.733\pm0.036$ (95% confidence interval: 0.662 to 0.804) was obtained. Thus, the index formula 28 was found to be a useful index with high diagnostic performance. In addition to that, a plurality of logistic regression equations having a discrimination performance equivalent to that of the index formula 28 was obtained. Those x's are presented in FIG. 69 and FIG. 70. The equations presented in FIG. 69 and FIG. 70 may be equations obtained by transformation of monotonic increase or monotonic decrease, such as linear transformation such as addition of a constant number or multiplication of a constant number, and logit transformation.

Subsequently, an index by which the performance of discriminating between the 2 groups of the active phase Crohn disease group and the active phase ulcerative colitis group is maximized was eagerly searched by linear discriminant analysis, and an index formula 29 was obtained among a plurality of indices having an equivalent performance.

```
Index formula 29:

0.32986+0.00251×Tau+0.00509×Ser+0.00146×Ala-

0.02369×Cys+0.01191×Phe-0.01056×Arg
```

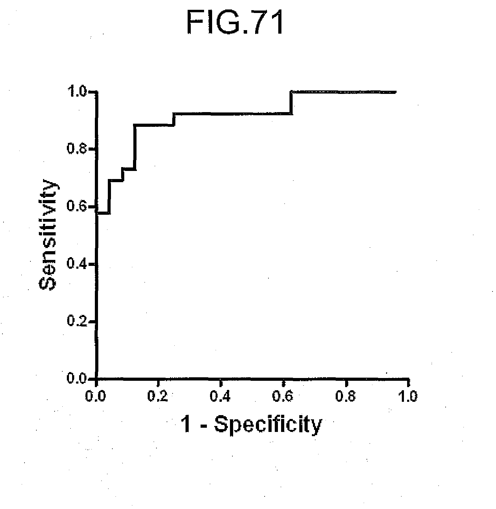

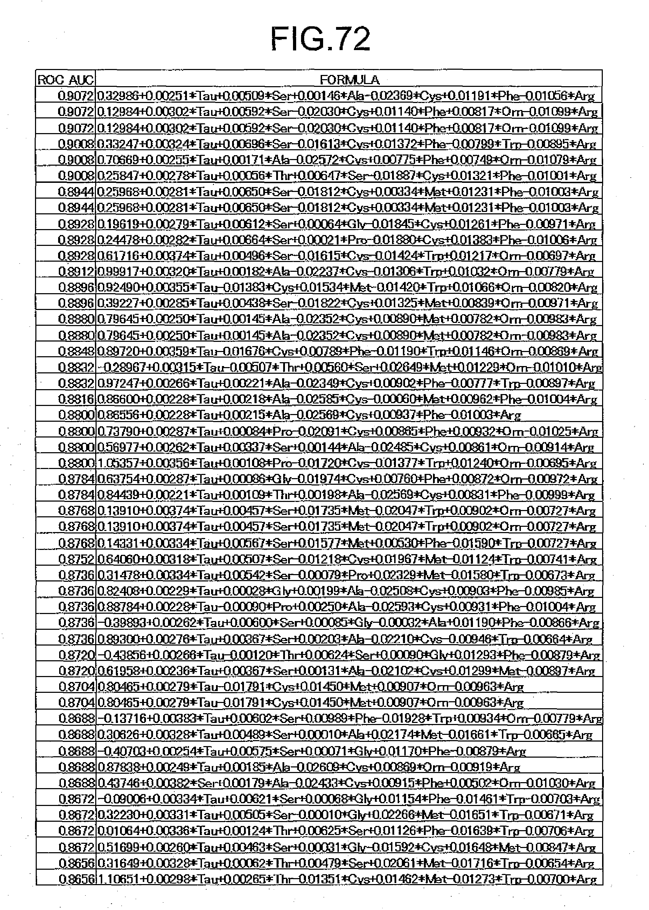

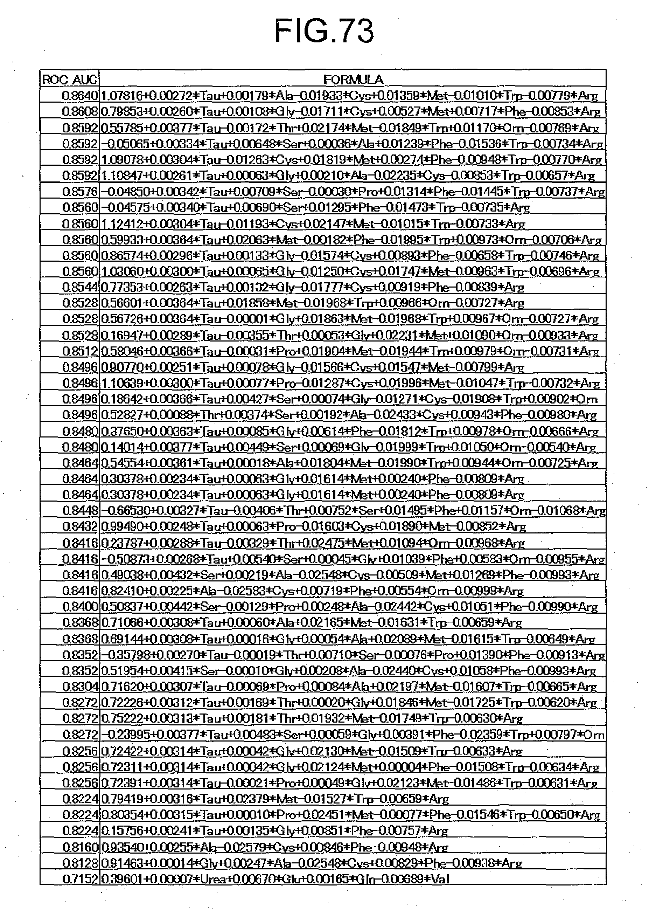

An evaluation was performed on the discrimination between the 2 groups of the active phase Crohn disease group and the active phase ulcerative colitis group based on the index formula 29, on the basis of the AUC of the ROC curve (FIG. 71), and an AUC of $0.907\pm0.044$ (95% confidence interval: 0.822 to 0.992) was obtained. Thus, the index formula 29 was found to be a useful index with high diagnostic performance. In addition to that, a plurality of linear discriminants having a discrimination performance equivalent to that of the index formula 29 was obtained. Those linear discriminants are presented in FIG. 72 and FIG. 73. The discriminants presented in FIG. 72 and FIG. 73 may be discriminants obtained by transformation of monotonic increase or monotonic decrease, such as linear transformation such as addition of a constant number or multiplication of a constant number, and logit transformation.

Subsequently, an index by which the performance of discriminating between the 2 groups of the active phase Crohn disease group and the active phase ulcerative colitis group is maximized was eagerly searched by logistic regression analysis, and an index formula 30 was obtained among a plurality of indices having an equivalent performance.

```
Index formula 30: 1/(1+exp(-x))

provided that x=-3.43602+0.02849×Tau+0.04618×Ser-

0.18815×Cys+0.11767×Phe+0.06761×Orn-0.08874×Arg
```

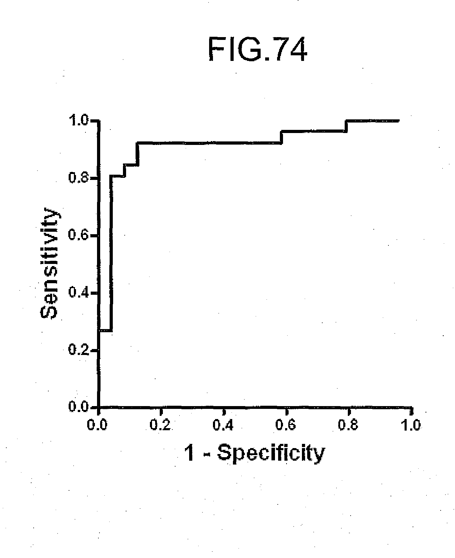

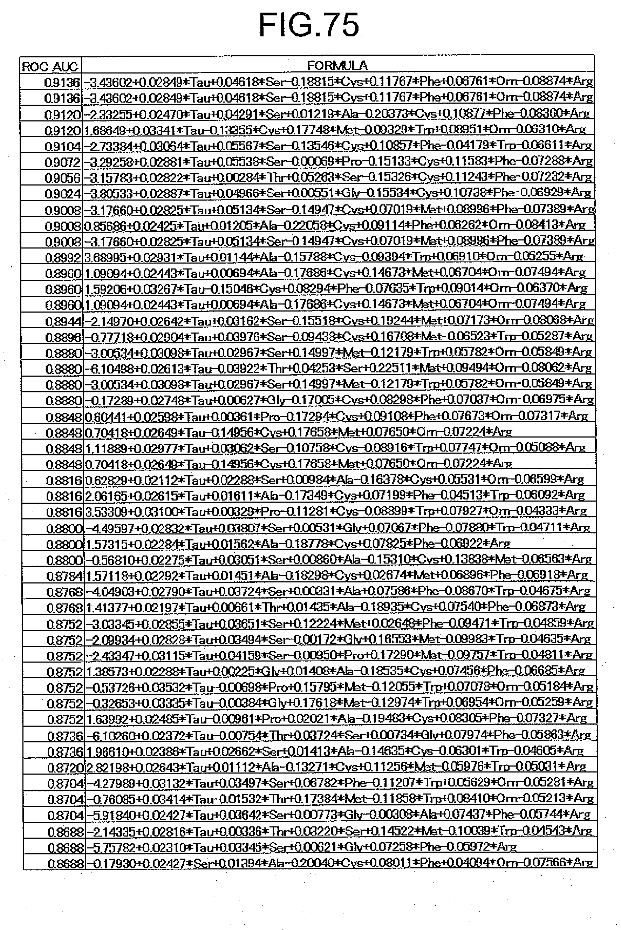

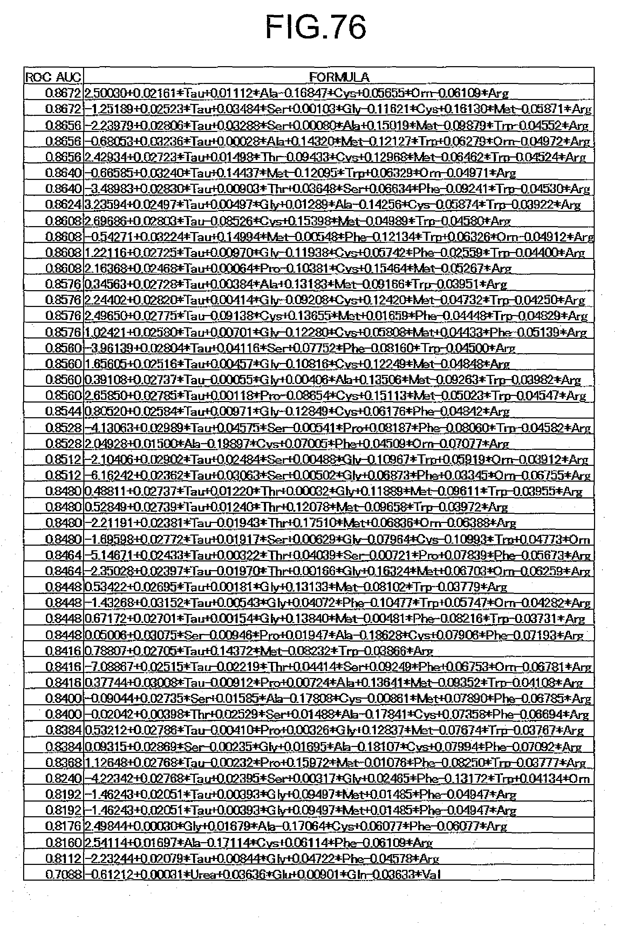

An evaluation was performed on the discrimination between the 2 groups of the active phase Crohn disease group and the active phase ulcerative colitis group based on the index formula 30, on the basis of the AUC of the ROC curve (FIG. 74), and an AUC of $0.914\pm0.042$ (95% confidence interval: 0.832 to 0.996) was obtained. Thus, the index formula 30 was found to be a useful index with high diagnostic performance. In addition to that, a plurality of logistic regression equations having a discrimination performance equivalent to that of the index formula 30 was obtained. Those x's are presented in FIG. 75 and FIG. 76. The equations presented in FIG. 75 and FIG. 76 may be equations obtained by transformation of monotonic increase or monotonic decrease, such as linear transformation such as addition of a constant number or multiplication of a constant number, and logit transformation.

Subsequently, an index by which the performance of discriminating between the 2 groups of the active phase Crohn disease group and the remission phase Crohn disease group is maximized was eagerly searched by linear discriminant analysis, and an index formula 31 was obtained among a plurality of indices having an equivalent performance.

```
Index formula 31:

1.44828+0.00339×Tau+0.00228×Gly+0.00557×Leu-0.02025×His-

0.01369×Trp-0.00306×Lys
```

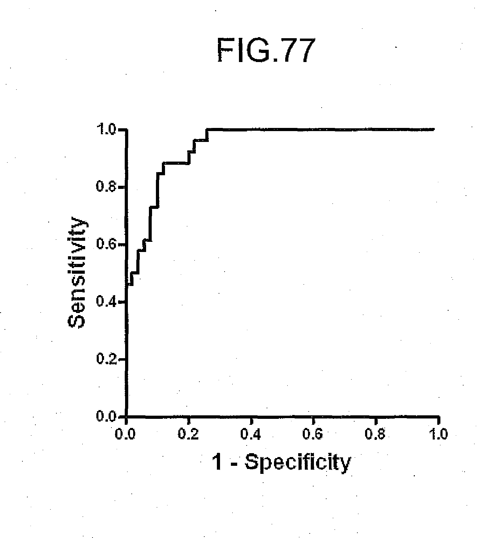

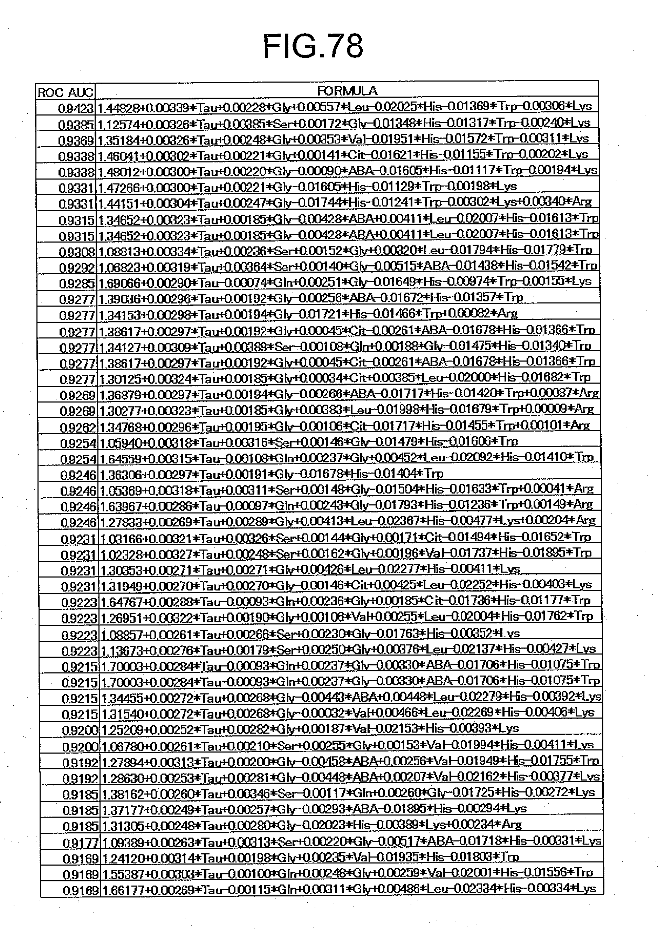

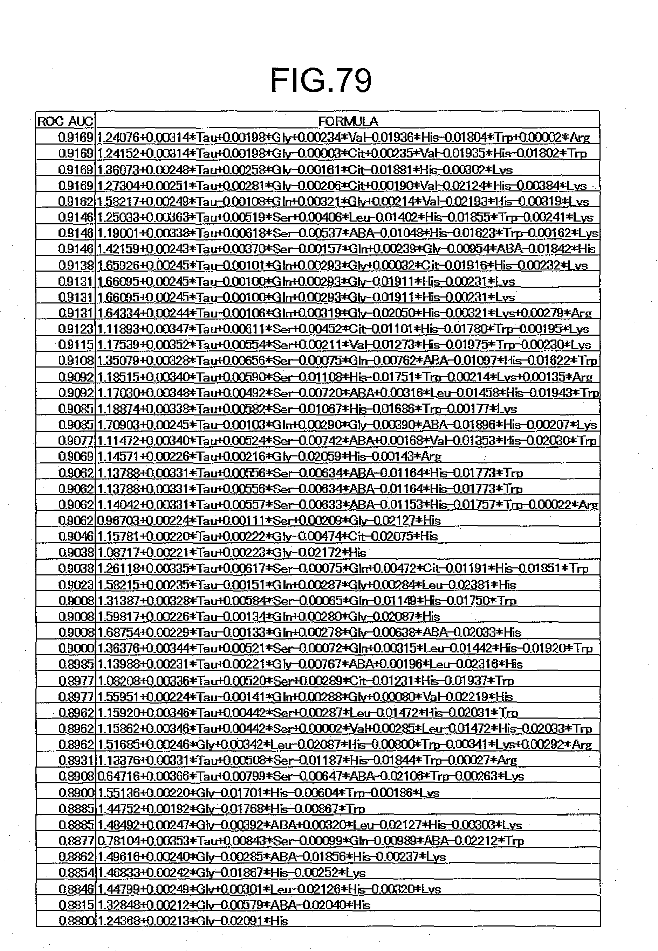

An evaluation was performed on the discrimination between the 2 groups of the active phase Crohn disease group and the remission phase Crohn disease group based on the index formula 31, on the basis of the AUC of the ROC curve (FIG. 77), and an AUC of 0.942±0.033 (95% confidence interval: 0.877 to 0.993) was obtained. Thus, the index formula 31 was found to be a useful index with high diagnostic performance. In addition to that, a plurality of linear discriminants having a discrimination performance equivalent to that of the index formula 31 was obtained. Those linear discriminants are presented in FIG. 78 and FIG. 79. The discriminants presented in FIG. 78 and FIG. 79 may be discriminants obtained by transformation of monotonic increase or monotonic decrease, such as linear transformation such as addition of a constant number or multiplication of a constant number, and logit transformation.

Subsequently, an index by which the performance of discriminating between the 2 groups of the active phase Crohn disease group and the remission phase Crohn disease group is maximized was eagerly searched by logistic regression analysis, and an index formula 32 was obtained among a plurality of indices having an equivalent performance.

```
Index formula 32: 1/(1+exp(-x))
```

provided that

```
x=8.98649+0.03455×Tau+0.02334×Gly+0.02746×Val-0.18880×His-

0.11074×Trp-0.03571×Lys
```

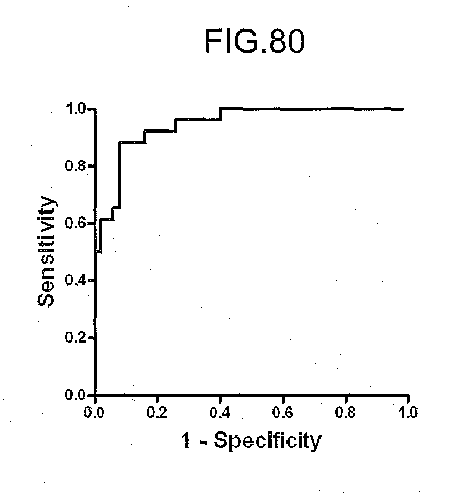

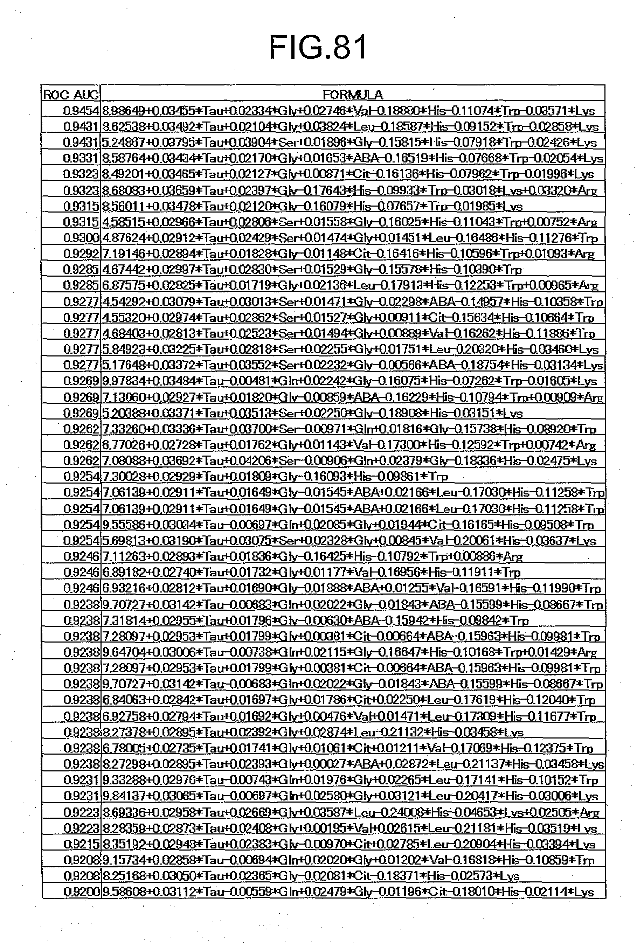

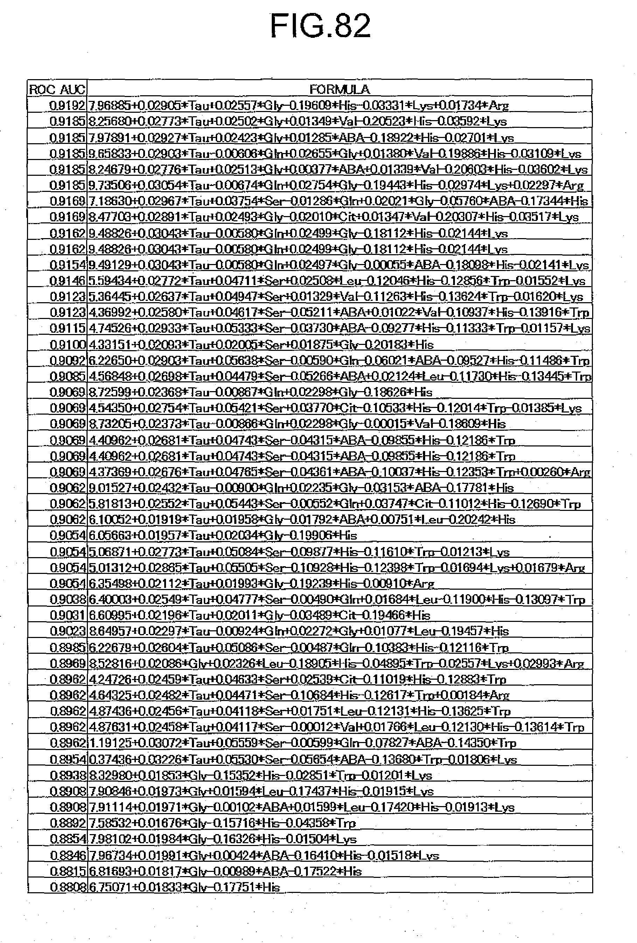

An evaluation was performed on the discrimination between the 2 groups of the active phase Crohn disease group and the remission phase Crohn disease group based on the index formula 32, on the basis of the AUC of the ROC curve (FIG. 80), and an AUC of 0.945±0.032 (95% confidence interval: 0.882 to 0.993) was obtained. Thus, the index formula 32 was found to be a useful index with high diagnostic performance. In addition to that, a plurality of logistic regression equations having a discrimination performance equivalent to that of the index formula 32 was obtained. Those x's are presented in FIG. 81 and FIG. 82. The equations presented in FIG. 81 and FIG. 82 may be equations obtained by transformation of monotonic increase or monotonic decrease, such as linear transformation such as addition of a constant number or multiplication of a constant number, and logit transformation.

Subsequently, an index by which the performance of discriminating between the 2 groups of the active phase ulcerative colitis group and the remission phase ulcerative colitis group is maximized was eagerly searched by linear discriminant analysis, and an index formula 33 was obtained among a plurality of indices having an equivalent performance.

```
Index formula 33: 2.64681+0.00056×Tau-0.00013×Urea-

0.00749×Glu-0.00154×Gln+0.00794×Cit-0.01301×His
```

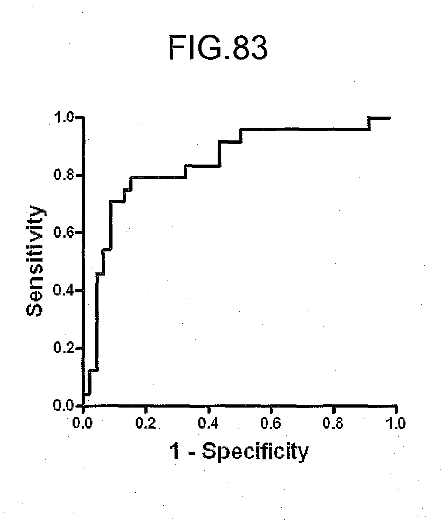

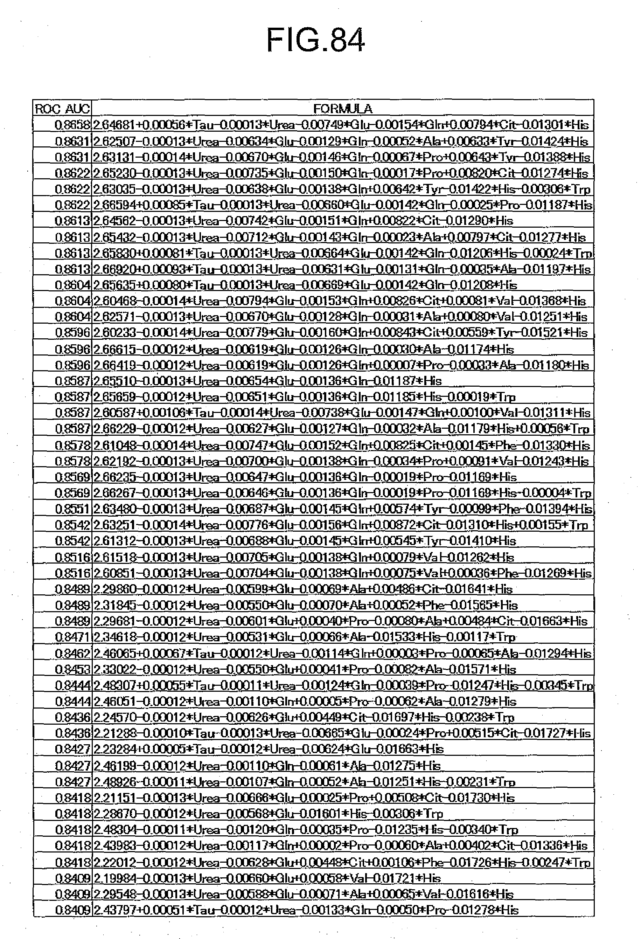

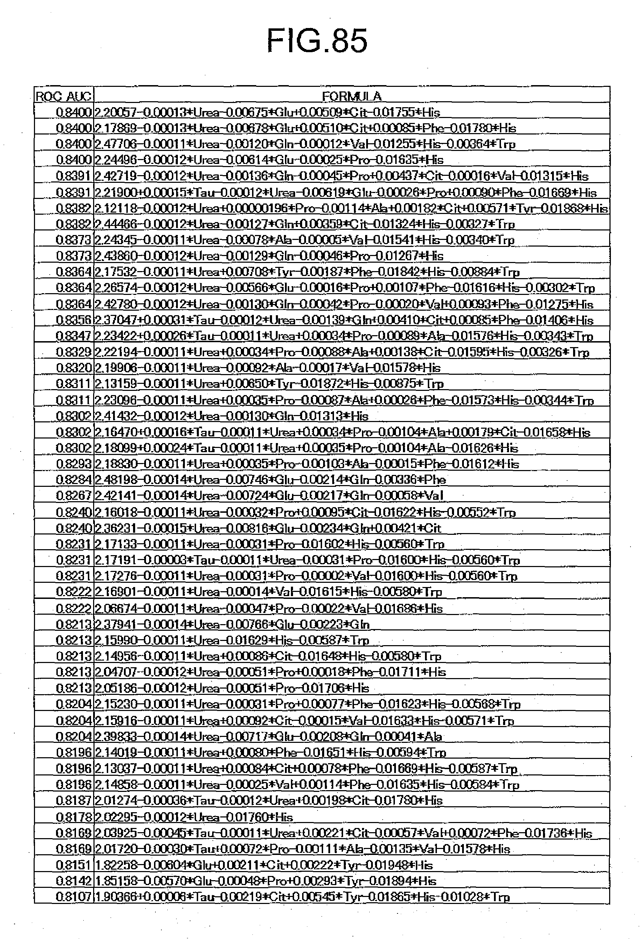

An evaluation was performed on the discrimination between the 2 groups of the active phase ulcerative colitis group and the remission phase ulcerative colitis group based on the index formula 33, on the basis of the AUC of the ROC curve (FIG. 83), and an AUC of 0.866±0.051 (95% confidence interval: 0.766 to 0.966) was obtained. Thus, the index formula 33 was found to be a useful index with high diagnostic performance. In addition to that, a plurality of linear discriminants having a discrimination performance equivalent to that of the index formula 33 was obtained. Those linear discriminants are presented in FIG. 84 and FIG. 85. The discriminants presented in FIG. 84 and FIG. 85 may be discriminants obtained by transformation of monotonic increase or monotonic decrease, such as linear transformation such as addition of a constant number or multiplication of a constant number, and logit transformation.

Subsequently, an index by which the performance of discriminating between the 2 groups of the active phase ulcerative colitis group and the remission phase ulcerative colitis group is maximized was eagerly searched by logistic regression analysis, and an index formula 34 was obtained among a plurality of indices having an equivalent performance.

```
Index formula 34: 1/(1+exp(-x))

provided that x=13.05911-0.00096×Urea-0.04677×Glu-

0.00985×Gln+0.05012×Cit+0.04204×Tyr-0.09780×His
```

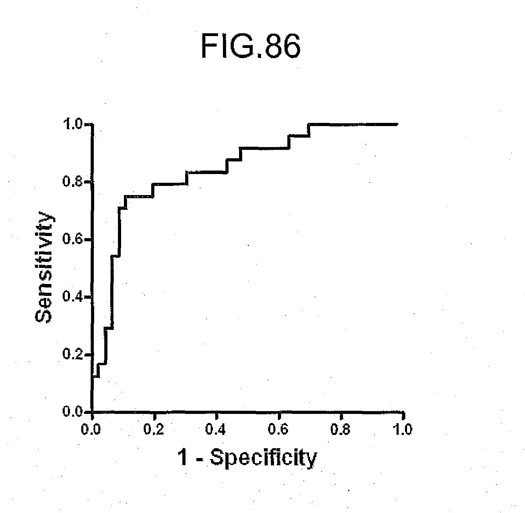

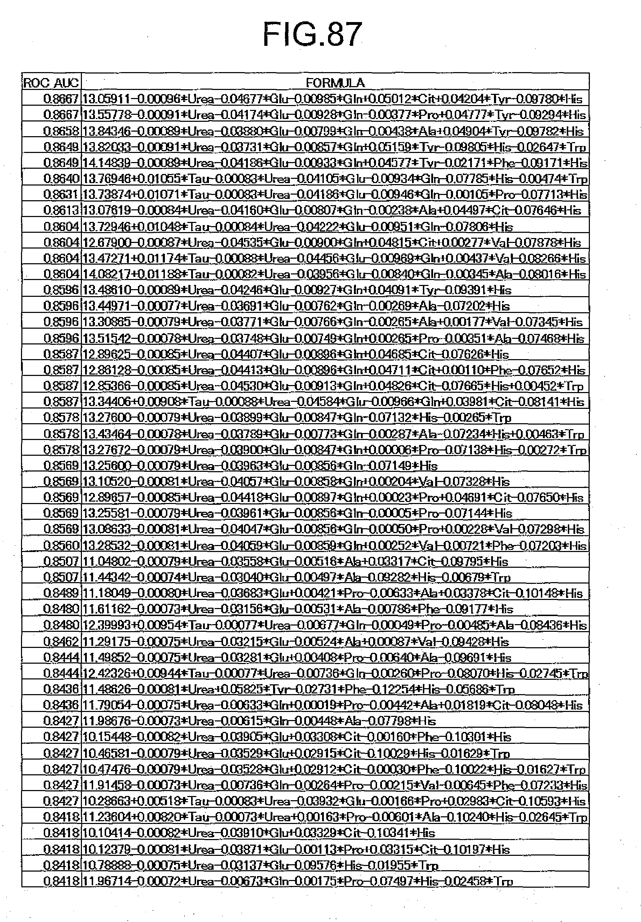

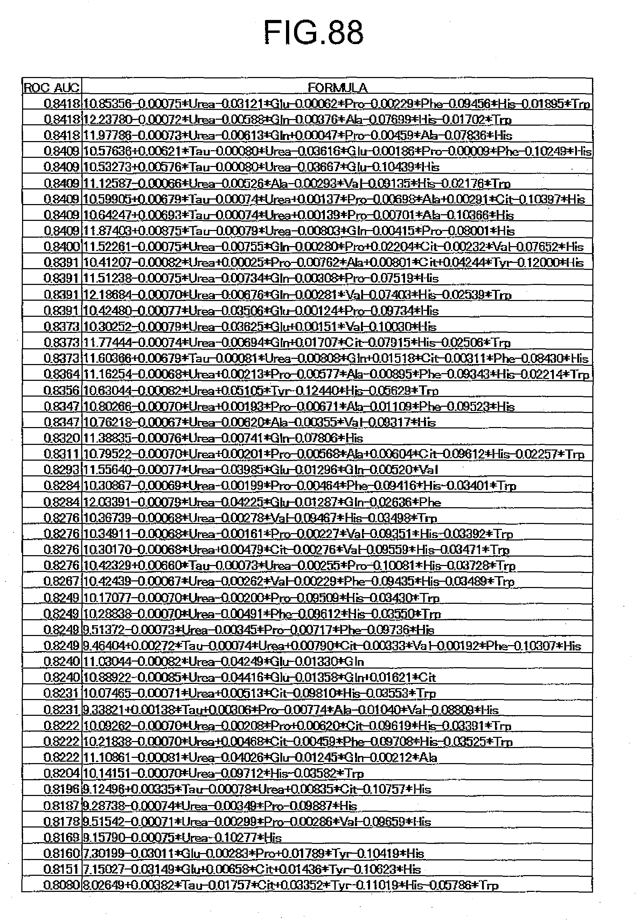

An evaluation was performed on the discrimination between the 2 groups of the active phase ulcerative colitis group and the remission phase ulcerative colitis group based on the index formula 34, on the basis of the AUC of the ROC curve (FIG. 86), and an AUC of $0.867\pm0.051$ (95% confidence interval: 0.768 to 0.966) was obtained. Thus, the index formula 34 was found to be a useful index with high diagnostic performance. In addition to that, a plurality of logistic regression equations having a discrimination performance equivalent to that of the index formula 34 was obtained. Those x's are presented in FIG. 87 and FIG. 88. The equations presented in FIG. 87 and FIG. 88 may be equations obtained by transformation of monotonic increase or monotonic decrease, such as linear transformation such as addition of a constant number or multiplication of a constant number, and logit transformation.

Example 6

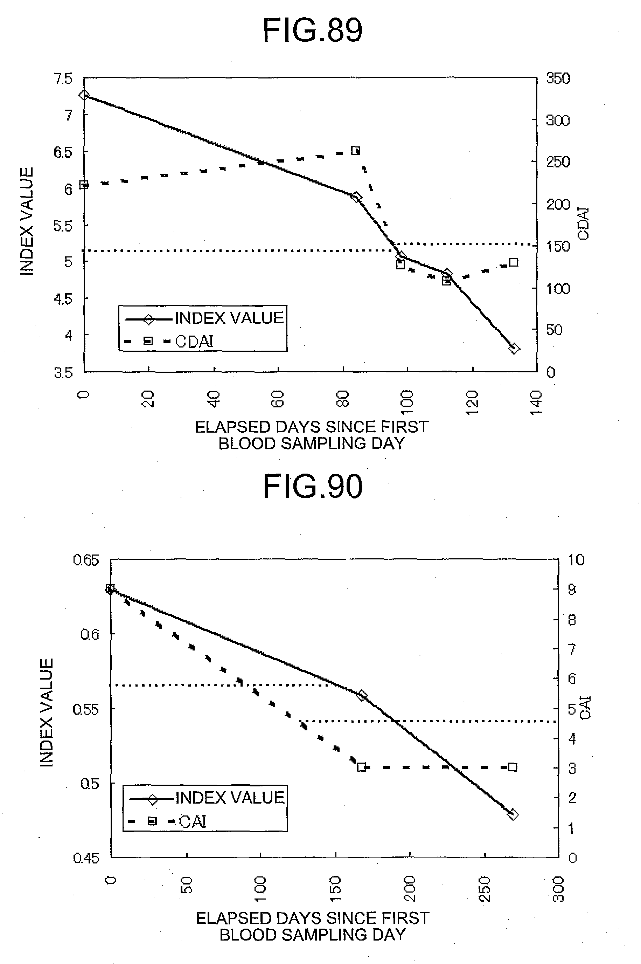

Using the data obtained from same subjects but on different blood sampling days among the samples measured in Example 1, the change over time in the values of the index formula 16 obtained in Example 4 that is useful for the discrimination between the 2 groups of the active phase Crohn disease group and the remission phase Crohn disease group, was compared with the change over time in the values of CDAI, which is a conventional activity index, and thereby an evaluation of the index formula 16 was performed (FIG. 89). In FIG. 89, the dotted line on the left side indicates the criterion value of the index formula 16 in the discrimination of the 2 groups of the active phase Crohn disease group and the remission phase Crohn disease group, and the dotted line on the right side indicates the criterion value of the CDAI in the discrimination of the 2 groups of the active phase Crohn disease group and the remission phase Crohn disease group.

While the values of CDAI are lower than the criterion value of 150 and undergo a transition to the level of the remission phase over the period from the second blood sampling day to the third blood sampling day, the values of the index formula 16 are also lower the criterion value of 5.102 and undergo a transition to the level of the remission phase. Thus, it was proved that the index formula 16 is a useful index even for the monitoring of the condition of Crohn disease in a same patient.

Example 7

Using the data obtained from same subjects but on different blood sampling days among the samples measured in Example 1, the change over time in the values of an index formula 33', which is equivalent to the index formula 33 obtained in Example 5 that is useful for the discrimination between the 2 groups of the active phase ulcerative colitis group and the remission phase ulcerative colitis group, was compared with the change over time in the values of CAI, which is a conventional activity index, and thereby an evaluation of the index formula 33' was performed (FIG. 90). Here, the values of the constant and coefficients in the index formula 33' are only exemplary after all, and are not limited to these. In FIG. 90, the dotted line on the left side indicates the criterion value of the index formula 33' in the discrimination of the 2 groups of the active phase ulcerative colitis group and the remission phase ulcerative colitis group, and the dotted line on the right side indicates the criterion value of the CAI in the discrimination of the 2 groups of the active phase ulcerative colitis group and the remission phase ulcerative colitis group.

```
Index formula 33': 2.4267-0.011723×His-0.0010018×Gln-

0.0001328×Urea-0.0056998×Glu
```

While the values of CAI are lower than the criterion value of 5 and undergo a transition to the level of the remission phase over the period from the first blood sampling day to the second blood sampling day, the values of the index formula 33' are also lower the criterion value of 5.562 and undergo a transition to the level of the remission phase. Thus, it was proved that the index formula 33' is a useful index even for the monitoring of the condition of ulcerative colitis in a same patient.

Example 8

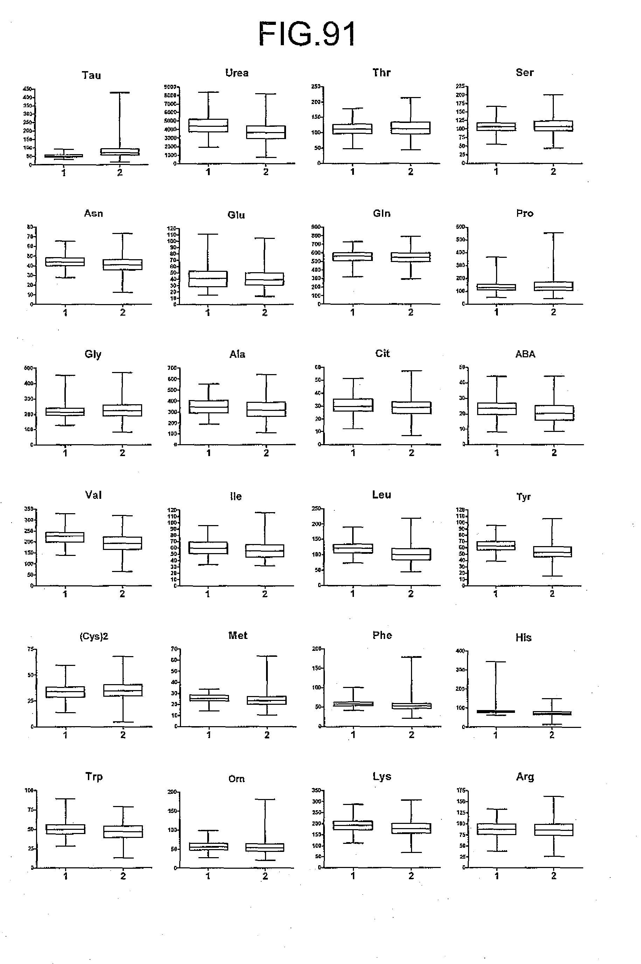

Blood samples obtained from a group of 409 inflammatory bowel disease patients determined as having inflammatory bowel disease, and blood samples of a group of 409 healthy subjects selected to match the group of inflammatory bowel disease patients in gender and age, were subjected to measurement of the amino acid concentration in blood by the amino acid analysis method. FIG. 91 is a boxplot showing the distributions of amino acid explanatory variables in the respective groups (1: healthy subject group, 2: inflammatory bowel disease group). In FIG. 91, the vertical axis indicates amino acid concentration, with the unit of the concentration being nmol/ml, and ABA and Cys in the figure represent $\alpha$-ABA ($\alpha$-aminobutyric acid)

and Cystine, respectively.

A t-test between 2 groups was performed for the purpose of discriminating between the 2 groups of the healthy subject group and the inflammatory bowel disease group. In the inflammatory bowel disease group as compared with the healthy subject group, Tau, Pro and Cys significantly increased (probability of significant difference $P<0.05$), and Urea, Asn, Ala, Cit, ABA, Val, Met, Ile, Leu, Tyr, Phe, His, Trp, Orn and Lys significantly decreased (probability of significant difference $P<0.05$). Thus, it was made clear that the amino acid explanatory variables Tau, Pro, Cys, Urea, Asn, Ala, Cit, ABA, Val, Met, Ile, Leu, Tyr, Phe, His, Trp, Orn and Lys have an ability to discriminate between the 2 groups of the healthy subject group and the inflammatory bowel disease group.

Example 9

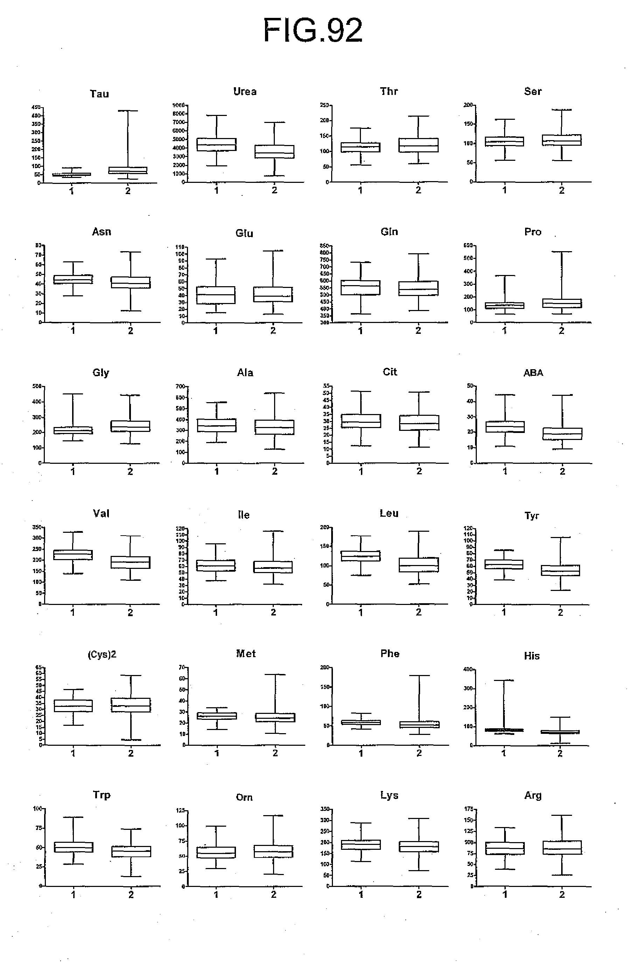

Blood samples obtained from a group of 152 Crohn disease patients determined as having Crohn disease based on the blood samples of Example 8, and blood samples of a group of 152 healthy subjects selected to match the group of Crohn disease patients in gender and age, were subjected to measurement of the amino acid concentration in blood by the amino acid analysis method. FIG. 92 is a boxplot showing the distributions of amino acid explanatory variables in the respective groups (1: healthy subject group, 2: Crohn disease group). In FIG. 92, the vertical axis indicates amino acid concentration, with the unit of the concentration being nmol/ml, and ABA and Cys in the figure represent $\alpha$-ABA ($\alpha$-aminobutyric acid) and Cystine, respectively.

A t-test between 2 groups was performed for the purpose of discriminating between the 2 groups of the healthy subject group and the Crohn disease group. In the Crohn disease group as compared with the healthy subject group, Tau, Thr, Pro and Gly significantly increased (probability of significant difference $P<0.05$), and Urea, Asn, ABA, Val, Leu, Tyr, Phe, His, Trp and Lys significantly decreased (probability of significant difference $P<0.05$). Thus, it was made clear that the amino acid explanatory variables Tau, Thr, Pro, Gly, Urea, Asn, ABA, Val, Leu, Tyr, Phe, His, Trp and Lys have an ability to discriminate between the 2 groups of the healthy subject group and the Crohn disease group.

Example 10

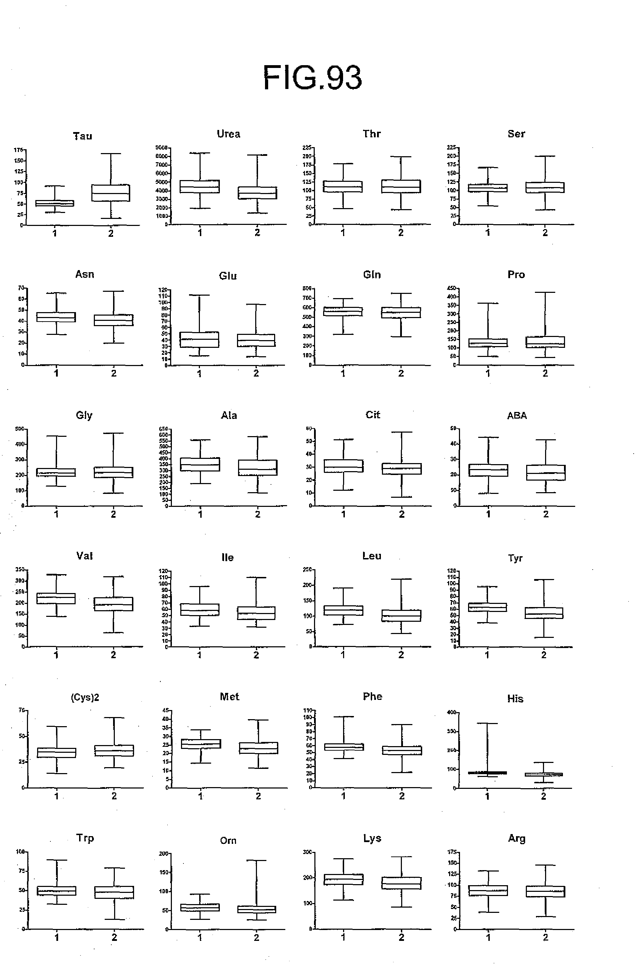

Blood samples obtained from a group of 257 ulcerative colitis patients determined as having ulcerative colitis based on the blood samples of Example 8, and blood samples of a group of 257 healthy subjects selected to match the group of ulcerative colitis patients in gender and age, were subjected to measurement of the amino acid concentration in blood by the amino acid analysis method. FIG. 93 is a boxplot showing the distributions of amino acid explanatory variables in the respective groups (1: healthy subject group, 2: ulcerative colitis group). In FIG. 93, the vertical axis indicates amino acid concentration, with the unit of the concentration being nmol/ml, and ABA and Cys in the figure represent $\alpha$-ABA ($\alpha$-aminobutyric acid) and Cystine, respectively.

A t-test between 2 groups was performed for the purpose of discriminating between the 2 groups of the healthy subject group and the ulcerative colitis group. In the ulcerative colitis group as compared with the healthy subject group, Tau and Cys significantly increased (probability of significant difference $P<0.05$), and Urea, Asn, Ala, Cit, ABA, Val, Met, Ile, Leu, Tyr, Phe, His, Trp, Orn and Lys significantly decreased (probability of significant difference $P<0.05$). Thus, it was made clear that the amino acid explanatory variables Tau, Cys, Urea, Asn, Ala, Cit, ABA, Val, Met, Ile, Leu, Tyr, Phe, His, Trp, Orn and Lys have an ability to discriminate between the 2 groups of the healthy subject group and the ulcerative colitis group.

Example 11

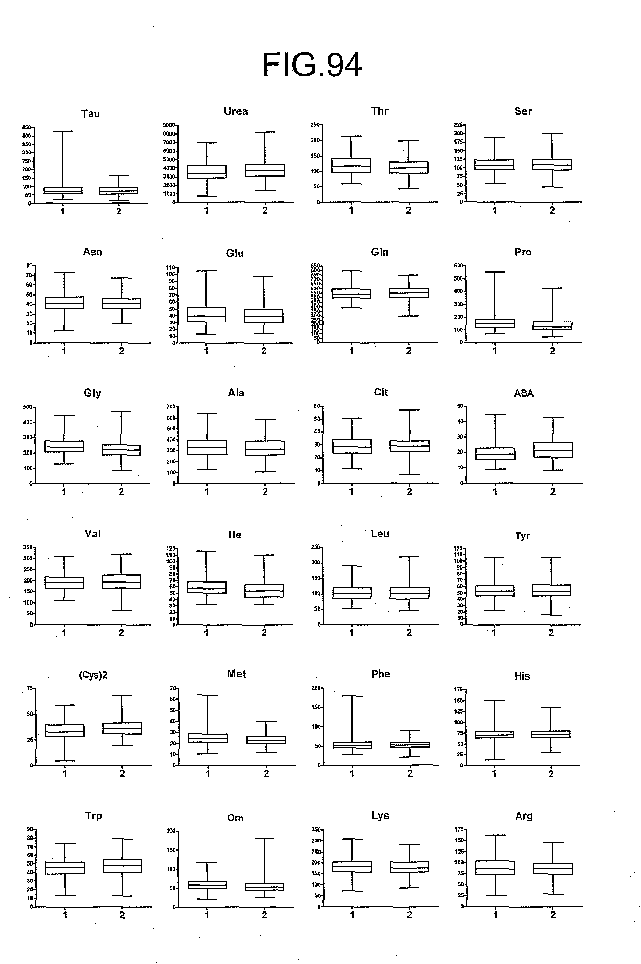

Blood samples obtained from a group of 152 Crohn disease patients determined as having Crohn disease and blood samples obtained from a group of 152 ulcerative colitis patients determined as having ulcerative colitis, all based on the blood samples of Example 8, were subjected to measurement of the amino acid concentration in blood by the amino acid analysis method. FIG. 94 is a boxplot showing the distributions of amino acid explanatory variables in the respective groups (1: Crohn disease group, 2: ulcerative colitis group). In FIG. 94, the vertical axis indicates amino acid concentration, with the unit of the concentration being nmol/ml, and ABA and Cys in the figure represent $\alpha$-ABA ($\alpha$-aminobutyric acid) and Cystine, respectively.

A t-test between 2 groups was performed for the purpose of discriminating between the 2 groups of the Crohn disease group and the ulcerative colitis group. In the Crohn disease group as compared with the ulcerative colitis group, Thr, Pro, Gly, ABA, Met, Ile and Orn significantly increased (probability of significant difference $P<0.05$), and ABA and Cys significantly decreased (probability of significant difference $P<0.05$). Thus, it was made clear that the amino acid explanatory variables Thr, Pro, Gly, Met, Ile, Orn, ABA and Cys have an ability to discriminate between the 2 groups of the Crohn disease group and the ulcerative colitis group.

Example 12

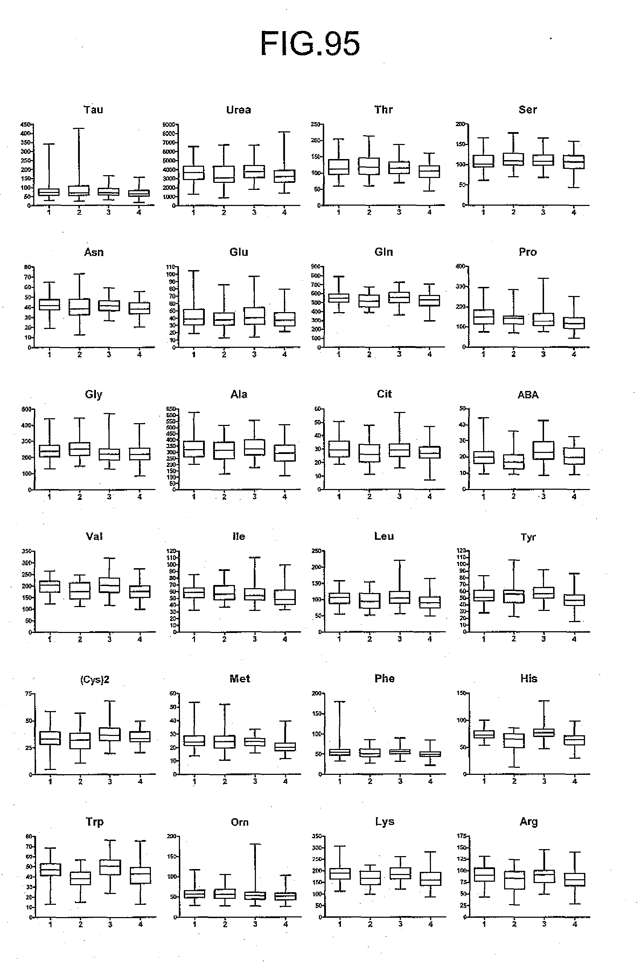

Based on the blood samples of Example 8, the Crohn disease group of Example 9 was further divided into a remission phase Crohn disease group and an active phase Crohn disease group, and the ulcerative colitis group of Example 10 was further divided into a remission phase ulcerative colitis group and an active phase ulcerative colitis group. FIG. 95 is a boxplot showing the distributions of amino acid explanatory variables in the respective groups (1: remission phase Crohn disease group, 2: active phase Crohn disease group, 3: remission phase ulcerative colitis group, 4: active phase ulcerative colitis group). The number of patients in each of the respective groups was 82 for the remission phase Crohn disease group, 34 for the active phase Crohn disease group, 114 for the remission phase ulcerative colitis group, and 60 for the active phase ulcerative colitis group. In FIG. 95, the vertical axis indicates amino acid concentration, with the unit of the concentration being nmol/ml, and ABA and Cys in the figure represent $\alpha$-ABA ($\alpha$-aminobutyric acid) and Cystine, respectively.

First, a t-test between 2 groups was performed for the purpose of discriminating between the 2 groups of the active phase Crohn disease group and the active phase ulcerative colitis group. In the active phase Crohn disease group as compared with the active phase ulcerative colitis group, Tau, Thr, Ser, Gly, Met and Ile significantly increased (probability of significant difference $P<0.05$). Thus, it was made clear that the amino acid explanatory variables Tau, Thr, Ser, Gly, Met and Ile have an ability to discriminate between the 2 groups of the active phase Crohn disease group and the active phase ulcerative colitis group.

Subsequently, a t-test between 2 groups was performed for the purpose of discriminating between the 2 groups of the active phase Crohn disease group and the remission phase Crohn disease group. In the active phase Crohn disease group as compared with the remission phase Crohn disease group, Gln, Cit, Val, Leu, His, Trp, Lys and Arg significantly decreased (probability of significant difference $P<0.05$). Thus, it was made clear that the amino acid explanatory variables Gln, Cit, Val, Leu, His, Trp, Lys and Arg have an ability to discriminate between the 2 groups of the active phase Crohn disease group and the remission phase Crohn disease group.

Subsequently, a t-test between 2 groups was performed for the purpose of discriminating between the 2 groups of the active phase ulcerative colitis group and the remission phase ulcerative colitis group. In the active phase ulcerative colitis group as compared with the remission phase ulcerative colitis group, Urea, Thr, Asn, Gln, Pro, Ala, Cit, ABA, Val, Met, Leu, Tyr, Phe, His, Trp, Lys and Arg significantly decreased (probability of significant difference $P<0.05$). Thus, it was made clear that the amino acid explanatory variables Urea, Thr, Asn, Gln, Pro, Ala, ABA, Val, Met, Leu, Tyr, Phe, His, Trp, Lys and Arg have an ability to discriminate between the 2 groups of the active phase ulcerative colitis group and the remission phase ulcerative colitis group.

Example 13

The sample data used in Example 8 were used. Using a method described in International Publication WO 2004/052191 that is an international application filed by the present applicant, first, an index by which the performance of discriminating between the 2 groups of the healthy subject group and the inflammatory bowel disease group is maximized was eagerly searched, and an index formula 35 was obtained among a plurality of indices having an equivalent performance.

```
Index formula 35: (Tau)/(Tyr)+(Thr+Arg)/(Cit+His)
```

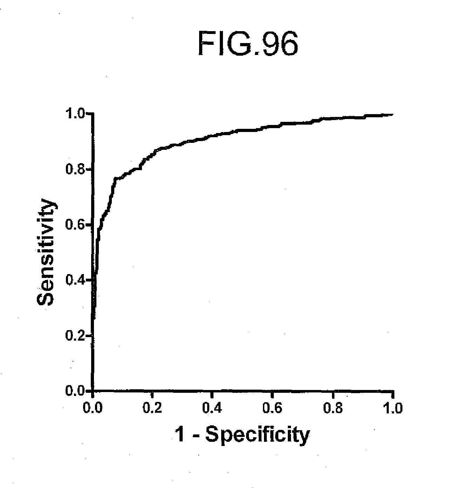

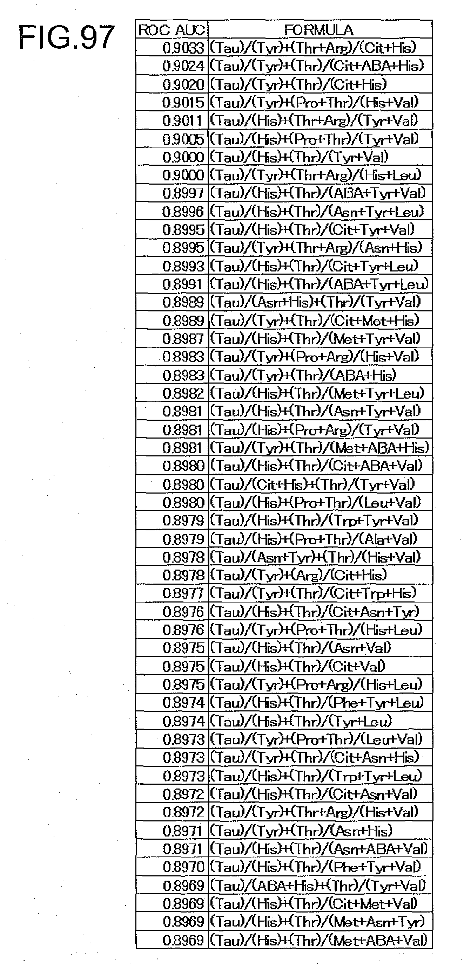

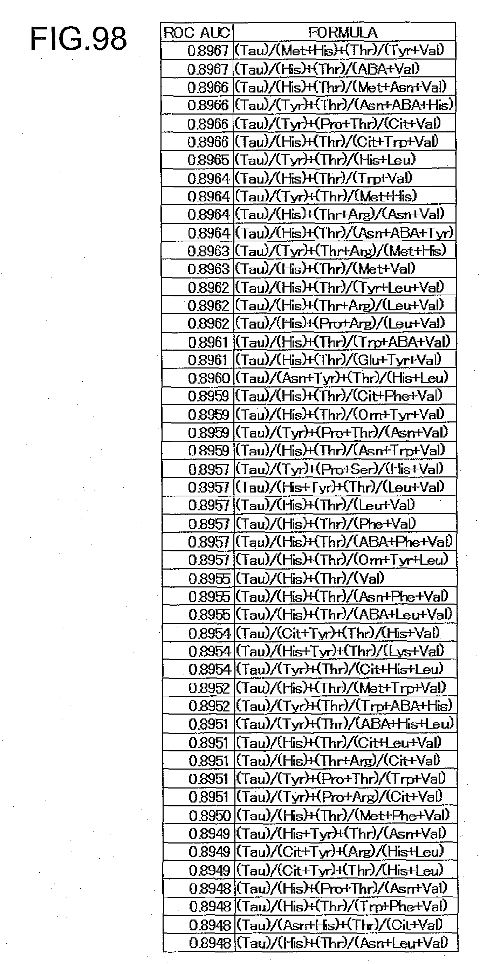

An evaluation was performed on the discrimination between the 2 groups of the healthy subject group and the inflammatory bowel disease group based on the index formula 35, on the basis of the AUC of the ROC curve (FIG. 96), and an AUC of 0.903±0.011 (95% confidence interval: 0.881 to 0.925) was obtained. Thus, the index formula 35 was found to be a useful index with high diagnostic performance. In addition to that, a plurality of fractional expressions having a discrimination performance equivalent to that of the index formula 35 was obtained. Those fractional expressions are presented in FIG. 97 and FIG. 98.

Example 14

The sample data used in Example 9 were used. An index by which the performance of discriminating between the 2 groups of the healthy subject group and the Crohn disease group is maximized was eagerly searched, and an index formula 36 was obtained among a plurality of indices having an equivalent performance.

```
Index formula 36: (Tau)/(Tyr)+(Pro+Orn+Arg)/(Val)
```

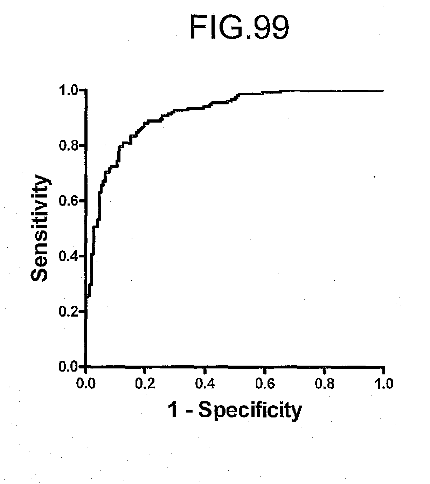

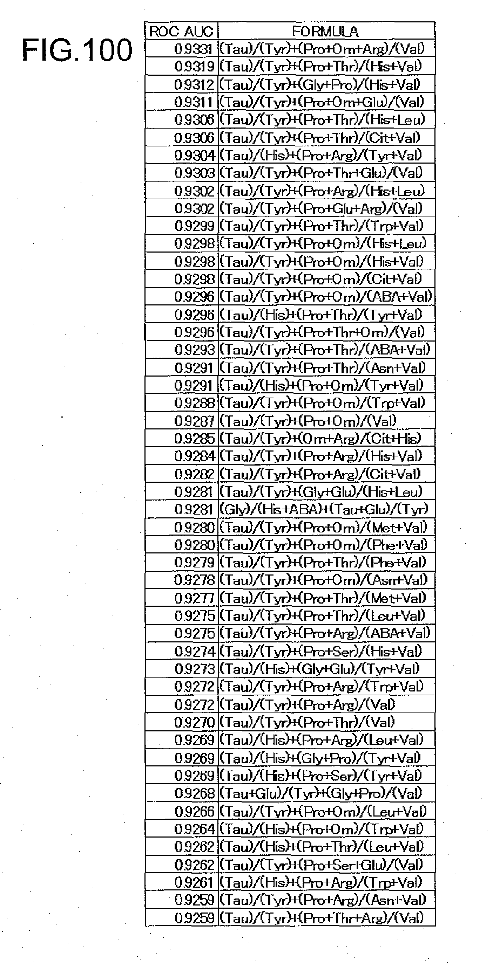

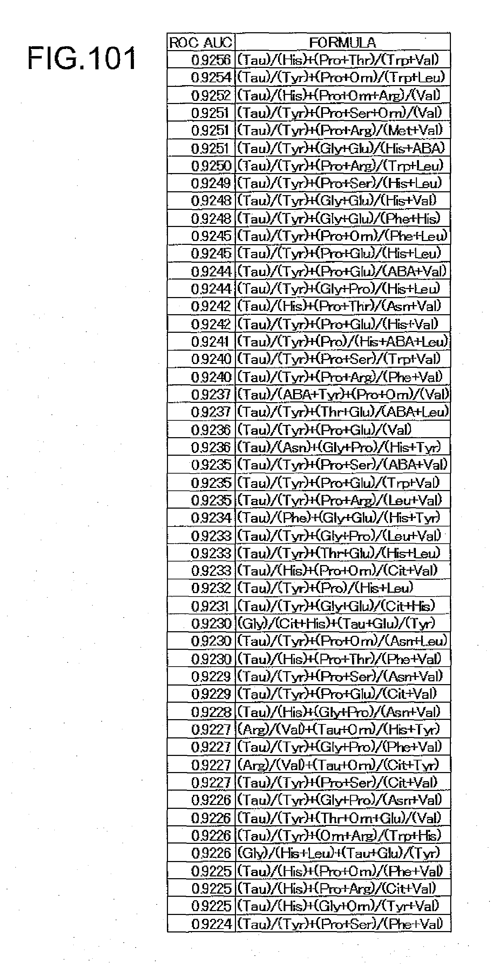

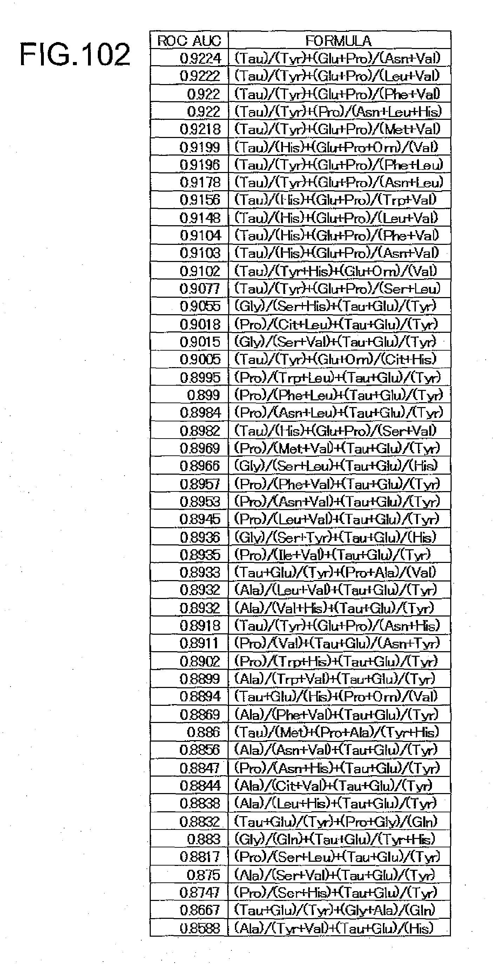

An evaluation was performed on the discrimination between the 2 groups of the healthy subject group and the Crohn disease group based on the index formula 36, on the basis of the AUC of the ROC curve (FIG. 99), and an AUC of 0.933±0.015 (95% confidence interval: 0.904 to 0.962) was obtained. Thus, the index formula 36 was found to be a useful index with high diagnostic performance. In addition to that, a plurality of fractional expressions having a discrimination performance equivalent to that of the index formula 36 was obtained. Those fractional expressions are presented in FIG. 100, FIG. 101 and FIG. 102.

Example 15

The sample data used in Example 10 were used. An index by which the performance of discriminating between the 2 groups of the healthy subject group and the ulcerative colitis group is maximized was eagerly searched, and an index formula 37 was obtained among a plurality of indices having an equivalent performance.

```
Index formula 37: (Tau)/(Tyr)+(Thr)/(Cit+His)
```

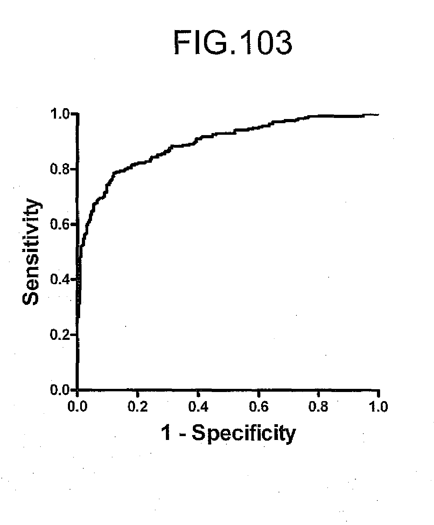

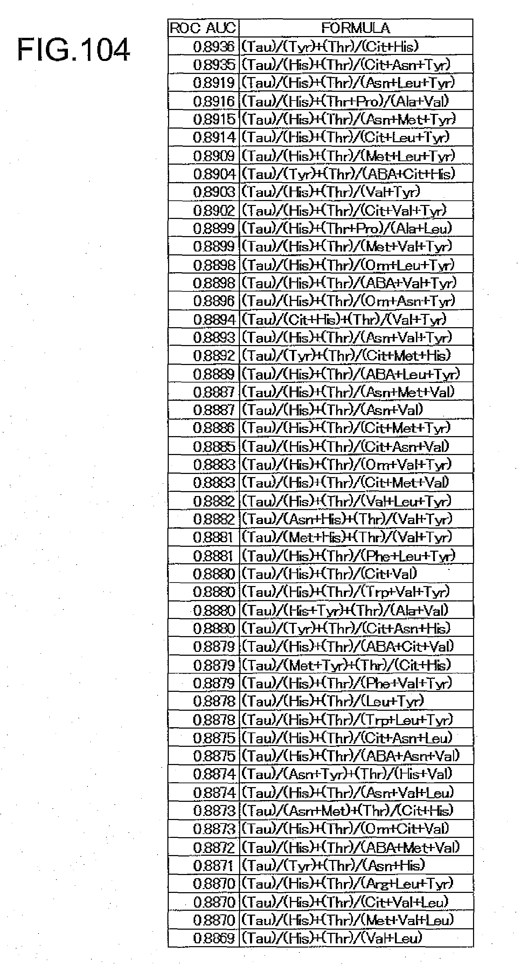

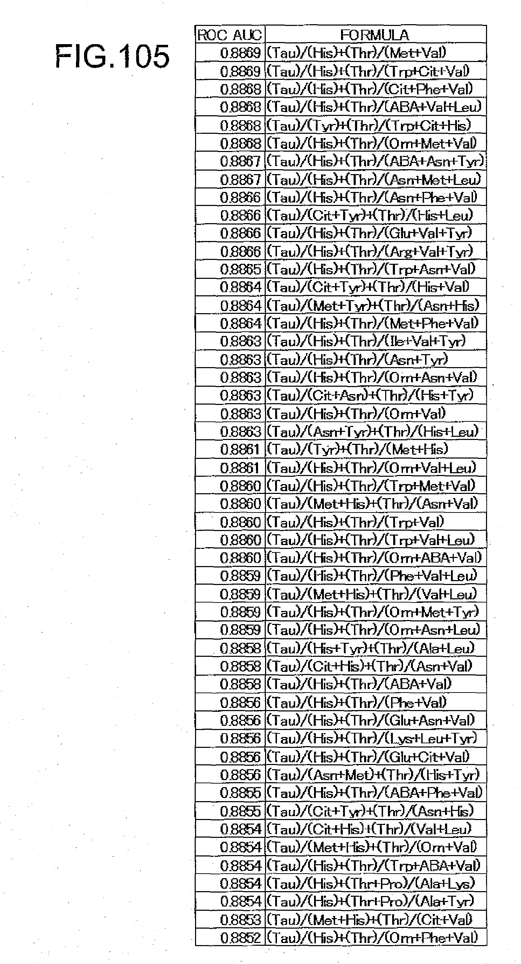

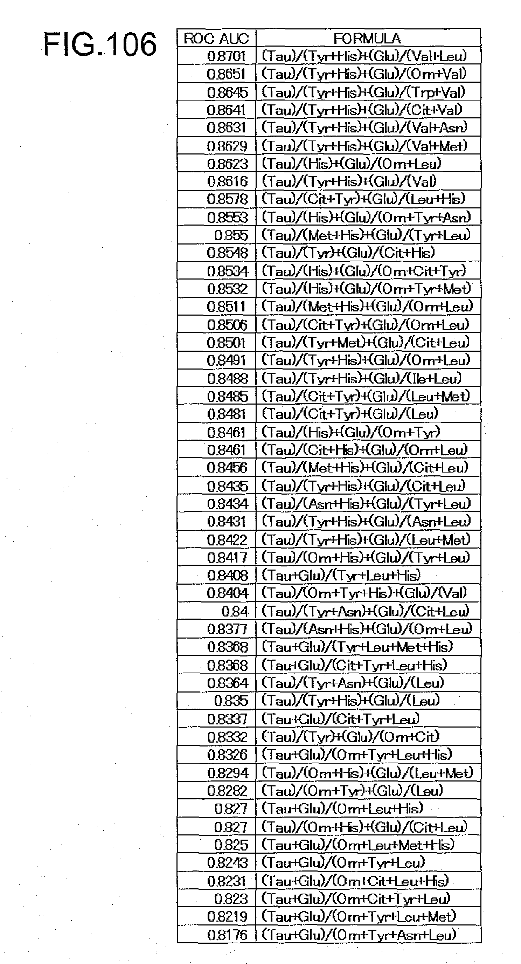

An evaluation was performed on the discrimination between the 2 groups of the healthy subject group and the ulcerative colitis group based on the index formula 37, on the basis of the AUC of the ROC curve (FIG. 103), and an AUC of 0.894±0.014 (95% confidence interval: 0.866 to 0.922) was obtained. Thus, the index formula 37 was found to be a useful index with high diagnostic performance. In addition to that, a plurality of fractional expressions having a discrimination performance equivalent to that of the index formula 37 was obtained. Those fractional expressions are presented in FIG. 104, FIG. 105 and FIG. 106.

Example 16

The sample data used in Example 8 were used. An index by which the performance of discriminating between the 2 groups of the Crohn disease group and the ulcerative colitis group is maximized was eagerly searched, and an index formula 38 was obtained among a plurality of indices having an equivalent performance.

```
Index formula 38: (Met+Ile)/(Val+Trp+ABA)
```

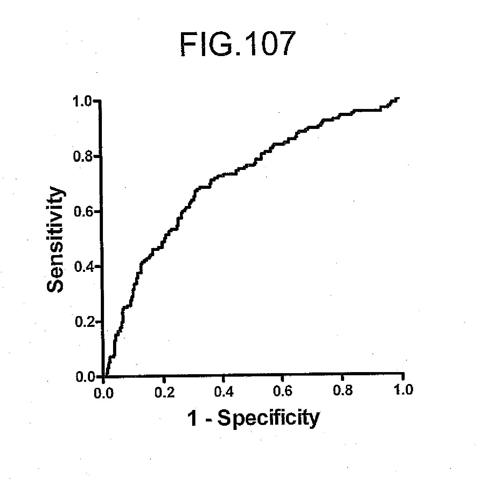

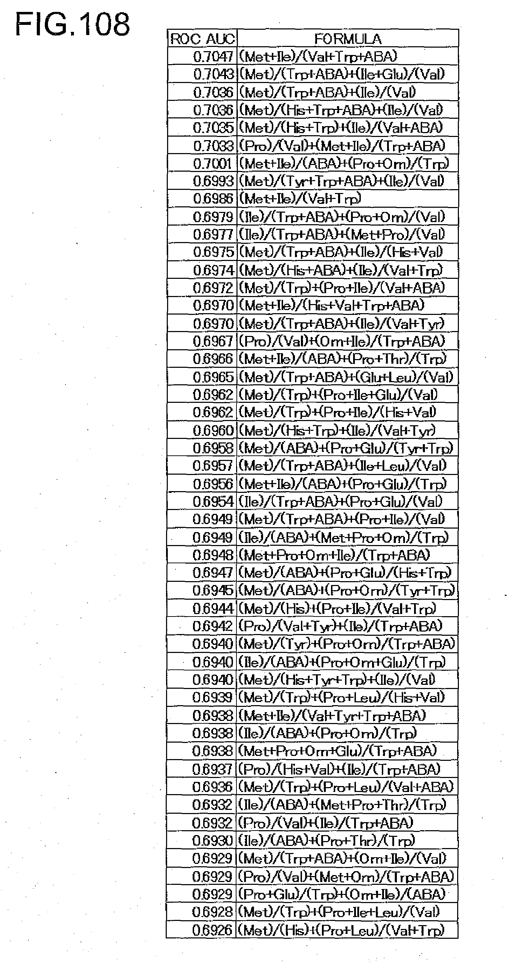

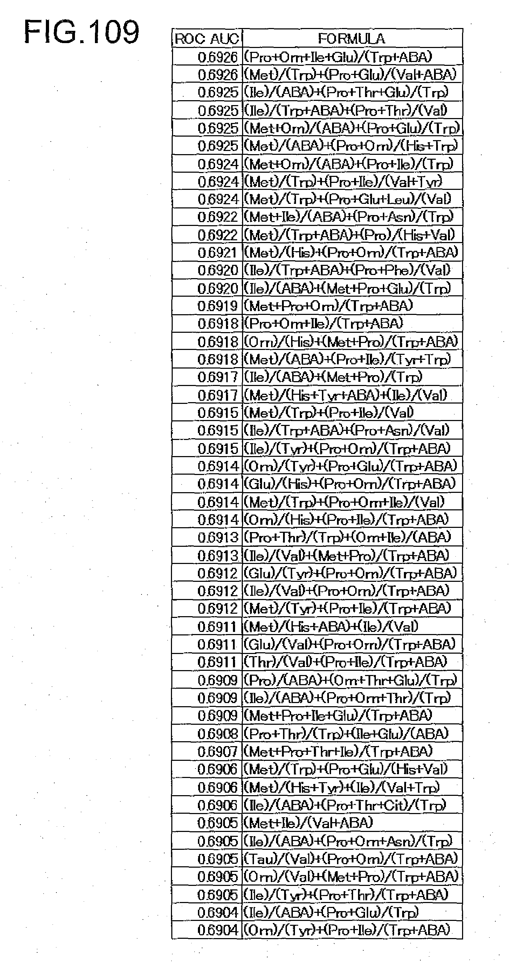

An evaluation was performed on the discrimination between the 2 groups of the Crohn disease group and the ulcerative colitis group based on the index formula 38, on the basis of the AUC of the ROC curve (FIG. 107), and an AUC of 0.705±0.027 (95% confidence interval: 0.651 to 0.759) was obtained. Thus, the index formula 38 was found to be a useful index with high diagnostic performance. In addition to that, a plurality of fractional expressions having a discrimination performance equivalent to that of the index formula 38 was obtained. Those fractional expressions are presented in FIG. 108 and FIG. 109.

Subsequently, an index by which the performance of discriminating between the 2 groups of the active phase Crohn disease group and the active phase ulcerative colitis group is maximized was eagerly searched, and an index formula 39 was obtained among a plurality of indices having an equivalent performance.

```
Index formula 39: (Thr)/(Val)+(Met+Tyr)/(ABA+Trp)
```

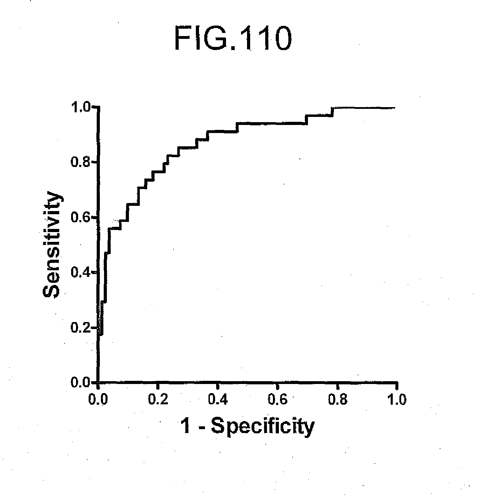

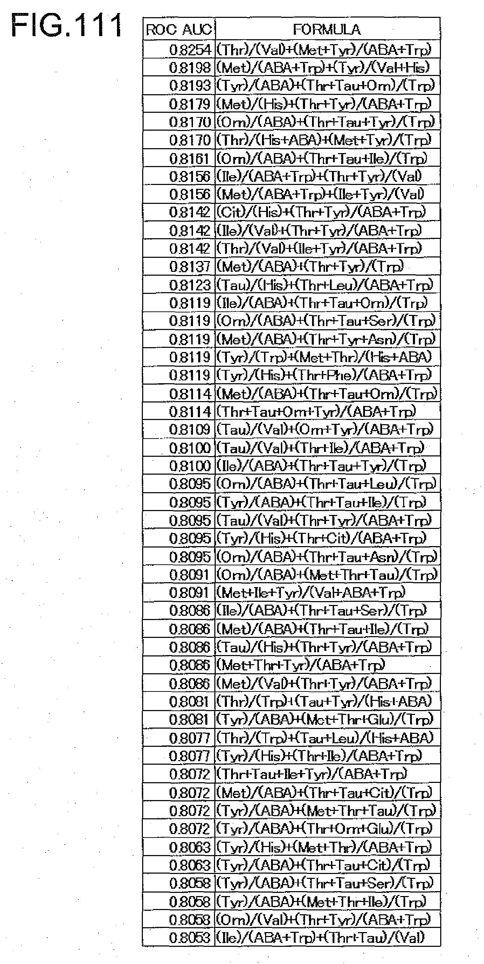

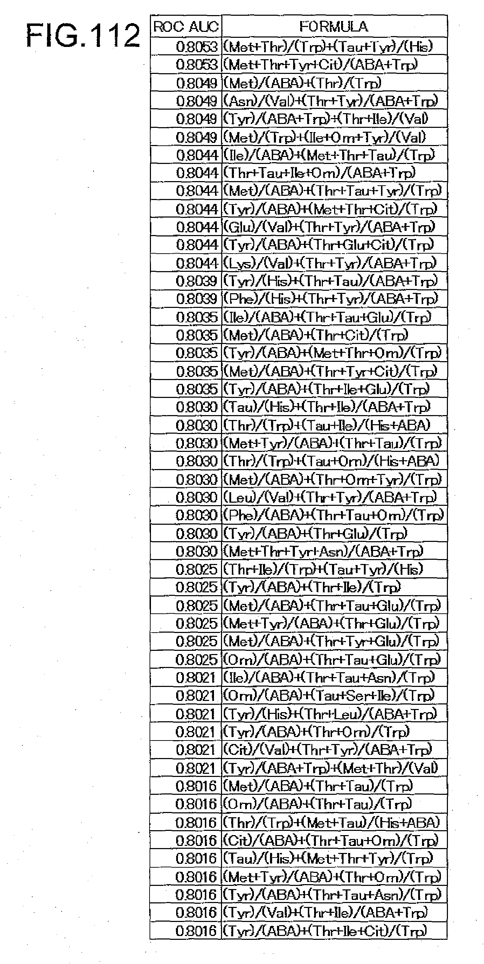

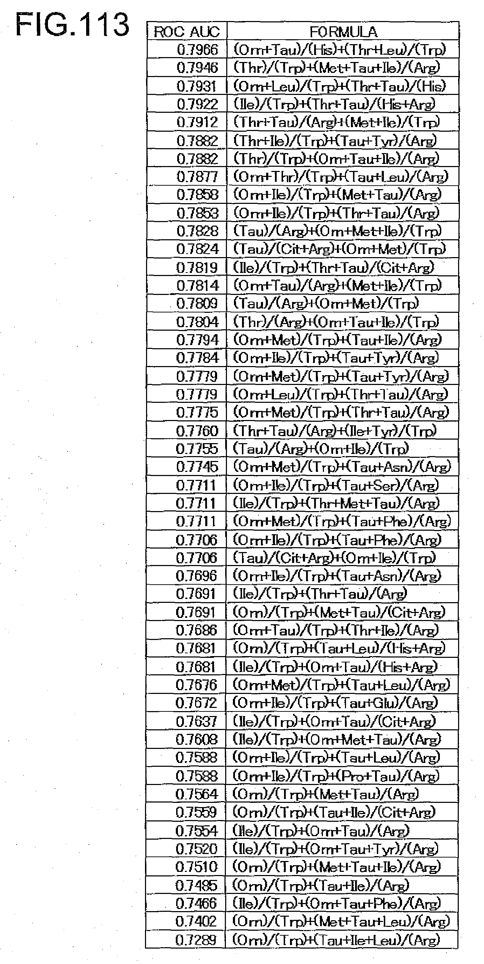

An evaluation was performed on the discrimination between the 2 groups of the active phase Crohn disease group and the active phase ulcerative colitis group based on the index formula 39, on the basis of the AUC of the ROC curve (FIG. 110), and an AUC of $0.825\pm0.048$ (95% confidence interval: 0.731 to 0.919) was obtained. Thus, the index formula 39 was found to be a useful index with high diagnostic performance. In addition to that, a plurality of fractional expressions having a discrimination performance equivalent to that of the index formula 39 was obtained. Those fractional expressions are presented in FIG. 111, FIG. 112 and FIG. 113.

Subsequently, an index by which the performance of discriminating between the 2 groups of the active phase Crohn disease group and the remission phase Crohn disease group is maximized was eagerly searched, and an index formula 40 was obtained among a plurality of indices having an equivalent performance.

```
Index formula 40: (Tau)/(Gln)+(Ser+Ile)/(Trp+His)
```

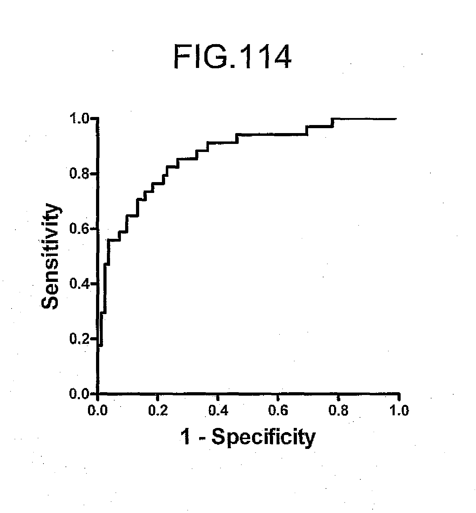

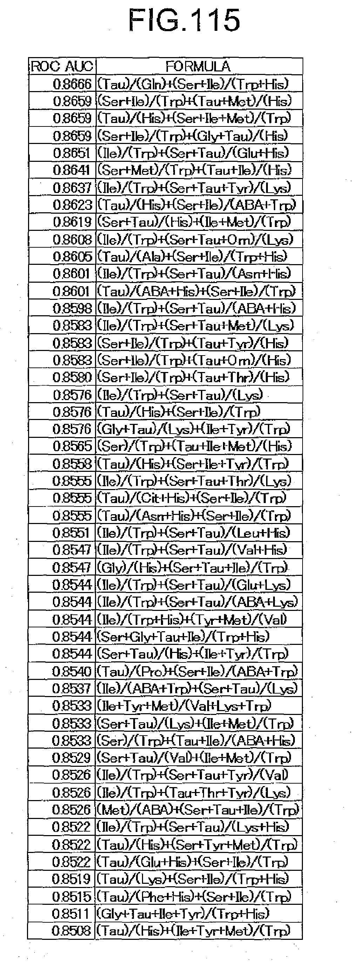

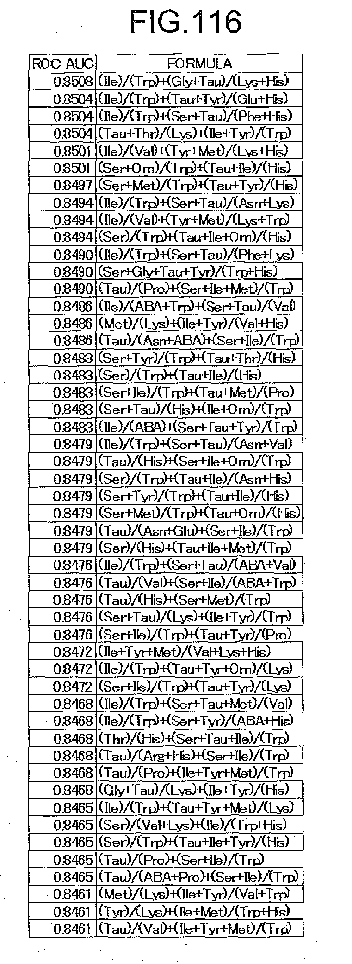

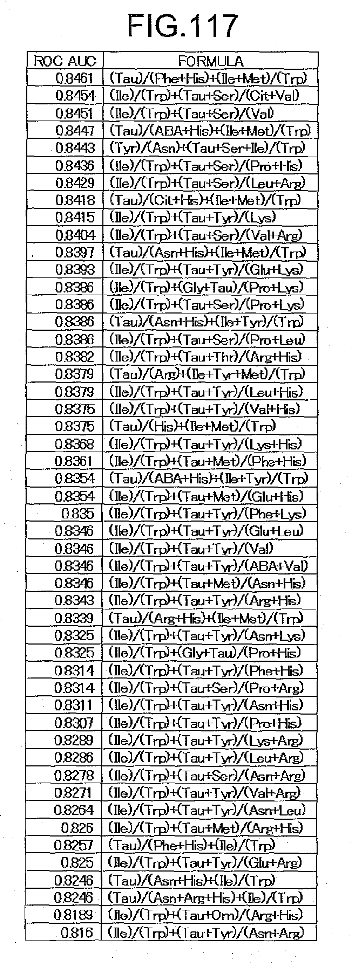

An evaluation was performed on the discrimination between the 2 groups of the active phase Crohn disease group and the remission phase Crohn disease group based on the index formula 40, on the basis of the AUC of the ROC curve (FIG. 114), and an AUC of $0.867\pm0.042$ (95% confidence interval: 0.785 to 0.949) was obtained. Thus, the index formula 40 was found to be a useful index with high diagnostic performance. In addition to that, a plurality of fractional expressions having a discrimination performance equivalent to that of the index formula 40 was obtained. Those fractional expressions are presented in FIG. 115, FIG. 116 and FIG. 117.

Subsequently, an index by which the performance of discriminating between the 2 groups of the active phase ulcerative colitis group and the remission phase ulcerative colitis group is maximized was eagerly searched, and an index formula 41 was obtained among a plurality of indices having an equivalent performance.

```
Index formula 41: (Orn)/(Trp+His)
```

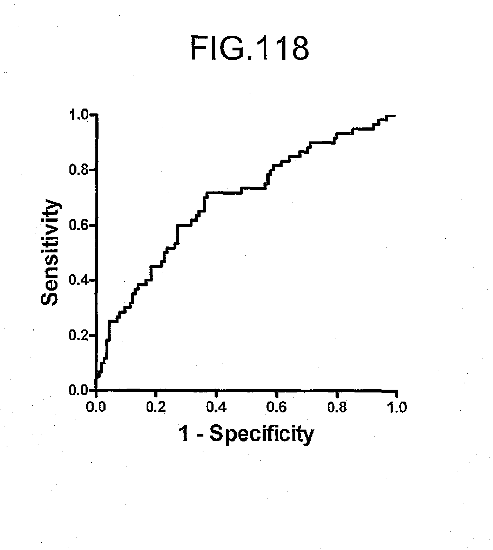

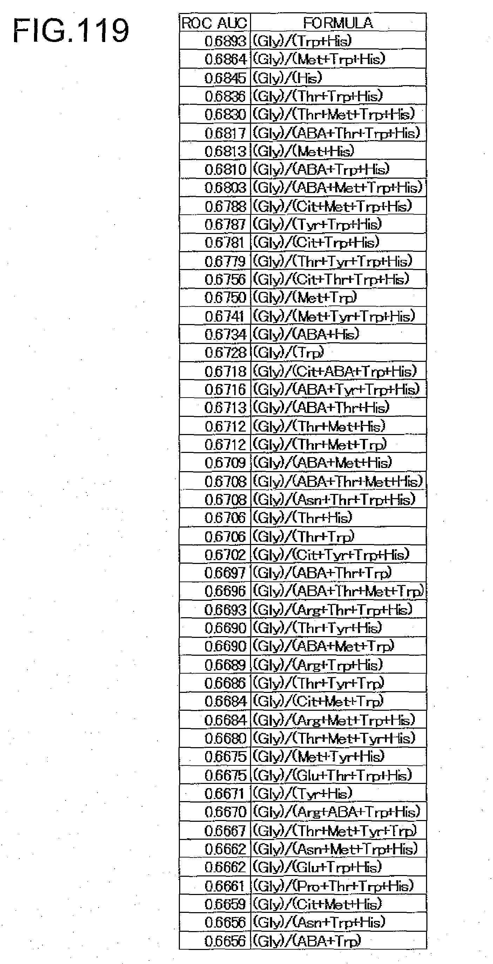

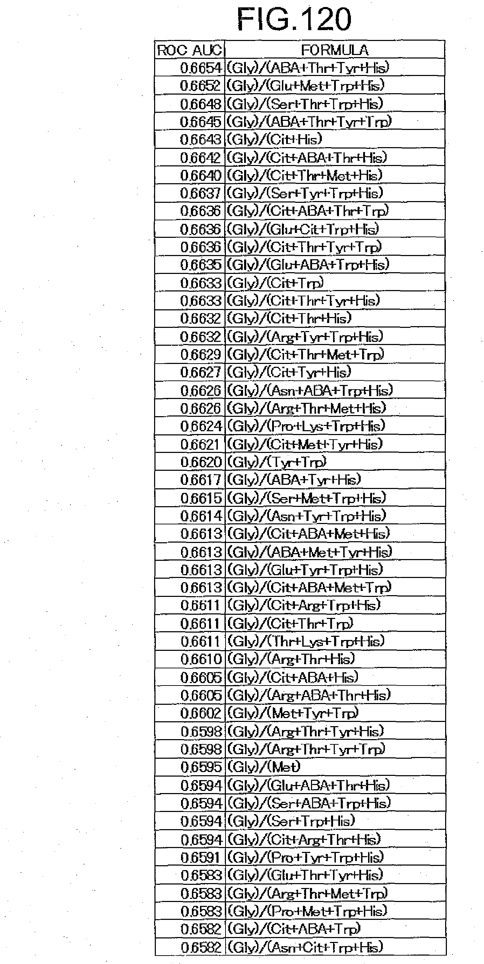

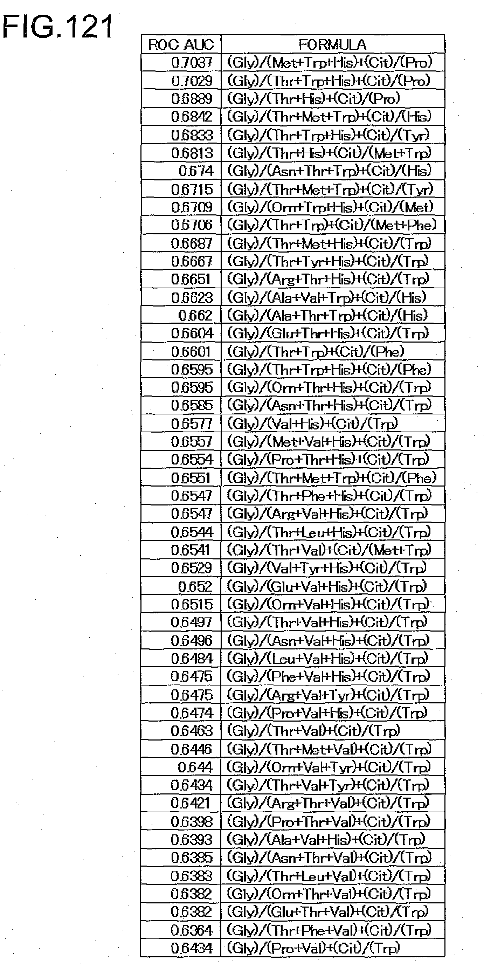

An evaluation was performed on the discrimination between the 2 groups of the active phase ulcerative colitis group and the remission phase ulcerative colitis group based on the index formula 41, on the basis of the AUC of the ROC curve (FIG. 118), and an AUC of $0.689\pm0.044$ (95% confidence interval: 0.603 to 0.775) was obtained. Thus, the index formula 41 was found to be a useful index with high diagnostic performance. In addition to that, a plurality of fractional expressions having a discrimination performance equivalent to that of the index formula 41 was obtained. Those fractional expressions are presented in FIG. 119, FIG. 120 and FIG. 121.

Example 17

The sample data used in Example 8 were used. Using a method (method for searching a multivariate discriminant) described in International Publication WO 2006/098192 that is an international application filed by the present applicant, first, an index by which the performance of discriminating between the 2 groups of the healthy subject group and the inflammatory bowel disease group is maximized was eagerly searched by linear discriminant analysis, and an index formula 42 was obtained among a plurality of indices having an equivalent performance.

```
Index formula 42: 0.8206+0.0061×Tau+0.0021×Thr+0.0176×Ile-

0.0111×Leu-0.0085×Tyr-0.0031×His
```

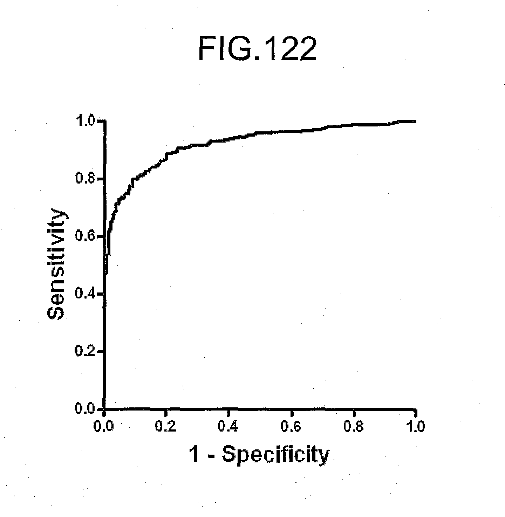

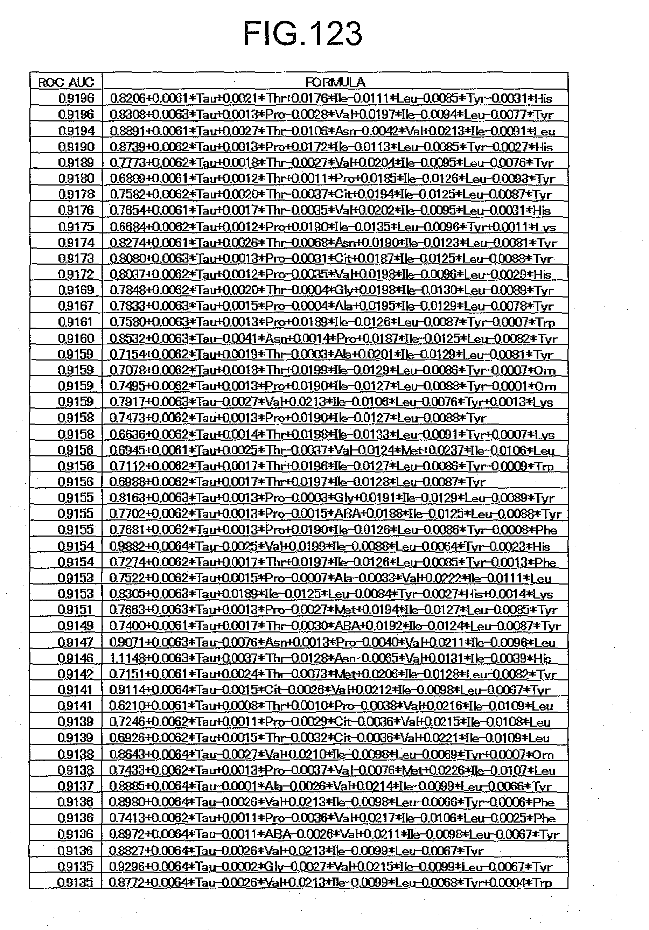

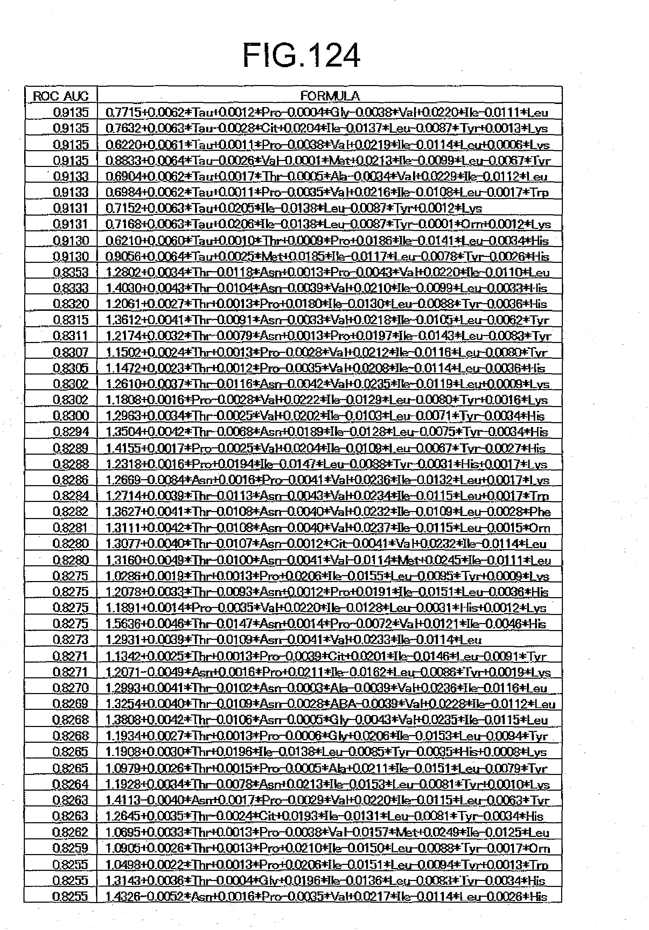

An evaluation was performed on the discrimination between the 2 groups of the healthy subject group and the inflammatory bowel disease group based on the index formula 42, on the basis of the AUC of the ROC curve (FIG. 122), and an AUC of $0.916\pm0.010$ (95% confidence interval: 0.896 to 0.936) was obtained. Thus, the index formula 42 was found to be a useful index with high diagnostic performance. In addition to that, a plurality of linear discriminants having a discrimination performance equivalent to that of the index formula 42 was obtained. Those linear discriminants are presented in FIG. 123 and FIG. 124. The discriminants presented in FIG. 123 and FIG. 124 may be discriminants obtained by transformation of monotonic increase or monotonic decrease, such as linear transformation such as addition of a constant number or multiplication of a constant number, and logit transformation.

Subsequently, an index by which the performance of discriminating between the 2 groups of the healthy subject group and the inflammatory bowel disease group is maximized was eagerly searched by logistic regression analysis, and an index formula 43 was obtained among a plurality of indices having an equivalent performance.

```
Index formula 43: 1/(1+exp(-x))

provided that x=0.3249+0.1049×Tau+0.0102×Pro-

0.0254×Val+0.2065×Ile-0.0994×Leu-0.0612×Tyr
```

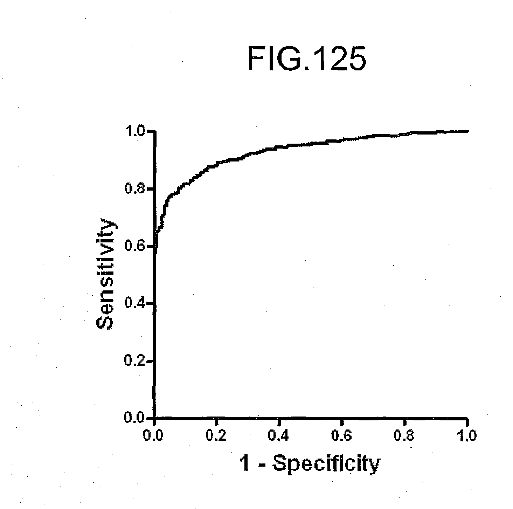

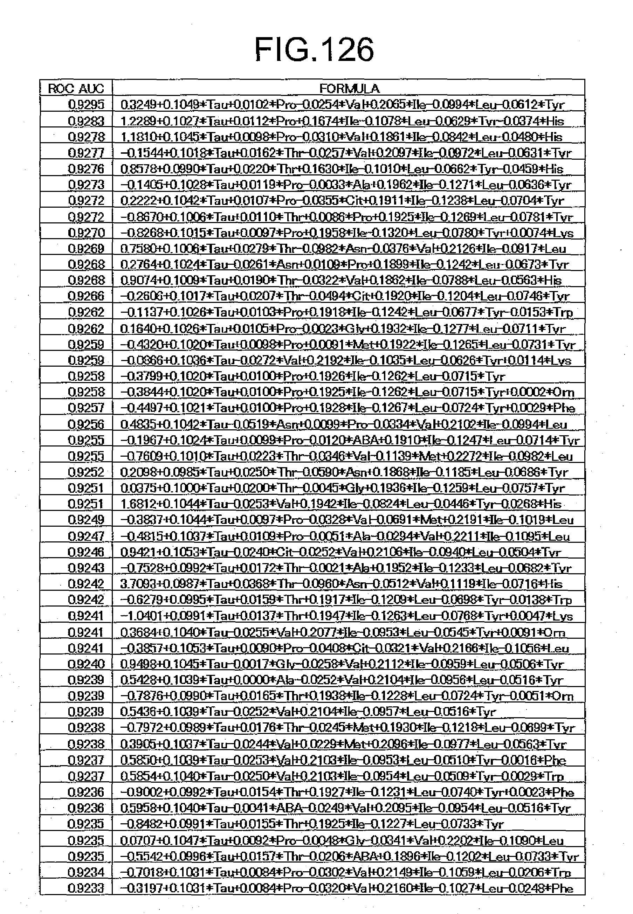

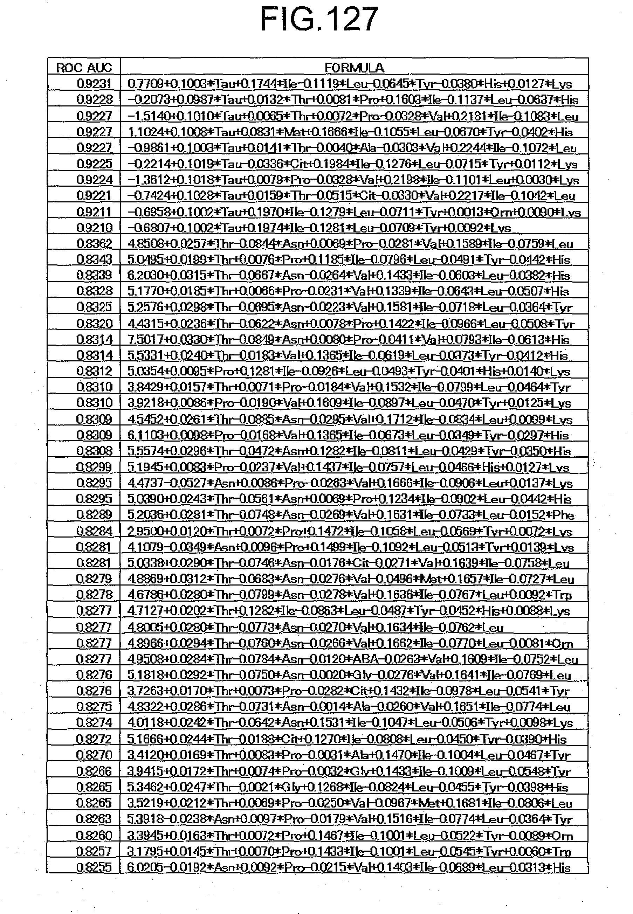

An evaluation was performed on the discrimination between the 2 groups of the healthy subject group and the inflammatory bowel disease group based on the index formula 43, on the basis of the AUC of the ROC curve (FIG. 125), and an AUC of $0.930\pm0.009$ (95% confidence interval: 0.912 to 0.948) was obtained. Thus, the index formula 43 was found to be a useful index with high diagnostic performance. In addition to that, a plurality of logistic regression equations having a discrimination performance equivalent to that of the index formula 43 was obtained. Those x's are presented in FIG. 126 and FIG. 127. The equations presented in FIG. 126 and FIG. 127 may be equations obtained by transformation of monotonic increase or monotonic decrease, such as linear transformation such as addition of a constant number or multiplication of a constant number, and logit transformation.

Example 18

The sample data used in Example 9 were used. An index by which the performance of discriminating between the 2 groups of the healthy subject group and the Crohn disease group is maximized was eagerly searched by linear discriminant analysis, and an index formula 44 was obtained among a plurality of indices having an equivalent performance.

```
Index formula 44: 0.9579+0.0040×Tau+0.0022×Pro-

0.0046×Val+0.0212×Ile-0.0082×Leu-0.0078×Tyr
```

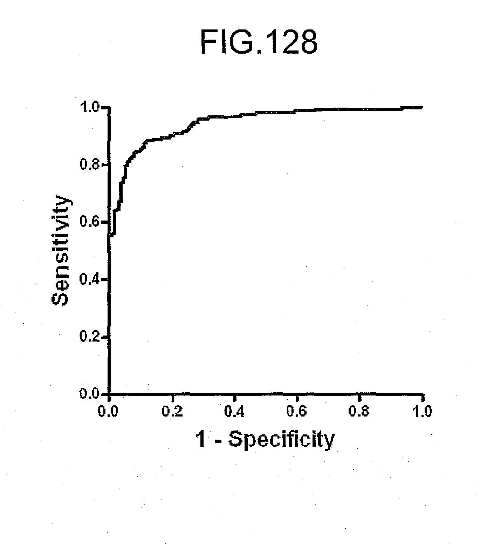

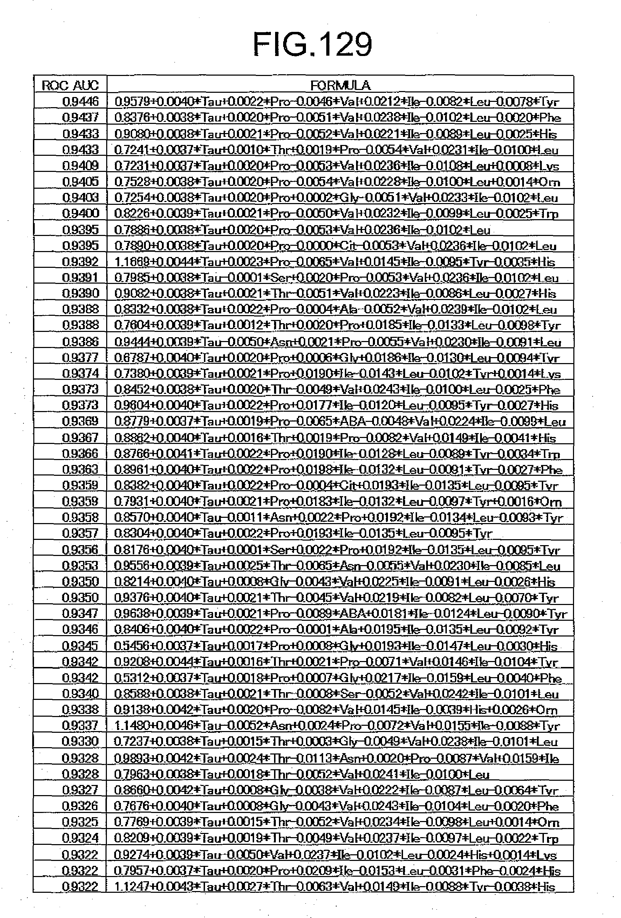

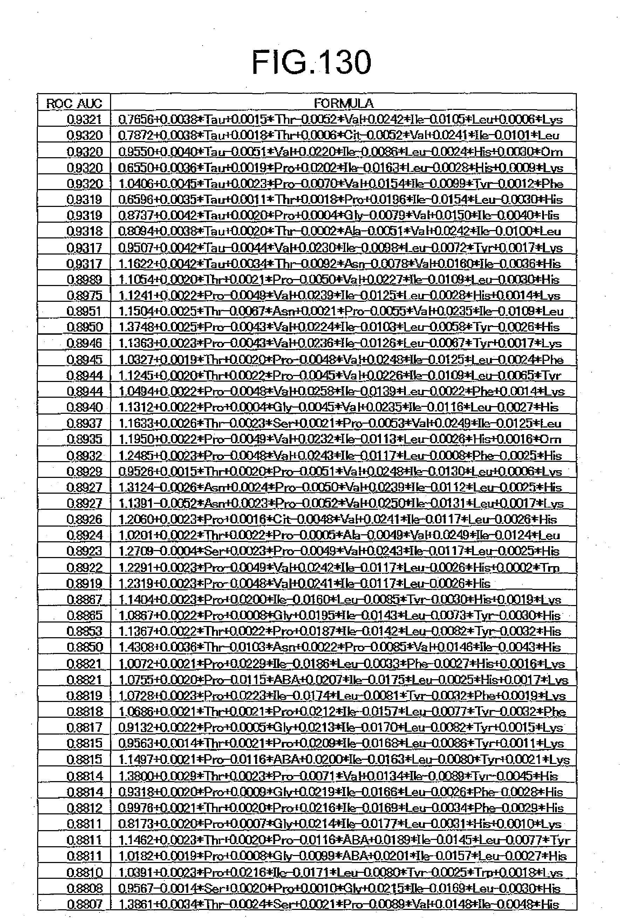

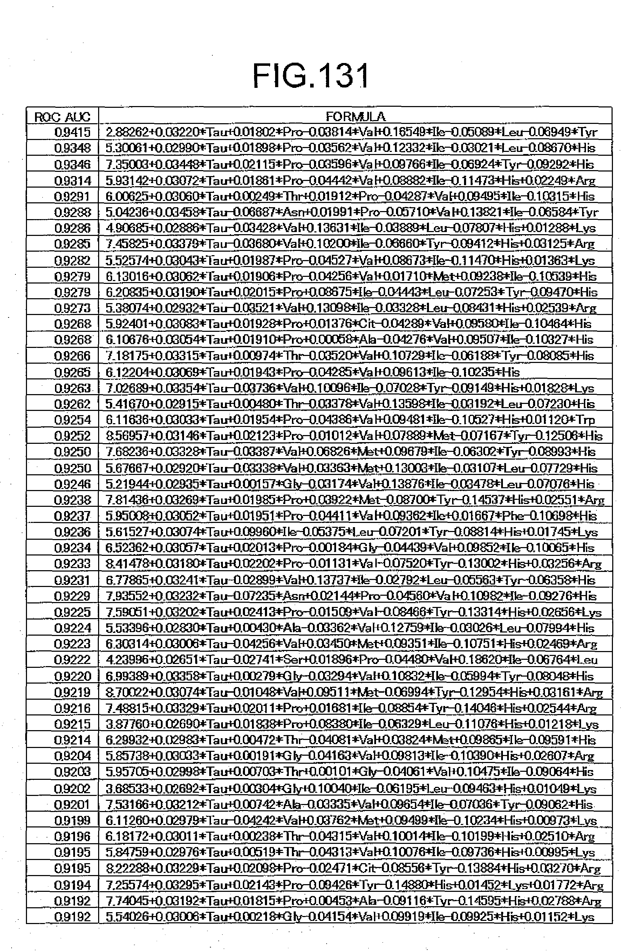

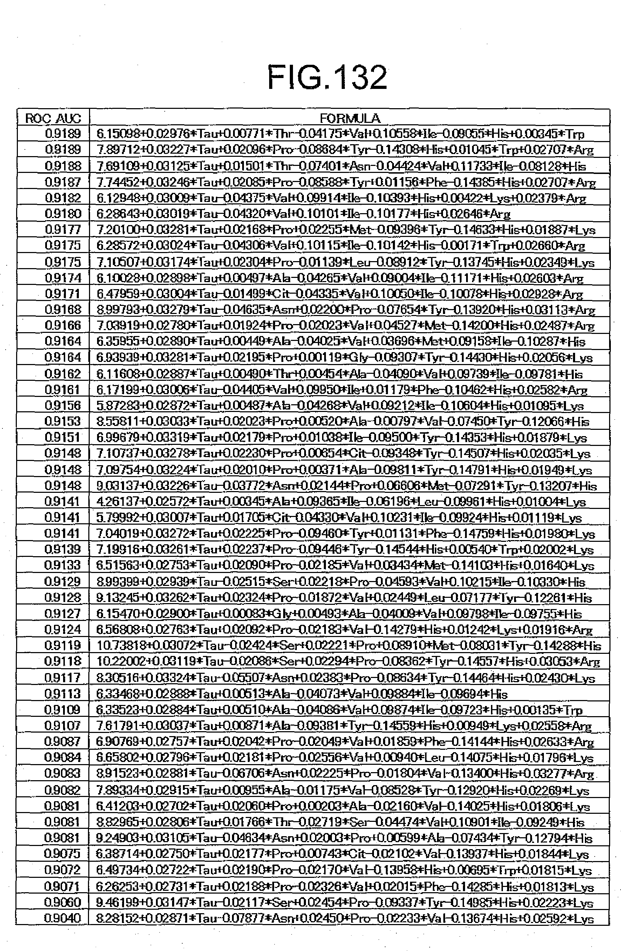

An evaluation was performed on the discrimination between the 2 groups of the healthy subject group and the Crohn disease group based on the index formula 44, on the basis of the AUC of the ROC curve (FIG. 128), and an AUC of $0.945\pm0.014$ (95% confidence interval: 0.918 to 0.972) was obtained. Thus, the index formula 44 was found to be a useful index with high diagnostic performance. In addition to that, a plurality of linear discriminants having a discrimination performance equivalent to that of the index formula 44 was obtained. Those discriminants are presented in FIG. 129, FIG. 130, FIG. 131 and FIG. 132. The discriminants presented in FIG. 129, FIG. 130, FIG. 131 and FIG. 132 may be discriminants obtained by transformation of monotonic increase or monotonic decrease, such as linear transformation such as addition of a constant number or multiplication of a constant number, and logit transformation.

Subsequently, an index by which the performance of discriminating between the 2 groups of the healthy subject group and the Crohn disease group is maximized was eagerly searched by logistic regression analysis, and an index formula 45 was obtained among a plurality of indices having an equivalent performance.

```
Index formula 45: 1/(1+exp(-x))

provided that x=2.4178+0.1251×Tau+0.0182×Pro-

0.0534×Val+0.2866×Ile-0.1248×Leu-0.0544×His
```

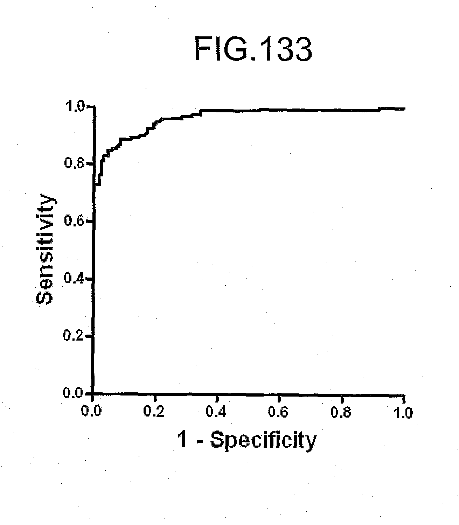

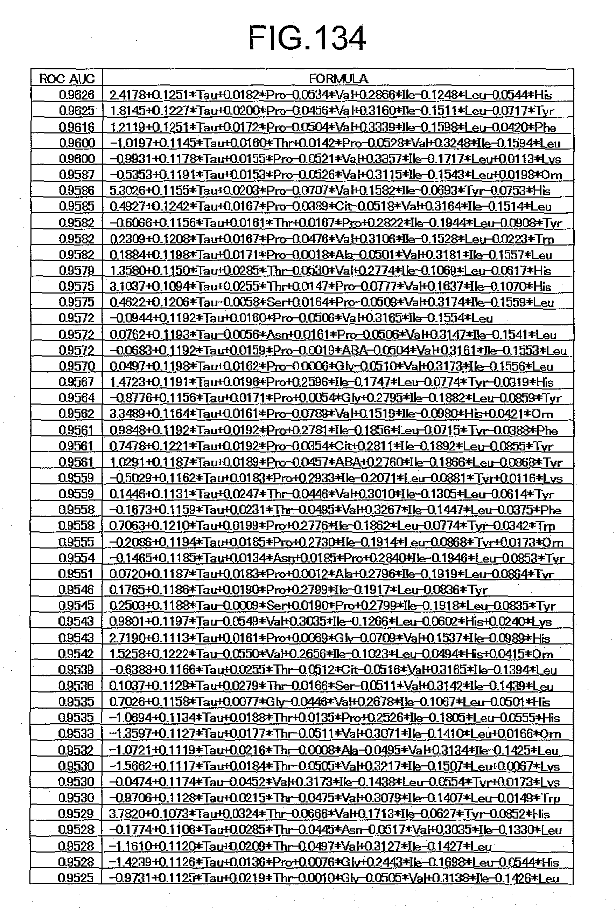

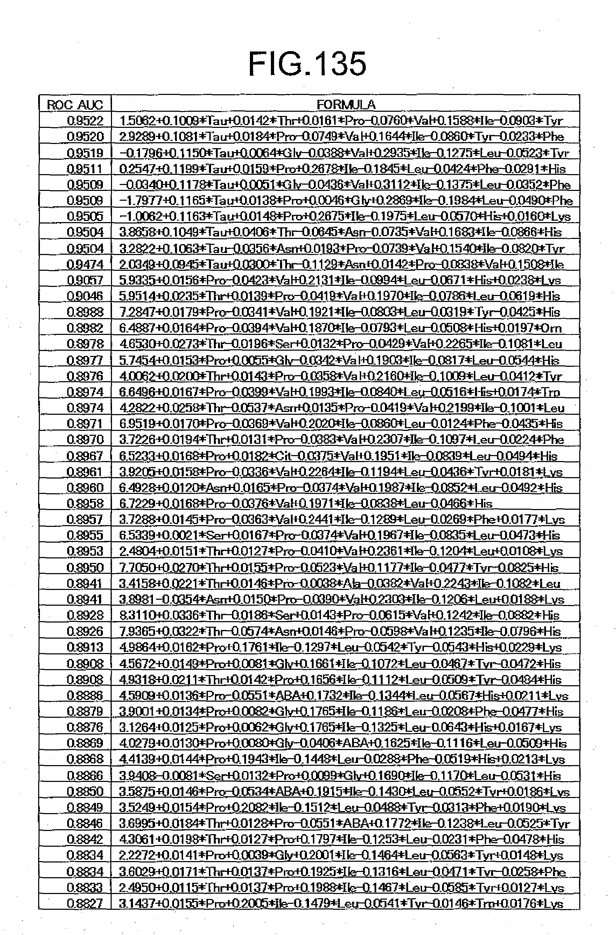

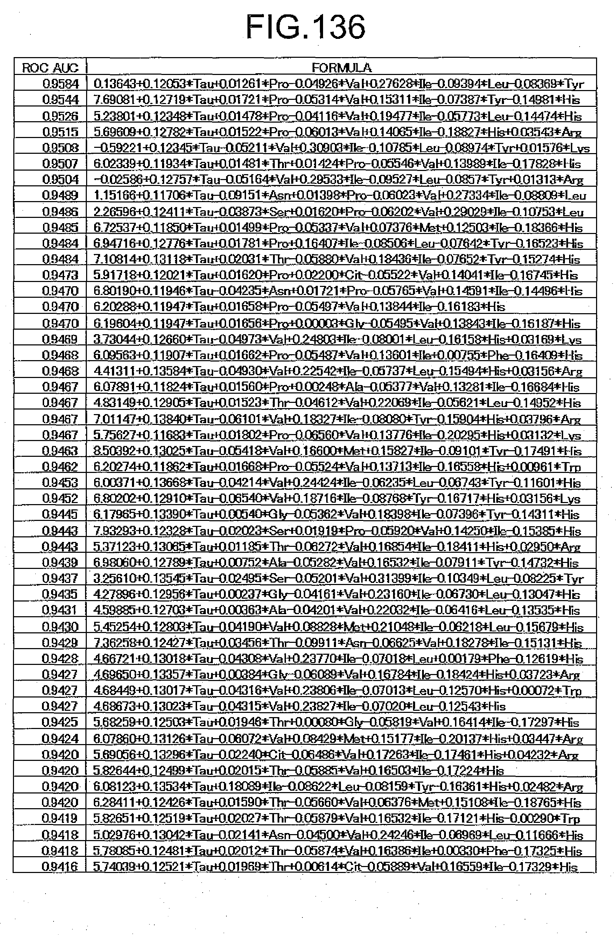

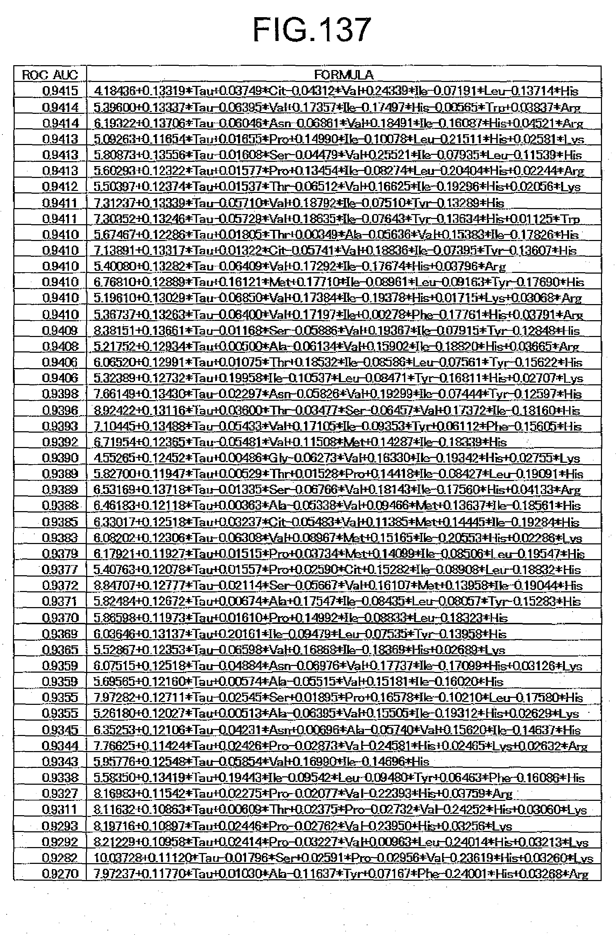

An evaluation was performed on the discrimination between the 2 groups of the healthy subject group and the Crohn disease group based on the index formula 45, on the basis of the AUC of the ROC curve (FIG. 133), and an AUC of $0.963\pm0.011$ (95% confidence interval: 0.941 to 0.985) was obtained. Thus, the index formula 45 was found to be a useful index with high diagnostic performance. In addition to that, a plurality of logistic regression equations having a discrimination performance equivalent to that of the index formula 45 was obtained. Those x's are presented in FIG. 134, FIG. 135, FIG. 136 and FIG. 137. The equations presented in FIG. 134, FIG. 135, FIG. 136 and FIG. 137 may be equations obtained by transformation of monotonic increase or monotonic decrease, such as linear transformation such as addition of a constant number or multiplication of a constant number, and logit transformation.

Example 19

The sample data used in Example 10 were used. An index by which the performance of discriminating between the 2 groups of the healthy subject group and the ulcerative colitis group is maximized was eagerly searched by linear discriminant analysis, and an index formula 46 was obtained among a plurality of indices having an equivalent performance.

```
Index formula 46: 0.7029+0.0097×Tau-0.0004×Ala+0.0207×Ile-

0.0126×Leu-0.0054×Tyr-0.0022×His
```

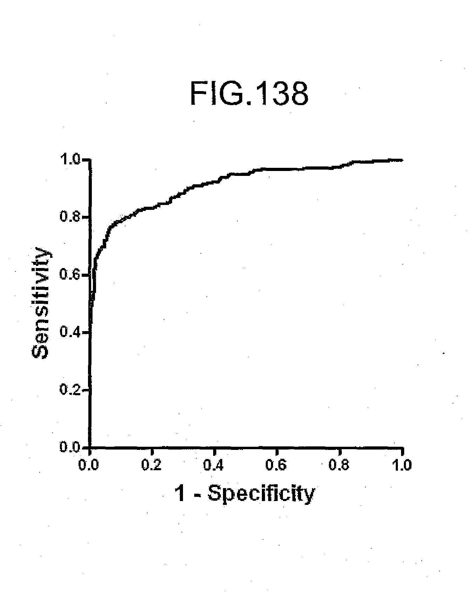

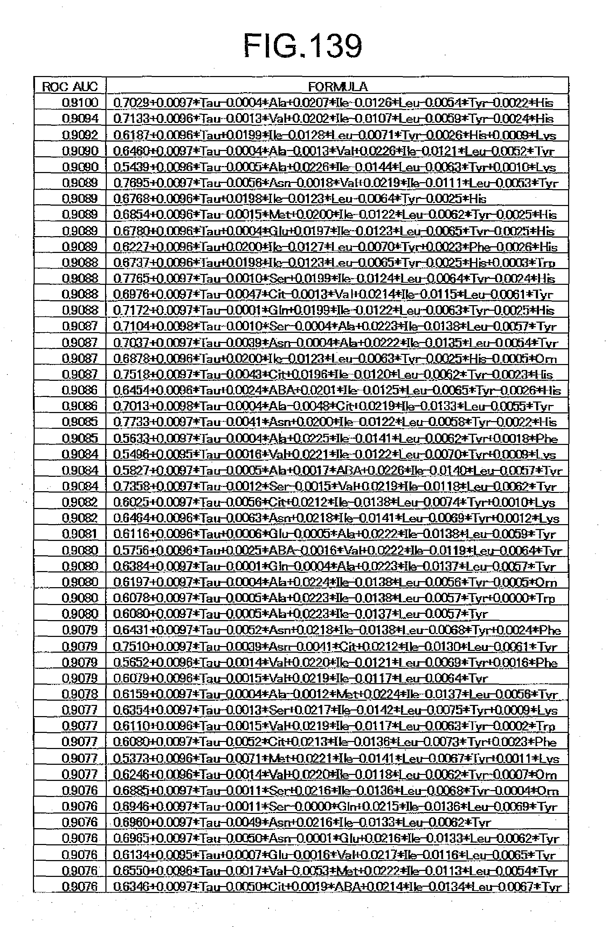

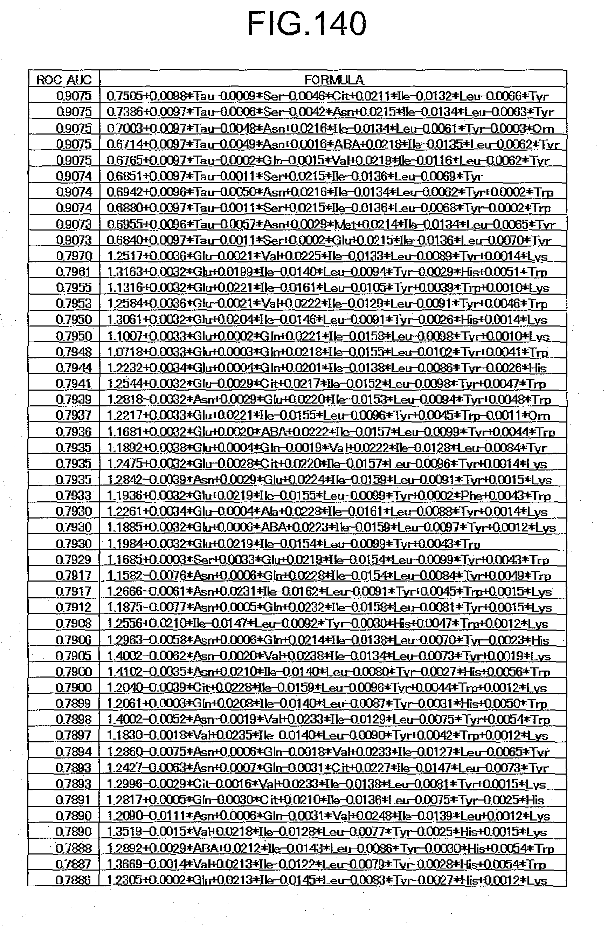

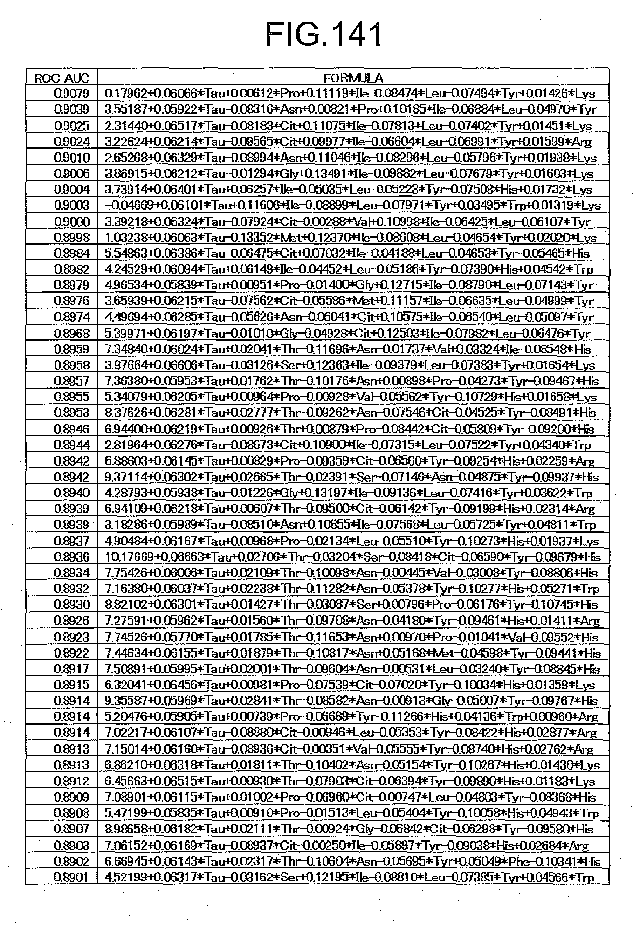

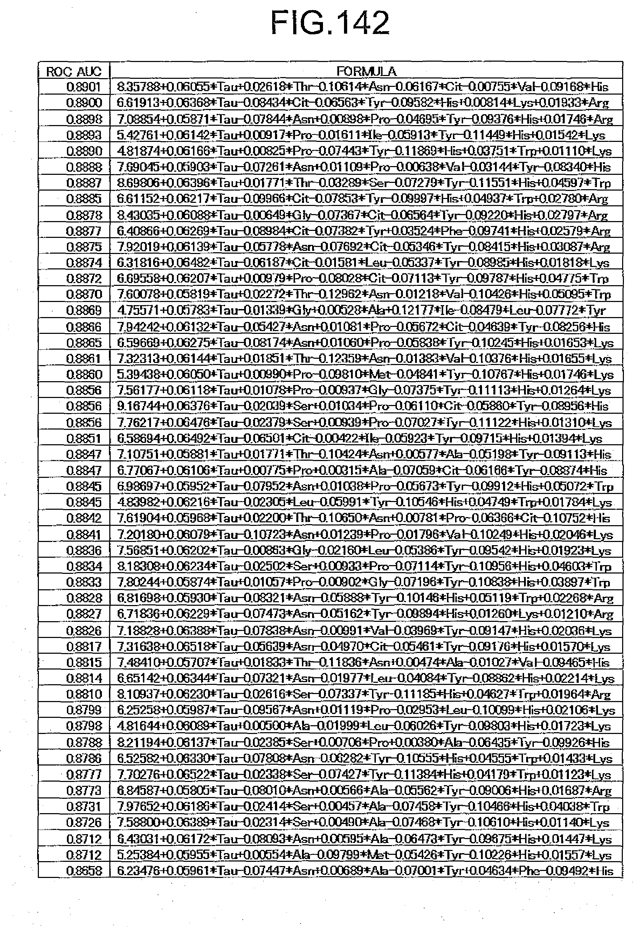

An evaluation was performed on the discrimination between the 2 groups of the healthy subject group and the ulcerative colitis group based on the index formula 46, on the basis of the AUC of the ROC curve (FIG. 138), and an AUC of $0.910\pm0.025$ (95% confidence interval: 0.846 to 0.942) was obtained. Thus, the index formula 46 was found to be a useful index with high diagnostic performance. In addition to that, a plurality of linear discriminants having a discrimination performance equivalent to that of the index formula 46 was obtained. Those discriminants are presented in FIG. 139, FIG. 140, FIG. 141 and FIG. 142. The discriminants presented in FIG. 139, FIG. 140, FIG. 141 and FIG. 142 may be discriminants obtained by transformation of monotonic increase or monotonic decrease, such as linear transformation such as addition of a constant number or multiplication of a constant number, and logit transformation.
Subsequently, an index by which the performance of discriminating between the 2 groups of the healthy subject group and the ulcerative colitis group is maximized was eagerly searched by logistic regression analysis, and an index formula 47 was obtained among a plurality of indices having an equivalent performance.

```
Index formula 47: 1/(1+exp(-x))

provided that x=1.1801+0.0964×Tau-0.0012×ABA+0.1498×Ile-

0.0898×Leu-0.0541×Tyr-0.0309×His
```

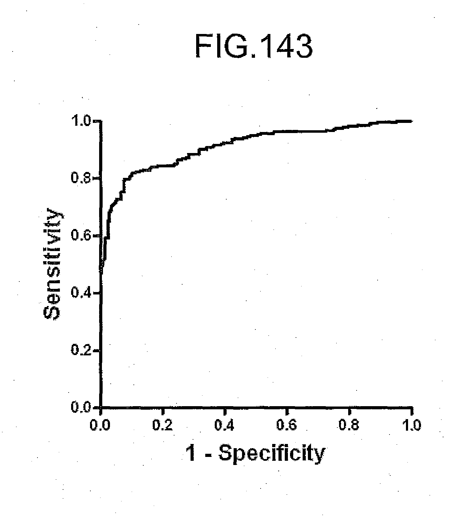

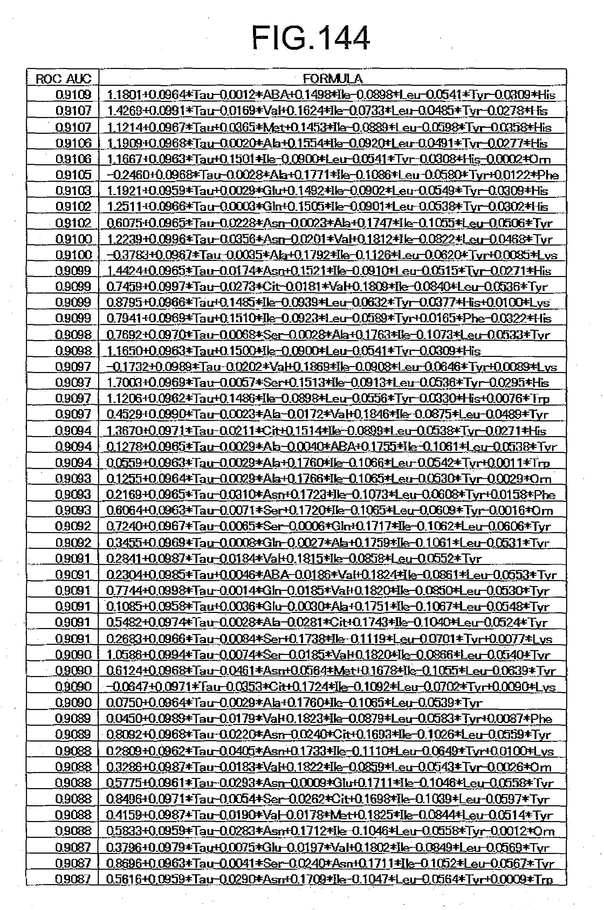

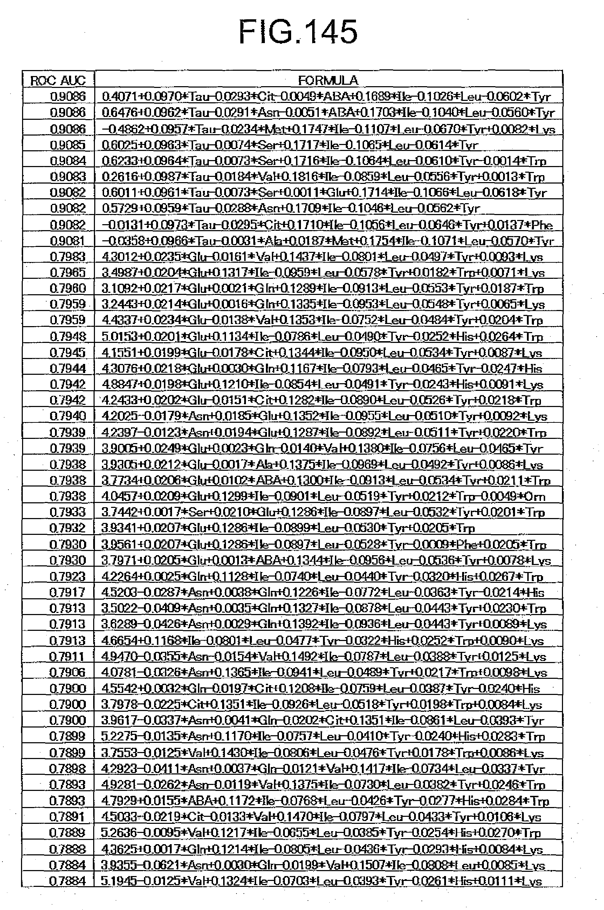

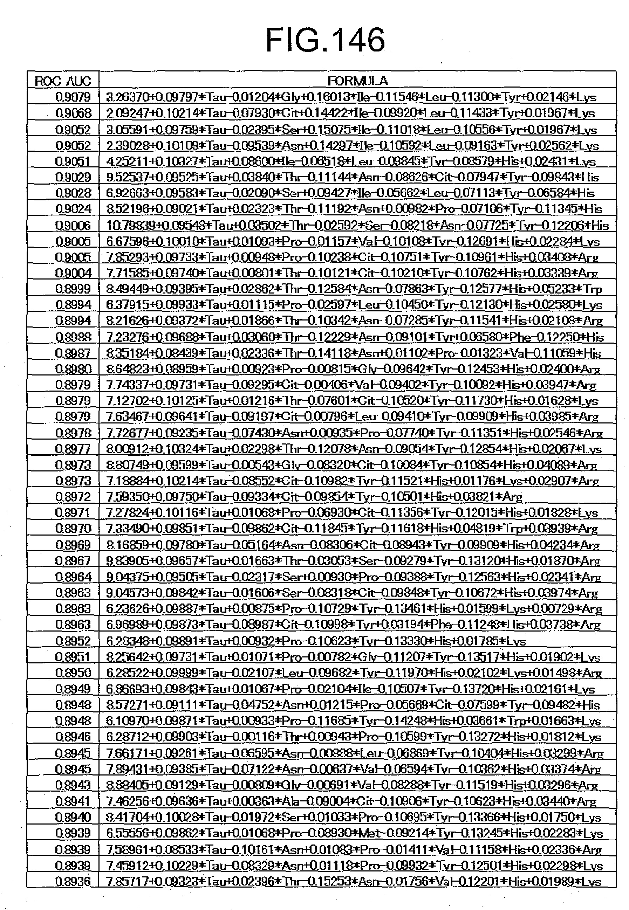

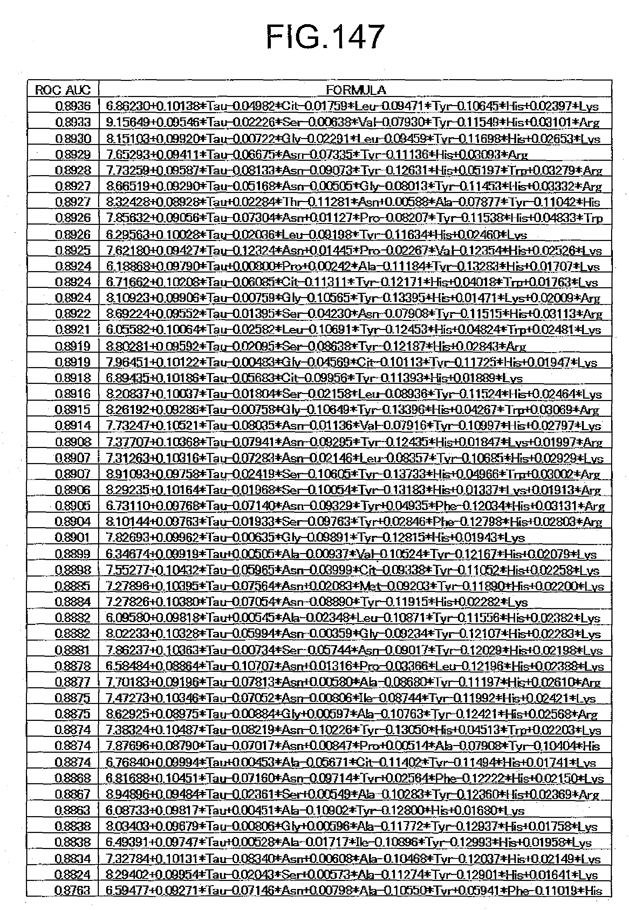

An evaluation was performed on the discrimination between the 2 groups of the healthy subject group and the ulcerative colitis group based on the index formula 47, on the basis of the AUC of the ROC curve (FIG. 143), and an AUC of $0.911\pm0.021$ (95% confidence interval: 0.869 to 0.953) was obtained. Thus, the index formula 47 was found to be a useful index with high diagnostic performance. In addition to that, a plurality of logistic regression equations having a discrimination performance equivalent to that of the index formula 47 was obtained. Those x's are presented in FIG. 144, FIG. 145, FIG. 146 and FIG. 147. The equations presented in FIG. 144, FIG. 145, FIG. 146 and FIG. 147 may be equations obtained by transformation of monotonic increase or monotonic decrease, such as linear transformation such as addition of a constant number or multiplication of a constant number, and logit transformation.

Example 20

The sample data used in Example 11 were used. An index by which the performance of discriminating between the 2 groups of the Crohn disease group and the ulcerative colitis group is maximized was eagerly searched by linear discriminant analysis, and an index formula 48 was obtained among a plurality of indices having an equivalent performance.

```
Index formula 48: 0.0880+0.0011×Tau+0.0001×Ala+0.0151×Ile-

0.0071×Leu-0.0020×Tyr+0.0020×His
```

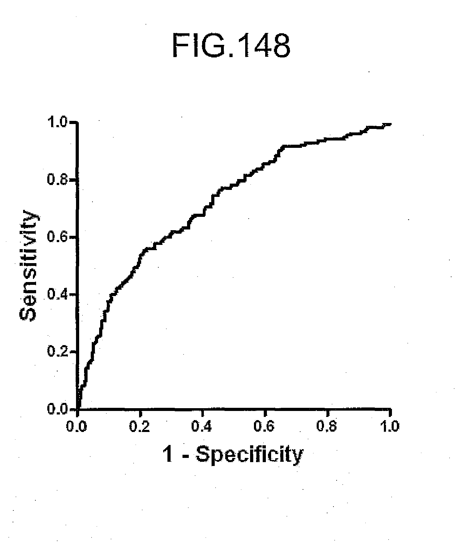

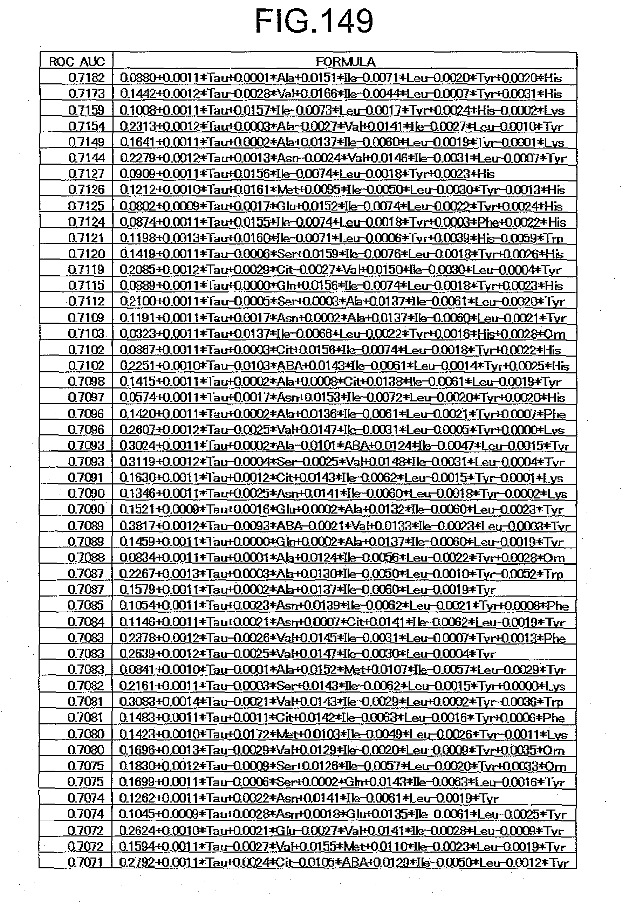

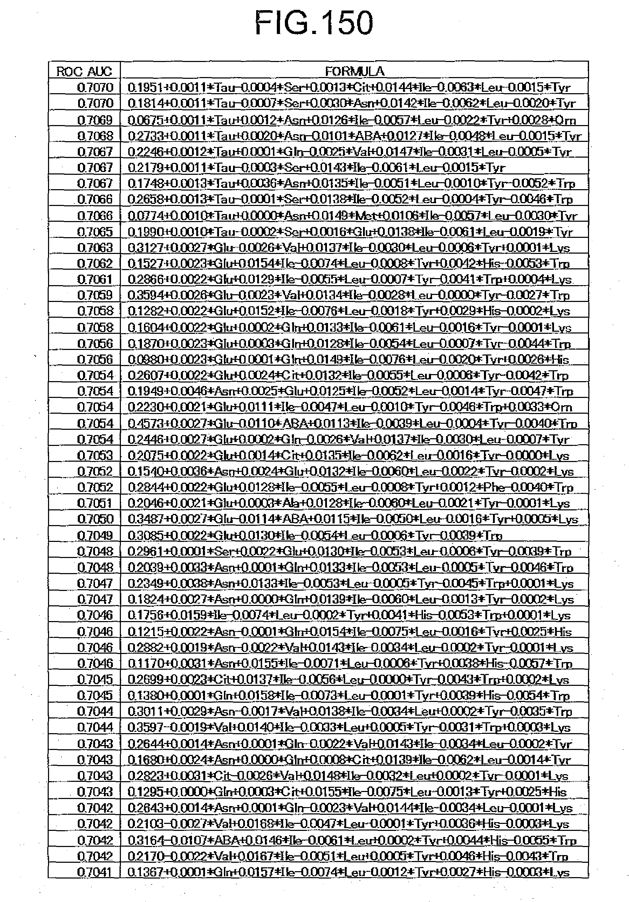

An evaluation was performed on the discrimination between the 2 groups of the Crohn disease group and the ulcerative colitis group based on the index formula 48, on the basis of the AUC of the ROC curve (FIG. 148), and an AUC of 0.718±0.027 (95% confidence interval: 0.665 to 0.771) was obtained. Thus, the index formula 48 was found to be a useful index with high diagnostic performance. In addition to that, a plurality of linear discriminants having a discrimination performance equivalent to that of the index formula 48 was obtained. Those discriminants are presented in FIG. 149 and FIG. 150. The discriminants presented in FIG. 149 and FIG. 150 may be discriminants obtained by transformation of monotonic increase or monotonic decrease, such as linear transformation such as addition of a constant number or multiplication of a constant number, and logit transformation.

Subsequently, an index by which the performance of discriminating between the 2 groups of the Crohn disease group and the ulcerative colitis group is maximized was eagerly searched by logistic regression analysis, and an index formula 49 was obtained among a plurality of indices having an equivalent performance.

```
Index formula 49: 1/(1+exp(-x))

provided that x=-0.1542+0.0121×Glu-0.0536×ABA+0.0505×Ile-

0.0171×Leu-0.0015×Tyr-0.0177×Trp
```

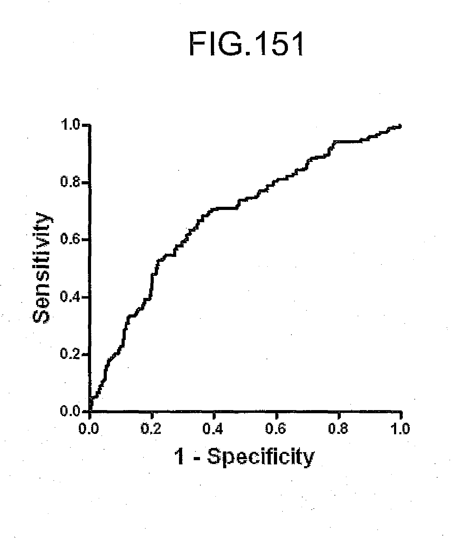

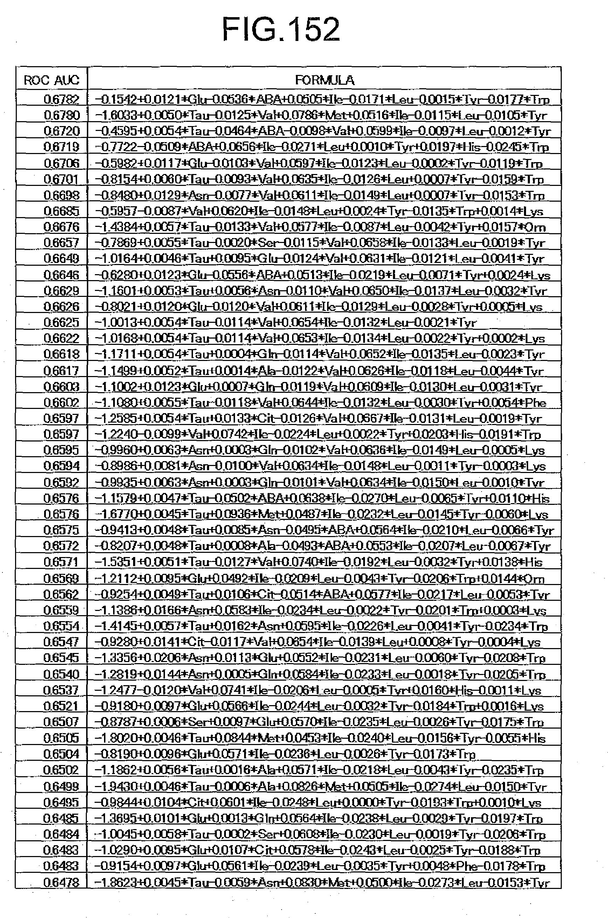

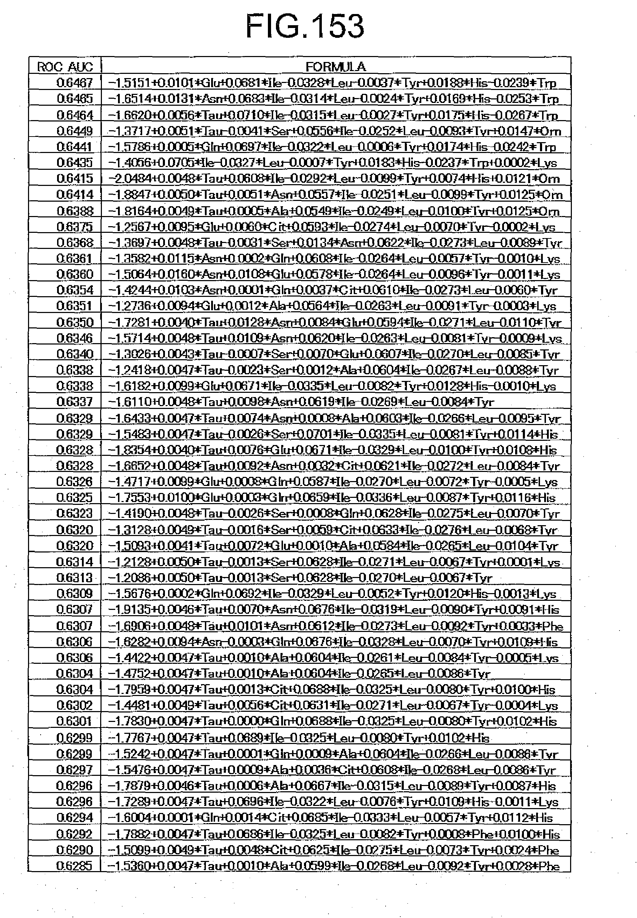

An evaluation was performed on the discrimination between the 2 groups of the Crohn disease group and the ulcerative colitis group based on the index formula 49, on the basis of the AUC of the ROC curve (FIG. 151), and an AUC of 0.678±0.028 (95% confidence interval: 0.623 to 0.733) was obtained. Thus, the index formula 49 was found to be a useful index with high diagnostic performance. In addition to that, a plurality of logistic regression equations having a discrimination performance equivalent to that of the index formula 49 was obtained. Those x's are presented in FIG. 152 and FIG. 153. The equations presented in FIG. 152 and FIG. 153 may be equations obtained by transformation of monotonic increase or monotonic decrease, such as linear transformation such as addition of a constant number or multiplication of a constant number, and logit transformation.

Example 21

The sample data used in Example 8 were used. An index by which the performance of discriminating between the 2 groups of the active phase Crohn disease group and the active phase ulcerative colitis group is maximized was eagerly searched by linear discriminant analysis, and an index formula 50 was obtained among a plurality of indices having an equivalent performance.

```
Index formula 50: -0.0123+0.0024×Tau+0.0040×Thr-

0.0119×ABA+0.0128×Met+0.0064×Tyr-0.0160×Trp
```

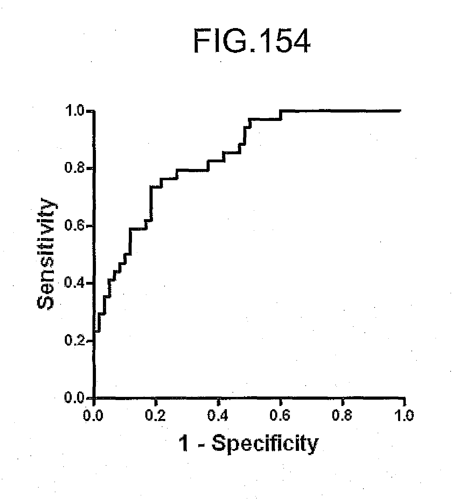

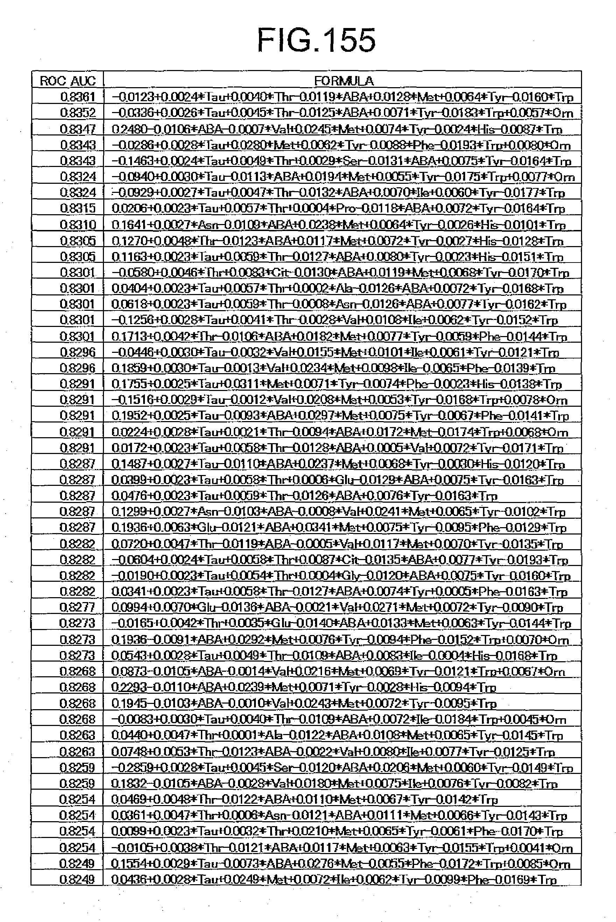

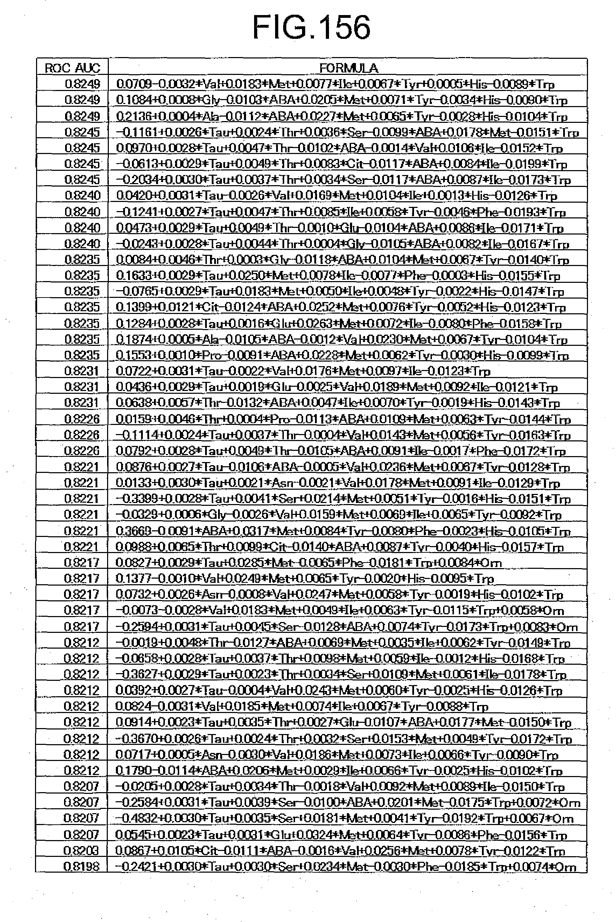

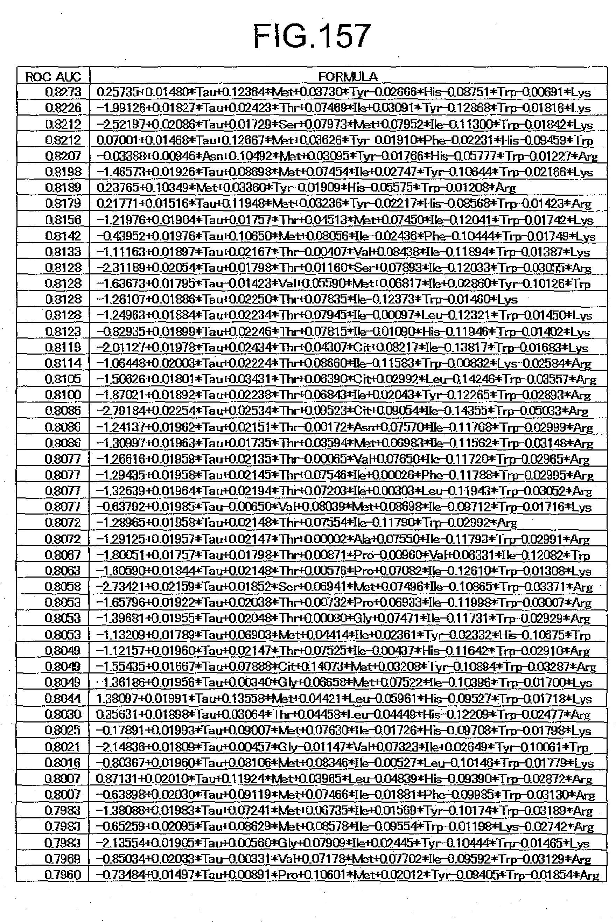

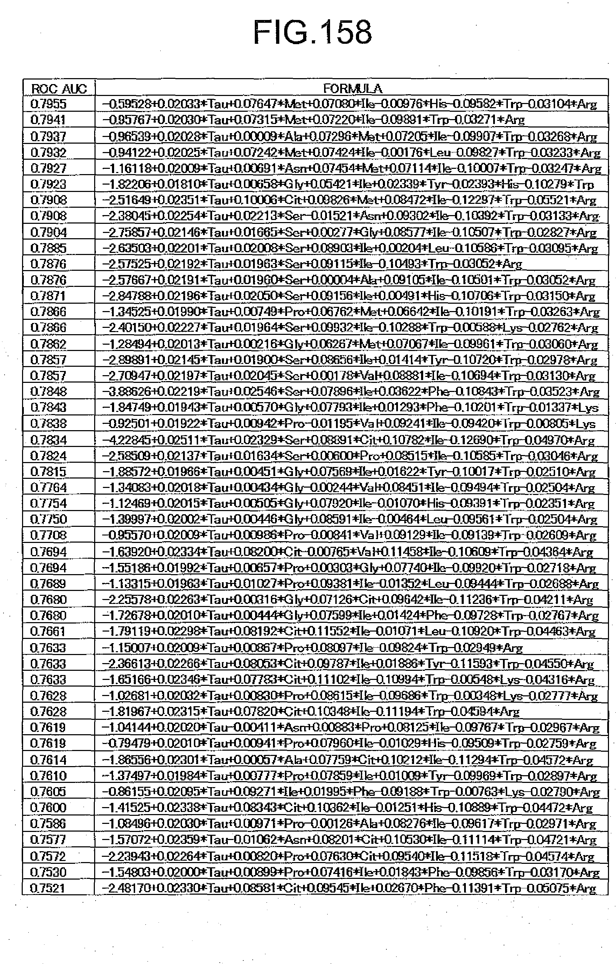

An evaluation was performed on the discrimination between the 2 groups of the active phase Crohn disease group and the active phase ulcerative colitis group based on the index formula 50, on the basis of the AUC of the ROC curve (FIG. 154), and an AUC of 0.836±0.047 (95% confidence interval: 0.744 to 0.928) was obtained. Thus, the index formula 50 was found to be a useful index with high diagnostic performance. In addition to that, a plurality of linear discriminants having a discrimination performance equivalent to that of the index formula 50 was obtained. Those discriminants are presented in FIG. 155, FIG. 156, FIG. 157 and FIG. 158. The discriminants presented in FIG. 155, FIG. 156, FIG. 157 and FIG. 158 may be discriminants obtained by transformation of monotonic increase or monotonic decrease, such as linear transformation such as addition of a constant number or multiplication of a constant number, and logit transformation.

Subsequently, an index by which the performance of discriminating between the 2 groups of the active phase Crohn disease group and the active phase ulcerative colitis group is maximized was eagerly searched by logistic regression analysis, and an index formula 51 was obtained among a plurality of indices having an equivalent performance.

```
Index formula 51: 1/(1+exp(-x))

provided that x=-3.5145+0.0229×Tau-

0.0715×ABA+0.1245×Met+0.0271×Tyr-0.1170×Trp+0.0521×Orn
```

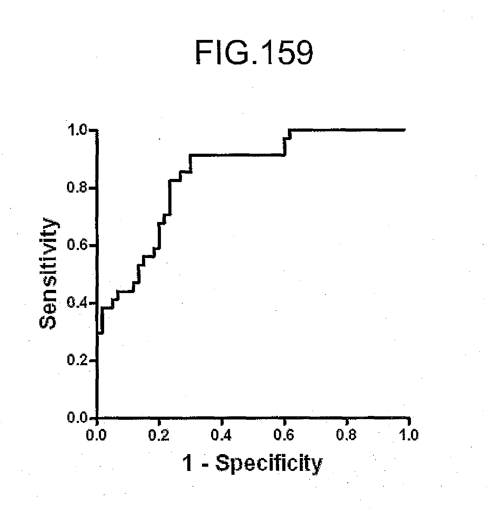

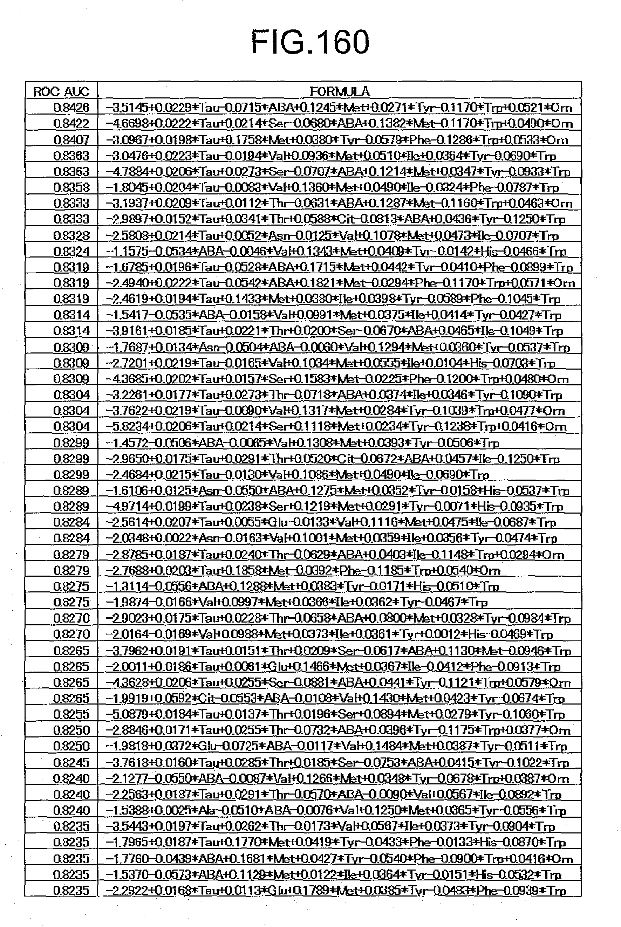

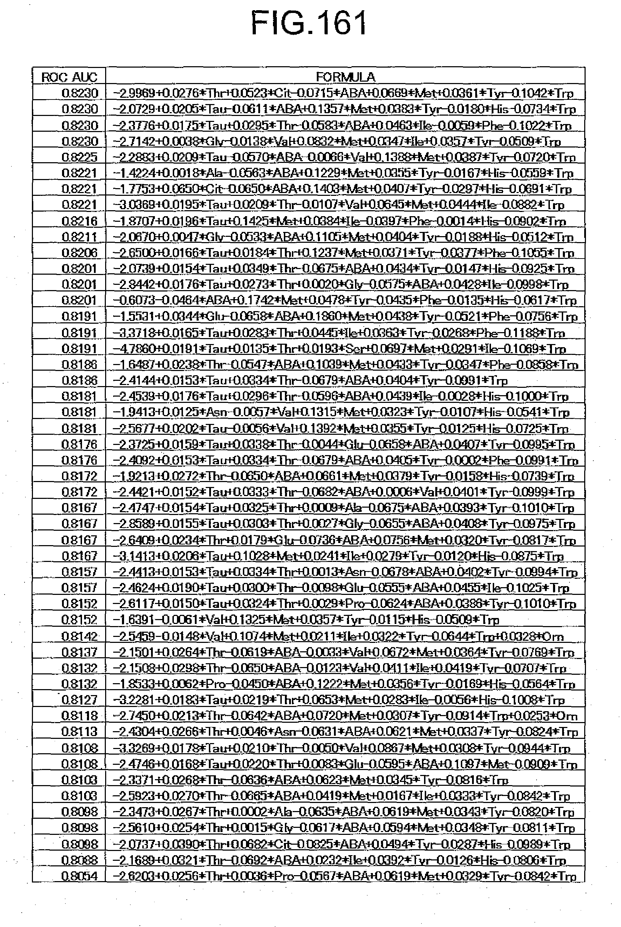

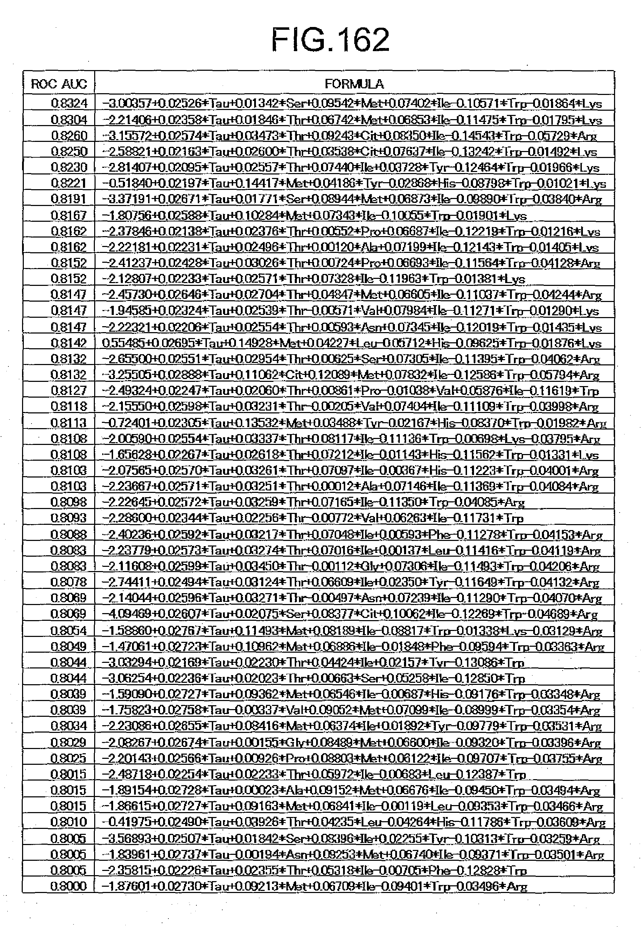

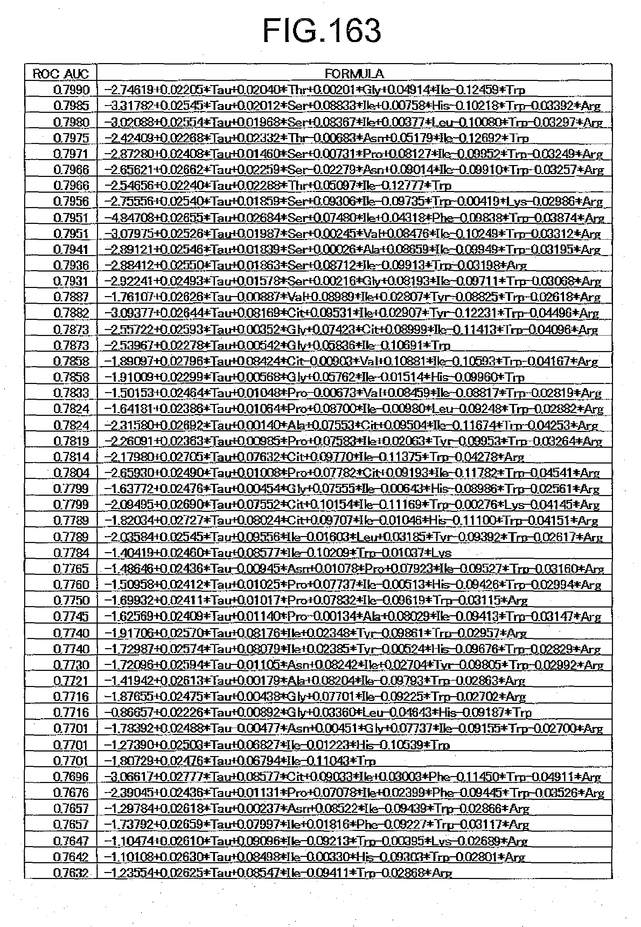

An evaluation was performed on the discrimination between the 2 groups of the active phase Crohn disease group and the active phase ulcerative colitis group based on the index formula 51, on the basis of the AUC of the ROC curve (FIG. 159), and an AUC of $0.843\pm0.046$ (95% confidence interval: 0.753 to 0.933) was obtained. Thus, the index formula 51 was found to be a useful index with high diagnostic performance. In addition to that, a plurality of logistic regression equations having a discrimination performance equivalent to that of the index formula 51 was obtained. Those x's are presented in FIG. 160, FIG. 161, FIG. 162 and FIG. 163. The equations presented in FIG. 160, FIG. 161, FIG. 162 and FIG. 163 may be equations obtained by transformation of monotonic increase or monotonic decrease, such as linear transformation such as addition of a constant number or multiplication of a constant number, and logit transformation.

Subsequently, an index by which the performance of discriminating between the 2 groups of the active phase Crohn disease group and the remission phase Crohn disease group is maximized was eagerly searched by linear discriminant analysis, and an index formula 52 was obtained among a plurality of indices having an equivalent performance.

```
Index formula 52: 0.7603+0.0026×Tau+0.0042×Ser+0.0120×Ile-

0.0104×His-0.0147×Trp-0.0026×Lys
```

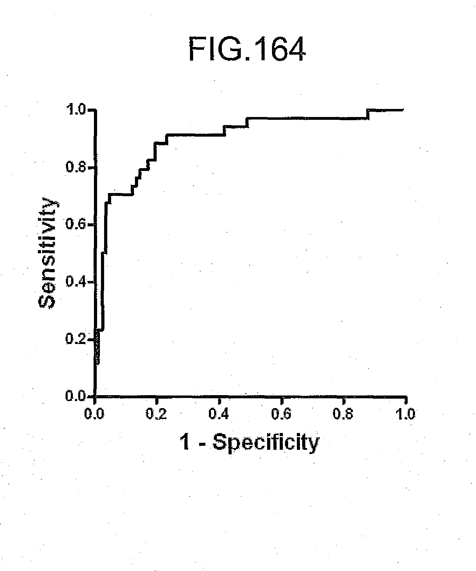

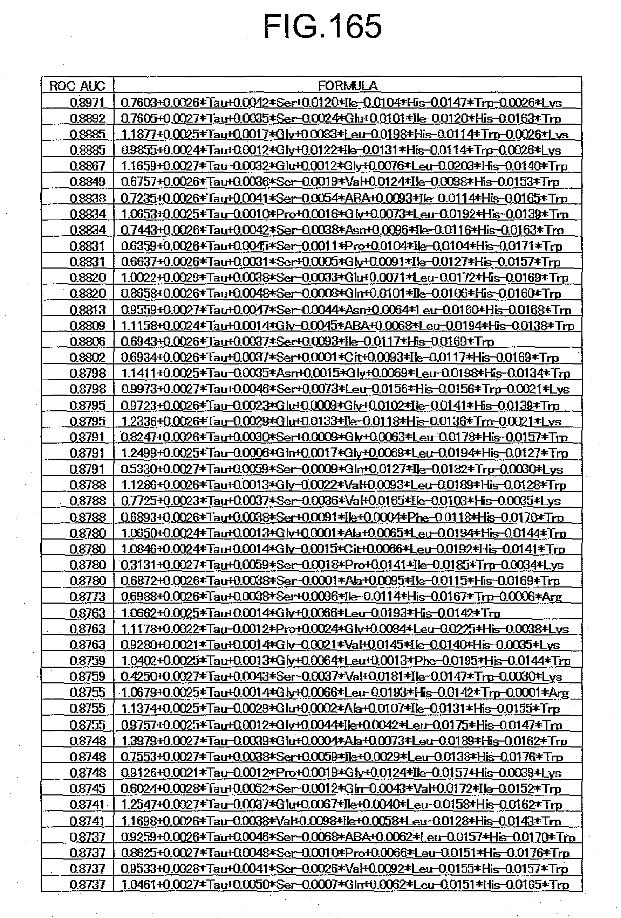

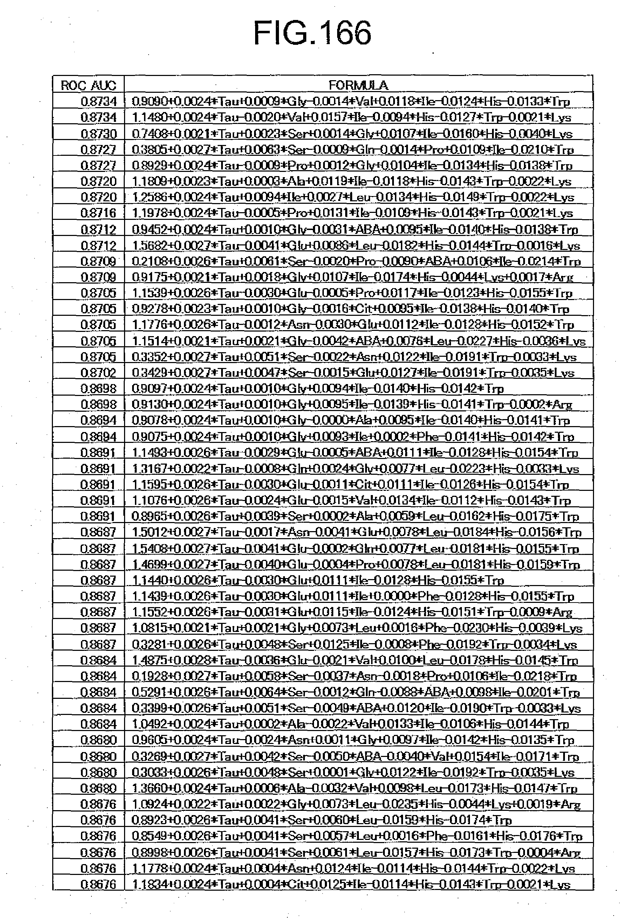

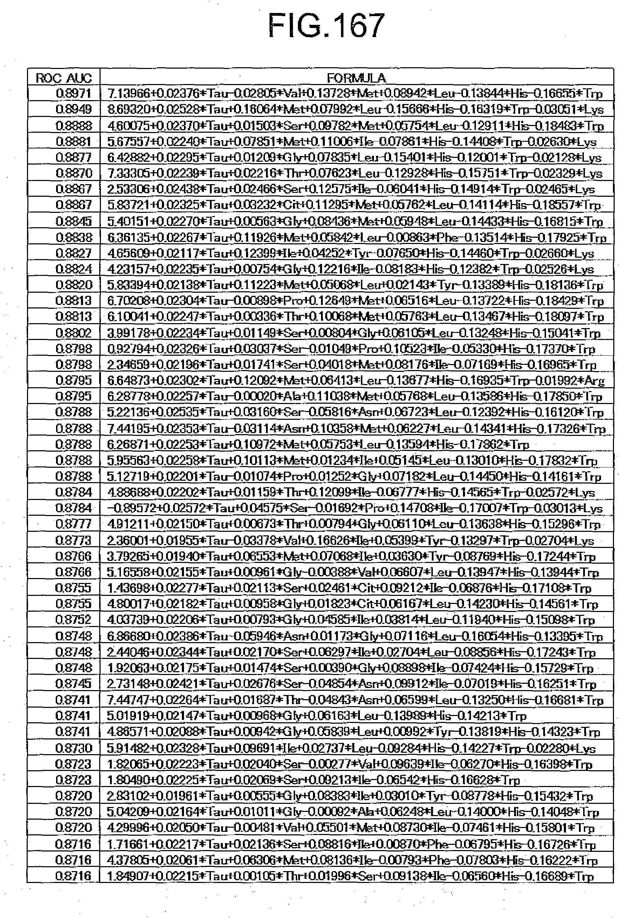

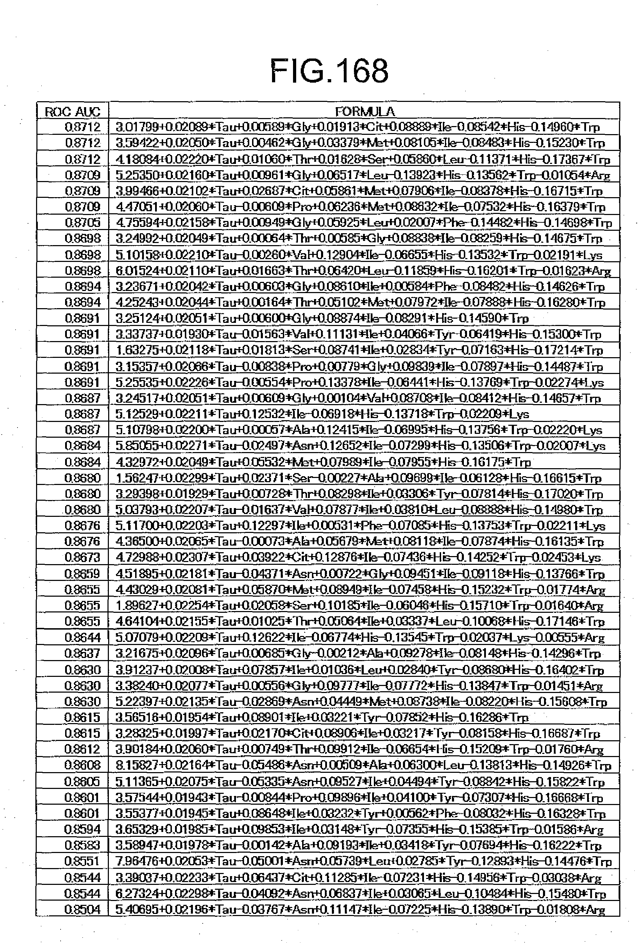

An evaluation was performed on the discrimination between the 2 groups of the active phase Crohn disease group and the remission phase Crohn disease group based on the index formula 52, on the basis of the AUC of the ROC curve (FIG. 164), and an AUC of $0.897\pm0.037$ (95% confidence interval: 0.824 to 0.970) was obtained. Thus, the index formula 52 was found to be a useful index with high diagnostic performance. In addition to that, a plurality of linear discriminants having a discrimination performance equivalent to that of the index formula 52 was obtained. Those discriminants are presented in FIG. 165, FIG. 166, FIG. 167 and FIG. 168. The discriminants presented in FIG. 165, FIG. 166, FIG. 167 and FIG. 168 may be discriminants obtained by transformation of monotonic increase or monotonic decrease, such as linear transformation such as addition of a constant number or multiplication of a constant number, and logit transformation.

Subsequently, an index by which the performance of discriminating between the 2 groups of the active phase Crohn disease group and the remission phase Crohn disease group is maximized was eagerly searched by logistic regression analysis, and an index formula 53 was obtained among a plurality of indices having an equivalent performance.

```
Index formula 53: 1/(1+exp(-x))

provided that x=2.1059+0.0194×Tau+0.0394×Ser+0.1120×Ile-

0.0992×His-0.1170×Trp-0.0216×Lys
```

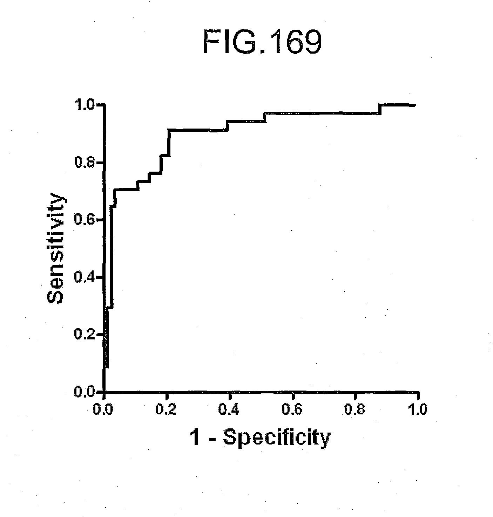

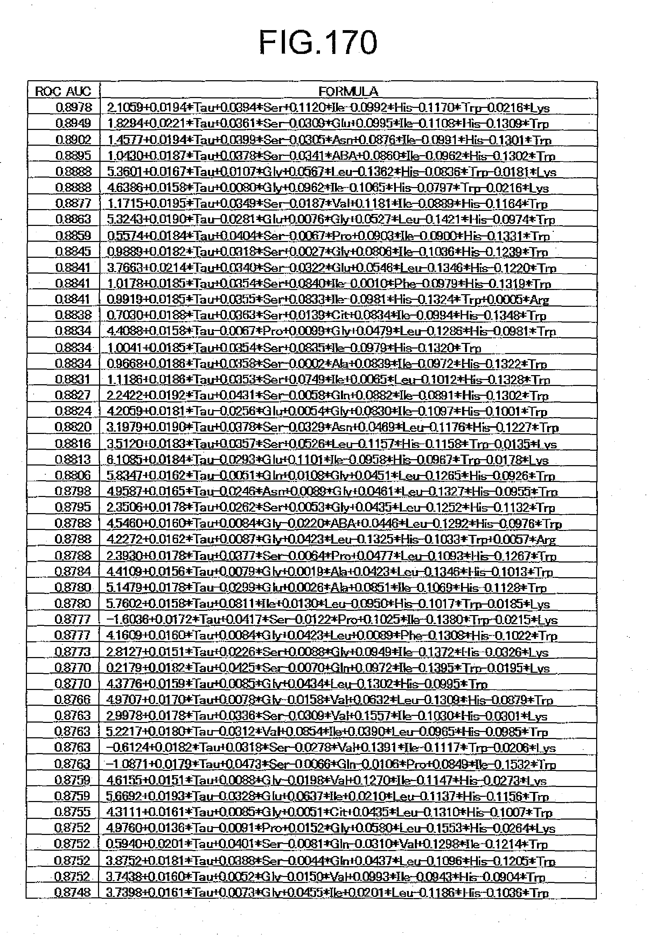

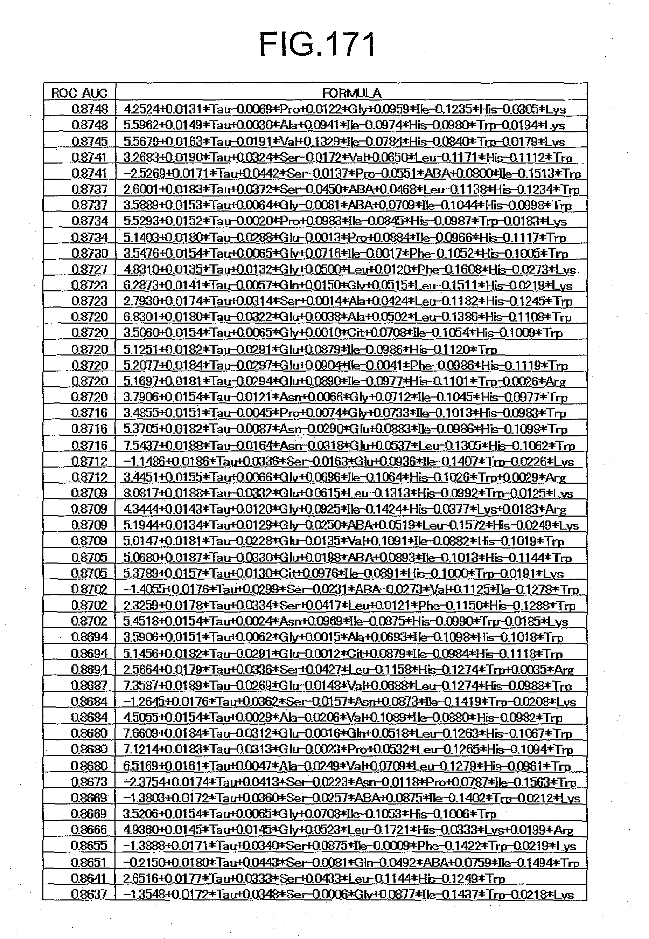

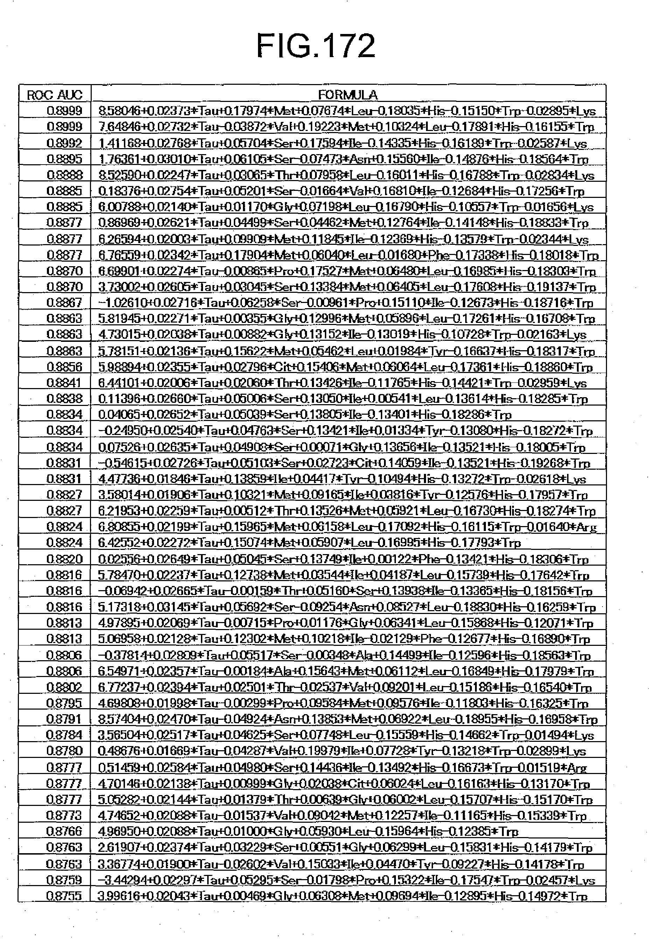

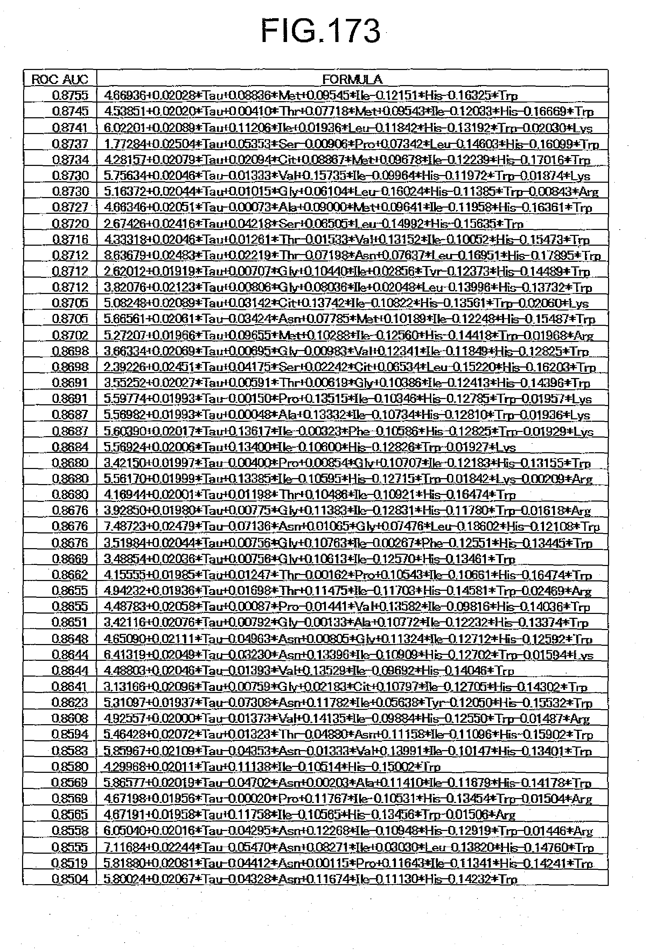

An evaluation was performed on the discrimination between the 2 groups of the active phase Crohn disease group and the remission phase Crohn disease group based on the index formula 53, on the basis of the AUC of the ROC curve (FIG. 169), and an AUC of 0.898±0.037 (95% confidence interval: 0.825 to 0.971) was obtained. Thus, the index formula 53 was found to be a useful index with high diagnostic performance. In addition to that, a plurality of logistic regression equations having a discrimination performance equivalent to that of the index formula 53 was obtained. Those x's are presented in FIG. 170, FIG. 171, FIG. 172 and FIG. 173. The equations presented in FIG. 170, FIG. 171, FIG. 172 and FIG. 173 may be equations obtained by transformation of monotonic increase or monotonic decrease, such as linear transformation such as addition of a constant number or multiplication of a constant number, and logit transformation.

Subsequently, an index by which the performance of discriminating between the 2 groups of the active phase ulcerative colitis group and the remission phase ulcerative colitis group is maximized was eagerly searched by linear discriminant analysis, and an index formula 54 was obtained among a plurality of indices having an equivalent performance.

```
Index formula 54: 1.7238-0.0031×Val-0.0106×Met+0.0040×Leu-

0.0033×Tyr-0.0121×His+0.0015×Arg
```

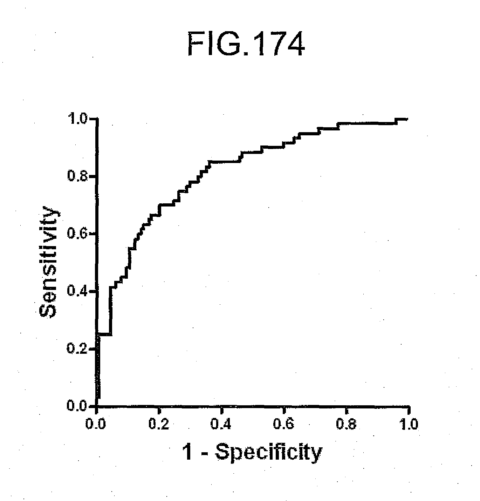

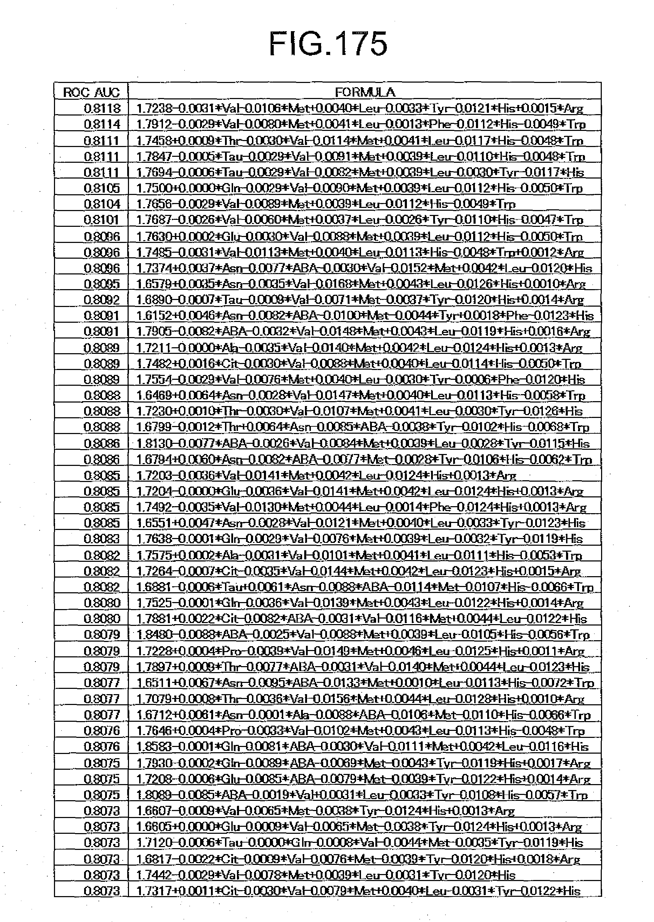

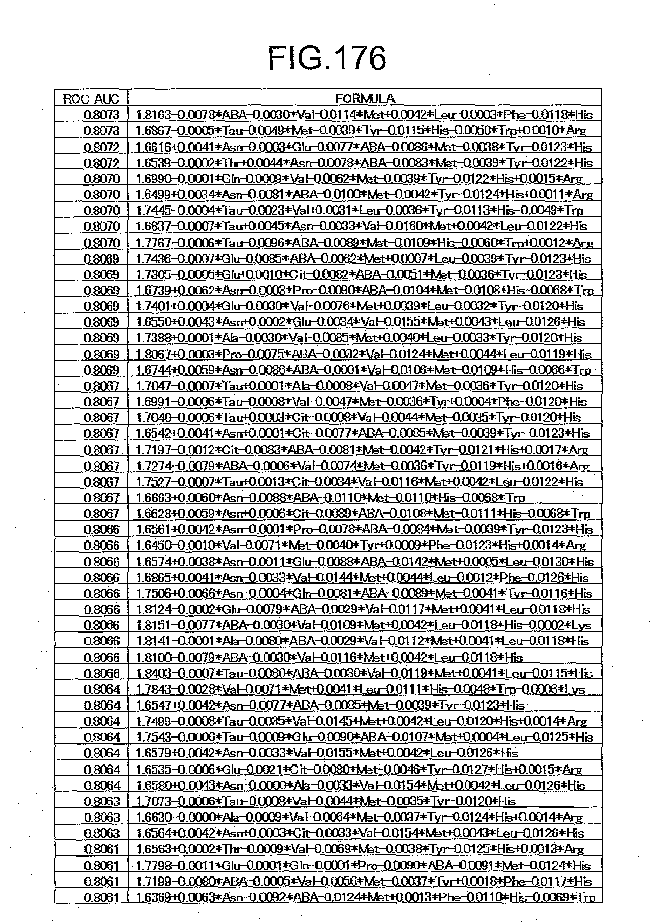

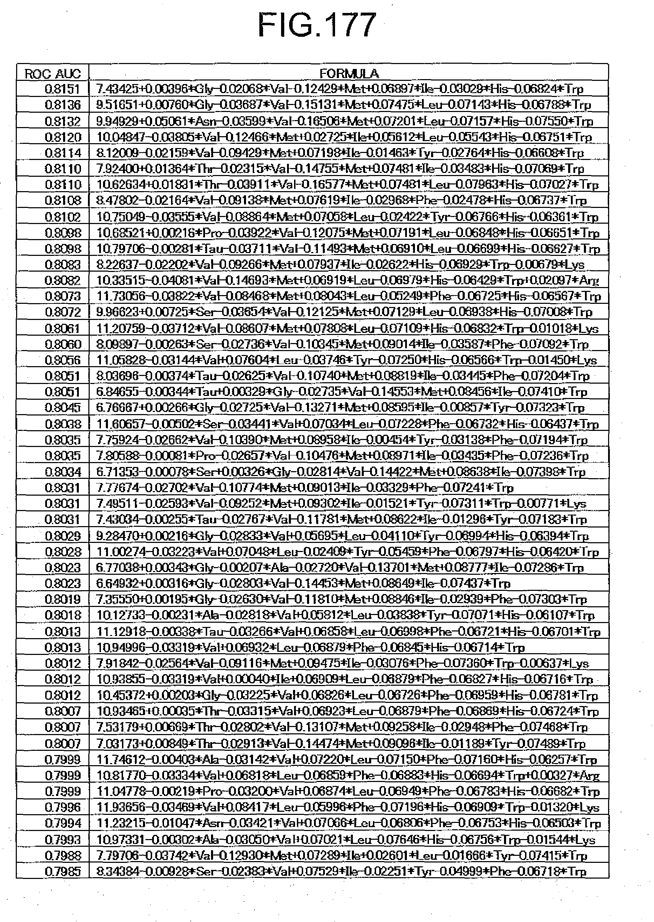

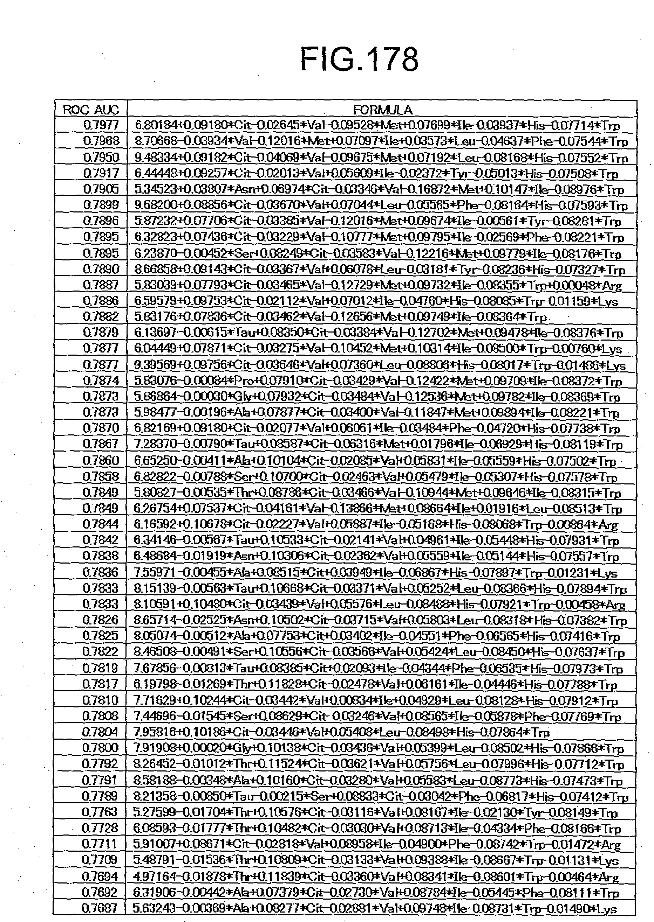

An evaluation was performed on the discrimination between the 2 groups of the active phase ulcerative colitis group and the remission phase ulcerative colitis group based on the index formula 54, on the basis of the AUC of the ROC curve (FIG. 174), and an AUC of 0.812±0.037 (95% confidence interval: 0.740 to 0.884) was obtained. Thus, the index formula 54 was found to be a useful index with high diagnostic performance. In addition to that, a plurality of linear discriminants having a discrimination performance equivalent to that of the index formula 54 was obtained. Those discriminants are presented in FIG. 175, FIG. 176, FIG. 177 and FIG. 178. The discriminants presented in FIG. 175, FIG. 176, FIG. 177 and FIG. 178 may be discriminants obtained by transformation of monotonic increase or monotonic decrease, such as linear transformation such as addition of a constant number or multiplication of a constant number, and logit transformation.

Subsequently, an index by which the performance of discriminating between the 2 groups of the active phase ulcerative colitis group and the remission phase ulcerative colitis group is maximized was eagerly searched by logistic regression analysis, and an index formula 55 was obtained among a plurality of indices having an equivalent performance.

```
Index formula 55: 1/(1+exp(-x))

provided that x=7.8348-0.0197×Val-0.0744×Met+0.0256×Leu-

0.0173×Tyr-0.0770×His+0.0088×Arg
```

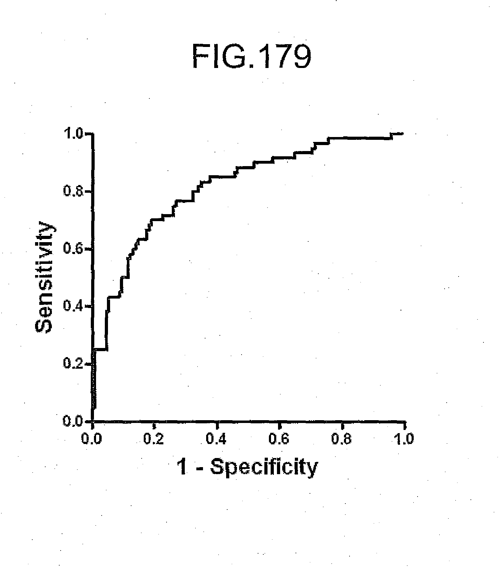

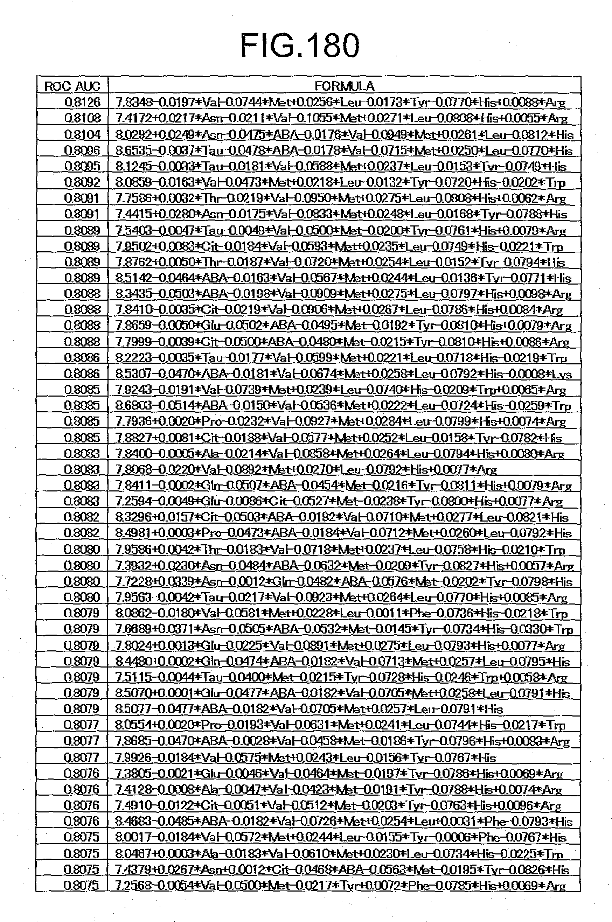

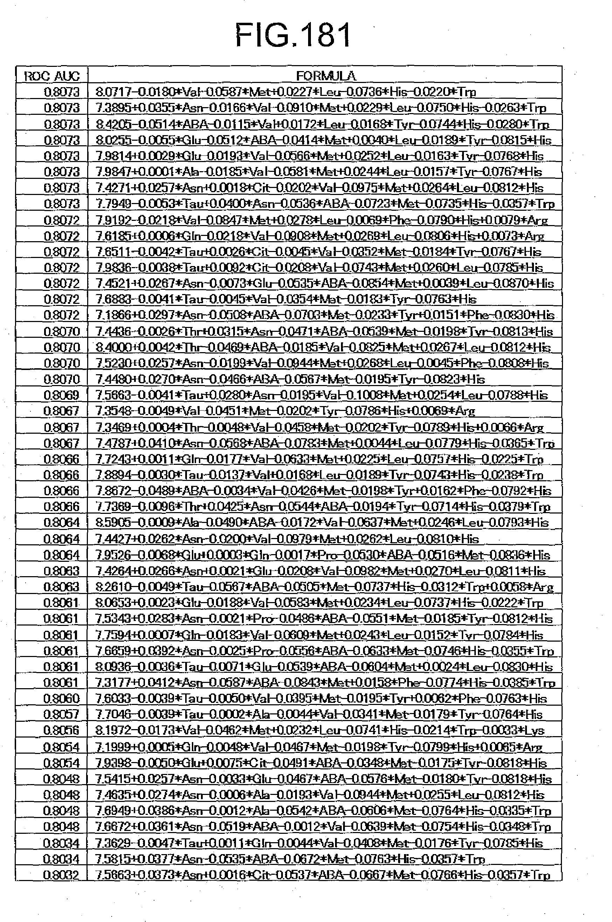

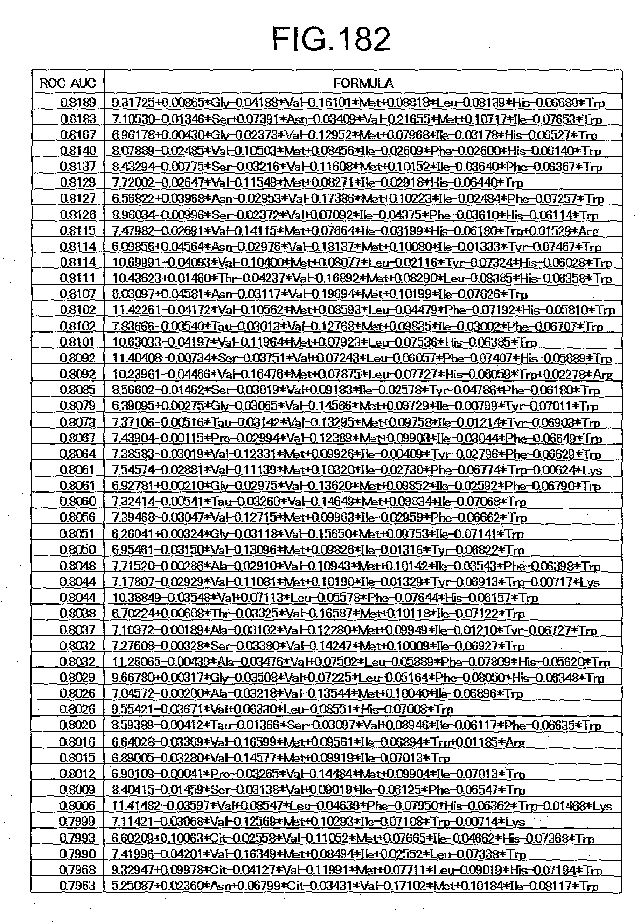

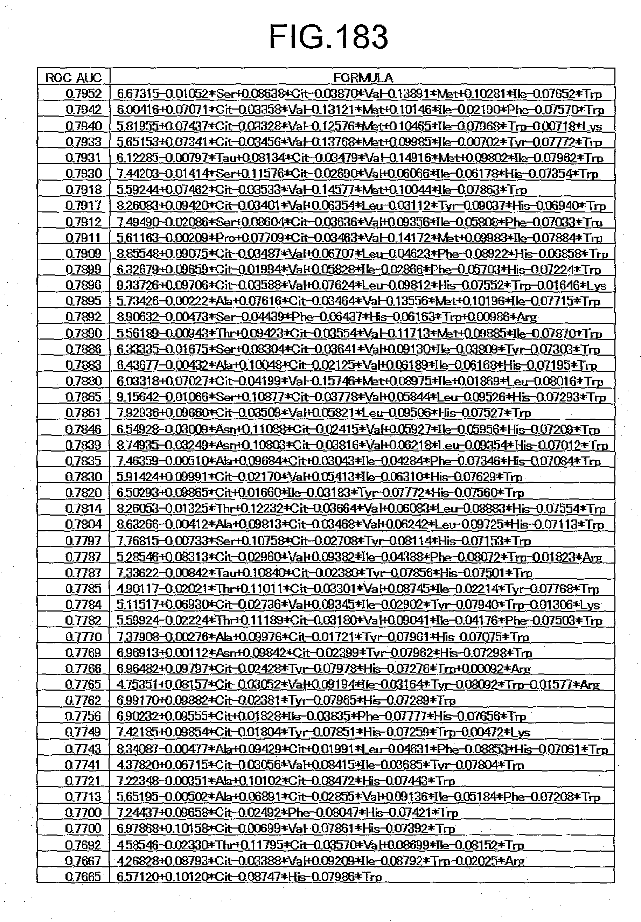

An evaluation was performed on the discrimination between the 2 groups of the active phase ulcerative colitis group and the remission phase ulcerative colitis group based on the index formula 55, on the basis of the AUC of the ROC curve (FIG. 179), and an AUC of 0.813±0.037 (95% confidence interval: 0.741 to 0.885) was obtained. Thus, the index formula 55 was found to be a useful index with high diagnostic performance. In addition to that, a plurality of logistic regression equations having a discrimination performance equivalent to that of the index formula 55 was obtained. Those x's are presented in FIG. 180, FIG. 181, FIG. 182 and FIG. 183. The equations presented in FIG. 180, FIG. 181, FIG. 182 and FIG. 183 may be equations obtained by transformation of monotonic increase or monotonic decrease, such as linear transformation such as addition of a constant number or multiplication of a constant number, and logit transformation.

Example 22

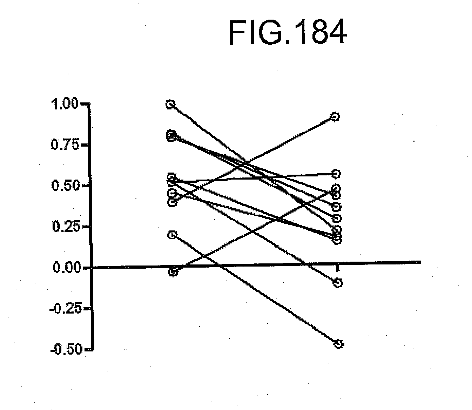

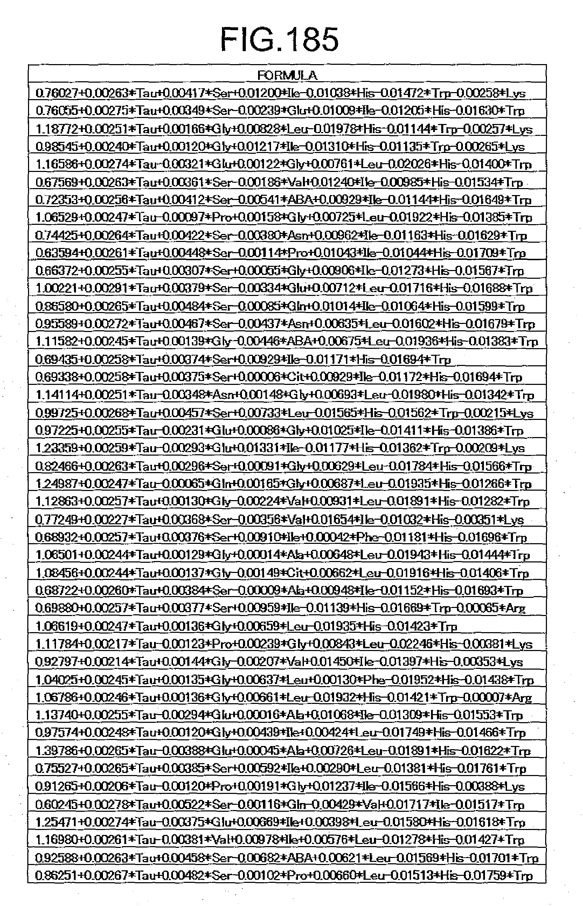

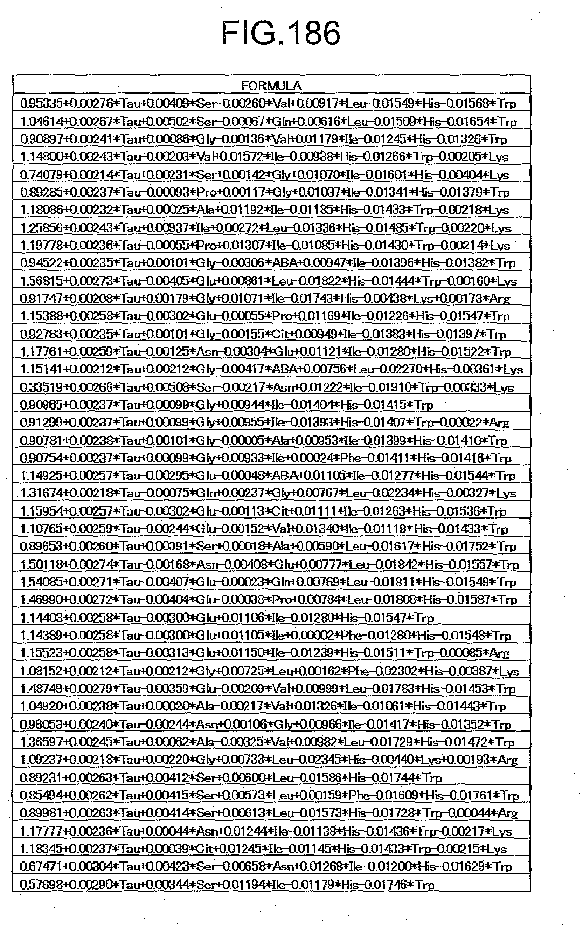

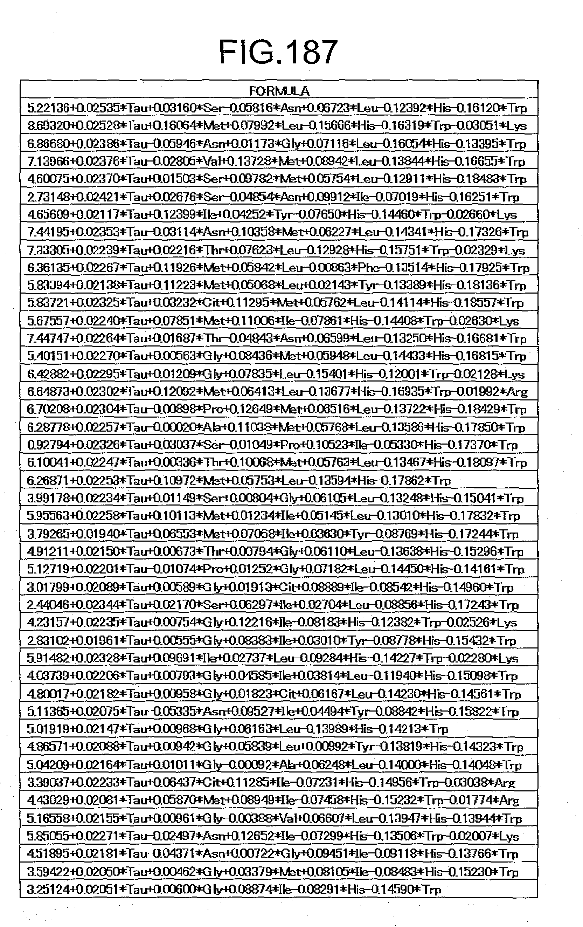

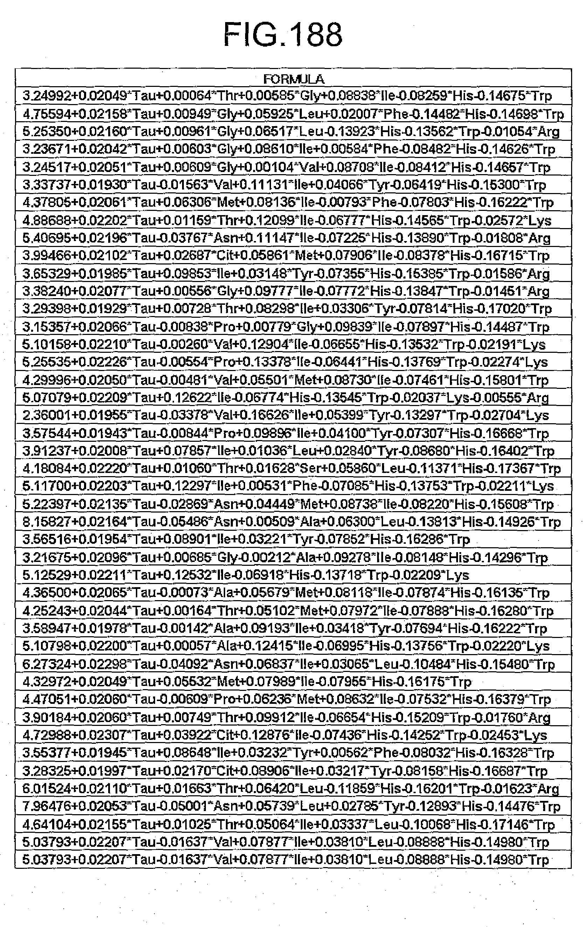

Using the data obtained from same patients but on different blood sampling days among the samples measured in Example 8, the changes over time in the values of an index formula 52', which is equivalent to the index formula 52 obtained in Example 21 that is useful for the discrimination between the 2 groups of the active phase Crohn disease group and the remission phase Crohn disease group by linear discriminant analysis, were compared for the period from the active phase to the remission phase, and thereby an evaluation of the index formula 52' was performed (FIG. 184). In FIG. 184, the samples connected by a line indicate that the samples belong to a same patient. A paired T-test between 2 groups was performed for the purpose of discriminating between the 2 groups of the active phase group and the remission phase group. In the remission phase group as compared with the active phase group, the values of the index formula significantly decreased (probability of significant difference P<0.05). It was made clear that the index formula 52' is a useful index even for the monitoring of the condition of Crohn disease in a same patient. In addition to that, a plurality of linear discriminants of which the values significantly decreased (probability of significant difference P<0.05) in the remission phase as compared with the active phase, in the same manner as the index formula 52' did, were obtained. Those discriminants are presented in FIG. 185, FIG. 186, FIG. 187 and FIG. 188. The discriminants presented in FIG. 185, FIG. 186, FIG. 187 and FIG. 188 may be discriminants obtained by transformation of monotonic increase or monotonic decrease, such as linear transformation such as addition of a constant number or multiplication of a constant number, and logit transformation.

```
Index formula 52': 0.7603+0.0026×Tau+0.0042×Ser+0.0120×Ile-

0.0104×His-0.0147×Trp-0.0026×Lys
```

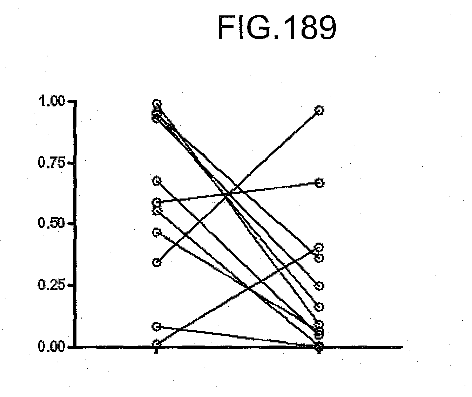

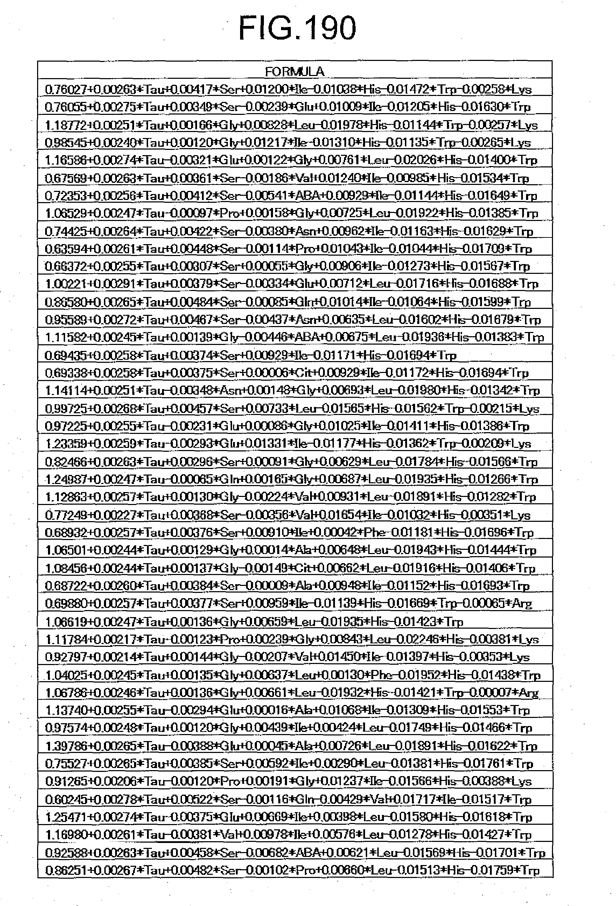

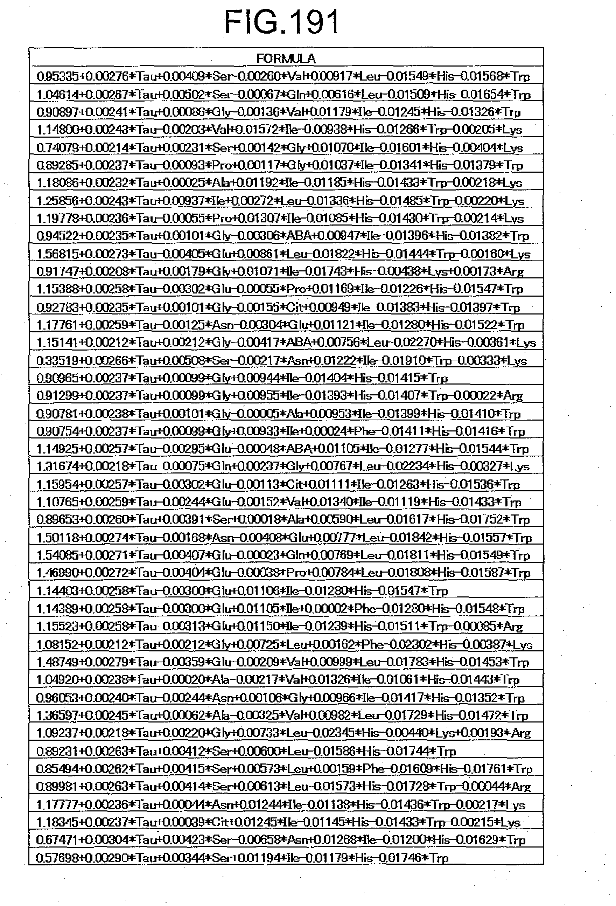

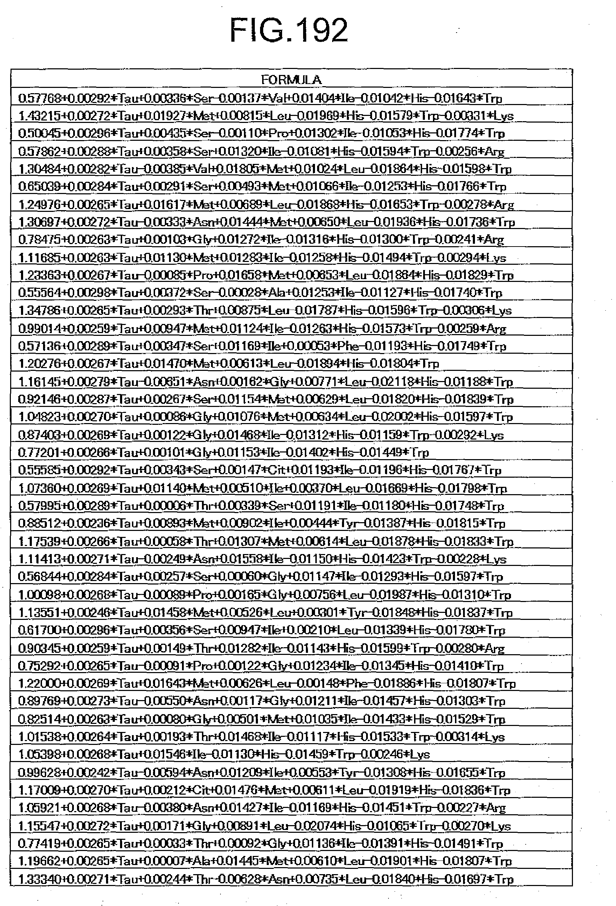

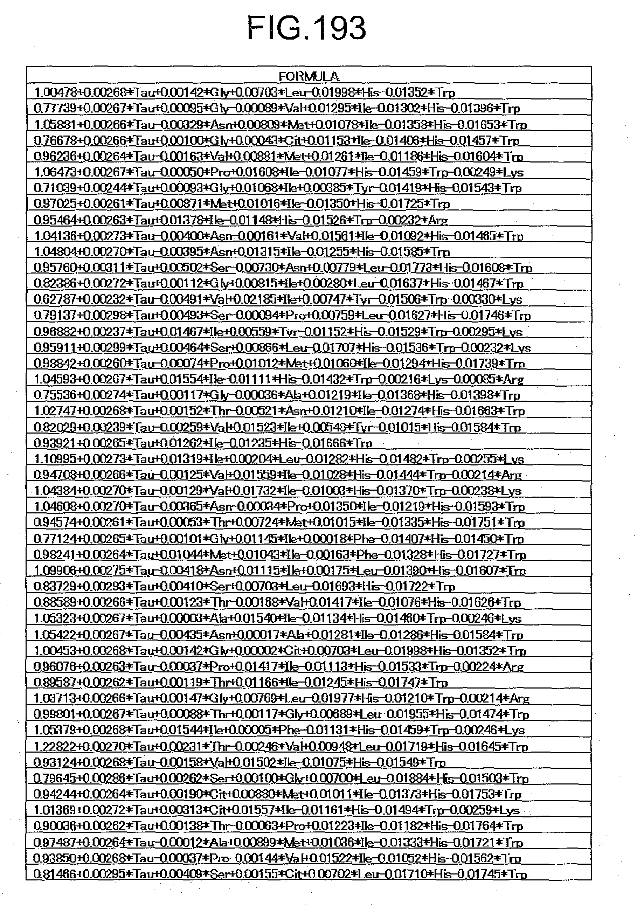

Subsequently, using the data obtained from same patients but on different blood sampling days among the samples measured in Example 8, the changes over time in the values of an index formula 53', which is equivalent to the index formula 53 obtained in Example 21 that is useful for the discrimination between the 2 groups of the active phase Crohn disease group and the remission phase Crohn disease group by logistic regression analysis, were compared for the period from the active phase to the remission phase, and thereby an evaluation of the index formula 53' was performed (FIG. 189). In FIG. 189, the samples connected by a line indicate that the samples belong to a same patient. A paired T-test between 2 groups was performed for the purpose of discriminating between the 2 groups of the active phase group and the remission phase group. In the remission phase group as compared with the active phase group, the values of the index formula significantly decreased (probability of significant difference P<0.05). It was made clear that the index formula 53' is a useful index even for the monitoring of the condition of Crohn disease in a same patient. In addition to that, a plurality of logistic regression equations of which the values significantly decreased (probability of significant difference P<0.05) in the remission phase as compared with the active phase, in the same manner as the index formula 53' did, were obtained. Those x's are presented in FIG. 190, FIG. 191, FIG. 192 and FIG. 193. The equations presented in FIG. 190, FIG. 191, FIG. 192 and FIG. 193 may be equations obtained by transformation of monotonic increase or monotonic decrease, such as linear transformation such as addition of a constant number or multiplication of a constant number, and logit transformation.

```
Index formula 53': 1/(1+exp(-x))

provided that x=5.5692-0.106×His-0.1283×Trp-

0.0193×Lys+0.0201×Tau+0.134×Ile
```

Example 23

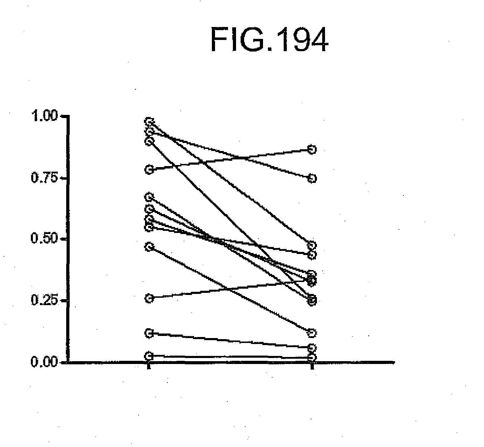

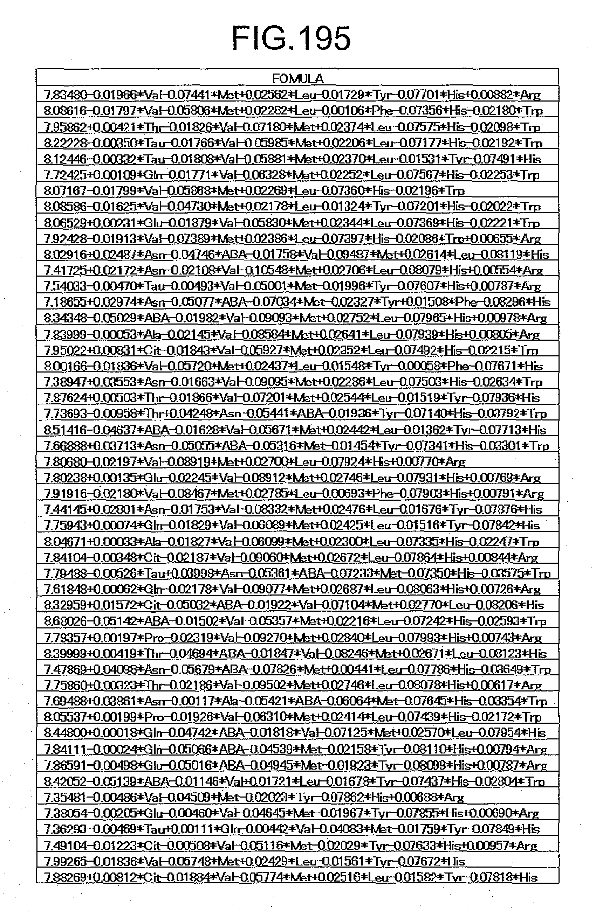

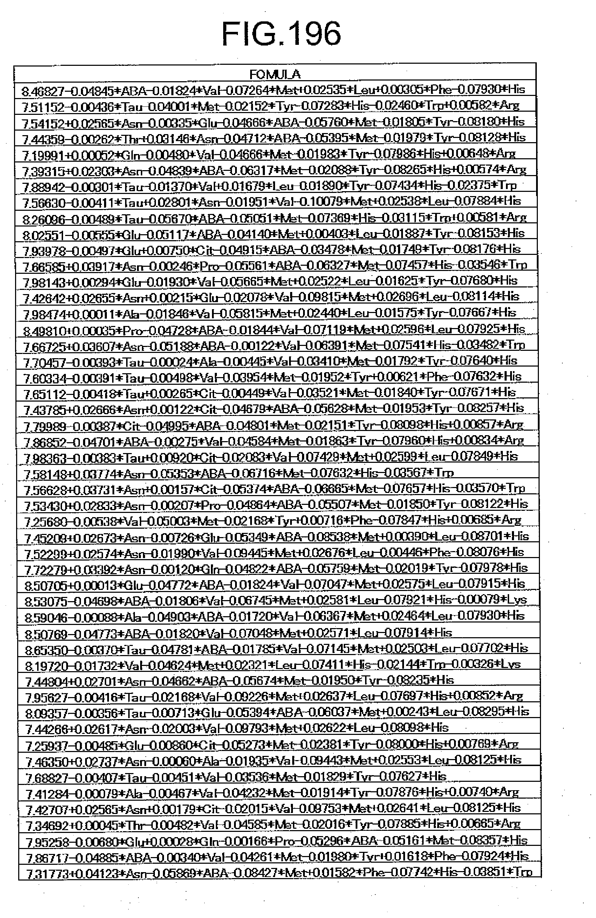

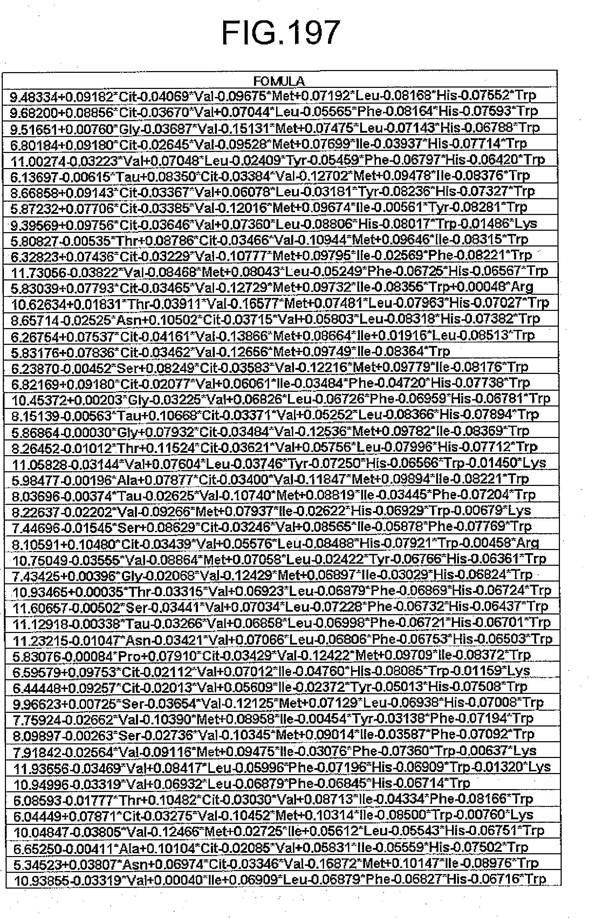

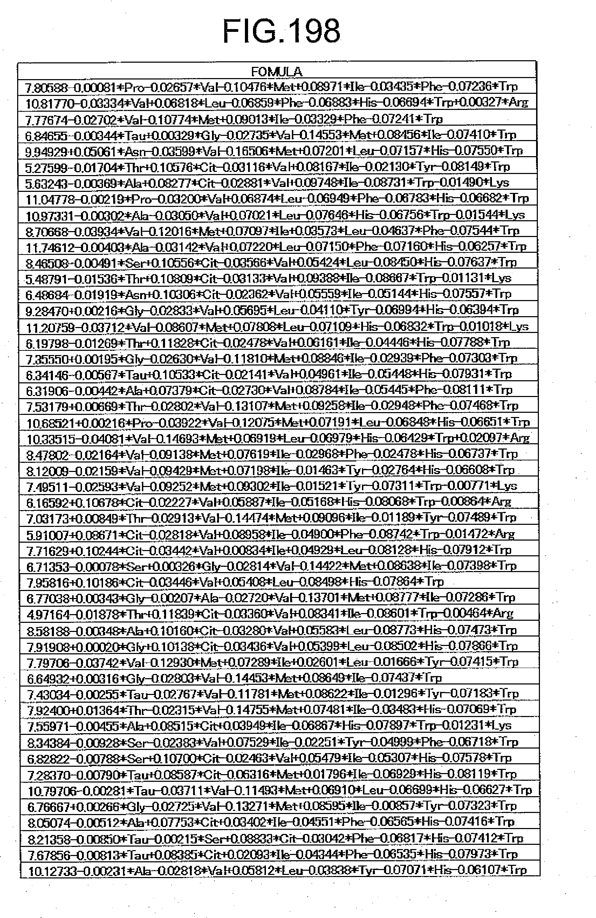

Using the data obtained from same patients but on different blood sampling days among the samples measured in Example 8, the changes over time in the values of an index formula 54', which is equivalent to the index formula 54 obtained in Example 21 that is useful for the discrimination between the 2 groups of the active phase ulcerative colitis group and the remission phase ulcerative colitis group by linear discriminant analysis, were compared for the period from the active phase to the remission phase, and thereby an evaluation of the index formula 54' was performed (FIG. 194). In FIG. 194, the samples connected by a line indicate that the samples belong to a same patient. A paired T-test between 2 groups was performed for the purpose of discriminating between the 2 groups of the active phase group and the remission phase group. In the remission phase group as compared with the active phase group, the values of the index formula significantly decreased (probability of significant difference P<0.05). It was made clear that the index formula 54' is a useful index even for the monitoring of the condition of ulcerative colitis in a same patient. In addition to that, a plurality of linear discriminants of which the values significantly decreased (probability of significant difference P<0.05) in the remission phase as compared with the active phase, in the same manner as the index formula 54' did, were obtained. Those discriminants are presented in FIG. 195, FIG. 196, FIG. 197 and FIG. 198. The discriminants presented in FIG. 195, FIG. 196, FIG. 197 and FIG. 198 may be discriminants obtained by transformation of monotonic increase or monotonic decrease, such as linear transformation such as addition of a constant number or multiplication of a constant number, and logit transformation.

```
Index formula 54': 1.52561+0.01450×Cit-0.00423×Val-
0.01341×Met+0.01245×Ile-0.00764×His-0.01173×Trp
```

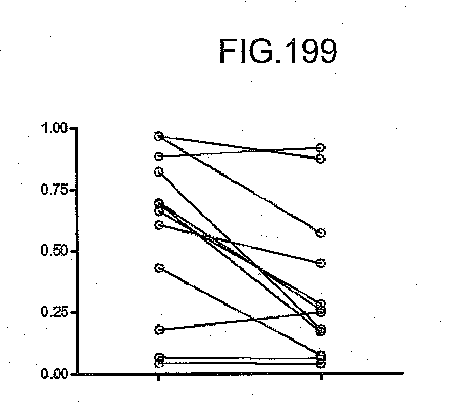

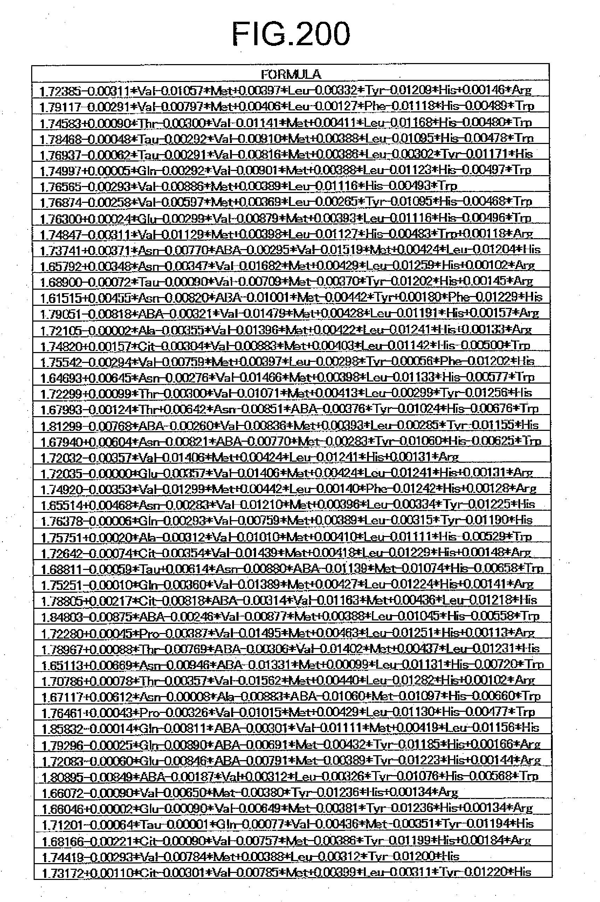

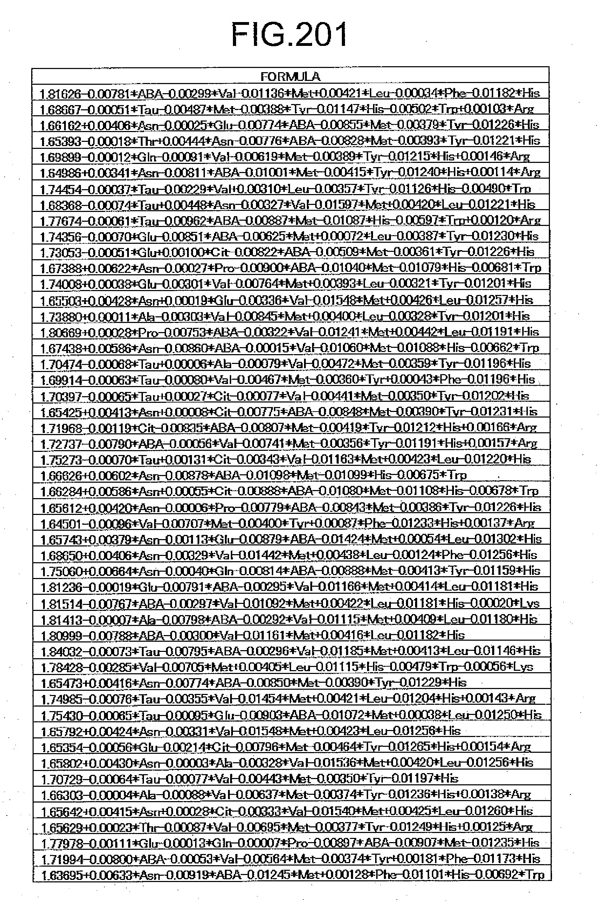

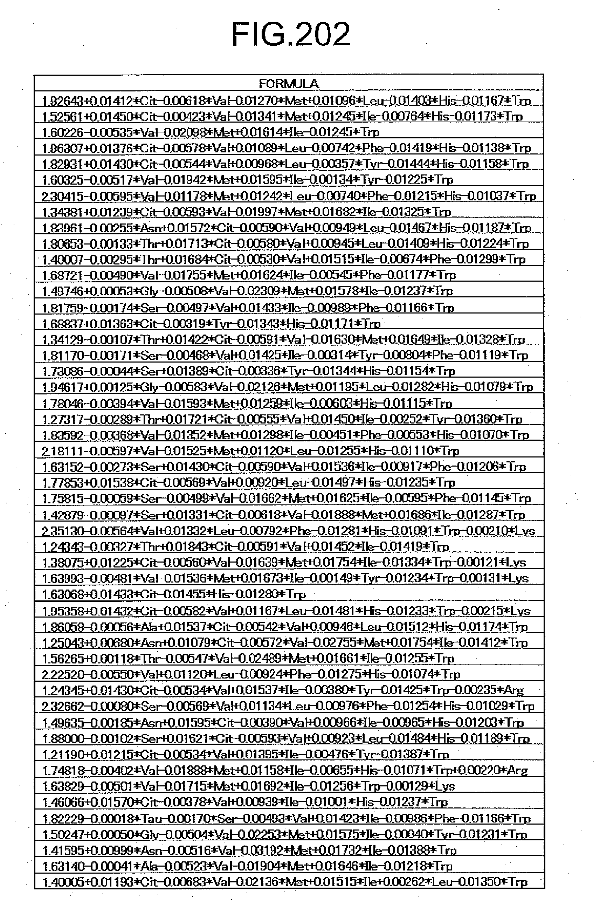

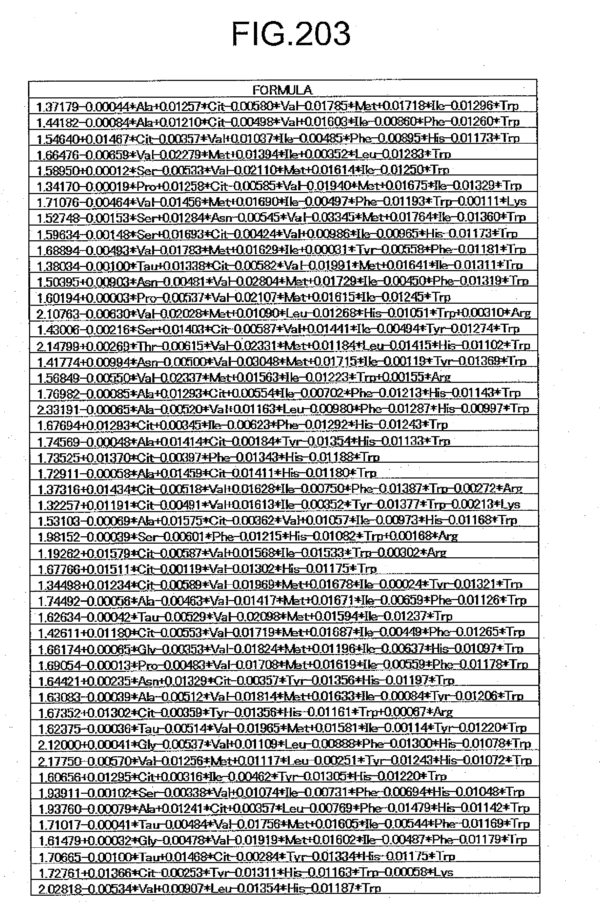

Subsequently, using the data obtained from same patients but on different blood sampling days among the samples measured in Example 8, the changes over time in the values of an index formula 55', which is equivalent to the index formula 55 obtained in Example 21 that is useful for the discrimination between the 2 groups of the active phase ulcerative colitis group and the remission phase ulcerative colitis group by logistic regression analysis, were compared for the period from the active phase to the remission phase, and thereby an evaluation of the index formula 55' was performed (FIG. 199). In FIG. 199, the samples connected by a line indicate that the samples belong to a same patient. A paired T-test between 2 groups was performed for the purpose of discriminating between the 2 groups of the active phase group and the remission phase group. In the remission phase group as compared with the active phase group, the values of the index formula significantly decreased (probability of significant difference P<0.05). It was made clear that the index formula 55' is a useful index even for the monitoring of the condition of ulcerative colitis in a same patient. In addition to that, a plurality of logistic regression equations of which the values significantly decreased (probability of significant difference P<0.05) in the remission phase as compared with the active phase, in the same manner as the index formula 55' did, were obtained. Those x's are presented in FIG. 200, FIG. 201, FIG. 202 and FIG. 203. The equations presented in FIG. 200, FIG. 201, FIG. 202 and FIG. 203 may be equations obtained by transformation of monotonic increase or monotonic decrease, such as linear transformation such as addition of a constant number or multiplication of a constant number, and logit transformation.

```
Index formula 55': 1/(1+exp(-x))
provided that x=7.1113-0.0564×His-0.0796×Trp-0.0208×Val-
0.1037×Met+0.0635×Ile+0.1045×Cit
```

INDUSTRIAL APPLICABILITY

As described above, the method of evaluating IBD, the amino acid data processor, the amino acid data-processing method, the amino acid data-processing system, the amino acid data-processing program and the recording medium according to the present invention may be practiced widely in many industrial fields, in particular in pharmaceutical, food, and medical fields, and are extremely useful in the field of bioinformatics,for example, performing disease state prediction of IBD, disease risk prediction, and proteome or metabolome analysis.

**Claims**

1.  A method of evaluating IBD, comprising:

    a measuring step of measuring amino acid concentration data on a concentration value of amino acid in blood collected from a subject to be evaluated; and
    a concentration value criterion evaluating step of evaluating an inflammatory bowel disease state in the subject, based on the amino acid concentration data of the subject measured at the measuring step.

2.  The method of evaluating IBD according to claim 1, wherein the concentration value criterion evaluating step further includes a concentration value criterion discriminating step of discriminating between inflammatory bowel disease and inflammatory bowel disease-free in the subject, based on the concentration value of at least one of Tau, Urea, Asn, Glu, Gln, Pro, Ala, Cit, ABA, Val, Cys, Met, Ile, Leu, Tyr, Phe, His, Trp, Orn and Lys contained in the amino acid concentration data of the subject measured at the measuring step.

3.  The method of evaluating IBD according to claim 1, wherein the concentration value criterion evaluating step further includes:

    a discriminant value calculating step of calculating a discriminant value that is a value of multivariate discriminant, based on both the concentration value of at least one of Tau, Urea, Asn, Glu, Gln, Pro, Ala, Cit, Gly, ABA, Val, Cys, Leu, Tyr, Phe, His, Trp, Orn and Lys contained in the amino acid concentration data of the subject measured at the measuring step and a previously established multivariate discriminant with the concentration of the amino acid as explanatory variable, where the concentration value of at least one of Tau, Urea, Asn, Glu, Gln, Pro, Ala, Cit, Gly, ABA, Val, Cys, Leu, Tyr, Phe, His, Trp, Orn and Lys is contained as the explanatory variable; and a discriminant value criterion discriminating step of discriminating between inflammatory bowel disease and inflammatory bowel disease-free in the subject, based on the discriminant value calculated at the discriminant value calculating step.

4. The method of evaluating IBD according to claim 3, wherein the multivariate discriminant is expressed by one fractional expression or the sum of a plurality of the fractional expressions, and contains the concentration value of at least one of Tau, Glu, Pro and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contains the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau, Glu, Pro and Cys as the explanatory variable in the denominator in the fractional expression constituting the multivariate discriminant; or

the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Tau, Leu, Tyr, His, Ile and Thr as the explanatory variables.

5. The method of evaluating IBD according to claim 1, wherein at the concentration value criterion evaluating step, Crohn disease state or ulcerative colitis state in the subject is evaluated, based on the amino acid concentration data of the subject measured at the measuring step.

6. The method of evaluating IBD according to claim 5, wherein the concentration value criterion evaluating step further includes a concentration value criterion discriminating step of discriminating between Crohn disease and Crohn disease-free in the subject, based on the concentration value of at least one of Tau, Thr, Urea, Asn, Glu, Gln, Pro, Gly, ABA, Val, Cys, Leu, Tyr, Phe, His, Trp and Lys contained in the amino acid concentration data of the subject measured at the measuring step, or discriminating between ulcerative colitis and ulcerative colitis-free in the subject, based on the concentration value of at least one of Tau, Urea, Asn, Gln, Ala, Cit, ABA, Val, Cys, Met, Ile, Leu, Tyr, Phe, His, Trp, Orn and Lys contained in the amino acid concentration data of the subject measured at the measuring step.

7. The method of evaluating IBD according to claim 5, wherein the concentration value criterion evaluating step further includes a concentration value criterion discriminating step of discriminating between active phase and remission phase of Crohn disease or ulcerative colitis in the subject, based on the amino acid concentration data of the subject measured at the measuring step.

8. The method of evaluating IBD according to claim 7, wherein at the concentration value criterion discriminating step, discrimination between active phase and remission phase of Crohn disease in the subject is conducted based on the concentration value of at least one of Gln, Cit, Val, Leu, His, Trp, Lys and Arg contained in the amino acid concentration data of the subject measured at the measuring step, or discrimination between active phase and remission phase of ulcerative colitis in the subject is conducted based on the concentration value of at least one of Urea, Gln, Thr, Asn, Pro, Ala, ABA, Val, Met, Leu, Tyr, Phe, His, Trp, Lys, Arg and Cys contained in the amino acid concentration data of the subject measured at the measuring step.

9. The method of evaluating IBD according to claim 5, wherein the concentration value criterion evaluating step further includes:

a discriminant value calculating step of calculating a discriminant value that is a value of multivariate discriminant, based on both the concentration value of at least one of Tau, Thr, Urea, Asn, Glu, Gln, Pro, Gly, ABA, Val, Cys, Leu, Tyr, Phe, His, Trp and Lys contained in the amino acid concentration data of the subject measured at the measuring step and a previously established multivariate discriminant with the concentration of the amino acid as explanatory variable, where the concentration value of at least one of Tau, Thr, Urea, Asn, Glu, Gln, Pro, Gly, ABA, Val, Cys, Leu, Tyr, Phe, His, Trp and Lys is contained as the explanatory variable, or calculating a discriminant value that is a value of multivariate discriminant, based on both the concentration value of at least one of Tau, Urea, Asn, Gln, Ala, Cit, ABA, Val, Cys, Met, Ile, Leu, Tyr, Phe, His, Trp, Orn and Lys contained in the amino acid concentration data of the subject measured at the measuring step and a previously established multivariate discriminant with the concentration of the amino acid as explanatory variable, where the concentration value of at least one of Tau, Urea, Asn, Gln, Ala, Cit, ABA, Val, Cys, Met, Ile, Leu, Tyr, Phe, His, Trp, Orn and Lys is contained as the explanatory variable; and

a discriminant value criterion discriminating step of discriminating between Crohn disease and Crohn disease-free in the subject, based on the discriminant value calculated at the discriminant value calculating step, or discriminating between ulcerative colitis and ulcerative colitis-free in the subject, based on the discriminant value calculated at the discriminant value calculating step.

10. The method of evaluating IBD according to claim 9, wherein when discrimination between Crohn disease and Crohn disease-free is conducted at the discriminant value criterion discriminating step,

the multivariate discriminant is expressed by one fractional expression or the sum of a plurality of fractional expressions, and contains the concentration value of at least one of Tau, Glu, Pro and Gly as the explanatory variable in the numerator in the fractional expression constituting the multivariate discriminant, and the concentration value of at least one of Urea, Asn, Gln, Thr, ABA, Val, Cys, Leu, Tyr, Phe, His, Trp and Lys as the explanatory variable in the denominator in the fractional expression constituting the multivariate discriminant, or contains the concentration value of at least one of Urea, Asn, Gln, Thr, ABA, Val, Cys, Leu, Tyr, Phe, His, Trp and Lys as the explanatory variable in the numerator in the fractional expression constituting the multivariate discriminant, and the concentration value of at least one of Tau, Glu, Pro and Gly as the explanatory variable in the denominator in the fractional expression constituting the multivariate discriminant; or

the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Tau, Val, Leu, Tyr, Pro and Ile as the explanatory variables; and

when discrimination between ulcerative colitis and ulcerative colitis-free is conducted at the discriminant value criterion discriminating step,

the multivariate discriminant is expressed by one fractional expression or the sum of a plurality of fractional expressions, and contains the concentration value of at least one of Tau and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contains the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau and Cys as the explanatory variable in the denominator in the fractional expression constituting the multivariate discriminant; or

the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Tau, Tyr, Leu, His, Ala and Ile as the explanatory variables.

11. The method of evaluating IBD according to claim 5, wherein the concentration value criterion evaluating step further includes:

a discriminant value calculating step of calculating a discriminant value that is a value of multivariate discriminant, based on both the amino acid concentration data of the subject measured at the measuring step and a previously established multivariate discriminant with the concentration of the amino acid as explanatory variable; and

a discriminant value criterion discriminating step of discriminating between active phase and remission phase of Crohn disease or ulcerative colitis in the subject, based on the discriminant value calculated at the discriminant value calculating step.

12. The method for evaluating IBD according to claim 5, wherein at the measuring step, a plurality of the amino acid concentration data are measured from every blood sample of the plurality of blood samples collected from the subject, and

the concentration value criterion evaluating step further includes:

a discriminant value calculating step of calculating a discriminant value that is a value of multivariate discriminant, based on both the plurality of the amino acid concentration data of the subject measured at the measuring step and a previously established multivariate discriminant with the concentration of the amino acid as explanatory variable, for each of the plurality of the amino acid concentration data; and

a discriminant value criterion condition evaluating step of evaluating a condition of Crohn disease or ulcerative colitis in the subject, based on the plurality of discriminant values calculated at the discriminant value calculating step.

13. The method of evaluating IBD according to claim 11 or 12, wherein when discrimination between active phase and remission phase of Crohn disease is conducted at the discriminant value criterion discriminating step, or when the condition of Crohn disease is evaluated at the discriminant value criterion condition evaluating step,

at the discriminant value calculating step, one or a plurality of the discriminant values are calculated based on both the concentration value of at least one of Gln, Cit, Val, Leu, His, Trp, Lys and Arg contained in one or a plurality of the amino acid concentration data of the subject measured at the measuring step, and the multivariate discriminant containing the concentration value of at least one of Gln, Cit, Val, Leu, His, Trp, Lys and Arg as the explanatory variable; and

when discrimination between active phase and remission phase of ulcerative colitis is conducted at the discriminant value criterion discriminating step, or when the condition of ulcerative colitis is evaluated at the discriminant value criterion condition evaluating step,

at the discriminant value calculating step, one or a plurality of the discriminant values are calculated based on both the concentration value of at least one of Tau, Urea, Thr, Asn, Pro, Gln, Ala, Cit, ABA, Val, Met, Leu, Tyr, Phe, His, Trp, Lys, Arg and Cys contained in one or a plurality of the amino acid concentration data of the subject measured at the measuring step, and the multivariate discriminant containing the concentration value of at least one of Tau, Urea, Thr, Asn, Pro, Gln, Ala, Cit, ABA, Val, Met, Leu, Tyr, Phe, His, Trp, Lys, Arg and Cys as the explanatory variable.

14. The method of evaluating IBD according to claim 13, wherein when discrimination between active phase and remission phase of Crohn disease is conducted at the discriminant value criterion discriminating step, or when the condition of Crohn disease is evaluated at the discriminant value criterion condition evaluating step,

the multivariate discriminant is expressed by one fractional expression or the sum of a plurality of fractional expressions and contains the concentration value of at least one of Gln, Cit, Val, Leu, His, Trp, Lys and Arg as the explanatory variable in any one of the numerator and denominator or both in the fractional expression constituting the multivariate discriminant, or

the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of His, Trp, Lys, Ser, Tau and Ile as the explanatory variables; and

when discrimination between active phase and remission phase of ulcerative colitis is conducted at the discriminant value criterion discriminating step, or when the condition of ulcerative colitis is evaluated at the discriminant value criterion condition evaluating step,

the multivariate discriminant is expressed by one fractional expression or the sum of a plurality of fractional expressions and contains the concentration value of at least one of Tau, Urea, Thr, Asn, Pro, Gln, Ala, Cit, ABA, Val, Met, Leu, Tyr, Phe, His, Trp, Lys, Arg and Cys as the explanatory variable in any one of the numerator and denominator or both in the fractional expression constituting the multivariate discriminant, or

the multivariate discriminant is any one of a logistic regression equation, a linear discriminant, a multiple regression equation, a discriminant prepared by a support vector machine, a discriminant prepared by a Mahalanobis' distance method, a discriminant prepared by canonical discriminant analysis and a discriminant prepared by a decision tree, and has the concentration value of His, Tyr, Val, Met, Leu and Arg as the explanatory variables.

15. The method of evaluating IBD according to claim 1, wherein the concentration value criterion evaluating step further includes a concentration value criterion discriminating step of discriminating between Crohn disease and ulcerative colitis in the subject, based on the amino acid concentration data of the subject measured at the measuring step.

16. The method of evaluating IBD according to claim 15, wherein at the concentration value criterion discriminating step, discrimination between Crohn disease and ulcerative colitis in the subject is conducted based on the concentration value of at least one of Cys, ABA, Thr, Pro, Gly, Met, Ile and Orn contained in the amino acid concentration data of the subject measured at the measuring step.

17. The method of evaluating IBD according to claim 15, wherein at the concentration value criterion discriminating step, discrimination between Crohn disease in active phase and ulcerative colitis in active phase in the subject is conducted based on the amino acid concentration data of the subject measured at the measuring step.

18. The method of evaluating IBD according to claim 17, wherein at the concentration value criterion discriminating step, discrimination between Crohn disease in active phase and ulcerative colitis in active phase in the subject is conducted based on the concentration value of at least one of Tau, Thr, Ser, Gly, Met and Ile contained in the amino acid concentration data of the subject measured at the measuring step.

19. The method of evaluating IBD according to claim 1, wherein the concentration value criterion evaluating step further includes:

a discriminant value calculating step of calculating a discriminant value that is a value of multivariate discriminant, based on both the amino acid concentration data of the subject measured at the measuring step, and a previously established multivariate discriminant with the concentration of the amino acid as explanatory variable; and
a discriminant value criterion discriminating step of discriminating between Crohn disease and ulcerative colitis in the subject, based on the discriminant value calculated at the discriminant value calculating step.

20. The method of evaluating IBD according to claim 19, wherein at the discriminant value calculating step, the discriminant value is calculated based on both the concentration value of at least one of Cys, ABA, Thr, Pro, Gly, Met, Ile and Orn contained in the amino acid concentration data of the subject measured at the measuring step, and the previously established multivariate discriminant containing the concentration value of at least one of Cys, ABA, Thr, Pro, Gly, Met, Ile and Orn as the explanatory variable.

21. The method of evaluating IBD according to claim 20, wherein the multivariate discriminant is expressed by one fractional expression or the sum of a plurality of fractional expressions, and contains the concentration value of at least one of Cys and ABA as the explanatory variable in the numerator in the fractional expression constituting the multivariate discriminant and the concentration value of at least one of Thr, Pro, Gly, Met, Ile and Orn as the explanatory variable in the denominator in the fractional expression constituting the multivariate discriminant, or contains the concentration value of at least one of Thr, Pro, Gly, Met, Ile and Orn as the explanatory variable in the numerator in the fractional expression constituting the multivariate discriminant and the concentration value of at least one of Cys and ABA as the explanatory variable in the denominator in the fractional expression constituting the multivariate discriminant; or
the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Met, Pro, Ile, Trp, Tau and Val as the explanatory variables.

22. The method of evaluating IBD according to claim 19, wherein at the discriminant value criterion discriminating step, discrimination between Crohn disease in active phase and ulcerative colitis in active phase in the subject is conducted based on the discriminant value calculated at the discriminant value calculating step.

23. The method of evaluating IBD according to claim 22, wherein at the discriminant value calculating step, the discriminant value is calculated based on both the concentration value of at least one of Tau, Thr, Ser, Gly, Met and Ile contained in the amino acid concentration data of the subject measured at the measuring step, and the multivariate discriminant containing the concentration value of at least one of Tau, Thr, Ser, Gly, Met and Ile as the explanatory variable.

24. The method of evaluating IBD according to claim 23, wherein the multivariate discriminant is expressed by one fractional expression or the sum of a plurality of fractional expressions, and contains the concentration value of at least one of Tau, Thr, Ser, Gly, Met and Ile as the explanatory variable in any one of the numerator and denominator or both in the fractional expression constituting the multivariate discriminant; or
the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Met, Tau, Trp, Tyr, Orn and Phe as the explanatory variables.

25. An amino acid data processor comprising a control unit and a memory unit to discriminate between inflammatory bowel disease and inflammatory bowel disease-free in a subject to be evaluated, wherein the control unit includes:

a discriminant value-calculating unit that calculates a discriminant value that is a value of multivariate discriminant, based on both the concentration value of at least one of Tau, Urea, Asn, Glu, Gln, Pro, Ala, Cit, Gly, ABA, Val, Cys, Leu, Tyr, Phe, His, Trp, Orn and Lys contained in previously obtained amino acid concentration data on a concentration value of amino acid in the subject and a multivariate discriminant with the concentration of the amino acid as explanatory variable stored in the memory unit, where the concentration value of at least one of Tau, Urea, Asn, Glu, Gln, Pro, Ala, Cit, Gly, ABA, Val, Cys, Leu, Tyr, Phe, His, Trp, Orn and Lys is contained as the explanatory variable; and

a discriminant value criterion-discriminating unit that discriminates between inflammatory bowel disease and inflammatory bowel disease-free in the subject, based on the discriminant value calculated by the discriminant value-calculating unit.

26. The amino acid data processor according to claim 25, wherein the multivariate discriminant is expressed by one fractional expression or the sum of a plurality of the fractional expressions, and contains the concentration value of at least one of Tau, Glu, Pro and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contains the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau, Glu, Pro and Cys as the explanatory variable in the denominator in the fractional expression constituting the multivariate discriminant; or

the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Tau, Leu, Tyr, His, Ile and Thr as the explanatory variables.

27. An amino acid data processor comprising a control unit and a memory unit to discriminate between Crohn disease and Crohn disease-free or between ulcerative colitis and ulcerative colitis-free in a subject to be evaluated, wherein the control unit includes:

a discriminant value-calculating unit that calculates a discriminant value that is a value of multivariate discriminant, based on both the concentration value of at least one of Tau, Thr, Urea, Asn, Glu, Gln, Pro, Gly, ABA, Val, Cys, Leu, Tyr, Phe, His, Trp and Lys contained in previously obtained amino acid concentration data on a concentration value of amino acid in the subject and a multivariate discriminant with the concentration of the amino acid as explanatory variable stored in the memory unit, where the concentration value of at least one of Tau, Thr, Urea, Asn, Glu, Gln, Pro, Gly, ABA, Val, Cys, Leu, Tyr, Phe, His, Trp and Lys is contained as the explanatory variable, or calculates a discriminant value that is a value of multivariate discriminant, based on both the concentration value of at least one of Tau, Urea, Asn, Gln, Ala, Cit, ABA, Val, Cys, Met, Ile, Leu, Tyr, Phe, His, Trp, Orn and Lys contained in previously obtained amino acid concentration data on a concentration value of amino acid in the subject and a multivariate discriminant with the concentration of the amino acid as explanatory variable stored in the memory unit, where the concentration value of at least one of Tau, Urea, Asn, Gln, Ala, Cit, ABA, Val, Cys, Met, Ile, Leu, Tyr, Phe, His, Trp, Orn and Lys is contained as the explanatory variable; and

a discriminant value criterion-discriminating unit that discriminates between Crohn disease and Crohn disease-free in the subject, based on the discriminant value calculated by the discriminant value-calculating unit, or discriminates between ulcerative colitis and ulcerative colitis-free in the subject, based on the discriminant value calculated by the discriminant value-calculating unit.

28. The amino acid data processor according to claim 27, wherein when the discriminant value criterion-discriminating unit discriminates between Crohn disease and Crohn disease-free, the multivariate discriminant is expressed by one fractional expression or the sum of a plurality of fractional expressions, and contains the concentration value of at least one of Tau, Glu, Pro and Gly as the explanatory variable in the numerator in the fractional expression constituting the multivariate discriminant, and the concentration value of at least one of Urea, Asn, Gln, Thr, ABA, Val, Cys, Leu, Tyr, Phe, His, Trp and Lys as the explanatory variable in the denominator in the fractional expression constituting the multivariate discriminant, or contains the concentration value of at least one of Urea, Asn, Gln, Thr, ABA, Val, Cys, Leu, Tyr, Phe, His, Trp and Lys as the explanatory variable in the numerator in the fractional expression constituting the multivariate discriminant, and the concentration value of at least one of Tau, Glu, Pro and Gly as the explanatory variable in the denominator in the fractional expression constituting the multivariate discriminant; or

the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Tau, Val, Leu, Tyr, Pro and Ile as the explanatory variables; and when the discriminant value criterion-discriminating unit discriminates between ulcerative colitis and ulcerative colitis-free, the multivariate discriminant is expressed by one fractional expression or the sum of a plurality of fractional expressions, and contains the concentration value of at least one of Tau and Cys as the explanatory variable in the numerator in the fractional expression constituting the multivariate discriminant, and the concentration value of at least one of Urea, Asn, Gln, Ala, Cit, ABA, Val, Met, Ile, Leu, Tyr, Phe, His, Trp, Orn and Lys as the explanatory variable in the denominator in the fractional expression constituting the multivariate discriminant, or contains the concentration value of at least one of Urea, Asn, Gln, Ala, Cit, ABA, Val, Met, Ile, Leu, Tyr, Phe, His, Trp, Orn and Lys as the explanatory variable in the numerator in the fractional expression constituting the multivariate discriminant, and the concentration value of at least one of Tau and Cys as the explanatory variable in the denominator in the fractional expression constituting the multivariate discriminant; or

the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Tau, Tyr, Leu, His, Ala and Ile as the explanatory variables.

29. An amino acid data processor comprising a control unit and a memory unit to discriminate between active phase and remission phase of Crohn disease or ulcerative colitis in a subject to be evaluated, wherein the control unit includes:

a discriminant value-calculating unit that calculates a discriminant value that is a value of multivariate discriminant, based on both previously obtained amino acid concentration data on a concentration value of amino acid in the subject and a multivariate discriminant with the concentration of the amino acid as explanatory variable stored in the memory unit; and
a discriminant value criterion-discriminating unit that discriminates between active phase and remission phase of Crohn disease or ulcerative colitis in the subject, based on the discriminant value calculated by the discriminant value-calculating unit.

30. An amino acid data processor comprising a control unit and a memory unit to evaluate a condition of Crohn disease or ulcerative colitis in a subject to be evaluated, wherein the control unit includes:

a discriminant value-calculating unit that calculates a discriminant value that is a value of multivariate discriminant, based on both a plurality of previously obtained amino acid concentration data on a concentration value of amino acid in the subject and a multivariate discriminant with the concentration of the amino acid as explanatory variable stored in the memory unit, for each of the plurality of the amino acid concentration data; and
a discriminant value criterion-condition evaluating unit that evaluates the condition of Crohn disease or ulcerative colitis in the subject, based on the plurality of the discriminant values calculated by the discriminant value-calculating unit.

31. The amino acid data processor according to claim 29 or 30, wherein when the discriminant value criterion-discriminating unit discriminates between active phase and remission phase of Crohn disease, or when the discriminant value criterion-condition evaluating unit evaluates the condition of Crohn disease,
the discriminant value-calculating unit calculates one or a plurality of the discriminant values, based on both the concentration value of at least one of Gln, Cit, Val, Leu, His, Trp, Lys and Arg contained in one or a plurality of the amino acid concentration data, and the multivariate discriminant containing the concentration value of at least one of Gln, Cit, Val, Leu, His, Trp, Lys and Arg as the explanatory variable; and
when the discriminant value criterion-discriminating unit discriminates between active phase and remission phase of ulcerative colitis, or when the discriminant value criterion-condition evaluating unit evaluates the condition of ulcerative colitis,
the discriminant value-calculating unit calculates one or a plurality of the discriminant values, based on both the concentration value of at least one of Tau, Urea, Thr, Asn, Pro, Gln, Ala, Cit, ABA, Val, Met, Leu, Tyr, Phe, His, Trp, Lys, Arg and Cys contained in one or a plurality of the amino acid concentration data, and the multivariate discriminant containing the concentration value of at least one of Tau, Urea, Thr, Asn, Pro, Gln, Ala, Cit, ABA, Val, Met, Leu, Tyr, Phe, His, Trp, Lys, Arg and Cys as the explanatory variable.

**32.** The amino acid data processor according to claim 31, wherein when the discriminant value criterion-discriminating unit discriminates between active phase and remission phase of Crohn disease, or when the discriminant value criterion-condition evaluating unit evaluates the condition of Crohn disease,
the multivariate discriminant is expressed by one fractional expression or the sum of a plurality of fractional expressions and contains the concentration value of at least one of Gln, Cit, Val, Leu, His, Trp, Lys and Arg as the explanatory variable in any one of the numerator and denominator or both in the fractional expression constituting the multivariate discriminant, or
the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of His, Trp, Lys, Ser, Tau and Ile as the explanatory variables; and
when the discriminant value criterion-discriminating unit discriminates between active phase and remission phase of ulcerative colitis, or when the discriminant value criterion-condition evaluating unit evaluates the condition of ulcerative colitis,
the multivariate discriminant is expressed by one fractional expression or the sum of a plurality of fractional expressions and contains the concentration value of at least one of Tau, Urea, Thr, Asn, Pro, Gln, Ala, Cit, ABA, Val, Met, Leu, Tyr, Phe, His, Trp, Lys, Arg and Cys as the explanatory variable in any one of the numerator and denominator or both in the fractional expression constituting the multivariate discriminant, or
the multivariate discriminant is any one of a logistic regression equation, a linear discriminant, a multiple regression equation, a discriminant prepared by a support vector machine, a discriminant prepared by a Mahalanobis' distance method, a discriminant prepared by canonical discriminant analysis and a discriminant prepared by a decision tree, and has the concentration value of His, Tyr, Val, Met, Leu and Arg as the explanatory variables.

**33.** An amino acid data processor comprising a control unit and a memory unit to discriminate between Crohn disease and ulcerative colitis in a subject to be evaluated, wherein the control unit includes:

a discriminant value-calculating unit that calculates a discriminant value that is a value of multivariate discriminant, based on both previously obtained amino acid concentration data on a concentration value of amino acid in the subject and a multivariate discriminant with the concentration of the amino acid as explanatory variable stored in the memory unit; and
a discriminant value criterion-discriminating unit that discriminates between Crohn disease and ulcerative colitis in the subject, based on the discriminant value calculated by the discriminant value-calculating unit.

**34.** The amino acid data processor according to claim 33, wherein the discriminant value-calculating unit calculates the discriminant value that is a value of the multivariate discriminant, based on both the concentration value of at least one of Cys, ABA, Thr, Pro, Gly, Met, Ile and Orn contained in the amino acid concentration data, and the multivariate discriminant containing the concentration value of at least one of Cys, ABA, Thr, Pro, Gly, Met, Ile and Orn as the explanatory variable.

**35.** The amino acid data processor according to claim 34, wherein the multivariate discriminant is expressed by one fractional expression or the sum of a plurality of fractional expressions, and contains the concentration value of at least one of Cys and ABA as the explanatory variable in the numerator in the fractional expression constituting the multivariate discriminant and the concentration value of at least one of Thr, Pro, Gly, Met, Ile and Orn as the explanatory variable in the denominator in the fractional expression constituting the multivariate discriminant, or contains the concentration value of at least one of Thr, Pro, Gly, Met, Ile and Orn as the explanatory variable in the numerator in the fractional expression constituting the multivariate discriminant and the concentration value of at least one of Cys and ABA as the explanatory variable in the denominator in the fractional expression constituting the multivariate discriminant; or
the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Met, Pro, Ile, Trp, Tau and Val as the explanatory variables.

**36.** The amino acid data processor according to claim 33, wherein the discriminant value criterion-discriminating unit discriminates between Crohn disease in active phase and ulcerative colitis in active phase in the subject, based on the discriminant value calculated by the discriminant value-calculating unit.

**37.** The amino acid data processor according to claim 36, wherein the discriminant value-calculating unit calculates the discriminant value, based on both the concentration value of at least one of Tau, Thr, Ser, Gly, Met and Ile contained in the amino acid concentration data, and the multivariate discriminant containing the concentration value of at least one of Tau, Thr, Ser, Gly, Met and Ile as the explanatory variable.

38. The amino acid data processor according to claim 37, wherein the multivariate discriminant is expressed by one fractional expression or the sum of a plurality of fractional expressions, and contains the concentration value of at least one of Tau, Thr, Ser, Gly, Met and Ile as the explanatory variable in any one of the numerator and denominator or both in the fractional expression constituting the multivariate discriminant; or
the multivariate discriminant is any one of a logistic regression equation, a linear discriminant, a multiple regression equation, a discriminant prepared by a support vector machine, a discriminant prepared by a Mahalanobis' generalized distance method, a discriminant prepared by canonical discriminant analysis and a discriminant prepared by a decision tree, and has the concentration value of Met, Tau, Trp, Tyr, Orn and Phe as the explanatory variables.

39. An amino acid data-processing method of discriminating between inflammatory bowel disease and inflammatory bowel disease-free in a subject to be evaluated, the method is carried out with an information processing apparatus including a control unit and a memory unit, the method comprising:

(i) a discriminant value calculating step of calculating a discriminant value that is a value of multivariate discriminant, based on both the concentration value of at least one of Tau, Urea, Asn, Glu, Gln, Pro, Ala, Cit, Gly, ABA, Val, Cys, Leu, Tyr, Phe, His, Trp, Orn and Lys contained in previously obtained amino acid concentration data on a concentration value of amino acid in the subject and a multivariate discriminant with the concentration of the amino acid as explanatory variable stored in the memory unit, where the concentration value of at least one of Tau, Urea, Asn, Glu, Gln, Pro, Ala, Cit, Gly, ABA, Val, Cys, Leu, Tyr, Phe, His, Trp, Orn and Lys is contained as the explanatory variable; and
(ii) a discriminant value criterion discriminating step of discriminating between inflammatory bowel disease and inflammatory bowel disease-free in the subject, based on the discriminant value calculated at the discriminant value calculating step,

wherein the steps (i) and (ii) are executed by the control unit.

40. An amino acid data-processing method of discriminating between Crohn disease and Crohn disease-free or between ulcerative colitis and ulcerative colitis-free in a subject to be evaluated, the method is carried out with an information processing apparatus including a control unit and a memory unit, the method comprising:

(i) a discriminant value calculating step of calculating a discriminant value that is a value of multivariate discriminant, based on both the concentration value of at least one of Tau, Thr, Urea, Asn, Glu, Gln, Pro, Gly, ABA, Val, Cys, Leu, Tyr, Phe, His, Trp and Lys contained in previously obtained amino acid concentration data on a concentration value of amino acid in the subject and a multivariate discriminant with the concentration of the amino acid as explanatory variable stored in the memory unit, where the concentration value of at least one of Tau, Thr, Urea, Asn, Glu, Gln, Pro, Gly, ABA, Val, Cys, Leu, Tyr, Phe, His, Trp and Lys is contained as the explanatory variable, or calculating a discriminant value that is a value of multivariate discriminant, based on both the concentration value of at least one of Tau, Urea, Asn, Gln, Ala, Cit, ABA, Val, Cys, Met, Ile, Leu, Tyr, Phe, His, Trp, Orn and Lys contained in previously obtained amino acid concentration data on a concentration value of amino acid in the subject and a multivariate discriminant with the concentration of the amino acid as explanatory variable stored in the memory unit, where the concentration value of at least one of Tau, Urea, Asn, Gln, Ala, Cit, ABA, Val, Cys, Met, Ile, Leu, Tyr, Phe, His, Trp, Orn and Lys is contained as the explanatory variable; and
(ii) a discriminant value criterion discriminating step of discriminating between Crohn disease and Crohn disease-free in the subject, based on the discriminant value calculated at the discriminant value calculating step, or discriminating between ulcerative colitis and ulcerative colitis-free in the subject, based on the discriminant value calculated at the discriminant value calculating step,

wherein the steps (i) and (ii) are executed by the control unit.

41. An amino acid data-processing method of discriminating between active phase and remission phase of Crohn disease or ulcerative colitis in a subject to be evaluated, the method is carried out with an information processing apparatus including a control unit and a memory unit, the method comprising:

(i) a discriminant value calculating step of calculating a discriminant value that is a value of multivariate discriminant, based on both previously obtained amino acid concentration data on a concentration value of amino acid in the subject and a multivariate discriminant with the concentration of the amino acid as explanatory variable stored in the memory unit; and

(ii) a discriminant value criterion discriminating step of discriminating between active phase and remission phase of Crohn disease or ulcerative colitis in the subject, based on the discriminant value calculated at the discriminant value calculating step,

wherein the steps (i) and (ii) are executed by the control unit.

42. An amino acid data-processing method of evaluating a condition of Crohn disease or ulcerative colitis in a subject to be evaluated, the method is carried out with an information processing apparatus including a control unit and a memory unit, the method comprising:

(i) a discriminant value calculating step of calculating a discriminant value that is a value of multivariate discriminant, based on both a plurality of previously obtained amino acid concentration data on a concentration value of amino acid in the subject and a multivariate discriminant with the concentration of the amino acid as explanatory variable stored in the memory unit, for each of the plurality of the amino acid concentration data; and

(ii) a discriminant value criterion condition evaluating step of evaluating the condition of Crohn disease or ulcerative colitis in the subject, based on the plurality of the discriminant values calculated at the discriminant value calculating step,

wherein the steps (i) and (ii) are executed by the control unit.

43. An amino acid data-processing method of discriminating between Crohn disease and ulcerative colitis in a subject to be evaluated, the method is carried out with an information processing apparatus including a control unit and a memory unit, the method comprising:

(i) a discriminant value calculating step of calculating a discriminant value that is a value of multivariate discriminant, based on both previously obtained amino acid concentration data on a concentration value of amino acid in the subject and a multivariate discriminant with the concentration of the amino acid as explanatory variable stored in the memory unit; and

(ii) a discriminant value criterion discriminating step of discriminating between Crohn disease and ulcerative colitis in the subject, based on the discriminant value calculated at the discriminant value calculating step,

wherein the steps (i) and (ii) are executed by the control unit.

44. The amino acid data-processing method according to claim 43, wherein at the discriminant value criterion discriminating step, discrimination between Crohn disease in active phase and ulcerative colitis in active phase in the subject is conducted based on the discriminant value calculated at the discriminant value calculating step.

45. An amino acid data-processing system comprising an amino acid data processor including a control unit and a memory unit to discriminate between inflammatory bowel disease and inflammatory bowel disease-free in a subject to be evaluated and an information communication terminal apparatus that provides amino acid concentration data on the concentration value of amino acid in the subject connected to each other communicatively via a network, wherein the information communication terminal apparatus includes:

an amino acid concentration data-sending unit that transmits the amino acid concentration data of the subject to the amino acid data processor; and

a discrimination result-receiving unit that receives the discrimination result as to discrimination between inflammatory bowel disease and inflammatory bowel disease-free of the subject transmitted from the amino acid data processor,

wherein the control unit of the amino acid data processor includes:

an amino acid concentration data-receiving unit that receives the amino acid concentration data of the subject transmitted from the information communication terminal apparatus;

a discriminant value-calculating unit that calculates a discriminant value that is a value of multivariate discriminant, based on both the concentration value of at least one of Tau, Urea, Asn, Glu, Gln, Pro, Ala, Cit, Gly, ABA, Val, Cys, Leu, Tyr, Phe, His, Trp, Orn and Lys contained in the amino acid concentration data of the subject received by the amino acid concentration data-receiving unit and a multivariate discriminant with the concentration of the amino acid as explanatory variable stored in the memory unit, where the concentration value of at least one of Tau, Urea, Asn, Glu, Gln, Pro, Ala, Cit, Gly, ABA, Val, Cys, Leu, Tyr, Phe, His, Trp, Orn and Lys is contained as the explanatory variable;

a discriminant value criterion-discriminating unit that discriminates between inflammatory bowel disease and inflammatory bowel disease-free in the subject, based on the discriminant value calculated by the discriminant value-calculating unit; and

a discrimination result-sending unit that transmits the discrimination result of the subject obtained by the discriminant value criterion-discriminating unit to the information communication terminal apparatus.

46. An amino acid data-processing system comprising an amino acid data processor including a control unit and a memory unit to discriminate between Crohn disease and Crohn disease-free or between ulcerative colitis and ulcerative colitis-free in a subject to be evaluated and an information communication terminal apparatus that provides amino acid concentration data on the concentration value of amino acid in the subject connected to each other communicatively via a network,

wherein the information communication terminal apparatus includes:

an amino acid concentration data-sending unit that transmits the amino acid concentration data of the subject to the amino acid data processor; and

a discrimination result-receiving unit that receives the discrimination result as to discrimination between Crohn disease and Crohn disease-free or between ulcerative colitis and ulcerative colitis-free of the subject transmitted from the amino acid data processor,

wherein the control unit of the amino acid data processor includes:

an amino acid concentration data-receiving unit that receives the amino acid concentration data of the subject transmitted from the information communication terminal apparatus;

a discriminant value-calculating unit that calculates a discriminant value that is a value of multivariate discriminant, based on both the concentration value of at least one of Tau, Thr, Urea, Asn, Glu, Gln, Pro, Gly, ABA, Val, Cys, Leu, Tyr, Phe, His, Trp and Lys contained in the amino acid concentration data of the subject received by the amino acid concentration data-receiving unit and a multivariate discriminant with the concentration of the amino acid as explanatory variable stored in the memory unit, where the concentration value of at least one of Tau, Thr, Urea, Asn, Glu, Gln, Pro, Gly, ABA, Val, Cys, Leu, Tyr, Phe, His, Trp and Lys is contained as the explanatory variable, or calculates a discriminant value that is a value of multivariate discriminant, based on both the concentration value of at least one of Tau, Urea, Asn, Gln, Ala, Cit, ABA, Val, Cys, Met, Ile, Leu, Tyr, Phe, His, Trp, Orn and Lys contained in the amino acid concentration data of the subject received by the amino acid concentration data-receiving unit and a multivariate discriminant with the concentration of the amino acid as explanatory variable stored in the memory unit, where the concentration value of at least one of Tau, Urea, Asn, Gln, Ala, Cit, ABA, Val, Cys, Met, Ile, Leu, Tyr, Phe, His, Trp, Orn and Lys is contained as the explanatory variable;

a discriminant value criterion-discriminating unit that discriminates between Crohn disease and Crohn disease-free in the subject, based on the discriminant value calculated by the discriminant value-calculating unit, or discriminates between ulcerative colitis and ulcerative colitis-free in the subject, based on the discriminant value calculated by the discriminant value-calculating unit; and

a discrimination result-sending unit that transmits the discrimination result of the subject obtained by the discriminant value criterion-discriminating unit to the information communication terminal apparatus.

47. An amino acid data-processing system comprising an amino acid data processor including a control unit and a memory unit to discriminate between active phase and remission phase of Crohn disease or ulcerative colitis in a subject to be evaluated and an information communication terminal apparatus that provides amino acid concentration data on the concentration value of amino acid in the subject connected to each other communicatively via a network, wherein the information communication terminal apparatus includes:

an amino acid concentration data-sending unit that transmits the amino acid concentration data of the subject to the amino acid data processor; and

a discrimination result-receiving unit that receives the discrimination result as to discrimination between active phase and remission phase of Crohn disease or ulcerative colitis of the subject transmitted from the amino acid data processor,

wherein the control unit of the amino acid data processor includes:

an amino acid concentration data-receiving unit that receives the amino acid concentration data of the subject transmitted from the information communication terminal apparatus;

a discriminant value-calculating unit that calculates a discriminant value that is a value of multivariate discriminant, based on both the amino acid concentration data of the subject received by the amino acid concentration data-receiving unit and a multivariate discriminant with the concentration of the amino acid as explanatory variable stored in the memory unit;

a discriminant value criterion-discriminating unit that discriminates between active phase and remission phase of Crohn disease or ulcerative colitis in the subject, based on the discriminant value calculated by the discriminant value-calculating unit; and

a discrimination result-sending unit that transmits the discrimination result of the subject obtained by the discriminant value criterion-discriminating unit to the information communication terminal apparatus.

48. An amino acid data-processing system comprising an amino acid data processor including a control unit and a memory unit to evaluate a condition of Crohn disease or ulcerative colitis in a subject to be evaluated and an information communication terminal apparatus that provides a plurality of amino acid concentration data on the concentration value of amino acid in the subject connected to each other communicatively via a network, wherein the information communication terminal apparatus includes:

an amino acid concentration data-sending unit that transmits the plurality of the amino acid concentration data of the subject to the amino acid data processor; and

an evaluation result-receiving unit that receives the evaluation result as to the condition of Crohn disease or ulcerative colitis of the subject transmitted from the amino acid data processor,

wherein the control unit of the amino acid data processor includes:

an amino acid concentration data-receiving unit that receives the plurality of the amino acid concentration data of the subject transmitted from the information communication terminal apparatus;

a discriminant value-calculating unit that calculates a discriminant value that is a value of multivariate discriminant, based on both the plurality of the amino acid concentration data of the subject received by the amino acid concentration data-receiving unit and a multivariate discriminant with the concentration of the amino acid as explanatory variable stored in the memory unit, for each of the plurality of the amino acid concentration data;

a discriminant value criterion-condition evaluating unit that evaluates the condition of Crohn disease or ulcerative colitis in the subject, based on the plurality of the discriminant values calculated by the discriminant value-calculating unit; and

an evaluation result-sending unit that transmits the evaluation result of the subject obtained by the discriminant value criterion-condition evaluating unit to the information communication terminal apparatus.

49. An amino acid data-processing system comprising an amino acid data processor including a control unit and a memory unit to discriminate between Crohn disease and ulcerative colitis in a subject to be evaluated and an information communication terminal apparatus that provides amino acid concentration data on the concentration value of amino acid in the subject connected to each other communicatively via a network, wherein the information communication terminal apparatus includes:

an amino acid concentration data-sending unit that transmits the amino acid concentration data of the subject to the amino acid data processor; and

a discrimination result-receiving unit that receives the discrimination result as to discrimination between Crohn disease and ulcerative colitis of the subject transmitted from the amino acid data processor,

wherein the control unit of the amino acid data processor includes:

an amino acid concentration data-receiving unit that receives the amino acid concentration data of the subject transmitted from the information communication terminal apparatus;

a discriminant value-calculating unit that calculates a discriminant value that is a value of multivariate discriminant, based on both the amino acid concentration data of the subject received by the amino acid concentration data-receiving unit and a multivariate discriminant with the concentration of the amino acid as explanatory variable stored in the memory unit;

a discriminant value criterion-discriminating unit that discriminates between Crohn disease and ulcerative colitis in the subject, based on the discriminant value calculated by the discriminant value-calculating unit; and

a discrimination result-sending unit that transmits the discrimination result of the subject obtained by the discriminant value criterion-discriminating unit to the information communication terminal apparatus.

50. The amino acid data-processing system according to claim 49, wherein the discrimination result is a discrimination result as to discrimination between Crohn disease in active phase and ulcerative colitis in active phase, and the discriminant value criterion-discriminating unit discriminates between Crohn disease in active phase and ulcerative colitis in active phase in the subject, based on the discriminant value calculated by the discriminant value-calculating unit.

51. An amino acid data-processing program product that makes an information processing apparatus including a control unit and a memory unit execute a method of discriminating between inflammatory bowel disease and inflammatory bowel disease-free in a subject to be evaluated, the method comprising:

(i) a discriminant value calculating step of calculating a discriminant value that is a value of multivariate discriminant, based on both the concentration value of at least one of Tau, Urea, Asn, Glu, Gln, Pro, Ala, Cit, Gly, ABA, Val, Cys, Leu, Tyr, Phe, His, Trp, Orn and Lys contained in previously obtained amino acid concentration data on a concentration value of amino acid in the subject and a multivariate discriminant with the concentration of the amino acid as explanatory variable stored in the memory unit, where the concentration value of at least one of Tau, Urea, Asn, Glu, Gln, Pro, Ala, Cit, Gly, ABA, Val, Cys, Leu, Tyr, Phe, His, Trp, Orn and Lys is contained as the explanatory variable; and
(ii) a discriminant value criterion discriminating step of discriminating between inflammatory bowel disease and inflammatory bowel disease-free in the subject, based on the discriminant value calculated at the discriminant value calculating step,

wherein the steps (i) and (ii) are executed by the control unit.

52. An amino acid data-processing program product that makes an information processing apparatus including a control unit and a memory unit execute a method of discriminating between Crohn disease and Crohn disease-free or between ulcerative colitis and ulcerative colitis-free in a subject to be evaluated, the method comprising:

(i) a discriminant value calculating step of calculating a discriminant value that is a value of multivariate discriminant, based on both the concentration value of at least one of Tau, Thr, Urea, Asn, Glu, Gln, Pro, Gly, ABA, Val, Cys, Leu, Tyr, Phe, His, Trp and Lys contained in previously obtained amino acid concentration data on a concentration value of amino acid in the subject and a multivariate discriminant with the concentration of the amino acid as explanatory variable stored in the memory unit, where the concentration value of at least one of Tau, Thr, Urea, Asn, Glu, Gln, Pro, Gly, ABA, Val, Cys, Leu, Tyr, Phe, His, Trp and Lys is contained as the explanatory variable, or calculating a discriminant value that is a value of multivariate discriminant, based on both the concentration value of at least one of Tau, Urea, Asn, Gln, Ala, Cit, ABA, Val, Cys, Met, Ile, Leu, Tyr, Phe, His, Trp, Orn and Lys contained in previously obtained amino acid concentration data on a concentration value of amino acid in the subject and a multivariate discriminant with the concentration of the amino acid as explanatory variable stored in the memory unit, where the concentration value of at least one of Tau, Urea, Asn, Gln, Ala, Cit, ABA, Val, Cys, Met, Ile, Leu, Tyr, Phe, His, Trp, Orn and Lys is contained as the explanatory variable; and
(ii) a discriminant value criterion discriminating step of discriminating between Crohn disease and Crohn disease-free in the subject, based on the discriminant value calculated at the discriminant value calculating step, or discriminating between ulcerative colitis and ulcerative colitis-free in the subject, based on the discriminant value calculated at the discriminant value calculating step,

wherein the steps (i) and (ii) are executed by the control unit.

53. An amino acid data-processing program product that makes an information processing apparatus including a control unit and a memory unit execute a method of discriminating between active phase and remission phase of Crohn disease or ulcerative colitis in a subject to be evaluated, the method comprising:

(i) a discriminant value calculating step of calculating a discriminant value that is a value of multivariate discriminant, based on both previously obtained amino acid concentration data on a concentration value of amino acid in the subject and a multivariate discriminant with the concentration of the amino acid as explanatory variable stored in the memory unit; and

(ii) a discriminant value criterion discriminating step of discriminating between active phase and remission phase of Crohn disease or ulcerative colitis in the subject, based on the discriminant value calculated at the discriminant value calculating step,

wherein the steps (i) and (ii) are executed by the control unit.

54. An amino acid data-processing program product that makes an information processing apparatus including a control unit and a memory unit execute a method of evaluating a condition of Crohn disease or ulcerative colitis in a subject to be evaluated, the method comprising:

(i) a discriminant value calculating step of calculating a discriminant value that is a value of multivariate discriminant, based on both a plurality of previously obtained amino acid concentration data on a concentration value of amino acid in the subject and a multivariate discriminant with the concentration of the amino acid as explanatory variable stored in the memory unit, for each of the plurality of the amino acid concentration data; and

(ii) a discriminant value criterion condition evaluating step of evaluating the condition of Crohn disease or ulcerative colitis in the subject, based on the plurality of the discriminant values calculated at the discriminant value calculating step,

wherein the steps (i) and (ii) are executed by the control unit.

55. An amino acid data-processing program product that makes an information processing apparatus including a control unit and a memory unit execute a method of discriminating between Crohn disease and ulcerative colitis in a subject to be evaluated, the method comprising:

(i) a discriminant value calculating step of calculating a discriminant value that is a value of multivariate discriminant, based on both previously obtained amino acid concentration data on a concentration value of amino acid in the subject and a multivariate discriminant with the concentration of the amino acid as explanatory variable stored in the memory unit; and

(ii) a discriminant value criterion discriminating step of discriminating between Crohn disease and ulcerative colitis in the subject, based on the discriminant value calculated at the discriminant value calculating step,

wherein the steps (i) and (ii) are executed by the control unit.

56. The amino acid data-processing program product according to claim 55, wherein at the discriminant value criterion discriminating step, discrimination between Crohn disease in active phase and ulcerative colitis in active phase in the subject is conducted based on the discriminant value calculated at the discriminant value calculating step.

57. A computer-readable recording medium, comprising the amino acid data-processing program product according to any one of claims 51 to 56 recorded thereon.

# FIG.1

(BASIC PRINCIPLE OF THE INVENTION)

BLOOD SAMPLE OF
SUBJECT TO BE
EVALUATED

S-11

MEASUREMENT OF AMINO
ACID CONCENTRATION
DATA

S-12

EVALUATION OF IBD STATE ← AMINO ACID
CONCENTRATION
DATA OF SUBJECT
TO BE EVALUATED

EVALUATION
RESULTS

# FIG.2

START

MEASURE AMINO ACID CONCENTRATION DATA — SA-11

REMOVE DATA — SA-12

DISCRIMINATE BETWEEN IBD AND IBD-FREE,
BETWEEN CD AND CD-FREE,
BETWEEN ACTIVE PHASE AND REMISSION PHASE
OF CD,
BETWEEN UC AND UC-FREE,
BETWEEN ACTIVE PHASE AND REMISSION PHASE
OF UC, BETWEEN CD AND UC,
OR BETWEEN CD IN ACTIVE PHASE AND UC IN
ACTIVE PHASE, OR EVALUATE CONDITION OF CD,
OR CONDITION OF UC — SA-13

END

# FIG.3

(BASIC PRINCIPLE OF THE INVENTION)

IBD-EVALUATING
APPARATUS

| MEMORY DEVICE | CONTROL DEVICE |
|---|---|

MULTIVARIATE
DISCRIMINANT

S-21

CALCULATION OF
DISCRIMINANT VALUE

AMINO ACID
CONCENTRATION
DATA OF SUBJECT
TO BE EVALUATED

S-22

EVALUATION OF IBD
STATE

EVALUATION
RESULTS

# FIG.4

200

CLIENT APPARATUS
(INFORMATION
COMMUNICATION
TERMINAL
APPARATUS)

100

200

CLIENT APPARATUS
(INFORMATION
COMMUNICATION
TERMINAL
APPARATUS)

IBD-
EVALUATING
APPARATUS

300

NETWORK

200

CLIENT APPARATUS
(INFORMATION
COMMUNICATION
TERMINAL
APPARATUS)

200

CLIENT APPARATUS
(INFORMATION
COMMUNICATION
TERMINAL
APPARATUS)

# FIG.5

200

CLIENT APPARATUS
(INFORMATION
COMMUNICATION
TERMINAL
APPARATUS)

200

CLIENT APPARATUS
(INFORMATION
COMMUNICATION
TERMINAL
APPARATUS)

100

IBD-
EVALUATING
APPARATUS

300

NETWORK

200

CLIENT APPARATUS
(INFORMATION
COMMUNICATION
TERMINAL
APPARATUS)

400

DATABASE
APPARATUS

200

CLIENT APPARATUS
(INFORMATION
COMMUNICATION
TERMINAL
APPARATUS)

FIG.6

# FIG.7

106a

| USER ID | USER PASSWORD | NAME | ORGANI-ZATION ID | DEPART-MENT ID | DEPART-MENT NAME | E-MAIL ADDRESS | . . . |
|---|---|---|---|---|---|---|---|
| . . . | . . . | . . . | . . . | . . . | . . . | . . . | . . . |

# FIG.8

106b

| INDIVIDUAL (SAMPLE) NO. | AMINO ACID CONCENTRATION DATA | | | | | |
|---|---|---|---|---|---|---|
| | Gly | Leu | Val | Ile | Phe | . . . |
| U-1 | 9.5 | 11.2 | 2.7 | 8.5 | 4.9 | . . . |
| U-2 | 8.5 | 10.5 | 3.9 | 9.8 | 6.1 | . . . |
| . . . | . . . | . . . | . . . | . . . | . . . | . . . |

# FIG.9

EP 2 124 059 A1

106c

| INDIVIDUAL (SAMPLE) NO. | IBD STATE INDEX DATA (T) | | | | AMINO ACID CONCENTRATION DATA | | | | | |
|---|---|---|---|---|---|---|---|---|---|---|
| | $T_1$ | $T_2$ | $T_3$ | ... | Gly | Leu | Val | Ile | Phe | ... |
| A-1 | 23.4 | 62.5 | 37.1 | ... | 9.5 | 11.2 | 2.7 | 8.5 | 4.9 | ... |
| A-2 | 27.5 | 66.1 | 39.5 | ... | 8.5 | 10.5 | 3.9 | 9.8 | 6.1 | ... |
| ⋮ | ⋮ | ⋮ | ⋮ | ⋮ | ⋮ | | | | | |

# FIG.10

106d

| INDIVIDUAL (SAMPLE) NO. | IBD STATE INDEX DATA (T) | AMINO ACID CONCENTRATION DATA | | | |
|---|---|---|---|---|---|
| | $T_2$ | Gly | Leu | Phe | ... |
| A-1 | 62.5 | 9.5 | 11.2 | 4.9 | ... |
| A-2 | 66.1 | 8.5 | 10.5 | 6.1 | ... |
| : | : | : | | | : |

# FIG.11

106e1

| RANK | CANDIDATE MULTIVARIATE DISCRIMINANT |
|---|---|
| 1 | $F_1$(Gly,Leu,Phe,...) |
| 2 | $F_2$(Gly,Leu,Phe,...) |
| 3 | $F_3$(Gly,Leu,Phe,...) |
| : | : |

# FIG.12

106e2

| RANK | CANDIDATE MULTIVARIATE DISCRIMINANT | VERIFICATION RESULT |
|---|---|---|
| 1 | $F_k$(Gly,Leu,Phe,...) | 1.22 |
| 2 | $F_m$(Gly,Leu,Phe,...) | 2.28 |
| 3 | $F_l$(Gly,Leu,Phe,...) | 2.95 |
| ⋮ | ⋮ | ⋮ |

# FIG.13

106e3

| INDIVIDUAL (SAMPLE) NO. | IBD STATE INDEX DATA (T) | AMINO ACID CONCENTRATION DATA | | |
|---|---|---|---|---|
| | $T_2$ | Leu | Phe | ... |
| A-1 | 62.5 | 11.2 | 4.9 | ... |
| A-2 | 66.1 | 10.5 | 6.1 | ... |
| ⋮ | ⋮ | | | |

# FIG.14

106e4

| RANK | MULTIVARIATE DISCRIMINANT | THRESHOLD VALUE | VERIFICATION RESULT |
|---|---|---|---|
| 1 | $F_p(Phe,...)$ | 0.23 | 0.62 |
| 2 | $F_p(Gly,Leu,Phe)$ | -2.12 | 1.02 |
| 3 | $F_k(Gly,Leu,Phe,...)$ | 1.23 | 1.22 |
| ⋮ | ⋮ | ⋮ | ⋮ |

# FIG.15

106f

| INDIVIDUAL (SAMPLE) NO. | RANK | DISCRIMINANT VALUE |
|---|---|---|
| U-1 | 1 | 1.13 |
| ⋮ | ⋮ | ⋮ |

# FIG.16

106g

| INDIVIDUAL (SAMPLE) NO. | AMINO ACID CONCENTRATION DATA | | | | DISCRIM-INANT VALUE | EVALUA-TION RESULT |
|---|---|---|---|---|---|---|
| | Gly | Leu | Phe | ⋯ | | |
| U-1 | 9.5 | 11.2 | 4.9 | ⋯ | | |
| U-2 | 8.5 | 10.5 | 6.1 | ⋯ | | |
| ⋮ | ⋮ | ⋮ | ⋮ | ⋮ | | |

# FIG.17

102h

MULTIVARIATE DISCRIMINANT-PREPARING PART

CANDIDATE MULTIVARIATE DISCRIMINANT-PREPARING PART　102h1

CANDIDATE MULTIVARIATE DISCRIMINANT-VERIFYING PART　102h2

EXPLANATORY VARIABLE-SELECTING PART　102h3

# FIG.18

~102j

DISCRIMINANT VALUE CRITERION-
EVALUATING PART

| DISCRIMINANT VALUE CRITERION-DISCRIMINATING PART | ~102j1 |

| DISCRIMINANT VALUE CRITERION-CONDITION EVALUATING PART | ~102j2 |

# FIG.19

200

CLIENT APPARATUS

250    270

INPUT DEVICE

260

OUTPUT DEVICE

261 — MONITOR

262 — PRINTER

INPUT/OUTPUT IF

240    220    230

RAM    ROM    HD

280

COMMUNICATION IF

300

NETWORK

210

CONTROL DEVICE

| WEB BROWSER | ~211 |
| ELECTRONIC MAILER | ~212 |
| RECEIVING PART | ~213 |
| SENDING PART | ~214 |

# FIG.20

# FIG.21

(DATABASE APPARATUS 400)    (IBD-EVALUATING APPARATUS 100)    (CLIENT APPARATUS 200)

START    START    START

SA-22    SA-23    SA-21

| TRANSMIT MULTIVARIATE DISCRIMINANT | → | RECEIVE MULTIVARIATE DISCRIMINANT AND AMINO ACID CONCENTRATION DATA | ← | TRANSMIT AMINO ACID CONCENTRATION DATA |

SA-24

REMOVE DATA

SA-25

CALCULATE DISCRIMINANT VALUE

SA-26

·DISCRIMINATE BETWEEN IBD AND IBD-FREE,
BETWEEN CD AND CD-FREE,
BETWEEN ACTIVE PHASE AND REMISSION PHASE OF CD,
BETWEEN UC AND UC-FREE,
BETWEEN ACTIVE PHASE AND REMISSION PHASE OF UC,
BETWEEN CD AND UC, OR BETWEEN CD IN ACTIVE PHASE AND UC IN ACTIVE PHASE,
·OR EVALUATE CONDITION OF CD, OR CONDITION OF UC

SA-27

TRANSMIT EVALUATION RESULT

SA-28    SA-29

STORE EVALUATION RESULT    OUTPUT EVALUATION RESULT

END    END    END

# FIG.22

START

PREPARE CANDIDATE
MULTIVARIATE DISCRIMINANT — SB-21

VERIFY CANDIDATE
MULTIVARIATE DISCRIMINANT — SB-22

SELECT EXPLANATORY
VARIABLE OF CANDIDATE
MULTIVARIATE
DISCRIMINANT — SB-23

SB-24

ALL
COMBINATIONS
WERE ENDED?

NO

YES

SELECT CANDIDATE
MULTIVARIATE DISCRIMINANT
(DECIDE MULTIVARIATE
DISCRIMINANT) — SB-25

END

# FIG.23

# FIG.24

# FIG.25

# FIG.26

# FIG.27

| ROC AUC | FORMULA |
|---|---|
| 0.9480 | (Orn)/(Val)+(Tau+Cys)/(Trp+His) |
| 0.9452 | (Tau)/(ABA+Tyr+Trp)+(Cys)/(His) |
| 0.9452 | (Tau)/(Leu+His)+(Cys)/(Tyr+Trp) |
| 0.9443 | (Tau)/(Asn+Tyr+Trp)+(Cys)/(His) |
| 0.9441 | (Tau)/(Tyr+Trp)+(Cys)/(His) |
| 0.9439 | (Tau)/(Tyr+His)+(Cys)/(ABA+Trp) |
| 0.9436 | (Tau)/(Tyr+Val)+(Cys)/(Trp+His) |
| 0.9435 | (Tau)/(Tyr+Leu)+(Cys)/(Trp+His) |
| 0.9434 | (Gly)/(Urea)+(Tau+Cys)/(Trp+His) |
| 0.9433 | (Orn)/(Val)+(Tau+Cys)/(Asn+His) |
| 0.9433 | (Tau)/(Tyr+Leu)+(Cys)/(ABA+Trp) |
| 0.9428 | (Tau)/(Tyr+Leu)+(Cys)/(Asn+Trp) |
| 0.9425 | (Tau)/(Asn+His)+(Cys)/(ABA+Trp) |
| 0.9425 | (Orn)/(Val)+(Tau+Cys)/(Tyr+Trp) |
| 0.9423 | (Orn)/(Val)+(Tau+Cys)/(Asn+Trp) |
| 0.9417 | (Orn)/(Val)+(Tau+Cys)/(Tyr+His) |
| 0.9413 | (Tau)/(Met+Tyr+Trp)+(Cys)/(His) |
| 0.9413 | (Tau)/(Tyr+Leu)+(Cys)/(Cit+Trp) |
| 0.9409 | (Tau)/(Asn+Leu)+(Cys)/(Tyr+Trp) |
| 0.9408 | (Tau)/(Asn+Leu)+(Cys)/(ABA+Trp) |
| 0.9407 | (Tau)/(Cit+Tyr+Trp)+(Cys)/(His) |
| 0.9406 | (Orn)/(Tyr+Trp)+(Tau+Cys)/(His) |
| 0.9405 | (Tau)/(His)+(Cys+Orn)/(Tyr+Trp) |
| 0.9405 | (Tau)/(Leu)+(Cys+Orn)/(Trp+His) |
| 0.9403 | (Tau)/(Asn+Val)+(Cys)/(Trp+His) |
| 0.9402 | (Tau)/(Tyr+Trp)+(Cys)/(ABA+His) |
| 0.9401 | (Tau)/(Asn+Val)+(Cys)/(Tyr+Trp) |
| 0.9400 | (Tau)/(Tyr+Leu)+(Cys)/(His) |
| 0.9400 | (Tau)/(Asn+Tyr)+(Cys)/(ABA+Trp) |
| 0.9400 | (Tau)/(Tyr)+(Cys+Orn)/(Cit+Trp) |
| 0.9399 | (Cys)/(Trp)+(Tau+Orn)/(Asn+Tyr) |
| 0.9397 | (Pro)/(Urea)+(Tau+Cys)/(Trp+His) |
| 0.9393 | (Gly)/(Urea)+(Tau+Cys)/(Tyr+His) |
| 0.9390 | (Tau)/(Trp+His)+(Cys)/(ABA+Tyr) |
| 0.9389 | (Orn)/(Leu)+(Tau+Cys)/(Asn+Trp) |
| 0.9389 | (Tau)/(Leu+His)+(Cys)/(Asn+Trp) |
| 0.9388 | (Tau)/(Trp+His)+(Cys+Orn)/(Val) |
| 0.9387 | (Tau)/(Asn+Leu)+(Cys)/(Trp+His) |
| 0.9387 | (Cys)/(Trp)+(Tau+Orn)/(Cit+Tyr) |
| 0.9385 | (Tau)/(Trp+Val)+(Cys)/(Tyr+His) |
| 0.9384 | (Tau)/(Val+His)+(Cys)/(Tyr+Trp) |
| 0.9383 | (Tau)/(Cit+Val)+(Cys)/(Tyr+Trp) |
| 0.9383 | (Tau)/(Trp+Leu)+(Cys)/(His) |
| 0.9382 | (Tau)/(ABA+Asn+Trp)+(Cys)/(His) |
| 0.9381 | (Tau)/(Asn+Leu)+(Cys)/(Cit+Trp) |
| 0.9380 | (Tau)/(Tyr+His)+(Cys)/(Asn+Trp) |
| 0.9379 | (Cys)/(Leu)+(Tau+Orn)/(Trp+His) |
| 0.9377 | (Tau)/(ABA+Val)+(Cys)/(Tyr+Trp) |
| 0.9376 | (Cys)/(Tyr)+(Tau+Orn)/(Asn+Trp) |
| 0.9375 | (Tau)/(Tyr+Leu)+(Cys)/(Cit+His) |

# FIG.28

| ROC AUC | FORMULA |
|---|---|
| 0.9374 | (Tau)/(Trp+Leu)+(Cys)/(ABA+His) |
| 0.9373 | (Tau)/(Asn+Tyr)+(Cys)/(Trp) |
| 0.9373 | (Tau)/(Trp+His)+(Cys)/(Tyr) |
| 0.9373 | (Tau)/(Cit+Val)+(Cys)/(Trp+His) |
| 0.9372 | (Orn)/(Leu)+(Tau+Cys)/(Cit+Trp) |
| 0.9372 | (Tau)/(His)+(Cys+Orn)/(Asn+Trp) |
| 0.9372 | (Tau)/(ABA+Val)+(Cys)/(Trp+His) |
| 0.9371 | (Cys)/(His)+(Tau+Orn)/(Tyr+Trp) |
| 0.9371 | (Tau)/(Trp+Leu)+(Cys)/(Cit+His) |
| 0.9370 | (Cys)/(Tyr)+(Tau+Orn)/(Asn+His) |
| 0.9370 | (Tau)/(Tyr+Trp)+(Cys)/(Cit+His) |
| 0.9370 | (Orn)/(Gln)+(Tau+Cys)/(Trp+His) |
| 0.9369 | (Tau)/(Tyr+Leu)+(Cys)/(ABA+His) |
| 0.9369 | (Tau)/(Cit+Leu)+(Cys)/(Tyr+Trp) |
| 0.9368 | (Tau)/(Trp)+(Cys+Glu+Orn)/(Leu) |
| 0.9367 | (Tau)/(Asn+Trp)+(Cys)/(His) |
| 0.9367 | (Tau)/(Cit+Leu)+(Cys)/(ABA+Trp) |
| 0.9367 | (Tau)/(Ser+Leu)+(Cys)/(Trp+His) |
| 0.9366 | (Tau)/(Asn+Leu)+(Cys)/(His) |
| 0.9366 | (Tau)/(Tyr+His)+(Cys)/(Cit+Trp) |
| 0.9366 | (Cys)/(His)+(Tau+Orn)/(Asn+Tyr) |
| 0.9365 | (Cys)/(Tyr)+(Tau+Orn)/(Trp+His) |
| 0.9365 | (Tau)/(ABA+Tyr)+(Cys)/(Trp) |
| 0.9365 | (Tau)/(Asn+Tyr+Leu)+(Cys)/(His) |
| 0.9365 | (Tau)/(Asn+His)+(Cys)/(Tyr+Trp) |
| 0.9365 | (Tau)/(ABA+Asn+Tyr)+(Cys)/(His) |
| 0.9364 | (Tau)/(Trp+Leu)+(Cys)/(Cit+Tyr) |
| 0.9364 | (Tau)/(ABA+Tyr+Leu)+(Cys)/(His) |
| 0.9363 | (Tau)/(Val+Leu)+(Cys)/(Trp+His) |
| 0.9362 | (Tau)/(Leu)+(Cys+Orn)/(Asn+Trp) |
| 0.9190 | (Tau)/(Asn)+(Gly+Orn)/(Val+His) |
| 0.9187 | (Tau)/(Asn)+(Gly+Pro)/(Val+His) |
| 0.9186 | (Tau)/(Asn)+(Gly+Pro)/(Trp+Val) |
| 0.9185 | (Tau)/(Asn)+(Gly+Orn)/(Trp+Val) |
| 0.9180 | (Tau)/(Asn+Trp)+(Orn)/(Leu+His) |
| 0.9172 | (Tau)/(Asn)+(Gly+Pro)/(Tyr+Val) |
| 0.9171 | (Tau)/(Asn+Trp)+(Orn)/(Tyr+Leu) |
| 0.8528 | (Cys)/(Asn+Tyr)+(Gly+Pro)/(Urea) |
| 0.8515 | (Cys)/(Tyr+His)+(Gly+Pro)/(Urea) |
| 0.8480 | (Cys)/(Tyr+His)+(Glu+Gly)/(Urea) |
| 0.8471 | (Cys)/(Asn+Tyr+His)+(Gly)/(Urea) |
| 0.8465 | (Cys)/(Tyr+His)+(Gly+Orn)/(Urea) |
| 0.8464 | (Cys)/(Tyr+His)+(Pro+Orn)/(Urea) |
| 0.7486 | (Gly)/(His)+(Pro+Orn)/(Asn+Trp) |
| 0.7479 | (Pro)/(Val)+(Gly+Orn)/(Asn+His) |
| 0.7474 | (Gly)/(His)+(Pro+Orn)/(Met+Asn) |
| 0.7466 | (Pro)/(Met+Asn)+(Gly+Orn)/(His) |
| 0.7464 | (Gly)/(Met+Asn+His)+(Orn)/(Leu) |
| 0.7464 | (Orn)/(Leu)+(Gly+Pro)/(Asn+His) |
| 0.7463 | (Gly)/(His)+(Pro)/(Asn+Trp) |

# FIG.29

# FIG.30

| ROC AUC | FORMULA |
| --- | --- |
| 0.9699 | (Orn)/(Val)+(Tau+Cys)/(His+Trp) |
| 0.9679 | (Tau)/(Leu)+(Cys+Orn)/(His+Trp) |
| 0.9674 | (Tau+Cys+Orn)/(Asn+Val+Trp) |
| 0.9663 | (Tau+Cys+Orn)/(His+Val+Trp) |
| 0.9655 | (Tau)/(Leu)+(Cys+Orn)/(Asn+Trp) |
| 0.9651 | (Tau+Cys+Orn)/(Asn+His+Val) |
| 0.9647 | (Tau)/(His+Trp)+(Cys+Orn)/(Val) |
| 0.9639 | (Tau+Cys+Orn)/(Tyr+Val+Trp) |
| 0.9637 | (Cys)/(Trp)+(Tau+Orn)/(Asn+Tyr) |
| 0.9625 | (Orn)/(Leu)+(Tau+Cys)/(Asn+Trp) |
| 0.9623 | (Tau+Cys+Orn)/(Cit+Val+Trp) |
| 0.9622 | (Cys)/(His+Trp)+(Tau+Orn)/(Val) |
| 0.9621 | (Tau+Cys)/(Trp)+(Pro+Orn)/(Val) |
| 0.9618 | (Tau)/(Trp)+(Glu+Cys+Orn)/(Leu) |
| 0.9617 | (Tau+Cys+Orn)/(Asn+Tyr+Val) |
| 0.9616 | (Tau+Cys+Orn)/(Tyr+His+Val) |
| 0.9613 | (Cys)/(Leu)+(Tau+Orn)/(Asn+Trp) |
| 0.9613 | (Orn)/(Val)+(Tau+Cys)/(Tyr+Trp) |
| 0.9613 | (Tau+Cys+Orn)/(His+Leu+Trp) |
| 0.9610 | (Cys)/(Leu)+(Tau+Orn)/(His+Trp) |
| 0.9606 | (Cys)/(Asn+His)+(Tau+Orn)/(Val) |
| 0.9606 | (Tau)/(Leu)+(Cys+Orn)/(Tyr+Trp) |
| 0.9603 | (Orn)/(Val)+(Tau+Cys)/(Tyr+His) |
| 0.9601 | (Tau+Cys)/(Trp)+(Glu+Orn)/(Leu) |
| 0.9600 | (Cys)/(Tyr+His)+(Tau+Orn)/(Val) |
| 0.9598 | (Cys)/(Asn+Tyr)+(Tau+Orn)/(Val) |
| 0.9598 | (Orn)/(Val)+(Tau+Cys)/(Asn+Trp) |
| 0.9596 | (Tau)/(Tyr)+(Cys+Orn)/(Cit+Trp) |
| 0.9596 | (Cys)/(Asn+Trp)+(Tau+Orn)/(Val) |
| 0.9596 | (Pro)/(Tyr+Leu)+(Tau+Cys)/(Trp) |
| 0.9593 | (Orn)/(Leu)+(Tau+Cys)/(His+Trp) |
| 0.9592 | (Tau+Cys+Orn)/(Cit+Tyr+Val) |
| 0.9592 | (Tau+Cys)/(Trp)+(Glu+Pro)/(Val) |
| 0.9591 | (Tau+Cys+Orn)/(Cit+Asn+Val) |
| 0.9591 | (Tau)/(Tyr+His)+(Cys+Orn)/(Val) |
| 0.9591 | (Cys)/(His)+(Tau+Orn)/(Tyr+Trp) |
| 0.9591 | (Cys)/(His)+(Tau+Orn)/(Leu+Trp) |
| 0.9591 | (Pro)/(His+Leu)+(Tau+Cys)/(Trp) |
| 0.9590 | (Cys)/(Tyr)+(Tau+Orn)/(Asn+Trp) |
| 0.9590 | (Tau+Cys+Orn)/(Asn+Leu+Trp) |
| 0.9590 | (Tau)/(Tyr+Trp)+(Cys+Orn)/(Val) |
| 0.9589 | (Cys)/(Tyr+Trp)+(Tau+Orn)/(Val) |
| 0.9587 | (Tau)/(His)+(Cys+Orn)/(Tyr+Trp) |
| 0.9586 | (Tau)/(Trp)+(Cys+Pro+Orn)/(Val) |
| 0.9586 | (Pro)/(Phe+Leu)+(Tau+Cys)/(Trp) |
| 0.9581 | (Pro)/(Met+Val)+(Tau+Cys)/(Trp) |
| 0.9581 | (Pro)/(Asn+Leu)+(Tau+Cys)/(Trp) |
| 0.9580 | (Tau+Cys+Orn)/(Cit+His+Val) |
| 0.9579 | (Cys)/(Cit+His)+(Tau+Orn)/(Val) |
| 0.9579 | (Orn)/(His+Trp)+(Tau+Cys)/(Val) |

# FIG.31

| ROC AUC | FORMULA |
|---|---|
| 0.9579 | (Tau)/(Tyr+Leu)+(Cys)/(ABA+Trp) |
| 0.9579 | (Pro)/(Val)+(Tau+Cys)/(Trp) |
| 0.9578 | (Tau+Cys+Orn)/(Phe+Val+Trp) |
| 0.9576 | (Tau)/(Asn+His)+(Cys+Orn)/(Val) |
| 0.9575 | (Cys)/(Trp)+(Tau+Orn)/(Cit+Tyr) |
| 0.9575 | (Cys)/(His)+(Tau+Orn)/(Tyr+Leu) |
| 0.9575 | (Pro)/(Asn+Val)+(Tau+Cys)/(Trp) |
| 0.9572 | (Orn)/(Lys)+(Tau+Cys)/(Tyr+Trp) |
| 0.9572 | (Tau)/(Asn+Trp)+(Cys+Orn)/(Val) |
| 0.9572 | (Tau+Glu+Cys+Orn)/(Val+Trp) |
| 0.9571 | (Pro)/(ABA+Val)+(Tau+Cys)/(Trp) |
| 0.9568 | (Pro)/(Tyr+Val)+(Tau+Cys)/(Trp) |
| 0.9567 | (Orn)/(Val)+(Tau+Cys)/(Asn+His) |
| 0.9567 | (Tau+Cys+Orn)/(Tyr+Leu+Trp) |
| 0.9566 | (Orn)/(Lys)+(Tau+Cys)/(His+Trp) |
| 0.9566 | (Pro)/(Ile+Val)+(Tau+Cys)/(Trp) |
| 0.9565 | (Tau)/(Trp)+(Glu+Pro+Orn)/(Val) |
| 0.9565 | (Pro)/(Cit+Val)+(Tau+Cys)/(Trp) |
| 0.9563 | (Tau)/(Asn)+(Cys+Orn)/(Trp) |
| 0.9563 | (Orn)/(Tyr+His)+(Tau+Cys)/(Val) |
| 0.9563 | (Pro)/(Ile+Leu)+(Tau+Cys)/(Trp) |
| 0.9561 | (Tau)/(Trp)+(Cys+Orn)/(His) |
| 0.9561 | (Orn)/(Leu)+(Tau+Cys)/(Tyr+Trp) |
| 0.9561 | (Cys)/(Val)+(Tau+Orn)/(His+Trp) |
| 0.9561 | (Tau)/(Trp)+(Glu+Orn)/(Leu) |
| 0.9559 | (Orn)/(Tyr+Trp)+(Tau+Cys)/(His) |
| 0.9558 | (Cys)/(Lys)+(Tau+Orn)/(Tyr+Val) |
| 0.9558 | (Tau)/(Asn+Leu)+(Cys)/(ABA+Trp) |
| 0.9558 | (Tau)/(His+Leu)+(Cys)/(Tyr+Trp) |
| 0.9557 | (Tau+Cys+Orn)/(Met+Val+Trp) |
| 0.9547 | (Tau)/(Trp)+(Pro+Orn)/(Val) |
| 0.9536 | (Tau)/(Trp)+(Pro+Orn)/(Met+Val) |
| 0.9536 | (Tau)/(Trp)+(Pro+Orn)/(Asn+Val) |
| 0.9536 | (Pro)/(Urea)+(Tau+Orn)/(Val+Trp) |
| 0.9524 | (Tau)/(Trp)+(Pro+Orn)/(Phe+Val) |
| 0.9524 | (Tau+Orn)/(His)+(Glu+Gly)/(Val) |
| 0.9524 | (Glu)/(Gln)+(Tau+Orn)/(Val+Trp) |
| 0.8418 | (Orn)/(His)+(Gly+Pro)/(Asn+Val) |
| 0.8386 | (Orn)/(Asn)+(Gly+Pro)/(Val+Trp) |
| 0.8360 | (Orn)/(Asn)+(Gly+Pro)/(Tyr+Val) |
| 0.8356 | (Orn)/(Asn)+(Gly+Pro)/(Cit+Val) |
| 0.8355 | (Orn)/(Tyr)+(Gly+Pro)/(Asn+His) |
| 0.8354 | (Gly)/(Val)+(Pro+Orn)/(Asn+His) |
| 0.8352 | (Orn)/(His)+(Gly+Pro)/(Val+Trp) |
| 0.8352 | (Orn)/(His)+(Gly+Pro)/(Tyr+Val) |
| 0.8348 | (Gly)/(Asn)+(Pro+Orn+Thr)/(His) |
| 0.8348 | (Orn)/(Trp)+(Gly+Pro)/(Asn+His) |
| 0.8347 | (Orn)/(Leu)+(Gly+Pro)/(Asn+His) |
| 0.8347 | (Pro)/(Val)+(Gly+Orn)/(Asn+His) |
| 0.8344 | (Orn)/(Asn)+(Gly+Pro)/(His+Val) |

# FIG.32

# FIG.33

| ROC AUC | FORMULA |
|---|---|
| 0.9453 | (Tau)/(ABA+Asn+Tyr)+(Cys)/(His) |
| 0.9450 | (Tau)/(Asn+Trp+Tyr)+(Cys)/(His) |
| 0.9435 | (Tau)/(ABA+Trp+Tyr)+(Cys)/(His) |
| 0.9418 | (Tau)/(Asn+His)+(Cys)/(ABA+Tyr) |
| 0.9413 | (Tau)/(Asn+Met+Tyr)+(Cys)/(His) |
| 0.9403 | (Tau)/(Phe+Asn+Tyr)+(Cys)/(His) |
| 0.9401 | (Tau)/(ABA+Asn+Trp)+(Cys)/(His) |
| 0.9398 | (Tau)/(Trp+Met+Tyr)+(Cys)/(His) |
| 0.9394 | (Tau)/(Trp+His)+(Cys)/(ABA+Tyr) |
| 0.9388 | (Tau)/(Cit+Trp+Tyr)+(Cys)/(His) |
| 0.9386 | (Tau)/(Cit+Asn+Tyr)+(Cys)/(His) |
| 0.9383 | (Tau)/(Asn+Tyr)+(Cys)/(His) |
| 0.9380 | (Tau)/(Asn+Trp+Met)+(Cys)/(His) |
| 0.9370 | (Tau)/(Phe+Asn+Trp)+(Cys)/(His) |
| 0.9368 | (Tau)/(ABA+His)+(Cys)/(Tyr) |
| 0.9368 | (Tau)/(Ile+Asn+Tyr)+(Cys)/(His) |
| 0.9366 | (Tau)/(Trp+Tyr)+(Cys)/(His) |
| 0.9365 | (Tau)/(Asn+Met+Leu)+(Cys)/(His) |
| 0.9364 | (Tau)/(Leu+Tyr)+(Cys)/(His) |
| 0.9362 | (Tau)/(Asn+His)+(Cys)/(ABA+Trp) |
| 0.9362 | (Tau)/(ABA+Met+Tyr)+(Cys)/(His) |
| 0.9360 | (Tau)/(Asn+Leu)+(Cys)/(His) |
| 0.9360 | (Tau)/(ABA+Phe+Tyr)+(Cys)/(His) |
| 0.9358 | (Tau)/(Leu+Tyr)+(Cys)/(Cit+Trp) |
| 0.9356 | (Tau)/(Leu+His)+(Cys)/(Cit+Tyr) |
| 0.9353 | (Tau)/(Tyr+His)+(Cys)/(ABA+Trp) |
| 0.9353 | (Tau)/(Leu+Tyr)+(Cys)/(Cit+His) |
| 0.9353 | (Tau)/(Asn+Leu+Tyr)+(Cys)/(His) |
| 0.9352 | (Tau)/(ABA+Cit+Tyr)+(Cys)/(His) |
| 0.9352 | (Tau)/(Phe+Trp+Tyr)+(Cys)/(His) |
| 0.9348 | (Tau)/(ABA+Leu+Tyr)+(Cys)/(His) |
| 0.9348 | (Tau)/(Leu+His)+(Cys)/(Trp+Tyr) |
| 0.9348 | (Tau)/(Met+Leu+Tyr)+(Cys)/(His) |
| 0.9346 | (Tau)/(ABA+Asn+Leu)+(Cys)/(His) |
| 0.9346 | (Tau)/(Leu+Tyr)+(Cys)/(Trp+His) |
| 0.9341 | (Tau)/(Asn+Leu)+(Cys)/(Cit+Trp) |
| 0.9340 | (Tau)/(Phe+Met+Tyr)+(Cys)/(His) |
| 0.9339 | (Tau)/(Cit+Asn+Leu)+(Cys)/(His) |
| 0.9337 | (Tau)/(Met+His)+(Cys)/(ABA+Tyr) |
| 0.9336 | (Tau)/(Trp+His)+(Cys)/(Cit+Tyr) |
| 0.9336 | (Tau)/(Asn+His)+(Cys)/(Trp+Tyr) |
| 0.9335 | (Tau)/(Cit+Phe+Tyr)+(Cys)/(His) |
| 0.9335 | (Tau)/(Cit+Leu+Tyr)+(Cys)/(His) |
| 0.9335 | (Tau)/(Trp+His)+(Cys)/(Asn+Tyr) |
| 0.9333 | (Tau)/(Ile+Trp+Tyr)+(Cys)/(His) |
| 0.9333 | (Tau)/(Val+Tyr)+(Cys)/(Trp+His) |
| 0.9332 | (Tau)/(Tyr+His)+(Cys)/(Cit+Trp) |
| 0.9332 | (Tau)/(Ile+Asn+Trp)+(Cys)/(His) |
| 0.9332 | (Tau)/(Asn+Trp+Leu)+(Cys)/(His) |
| 0.9331 | (Tau)/(Asn+Trp)+(Cys)/(His) |

FIG.34

| ROC AUC | FORMULA |
|---|---|
| 0.9330 | (Tau)/(Trp+His)+(Cys)/(Tyr) |
| 0.9330 | (Tau)/(ABA+Cit+His)+(Cys)/(Tyr) |
| 0.9329 | (Tau)/(Asn+Tyr)+(Cys)/(ABA+Trp) |
| 0.9329 | (Tau)/(Cit+Met+Leu)+(Cys)/(His) |
| 0.9327 | (Tau)/(Asn+His)+(Cys)/(Tyr) |
| 0.9327 | (Tau)/(Trp+Leu)+(Cys)/(His) |
| 0.9327 | (Tau)/(Cit+Asn+Trp)+(Cys)/(His) |
| 0.9326 | (Tau)/(ABA+Asn+His)+(Cys)/(Tyr) |
| 0.9326 | (Tau)/(Tyr+His)+(Cys)/(Asn+Trp) |
| 0.9325 | (Tau)/(Leu+His)+(Cys)/(Asn+Trp) |
| 0.9324 | (Tau)/(ABA+Phe+Asn)+(Cys)/(His) |
| 0.9324 | (Tau)/(Met+His)+(Cys)/(ABA+Trp) |
| 0.9324 | (Tau)/(Phe+Asn+Met)+(Cys)/(His) |
| 0.9324 | (Tau)/(Val+Tyr)+(Cys)/(Asn+His) |
| 0.9322 | (Tau)/(His)+(Cys)/(Tyr) |
| 0.9322 | (Tau)/(Asn+Val)+(Cys)/(Trp+His) |
| 0.9322 | (Tau)/(Trp+Met+Leu)+(Cys)/(His) |
| 0.9321 | (Tau)/(ABA+Tyr)+(Cys)/(His) |
| 0.9321 | (Tau)/(Trp+Tyr)+(Cys)/(ABA+His) |
| 0.9321 | (Tau)/(Cit+Met+Tyr)+(Cys)/(His) |
| 0.9321 | (Tau)/(Leu+Tyr)+(Cys)/(Asn+Trp) |
| 0.9320 | (Tau)/(Asn+Tyr)+(Cys)/(ABA+His) |
| 0.9317 | (Tau)/(Phe+Tyr)+(Cys)/(His) |
| 0.9317 | (Tau)/(ABA+Trp+His)+(Cys)/(Tyr) |
| 0.9316 | (Tau)/(Gly+Val)+(Cys)/(Tyr+His) |
| 0.9316 | (Tau)/(Cit+Ile+Tyr)+(Cys)/(His) |
| 0.9315 | (Tau)/(ABA+Asn+Trp)+(Cys)/(Tyr) |
| 0.9315 | (Tau)/(Leu+His)+(Cys)/(Cit+Trp) |
| 0.9314 | (Tau)/(ABA+Trp+Leu)+(Cys)/(His) |
| 0.9314 | (Tau)/(Asn+Leu)+(Cys)/(Cit+His) |
| 0.9314 | (Tau)/(Ile+Phe+Tyr)+(Cys)/(His) |
| 0.9313 | (Tau)/(Met+Leu)+(Cys)/(Cit+Trp) |
| 0.9313 | (Tau)/(Trp+Leu)+(Cys)/(Cit+Tyr) |
| 0.9313 | (Tau)/(Cit+Trp+Leu)+(Cys)/(His) |
| 0.9313 | (Tau)/(Phe+Asn+Leu)+(Cys)/(His) |
| 0.9312 | (Tau)/(Cit+His)+(Cys)/(ABA+Tyr) |
| 0.8907 | (Tau)/(Asn+Met+His) |
| 0.8902 | (Tau)/(Asn+His) |
| 0.8896 | (Tau)/(Asn+Trp+His) |
| 0.8893 | (Tau)/(Asn+Trp+Met+His) |
| 0.8880 | (Tau)/(Asn+Trp+Tyr+His) |
| 0.8877 | (Tau)/(Asn+Trp+Leu+His) |
| 0.8876 | (Tau)/(Asn+Leu+Tyr+His) |
| 0.8546 | (Cys)/(Met+Tyr+His)+(Glu)/(Urea) |
| 0.8543 | (Cys)/(Asn+Tyr+His)+(Glu)/(Urea) |
| 0.8531 | (Cys)/(Tyr+His)+(Glu)/(Urea) |
| 0.8531 | (Cys)/(Tyr+His)+(Glu)/(Ile+Urea) |
| 0.8530 | (Cys)/(Tyr+His)+(Glu)/(Orn+Urea) |
| 0.8530 | (Cys)/(Tyr+His)+(Glu)/(Ala+Urea) |
| 0.8530 | (Cys)/(Tyr+His)+(Glu)/(Phe+Urea) |

# FIG.35

**FIG.36**

| ROC AUC | FORMULA |
|---|---|
| 0.7365 | (Tau)/(Ser)+(Pro+Orn)/(Cys+Trp) |
| 0.7347 | (Orn)/(Ser)+(Pro+Met+Leu)/(Val) |
| 0.7329 | (Gly)/(Cys+Trp)+(Pro+Tau)/(Ser) |
| 0.7326 | (Orn)/(Ser)+(Pro+Phe+Leu)/(Val) |
| 0.7313 | (Met)/(Cys)+(Gly+Pro)/(Ser+Trp) |
| 0.7312 | (Orn)/(Ser)+(Pro+Leu)/(Val+Cys) |
| 0.7305 | (Orn)/(Ser)+(Pro+Leu)/(Val) |
| 0.7304 | (Orn)/(Trp)+(Pro+Tau)/(Ser+Cys) |
| 0.7300 | (Pro+Leu)/(Val)+(Met+Orn)/(Ser) |
| 0.7299 | (Gly)/(Ser)+(Pro+Glu)/(Cys+Trp) |
| 0.7298 | (Gly)/(Ser)+(Pro+Tau)/(Cys+Trp) |
| 0.7284 | (Tau)/(Val)+(Pro+Orn)/(Cys+Trp) |
| 0.7284 | (Orn)/(Ser+Cys)+(Pro+Leu)/(Val) |
| 0.7283 | (Gly+Pro+Glu)/(Ser+Cys+Trp) |
| 0.7282 | (Pro+Phe)/(Val)+(Met+Orn)/(Ser) |
| 0.7281 | (Orn)/(Ser)+(Pro+Phe)/(Val+Cys) |
| 0.7280 | (Gly)/(Ser)+(Pro+Orn)/(Cys+Trp) |
| 0.7279 | (Orn)/(Ser)+(Pro+Tau+Leu)/(Val) |
| 0.7278 | (Orn)/(Trp)+(Gly+Pro)/(Ser+Cys) |
| 0.7278 | (Orn)/(Ser)+(Pro+Met+Phe)/(Val) |
| 0.7276 | (Gly)/(Ser)+(Pro+Tyr)/(Cys+Trp) |
| 0.7276 | (Orn)/(Ser)+(Pro+Tau)/(Val+Trp) |
| 0.7275 | (Pro)/(Ser)+(Gly+Glu)/(Cys+Trp) |
| 0.7274 | (Orn)/(Ser)+(Pro+Phe)/(Val+Trp) |
| 0.7273 | (Pro+Orn+Tau+Leu)/(Val+Trp) |
| 0.7272 | (Pro)/(Ser)+(Gly+Tau)/(Cys+Trp) |
| 0.7271 | (Pro+Met+Orn+Tau)/(Cys+Trp) |
| 0.7271 | (Met)/(Trp)+(Pro+Tau)/(Val+Ser) |
| 0.7270 | (Pro+Ile)/(Val)+(Met+Orn)/(Ser) |
| 0.7269 | (Orn)/(Ser)+(Pro+Leu)/(Val+Trp) |
| 0.7269 | (Gly+Pro+Tau)/(Ser+Cys+Trp) |
| 0.7266 | (Pro)/(Trp)+(Gly+Tau)/(Ser+Cys) |
| 0.7266 | (Gly)/(Cys+Trp)+(Pro+Glu)/(Ser) |
| 0.7263 | (Pro)/(Cys+Trp)+(Gly+Tau)/(Ser) |
| 0.7263 | (Orn)/(Ser)+(Pro+Ile+Leu)/(Val) |
| 0.7261 | (Pro)/(Trp)+(Orn+Tau)/(Cys+ABA) |
| 0.7260 | (Orn)/(Cys+Trp)+(Pro+Tau)/(Val) |
| 0.7260 | (Orn)/(Ser)+(Pro+Met)/(Val+Cys) |
| 0.7259 | (Orn)/(Ser)+(Pro+Tau+Ile)/(Val) |
| 0.7258 | (Orn)/(Ser)+(Pro+Met)/(Val+Trp) |
| 0.7258 | (Orn)/(Ser)+(Pro+Tyr+Leu)/(Val) |
| 0.7255 | (Orn)/(Cys)+(Gly+Pro)/(Ser+Trp) |
| 0.7255 | (Pro)/(Val)+(Gly+Glu)/(Ser+Trp) |
| 0.7254 | (Pro+Tau)/(Trp)+(Met+Orn)/(Cys) |
| 0.7252 | (Orn)/(Ser+Trp)+(Pro+Leu)/(Val) |
| 0.7251 | (Gly)/(Val)+(Pro+Tau)/(Cys+Trp) |
| 0.7251 | (Tau)/(Ser)+(Pro+Met)/(Cys+Trp) |
| 0.7249 | (Pro+Tau)/(Ser)+(Met+Orn)/(Trp) |
| 0.7249 | (Pro)/(Val)+(Orn+Tau)/(Ser+Trp) |
| 0.7249 | (Gly+Tau)/(Trp)+(Pro+Glu)/(Cys) |

FIG.37

| ROC AUC | FORMULA |
|---|---|
| 0.7248 | (Gly)/(Ser)+(Pro+Met)/(Cys+Trp) |
| 0.7248 | (Pro)/(Cys)+(Met+Orn+Tau)/(Trp) |
| 0.7245 | (Pro)/(Cys+Trp)+(Orn+Tau)/(Ser) |
| 0.7245 | (Gly)/(Val)+(Pro+Tau)/(Ser+Trp) |
| 0.7245 | (Orn)/(Ser)+(Pro+Met+Ile)/(Val) |
| 0.7244 | (Pro)/(Val)+(Met+Orn)/(Ser+Trp) |
| 0.7244 | (Pro+Orn+Phe)/(Val+Ser+Trp) |
| 0.7243 | (Gly+Pro)/(Val)+(Orn+Leu)/(Ser) |
| 0.7242 | (Tau)/(Ser)+(Gly+Pro)/(Cys+Trp) |
| 0.7242 | (Orn)/(Ser)+(Pro+Ile+Phe)/(Val) |
| 0.7242 | (Pro)/(Val)+(Gly+Ile)/(Ser+Trp) |
| 0.7241 | (Gly+Pro+Met)/(Ser+Cys+Trp) |
| 0.7241 | (Tau)/(Ser)+(Pro+Thr)/(Cys+Trp) |
| 0.7239 | (Gly+Pro+Orn)/(Ser+Cys+Trp) |
| 0.7239 | (Pro)/(Val)+(Orn+Phe)/(Ser+Trp) |
| 0.7238 | (Pro+Orn+Tau)/(Val+Ser+Trp) |
| 0.7237 | (Orn)/(Ser)+(Pro+Tyr)/(Val+Cys) |
| 0.7237 | (Pro+Orn+Tau+Ile)/(Val+Ser) |
| 0.7235 | (Met)/(Ser)+(Pro+Orn)/(Val+Trp) |
| 0.7234 | (Pro+Orn)/(Trp)+(Met+Tau)/(Cys) |
| 0.7234 | (Pro+Tyr)/(Val)+(Met+Orn)/(Ser) |
| 0.7233 | (Gly)/(Ser+Trp)+(Pro+Leu)/(Val) |
| 0.7232 | (Pro+Orn+Tau)/(Cys+Trp) |
| 0.7232 | (Pro)/(Val)+(Gly+Phe)/(Ser+Trp) |
| 0.7231 | (Orn)/(Ser+ABA)+(Pro+Leu)/(Val) |
| 0.7231 | (Gly)/(Val)+(Pro+Glu)/(Cys+Trp) |
| 0.7231 | (Gly+Tau)/(Ser)+(Pro+Orn)/(Trp) |
| 0.7229 | (Pro+Tau)/(Val)+(Met+Orn)/(Ser) |
| 0.7228 | (Pro)/(Val)+(Gly+Orn)/(Ser+Trp) |
| 0.7227 | (Pro)/(Cys)+(Orn+Tau+Glu)/(Trp) |
| 0.7227 | (Orn)/(Ser)+(Pro+Phe)/(Val) |
| 0.7224 | (Gly)/(Cys+Trp)+(Pro+Tyr)/(Ser) |
| 0.7224 | (Pro)/(Cys)+(Orn+Tau+Asn)/(Trp) |
| 0.7223 | (Pro)/(Val)+(Gly+Tau)/(Ser+Trp) |
| 0.7223 | (Pro)/(Cys)+(Orn+Tau+Tyr)/(Trp) |
| 0.7222 | (Gly)/(Val)+(Pro+Leu)/(Ser+Trp) |
| 0.7221 | (Pro)/(Val)+(Gly+Met)/(Ser+Trp) |
| 0.7221 | (Gly+Tau)/(Ser)+(Pro+Met)/(Trp) |
| 0.7221 | (Pro)/(Ser)+(Gly+Tau)/(Val+Trp) |
| 0.7221 | (Gly)/(Ser)+(Pro+Phe)/(Cys+Trp) |
| 0.7220 | (Gly)/(Ser+Trp)+(Pro+Ile)/(Val) |
| 0.7220 | (Thr)/(Ser)+(Pro+Tau)/(Cys+Trp) |
| 0.7220 | (Pro+Orn+Tau+Ile)/(Val+Trp) |
| 0.7219 | (Pro)/(Cys+Trp)+(Gly+Glu)/(Ser) |
| 0.7219 | (Pro)/(Val+Cys)+(Met+Orn)/(Ser) |
| 0.7219 | (Pro+Met+Orn)/(Val+Ser+Trp) |
| 0.7218 | (Met)/(Trp)+(Gly+Pro)/(Ser+Cys) |
| 0.7218 | (Tau)/(Ser)+(Pro+Tyr)/(Cys+Trp) |
| 0.7218 | (Pro)/(Val)+(Met+Orn)/(Ser+Cys) |
| 0.7217 | (Orn)/(Cys)+(Pro+Tau)/(ABA+Trp) |

121

# FIG.38

FIG.39

| ROC AUC | FORMULA |
|---------|---------|
| 0.9054 | (Met+Orn)/(Trp)+(Tau+Ser)/(Arg) |
| 0.9022 | (Gly+Tau)/(Arg)+(Met+Orn)/(Cys) |
| 0.9006 | (Tau+Ser)/(Arg)+(Orn+Tyr)/(Cys) |
| 0.9006 | (Met)/(Trp)+(Tau+Orn)/(ABA+Arg) |
| 0.8990 | (Phe)/(Cys)+(Tau+Ser+Tyr)/(Arg) |
| 0.8990 | (Gly+Tau)/(Arg)+(Orn+Phe)/(Cys) |
| 0.8990 | (Tyr)/(Cys)+(Gly+Tau)/(Trp+Arg) |
| 0.8990 | (Ala)/(Trp+Cys)+(Tau+Ser)/(Arg) |
| 0.8990 | (Tau+Ser)/(Arg)+(Orn+Phe)/(Cys) |
| 0.8974 | (Orn)/(Trp)+(Tau+Ser)/(ABA+Arg) |
| 0.8974 | (Gly+Ala)/(Arg)+(Tau+Ser)/(Cys) |
| 0.8974 | (Phe)/(Cys)+(Tau+Ser+Asn)/(Arg) |
| 0.8974 | (Gly+Tau)/(Arg)+(Phe+Tyr)/(Cys) |
| 0.8974 | (Phe)/(ABA)+(Gly+Tau+Ser)/(Arg) |
| 0.8974 | (Met)/(Trp)+(Tau+Ser)/(His+Arg) |
| 0.8974 | (Tau+Ala)/(Arg)+(Ser+Tyr)/(Cys) |
| 0.8974 | (Met)/(Trp)+(Tau+Orn)/(Cys+Arg) |
| 0.8974 | (Phe)/(Arg)+(Tau+Ser+Orn)/(Val) |
| 0.8958 | (Met+Orn)/(Trp)+(Tau+Tyr)/(Arg) |
| 0.8958 | (Tyr)/(Cys)+(Tau+Ala)/(Trp+Arg) |
| 0.8958 | (Gly)/(Arg)+(Tau+Orn)/(ABA+Trp) |
| 0.8958 | (Gly+Tau)/(Arg)+(Orn+Tyr)/(Cys) |
| 0.8958 | (Gly)/(Arg)+(Tau+Phe)/(ABA+Cys) |
| 0.8958 | (Phe)/(Cys)+(Gly+Tau)/(ABA+Arg) |
| 0.8958 | (Tau+Ala)/(Arg)+(Orn+Phe)/(Cys) |
| 0.8942 | (Gly+Tau)/(Arg)+(Met+Asn)/(Cys) |
| 0.8942 | (Tau+Ala)/(Arg)+(Ser+Phe)/(Cys) |
| 0.8942 | (Ser)/(ABA+Trp)+(Met+Tau)/(Arg) |
| 0.8942 | (Met)/(Trp)+(Tau+Ser)/(Cys+Arg) |
| 0.8942 | (Met)/(Arg)+(Tau+Orn+Phe)/(Val) |
| 0.8942 | (Phe)/(Cys)+(Gly+Tau)/(His+Arg) |
| 0.8942 | (Met)/(Trp)+(Tau+Orn)/(His+Arg) |
| 0.8942 | (Phe)/(Cys)+(Tau+Ser+Orn)/(Arg) |
| 0.8926 | (Phe)/(Cys)+(Met+Tau+Ser)/(Arg) |
| 0.8926 | (Phe)/(Cys)+(Tau+Ser)/(Arg) |
| 0.8926 | (Ser)/(ABA+Trp)+(Tau+Phe)/(Arg) |
| 0.8926 | (Tau+Ala)/(Arg)+(Phe+Tyr)/(Cys) |
| 0.8926 | (Tau+Ser)/(Arg)+(Phe+Tyr)/(Cys) |
| 0.8926 | (Met)/(Trp)+(Tau+Orn+Tyr)/(Lys) |
| 0.8926 | (Gly+Tau+Orn+Phe)/(Cys+Arg) |
| 0.8926 | (Met+Orn)/(Trp)+(Tau+Asn)/(Arg) |
| 0.8926 | (Phe)/(Cys)+(Tau+Ser+Cit)/(Arg) |
| 0.8926 | (Phe)/(Arg)+(Tau+Orn+Ile)/(Val) |
| 0.8926 | (Met)/(Cys)+(Tau+Ser)/(Trp+Arg) |
| 0.8926 | (Phe)/(Arg)+(Tau+Pro+Ser)/(Val) |
| 0.8910 | (Phe)/(Trp)+(Tau+Ala)/(Cys+Arg) |
| 0.8910 | (Phe)/(Cys)+(Tau+Ala)/(Trp+Arg) |
| 0.8910 | (Met+Phe)/(Trp)+(Tau+Ser)/(Arg) |
| 0.8910 | (Phe)/(Cys)+(Tau+Ser)/(ABA+Arg) |
| 0.8910 | (Gly+Tau)/(Arg)+(Ile+Tyr)/(Cys) |

# FIG.40

| ROC AUC | FORMULA |
|---|---|
| 0.8910 | (Gly)/(Arg)+(Tau+Ser)/(ABA+Trp) |
| 0.8910 | (Phe)/(Arg)+(Tau+Orn+Asn)/(Val) |
| 0.8910 | (Met)/(Trp)+(Tau+Ser)/(ABA+Arg) |
| 0.8910 | (Met)/(Trp)+(Tau+Pro+Ser)/(Val) |
| 0.8910 | (Gly)/(Arg)+(Tau+Orn)/(His+Cys) |
| 0.8910 | (Gly)/(Arg)+(Tau+Phe)/(ABA+Trp) |
| 0.8910 | (Phe)/(ABA+Cys)+(Gly+Tau)/(Arg) |
| 0.8910 | (Phe)/(Cys)+(Gly+Tau)/(Trp+Arg) |
| 0.8910 | (Met+Phe)/(Cys)+(Tau+Ala)/(Arg) |
| 0.8901 | (Phe)/(Val)+(Met+Cit)/(ABA+Arg) |
| 0.8894 | (Met+Phe)/(Cys)+(Tau+Ser)/(Arg) |
| 0.8894 | (Ser)/(Cys)+(Tau+Ala+Phe)/(Arg) |
| 0.8894 | (Tyr)/(Cys)+(Gly+Tau)/(ABA+Arg) |
| 0.8894 | (Met+Cit)/(Trp)+(Tau+Orn)/(Arg) |
| 0.8894 | (Tyr)/(Cys)+(Gly+Tau+Phe)/(Arg) |
| 0.8894 | (Met+Orn)/(Trp)+(Tau+Phe)/(Arg) |
| 0.8894 | (Gly)/(Arg)+(Tau+Orn)/(ABA+Cys) |
| 0.8894 | (Gly)/(Arg)+(Tau+Phe)/(ABA+His) |
| 0.8894 | (Phe)/(His)+(Gly+Tau)/(ABA+Arg) |
| 0.8894 | (Phe)/(His)+(Gly+Tau)/(Cys+Arg) |
| 0.8894 | (Tau)/(Arg)+(Met+Orn)/(ABA+Trp) |
| 0.8894 | (Met)/(ABA)+(Tau+Ser+Orn)/(Arg) |
| 0.8894 | (Met+Orn)/(Cys)+(Tau+Ser)/(Arg) |
| 0.8894 | (Tau+Ala)/(Arg)+(Ser+Cit)/(Cys) |
| 0.8878 | (Phe)/(Cys)+(Tau+Ala)/(ABA+Arg) |
| 0.8878 | (Orn)/(Trp)+(Tau+Ser)/(Cys+Arg) |
| 0.8878 | (Gly+Tau)/(Arg)+(Met+Tyr)/(Cys) |
| 0.8878 | (Met)/(ABA)+(Tau+Ser+Phe)/(Arg) |
| 0.8878 | (Phe)/(Cys)+(Tau+Ser+Ile)/(Arg) |
| 0.8878 | (Phe)/(ABA)+(Gly+Tau+Glu)/(Arg) |
| 0.8878 | (Tau+Ala)/(Arg)+(Phe+Asn)/(Cys) |
| 0.8878 | (Phe)/(ABA)+(Gly+Tau+Orn)/(Arg) |
| 0.8878 | (Gly+Orn)/(Val)+(Tau+Phe)/(Arg) |
| 0.8878 | (Tau+Ser)/(Arg)+(Tyr+Cit)/(Cys) |
| 0.8878 | (Orn)/(Cys)+(Gly+Met+Tau)/(Arg) |
| 0.8878 | (Phe)/(ABA)+(Gly+Tau+Ile)/(Arg) |
| 0.8878 | (Met+Orn)/(Trp)+(Tau+Cit)/(Arg) |
| 0.8878 | (Phe)/(ABA)+(Gly+Met+Tau)/(Arg) |
| 0.8878 | (Phe)/(Cys)+(Gly+Met+Tau)/(Arg) |
| 0.8878 | (Phe)/(Cys)+(Gly+Tau+Tyr)/(Arg) |
| 0.8878 | (Phe)/(ABA)+(Gly+Tau+Asn)/(Arg) |
| 0.8884 | (Met)/(Trp+Arg)+(Cit)/(Val+His) |
| 0.8873 | (Met+Cit)/(Arg)+(Orn+Phe)/(Val) |
| 0.8798 | (Phe)/(Cys)+(Ala+Ser)/(ABA+Arg) |
| 0.8798 | (Ser)/(Arg)+(Phe+Cit)/(ABA+Cys) |
| 0.8782 | (Ala+Ser)/(Arg)+(Phe+Asn)/(Cys) |
| 0.8782 | (Ala)/(Trp+Arg)+(Met+Tyr)/(Cys) |
| 0.8782 | (Ser)/(Arg)+(Phe+Asn)/(ABA+Cys) |
| 0.8734 | (Met)/(Arg)+(Phe+Asn)/(Leu+Lys) |
| 0.8718 | (Met)/(Arg)+(Phe+Asn)/(Lys+His) |

## FIG.41

**FIG.42**

| ROC AUC | FORMULA |
|---------|---------|
| 0.9223 | (Gly)/(Lys)+(Ser+Tau+Met)/(His) |
| 0.9215 | (Gly)/(Lys)+(Ser+Tau)/(Trp+His) |
| 0.9192 | (Tau)/(His)+(Ser+Ile)/(ABA+Trp) |
| 0.9185 | (Met)/(Trp)+(Ser+Tau)/(Lys+His) |
| 0.9177 | (Ser+Ile)/(His)+(Tau+Gly)/(Lys) |
| 0.9177 | (Tau)/(Lys)+(Ser+Ile)/(Trp+His) |
| 0.9169 | (Ser)/(Trp)+(Tau+Gly+Ile)/(His) |
| 0.9169 | (Ser+Tyr)/(Trp)+(Tau+Gly)/(His) |
| 0.9162 | (Gly)/(Lys)+(Ser+Tau+Ile)/(His) |
| 0.9162 | (Ser+Tau+Gly+Ile)/(Trp+His) |
| 0.9154 | (Ser+Tau)/(His)+(Gly+Orn)/(Lys) |
| 0.9154 | (Ser+Tau+Gly+Tyr)/(Trp+His) |
| 0.9154 | (Ser)/(Trp)+(Tau+Gly+Tyr)/(His) |
| 0.9146 | (Ser+Met)/(Trp)+(Tau+Gly)/(His) |
| 0.9146 | (Ser)/(Trp)+(Tau+Gly+Met)/(His) |
| 0.9146 | (Ile)/(Trp)+(Ser+Tau+Gly)/(His) |
| 0.9138 | (Ser+Ile)/(Trp)+(Tau+Gly)/(His) |
| 0.9131 | (Ser)/(Trp)+(Tau+Gly)/(His) |
| 0.9131 | (Ile)/(ABA+Trp)+(Ser+Tau)/(Lys) |
| 0.9123 | (Ile)/(Trp)+(Ser+Tau)/(Glu+His) |
| 0.9115 | (Ser+Tau+Gly)/(Trp+His) |
| 0.9115 | (Tau)/(Pro)+(Ser+Gly)/(Trp+His) |
| 0.9115 | (Ser+Tau+Gly+Met)/(Trp+His) |
| 0.9115 | (Met)/(Trp)+(Ser+Tau)/(ABA+Lys) |
| 0.9108 | (Ser)/(Pro)+(Tau+Gly)/(Trp+His) |
| 0.9108 | (Ser)/(Trp)+(Tau+Gly)/(ABA+His) |
| 0.9108 | (Ser)/(Lys)+(Tau+Gly)/(Trp+His) |
| 0.9108 | (Ser+Met)/(Trp)+(Tau+Ile)/(His) |
| 0.9108 | (Ser)/(Trp)+(Tau+Gly+Orn)/(His) |
| 0.9100 | (Ser+Tau)/(Trp)+(Gly+Met)/(His) |
| 0.9100 | (Ser+Gly)/(His)+(Tau+Tyr)/(Trp) |
| 0.9092 | (Ser+Tau)/(His)+(Gly+Ile)/(Lys) |
| 0.9092 | (Ser+Met)/(Trp)+(Tau+Orn)/(His) |
| 0.9092 | (Tau+Gly)/(Lys)+(Ile+Tyr)/(His) |
| 0.9085 | (Ser+Tau)/(His)+(Gly+Met)/(Lys) |
| 0.9085 | (Gly)/(His)+(Ser+Tau+Met)/(Trp) |
| 0.9085 | (Ser+Tau)/(His)+(Met+Ile)/(Trp) |
| 0.9085 | (Ser+Tau)/(Trp)+(Gly+Tyr)/(His) |
| 0.9077 | (Met)/(Trp)+(Ser+Tau)/(Lys) |
| 0.9077 | (Ile)/(Lys)+(Ser+Tau+Gly)/(His) |
| 0.9077 | (Met)/(Trp)+(Ser+Tau+Orn)/(Lys) |
| 0.9077 | (Ile)/(ABA+Trp)+(Ser+Tau)/(Val) |
| 0.9077 | (Ala)/(Pro)+(Ser+Tau+Gly)/(His) |
| 0.9077 | (Gly)/(Lys)+(Ser+Tau+Tyr)/(His) |
| 0.9069 | (Gly)/(His)+(Ser+Tau)/(Trp) |
| 0.9069 | (Tau)/(His)+(Ser+Met+Ile)/(Trp) |
| 0.9069 | (Met)/(Trp)+(Ser+Tau)/(Val+His) |
| 0.9069 | (Ser+Tau)/(Trp)+(Gly+Ile)/(His) |
| 0.9069 | (Ser+Gly)/(His)+(Tau+Ile)/(Trp) |
| 0.9069 | (Tau)/(Gln)+(Ser+Ile)/(Trp+His) |

# FIG.43

| ROC AUC | FORMULA |
|---|---|
| 0.9069 | (Ser+Tau+Gly+Orn)/(Trp+His) |
| 0.9069 | (Met)/(Trp)+(Ser+Tau+Tyr)/(Lys) |
| 0.9062 | (Gly)/(ABA+His)+(Ser+Tau)/(Trp) |
| 0.9062 | (Tau)/(His)+(Ser+Met)/(Trp) |
| 0.9062 | (Tau)/(Pro)+(Ser+Met)/(ABA+Trp) |
| 0.9062 | (Ile)/(Pro)+(Ser+Tau+Gly)/(His) |
| 0.9062 | (Ile)/(Trp)+(Ser+Tau)/(ABA+Leu) |
| 0.9062 | (Ser)/(Trp)+(Tau+Ile)/(ABA+His) |
| 0.9062 | (Tyr)/(Trp)+(Ser+Tau+Gly)/(His) |
| 0.9054 | (Met)/(Trp)+(Ser+Tau+Ile)/(Lys) |
| 0.9054 | (Ser+Ile)/(Trp)+(Tau+Met)/(His) |
| 0.9054 | (Ser+Gly)/(His)+(Tau+Ile)/(Pro) |
| 0.9054 | (Tau)/(Pro)+(Ser+Ile)/(ABA+Trp) |
| 0.9054 | (Ile)/(Trp)+(Ser+Tau)/(ABA+His) |
| 0.9054 | (Tau+Gly)/(His)+(Ile+Tyr)/(Trp) |
| 0.9046 | (Gly)/(Lys)+(Ser+Tau)/(Cit+His) |
| 0.9046 | (Ser)/(Val)+(Tau+Gly)/(Trp+His) |
| 0.9046 | (Ser+Gly)/(His)+(Tau+Met)/(Trp) |
| 0.9046 | (Ser)/(Trp)+(Tau+Met+Ile)/(His) |
| 0.9046 | (Ile)/(Glu+Trp)+(Ser+Tau)/(Lys) |
| 0.9046 | (Tau)/(Val)+(Ser+Ile)/(Trp+His) |
| 0.9046 | (Ser+Tau)/(Trp)+(Gly+Orn)/(His) |
| 0.9038 | (Gly)/(His)+(Ser+Tau+Ile)/(Trp) |
| 0.9038 | (Gly)/(Lys)+(Ser+Tau+Orn)/(His) |
| 0.9038 | (Ser+Orn)/(Trp)+(Tau+Gly)/(His) |
| 0.9038 | (Tau)/(His)+(Ser+Orn)/(ABA+Trp) |
| 0.9038 | (Ala)/(Lys)+(Ser+Tau+Gly)/(His) |
| 0.9038 | (Met)/(ABA)+(Ser+Tau+Tyr)/(Trp) |
| 0.9038 | (Tau+Gly)/(Lys)+(Met+Tyr)/(His) |
| 0.9038 | (Tau+Gly)/(His)+(Met+Tyr)/(Trp) |
| 0.8923 | (Ile)/(Leu)+(Ser+Tyr)/(Lys+Val) |
| 0.8862 | (Gly)/(Leu+Lys)+(Ile+Tyr)/(His) |
| 0.8862 | (Ser+Tyr)/(Val)+(Met+Ile)/(His) |
| 0.8854 | (Met)/(Lys)+(Ile+Tyr)/(Val+His) |
| 0.8823 | (Ser)/(Val)+(Ile+Tyr)/(Cys+His) |
| 0.8800 | (Gly)/(Glu+Lys)+(Ile+Tyr)/(His) |
| 0.8800 | (Gly)/(Lys+Trp)+(Ile+Tyr)/(His) |
| 0.8792 | (Gly)/(Lys)+(Met+Ile+Tyr)/(His) |
| 0.8785 | (Gly)/(Cys+Lys)+(Ile+Tyr)/(His) |
| 0.8777 | (Gly)/(Phe+Lys)+(Ile+Tyr)/(His) |
| 0.8777 | (Ile)/(Cys+ABA)+(Ser+Tyr)/(His) |
| 0.8777 | (Ile)/(Leu)+(Ser+Tyr)/(Val+His) |
| 0.8769 | (Ala)/(Pro)+(Ser+Gly+Ile)/(His) |
| 0.8769 | (Met)/(His)+(Ser+Tyr)/(Lys+Val) |
| 0.8769 | (Ile)/(Leu)+(Ser+Tyr)/(Lys+His) |
| 0.8769 | (Gly)/(Pro+Lys)+(Met+Tyr)/(His) |
| 0.8762 | (Gly)/(ABA+Lys)+(Ile+Tyr)/(His) |
| 0.8762 | (Gly)/(Lys+Val)+(Ile+Tyr)/(His) |
| 0.8762 | (Ala)/(Pro+ABA)+(Ser+Gly)/(His) |
| 0.8762 | (Ile)/(Val)+(Met+Tyr)/(Leu+Lys) |

# FIG.44

FIG.45

| ROC AUC | FORMULA |
|---------|---------|
| 0.6993 | (Orn)/(Glu+Trp+His) |
| 0.6984 | (Orn)/(Cit+Glu+Trp+His) |
| 0.6920 | (Orn)/(Glu+His) |
| 0.6911 | (Orn)/(Cit+Glu+Trp) |
| 0.6902 | (Orn)/(Glu+ABA+His) |
| 0.6875 | (Orn)/(Cit+Glu+ABA+His) |
| 0.6875 | (Orn)/(Glu+ABA+Phe+His) |
| 0.6857 | (Orn)/(Cit+Glu+ABA+Trp) |
| 0.6848 | (Orn)/(Glu+ABA+Trp+His) |
| 0.6839 | (Orn)/(Cit+Glu+His) |
| 0.6839 | (Orn)/(Cit+Met+Glu+Trp) |
| 0.6839 | (Orn)/(Met+Glu+ABA+His) |
| 0.6830 | (Orn)/(Cit+Trp+His) |
| 0.6830 | (Orn)/(Cys+Glu+ABA+His) |
| 0.6830 | (Orn)/(Cit+Glu+Val+Urea) |
| 0.6830 | (Orn)/(Tau+Glu+Urea+Gln) |
| 0.6821 | (Orn)/(Cys+Glu+Val+Urea) |
| 0.6821 | (Orn)/(Thr+Glu+ABA+Phe) |
| 0.6821 | (Orn)/(Thr+Glu+Trp+His) |
| 0.6821 | (Orn)/(Cit+ABA+Trp+His) |
| 0.6821 | (Orn)/(Pro+Glu+Urea+Gln) |
| 0.6821 | (Orn)/(Glu+Val+Urea+Gln) |
| 0.6812 | (Orn)/(Trp+His) |
| 0.6812 | (Orn)/(Thr+Glu+Phe+His) |
| 0.6812 | (Orn)/(Cit+Glu+Phe+His) |
| 0.6812 | (Orn)/(Glu+Val+Urea+Phe) |
| 0.6812 | (Orn)/(Glu+Val+Urea+His) |
| 0.6803 | (Orn)/(Glu+Val+Urea) |
| 0.6803 | (Orn)/(Glu+Phe+His) |
| 0.6803 | (Orn)/(Asn+Glu+Val+Urea) |
| 0.6803 | (Orn)/(Thr+Tau+Glu+Urea) |
| 0.6803 | (Orn)/(Thr+Glu+Urea+Gln) |
| 0.6803 | (Orn)/(Thr+Glu+Urea+His) |
| 0.6803 | (Orn)/(Cit+Glu+Urea+His) |
| 0.6803 | (Orn)/(Met+Glu+ABA+Phe) |
| 0.6803 | (Orn)/(Met+Glu+Val+Urea) |
| 0.6803 | (Orn)/(Glu+ABA+Val+Urea) |
| 0.6803 | (Orn)/(Glu+Tyr+Val+Urea) |
| 0.6803 | (Orn)/(Glu+Leu+Urea+His) |
| 0.6803 | (Orn)/(Glu+Val+Trp+Urea) |
| 0.6803 | (Orn)/(Glu+Val+Gln+His) |
| 0.6803 | (Orn)/(Glu+Trp+Urea+Gln) |
| 0.6803 | (Orn)/(Glu+Trp+Phe+His) |
| 0.6803 | (Orn)/(Glu+Urea+Gln+His) |
| 0.6793 | (Orn)/(Cit+Trp) |
| 0.6793 | (Orn)/(Cit+His) |
| 0.6793 | (Orn)/(Met+Glu+His) |
| 0.6793 | (Orn)/(Glu+Leu+Urea) |
| 0.6793 | (Orn)/(Glu+Urea+Gln) |
| 0.6793 | (Orn)/(Arg+Glu+ABA+Urea) |

# FIG.46

| ROC AUC | FORMULA |
|---------|---------|
| 0.6793 | (Orn)/(Asn+Glu+ABA+His) |
| 0.6793 | (Orn)/(Cys+Tau+Urea+Gln) |
| 0.6793 | (Orn)/(Cys+Glu+Urea+Gln) |
| 0.6793 | (Orn)/(Cys+Glu+Urea+His) |
| 0.6793 | (Orn)/(Thr+Ser+Val+Urea) |
| 0.6793 | (Orn)/(Thr+Glu+ABA+His) |
| 0.6793 | (Orn)/(Thr+Glu+Trp+Urea) |
| 0.6793 | (Orn)/(Cit+Glu+Leu+Urea) |
| 0.6793 | (Orn)/(Cit+Val+Urea+Gln) |
| 0.6793 | (Orn)/(Cit+Val+Urea+His) |
| 0.6793 | (Orn)/(Ser+Glu+Urea+Gln) |
| 0.6793 | (Orn)/(Pro+ABA+Urea+Gln) |
| 0.6793 | (Orn)/(Pro+Trp+Urea+Gln) |
| 0.6793 | (Orn)/(Pro+Urea+Phe+Gln) |
| 0.6793 | (Orn)/(Tau+Met+Urea+Gln) |
| 0.6793 | (Orn)/(Tau+Val+Urea+Gln) |
| 0.6793 | (Orn)/(Met+Glu+Urea+Gln) |
| 0.6793 | (Orn)/(Glu+ABA+Trp+Phe) |
| 0.6793 | (Orn)/(Glu+ABA+Urea+Gln) |
| 0.6793 | (Orn)/(Glu+Tyr+Urea+His) |
| 0.6793 | (Orn)/(Glu+Urea+Phe+Gln) |
| 0.6793 | (Orn)/(Glu+Urea+Phe+His) |
| 0.6793 | (Orn)/(ABA+Val+Urea+Gln) |
| 0.6793 | (Orn)/(Leu+Urea+Gln+His) |
| 0.6784 | (Orn)/(Cys+Glu+His) |
| 0.6784 | (Orn)/(Pro+Urea+Gln) |
| 0.6784 | (Orn)/(Tau+Urea+Gln) |
| 0.6784 | (Orn)/(Glu+ABA+Phe) |
| 0.6784 | (Orn)/(Glu+Urea+His) |
| 0.6784 | (Orn)/(Trp+Urea+Gln) |
| 0.6784 | (Orn)/(Ile+Pro+Urea+Gln) |
| 0.6784 | (Orn)/(Ile+Glu+Urea+His) |
| 0.6784 | (Orn)/(Arg+Glu+Val+Urea) |
| 0.6784 | (Orn)/(Cys+Pro+Urea+Gln) |
| 0.6784 | (Orn)/(Cys+Glu+Trp+His) |
| 0.6784 | (Orn)/(Thr+Glu+Val+Urea) |
| 0.6784 | (Orn)/(Thr+Val+Urea+Gln) |
| 0.6784 | (Orn)/(Cit+Pro+Urea+Gln) |
| 0.6784 | (Orn)/(Cit+Met+Glu+His) |
| 0.6784 | (Orn)/(Cit+Glu+Urea+Phe) |
| 0.6784 | (Orn)/(Cit+Glu+Urea+Gln) |
| 0.6784 | (Orn)/(Cit+Val+Urea+Phe) |
| 0.6784 | (Orn)/(Cit+Urea+Gln+His) |
| 0.6784 | (Orn)/(Ser+Val+Urea+His) |
| 0.6784 | (Orn)/(Pro+Glu+Urea+His) |
| 0.6784 | (Orn)/(Tau+Glu+Val+Urea) |
| 0.6784 | (Orn)/(Tau+ABA+Urea+Gln) |
| 0.6784 | (Orn)/(Tau+Trp+Urea+Gln) |
| 0.6784 | (Orn)/(Tau+Urea+Gln+His) |
| 0.6784 | (Orn)/(Met+Glu+Tyr+Urea) |

# FIG.47

# FIG.48

| ROC AUC | FORMULA |
|---|---|
| 0.9312 | 1.29777+0.00433*Tau-0.00007*Urea+0.01967*Cys-0.01055*Tyr-0.00479*His-0.00736*Trp |
| 0.9290 | 1.37626+0.00448*Tau-0.00008*Urea-0.01008*Asn+0.01688*Cys-0.00511*His-0.00961*Trp |
| 0.9286 | 1.21629+0.00436*Tau-0.00007*Urea-0.00120*Val+0.02013*Cys-0.01074*Tyr-0.00846*Trp |
| 0.9284 | 1.35522+0.00415*Tau-0.00008*Urea-0.00890*Asn+0.02140*Cys-0.01112*Tyr-0.00491*His |
| 0.9278 | 1.31708+0.00444*Tau-0.00008*Urea-0.00934*Asn+0.02108*Cys-0.01036*Tyr-0.00795*Trp |
| 0.9274 | 1.20910+0.00431*Tau-0.00007*Urea+0.01990*Cys-0.00217*Leu-0.01028*Tyr-0.00874*Trp |
| 0.9271 | 1.32958+0.00431*Tau-0.00007*Urea-0.00187*Val+0.01616*Cys-0.00567*His-0.00923*Trp |
| 0.9266 | 1.16851+0.00411*Tau-0.00009*Urea+0.02030*Cys-0.01329*Tyr+0.00233*Phe-0.00676*His |
| 0.9263 | 1.25956+0.00400*Tau-0.00008*Urea+0.02028*Cys-0.00189*Leu-0.01117*Tyr-0.00561*His |
| 0.9263 | 1.21623+0.00450*Tau-0.00008*Urea+0.01483*Cys-0.00673*His-0.01120*Trp |
| 0.9263 | 1.29109+0.00403*Tau-0.00007*Urea-0.00128*Val+0.02050*Cys-0.01114*Tyr-0.00562*His |
| 0.9260 | 1.24421+0.00449*Tau-0.00008*Urea+0.01523*Cys-0.00658*His-0.01087*Trp-0.00040*Lys |
| 0.9259 | 1.20325+0.00412*Tau-0.00008*Urea+0.02025*Cys+0.00097*Met-0.01277*Tyr-0.00640*His |
| 0.9258 | 1.21237+0.00412*Tau-0.00008*Urea+0.02022*Cys-0.01262*Tyr-0.00631*His |
| 0.9258 | 1.23633+0.00450*Tau-0.00008*Urea+0.01492*Cys-0.00101*Phe-0.00650*His-0.01102*Trp |
| 0.9257 | 1.23813+0.00449*Tau-0.00008*Urea+0.01491*Cys-0.00233*Met-0.00648*His-0.01089*Trp |
| 0.9255 | 1.30278+0.00426*Tau-0.00007*Urea+0.01598*Cys-0.00309*Leu-0.00532*His-0.00975*Trp |
| 0.9253 | 1.22426+0.00412*Tau-0.00008*Urea+0.02034*Cys-0.01251*Tyr-0.00624*His-0.00016*Lys |
| 0.9253 | 1.35311+0.00434*Tau-0.00008*Urea-0.01091*Asn+0.01790*Cys-0.00339*Leu-0.00994*Trp |
| 0.9252 | 1.13359+0.00447*Tau-0.00008*Urea+0.01982*Cys+0.00026*Met-0.01208*Tyr-0.00955*Trp |
| 0.9251 | 1.13652+0.00447*Tau-0.00008*Urea+0.01982*Cys-0.01204*Tyr-0.00952*Trp |
| 0.9251 | 1.13612+0.00447*Tau-0.00008*Urea+0.01981*Cys-0.01204*Tyr-0.00952*Trp+0.00001*Lys |
| 0.9249 | 1.11775+0.00448*Tau-0.00008*Urea+0.01984*Cys-0.01229*Tyr+0.00078*Phe-0.00964*Trp |
| 0.9247 | 1.41039+0.00408*Tau-0.00942*Asn-0.00214*Val+0.02143*Cys-0.00974*Tyr-0.00473*His |
| 0.9244 | 1.34101+0.00410*Tau-0.00007*Urea-0.01083*Asn-0.00181*Val+0.02231*Cys-0.01046*Tyr |
| 0.9242 | 1.40396+0.00438*Tau-0.00007*Urea-0.01171*Asn-0.00228*Val+0.01841*Cys-0.00908*Trp |
| 0.9235 | 1.27988+0.00428*Tau+0.01918*Cys-0.00232*Leu-0.00972*Tyr-0.00482*His-0.00818*Trp |
| 0.9232 | 1.33974+0.00445*Tau-0.00812*Asn+0.02013*Cys-0.01034*Tyr-0.00458*His-0.00795*Trp |
| 0.9232 | 1.32656+0.00407*Tau-0.00894*Asn+0.02092*Cys-0.00262*Leu-0.01042*Tyr-0.00511*His |
| 0.9227 | 1.26475+0.00423*Tau-0.00010*Urea-0.01204*Asn+0.01672*Cys-0.00747*His |
| 0.9227 | 1.45083+0.00404*Tau-0.00007*Urea-0.01154*Asn-0.00254*Val+0.01858*Cys-0.00536*His |
| 0.9226 | 1.27781+0.00408*Tau-0.00008*Urea-0.01045*Asn+0.02194*Cys-0.00240*Leu-0.01084*Tyr |
| 0.9225 | 1.31321+0.00430*Tau-0.00155*Val+0.01952*Cys-0.00985*Tyr-0.00494*His-0.00752*Trp |
| 0.9224 | 1.28350+0.00423*Tau-0.00010*Urea-0.01171*Asn+0.01696*Cys-0.00735*His-0.00030*Lys |
| 0.9215 | 1.36550+0.00437*Tau-0.00984*Asn-0.00201*Val+0.02111*Cys-0.00914*Tyr-0.00772*Trp |
| 0.9213 | 1.22936+0.00424*Tau-0.00956*Asn+0.02093*Cys+0.00300*Met-0.01297*Tyr-0.00644*His |
| 0.9211 | 1.30544+0.00437*Tau-0.00923*Asn+0.02056*Cys-0.00276*Leu-0.00925*Tyr-0.00873*Trp |
| 0.9209 | 1.28419+0.00423*Tau-0.00010*Urea-0.01168*Asn+0.01674*Cys-0.00229*Met-0.00722*His |
| 0.9209 | 1.31245+0.00423*Tau-0.00009*Urea-0.01185*Asn+0.01692*Cys-0.00227*Phe-0.00688*His |
| 0.9206 | 1.19773+0.00446*Tau+0.01905*Cys+0.00250*Met-0.01179*Tyr-0.00588*His-0.00904*Trp |
| 0.9206 | 1.38978+0.00433*Tau-0.00958*Asn+0.01710*Cys-0.00360*Leu-0.00453*His-0.00966*Trp |
| 0.9205 | 1.46459+0.00434*Tau-0.01035*Asn-0.00258*Val+0.01784*Cys-0.00450*His-0.00837*Trp |
| 0.9203 | 1.24744+0.00424*Tau-0.00927*Asn+0.02075*Cys-0.01262*Tyr-0.00622*His+0.00008*Lys |
| 0.9202 | 1.21909+0.00445*Tau+0.01900*Cys-0.01147*Tyr-0.00567*His-0.00882*Trp |
| 0.9202 | 1.22074+0.00445*Tau+0.01901*Cys-0.01146*Tyr-0.00567*His-0.00881*Trp-0.00002*Lys |
| 0.9201 | 1.09354+0.00405*Tau-0.00008*Urea-0.00103*Val+0.02087*Cys-0.00159*Leu-0.01270*Tyr |
| 0.9201 | 1.20065+0.00446*Tau+0.01901*Cys-0.01174*Tyr+0.00095*Phe-0.00586*His-0.00892*Trp |
| 0.9198 | 1.25240+0.00424*Tau-0.00919*Asn+0.02080*Cys-0.01257*Tyr-0.00620*His |
| 0.9198 | 1.25277+0.00424*Tau-0.00919*Asn+0.02080*Cys-0.01257*Tyr-0.00002*Phe-0.00620*His |
| 0.9198 | 1.36803+0.00400*Tau-0.00008*Urea-0.01102*Asn+0.01789*Cys-0.00346*Leu-0.00563*His |

# FIG.49

| ROC AUC | FORMULA |
|---|---|
| 0.9197 | 1.01238+0.00403*Tau-0.00009*Urea+0.02079*Cys-0.00316*Leu-0.01348*Tyr+0.00215*Phe |
| 0.9197 | 1.15390+0.00437*Tau-0.00008*Urea-0.00047*Val+0.01587*Cys-0.00343*Leu-0.01201*Trp |
| 0.9195 | 1.12970+0.00437*Tau-0.00008*Urea+0.01571*Cys-0.00408*Leu+0.00052*Phe-0.01234*Trp |
| 0.9194 | 1.13995+0.00438*Tau-0.00008*Urea+0.01573*Cys-0.00398*Leu-0.01226*Trp |
| 0.9193 | 1.11801+0.00437*Tau-0.00008*Urea+0.01543*Cys-0.00417*Leu-0.01250*Trp+0.00036*Lys |
| 0.9192 | 1.05189+0.00405*Tau-0.00008*Urea+0.02070*Cys+0.00038*Met-0.00282*Leu-0.01306*Tyr |
| 0.9192 | 1.05206+0.00405*Tau-0.00009*Urea+0.02065*Cys-0.00282*Leu-0.01304*Tyr+0.00005*Lys |
| 0.9192 | 1.25209+0.00406*Tau-0.00208*Val+0.02016*Cys-0.01144*Tyr-0.00628*His |
| 0.9191 | 1.25281+0.00412*Tau-0.01244*Asn-0.00302*Val+0.02161*Cys-0.01104*Tyr+0.00095*Lys |
| 0.9190 | 1.22891+0.00405*Tau-0.00221*Val+0.02029*Cys+0.00342*Met-0.01179*Tyr-0.00654*His |
| 0.9190 | 1.18270+0.00424*Tau-0.00009*Urea-0.01117*Asn+0.02206*Cys+0.00101*Met-0.01313*Tyr |
| 0.9190 | 1.26133+0.00413*Tau-0.01220*Asn-0.00294*Val+0.02240*Cys+0.00494*Met-0.01131*Tyr |
| 0.9189 | 1.28287+0.00464*Tau-0.01041*Asn+0.01570*Cys+0.00019*Met-0.00627*His-0.01161*Trp |
| 0.9189 | 1.21446+0.00404*Tau-0.00230*Val+0.02027*Cys-0.01196*Tyr+0.00258*Phe-0.00670*His |
| 0.9189 | 1.28555+0.00464*Tau-0.01036*Asn+0.01572*Cys-0.00625*His-0.01157*Trp-0.00002*Lys |
| 0.9188 | 1.28431+0.00464*Tau-0.01038*Asn+0.01570*Cys-0.00625*His-0.01159*Trp |
| 0.9188 | 1.17742+0.00424*Tau-0.00009*Urea-0.01126*Asn+0.02187*Cys-0.01312*Tyr+0.00023*Lys |
| 0.9188 | 1.24255+0.00405*Tau-0.00213*Val+0.02005*Cys-0.01150*Tyr-0.00632*His+0.00017*Lys |
| 0.9187 | 1.05581+0.00405*Tau-0.00008*Urea+0.02069*Cys-0.00279*Leu-0.01302*Tyr |
| 0.9183 | 1.10149+0.00410*Tau-0.00008*Urea-0.00189*Val+0.02102*Cys-0.01299*Tyr |
| 0.9182 | 1.19287+0.00424*Tau-0.00009*Urea-0.01101*Asn+0.02200*Cys-0.01297*Tyr |
| 0.9182 | 1.29100+0.00414*Tau-0.01146*Asn-0.00270*Val+0.02209*Cys-0.01086*Tyr |
| 0.9182 | 1.19444+0.00424*Tau-0.00009*Urea-0.01101*Asn+0.02200*Cys-0.01295*Tyr-0.00006*Phe |
| 0.9181 | 1.15719+0.00402*Tau+0.01986*Cys+0.00365*Met-0.00301*Leu-0.01219*Tyr-0.00674*His |
| 0.9178 | 1.07491+0.00409*Tau-0.00008*Urea-0.00202*Val+0.02110*Cys-0.01333*Tyr+0.00138*Phe |
| 0.9176 | 1.38667+0.00405*Tau-0.01281*Asn-0.00366*Val+0.01774*Cys-0.00608*His+0.00082*Lys |
| 0.9174 | 1.18473+0.00404*Tau+0.01974*Cys-0.00275*Leu-0.01184*Tyr-0.00649*His |
| 0.9162 | 1.04445+0.00421*Tau-0.00010*Urea+0.01416*Cys-0.00994*His |
| 0.9158 | 0.94214+0.00424*Tau-0.00010*Urea+0.02068*Cys-0.01566*Tyr |
| 0.9143 | 1.55166+0.00482*Tau-0.00006*Urea-0.00151*Val-0.00570*His-0.00999*Trp+0.00128*Lys |
| 0.9119 | 1.51897+0.00473*Tau-0.00007*Urea-0.00312*Leu-0.00526*His-0.01039*Trp+0.00161*Lys |
| 0.9115 | 1.64519+0.00456*Tau-0.00007*Urea-0.01004*Asn-0.00236*Val-0.00597*His+0.00178*Lys |
| 0.9111 | 1.49734+0.00493*Tau-0.00008*Urea-0.00401*Met-0.00608*His-0.01090*Trp+0.00106*Lys |
| 0.9085 | 1.55540+0.00495*Tau-0.00007*Urea-0.00607*His-0.01050*Trp |
| 0.9085 | 1.52201+0.00471*Tau-0.00009*Urea-0.00952*Asn-0.00754*His+0.00073*Lys |
| 0.9055 | 1.37942+0.00465*Tau-0.00009*Urea-0.00912*His |
| 0.9050 | 1.19362+0.00473*Tau-0.01126*His |
| 0.8796 | 1.57374-0.00009*Urea-0.00865*Asn+0.02411*Cys-0.01225*Tyr+0.00259*Phe-0.00602*His |
| 0.8786 | 1.59457-0.00009*Urea-0.00910*Asn+0.02420*Cys+0.00380*Met-0.01200*Tyr-0.00582*His |
| 0.8782 | 1.67335-0.00008*Urea-0.00844*Asn+0.02398*Cys-0.00256*Leu-0.00955*Tyr-0.00458*His |
| 0.8777 | 1.67318-0.00008*Urea-0.00805*Asn+0.02370*Cys-0.01037*Tyr-0.00471*His-0.00445*Trp |
| 0.8773 | 1.62284-0.00009*Urea-0.00864*Asn+0.02403*Cys-0.01151*Tyr-0.00552*His |
| 0.8767 | 1.72777-0.00007*Urea-0.00889*Asn-0.00177*Val+0.02438*Cys-0.00941*Tyr-0.00452*His |
| 0.8300 | 1.94177-0.00007*Urea-0.00789*Asn-0.00385*Leu-0.00469*Tyr-0.00579*His+0.00248*Lys |
| 0.8260 | 1.92740-0.00008*Urea-0.00717*Asn-0.00704*Tyr+0.00039*Phe-0.00705*His+0.00150*Lys |
| 0.8248 | 1.87884-0.00008*Urea-0.00802*Asn-0.00490*Leu+0.00202*Phe-0.00671*His+0.00214*Lys |
| 0.8244 | 1.94172-0.00007*Urea-0.00927*Asn-0.00094*Val-0.00351*Leu-0.00622*His+0.00236*Lys |
| 0.8238 | 1.90871-0.00008*Urea-0.00898*Asn-0.00186*Met-0.00453*Leu-0.00628*His+0.00240*Lys |
| 0.8232 | 1.96661-0.00007*Urea-0.00814*Asn-0.00429*Leu-0.00494*His-0.00738*Trp+0.00267*Lys |
| 0.8227 | 1.84511-0.00007*Urea-0.00362*Leu-0.00589*Tyr-0.00683*His+0.00183*Lys |

## FIG.50

# FIG.51

| ROC AUC | FORMULA |
|---|---|
| 0.9529 | 2.57627+0.11594*Tau-0.00072*Urea+0.21479*Cys-0.08691*Tyr-0.04143*His-0.09405*Trp |
| 0.9507 | 2.35968+0.11082*Tau-0.00070*Urea+0.20670*Cys-0.02219*Leu-0.07770*Tyr-0.10813*Trp |
| 0.9498 | 2.53977+0.11197*Tau-0.00071*Urea-0.01318*Val+0.20514*Cys-0.08016*Tyr-0.10269*Trp |
| 0.9494 | 2.82828+0.11588*Tau-0.00062*Urea+0.17569*Cys-0.02844*Leu-0.04346*His-0.11849*Trp |
| 0.9492 | 2.35246+0.10887*Tau-0.00077*Urea-0.03887*Asn+0.20675*Cys-0.08873*Tyr-0.10172*Trp |
| 0.9490 | 1.47153+0.11125*Tau-0.00081*Urea+0.20046*Cys-0.09838*Tyr-0.11543*Trp+0.00350*Lys |
| 0.9488 | 1.73513+0.11044*Tau-0.00080*Urea+0.20244*Cys-0.09523*Tyr-0.11239*Trp |
| 0.9488 | 1.42176+0.11152*Tau-0.00081*Urea+0.20329*Cys+0.02895*Met-0.10152*Tyr-0.11506*Trp |
| 0.9484 | 1.23213+0.11221*Tau-0.00084*Urea+0.20436*Cys-0.10225*Tyr+0.02112*Phe-0.11794*Trp |
| 0.9480 | 2.50626+0.11559*Tau+0.20470*Cys-0.02679*Leu-0.07248*Tyr-0.04760*His-0.09015*Trp |
| 0.9476 | 2.67644+0.11572*Tau-0.01671*Val+0.20322*Cys-0.07590*Tyr-0.04666*His-0.08018*Trp |
| 0.9476 | 2.65969+0.11226*Tau-0.00069*Urea-0.04508*Asn+0.17015*Cys-0.05169*His-0.11215*Trp |
| 0.9475 | 3.09820+0.11603*Tau-0.00064*Urea-0.01754*Val+0.17296*Cys-0.04397*His-0.11009*Trp |
| 0.9469 | 2.72319+0.10892*Tau-0.00066*Urea-0.05437*Asn+0.17367*Cys-0.02931*Leu-0.12267*Trp |
| 0.9467 | 3.19958+0.10989*Tau-0.00066*Urea-0.06221*Asn-0.02005*Val+0.17613*Cys-0.11035*Trp |
| 0.9466 | 2.83847+0.10422*Tau-0.00059*Urea-0.01575*Val+0.21724*Cys-0.09364*Tyr-0.05546*His |
| 0.9465 | 1.94209+0.11469*Tau-0.00076*Urea+0.15659*Cys+0.01441*Phe-0.06295*His-0.12445*Trp |
| 0.9457 | 2.54678+0.10081*Tau-0.00062*Urea+0.21797*Cys-0.02131*Leu-0.09746*Tyr-0.05631*His |
| 0.9454 | 2.18108+0.11379*Tau-0.00073*Urea+0.15805*Cys-0.05902*His-0.12119*Trp |
| 0.9454 | 2.17730+0.11379*Tau-0.00073*Urea+0.15801*Cys+0.00048*Met-0.05908*His-0.12125*Trp |
| 0.9453 | 1.69626+0.10195*Tau-0.00073*Urea+0.21208*Cys-0.11393*Tyr+0.02226*Phe-0.07216*His |
| 0.9450 | 2.22253+0.11377*Tau-0.00073*Urea+0.16000*Cys-0.05785*His-0.12043*Trp-0.00132*Lys |
| 0.9449 | 2.09427+0.11192*Tau-0.03530*Asn+0.19979*Cys-0.08374*Tyr-0.04883*His-0.08700*Trp |
| 0.9448 | 2.20942+0.10062*Tau-0.00069*Urea+0.21070*Cys-0.10740*Tyr-0.06795*His |
| 0.9448 | 2.01714+0.10082*Tau-0.00070*Urea+0.21126*Cys+0.01892*Met-0.11122*Tyr-0.06902*His |
| 0.9447 | 2.24185+0.10061*Tau-0.00069*Urea+0.21114*Cys-0.10694*Tyr-0.06757*His-0.00058*Lys |
| 0.9445 | 2.75716+0.09970*Tau-0.00067*Urea-0.04271*Asn+0.21648*Cys-0.10042*Tyr-0.05921*His |
| 0.9445 | 1.33205+0.11188*Tau-0.00074*Urea+0.16255*Cys-0.03876*Leu+0.02871*Phe-0.14501*Trp |
| 0.9441 | 1.90873+0.10982*Tau-0.00071*Urea+0.16190*Cys-0.03291*Leu-0.13645*Trp |
| 0.9440 | 2.65365+0.10889*Tau-0.05553*Asn-0.01909*Val+0.19595*Cys-0.07140*Tyr-0.08757*Trp |
| 0.9440 | 1.69548+0.11325*Tau+0.19385*Cys-0.08977*Tyr-0.05499*His-0.09115*Trp |
| 0.9437 | 2.47613+0.11465*Tau-0.04690*Asn+0.18228*Cys-0.03240*Leu-0.04432*His-0.11078*Trp |
| 0.9437 | 1.66366+0.11000*Tau-0.00071*Urea+0.15618*Cys-0.03656*Leu-0.14114*Trp+0.00581*Lys |
| 0.9437 | 1.55319+0.11364*Tau+0.19158*Cys-0.09181*Tyr-0.05604*His-0.09250*Trp+0.00251*Lys |
| 0.9437 | 1.35079+0.11427*Tau+0.19340*Cys-0.09437*Tyr+0.01590*Phe-0.05789*His-0.09307*Trp |
| 0.9437 | 2.15027+0.10824*Tau-0.04689*Asn+0.19394*Cys-0.02759*Leu-0.07101*Tyr-0.10155*Trp |
| 0.9435 | 2.97812+0.11416*Tau-0.05712*Asn-0.02174*Val+0.18158*Cys-0.04283*His-0.09473*Trp |
| 0.9434 | 2.85360+0.09458*Tau-0.00064*Urea-0.06820*Asn-0.01869*Val+0.20938*Cys-0.09026*Tyr |
| 0.9434 | 1.42963+0.11420*Tau+0.19274*Cys+0.03292*Met-0.09541*Tyr-0.05676*His-0.09387*Trp |
| 0.9433 | 2.15777+0.11003*Tau-0.00070*Urea-0.00712*Val+0.16285*Cys-0.02439*Leu-0.13228*Trp |
| 0.9432 | 3.37857+0.10314*Tau-0.05016*Asn-0.02066*Val+0.21050*Cys-0.08552*Tyr-0.05346*His |
| 0.9420 | 2.27697+0.10589*Tau-0.02266*Val+0.20594*Cys+0.04617*Met-0.10056*Tyr-0.06612*His |
| 0.9418 | 2.36961+0.10515*Tau-0.02236*Val+0.20105*Cys-0.09570*Tyr-0.06655*His+0.00536*Lys |
| 0.9417 | 2.08684+0.10655*Tau-0.02286*Val+0.20533*Cys-0.09955*Tyr+0.02674*Phe-0.06811*His |
| 0.9406 | 2.93609+0.09310*Tau-0.00067*Urea-0.07322*Asn+0.14498*Cys-0.08616*His |
| 0.9406 | 2.96763+0.09301*Tau-0.00067*Urea-0.07272*Asn+0.14517*Cys-0.00195*Phe-0.08561*His |
| 0.9404 | 2.66109+0.10439*Tau-0.02073*Val+0.20370*Cys-0.09295*Tyr-0.06455*His |
| 0.9402 | 2.30775+0.09056*Tau-0.00071*Urea-0.06324*Asn+0.20663*Cys-0.02185*Leu-0.09845*Tyr |
| 0.9401 | 3.01486+0.09313*Tau-0.00067*Urea-0.07023*Asn+0.14586*Cys-0.01322*Met-0.08486*His |
| 0.9401 | 2.02399+0.11173*Tau-0.06458*Asn+0.15993*Cys+0.01663*Met-0.06740*His-0.11227*Trp |

# FIG.52

| ROC AUC | FORMULA |
|---|---|
| 0.9398 | 2.98464+0.09316*Tau-0.00067*Urea-0.07022*Asn+0.14722*Cys-0.08462*His-0.00210*Lys |
| 0.9396 | 3.63666+0.09714*Tau-0.00053*Urea-0.06670*Asn-0.02299*Val+0.16819*Cys-0.05786*His |
| 0.9396 | 2.79596+0.09850*Tau-0.04375*Asn+0.20788*Cys-0.02506*Leu-0.09184*Tyr-0.05981*His |
| 0.9394 | 3.03195+0.09203*Tau-0.00059*Urea-0.06217*Asn+0.15833*Cys-0.02746*Leu-0.06332*His |
| 0.9393 | 2.30977+0.09927*Tau-0.05898*Asn+0.19824*Cys+0.03871*Met-0.10820*Tyr-0.07673*His |
| 0.9392 | 2.09150+0.11125*Tau-0.06141*Asn+0.16133*Cys-0.06581*His-0.11063*Trp |
| 0.9392 | 1.03339+0.09571*Tau-0.00073*Urea-0.02094*Val+0.20300*Cys-0.11179*Tyr+0.01890*Phe |
| 0.9392 | 2.09091+0.11125*Tau-0.06145*Asn+0.16128*Cys-0.06584*His-0.11064*Trp+0.00003*Lys |
| 0.9387 | 0.46238+0.09187*Tau-0.00079*Urea+0.20413*Cys-0.03016*Leu-0.11868*Tyr+0.02672*Phe |
| 0.9387 | 1.71801+0.10234*Tau+0.20565*Cys+0.05583*Met-0.03147*Leu-0.10623*Tyr-0.07007*His |
| 0.9384 | 0.75169+0.09121*Tau-0.00077*Urea+0.20326*Cys+0.03905*Met-0.02841*Leu-0.11888*Tyr |
| 0.9383 | 2.29403+0.09944*Tau-0.05379*Asn+0.19856*Cys-0.10519*Tyr+0.01127*Phe-0.07724*His |
| 0.9382 | 0.90505+0.09045*Tau-0.00077*Urea+0.19834*Cys-0.02703*Leu-0.11430*Tyr+0.00341*Lys |
| 0.9381 | 2.51509+0.09882*Tau-0.05133*Asn+0.19830*Cys-0.10229*Tyr-0.07505*His |
| 0.9381 | 2.44978+0.09886*Tau-0.05320*Asn+0.19729*Cys-0.10312*Tyr-0.07593*His+0.00161*Lys |
| 0.9380 | 1.48967+0.09440*Tau-0.00070*Urea-0.01929*Val+0.20022*Cys-0.10609*Tyr |
| 0.9379 | 1.47354+0.09344*Tau-0.00071*Urea-0.01443*Val+0.20074*Cys-0.00893*Leu-0.10518*Tyr |
| 0.9377 | 1.09668+0.09037*Tau-0.00076*Urea+0.19990*Cys-0.02530*Leu-0.11138*Tyr |
| 0.9373 | 2.15577+0.10094*Tau+0.20322*Cys-0.02702*Leu-0.09759*Tyr-0.06807*His |
| 0.9371 | 3.36346+0.09848*Tau-0.08283*Asn-0.02937*Val+0.15806*Cys-0.06850*His+0.00731*Lys |
| 0.9363 | 2.36009+0.09797*Tau-0.09938*Asn-0.02776*Val+0.19881*Cys+0.08501*Met-0.10351*Tyr |
| 0.9361 | 2.24489+0.09633*Tau-0.09656*Asn-0.02845*Val+0.19162*Cys-0.09647*Tyr+0.01209*Lys |
| 0.9355 | 1.55374+0.08992*Tau-0.00081*Urea-0.07094*Asn+0.20576*Cys-0.11720*Tyr+0.00406*Phe |
| 0.9355 | 1.38092+0.09053*Tau-0.00081*Urea-0.07899*Asn+0.20791*Cys+0.03996*Met-0.12348*Tyr |
| 0.9349 | 2.69172+0.09395*Tau-0.07977*Asn-0.02460*Val+0.19475*Cys-0.09162*Tyr |
| 0.9348 | 1.64826+0.08983*Tau-0.00080*Urea-0.07019*Asn+0.20550*Cys-0.11573*Tyr |
| 0.9347 | 1.81644+0.09416*Tau-0.00071*Urea+0.12499*Cys-0.10273*His |
| 0.9346 | 1.48664+0.08999*Tau-0.00081*Urea-0.07387*Asn+0.20483*Cys-0.11854*Tyr+0.00290*Lys |
| 0.9315 | 5.30799+0.10629*Tau-0.00049*Urea-0.01571*Val-0.04943+His-0.10048*Trp+0.01636*Lys |
| 0.9310 | 0.26702+0.08900*Tau-0.00087*Urea+0.19365*Cys-0.13277*Tyr |
| 0.9293 | 4.80516+0.10345*Tau-0.00057*Urea-0.00212*Met-0.06275*His-0.10395*Trp+0.01089*Lys |
| 0.9291 | 4.68801+0.10783*Tau-0.00048*Urea-0.02978*Leu-0.04282*His-0.10951*Trp+0.01877*Lys |
| 0.9258 | 5.76142+0.09447*Tau-0.00039*Urea-0.06928*Asn-0.02037*Val-0.06206*His+0.01566*Lys |
| 0.9248 | 5.36749+0.10106*Tau-0.00054*Urea-0.05185*His-0.09330*Trp |
| 0.9227 | 4.98562+0.09010*Tau-0.00052*Urea-0.06111*Asn-0.08085*His+0.00715*Lys |
| 0.9146 | 4.25767+0.08939*Tau-0.00053*Urea-0.08710*His |
| 0.9054 | 3.47945+0.08917*Tau-0.10376*His |
| 0.8813 | 7.34428-0.00059*Urea-0.04891*Asn+0.17633*Cys-0.08411*Tyr+0.01645*Phe-0.05176*His |
| 0.8810 | 7.51259-0.00057*Urea-0.05071*Asn+0.17663*Cys+0.02547*Met-0.08366*Tyr-0.05111*His |
| 0.8803 | 7.67954-0.00056*Urea-0.04843*Asn+0.17540*Cys-0.08078*Tyr-0.04805*His |
| 0.8797 | 8.04904-0.00056*Urea-0.04595*Asn+0.17362*Cys-0.07428*Tyr-0.04016*His-0.02946*Trp |
| 0.8784 | 8.25294-0.00049*Urea-0.04935*Asn-0.01117*Val+0.17783*Cys-0.06959*Tyr-0.03831*His |
| 0.8778 | 7.97450-0.00052*Urea-0.04802*Asn+0.17926*Cys-0.01897*Leu-0.07019*Tyr-0.03667*His |
| 0.8382 | 9.27114-0.00042*Urea-0.04969*Asn-0.02043*Leu-0.02889*Tyr-0.05206*His+0.01728*Lys |
| 0.8360 | 9.14430-0.00048*Urea-0.04666*Asn-0.03972*Tyr+0.00794*Phe-0.06423*His+0.01231*Lys |
| 0.8341 | 9.32137-0.00041*Urea-0.05548*Asn-0.00792*Val-0.01496*Leu-0.05639*His+0.01634*Lys |
| 0.8326 | 8.85831-0.00044*Urea-0.05549*Asn-0.02594*Leu+0.01253*Phe-0.05813*His+0.01427*Lys |
| 0.8320 | 9.52934-0.00042*Urea-0.05025*Asn-0.02378*Leu-0.04655*His-0.04872*Trp+0.01928*Lys |
| 0.8319 | 9.01104-0.00043*Urea-0.05452*Asn+0.00251*Met-0.02441*Leu-0.05658*His+0.01480*Lys |
| 0.8295 | 8.54215-0.00043*Urea-0.01927*Leu-0.03318*Tyr-0.05825*His+0.01299*Lys |

# FIG.53

# FIG.54

| ROC AUC | FORMULA |
|---|---|
| 0.9332 | 1.10711+0.00426*Tau+0.01052*Glu+0.00120*Gly-0.00247*Val-0.00955*Tyr-0.01140*Trp |
| 0.9314 | 1.02806+0.00429*Tau+0.01001*Glu+0.00122*Gly-0.00307*Leu-0.00983*Tyr-0.01264*Trp |
| 0.9307 | 1.02419+0.00442*Tau+0.01054*Glu+0.00126*Gly-0.01075*ABA-0.01122*Tyr-0.01390*Trp |
| 0.9303 | 0.94421+0.00430*Tau-0.00007*Urea+0.00816*Glu+0.00115*Gly-0.00349*Leu-0.01427*Trp |
| 0.9303 | 1.00081+0.00433*Tau-0.00006*Urea+0.00862*Glu+0.00113*Gly-0.00257*Val-0.01349*Trp |
| 0.9302 | 1.04595+0.00423*Tau+0.00890*Glu+0.00126*Gly-0.00306*Val-0.00377*His-0.01208*Trp |
| 0.9301 | 0.97137+0.00391*Tau-0.00006*Urea+0.01038*Glu+0.00146*Gly-0.00303*Val-0.01125*Tyr |
| 0.9299 | 1.07219+0.00383*Tau+0.01044*Glu+0.00160*Gly-0.00311*Val-0.01071*Tyr-0.00491*His |
| 0.9288 | 1.01582+0.00444*Tau-0.00007*Urea+0.00899*Glu+0.00126*Gly-0.01013*Tyr-0.01299*Trp |
| 0.9285 | 1.48917+0.00433*Tau-0.00005*Urea+0.00965*Glu-0.00188*Val-0.00854*Tyr-0.01220*Trp |
| 0.9281 | 0.95051+0.00426*Tau+0.00826*Glu+0.00129*Gly-0.00395*Leu-0.00388*His-0.01355*Trp |
| 0.9279 | 1.09860+0.00435*Tau-0.00852*Asn+0.00893*Glu+0.00140*Gly-0.00312*Val-0.01213*Trp |
| 0.9278 | 1.04915+0.00515*Tau-0.00008*Urea+0.00121*Gly+0.00160*Phe-0.00467*His-0.01239*Trp |
| 0.9275 | 1.05809+0.00398*Tau-0.00793*Asn+0.01049*Glu+0.00168*Gly-0.00358*Val-0.00994*Tyr |
| 0.9271 | 1.44542+0.00433*Tau-0.00006*Urea+0.00924*Glu-0.00235*Leu-0.00857*Tyr-0.01295*Trp |
| 0.9265 | 1.01842+0.00443*Tau+0.00906*Glu+0.00141*Gly-0.01079*Tyr-0.00392*His-0.01235*Trp |
| 0.9263 | 1.05082+0.00422*Tau+0.01030*Glu+0.00112*Gly-0.01005*ABA-0.00319*Val-0.01357*Trp |
| 0.9263 | 1.12549+0.00512*Tau-0.00007*Urea+0.00121*Gly-0.00052*Val-0.00418*His-0.01133*Trp |
| 0.9261 | 1.03884+0.00391*Tau-0.00938*Asn+0.00869*Glu+0.00183*Gly-0.00401*Val-0.00477*His |
| 0.9260 | 0.99288+0.00439*Tau-0.00837*Asn+0.00826*Glu+0.00142*Gly-0.00398*Leu-0.01380*Trp |
| 0.9259 | 0.92343+0.00385*Tau+0.00897*Glu+0.00163*Gly-0.00426*Val-0.00177*Phe-0.00609*His |
| 0.9257 | 1.07862+0.00515*Tau-0.00008*Urea+0.00124*Gly+0.00038*ABA-0.00441*His-0.01207*Trp |
| 0.9254 | 1.00287+0.00461*Tau-0.00644*Asn+0.00892*Glu+0.00145*Gly-0.01017*Tyr-0.01363*Trp |
| 0.9254 | 1.03749+0.00379*Tau+0.01217*Glu+0.00146*Gly-0.01083*ABA-0.00354*Val-0.01163*Tyr |
| 0.9254 | 1.47702+0.00425*Tau+0.01073*Glu-0.00955*ABA-0.00284*Leu-0.00916*Tyr-0.01330*Trp |
| 0.9253 | 1.08190+0.00515*Tau-0.00008*Urea+0.00124*Gly-0.00438*His-0.01206*Trp |
| 0.9251 | 0.89839+0.00385*Tau+0.00889*Glu+0.00160*Gly-0.00445*Val-0.00641*His |
| 0.9251 | 0.94354+0.00385*Tau+0.00971*Glu+0.00169*Gly-0.00373*Leu-0.01152*Tyr-0.00547*His |
| 0.9250 | 1.00028+0.00437*Tau+0.00929*Glu+0.00115*Gly-0.00322*Val-0.00220*Phe-0.01389*Trp |
| 0.9250 | 0.95967+0.00434*Tau+0.00919*Glu+0.00110*Gly-0.00267*Val-0.00144*Leu-0.01426*Trp |
| 0.9250 | 0.97137+0.00399*Tau-0.00007*Urea-0.01103*Asn+0.00842*Glu+0.00175*Gly-0.00373*Val |
| 0.9250 | 1.19300+0.00514*Tau-0.00008*Urea-0.00786*Asn+0.00146*Gly-0.00325*His-0.01051*Trp |
| 0.9248 | 0.91345+0.00464*Tau+0.00926*Glu+0.00124*Gly-0.01171*Tyr-0.01500*Trp |
| 0.9247 | 1.54339+0.00422*Tau+0.01119*Glu-0.00920*ABA-0.00233*Val-0.00889*Tyr-0.01209*Trp |
| 0.9247 | 0.95845+0.00423*Tau+0.00972*Glu+0.00115*Gly-0.01045*ABA-0.00423*Leu-0.01501*Trp |
| 0.9247 | 0.89396+0.00383*Tau+0.00888*Glu+0.00160*Gly-0.00402*Val-0.00079*Leu-0.00633*His |
| 0.9247 | 1.12725+0.00509*Tau-0.00007*Urea+0.00121*Gly-0.00110*Leu-0.00403*His-0.01124*Trp |
| 0.9245 | 0.93689+0.00382*Tau-0.00007*Urea+0.00830*Glu+0.00160*Gly-0.00346*Val-0.00590*His |
| 0.9241 | 0.96402+0.00438*Tau+0.00920*Glu+0.00110*Gly-0.00347*Val-0.01429*Trp |
| 0.9240 | 0.86536+0.00456*Tau-0.00008*Urea+0.00791*Glu+0.00118*Gly-0.00816*ABA-0.01676*Trp |
| 0.9239 | 0.96068+0.00394*Tau+0.01107*Glu+0.00148*Gly-0.00374*Val-0.01174*Tyr-0.00147*Phe |
| 0.9239 | 0.96930+0.00458*Tau+0.00943*Glu+0.00130*Gly-0.01105*Tyr-0.00259*Phe-0.01430*Trp |
| 0.9237 | 0.93727+0.00395*Tau+0.01106*Glu+0.00145*Gly-0.00391*Val-0.01203*Tyr |
| 0.9237 | 0.86251+0.00390*Tau-0.00007*Urea+0.00969*Glu+0.00152*Gly-0.00379*Leu-0.01169*Tyr |
| 0.9235 | 0.93507+0.00393*Tau+0.01105*Glu+0.00145*Gly-0.00369*Val-0.00041*Leu-0.01198*Tyr |
| 0.9235 | 0.96332+0.00461*Tau-0.00008*Urea-0.00900*Asn+0.00684*Glu+0.00148*Gly-0.01456*Trp |
| 0.9230 | 0.85930+0.00440*Tau+0.00852*Glu+0.00112*Gly-0.00462*Leu-0.01590*Trp |
| 0.9230 | 0.92267+0.00447*Tau-0.00008*Urea+0.00685*Glu+0.00135*Gly-0.00433*His-0.01416*Trp |
| 0.9227 | 0.89420+0.00400*Tau-0.00814*Asn+0.00971*Glu+0.00177*Gly-0.00445*Leu-0.01098*Tyr |
| 0.9225 | 0.91181+0.00438*Tau+0.00867*Glu+0.00119*Gly-0.00416*Leu-0.00273*Phe-0.01529*Trp |

# FIG.55

| ROC AUC | FORMULA |
|---|---|
| 0.9225 | 1.42584+0.00437*Tau+0.01020*Glu-0.00273*Val-0.00940*Tyr+0.00165*Phe-0.01285*Trp |
| 0.9224 | 0.97078+0.00481*Tau-0.00010*Urea-0.01027*Asn+0.00192*Gly+0.00105*ABA-0.00602*His |
| 0.9223 | 0.97919+0.00482*Tau-0.00009*Urea-0.01017*Asn+0.00191*Gly-0.00594*His |
| 0.9221 | 1.37871+0.00457*Tau-0.00007*Urea+0.00862*Glu-0.00977*Tyr-0.01449*Trp |
| 0.9220 | 1.53976+0.00437*Tau-0.00356*Asn+0.00996*Glu-0.00251*Val-0.00827*Tyr-0.01213*Trp |
| 0.9220 | 0.99061+0.00482*Tau-0.00009*Urea-0.01014*Asn+0.00192*Gly-0.00047*Phe-0.00583*His |
| 0.9220 | 1.45876+0.00435*Tau+0.01020*Glu-0.00233*Val-0.00044*Leu-0.00905*Tyr-0.01268*Trp |
| 0.9219 | 1.44338+0.00445*Tau-0.00006*Urea+0.00957*Glu-0.00766*ABA-0.00969*Tyr-0.01407*Trp |
| 0.9219 | 1.12833+0.00480*Tau-0.00008*Urea-0.00943*Asn+0.00176*Gly-0.00139*Val-0.00496*His |
| 0.9218 | 1.46065+0.00436*Tau+0.01022*Glu-0.00257*Val-0.00911*Tyr-0.01267*Trp |
| 0.9218 | 1.35237+0.00458*Tau-0.00007*Urea+0.00855*Glu-0.01000*Tyr+0.00106*Phe-0.01469*Trp |
| 0.9216 | 1.45878+0.00447*Tau-0.00007*Urea+0.00849*Glu-0.00931*Tyr-0.00221*His-0.01320*Trp |
| 0.9212 | 0.87527+0.00463*Tau-0.00008*Urea+0.00725*Glu+0.00125*Gly-0.00333*Phe-0.01625*Trp |
| 0.9210 | 1.34077+0.00442*Tau-0.00006*Urea+0.00841*Glu-0.00264*Val-0.01460*Trp |
| 0.9209 | 0.95680+0.00538*Tau-0.00009*Urea+0.00105*Gly-0.01518*Trp |
| 0.9208 | 0.92355+0.00402*Tau-0.00008*Urea+0.00846*Glu+0.00176*Gly-0.01203*Tyr-0.00592*His |
| 0.9206 | 1.52018+0.00393*Tau-0.00005*Urea+0.00965*Glu-0.00286*Val-0.01010*Tyr-0.00369*His |
| 0.9206 | 1.03846+0.00532*Tau-0.00008*Urea+0.00103*Gly-0.00086*Val-0.01374*Trp |
| 0.9206 | 1.38445+0.00441*Tau+0.00967*Glu-0.00315*Leu-0.00941*Tyr-0.01401*Trp |
| 0.9204 | 0.79853+0.00469*Tau-0.00009*Urea+0.00687*Glu+0.00117*Gly-0.01726*Trp |
| 0.9201 | 0.88918+0.00420*Tau-0.00703*Asn+0.00837*Glu+0.00200*Gly-0.01236*Tyr-0.00808*His |
| 0.9200 | 1.04043+0.00526*Tau-0.00008*Urea+0.00103*Gly-0.00166*Leu-0.01357*Trp |
| 0.9200 | 1.28743+0.00441*Tau-0.00007*Urea+0.00790*Glu-0.00353*Leu-0.01547*Trp |
| 0.9196 | 1.28437+0.00400*Tau-0.00007*Urea+0.00928*Glu-0.00416*Leu-0.01160*Tyr |
| 0.9196 | 1.43892+0.00393*Tau-0.00007*Urea+0.00890*Glu-0.00335*Leu-0.01056*Tyr-0.00399*His |
| 0.9193 | 1.03118+0.00506*Tau-0.00821*Asn+0.00185*Gly-0.00160*ABA-0.00785*Tyr-0.00607*His |
| 0.9192 | 0.80922+0.00419*Tau-0.00010*Urea-0.01051*Asn+0.00572*Glu+0.00209*Gly-0.00635*His |
| 0.9192 | 1.38849+0.00400*Tau-0.00006*Urea+0.01009*Glu-0.00336*Val-0.01105*Tyr |
| 0.9184 | 1.12245+0.00530*Tau-0.00008*Urea-0.00909*Asn+0.00136*Gly-0.01247*Trp |
| 0.9184 | 1.07874+0.00475*Tau-0.00008*Urea-0.00936*Asn+0.00180*Gly-0.00194*Leu-0.00505*His |
| 0.9178 | 1.19149+0.00533*Tau-0.00683*Asn+0.00145*Gly-0.00531*Tyr-0.00368*His-0.01058*Trp |
| 0.9175 | 0.60569+0.00441*Tau-0.01213*Asn+0.00538*Glu+0.00225*Gly-0.00825*His |
| 0.9173 | 1.36254+0.00420*Tau-0.00008*Urea+0.00788*Glu-0.01250*Tyr-0.00524*His |
| 0.9168 | 1.40283+0.00529*Tau-0.00008*Urea-0.00341*His-0.01412*Trp |
| 0.9160 | 1.07944+0.00534*Tau-0.00859*Asn+0.00150*Gly-0.00412*His-0.01265*Trp |
| 0.9159 | 0.78633+0.00477*Tau-0.00010*Urea+0.00170*Gly-0.00799*His |
| 0.9159 | 1.45303+0.00388*Tau+0.01185*Glu-0.01069*ABA-0.00386*Val-0.01143*Tyr |
| 0.9159 | 0.79214+0.00477*Tau-0.00010*Urea+0.00170*Gly-0.00060*ABA-0.00794*His |
| 0.9151 | 0.89343+0.00501*Tau-0.01130*Asn+0.00208*Gly-0.00405*Phe-0.00666*His |
| 0.9101 | 0.60000+0.00496*Tau+0.00180*Gly-0.00562*ABA-0.00967*His |
| 0.8894 | 1.39112-0.00006*Urea+0.01262*Glu+0.00171*Gly-0.00298*Val-0.01015*Tyr-0.00519*His |
| 0.8876 | 1.46790+0.01491*Glu+0.00137*Gly-0.01199*ABA-0.00322*Val-0.01003*Tyr-0.00842*Trp |
| 0.8871 | 1.42966+0.01436*Glu+0.00169*Gly-0.01054*ABA-0.00354*Val-0.01068*Tyr-0.00450*His |
| 0.8843 | 1.40254-0.00006*Urea+0.01313*Glu+0.00137*Gly-0.00280*Val-0.00970*Tyr-0.00857*Trp |
| 0.8840 | 1.29739-0.00007*Urea+0.01196*Glu+0.00178*Gly-0.00390*Leu-0.01038*Tyr-0.00537*His |
| 0.8831 | 1.34065-0.00005*Urea+0.01450*Glu+0.00156*Gly-0.01088*ABA-0.00361*Val-0.01129*Tyr |
| 0.8814 | 1.37016+0.01324*Glu+0.00171*Gly-0.00376*Val-0.01080*Tyr-0.00554*His |
| 0.8370 | 1.38604-0.00011*Urea-0.00971*Asn+0.00203*Gly+0.00027*Phe-0.00733*His |
| 0.8369 | 1.39280-0.00010*Urea-0.00969*Asn+0.00203*Gly-0.00727*His |
| 0.8276 | 1.20530-0.00011*Urea+0.00183*Gly-0.00922*His |

# FIG.56

# FIG.57

| ROC AUC | FORMULA |
|---|---|
| 0.9517 | 0.81765+0.11415*Tau+0.06468*Glu+0.01166*Gly-0.04213*Leu-0.03070*His-0.12380*Trp |
| 0.9508 | 1.22280+0.10911*Tau+0.06858*Glu+0.01150*Gly-0.03522*Leu-0.05490*Tyr-0.12901*Trp |
| 0.9495 | 1.61624+0.10397*Tau+0.07058*Glu+0.01220*Gly-0.02253*Val-0.05903*Tyr-0.11579*Trp |
| 0.9483 | 1.20759+0.08937*Tau+0.06968*Glu+0.01861*Gly-0.03139*Val-0.06555*Tyr-0.04552*His |
| 0.9479 | 1.07314+0.10963*Tau-0.00047*Urea+0.05465*Glu+0.00966*Gly-0.03498*Leu-0.13438*Trp |
| 0.9476 | 0.20379+0.10768*Tau+0.05856*Glu+0.01340*Gly-0.07424*Tyr-0.02428*His-0.12533*Trp |
| 0.9474 | 0.93311+0.10688*Tau+0.06851*Glu+0.01146*Gly-0.06651*ABA-0.07911*Tyr-0.14071*Trp |
| 0.9474 | 1.13585+0.10789*Tau+0.06520*Glu+0.01236*Gly-0.02736*Val-0.02931*His-0.10887*Trp |
| 0.9471 | 0.85060+0.11369*Tau-0.05472*Asn+0.06163*Glu+0.01146*Gly-0.04050*Leu-0.12419*Trp |
| 0.9470 | 0.68584+0.11216*Tau+0.06637*Glu+0.00881*Gly-0.03843*ABA-0.04013*Leu-0.14215*Trp |
| 0.9470 | 3.06010+0.11030*Tau+0.06996*Glu-0.02699*Val-0.05123*Tyr+0.04622*Phe-0.13540*Trp |
| 0.9469 | -0.14934+0.11664*Tau+0.06195*Glu+0.00829*Gly-0.04725*Leu+0.01834*Phe-0.14743*Trp |
| 0.9467 | 1.26126+0.10761*Tau-0.05902*Asn+0.06341*Glu+0.01257*Gly-0.02658*Val-0.10791*Trp |
| 0.9463 | 0.72351+0.11093*Tau+0.06388*Glu+0.00912*Gly-0.01291*Val-0.02759*Leu-0.13336*Trp |
| 0.9459 | 0.28367+0.11410*Tau+0.06143*Glu+0.00880*Gly-0.04363*Leu-0.14264*Trp |
| 0.9457 | 0.54821+0.10401*Tau-0.00048*Urea+0.05099*Glu+0.01230*Gly-0.06185*Tyr-0.13220*Trp |
| 0.9453 | 1.14204+0.10698*Tau+0.06814*Glu+0.00929*Gly-0.04329*ABA-0.02587*Val-0.12911*Trp |
| 0.9450 | -0.04557+0.10802*Tau+0.05669*Glu+0.01130*Gly-0.07893*Tyr-0.14283*Trp |
| 0.9450 | -0.14849+0.10841*Tau+0.05658*Glu+0.01121*Gly-0.08010*Tyr+0.00410*Phe-0.14401*Trp |
| 0.9448 | 4.13213+0.10848*Tau+0.07248*Glu-0.04149*ABA-0.03157*Leu-0.03948*Tyr-0.14099*Trp |
| 0.9448 | 0.36635+0.10670*Tau-0.04144*Asn+0.05700*Glu+0.01306*Gly-0.07356*Tyr-0.12719*Trp |
| 0.9448 | 4.59181+0.10445*Tau+0.07460*Glu-0.04340*ABA-0.02015*Val-0.04210*Tyr-0.12982*Trp |
| 0.9445 | 4.05839+0.10848*Tau-0.00034*Urea+0.06061*Glu-0.03021*Leu-0.02855*Tyr-0.13756*Trp |
| 0.9445 | 3.96467+0.10845*Tau+0.06778*Glu-0.00986*Val-0.02268*Leu-0.03672*Tyr-0.13495*Trp |
| 0.9445 | 4.27486+0.10570*Tau-0.01026*Asn+0.06786*Glu-0.02205*Val-0.03855*Tyr-0.12647*Trp |
| 0.9443 | 1.43993+0.10469*Tau-0.00046*Urea+0.05473*Glu+0.01000*Gly-0.02190*Val-0.12541*Trp |
| 0.9440 | 4.39214+0.10437*Tau-0.00031*Urea+0.06199*Glu-0.01851*Val-0.03233*Tyr-0.12888*Trp |
| 0.9440 | 4.09218+0.10565*Tau+0.06769*Glu-0.02208*Val-0.04051*Tyr-0.12951*Trp |
| 0.9437 | 3.62239+0.11049*Tau+0.06600*Glu-0.03485*Leu-0.03779*Tyr-0.14173*Trp |
| 0.9436 | 0.44495+0.10979*Tau+0.06304*Glu+0.00869*Gly-0.02989*Val+0.01524*Phe-0.13177*Trp |
| 0.9434 | 0.20985+0.09262*Tau+0.06221*Glu+0.01799*Gly-0.04141*Leu-0.07106*Tyr-0.04889*His |
| 0.9434 | 0.74985+0.10791*Tau+0.06230*Glu+0.00920*Gly-0.02793*Val-0.12853*Trp |
| 0.9434 | 3.43699+0.11190*Tau-0.00042*Urea+0.05625*Glu-0.03373*Leu-0.14535*Trp |
| 0.9429 | 0.84827+0.09548*Tau-0.07391*Asn+0.06307*Glu+0.01832*Gly-0.03554*Val-0.03714*His |
| 0.9427 | 0.04645+0.09372*Tau+0.06157*Glu+0.01638*Gly-0.03049*Val-0.01452*Leu-0.05299*His |
| 0.9427 | 1.30995+0.09196*Tau-0.09250*Asn+0.06548*Glu+0.01854*Gly-0.03001*Val-0.06273*Tyr |
| 0.9415 | 0.28336+0.09008*Tau-0.00038*Urea+0.05566*Glu+0.01663*Gly-0.03260*Val-0.04734*His |
| 0.9414 | 3.80286+0.10737*Tau-0.00041*Urea+0.05687*Glu-0.02110*Val-0.13658*Trp |
| 0.9413 | 0.09780+0.09296*Tau+0.06078*Glu+0.01637*Gly-0.03812*Val-0.05314*His |
| 0.9412 | -0.11201+0.09421*Tau+0.06066*Glu+0.01612*Gly-0.03943*Val+0.01083*Phe-0.05484*His |
| 0.9407 | 2.40155+0.10696*Tau-0.00052*Urea+0.04985*Glu-0.05396*Tyr+0.03110*Phe-0.15349*Trp |
| 0.9405 | -0.25806+0.10840*Tau-0.00059*Urea+0.03939*Glu+0.01156*Gly-0.02289*His-0.13488*Trp |
| 0.9399 | 0.83304+0.08337*Tau+0.07286*Glu+0.01490*Gly-0.05532*ABA-0.03104*Val-0.08087*Tyr |
| 0.9397 | 1.17562+0.11468*Tau-0.00053*Urea+0.01130*Gly-0.02074*Leu-0.01643*His-0.10729*Trp |
| 0.9395 | 0.79254+0.09354*Tau-0.00046*Urea-0.10538*Asn+0.05386*Glu+0.01734*Gly-0.03064*Val |
| 0.9395 | 3.61433+0.10483*Tau-0.00039*Urea+0.05813*Glu-0.03976*ABA-0.04705*Tyr-0.14699*Trp |
| 0.9394 | -0.01399+0.10822*Tau-0.00062*Urea-0.04916*Asn+0.03966*Glu+0.01214*Gly-0.13355*Trp |
| 0.9393 | 3.15735+0.10509*Tau-0.00046*Urea+0.05021*Glu-0.04525*Tyr-0.14650*Trp |
| 0.9382 | -0.07424+0.10868*Tau-0.00061*Urea+0.04408*Glu+0.00970*Gly-0.03864*ABA-0.15171*Trp |
| 0.9387 | 3.32375+0.10490*Tau-0.00044*Urea+0.05048*Glu-0.04322*Tyr-0.00692*His-0.14236*Trp |

EP 2 124 059 A1

# FIG.58

| ROC AUC | FORMULA |
|---|---|
| 0.9387 | 1.06918+0.11509*Tau-0.00058*Urea+0.00961*Gly-0.02103*Leu-0.11990*Trp |
| 0.9384 | 1.24647+0.11180*Tau-0.00054*Urea+0.01168*Gly-0.01138*Val-0.01715*His-0.10481*Trp |
| 0.9380 | -0.38113+0.10861*Tau-0.00067*Urea+0.03685*Glu+0.00946*Gly-0.15156*Trp |
| 0.9380 | -0.37525+0.10858*Tau-0.00067*Urea+0.03687*Glu+0.00947*Gly-0.00027*Phe-0.15146*Trp |
| 0.9377 | 1.12468+0.11266*Tau-0.00059*Urea+0.00899*Gly-0.01164*Val-0.11810*Trp |
| 0.9377 | 0.16465+0.09515*Tau-0.09800*Asn+0.05654*Glu+0.01807*Gly-0.03815*Leu-0.06730*Tyr |
| 0.9375 | 0.44391+0.08360*Tau-0.00039*Urea+0.05950*Glu+0.01509*Gly-0.02772*Val-0.07258*Tyr |
| 0.9371 | -0.17126+0.08743*Tau+0.06584*Glu+0.01449*Gly-0.03513*Val-0.08151*Tyr+0.01434*Phe |
| 0.9361 | 0.05979+0.08673*Tau+0.06521*Glu+0.01468*Gly-0.03019*Val-0.00625*Leu-0.07690*Tyr |
| 0.9356 | 0.43369+0.11072*Tau-0.00065*Urea-0.04129*Asn+0.01227*Gly-0.11922*Trp |
| 0.9350 | 0.33018+0.11023*Tau-0.00063*Urea+0.01152*Gly-0.01944*His-0.12004*Trp |
| 0.9348 | 0.08235+0.08846*Tau+0.06538*Glu+0.01474*Gly-0.03347*Val-0.07770*Tyr |
| 0.9347 | -0.05733+0.11095*Tau-0.00068*Urea+0.01116*Gly+0.02162*Phe-0.02346*His-0.12591*Trp |
| 0.9340 | 0.18358+0.11027*Tau-0.00065*Urea+0.01163*Gly+0.01593*ABA-0.02145*His-0.12023*Trp |
| 0.9339 | 0.60766+0.10959*Tau-0.00061*Urea-0.03266*Asn+0.01278*Gly-0.01465*His-0.11092*Trp |
| 0.9335 | -0.47149+0.08505*Tau-0.00053*Urea+0.04269*Glu+0.01815*Gly-0.08124*Tyr-0.05018*His |
| 0.9334 | -0.22786+0.08497*Tau-0.00051*Urea+0.05156*Glu+0.01440*Gly-0.03526*Leu-0.07723*Tyr |
| 0.9332 | 0.53614+0.11046*Tau-0.03160*Asn+0.01296*Gly-0.04637*Tyr-0.01813*His-0.10043*Trp |
| 0.9330 | 0.07145+0.11158*Tau-0.00069*Urea+0.00993*Gly-0.13562*Trp |
| 0.9313 | -0.50180+0.08940*Tau-0.00066*Urea-0.08459*Asn+0.02880*Glu+0.01748*Gly-0.05107*His |
| 0.9312 | 4.33251+0.08594*Tau-0.00042*Urea+0.05127*Glu-0.03639*Leu-0.06469*Tyr-0.02660*His |
| 0.9306 | -0.17105+0.08946*Tau-0.06959*Asn+0.04625*Glu+0.01911*Gly-0.08398*Tyr-0.04750*His |
| 0.9300 | 0.35987+0.10155*Tau-0.00054*Urea-0.07101*Asn+0.01625*Gly-0.02696*Leu-0.02785*His |
| 0.9296 | 4.95613+0.08415*Tau-0.00030*Urea+0.05766*Glu-0.02761*Val-0.05899*Tyr-0.02553*His |
| 0.9294 | 1.00791+0.10057*Tau-0.00048*Urea-0.06859*Asn+0.01630*Gly-0.01855*Val-0.02924*His |
| 0.9292 | -1.17589+0.09171*Tau-0.09635*Asn+0.02924*Glu+0.01775*Gly-0.07088*His |
| 0.9290 | 3.45658+0.08396*Tau-0.00053*Urea+0.04735*Glu-0.03606*Leu-0.07487*Tyr |
| 0.9289 | 4.14373+0.08276*Tau-0.00039*Urea+0.05513*Glu-0.02814*Val-0.06897*Tyr |
| 0.9286 | -0.13668+0.09312*Tau-0.00068*Urea-0.08071*Asn+0.01679*Gly+0.02639*ABA-0.05047*His |
| 0.9275 | -0.51006+0.11146*Tau-0.04383*Asn+0.01307*Gly-0.02428*His-0.11459*Trp |
| 0.9275 | 4.34756+0.08173*Tau+0.06645*Glu-0.04692*ABA-0.03179*Val-0.07447*Tyr |
| 0.9271 | 2.94843+0.10988*Tau-0.00061*Urea-0.00993*His-0.13350*Trp |
| 0.9268 | 0.02664+0.09280*Tau-0.00066*Urea-0.07755*Asn+0.01654*Gly-0.04702*His |
| 0.9264 | -0.00445+0.09276*Tau-0.00066*Urea-0.07789*Asn+0.01653*Gly+0.00176*Phe-0.04749*His |
| 0.9261 | 0.46572+0.09608*Tau-0.07033*Asn+0.01700*Gly+0.00673*ABA-0.06166*Tyr-0.04887*His |
| 0.9240 | -0.28683+0.09381*Tau-0.08404*Asn+0.01688*Gly-0.01937*Phe-0.05962*His |
| 0.9237 | -1.10938+0.09114*Tau-0.00072*Urea+0.01471*Gly+0.01704*ABA-0.07083*His |
| 0.9224 | -0.98115+0.09109*Tau-0.00070*Urea+0.01451*Gly-0.06767*His |
| 0.9214 | 3.79262+0.07981*Tau-0.00056*Urea+0.03656*Glu-0.07846*Tyr-0.04255*His |
| 0.9188 | -1.74150+0.09243*Tau+0.01413*Gly-0.00662*ABA-0.09030*His |
| 0.8931 | 6.34975-0.00036*Urea+0.09171*Glu+0.01658*Gly-0.02589*Val-0.07216*Tyr-0.03907*His |
| 0.8913 | 6.89801+0.10504*Glu+0.01278*Gly-0.08344*ABA-0.02463*Val-0.07542*Tyr-0.06013*Trp |
| 0.8900 | 6.43571+0.10140*Glu+0.01592*Gly-0.05978*ABA-0.02851*Val-0.07834*Tyr-0.03322*His |
| 0.8876 | 6.43194-0.00039*Urea+0.09163*Glu+0.01283*Gly-0.02286*Val-0.06865*Tyr-0.06077*Trp |
| 0.8874 | 5.53526-0.00043*Urea+0.08365*Glu+0.01571*Gly-0.03198*Leu-0.07555*Tyr-0.03926*His |
| 0.8866 | 6.04074+0.09325*Glu+0.01611*Gly-0.02945*Val-0.07348*Tyr-0.04248*His |
| 0.8858 | 5.86637-0.00033*Urea+0.10192*Glu+0.01431*Gly-0.07309*ABA-0.02700*Val-0.08463*Tyr |
| 0.8439 | 5.68993-0.00054*Urea-0.05791*Asn+0.01244*Gly+0.01178*Phe-0.06145*His |
| 0.8433 | 5.92244-0.00051*Urea-0.05512*Asn+0.01235*Gly-0.05858*His |
| 0.8388 | 5.08865-0.00055*Urea+0.01070*Gly-0.07168*His |

142

## FIG.59

# FIG.60

| ROC AUC | FORMULA |
|---|---|
| 0.9448 | 1.13409+0.00968*Tau-0.01219*Asn+0.01651*Cys-0.01445*His-0.00759*Trp+0.00134*Lys |
| 0.9446 | 1.28519+0.00918*Tau-0.00007*Urea-0.00939*Asn+0.01839*Cys-0.01261*His-0.00582*Trp |
| 0.9440 | 1.19879+0.00935*Tau-0.00008*Urea-0.01258*Asn+0.01656*Cys-0.01516*His+0.00121*Lys |
| 0.9435 | 1.05605+0.00905*Tau-0.01218*Asn+0.01899*Cys-0.00805*Tyr-0.01301*His+0.00144*Lys |
| 0.9434 | 1.22819+0.00905*Tau-0.01419*Asn-0.00250*Val+0.01743*Cys-0.01383*His+0.00187*Lys |
| 0.9432 | 1.14600+0.00929*Tau-0.00008*Urea+0.01650*Cys-0.01526*His-0.00762*Trp+0.00057*Lys |
| 0.9428 | 1.12331+0.00919*Tau-0.01348*Asn+0.01633*Cys-0.00309*Leu-0.01445*His+0.00174*Lys |
| 0.9428 | 1.16132+0.00873*Tau-0.00008*Urea+0.01964*Cys-0.00615*Tyr-0.01214*His-0.00513*Trp |
| 0.9425 | 1.55500+0.01061*Tau-0.00008*Urea-0.01184*Asn-0.01418*His-0.00699*Trp+0.00303*Lys |
| 0.9423 | 1.23465+0.00910*Tau-0.00008*Urea-0.01089*Asn+0.01797*Cys+0.00074*Phe-0.01477*His |
| 0.9422 | 1.23135+0.00866*Tau-0.00008*Urea-0.00916*Asn+0.02051*Cys-0.00648*Tyr-0.01126*His |
| 0.9419 | 1.20704+0.00891*Tau-0.00007*Urea+0.01773*Cys-0.00170*Leu-0.01343*His-0.00676*Trp |
| 0.9416 | 1.06957+0.00864*Tau-0.00008*Urea+0.01915*Cys-0.00866*Tyr-0.01346*His+0.00074*Lys |
| 0.9416 | 1.24613+0.00903*Tau-0.00008*Urea-0.01021*Asn+0.01817*Cys-0.00185*Met-0.01429*His |
| 0.9414 | 1.24520+0.00909*Tau-0.00008*Urea-0.01075*Asn+0.01802*Cys-0.01452*His |
| 0.9414 | 1.07612+0.00852*Tau-0.00008*Urea+0.01999*Cys-0.00865*Tyr+0.00220*Phe-0.01362*His |
| 0.9409 | 1.16831+0.00917*Tau-0.00008*Urea+0.01723*Cys+0.00043*Phe-0.01503*His-0.00725*Trp |
| 0.9409 | 1.11226+0.00852*Tau-0.00008*Urea+0.01992*Cys-0.00797*Tyr-0.01313*His |
| 0.9409 | 1.19887+0.00892*Tau-0.00008*Urea+0.01810*Cys-0.00755*Met-0.01361*His-0.00628*Trp |
| 0.9407 | 1.48308+0.01044*Tau-0.01265*Asn-0.00320*Leu-0.01312*His-0.00759*Trp+0.00364*Lys |
| 0.9404 | 1.12815+0.00846*Tau-0.00008*Urea+0.02003*Cys-0.00385*Met-0.00712*Tyr-0.01279*His |
| 0.9404 | 1.17493+0.00916*Tau-0.00008*Urea+0.01727*Cys-0.01488*His-0.00718*Trp |
| 0.9404 | 1.24029+0.00888*Tau-0.00007*Urea-0.00113*Val+0.01813*Cys-0.01364*His-0.00653*Trp |
| 0.9404 | 1.27255+0.00885*Tau-0.00007*Urea-0.01021*Asn+0.01843*Cys-0.00162*Leu-0.01315*His |
| 0.9398 | 1.15850+0.00841*Tau-0.00008*Urea-0.00074*Val+0.02015*Cys-0.00707*Tyr-0.01267*His |
| 0.9398 | 1.00647+0.00901*Tau+0.01865*Cys-0.00699*Tyr-0.01387*His-0.00654*Trp+0.00066*Lys |
| 0.9397 | 1.32905+0.00876*Tau-0.00007*Urea-0.01051*Asn-0.00136*Val+0.01912*Cys-0.01278*His |
| 0.9394 | 1.57554+0.01044*Tau-0.01323*Asn-0.00198*Val-0.01344*His-0.00700*Trp+0.00354*Lys |
| 0.9392 | 1.18447+0.00936*Tau-0.01027*Asn+0.01810*Cys-0.00013*Met-0.01393*His-0.00677*Trp |
| 0.9391 | 1.18444+0.00937*Tau-0.01030*Asn+0.01809*Cys-0.01394*His-0.00678*Trp |
| 0.9389 | 1.13201+0.00844*Tau-0.00008*Urea+0.01986*Cys-0.00097*Leu-0.00716*Tyr-0.01268*His |
| 0.9389 | 1.18627+0.00936*Tau-0.01028*Asn+0.01810*Cys-0.00012*Phe-0.01391*His-0.00676*Trp |
| 0.9387 | 1.29281+0.00893*Tau-0.01012*Asn-0.00164*Val+0.01939*Cys-0.01198*His-0.00570*Trp |
| 0.9385 | 1.15408+0.00866*Tau-0.00994*Asn+0.02026*Cys-0.00152*Leu-0.00573*Tyr-0.01194*His |
| 0.9382 | 1.61482+0.01019*Tau-0.00007*Urea-0.01347*Asn-0.00165*Val-0.01450*His+0.00327*Lys |
| 0.9382 | 1.16310+0.00899*Tau-0.00937*Asn+0.02003*Cys-0.00529*Tyr-0.01180*His-0.00514*Trp |
| 0.9378 | 1.22502+0.00905*Tau-0.00964*Asn+0.01861*Cys-0.00203*Leu-0.01223*His-0.00627*Trp |
| 0.9378 | 1.11280+0.00890*Tau-0.01150*Asn+0.02028*Cys+0.00517*Met-0.00795*Tyr-0.01291*His |
| 0.9376 | 1.53105+0.01027*Tau-0.00008*Urea-0.01218*Asn-0.00456*Tyr-0.01420*His+0.00303*Lys |
| 0.9374 | 1.09938+0.00680*Tau-0.01037*Asn+0.02039*Cys-0.00736*Tyr+0.00116*Phe-0.01295*His |
| 0.9373 | 1.15542+0.00891*Tau-0.01137*Asn+0.01820*Cys-0.00267*Leu+0.00201*Phe-0.01456*His |
| 0.9373 | 1.11877+0.00880*Tau-0.01020*Asn+0.02035*Cys-0.00701*Tyr-0.01270*His |
| 0.9372 | 1.22454+0.00856*Tau-0.01027*Asn-0.00144*Val+0.02084*Cys-0.00515*Tyr-0.01159*His |
| 0.9371 | 1.53772+0.01017*Tau-0.00008*Urea-0.01303*Asn-0.00267*Leu-0.01435*His+0.00332*Lys |
| 0.9371 | 1.09877+0.00883*Tau-0.00008*Urea+0.01674*Cys-0.01673*Met-0.01542*His+0.00133*Lys |
| 0.9370 | 1.44908+0.01015*Tau-0.01326*Asn-0.00295*Leu-0.00303*Tyr-0.01427*His+0.00334*Lys |
| 0.9363 | 1.18009+0.00892*Tau-0.01108*Asn+0.01826*Cys-0.00231*Leu-0.01416*His |
| 0.9363 | 1.51368+0.01017*Tau-0.01404*Asn-0.00126*Val-0.00185*Leu-0.01501*His+0.00327*Lys |
| 0.9361 | 1.09610+0.00945*Tau-0.01319*Asn+0.01668*Cys-0.01676*His+0.00078*Lys |
| 0.9361 | 1.06842+0.00915*Tau+0.01629*Cys-0.00286*Leu-0.01464*His-0.00833*Trp+0.00101*Lys |

# FIG.61

| ROC AUC | FORMULA |
|---|---|
| 0.9500 | 4.79691+0.13819*Tau-0.13423*Asn+0.17057*Cys-0.08246*Tyr-0.14999*His+0.02202*Lys |
| 0.9493 | 5.24701+0.13723*Tau-0.13121*Asn+0.14143*Cys-0.15628*His-0.06891*Trp+0.01871*Lys |
| 0.9481 | 4.66332+0.12140*Tau-0.00059*Urea+0.17539*Cys-0.05349*Tyr-0.11618*His-0.06045*Trp |
| 0.9481 | 6.71183+0.13997*Tau-0.17092*Asn-0.02340*Val+0.14469*Cys-0.15583*His+0.02650*Lys |
| 0.9480 | 6.67125+0.13071*Tau-0.00061*Urea-0.14862*Asn+0.13571*Cys-0.16920*His+0.01826*Lys |
| 0.9476 | 5.77448+0.12136*Tau-0.00049*Urea-0.08529*Asn+0.18505*Cys-0.05608*Tyr-0.12734*His |
| 0.9476 | 6.07504+0.12476*Tau-0.00054*Urea-0.08326*Asn+0.16379*Cys-0.12797*His-0.06069*Trp |
| 0.9473 | 5.01858+0.12778*Tau-0.00068*Urea+0.12903*Cys-0.14922*His-0.09573*Trp+0.01251*Lys |
| 0.9473 | 9.37036+0.14438*Tau-0.00068*Urea-0.11937*Asn-0.15608*His-0.07870*Trp+0.03330*Lys |
| 0.9471 | 4.83252+0.12308*Tau-0.00070*Urea+0.14384*Cys+0.03106*Phe-0.14581*His-0.08904*Trp |
| 0.9470 | 6.18031+0.13517*Tau-0.16763*Asn+0.13557*Cys-0.03113*Leu-0.15861*His+0.02532*Lys |
| 0.9464 | 3.91581+0.12060*Tau-0.00062*Urea+0.16725*Cys-0.09699*Tyr-0.13807*His+0.01382*Lys |
| 0.9461 | 5.20426+0.12128*Tau-0.00058*Urea-0.00710*Val+0.15357*Cys-0.12470*His-0.07844*Trp |
| 0.9456 | 5.05736+0.12010*Tau-0.00058*Urea+0.15180*Cys-0.01103*Leu-0.12309*His-0.08093*Trp |
| 0.9454 | 2.97462+0.13132*Tau+0.16376*Cys-0.07731*Tyr-0.13962*His-0.06331*Trp+0.01416*Lys |
| 0.9452 | 5.06747+0.12097*Tau-0.00063*Urea+0.14764*Cys-0.13506*His-0.08138*Trp |
| 0.9452 | 5.09228+0.11937*Tau-0.00061*Urea+0.15288*Cys-0.03038*Met-0.13019*His-0.07646*Trp |
| 0.9450 | 4.00214+0.11593*Tau-0.00063*Urea+0.17829*Cys-0.08449*Tyr+0.02872*Phe-0.13740*His |
| 0.9450 | 5.37616+0.12711*Tau-0.09634*Asn-0.00978*Val+0.19261*Cys-0.05041*Tyr-0.12610*His |
| 0.9448 | 6.58176+0.12152*Tau-0.00044*Urea-0.10671*Asn-0.00975*Val+0.16555*Cys-0.13705*His |
| 0.9446 | 6.20491+0.12191*Tau-0.00058*Urea-0.11598*Asn+0.15449*Cys+0.02511*Phe-0.15925*His |
| 0.9446 | 5.55491+0.12809*Tau-0.09680*Asn-0.01124*Val+0.17467*Cys-0.12555*His-0.04860*Trp |
| 0.9446 | 4.69713+0.12758*Tau-0.08514*Asn+0.18645*Cys-0.04578*Tyr-0.12597*His-0.04000*Trp |
| 0.9445 | 4.51382+0.11541*Tau-0.00053*Urea-0.00480*Val+0.17981*Cys-0.07345*Tyr-0.12427*His |
| 0.9442 | 6.33037+0.12022*Tau-0.00052*Urea-0.10901*Asn+0.15721*Cys-0.14986*His |
| 0.9442 | 6.37366+0.12105*Tau-0.00052*Urea-0.11427*Asn+0.15534*Cys+0.01537*Met-0.15217*His |
| 0.9440 | 5.09452+0.12690*Tau-0.09855*Asn+0.16649*Cys-0.13968*His-0.05593*Trp |
| 0.9439 | 5.09673+0.12519*Tau-0.09487*Asn+0.18890*Cys-0.00974*Leu-0.05423*Tyr-0.12958*His |
| 0.9438 | 4.93303+0.12974*Tau-0.11509*Asn+0.18544*Cys+0.07184*Met-0.07238*Tyr-0.14141*His |
| 0.9437 | 8.31402+0.14408*Tau-0.14103*Asn-0.03793*Leu-0.13291*His-0.07246*Trp+0.04102*Lys |
| 0.9437 | 4.42330+0.11434*Tau-0.00055*Urea+0.17826*Cys-0.00485*Leu-0.07518*Tyr-0.12547*His |
| 0.9437 | 10.12562+0.14033*Tau-0.00052*Urea-0.16057*Asn-0.01864*Val-0.15379*His+0.03578*Lys |
| 0.9436 | 6.41117+0.11929*Tau-0.00047*Urea-0.10631*Asn+0.15968*Cys-0.01015*Leu-0.14075*His |
| 0.9436 | 5.00041+0.12731*Tau-0.10055*Asn+0.16556*Cys+0.00783*Phe-0.14208*His-0.05717*Trp |
| 0.9435 | 5.17452+0.12901*Tau-0.10852*Asn+0.16197*Cys+0.03339*Met-0.14389*His-0.05868*Trp |
| 0.9435 | 4.42211+0.11463*Tau-0.00057*Urea+0.17840*Cys-0.00638*Met-0.07619*Tyr-0.12878*His |
| 0.9435 | 4.60799+0.12645*Tau-0.10029*Asn+0.18973*Cys-0.06409*Tyr+0.01656*Phe-0.13966*His |
| 0.9434 | 4.85335+0.12567*Tau-0.09617*Asn+0.18969*Cys-0.06015*Tyr-0.13574*His |
| 0.9434 | 8.96394+0.14368*Tau-0.14333*Asn-0.02080*Val-0.14037*His-0.06168*Trp+0.03907*Lys |
| 0.9434 | 5.28166+0.12652*Tau-0.09322*Asn+0.17051*Cys-0.01482*Leu-0.12586*His-0.05512*Trp |
| 0.9432 | 4.39925+0.11483*Tau-0.00057*Urea+0.17809*Cys-0.07770*Tyr-0.12937*His |
| 0.9429 | 5.86020+0.13200*Tau-0.15014*Asn+0.14145*Cys-0.03828*Phe-0.16609*His+0.01887*Lys |
| 0.9428 | 3.74223+0.12434*Tau-0.00823*Val+0.18117*Cys-0.05534*Tyr-0.12048*His-0.05161*Trp |
| 0.9428 | 3.68229+0.12848*Tau-0.13293*Cys-0.02728*Leu-0.13842*His-0.09194*Trp+0.01521*Lys |
| 0.9427 | 3.37213+0.12395*Tau+0.17816*Cys-0.06184*Tyr-0.12974*His-0.05378*Trp |
| 0.9426 | 9.37457+0.13663*Tau-0.00052*Urea-0.15815*Asn-0.02920*Leu-0.15034*His+0.03558*Lys |
| 0.9426 | 3.58552+0.12326*Tau+0.17857*Cys-0.01221*Leu-0.05385*Tyr-0.11932*His-0.05588*Trp |
| 0.9426 | 9.22437+0.13840*Tau-0.00060*Urea-0.13161*Asn-0.05157*Tyr-0.15622*His+0.03281*Lys |
| 0.9418 | 4.14426+0.12824*Tau-0.01541*Val+0.13739*Cys-0.14488*His-0.08271*Trp+0.01324*Lys |
| 0.9418 | 6.01009+0.12569*Tau-0.11521*Asn-0.01450*Val+0.17141*Cys+0.00213*Leu-0.14266*His |

# FIG.62

# FIG.63

| ROC AUC | FORMULA |
|---|---|
| 0.9416 | 6.18738+0.12805*Tau-0.13159*Asn-0.01519*Val+0.16681*Cys+0.04895*Met-0.14665*His |
| 0.9416 | 5.87710+0.12621*Tau-0.11983*Asn-0.01442*Val+0.16983*Cys+0.01511*Phe-0.14604*His |
| 0.9415 | 5.99898+0.12542*Tau-0.11484*Asn-0.01338*Val+0.17100*Cys-0.14231*His |
| 0.9409 | 8.61816+0.14025*Tau-0.15236*Asn-0.03425*Leu-0.05151*Tyr-0.13972*His+0.04111*Lys |
| 0.9408 | 5.36438+0.13020*Tau-0.15143*Asn+0.14321*Cys-0.17298*His+0.01350*Lys |
| 0.9404 | 4.47099+0.11472*Tau-0.00062*Urea+0.13401*Cys-0.15847*Met-0.15588*His+0.01672*Lys |
| 0.9402 | 9.34665+0.13449*Tau-0.00060*Urea-0.14149*Asn-0.01625*Phe-0.16513*His+0.02774*Lys |
| 0.9399 | 4.83424+0.11153*Tau-0.00054*Urea-0.01074*Val+0.13928*Cys+0.00693*Phe-0.15305*His |
| 0.9397 | 4.89417+0.11118*Tau-0.00053*Urea-0.01005*Val+0.14014*Cys-0.00074*Leu-0.15045*His |
| 0.9396 | 5.65991+0.12273*Tau-0.11465*Asn+0.16376*Cys-0.01508*Leu-0.14713*His |
| 0.9396 | 4.16248+0.12222*Tau-0.01137*Val+0.15514*Cys-0.13561*His-0.07021*Trp |
| 0.9396 | 5.46587+0.12343*Tau-0.12215*Asn+0.16211*Cys-0.01947*Leu+0.02364*Phe-0.15086*His |
| 0.9395 | 4.90268+0.11129*Tau-0.00053*Urea-0.01042*Val+0.14022*Cys-0.15070*His |
| 0.9395 | 9.21453+0.13423*Tau-0.00062*Urea-0.14169*Asn-0.16906*His+0.02592*Lys |
| 0.9395 | 4.44794+0.11027*Tau-0.00065*Urea+0.14713*Cys-0.11560*Met+0.03661*Phe-0.15720*His |
| 0.9394 | 3.88624+0.12108*Tau+0.15331*Cys-0.01794*Leu-0.13185*His-0.07554*Trp |
| 0.9388 | 4.77189+0.11367*Tau-0.00054*Urea-0.01275*Val+0.13088*Cys-0.15635*His+0.00693*Lys |
| 0.9387 | 4.77394+0.10909*Tau-0.00058*Urea+0.14590*Cys-0.08256*Met-0.14821*His |
| 0.9381 | 5.31482+0.12356*Tau-0.12144*Asn+0.16080*Cys-0.15975*His |
| 0.9380 | 7.89829+0.13712*Tau-0.12242*Asn-0.15749*His-0.06983*Trp+0.02801*Lys |
| 0.9380 | 3.16819+0.11912*Tau+0.18164*Cys-0.08457*Tyr-0.14121*His |
| 0.9376 | 9.70560+0.14014*Tau-0.16208*Asn-0.02192*Val-0.03354*Phe-0.14718*His+0.03843*Lys |
| 0.9375 | 3.68038+0.12219*Tau+0.14622*Cys-0.15078*His-0.07845*Trp |
| 0.9373 | 4.47726+0.11116*Tau-0.00062*Urea+0.12653*Cys-0.16817*His+0.00235*Lys |
| 0.9371 | 3.81658+0.12177*Tau+0.14766*Cys-0.00934*Phe-0.14746*His-0.07630*Trp |
| 0.9371 | 9.08988+0.13761*Tau-0.16538*Asn-0.01376*Val-0.01916*Leu-0.14906*His+0.03634*Lys |
| 0.9363 | 4.63724+0.10902*Tau-0.00055*Urea+0.13523*Cys-0.01292*Leu-0.15189*His |
| 0.9362 | 9.31502+0.13845*Tau-0.16356*Asn-0.02261*Val-0.15310*His+0.03455*Lys |
| 0.9358 | 4.55260+0.11037*Tau-0.00061*Urea+0.13041*Cys-0.16514*His |
| 0.9358 | 4.60087+0.11025*Tau-0.00060*Urea+0.13108*Cys-0.00405*Phe-0.16349*His |
| 0.8825 | 8.40451-0.00044*Urea-0.05715*Asn+0.17320*Cys-0.01158*Leu-0.05364*Tyr-0.07100*His |
| 0.8825 | 8.40451-0.00044*Urea-0.05715*Asn+0.17320*Cys-0.01158*Leu-0.05364*Tyr-0.07100*His |
| 0.8825 | 7.93912-0.00051*Urea-0.05902*Asn+0.17561*Cys-0.06752*Tyr+0.02212*Phe-0.08334*His |
| 0.8798 | 7.44046-0.00049*Urea+0.17263*Cys-0.06702*Met-0.05876*Tyr-0.07855*His |
| 0.8777 | 7.78426-0.06804*Asn-0.01235*Val+0.17514*Cys-0.05326*Tyr+0.01655*Phe-0.07626*His |
| 0.8776 | 8.09661-0.08303*Asn-0.01492*Val+0.16812*Cys-0.05447*Tyr-0.07722*His+0.01192*Lys |
| 0.8770 | 7.44567-0.00051*Urea+0.16937*Cys-0.06677*Tyr-0.08341*His-0.01333*Trp |
| 0.8766 | 7.26995-0.00051*Urea+0.17041*Cys-0.07204*Tyr-0.08582*His |
| 0.8762 | 7.99024-0.06527*Asn-0.01144*Val+0.17508*Cys-0.05033*Tyr-0.07320*His |
| 0.8752 | 9.07066-0.00040*Urea-0.07066*Asn-0.01335*Val+0.15605*Cys-0.08832*His |
| 0.8400 | 11.47175-0.00046*Urea-0.06930*Asn-0.02236*Leu-0.02605*Tyr-0.08471*His+0.02121*Lys |
| 0.8388 | 12.17470-0.00045*Urea-0.06866*Asn-0.01433*Val-0.09296*His-0.02810*Trp+0.02235*Lys |
| 0.8373 | 11.67164-0.00044*Urea-0.07553*Asn-0.00545*Val-0.01865*Leu-0.09250*His+0.02004*Lys |
| 0.8372 | 11.35281-0.00045*Urea-0.06417*Asn-0.04112*Met-0.02307*Leu-0.08992*His+0.02044*Lys |
| 0.8370 | 11.70135-0.00044*Urea-0.05998*Asn-0.01343*Val-0.05645*Met-0.09252*His+0.02180*Lys |
| 0.8360 | 11.33658-0.00047*Urea-0.07611*Asn-0.02733*Leu+0.01801*Phe-0.09685*His+0.01809*Lys |
| 0.8358 | 11.84881-0.00045*Urea-0.06798*Asn-0.01254*Val-0.02522*Tyr-0.09028*His+0.02151*Lys |
| 0.8288 | 10.73800-0.00046*Urea-0.01030*Val-0.10796*Met-0.09530*His+0.01810*Lys |
| 0.8249 | 10.35841-0.00047*Urea-0.02102*Leu-0.10306*His+0.00996*Lys |
| 0.8239 | 11.81571-0.00041*Urea-0.04011*Asn-0.00818*Val-0.08743*His |

EP 2 124 059 A1

# FIG.64

| ROC AUC | FORMULA |
|---|---|
| 0.9416 | 6.18738+0.12805*Tau-0.13159*Asn-0.01519*Val+0.16681*Cys+0.04895*Met-0.14665*His |
| 0.9416 | 5.87710+0.12621*Tau-0.11983*Asn-0.01442*Val+0.16983*Cys+0.01511*Phe-0.14604*His |
| 0.9415 | 5.99898+0.12542*Tau-0.11484*Asn-0.01338*Val+0.17100*Cys-0.14231*His |
| 0.9409 | 8.61816+0.14025*Tau-0.15236*Asn-0.03425*Leu-0.05151*Tyr-0.13972*His+0.04111*Lys |
| 0.9408 | 5.36438+0.13020*Tau-0.15143*Asn+0.14321*Cys-0.17298*His+0.01350*Lys |
| 0.9404 | 4.47099+0.11472*Tau-0.00062*Urea+0.13401*Cys-0.15847*Met-0.15588*His+0.01672*Lys |
| 0.9402 | 9.34665+0.13449*Tau-0.00060*Urea-0.14149*Asn-0.01625*Phe-0.16513*His+0.02774*Lys |
| 0.9399 | 4.83424+0.11153*Tau-0.00054*Urea-0.01074*Val+0.13928*Cys+0.00693*Phe-0.15305*His |
| 0.9397 | 4.89417+0.11118*Tau-0.00053*Urea-0.01005*Val+0.14014*Cys-0.00074*Leu-0.15045*His |
| 0.9396 | 5.65991+0.12273*Tau-0.11465*Asn+0.16376*Cys-0.01508*Leu-0.14713*His |
| 0.9396 | 4.16248+0.12222*Tau-0.01137*Val+0.15514*Cys-0.13561*His-0.07021*Trp |
| 0.9396 | 5.46587+0.12343*Tau-0.12215*Asn+0.16211*Cys-0.01947*Leu+0.02364*Phe-0.15086*His |
| 0.9395 | 4.90268+0.11129*Tau-0.00053*Urea-0.01042*Val+0.14022*Cys-0.15070*His |
| 0.9395 | 9.21453+0.13423*Tau-0.00062*Urea-0.14169*Asn-0.16906*His+0.02592*Lys |
| 0.9395 | 4.44794+0.11027*Tau-0.00065*Urea+0.14713*Cys-0.11560*Met+0.03661*Phe-0.15720*His |
| 0.9394 | 3.88624+0.12108*Tau+0.15331*Cys-0.01794*Leu-0.13185*His-0.07554*Trp |
| 0.9388 | 4.77189+0.11367*Tau-0.00054*Urea-0.01275*Val+0.13088*Cys-0.15635*His+0.00693*Lys |
| 0.9387 | 4.77394+0.10909*Tau-0.00058*Urea+0.14590*Cys-0.08256*Met-0.14821*His |
| 0.9381 | 5.31482+0.12356*Tau-0.12144*Asn+0.16080*Cys-0.15975*His |
| 0.9380 | 7.89829+0.13712*Tau-0.12242*Asn-0.15749*His-0.06983*Trp+0.02801*Lys |
| 0.9380 | 3.16819+0.11912*Tau+0.18164*Cys-0.08457*Tyr-0.14121*His |
| 0.9376 | 9.70560+0.14014*Tau-0.16208*Asn-0.02192*Val-0.03354*Phe-0.14718*His+0.03843*Lys |
| 0.9375 | 3.68038+0.12219*Tau+0.14622*Cys-0.15078*His-0.07845*Trp |
| 0.9373 | 4.47726+0.11116*Tau-0.00062*Urea+0.12653*Cys-0.16817*His+0.00235*Lys |
| 0.9371 | 3.81658+0.12177*Tau+0.14766*Cys-0.00934*Phe-0.14746*His-0.07630*Trp |
| 0.9371 | 9.08988+0.13761*Tau-0.16538*Asn-0.01376*Val-0.01916*Leu-0.14906*His+0.03634*Lys |
| 0.9363 | 4.63724+0.10902*Tau-0.00055*Urea+0.13523*Cys-0.01292*Leu-0.15189*His |
| 0.9362 | 9.31502+0.13845*Tau-0.16356*Asn-0.02261*Val-0.15310*His+0.03455*Lys |
| 0.9358 | 4.55260+0.11037*Tau-0.00061*Urea+0.13041*Cys-0.16514*His |
| 0.9358 | 4.60087+0.11025*Tau-0.00060*Urea+0.13108*Cys-0.00405*Phe-0.16349*His |
| 0.8825 | 8.40451-0.00044*Urea-0.05715*Asn+0.17320*Cys-0.01158*Leu-0.05364*Tyr-0.07100*His |
| 0.8825 | 8.40451-0.00044*Urea-0.05715*Asn+0.17320*Cys-0.01158*Leu-0.05364*Tyr-0.07100*His |
| 0.8825 | 7.93912-0.00051*Urea-0.05902*Asn+0.17561*Cys-0.06752*Tyr+0.02212*Phe-0.08334*His |
| 0.8798 | 7.44046-0.00049*Urea+0.17263*Cys-0.06702*Met-0.05876*Tyr-0.07855*His |
| 0.8777 | 7.78426-0.06804*Asn-0.01235*Val+0.17514*Cys-0.05326*Tyr+0.01655*Phe-0.07626*His |
| 0.8776 | 8.09661-0.08303*Asn-0.01492*Val+0.16812*Cys-0.05447*Tyr-0.07722*His+0.01192*Lys |
| 0.8770 | 7.44567-0.00051*Urea+0.16937*Cys-0.06677*Tyr-0.08341*His-0.01333*Trp |
| 0.8766 | 7.26995-0.00051*Urea+0.17041*Cys-0.07204*Tyr-0.08582*His |
| 0.8762 | 7.99024-0.06527*Asn-0.01144*Val+0.17508*Cys-0.05033*Tyr-0.07320*His |
| 0.8752 | 9.07066-0.00040*Urea-0.07066*Asn-0.01335*Val+0.15605*Cys-0.08832*His |
| 0.8400 | 11.47175-0.00046*Urea-0.06930*Asn-0.02236*Leu-0.02605*Tyr-0.08471*His+0.02121*Lys |
| 0.8386 | 12.17470-0.00045*Urea-0.06866*Asn-0.01433*Val-0.09296*His-0.02810*Trp+0.02235*Lys |
| 0.8373 | 11.67164-0.00044*Urea-0.07553*Asn-0.00545*Val-0.01865*Leu-0.09250*His+0.02004*Lys |
| 0.8372 | 11.35281-0.00045*Urea-0.06417*Asn-0.04112*Met-0.02307*Leu-0.08992*His+0.02044*Lys |
| 0.8370 | 11.70135-0.00044*Urea-0.05998*Asn-0.01343*Val-0.05645*Met-0.09252*His+0.02180*Lys |
| 0.8360 | 11.33658-0.00047*Urea-0.07811*Asn-0.02733*Leu+0.01801*Phe-0.09685*His+0.01809*Lys |
| 0.8358 | 11.84881-0.00045*Urea-0.06798*Asn-0.01254*Val-0.02522*Tyr-0.09028*His+0.02151*Lys |
| 0.8288 | 10.73800-0.00046*Urea-0.01030*Val-0.10796*Met-0.09530*His+0.01810*Lys |
| 0.8249 | 10.35841-0.00047*Urea-0.02102*Leu-0.10306*His+0.00996*Lys |
| 0.8239 | 11.81571-0.00041*Urea-0.04011*Asn-0.00818*Val-0.08743*His |

148

# FIG.65

# FIG.66

| ROC AUC | FORMULA |
|---|---|
| 0.7345 | -0.01486+0.00202*Tau-0.00062*Thr+0.00149*Pro+0.00135*Gly-0.00704*Trp+0.00366*Orn |
| 0.7322 | 0.00537+0.00202*Tau+0.00166*Pro+0.00137*Gly-0.00036*Ala-0.00676*Trp+0.00335*Orn |
| 0.7313 | 0.04405+0.00201*Tau+0.00166*Pro+0.00116*Gly-0.00044*Ala+0.01182*Met-0.00817*Trp |
| 0.7311 | -0.07513+0.00204*Tau+0.00133*Pro+0.00088*Gly+0.01026*Met-0.00907*Trp+0.00301*Orn |
| 0.7310 | -0.01820+0.00200*Tau+0.00148*Pro+0.00125*Gly-0.00740*Trp+0.00323*Orn |
| 0.7303 | -0.07748+0.00199*Tau+0.00149*Pro+0.00122*Gly+0.00213*Phe-0.00814*Trp+0.00294*Orn |
| 0.7298 | 0.02554+0.00200*Tau-0.00073*Thr+0.00146*Pro+0.00113*Gly+0.01263*Met-0.00861*Trp |
| 0.7295 | 0.04512+0.00216*Tau-0.00136*Thr+0.00156*Pro+0.01728*Met-0.00990*Trp+0.00445*Orn |
| 0.7293 | -0.06592+0.00220*Tau-0.00198*Thr+0.00132*Gly+0.01630*Met-0.00784*Trp+0.00474*Orn |
| 0.7284 | -0.03956+0.00200*Tau+0.00148*Pro+0.00122*Gly-0.00767*Trp+0.00313*Orn+0.00025*Lys |
| 0.7272 | -0.06349+0.00203*Tau+0.00142*Pro+0.00123*Gly+0.00124*Ile-0.00753*Trp+0.00305*Orn |
| 0.7265 | 0.10573+0.00195*Tau+0.00176*Pro+0.00154*Gly-0.00029*Ala-0.00649*Trp |
| 0.7263 | -0.00056+0.00195*Tau+0.00177*Pro+0.00149*Gly-0.00032*Ala+0.00338*Phe-0.00767*Trp |
| 0.7259 | -0.11115+0.00176*Tau+0.00405*Glu+0.00135*Pro+0.00143*Gly-0.00857*Trp+0.00265*Orn |
| 0.7259 | 0.08030+0.00206*Tau+0.00159*Pro+0.01546*Met-0.00164*Ile-0.01015*Trp+0.00366*Orn |
| 0.7258 | 0.01916+0.00201*Tau+0.00167*Pro+0.00152*Gly-0.00035*Ala+0.00221*Ile-0.00665*Trp |
| 0.7257 | 0.02336+0.00199*Tau+0.00145*Pro+0.00103*Gly+0.01124*Met-0.00029*Phe-0.00877*Trp |
| 0.7257 | 0.03872+0.00199*Tau+0.00145*Pro+0.00104*Gly+0.01167*Met-0.00864*Trp-0.00028*Lys |
| 0.7254 | 0.02192+0.00198*Tau+0.00146*Pro+0.00103*Gly+0.01111*Met-0.00017*Ile-0.00883*Trp |
| 0.7249 | 0.01624+0.00198*Tau+0.00145*Pro+0.00104*Gly+0.01091*Met-0.00882*Trp |
| 0.7239 | 0.08675+0.00210*Tau+0.00153*Pro+0.01559*Met-0.00963*Trp+0.00373*Orn-0.00070*Lys |
| 0.7239 | -0.02089+0.00194*Tau-0.00007*Thr+0.00161*Pro+0.00139*Gly+0.00333*Phe-0.00818*Trp |
| 0.7239 | -0.02089+0.00194*Tau-0.00007*Thr+0.00161*Pro+0.00139*Gly+0.00333*Phe-0.00818*Trp |
| 0.7239 | 0.09722+0.00194*Tau+0.00040*Thr+0.00176*Pro+0.00146*Gly-0.00032*Ala-0.00670*Trp |
| 0.7238 | -0.02095+0.00194*Tau+0.00161*Pro+0.00138*Gly+0.00327*Phe-0.00820*Trp |
| 0.7238 | -0.03400+0.00194*Tau+0.00160*Pro+0.00136*Gly+0.00298*Phe-0.00835*Trp+0.00023*Lys |
| 0.7238 | -0.03400+0.00194*Tau+0.00160*Pro+0.00136*Gly+0.00298*Phe-0.00835*Trp+0.00023*Lys |
| 0.7237 | -0.19878+0.00172*Tau-0.00087*Thr+0.00157*Pro+0.00167*Gly-0.00053*Ala+0.00378*Orn |
| 0.7234 | 0.03785+0.00210*Tau+0.00155*Pro+0.01373*Met-0.01006*Trp+0.00349*Orn |
| 0.7234 | -0.01708-0.00167*Thr+0.00139*Pro+0.00111*Gly+0.01350*Met-0.00669*Trp+0.00389*Orn |
| 0.7232 | 0.06568+0.00212*Tau+0.00171*Pro-0.00031*Ala+0.01469*Met-0.00971*Trp+0.00363*Orn |
| 0.7230 | 0.04323+0.00195*Tau+0.00175*Pro+0.00147*Gly-0.00031*Ala-0.00717*Trp+0.00065*Lys |
| 0.7230 | 0.00104+0.00198*Tau+0.00011*Thr+0.00150*Pro+0.00138*Gly+0.00194*Ile-0.00732*Trp |
| 0.7229 | 0.00236+0.00199*Tau+0.00151*Pro+0.00140*Gly+0.00198*Ile-0.00725*Trp |
| 0.7228 | 0.09287+0.00211*Tau+0.00147*Pro+0.01685*Met-0.00310*Phe-0.00945*Trp+0.00379*Orn |
| 0.7228 | -0.03820+0.00196*Tau+0.00156*Pro+0.00137*Gly+0.00091*Ile+0.00264*Phe-0.00808*Trp |
| 0.7227 | -0.01338+0.00168*Tau+0.00481*Glu+0.00166*Pro+0.00178*Gly-0.00046*Ala-0.00765*Trp |
| 0.7221 | -0.03990+0.00168*Tau+0.00451*Glu+0.00144*Pro+0.00160*Gly-0.00839*Trp |
| 0.7219 | -0.03861+0.00168*Tau-0.00006*Thr+0.00452*Glu+0.00144*Pro+0.00162*Gly-0.00835*Trp |
| 0.7219 | -0.07452+0.00169*Tau+0.00443*Glu+0.00143*Pro+0.00156*Gly-0.00877*Trp+0.00038*Lys |
| 0.7218 | -0.08821+0.00169*Tau+0.00424*Glu+0.00145*Pro+0.00156*Gly+0.00173*Phe-0.00894*Trp |
| 0.7214 | 0.02445+0.00194*Tau+0.00006*Thr+0.00160*Pro+0.00135*Gly-0.00770*Trp+0.00059*Lys |
| 0.7212 | -0.06357+0.00170*Tau+0.00436*Glu+0.00141*Pro+0.00159*Gly+0.00067*Ile-0.00842*Trp |
| 0.7211 | -0.10425+0.00173*Tau+0.00444*Glu+0.00128*Pro+0.00121*Gly+0.01067*Met-0.01013*Trp |
| 0.7210 | 0.02443+0.00194*Tau+0.00160*Pro+0.00137*Gly-0.00768*Trp+0.00061*Lys |
| 0.7210 | -0.35698+0.00167*Tau-0.00220*Thr+0.00112*Pro+0.00136*Gly+0.01078*Met+0.00423*Orn |
| 0.7209 | -0.01296+0.00198*Tau+0.00151*Pro+0.00137*Gly+0.00166*Ile-0.00753*Trp+0.00029*Lys |
| 0.7206 | 0.19965+0.00207*Tau+0.00191*Pro-0.00001*Ala-0.00808*Trp+0.00413*Orn |
| 0.7194 | -0.17847+0.00169*Tau+0.00158*Pro+0.00156*Gly-0.00059*Ala+0.00330*Orn-0.00030*Lys |
| 0.7193 | -0.08660+0.00182*Tau-0.00075*Thr+0.00462*Glu+0.00191*Gly-0.00719*Trp+0.00358*Orn |

# FIG.67

| ROC AUC | FORMULA |
|---|---|
| 0.7188 | -0.28404+0.00165*Tau+0.00151*Pro+0.00134*Gly-0.00074*Ala+0.00765*Met+0.00303*Orn |
| 0.7181 | -0.31716+0.00141*Tau+0.00368*Glu+0.00151*Pro+0.00178*Gly-0.00076*Ala+0.00267*Orn |
| 0.7178 | -0.06313-0.00107*Thr+0.00532*Glu+0.00132*Pro+0.00143*Gly+0.01273*Met-0.00848*Trp |
| 0.7178 | 0.18906+0.00205*Tau+0.00047*Thr+0.00191*Pro-0.00006*Ala-0.00824*Trp+0.00378*Orn |
| 0.7177 | -0.15503+0.00185*Tau+0.00442*Glu+0.00131*Gly+0.01163*Met-0.00961*Trp+0.00278*Orn |
| 0.7173 | 0.18306+0.00205*Tau+0.00043*Thr+0.00188*Pro-0.00831*Trp+0.00377*Orn |
| 0.7173 | 0.14642+0.00210*Tau+0.00186*Pro-0.00004*Ala+0.00151*Ile-0.00817*Trp+0.00392*Orn |
| 0.7167 | 0.14199+0.00210*Tau+0.00183*Pro+0.00148*Ile-0.00824*Trp+0.00390*Orn |
| 0.7166 | -0.25976+0.00170*Tau+0.00153*Pro+0.00155*Gly-0.00064*Ala+0.00126*Ile+0.00300*Orn |
| 0.7162 | -0.12145+0.00184*Tau+0.00430*Glu+0.00174*Gly+0.00101*Ile-0.00769*Trp+0.00293*Orn |
| 0.7159 | -0.21322+0.00167*Tau+0.00158*Pro+0.00155*Gly-0.00061*Ala+0.00318*Orn |
| 0.7155 | -0.01911+0.00190*Tau+0.00338*Glu+0.00148*Pro+0.01428*Met-0.01120*Trp+0.00310*Orn |
| 0.7155 | 0.12189+0.00206*Tau+0.00191*Pro+0.00259*Phe-0.00898*Trp+0.00376*Orn |
| 0.7155 | 0.12189+0.00206*Tau+0.00191*Pro+0.00259*Phe-0.00898*Trp+0.00376*Orn |
| 0.7153 | -0.07516+0.00178*Tau-0.00112*Thr+0.00520*Glu+0.00165*Gly+0.01495*Met-0.00923*Trp |
| 0.7151 | 0.12505+0.00206*Tau+0.00192*Pro-0.00003*Ala+0.00260*Phe-0.00894*Trp+0.00377*Orn |
| 0.7150 | 0.19198+0.00205*Tau+0.00185*Pro-0.00021*Ala+0.01590*Met-0.00972*Trp |
| 0.7149 | -0.36739+0.00141*Tau-0.00137*Thr+0.00335*Glu+0.00115*Pro+0.00175*Gly+0.00341*Orn |
| 0.7149 | 0.13383+0.00206*Tau+0.00188*Pro-0.00874*Trp+0.00383*Orn+0.00064*Lys |
| 0.7148 | -0.00613+0.00550*Glu+0.00125*Pro+0.00122*Gly+0.01371*Met-0.00306*Phe-0.00833*Trp |
| 0.7148 | 0.08312-0.00061*Thr+0.00152*Pro+0.00117*Gly+0.01181*Met+0.00004*Phe-0.00677*Trp |
| 0.7146 | -0.08847+0.00180*Tau+0.00451*Glu+0.00177*Gly-0.00761*Trp+0.00306*Orn |
| 0.7146 | 0.11790+0.00205*Tau+0.00024*Thr+0.00189*Pro+0.00243*Phe-0.00905*Trp+0.00358*Orn |
| 0.7143 | -0.23871+0.00166*Tau-0.00109*Thr+0.00129*Pro+0.00155*Gly+0.00380*Orn-0.00019*Lys |
| 0.7141 | -0.26070+0.00165*Tau-0.00115*Thr+0.00129*Pro+0.00155*Gly+0.00377*Orn |
| 0.7140 | -0.30411+0.00168*Tau-0.00122*Thr+0.00123*Pro+0.00155*Gly+0.00112*Ile+0.00365*Orn |
| 0.7140 | -0.27905+0.00165*Tau-0.00121*Thr+0.00129*Pro+0.00155*Gly+0.00049*Phe+0.00373*Orn |
| 0.7137 | -0.10307+0.00181*Tau+0.00443*Glu+0.00176*Gly+0.00059*Phe-0.00779*Trp+0.00300*Orn |
| 0.7134 | 0.17003+0.00204*Tau+0.00173*Pro+0.01523*Met-0.00996*Trp |
| 0.7130 | -0.12452+0.00471*Glu+0.00125*Pro+0.00116*Gly+0.00975*Met-0.00874*Trp+0.00217*Orn |
| 0.7129 | -0.00144+0.00175*Tau+0.00547*Glu+0.00141*Gly+0.01668*Met-0.00391*Phe-0.00900*Trp |
| 0.7124 | -0.08224+0.00177*Tau+0.00493*Glu+0.00149*Gly+0.01232*Met-0.00947*Trp |
| 0.7118 | 0.20148+0.00204*Tau+0.00170*Pro+0.01677*Met-0.00147*Phe-0.00967*Trp |
| 0.7117 | -0.07017+0.00506*Glu+0.00132*Pro+0.00128*Gly+0.01022*Met-0.00872*Trp |
| 0.7115 | 0.19405+0.00202*Tau+0.00176*Pro+0.01611*Met-0.00081*Ile-0.01000*Trp |
| 0.7109 | -0.00766+0.00141*Pro+0.00094*Gly+0.00980*Met-0.00713*Trp+0.00281*Orn |
| 0.7096 | 0.07583+0.00152*Pro+0.00109*Gly+0.01042*Met-0.00695*Trp |
| 0.7091 | -0.00332+0.00171*Tau+0.00508*Glu+0.00200*Gly-0.00733*Trp |
| 0.7088 | 0.08874+0.00183*Tau+0.00382*Glu+0.00163*Pro+0.01565*Met-0.01126*Trp |
| 0.7086 | -0.31598+0.00177*Tau-0.00225*Thr+0.00156*Gly+0.01265*Met+0.00465*Orn |
| 0.7078 | -0.00828-0.00006*Thr+0.00512*Glu+0.00147*Pro+0.00166*Gly-0.00705*Trp |
| 0.7077 | -0.00945+0.00511*Glu+0.00147*Pro+0.00165*Gly-0.00709*Trp |
| 0.7070 | 0.15240+0.00189*Tau+0.00307*Glu+0.00186*Pro-0.00806*Trp+0.00379*Orn |
| 0.7061 | -0.01592+0.00155*Pro+0.00127*Gly+0.00223*Phe-0.00634*Trp+0.00272*Orn-0.00001*Lys |
| 0.7052 | -0.03580+0.00165*Tau+0.00186*Pro-0.00025*Ala+0.00013*Phe+0.00406*Orn-0.00006*Lys |
| 0.7050 | 0.08198+0.00166*Pro+0.00140*Gly-0.00590*Trp+0.00058*Lys |
| 0.7023 | -0.00920+0.00208*Tau+0.00155*Gly+0.00004*Ala-0.00659*Trp+0.00362*Orn+0.00029*Lys |
| 0.7020 | -0.29224+0.00158*Tau+0.00125*Pro+0.00135*Gly+0.00293*Orn |
| 0.6956 | 0.19933+0.00201*Tau+0.00203*Pro+0.00007*Ala+0.00107*Ile+0.00339*Phe-0.00915*Trp |
| 0.6941 | -0.22121+0.00153*Tau+0.00137*Pro+0.00151*Gly+0.00077*Phe |

# FIG.68

# FIG.69

| ROC AUC | FORMULA |
|---|---|
| 0.7329 | -2.46344+0.01083*Tau-0.00327*Thr+0.00661*Pro+0.00657*Gly-0.03267*Trp+0.01720*Orn |
| 0.7315 | -2.38090+0.01082*Tau-0.00744*Pro+0.00656*Gly-0.00167*Ala-0.03120*Trp+0.01509*Orn |
| 0.7307 | -2.53522+0.01073*Tau+0.00653*Pro+0.00696*Gly-0.03491*Trp+0.01414*Orn+0.00069*Lys |
| 0.7306 | -2.48234+0.01076*Tau+0.00653*Pro+0.00602*Gly-0.03419*Trp+0.01451*Orn |
| 0.7302 | -2.36954+0.01121*Tau+0.00789*Pro+0.00579*Gly-0.00233*Ala+0.05961*Met-0.03805*Trp |
| 0.7296 | -2.94846+0.01245*Tau-0.00982*Thr+0.00636*Gly+0.08338*Met-0.03833*Trp+0.02390*Orn |
| 0.7290 | -2.32500+0.01146*Tau-0.00701*Thr+0.00681*Pro+0.08678*Met-0.04621*Trp+0.02297*Orn |
| 0.7283 | -2.85579+0.01120*Tau+0.00583*Pro+0.00439*Gly+0.05203*Met-0.04250*Trp+0.01359*Orn |
| 0.7283 | -2.80325+0.01068*Tau+0.00656*Pro+0.00605*Gly+0.01053*Phe-0.03774*Trp+0.01298*Orn |
| 0.7282 | -2.43105+0.01119*Tau-0.00378*Thr+0.00664*Pro+0.00573*Gly+0.06276*Met-0.04067*Trp |
| 0.7272 | -2.67825+0.01078*Tau+0.00627*Pro+0.00604*Gly+0.00535*Ile-0.03467*Trp+0.01351*Orn |
| 0.7271 | -2.91317+0.00921*Tau+0.01806*Glu+0.00578*Pro+0.00726*Gly-0.03946*Trp+0.01147*Orn |
| 0.7270 | -1.95455+0.01059*Tau+0.00816*Pro+0.00741*Gly-0.00138*Ala-0.03045*Trp |
| 0.7258 | -2.34649+0.01068*Tau+0.00772*Pro+0.00744*Gly-0.00165*Ala+0.00961*Ile-0.03085*Trp |
| 0.7257 | -2.49837+0.01047*Tau+0.00820*Pro+0.00739*Gly-0.00155*Ala+0.01581*Phe-0.03558*Trp |
| 0.7254 | -2.46542+0.01110*Tau+0.00656*Pro+0.00517*Gly+0.05337*Met-0.04141*Trp |
| 0.7252 | -2.10030+0.01126*Tau+0.00706*Pro+0.07870*Met-0.00966*Ile-0.04737*Trp+0.01801*Orn |
| 0.7252 | -2.32406+0.01123*Tau+0.00652*Pro+0.00522*Gly+0.05953*Met-0.04023*Trp-0.00201*Lys |
| 0.7251 | -2.43037+0.01113*Tau+0.00654*Pro+0.00512*Gly+0.05502*Met-0.00138*Phe-0.04118*Trp |
| 0.7249 | -2.40767+0.01112*Tau+0.00664*Pro+0.00510*Gly+0.05569*Met-0.00179*Ile-0.04160*Trp |
| 0.7248 | -1.98838+0.01060*Tau+0.00145*Thr+0.00815*Pro+0.00712*Gly-0.00151*Ala-0.03101*Trp |
| 0.7244 | -2.58099+0.01046*Tau+0.00727*Pro+0.00686*Gly+0.01515*Phe-0.03820*Trp |
| 0.7244 | -2.18560+0.01053*Tau+0.00815*Pro+0.00713*Gly-0.00148*Ala-0.03293*Trp+0.00252*Lys |
| 0.7241 | -2.60498+0.01045*Tau+0.00726*Pro+0.00680*Gly+0.01453*Phe-0.03851*Trp+0.00048*Lys |
| 0.7241 | -2.60498+0.01045*Tau+0.00726*Pro+0.00680*Gly+0.01453*Phe-0.03851*Trp+0.00048*Lys |
| 0.7240 | -2.41368+0.01062*Tau+0.00014*Thr+0.00681*Pro+0.00685*Gly+0.00844*Ile-0.03384*Trp |
| 0.7239 | -2.18044+0.01121*Tau+0.00785*Pro-0.00166*Ala+0.07317*Met-0.04459*Trp+0.01735*Orn |
| 0.7239 | -2.57946+0.01045*Tau-0.00076*Thr+0.00732*Pro+0.00703*Gly+0.01574*Phe-0.03799*Trp |
| 0.7239 | -2.57946+0.01045*Tau-0.00076*Thr+0.00732*Pro+0.00703*Gly+0.01574*Phe-0.03799*Trp |
| 0.7238 | -2.97509+0.00944*Tau+0.01946*Glu+0.00554*Pro+0.00626*Gly+0.05169*Met-0.04725*Trp |
| 0.7235 | -2.41171+0.01062*Tau+0.00682*Pro+0.00688*Gly+0.00849*Ile-0.03376*Trp |
| 0.7234 | -2.45219+0.01059*Tau+0.00685*Pro+0.00678*Gly+0.00754*Ile-0.03458*Trp+0.00086*Lys |
| 0.7234 | -2.64577+0.01050*Tau+0.00707*Pro+0.00685*Gly+0.00348*Ile+0.01278*Phe-0.03772*Trp |
| 0.7232 | -2.29907+0.01115*Tau+0.00684*Pro+0.06739*Met-0.04649*Trp+0.01661*Orn |
| 0.7232 | -2.48844+0.00885*Tau+0.02117*Glu+0.00745*Pro+0.00888*Gly-0.00214*Ala-0.03574*Trp |
| 0.7231 | -2.58747+0.00891*Tau-0.00070*Thr+0.01997*Glu+0.00632*Pro+0.00824*Gly-0.03883*Trp |
| 0.7230 | -2.71979+0.00893*Tau+0.01955*Glu+0.00625*Pro+0.00788*Gly-0.04050*Trp+0.00132*Lys |
| 0.7228 | -2.71906+0.00902*Tau+0.01917*Glu+0.00613*Pro+0.00800*Gly+0.00314*Ile-0.03926*Trp |
| 0.7227 | -2.60404+0.00892*Tau+0.01981*Glu+0.00628*Pro+0.00807*Gly-0.03914*Trp |
| 0.7225 | -2.07102+0.01147*Tau+0.00656*Pro+0.08073*Met-0.04419*Trp+0.01891*Orn-0.00429*Lys |
| 0.7225 | -2.88719+0.00901*Tau+0.01858*Glu+0.00630*Pro+0.00794*Gly+0.00901*Phe-0.04185*Trp |
| 0.7224 | -3.25614+0.00894*Tau-0.00402*Thr+0.00693*Pro+0.00777*Gly-0.00246*Ala+0.01748*Orn |
| 0.7223 | -2.49220-0.00825*Thr+0.00619*Pro+0.00528*Gly+0.07123*Met-0.03174*Trp+0.01814*Orn |
| 0.7220 | -2.05230+0.01146*Tau+0.00642*Pro+0.08357*Met-0.01520*Phe-0.04370*Trp+0.01803*Orn |
| 0.7218 | -2.26639+0.01050*Tau-0.00000*Thr+0.00727*Pro+0.00666*Gly-0.03537*Trp+0.00228*Lys |
| 0.7217 | -2.26640+0.01050*Tau+0.00727*Pro+0.00666*Gly-0.03537*Trp+0.00228*Lys |
| 0.7206 | -2.88379+0.00991*Tau-0.00339*Thr+0.02035*Glu+0.00948*Gly-0.03352*Trp+0.01602*Orn |
| 0.7203 | -3.28849+0.01020*Tau+0.01965*Glu+0.00671*Gly+0.05743*Met-0.04515*Trp+0.01262*Orn |
| 0.7201 | -4.05944+0.00876*Tau-0.01018*Thr+0.00473*Pro+0.00640*Gly+0.05241*Met-0.01990*Orn |
| 0.7200 | -1.48235+0.01050*Tau+0.00148*Thr+0.00863*Pro-0.00034*Ala-0.03674*Trp+0.01852*Orn |

# FIG.70

| ROC AUC | FORMULA |
|---|---|
| 0.7199 | -1.45708+0.01054*Tau+0.00863*Pro-0.00019*Ala-0.03637*Trp+0.02000*Orn |
| 0.7199 | -3.13013+0.00895*Tau+0.00700*Pro+0.00733*Gly-0.00268*Ala+0.01526*Orn-0.00174*Lys |
| 0.7194 | -3.73182+0.00884*Tau+0.00688*Pro+0.00635*Gly-0.00367*Ala+0.04036*Met+0.01340*Orn |
| 0.7191 | -1.51633+0.01050*Tau+0.00128*Thr+0.00841*Pro-0.03720*Trp+0.01854*Orn |
| 0.7182 | -1.62967+0.01055*Tau+0.00844*Pro-0.00030*Ala+0.00531*Ile-0.03661*Trp+0.01896*Orn |
| 0.7181 | -2.69163-0.00550*Thr+0.02362*Glu+0.00585*Pro+0.00726*Gly+0.06349*Met-0.03964*Trp |
| 0.7181 | -1.65884+0.01055*Tau+0.00824*Pro+0.00514*Ile-0.03707*Trp+0.01882*Orn |
| 0.7177 | -1.75692+0.01043*Tau+0.00868*Pro-0.00026*Ala+0.01064*Phe-0.03980*Trp+0.01837*Orn |
| 0.7170 | -3.31094+0.00877*Tau+0.00703*Pro+0.00724*Gly-0.00281*Ala+0.01424*Orn |
| 0.7169 | -3.37077+0.00873*Tau-0.00491*Thr+0.00555*Pro+0.00724*Gly+0.01802*Orn-0.00145*Lys |
| 0.7168 | -1.78502+0.01043*Tau+0.00851*Pro+0.01057*Phe-0.04019*Trp+0.01823*Orn |
| 0.7168 | -1.78502+0.01043*Tau+0.00851*Pro+0.01057*Phe-0.04019*Trp+0.01823*Orn |
| 0.7167 | -3.52030+0.00880*Tau+0.00684*Pro+0.00731*Gly-0.00297*Ala+0.00575*Ile+0.01319*Orn |
| 0.7165 | -1.58824+0.01102*Tau+0.00884*Pro-0.00124*Ala+0.07673*Met-0.04398*Trp |
| 0.7163 | -2.91262+0.01000*Tau-0.00541*Thr+0.02295*Glu+0.00848*Gly+0.07225*Met-0.04386*Trp |
| 0.7162 | -1.79326+0.01042*Tau+0.00058*Thr+0.00847*Pro+0.01027*Phe-0.04033*Trp+0.01768*Orn |
| 0.7161 | -2.91045+0.00986*Tau+0.02009*Glu+0.00886*Gly-0.03515*Trp+0.01358*Orn |
| 0.7160 | -2.48564+0.00986*Tau+0.01402*Glu+0.00644*Pro+0.06997*Met-0.05110*Trp+0.01466*Orn |
| 0.7160 | -3.79046+0.00725*Tau+0.01627*Glu+0.00660*Pro+0.00857*Gly-0.00348*Ala+0.01182*Orn |
| 0.7158 | -3.05205+0.00993*Tau+0.01923*Glu+0.00876*Gly+0.00440*Ile-0.03549*Trp+0.01287*Orn |
| 0.7157 | -2.57210+0.00999*Tau+0.02382*Glu+0.00711*Gly+0.07770*Met-0.01643*Phe-0.04266*Trp |
| 0.7151 | -3.03120+0.00990*Tau+0.01959*Glu+0.00884*Gly+0.00435*Phe-0.03648*Trp+0.01308*Orn |
| 0.7148 | -1.69834+0.01046*Tau+0.00845*Pro-0.03910*Trp+0.01828*Orn+0.00240*Lys |
| 0.7147 | -3.99083+0.00718*Tau-0.00621*Thr+0.01454*Glu+0.00487*Pro+0.00835*Gly+0.01533*Orn |
| 0.7146 | -2.94166+0.00999*Tau+0.02176*Glu+0.00764*Gly+0.05867*Met-0.04448*Trp |
| 0.7142 | -2.03518-0.00339*Thr+0.00704*Pro+0.00570*Gly+0.05845*Met+0.00109*Phe-0.03155*Trp |
| 0.7138 | -2.42166+0.02420*Glu+0.00551*Pro+0.00606*Gly+0.06547*Met-0.01309*Phe-0.03878*Trp |
| 0.7138 | -3.62114+0.00853*Tau-0.00562*Thr+0.00550*Pro+0.00727*Gly+0.00256*Phe+0.01726*Orn |
| 0.7135 | -3.52117+0.00859*Tau-0.00537*Thr+0.00552*Pro+0.00721*Gly+0.01749*Orn |
| 0.7134 | -2.97904+0.02103*Glu+0.00537*Pro+0.00580*Gly+0.04975*Met-0.04037*Trp+0.00954*Orn |
| 0.7131 | -3.69848+0.00859*Tau-0.00558*Thr+0.00530*Pro+0.00726*Gly+0.00467*Ile+0.01671*Orn |
| 0.7126 | -1.69211+0.01098*Tau+0.00800*Pro+0.07235*Met-0.04546*Trp |
| 0.7125 | -2.71607+0.02234*Glu+0.00577*Pro+0.00641*Gly+0.04986*Met-0.04017*Trp |
| 0.7118 | -1.54734+0.01103*Tau+0.00820*Pro+0.07895*Met-0.00555*Ile-0.04593*Trp |
| 0.7111 | -1.53098+0.01118*Tau+0.00782*Pro+0.08119*Met-0.00816*Phe-0.04393*Trp |
| 0.7104 | -2.53006+0.00951*Tau+0.02245*Glu+0.00998*Gly-0.03437*Trp |
| 0.7099 | -1.60985+0.00942*Tau+0.01222*Glu+0.00824*Pro-0.04025*Trp+0.01799*Orn |
| 0.7097 | -2.36754+0.02253*Glu+0.00648*Pro+0.00809*Gly-0.03257*Trp |
| 0.7097 | -2.34894-0.00080*Thr+0.02271*Glu+0.00653*Pro+0.00829*Gly-0.03222*Trp |
| 0.7095 | -1.97020+0.00959*Tau+0.01586*Glu+0.00733*Pro+0.07421*Met-0.05075*Trp |
| 0.7094 | -3.94482+0.00960*Tau+0.01046*Thr+0.00727*Gly+0.06023*Met+0.02233*Orn |
| 0.7090 | -2.40484+0.00630*Pro+0.00445*Gly+0.05057*Met-0.03291*Trp+0.01228*Orn |
| 0.7090 | -2.03916+0.00695*Pro+0.00516*Gly+0.05120*Met-0.03202*Trp |
| 0.7063 | -2.36019+0.00810*Tau+0.00829*Pro-0.00125*Ala+0.00047*Phe+0.01928*Orn-0.00073*Lys |
| 0.7058 | -2.37299+0.00702*Pro+0.00607*Gly+0.01153*Phe-0.02861*Trp+0.01141*Orn-0.00033*Lys |
| 0.7056 | -1.89294+0.00760*Pro+0.00655*Gly-0.02666*Trp+0.00237*Lys |
| 0.7027 | -2.46477+0.01164*Tau+0.00751*Gly+0.00006*Ala-0.02962*Trp+0.01712*Orn+0.00070*Lys |
| 0.7022 | -3.65175+0.00830*Tau+0.00534*Pro+0.00635*Gly+0.01307*Orn |
| 0.6980 | -1.36467+0.01003*Tau+0.00953*Pro+0.00013*Ala+0.00388*Ile+0.01456*Phe-0.04011*Trp |
| 0.6922 | -3.38004+0.00805*Tau+0.00602*Pro+0.00708*Gly+0.00384*Phe |

# FIG.71

# FIG.72

| ROC AUC | FORMULA |
|---|---|
| 0.9072 | 0.32986+0.00251*Tau+0.00509*Ser+0.00146*Ala-0.02369*Cys+0.01191*Phe-0.01056*Arg |
| 0.9072 | 0.12984+0.00302*Tau+0.00592*Ser-0.02030*Cys+0.01140*Phe+0.00817*Orn-0.01099*Arg |
| 0.9072 | 0.12984+0.00302*Tau+0.00592*Ser-0.02030*Cys+0.01140*Phe+0.00817*Orn-0.01099*Arg |
| 0.9008 | 0.33247+0.00324*Tau+0.00696*Ser-0.01613*Cys+0.01372*Phe-0.00799*Trp-0.00895*Arg |
| 0.9008 | 0.70669+0.00255*Tau+0.00171*Ala-0.02572*Cys+0.00775*Phe+0.00749*Orn-0.01079*Arg |
| 0.9008 | 0.25847+0.00278*Tau+0.00056*Thr+0.00647*Ser-0.01887*Cys+0.01321*Phe-0.01001*Arg |
| 0.8944 | 0.25968+0.00281*Tau+0.00650*Ser-0.01812*Cys+0.00334*Met+0.01231*Phe-0.01003*Arg |
| 0.8944 | 0.25968+0.00281*Tau+0.00650*Ser-0.01812*Cys+0.00334*Met+0.01231*Phe-0.01003*Arg |
| 0.8928 | 0.19619+0.00279*Tau+0.00612*Ser+0.00064*Gly-0.01845*Cys+0.01261*Phe-0.00971*Arg |
| 0.8928 | 0.24478+0.00282*Tau+0.00664*Ser+0.00021*Pro-0.01880*Cys+0.01383*Phe-0.01006*Arg |
| 0.8928 | 0.61716+0.00374*Tau+0.00496*Ser-0.01615*Cys-0.01424*Trp+0.01217*Orn-0.00697*Arg |
| 0.8912 | 0.99917+0.00320*Tau+0.00182*Ala-0.02237*Cys-0.01306*Trp+0.01032*Orn-0.00779*Arg |
| 0.8896 | 0.92490+0.00355*Tau-0.01383*Cys+0.01534*Met-0.01420*Trp+0.01066*Orn-0.00820*Arg |
| 0.8896 | 0.39227+0.00285*Tau+0.00438*Ser-0.01822*Cys+0.01325*Met+0.00839*Orn-0.00971*Arg |
| 0.8880 | 0.79645+0.00250*Tau+0.00145*Ala-0.02352*Cys+0.00890*Met+0.00782*Orn-0.00983*Arg |
| 0.8880 | 0.79645+0.00250*Tau+0.00145*Ala-0.02352*Cys+0.00890*Met+0.00782*Orn-0.00983*Arg |
| 0.8848 | 0.89720+0.00359*Tau-0.01676*Cys+0.00789*Phe-0.01190*Trp+0.01146+0.00869-0.00869*Arg |
| 0.8832 | -0.28967+0.00315*Tau-0.00507*Thr+0.00560*Ser+0.02649*Met+0.01229*Orn-0.01010*Arg |
| 0.8832 | 0.97247+0.00266*Tau+0.00221*Ala-0.02349*Cys+0.00902*Phe-0.00777*Trp-0.00897*Arg |
| 0.8816 | 0.86600+0.00228*Tau+0.00218*Ala-0.02585*Cys-0.00060*Met+0.00962*Phe-0.01004*Arg |
| 0.8800 | 0.86556+0.00228*Tau+0.00215*Ala-0.02569*Cys+0.00937*Phe-0.01003*Arg |
| 0.8800 | 0.73790+0.00287*Tau+0.00084*Pro-0.02091*Cys+0.00885*Phe+0.00932*Orn-0.01025*Arg |
| 0.8800 | 0.56977+0.00262*Tau+0.00337*Ser+0.00144*Ala-0.02485*Cys+0.00861*Orn-0.00914*Arg |
| 0.8800 | 1.05357+0.00356*Tau+0.00108*Pro-0.01720*Cys-0.01377*Trp+0.01240*Orn-0.00695*Arg |
| 0.8784 | 0.63754+0.00287*Tau+0.00086*Gly-0.01974*Cys+0.00760*Phe+0.00872*Orn-0.00972*Arg |
| 0.8784 | 0.84439+0.00221*Tau+0.00109*Thr+0.00198*Ala-0.02569*Cys+0.00831*Phe-0.00999*Arg |
| 0.8768 | 0.13910+0.00374*Tau+0.00457*Ser+0.01735*Met-0.02047*Trp+0.00902*Orn-0.00727*Arg |
| 0.8768 | 0.13910+0.00374*Tau+0.00457*Ser+0.01735*Met-0.02047*Trp+0.00902*Orn-0.00727*Arg |
| 0.8768 | 0.14331+0.00334*Tau+0.00567*Ser+0.01577*Met+0.00530*Phe-0.01590*Trp-0.00727*Arg |
| 0.8752 | 0.64060+0.00318*Tau+0.00507*Ser-0.01218*Cys+0.01967*Met-0.01124*Trp-0.00741*Arg |
| 0.8736 | 0.31478+0.00334*Tau+0.00542*Ser-0.00079*Pro+0.02329*Met-0.01580*Trp-0.00673*Arg |
| 0.8736 | 0.82408+0.00229*Tau+0.00029*Gly+0.00199*Ala-0.02508*Cys+0.00903*Phe-0.00985*Arg |
| 0.8736 | 0.88784+0.00228*Tau-0.00090*Pro+0.00250*Ala-0.02593*Cys+0.00931*Phe-0.01004*Arg |
| 0.8736 | -0.39893+0.00262*Tau+0.00600*Ser+0.00085*Gly-0.00032*Ala+0.01190*Phe-0.00866*Arg |
| 0.8736 | 0.89300+0.00276*Tau+0.00367*Ser+0.00203*Ala-0.02210*Cys-0.00946*Trp-0.00664*Arg |
| 0.8720 | -0.43856+0.00266*Tau-0.00120*Thr+0.00624*Ser+0.00090*Gly+0.01293*Phe-0.00879*Arg |
| 0.8720 | 0.61958+0.00236*Tau+0.00367*Ser+0.00131*Ala-0.02102*Cys+0.01299*Met-0.00897*Arg |
| 0.8704 | 0.80465+0.00279*Tau-0.01791*Cys+0.01450*Met+0.00907*Orn-0.00963*Arg |
| 0.8704 | 0.80465+0.00279*Tau-0.01791*Cys+0.01450*Met+0.00907*Orn-0.00963*Arg |
| 0.8688 | -0.13716+0.00383*Tau+0.00602*Ser+0.00989*Phe-0.01928*Trp+0.00934*Orn-0.00779*Arg |
| 0.8688 | 0.30626+0.00328*Tau+0.00489*Ser+0.00010*Ala+0.02174*Met-0.01661*Trp-0.00665*Arg |
| 0.8688 | -0.40703+0.00254*Tau+0.00575*Ser+0.00071*Gly+0.01170*Phe-0.00879*Arg |
| 0.8688 | 0.87838+0.00249*Tau+0.00185*Ala-0.02609*Cys+0.00869*Orn-0.00919*Arg |
| 0.8688 | 0.43746+0.00382*Ser+0.00179*Ala-0.02433*Cys+0.00915*Phe+0.00502*Orn-0.01030*Arg |
| 0.8672 | -0.09006+0.00334*Tau+0.00621*Ser+0.00068*Gly+0.01154*Phe-0.01461*Trp-0.00703*Arg |
| 0.8672 | 0.32230+0.00331*Tau+0.00505*Ser-0.00010*Gly+0.02266*Met-0.01651*Trp-0.00671*Arg |
| 0.8672 | 0.01064+0.00336*Tau+0.00124*Thr+0.00625*Ser+0.01126*Phe-0.01639*Trp-0.00706*Arg |
| 0.8672 | 0.51699+0.00260*Tau+0.00463*Ser+0.00031*Gly-0.01592*Cys+0.01648*Met-0.00847*Arg |
| 0.8656 | 0.31649+0.00328*Tau+0.00062*Thr+0.00479*Ser+0.02061*Met-0.01716*Trp-0.00654*Arg |
| 0.8656 | 1.10651+0.00298*Tau+0.00265*Thr-0.01351*Cys+0.01462*Met-0.01273*Trp-0.00700*Arg |

# FIG.73

| ROC AUC | FORMULA |
|---|---|
| 0.8640 | 1.07816+0.00272*Tau+0.00179*Ala-0.01933*Cys+0.01359*Met-0.01010*Trp-0.00779*Arg |
| 0.8608 | 0.79853+0.00260*Tau+0.00103*Gly-0.01711*Cys+0.00527*Met+0.00717*Phe-0.00853*Arg |
| 0.8592 | 0.55785+0.00377*Tau-0.00172*Thr+0.02174*Met-0.01849*Trp+0.01170*Orn-0.00769*Arg |
| 0.8592 | -0.05065+0.00334*Tau+0.00648*Ser+0.00036*Ala+0.01239*Phe-0.01536*Trp-0.00734*Arg |
| 0.8592 | 1.09078+0.00304*Tau-0.01263*Cys+0.01819*Met+0.00274*Phe-0.00948*Trp-0.00770*Arg |
| 0.8592 | 1.10847+0.00261*Tau+0.00063*Gly+0.00210*Ala-0.02235*Cys-0.00853*Trp-0.00657*Arg |
| 0.8576 | -0.04850+0.00342*Tau+0.00709*Ser-0.00030*Pro+0.01314*Phe-0.01445*Trp-0.00737*Arg |
| 0.8560 | -0.04575+0.00340*Tau+0.00690*Ser+0.01295*Phe-0.01473*Trp-0.00735*Arg |
| 0.8560 | 1.12412+0.00304*Tau-0.01193*Cys+0.02147*Met-0.01015*Trp-0.00733*Arg |
| 0.8560 | 0.59933+0.00364*Tau+0.02063*Met-0.00182*Phe-0.01895*Trp+0.00973*Orn-0.00706*Arg |
| 0.8560 | 0.86574+0.00296*Tau+0.00133*Gly-0.01574*Cys+0.00893*Phe-0.00658*Trp-0.00746*Arg |
| 0.8560 | 1.03060+0.00300*Tau+0.00065*Gly-0.01250*Cys+0.01747*Met-0.00963*Trp-0.00696*Arg |
| 0.8544 | 0.77353+0.00263*Tau+0.00132*Gly-0.01777*Cys+0.00919*Phe-0.00839*Arg |
| 0.8528 | 0.56601+0.00364*Tau+0.01858*Met-0.01968*Trp+0.00966*Orn-0.00727*Arg |
| 0.8528 | 0.56726+0.00364*Tau-0.00001*Gly+0.01863*Met-0.01968*Trp+0.00967*Orn-0.00727*Arg |
| 0.8528 | 0.16947+0.00289*Tau-0.00355*Thr+0.00053*Gly+0.02231*Met-0.01090*Orn-0.00933*Arg |
| 0.8512 | 0.58046+0.00366*Tau-0.00031*Pro+0.01904*Met-0.01944*Trp+0.00979*Orn-0.00731*Arg |
| 0.8496 | 0.90770+0.00251*Tau+0.00078*Gly-0.01566*Cys+0.01547*Met-0.00799*Arg |
| 0.8496 | 1.10639+0.00300*Tau+0.00077*Pro-0.01287*Cys+0.01996*Met-0.01047*Trp-0.00732*Arg |
| 0.8496 | 0.18642+0.00366*Tau+0.00427*Ser+0.00074*Gly-0.01271*Cys-0.01908*Trp+0.00902*Orn |
| 0.8496 | 0.52827+0.00088*Thr+0.00374*Ser+0.00192*Ala-0.02433*Cys+0.00943*Phe-0.00980*Arg |
| 0.8480 | 0.37650+0.00363*Tau+0.00085*Gly+0.00614*Phe-0.01812*Trp+0.00978*Orn-0.00666*Arg |
| 0.8480 | 0.14014+0.00377*Tau+0.00449*Ser+0.00069*Gly-0.01999*Trp+0.01050*Orn-0.00540*Arg |
| 0.8464 | 0.54554+0.00361*Tau+0.00018*Ala+0.01804*Met-0.01990*Trp+0.00944*Orn-0.00725*Arg |
| 0.8464 | 0.30378+0.00234*Tau+0.00063*Gly+0.01614*Met+0.00240*Phe-0.00809*Arg |
| 0.8464 | 0.30378+0.00234*Tau+0.00063*Gly+0.01614*Met+0.00240*Phe-0.00809*Arg |
| 0.8448 | -0.66530+0.00327*Tau-0.00406*Thr+0.00752*Ser+0.01495*Phe+0.01157*Orn-0.01068*Arg |
| 0.8432 | 0.99490+0.00248*Tau+0.00063*Pro-0.01603*Cys+0.01890*Met-0.00852*Arg |
| 0.8416 | 0.23787+0.00288*Tau-0.00329*Thr+0.02475*Met+0.01094*Orn-0.00968*Arg |
| 0.8416 | -0.50873+0.00268*Tau+0.00540*Ser+0.00045*Gly+0.01039*Phe+0.00583*Orn-0.00955*Arg |
| 0.8416 | 0.49038+0.00432*Ser+0.00219*Ala-0.02548*Cys-0.00509*Met+0.01269*Phe-0.00993*Arg |
| 0.8416 | 0.82410+0.00225*Ala-0.02583*Cys+0.00719*Phe+0.00554*Orn-0.00999*Arg |
| 0.8400 | 0.50837+0.00442*Ser-0.00129*Pro+0.00248*Ala-0.02442*Cys+0.01051*Phe-0.00990*Arg |
| 0.8368 | 0.71066+0.00308*Tau+0.00060*Ala+0.02165*Met-0.01631*Trp-0.00659*Arg |
| 0.8368 | 0.69144+0.00308*Tau+0.00016*Gly+0.00054*Ala+0.02089*Met-0.01615*Trp-0.00649*Arg |
| 0.8352 | -0.35798+0.00270*Tau-0.00019*Thr+0.00710*Ser-0.00076*Pro+0.01390*Phe-0.00913*Arg |
| 0.8352 | 0.51954+0.00415*Ser-0.00010*Gly+0.00208*Ala-0.02440*Cys+0.01058*Phe-0.00993*Arg |
| 0.8304 | 0.71620+0.00307*Tau-0.00069*Pro+0.00084*Ala+0.02197*Met-0.01607*Trp-0.00665*Arg |
| 0.8272 | 0.72226+0.00312*Tau+0.00169*Thr+0.00020*Gly+0.01846*Met-0.01725*Trp-0.00620*Arg |
| 0.8272 | 0.75222+0.00313*Tau+0.00181*Thr+0.01932*Met-0.01749*Trp-0.00630*Arg |
| 0.8272 | -0.23995+0.00377*Tau+0.00483*Ser+0.00059*Gly+0.00391*Phe-0.02359*Trp+0.00797*Orn |
| 0.8256 | 0.72422+0.00314*Tau+0.00042*Gly+0.02130*Met-0.01509*Trp-0.00633*Arg |
| 0.8256 | 0.72311+0.00314*Tau+0.00042*Gly+0.02124*Met+0.00004*Phe-0.01508*Trp-0.00634*Arg |
| 0.8256 | 0.72391+0.00314*Tau-0.00021*Pro+0.00049*Gly+0.02123*Met-0.01486*Trp-0.00631*Arg |
| 0.8224 | 0.79419+0.00316*Tau+0.02379*Met-0.01527*Trp-0.00659*Arg |
| 0.8224 | 0.80354+0.00315*Tau+0.00010*Pro+0.02451*Met-0.00077*Phe-0.01546*Trp-0.00650*Arg |
| 0.8224 | 0.15756+0.00241*Tau+0.00135*Gly+0.00851*Phe-0.00757*Arg |
| 0.8160 | 0.93540+0.00255*Ala-0.02579*Cys+0.00846*Phe-0.00948*Arg |
| 0.8128 | 0.91463+0.00014*Gly+0.00247*Ala-0.02548*Cys+0.00829*Phe-0.00938*Arg |
| 0.7152 | 0.39601+0.00007*Urea+0.00670*Glu+0.00165*Gln-0.00689*Val |

# FIG.74

# FIG.75

| ROC AUC | FORMULA |
|---|---|
| 0.9136 | -3.43602+0.02849*Tau+0.04618*Ser-0.18815*Cys+0.11767*Phe+0.06761*Orn-0.08874*Arg |
| 0.9136 | -3.43602+0.02849*Tau+0.04618*Ser-0.18815*Cys+0.11767*Phe+0.06761*Orn-0.08874*Arg |
| 0.9120 | -2.33255+0.02470*Tau+0.04291*Ser+0.01219*Ala-0.20373*Cys+0.10877*Phe-0.08360*Arg |
| 0.9120 | 1.68649+0.03341*Tau-0.13355*Cys+0.17748*Met-0.09329*Trp+0.08951*Orn-0.06310*Arg |
| 0.9104 | -2.73384+0.03064*Tau+0.05567*Ser-0.13546*Cys+0.10857*Phe-0.04179*Trp-0.06611*Arg |
| 0.9072 | -3.29258+0.02881*Tau+0.05538*Ser-0.00069*Pro-0.15133*Cys+0.11583*Phe-0.07288*Arg |
| 0.9056 | -3.15783+0.02822*Tau+0.00284*Thr+0.05263*Ser-0.15326*Cys+0.11243*Phe-0.07232*Arg |
| 0.9024 | -3.80533+0.02887*Tau+0.04966*Ser+0.00551*Gly-0.15534*Cys+0.10738*Phe-0.06929*Arg |
| 0.9008 | -3.17660+0.02825*Tau+0.05134*Ser-0.14947*Cys+0.07019*Met+0.08996*Phe-0.07389*Arg |
| 0.9008 | 0.85696+0.02425*Tau+0.01205*Ala-0.22058*Cys+0.09114*Phe+0.06262*Orn-0.08413*Arg |
| 0.9008 | -3.17660+0.02825*Tau+0.05134*Ser-0.14947*Cys+0.07019*Met+0.08996*Phe-0.07389*Arg |
| 0.8992 | 3.68995+0.02931*Tau+0.01144*Ala-0.15788*Cys-0.09394*Trp+0.06910*Orn-0.05255*Arg |
| 0.8960 | 1.09094+0.02443*Tau+0.00694*Ala-0.17686*Cys+0.14673*Met+0.06704*Orn-0.07494*Arg |
| 0.8960 | 1.59206+0.03267*Tau-0.15046*Cys+0.08294*Phe-0.07635*Trp+0.09014*Orn-0.06370*Arg |
| 0.8960 | 1.09094+0.02443*Tau+0.00694*Ala-0.17686*Cys+0.14673*Met+0.06704*Orn-0.07494*Arg |
| 0.8944 | -2.14970+0.02642*Tau+0.03162*Ser-0.15518*Cys+0.19244*Met+0.07173*Orn-0.08068*Arg |
| 0.8896 | -0.77718+0.02904*Tau+0.03976*Ser-0.09438*Cys+0.16708*Met-0.06523*Trp-0.05287*Arg |
| 0.8880 | -3.00534+0.03098*Tau+0.02967*Ser+0.14997*Met-0.12179*Trp+0.05782*Orn-0.05849*Arg |
| 0.8880 | -6.10498+0.02613*Tau-0.03922*Thr+0.04253*Ser+0.22511*Met+0.09494*Orn-0.08062*Arg |
| 0.8880 | -3.00534+0.03098*Tau+0.02967*Ser+0.14997*Met-0.12179*Trp+0.05782*Orn-0.05849*Arg |
| 0.8880 | -0.17289+0.02748*Tau+0.00627*Gly-0.17005*Cys+0.08298*Phe+0.07037*Orn-0.06975*Arg |
| 0.8848 | 0.60441+0.02598*Tau+0.00361*Pro-0.17294*Cys+0.09108*Phe+0.07673*Orn-0.07317*Arg |
| 0.8848 | 0.70418+0.02649*Tau-0.14956*Cys+0.17658*Met+0.07650*Orn-0.07224*Arg |
| 0.8848 | 1.11889+0.02977*Tau+0.03062*Ser-0.10758*Cys-0.08916*Trp+0.07747*Orn-0.05088*Arg |
| 0.8848 | 0.70418+0.02649*Tau-0.14956*Cys+0.17658*Met+0.07650*Orn-0.07224*Arg |
| 0.8816 | 0.62829+0.02112*Tau+0.02283*Ser+0.00984*Ala-0.16378*Cys+0.05531*Orn-0.06599*Arg |
| 0.8816 | 2.06165+0.02615*Tau+0.01611*Ala-0.17349*Cys+0.07199*Phe-0.04513*Trp-0.06092*Arg |
| 0.8816 | 3.53309+0.03100*Tau+0.00329*Pro-0.11281*Cys-0.08899*Trp+0.07927*Orn-0.04333*Arg |
| 0.8800 | -4.49597+0.02832*Tau+0.03807*Ser+0.00531*Gly+0.07067*Phe-0.07880*Trp-0.04711*Arg |
| 0.8800 | 1.57315+0.02284*Tau+0.01562*Ala-0.18778*Cys+0.07825*Phe-0.06922*Arg |
| 0.8800 | -0.56810+0.02275*Tau+0.03051*Ser+0.00860*Ala-0.15310*Cys+0.13838*Met-0.06563*Arg |
| 0.8784 | 1.57118+0.02292*Tau+0.01451*Ala-0.18298*Cys+0.02674*Met+0.06896*Phe-0.06918*Arg |
| 0.8768 | -4.04903+0.02790*Tau+0.03724*Ser+0.00331*Ala+0.07586*Phe-0.08670*Trp-0.04675*Arg |
| 0.8768 | 1.41377+0.02197*Tau+0.00661*Thr+0.01435*Ala-0.18935*Cys+0.07540*Phe-0.06873*Arg |
| 0.8752 | -3.03345+0.02855*Tau+0.03651*Ser+0.12224*Met+0.02648*Phe-0.09471*Trp-0.04859*Arg |
| 0.8752 | -2.09934+0.02828*Tau+0.03494*Ser-0.00172*Gly+0.16553*Met-0.09983*Trp-0.04635*Arg |
| 0.8752 | -2.43347+0.03115*Tau+0.04159*Ser-0.00950*Pro+0.17290*Met-0.09757*Trp-0.04811*Arg |
| 0.8752 | 1.38573+0.02288*Tau+0.00225*Gly+0.01408*Ala-0.18535*Cys+0.07456*Phe-0.06685*Arg |
| 0.8752 | -0.53726+0.03532*Tau-0.00698*Pro+0.15795*Met-0.12055*Trp+0.07078*Orn-0.05184*Arg |
| 0.8752 | -0.32653+0.03335*Tau-0.00384*Gly+0.17618*Met-0.12974*Trp+0.06954*Orn-0.05259*Arg |
| 0.8752 | 1.63992+0.02485*Tau-0.00961*Pro+0.02021*Ala-0.19483*Cys+0.08305*Phe-0.07327*Arg |
| 0.8736 | -6.10260+0.02372*Tau-0.00754*Thr+0.03724*Ser+0.00734*Gly+0.07974*Phe-0.05863*Arg |
| 0.8736 | 1.96610+0.02386*Tau+0.02662*Ser+0.01413*Ala-0.14635*Cys-0.06301*Trp-0.04605*Arg |
| 0.8720 | 2.82198+0.02643*Tau+0.01112*Ala-0.13271*Cys+0.11256*Met-0.05976*Trp-0.05031*Arg |
| 0.8704 | -4.27989+0.03132*Tau+0.03497*Ser+0.06782*Phe-0.11207*Trp+0.05629*Orn-0.05281*Arg |
| 0.8704 | -0.76085+0.03414*Tau-0.01532*Thr+0.17384*Met-0.11858*Trp+0.08410*Orn-0.05213*Arg |
| 0.8704 | -5.91840+0.02427*Tau+0.03642*Ser+0.00773*Gly-0.00308*Ala+0.07437*Phe-0.05744*Arg |
| 0.8688 | -2.14335+0.02816*Tau+0.00336*Thr+0.03220*Ser+0.14522*Met-0.10039*Trp-0.04543*Arg |
| 0.8688 | -5.75782+0.02310*Tau+0.03345*Ser+0.00621*Gly+0.07258*Phe-0.05972*Arg |
| 0.8688 | -0.17930+0.02427*Ser+0.01394*Ala-0.20040*Cys+0.08011*Phe+0.04094*Orn-0.07566*Arg |

# FIG.76

| ROC AUC | FORMULA |
|---|---|
| 0.8672 | 2.50030+0.02161*Tau+0.01112*Ala-0.16847*Cys+0.05655*Orn-0.06109*Arg |
| 0.8672 | -1.25189+0.02523*Tau+0.03484*Ser+0.00103*Gly-0.11621*Cys+0.16130*Met-0.05871*Arg |
| 0.8656 | -2.23979+0.02806*Tau+0.03288*Ser+0.00080*Ala+0.15019*Met-0.09879*Trp-0.04552*Arg |
| 0.8656 | -0.68053+0.03236*Tau+0.00028*Ala+0.14320*Met-0.12127*Trp+0.06279*Orn-0.04972*Arg |
| 0.8656 | 2.42934+0.02723*Tau+0.01498*Thr-0.09433*Cys+0.12968*Met-0.06462*Trp-0.04524*Arg |
| 0.8640 | -0.66585+0.03240*Tau+0.14437*Met-0.12095*Trp+0.06329*Orn-0.04971*Arg |
| 0.8640 | -3.48983+0.02830*Tau+0.00903*Thr+0.03648*Ser+0.06634*Phe-0.09241*Trp-0.04530*Arg |
| 0.8624 | 3.23594+0.02497*Tau+0.00497*Gly+0.01289*Ala-0.14256*Cys-0.05874*Trp-0.03922*Arg |
| 0.8608 | 2.69686+0.02803*Tau-0.08526*Cys+0.15398*Met-0.04989*Trp-0.04580*Arg |
| 0.8608 | -0.54271+0.03224*Tau+0.14994*Met-0.00548*Phe-0.12134*Trp+0.06326*Orn-0.04912*Arg |
| 0.8608 | 1.22116+0.02725*Tau+0.00970*Gly-0.11938*Cys+0.05742*Phe-0.02559*Trp-0.04400*Arg |
| 0.8608 | 2.16368+0.02468*Tau+0.00064*Pro-0.10381*Cys+0.15464*Met-0.05267*Arg |
| 0.8576 | 0.34563+0.02728*Tau+0.00384*Ala+0.13183*Met-0.09166*Trp-0.03951*Arg |
| 0.8576 | 2.24402+0.02820*Tau+0.00414*Gly-0.09208*Cys+0.12420*Met-0.04732*Trp-0.04250*Arg |
| 0.8576 | 2.49650+0.02775*Tau-0.09138*Cys+0.13655*Met+0.01659*Phe-0.04448*Trp-0.04829*Arg |
| 0.8576 | 1.02421+0.02580*Tau+0.00701*Gly-0.12280*Cys+0.05808*Met+0.04433*Phe-0.05139*Arg |
| 0.8560 | -3.96139+0.02804*Tau+0.04116*Ser+0.07752*Phe-0.08160*Trp-0.04500*Arg |
| 0.8560 | 1.65605+0.02516*Tau+0.00457*Gly-0.10816*Cys+0.12249*Met-0.04848*Arg |
| 0.8560 | 0.39108+0.02737*Tau-0.00055*Gly+0.00406*Ala+0.13506*Met-0.09263*Trp-0.03982*Arg |
| 0.8560 | 2.65850+0.02785*Tau+0.00118*Pro-0.08654*Cys+0.15113*Met-0.05023*Trp-0.04547*Arg |
| 0.8544 | 0.80520+0.02584*Tau+0.00971*Gly-0.12849*Cys+0.06176*Phe-0.04842*Arg |
| 0.8528 | -4.13063+0.02989*Tau+0.04575*Ser-0.00541*Pro+0.03187*Phe-0.08060*Trp-0.04582*Arg |
| 0.8528 | 2.04928+0.01500*Ala-0.19897*Cys+0.07005*Phe+0.04509*Orn-0.07077*Arg |
| 0.8512 | -2.10406+0.02902*Tau+0.02484*Ser+0.00488*Gly-0.10967*Trp+0.05919*Orn-0.03912*Arg |
| 0.8512 | -6.16242+0.02362*Tau+0.03063*Ser+0.00502*Gly+0.06873*Phe+0.03345*Orn-0.06755*Arg |
| 0.8480 | 0.48811+0.02737*Tau+0.01220*Thr+0.00032*Gly+0.11889*Met-0.09611*Trp-0.03955*Arg |
| 0.8480 | 0.52849+0.02739*Tau+0.01240*Thr+0.12078*Met-0.09658*Trp-0.03972*Arg |
| 0.8480 | -2.21191+0.02381*Tau-0.01943*Thr+0.17510*Met+0.06836*Orn-0.06388*Arg |
| 0.8480 | -1.69598+0.02772*Tau+0.01917*Ser+0.00629*Gly-0.07964*Cys-0.10993*Trp+0.04773*Orn |
| 0.8464 | -5.14671+0.02433*Tau+0.00322*Thr+0.04039*Ser-0.00721*Pro+0.07839*Phe-0.05673*Arg |
| 0.8464 | -2.35028+0.02397*Tau-0.01970*Thr+0.00166*Gly+0.16324*Met+0.06703*Orn-0.06259*Arg |
| 0.8448 | 0.53422+0.02695*Tau+0.00181*Gly+0.13133*Met-0.08102*Trp-0.03779*Arg |
| 0.8448 | -1.43268+0.03152*Tau+0.00543*Gly+0.04072*Phe-0.10477*Trp+0.05747*Orn-0.04282*Arg |
| 0.8448 | 0.67172+0.02701*Tau+0.00154*Gly+0.13840*Met-0.00481*Phe-0.08216*Trp-0.03731*Arg |
| 0.8448 | 0.05006+0.03075*Ser-0.00946*Pro+0.01947*Ala-0.18628*Cys+0.07906*Phe-0.07193*Arg |
| 0.8416 | 0.78807+0.02705*Tau+0.14372*Met-0.08232*Trp-0.03866*Arg |
| 0.8416 | -7.08867+0.02515*Tau-0.02219*Thr+0.04414*Ser+0.09249*Phe+0.06753*Orn-0.06781*Arg |
| 0.8416 | 0.37744+0.03008*Tau-0.00912*Pro+0.00724*Ala+0.13641*Met-0.09352*Trp-0.04108*Arg |
| 0.8400 | -0.09044+0.02735*Ser+0.01585*Ala-0.17808*Cys-0.00861*Met+0.07890*Phe-0.06785*Arg |
| 0.8400 | -0.02042+0.00398*Thr+0.02529*Ser+0.01488*Ala-0.17841*Cys+0.07358*Phe-0.06694*Arg |
| 0.8384 | 0.53212+0.02786*Tau-0.00410*Pro+0.00326*Gly+0.12837*Met-0.07674*Trp-0.03767*Arg |
| 0.8384 | 0.09315+0.02869*Ser-0.00235*Gly+0.01695*Ala-0.18107*Cys+0.07994*Phe-0.07092*Arg |
| 0.8368 | 1.12648+0.02768*Tau-0.00232*Pro+0.15972*Met-0.01076*Phe-0.08250*Trp-0.03777*Arg |
| 0.8240 | -4.22342+0.02768*Tau+0.02395*Ser-0.00317*Gly+0.02465*Phe-0.13172*Trp+0.04134*Orn |
| 0.8192 | -1.46243+0.02051*Tau+0.00393*Gly+0.09497*Met+0.01485*Phe-0.04947*Arg |
| 0.8192 | -1.46243+0.02051*Tau+0.00393*Gly+0.09497*Met+0.01485*Phe-0.04947*Arg |
| 0.8176 | 2.49844+0.00030*Gly+0.01679*Ala-0.17064*Cys+0.06077*Phe-0.06077*Arg |
| 0.8160 | 2.54114+0.01697*Ala-0.17114*Cys+0.06114*Phe-0.06109*Arg |
| 0.8112 | -2.23244+0.02079*Tau+0.00844*Gly+0.04722*Phe-0.04578*Arg |
| 0.7088 | -0.61212+0.00031*Urea+0.03636*Glu+0.00901*Gln-0.03633*Val |

# FIG.77

# FIG.78

| ROC AUC | FORMULA |
|---|---|
| 0.9423 | 1.44828+0.00339*Tau+0.00228*Gly+0.00557*Leu-0.02025*His-0.01369*Trp-0.00306*Lys |
| 0.9385 | 1.12574+0.00326*Tau+0.00385*Ser+0.00172*Gly-0.01348*His-0.01317*Trp-0.00240*Lys |
| 0.9369 | 1.35184+0.00326*Tau+0.00248*Gly+0.00353*Val-0.01951*His-0.01572*Trp-0.00311*Lys |
| 0.9338 | 1.46041+0.00302*Tau+0.00221*Gly+0.00141*Cit-0.01621*His-0.01155*Trp-0.00202*Lys |
| 0.9338 | 1.48012+0.00300*Tau+0.00220*Gly-0.00090*ABA-0.01605*His-0.01117*Trp-0.00194*Lys |
| 0.9331 | 1.47266+0.00300*Tau+0.00221*Gly-0.01605*His-0.01129*Trp-0.00198*Lys |
| 0.9331 | 1.44151+0.00304*Tau+0.00247*Gly-0.01744*His-0.01241*Trp-0.00302*Lys+0.00340*Arg |
| 0.9315 | 1.34652+0.00323*Tau+0.00185*Gly-0.00428*ABA+0.00411*Leu-0.02007*His-0.01613*Trp |
| 0.9315 | 1.34652+0.00323*Tau+0.00185*Gly-0.00428*ABA+0.00411*Leu-0.02007*His-0.01613*Trp |
| 0.9308 | 1.08813+0.00334*Tau+0.00236*Ser+0.00152*Gly+0.00320*Leu-0.01794*His-0.01779*Trp |
| 0.9292 | 1.06823+0.00319*Tau+0.00364*Ser+0.00140*Gly-0.00515*ABA-0.01438*His-0.01542*Trp |
| 0.9285 | 1.69066+0.00290*Tau-0.00074*Gln+0.00251*Gly-0.01649*His-0.00974*Trp-0.00155*Lys |
| 0.9277 | 1.39036+0.00296*Tau+0.00192*Gly-0.00256*ABA-0.01672*His-0.01357*Trp |
| 0.9277 | 1.34153+0.00298*Tau+0.00194*Gly-0.01721*His-0.01466*Trp+0.00082*Arg |
| 0.9277 | 1.38617+0.00297*Tau+0.00192*Gly+0.00045*Cit-0.00261*ABA-0.01678*His-0.01366*Trp |
| 0.9277 | 1.34127+0.00309*Tau+0.00389*Ser-0.00108*Gln+0.00188*Gly-0.01475*His-0.01340*Trp |
| 0.9277 | 1.38617+0.00297*Tau+0.00192*Gly+0.00045*Cit-0.00261*ABA-0.01678*His-0.01366*Trp |
| 0.9277 | 1.30125+0.00324*Tau+0.00185*Gly+0.00034*Cit+0.00385*Leu-0.02000*His-0.01682*Trp |
| 0.9269 | 1.36879+0.00297*Tau+0.00194*Gly-0.00266*ABA-0.01717*His-0.01420*Trp+0.00087*Arg |
| 0.9269 | 1.30277+0.00323*Tau+0.00185*Gly+0.00383*Leu-0.01998*His-0.01679*Trp+0.00009*Arg |
| 0.9262 | 1.34768+0.00296*Tau+0.00195*Gly-0.00106*Cit-0.01717*His-0.01455*Trp+0.00101*Arg |
| 0.9254 | 1.05940+0.00318*Tau+0.00316*Ser+0.00146*Gly-0.01479*His-0.01606*Trp |
| 0.9254 | 1.64559+0.00315*Tau-0.00108*Gln+0.00237*Gly+0.00452*Leu-0.02092*His-0.01410*Trp |
| 0.9246 | 1.36306+0.00297*Tau+0.00191*Gly-0.01678*His-0.01404*Trp |
| 0.9246 | 1.05369+0.00318*Tau+0.00311*Ser+0.00148*Gly-0.01504*His-0.01633*Trp+0.00041*Arg |
| 0.9246 | 1.63967+0.00286*Tau-0.00097*Gln+0.00243*Gly-0.01793*His-0.01236*Trp+0.00149*Arg |
| 0.9246 | 1.27833+0.00269*Tau+0.00289*Gly+0.00413*Leu-0.02367*His-0.00477*Lys+0.00204*Arg |
| 0.9231 | 1.03166+0.00321*Tau+0.00326*Ser+0.00144*Gly+0.00171*Cit-0.01494*His-0.01652*Trp |
| 0.9231 | 1.02328+0.00327*Tau+0.00248*Ser+0.00162*Gly+0.00196*Val-0.01737*His-0.01895*Trp |
| 0.9231 | 1.30353+0.00271*Tau+0.00271*Gly+0.00426*Leu-0.02277*His-0.00411*Lys |
| 0.9231 | 1.31949+0.00270*Tau+0.00270*Gly-0.00146*Cit+0.00425*Leu-0.02252*His-0.00403*Lys |
| 0.9223 | 1.64767+0.00288*Tau-0.00093*Gln+0.00236*Gly+0.00185*Cit-0.01736*His-0.01177*Trp |
| 0.9223 | 1.26951+0.00322*Tau+0.00190*Gly+0.00106*Val+0.00255*Leu-0.02004*His-0.01762*Trp |
| 0.9223 | 1.08857+0.00261*Tau+0.00266*Ser+0.00230*Gly-0.01763*His-0.00352*Lys |
| 0.9223 | 1.13673+0.00276*Tau+0.00179*Ser+0.00250*Gly+0.00376*Leu-0.02137*His-0.00427*Lys |
| 0.9215 | 1.70003+0.00284*Tau-0.00093*Gln+0.00237*Gly-0.00330*ABA-0.01706*His-0.01075*Trp |
| 0.9215 | 1.70003+0.00284*Tau-0.00093*Gln+0.00237*Gly-0.00330*ABA-0.01706*His-0.01075*Trp |
| 0.9215 | 1.34455+0.00272*Tau+0.00268*Gly-0.00443*ABA+0.00448*Leu-0.02279*His-0.00392*Lys |
| 0.9215 | 1.31540+0.00272*Tau+0.00268*Gly-0.00032*Val+0.00466*Leu-0.02269*His-0.00406*Lys |
| 0.9200 | 1.25209+0.00252*Tau+0.00282*Gly+0.00187*Val-0.02153*His-0.00393*Lys |
| 0.9200 | 1.06780+0.00261*Tau+0.00210*Ser+0.00255*Gly+0.00153*Val-0.01994*His-0.00411*Lys |
| 0.9192 | 1.27894+0.00313*Tau+0.00200*Gly-0.00458*ABA+0.00256*Val-0.01949*His-0.01755*Trp |
| 0.9192 | 1.28630+0.00253*Tau+0.00281*Gly-0.00448*ABA+0.00207*Val-0.02162*His-0.00377*Lys |
| 0.9185 | 1.38162+0.00260*Tau+0.00346*Ser-0.00117*Gln+0.00260*Gly-0.01725*His-0.00272*Lys |
| 0.9185 | 1.37177+0.00249*Tau+0.00257*Gly-0.00293*ABA-0.01895*His-0.00294*Lys |
| 0.9185 | 1.31305+0.00248*Tau+0.00280*Gly-0.02023*His-0.00389*Lys+0.00234*Arg |
| 0.9177 | 1.09389+0.00263*Tau+0.00313*Ser+0.00220*Gly-0.00517*ABA-0.01718*His-0.00331*Lys |
| 0.9169 | 1.24120+0.00314*Tau+0.00198*Gly+0.00235*Val-0.01935*His-0.01803*Trp |
| 0.9169 | 1.55387+0.00303*Tau-0.00100*Gln+0.00248*Gly+0.00259*Val-0.02001*His-0.01556*Trp |
| 0.9169 | 1.66177+0.00269*Tau-0.00115*Gln+0.00311*Gly+0.00486*Leu-0.02334*His-0.00334*Lys |

162

# FIG.79

| ROC AUC | FORMULA |
|---|---|
| 0.9169 | 1.24076+0.00314*Tau+0.00198*Gly+0.00234*Val-0.01936*His-0.01804*Trp+0.00002*Arg |
| 0.9169 | 1.24152+0.00314*Tau+0.00198*Gly-0.00003*Cit+0.00235*Val-0.01935*His-0.01802*Trp |
| 0.9169 | 1.36073+0.00248*Tau+0.00258*Gly-0.00161*Cit-0.01881*His-0.00302*Lys |
| 0.9169 | 1.27304+0.00251*Tau+0.00281*Gly-0.00206*Cit+0.00190*Val-0.02124*His-0.00384*Lys |
| 0.9162 | 1.58217+0.00249*Tau-0.00108*Gln+0.00321*Gly+0.00214*Val-0.02193*His-0.00319*Lys |
| 0.9146 | 1.25033+0.00363*Tau+0.00519*Ser+0.00406*Leu-0.01402*His-0.01855*Trp-0.00241*Lys |
| 0.9146 | 1.19001+0.00338*Tau+0.00618*Ser-0.00537*ABA-0.01048*His-0.01823*Trp-0.00162*Lys |
| 0.9146 | 1.42159+0.00243*Tau+0.00370*Ser-0.00157*Gln+0.00239*Gly-0.00954*ABA-0.01842*His |
| 0.9138 | 1.65926+0.00245*Tau-0.00101*Gln+0.00293*Gly+0.00032*Cit-0.01916*His-0.00232*Lys |
| 0.9131 | 1.66095+0.00245*Tau-0.00100*Gln+0.00293*Gly-0.01911*His-0.00231*Lys |
| 0.9131 | 1.66095+0.00245*Tau-0.00100*Gln+0.00293*Gly-0.01911*His-0.00231*Lys |
| 0.9131 | 1.64334+0.00244*Tau-0.00106*Gln+0.00319*Gly-0.02050*His-0.00321*Lys+0.00279*Arg |
| 0.9123 | 1.11893+0.00347*Tau+0.00611*Ser+0.00452*Cit-0.01101*His-0.01780*Trp-0.00195*Lys |
| 0.9115 | 1.17539+0.00352*Tau+0.00554*Ser+0.00211*Val-0.01273*His-0.01975*Trp-0.00230*Lys |
| 0.9108 | 1.35079+0.00328*Tau+0.00656*Ser-0.00075*Gln-0.00762*ABA-0.01097*His-0.01822*Trp |
| 0.9092 | 1.18515+0.00340*Tau+0.00590*Ser-0.01108*His-0.01751*Trp-0.00214*Lys+0.00135*Arg |
| 0.9092 | 1.17030+0.00348*Tau+0.00492*Ser-0.00720*ABA+0.00316*Leu-0.01458*His-0.01943*Trp |
| 0.9085 | 1.18874+0.00338*Tau+0.00582*Ser-0.01067*His-0.01686*Trp-0.00177*Lys |
| 0.9085 | 1.70903+0.00245*Tau-0.00103*Gln+0.00290*Gly-0.00390*ABA-0.01896*His-0.00207*Lys |
| 0.9077 | 1.11472+0.00340*Tau+0.00524*Ser-0.00742*ABA+0.00168*Val-0.01353*His-0.02030*Trp |
| 0.9069 | 1.14571+0.00226*Tau+0.00216*Gly-0.02059*His-0.00143*Arg |
| 0.9062 | 1.13788+0.00331*Tau+0.00556*Ser-0.00634*ABA-0.01164*His-0.01773*Trp |
| 0.9062 | 1.13788+0.00331*Tau+0.00556*Ser-0.00634*ABA-0.01164*His-0.01773*Trp |
| 0.9062 | 1.14042+0.00331*Tau+0.00557*Ser-0.00633*ABA-0.01153*His-0.01757*Trp-0.00022*Arg |
| 0.9062 | 0.96703+0.00224*Tau+0.00111*Ser+0.00209*Gly-0.02127*His |
| 0.9046 | 1.15781+0.00220*Tau+0.00222*Gly-0.00474*Cit-0.02075*His |
| 0.9038 | 1.08717+0.00221*Tau+0.00223*Gly-0.02172*His |
| 0.9038 | 1.26118+0.00335*Tau+0.00617*Ser-0.00075*Gln+0.00472*Cit-0.01191*His-0.01851*Trp |
| 0.9023 | 1.58215+0.00235*Tau-0.00151*Gln+0.00287*Gly+0.00284*Leu-0.02381*His |
| 0.9008 | 1.31387+0.00328*Tau+0.00584*Ser-0.00065*Gln-0.01149*His-0.01750*Trp |
| 0.9008 | 1.59817+0.00226*Tau-0.00134*Gln+0.00280*Gly-0.02087*His |
| 0.9008 | 1.68754+0.00229*Tau-0.00133*Gln+0.00278*Gly-0.00638*ABA-0.02033*His |
| 0.9000 | 1.36376+0.00344*Tau+0.00521*Ser-0.00072*Gln+0.00315*Leu-0.01442*His-0.01920*Trp |
| 0.8985 | 1.13988+0.00231*Tau+0.00221*Gly-0.00767*ABA+0.00196*Leu-0.02316*His |
| 0.8977 | 1.08208+0.00336*Tau+0.00520*Ser+0.00289*Cit-0.01231*His-0.01937*Trp |
| 0.8977 | 1.55951+0.00224*Tau-0.00141*Gln+0.00288*Gly+0.00080*Val-0.02219*His |
| 0.8962 | 1.15920+0.00346*Tau+0.00442*Ser+0.00287*Leu-0.01472*His-0.02031*Trp |
| 0.8962 | 1.15862+0.00346*Tau+0.00442*Ser+0.00002*Val+0.00285*Leu-0.01472*His-0.02033*Trp |
| 0.8962 | 1.51685+0.00246*Gly+0.00342*Leu-0.02087*His-0.00800*Trp-0.00341*Lys+0.00292*Arg |
| 0.8931 | 1.13376+0.00331*Tau+0.00508*Ser-0.01187*His-0.01844*Trp-0.00027*Arg |
| 0.8908 | 0.64716+0.00366*Tau+0.00799*Ser-0.00647*ABA-0.02106*Trp-0.00263*Lys |
| 0.8900 | 1.55136+0.00220*Gly-0.01701*His-0.00604*Trp-0.00186*Lys |
| 0.8885 | 1.44752+0.00192*Gly-0.01768*His-0.00867*Trp |
| 0.8885 | 1.48492+0.00247*Gly-0.00392*ABA+0.00320*Leu-0.02127*His-0.00303*Lys |
| 0.8877 | 0.78104+0.00353*Tau+0.00843*Ser-0.00099*Gln-0.00989*ABA-0.02212*Trp |
| 0.8862 | 1.49616+0.00240*Gly-0.00285*ABA-0.01856*His-0.00237*Lys |
| 0.8854 | 1.46833+0.00242*Gly-0.01867*His-0.00252*Lys |
| 0.8846 | 1.44799+0.00249*Gly+0.00301*Leu-0.02126*His-0.00320*Lys |
| 0.8815 | 1.32848+0.00212*Gly-0.00579*ABA-0.02040*His |
| 0.8800 | 1.24368+0.00213*Gly-0.02091*His |

# FIG.80

# FIG.81

| ROC AUC | FORMULA |
|---|---|
| 0.9454 | 8.98649+0.03455*Tau+0.02334*Gly+0.02746*Val-0.18880*His-0.11074*Trp-0.03571*Lys |
| 0.9431 | 8.62538+0.03492*Tau+0.02104*Gly+0.03824*Leu-0.18587*His-0.09152*Trp-0.02858*Lys |
| 0.9431 | 5.24867+0.03795*Tau+0.03904*Ser+0.01896*Gly-0.15815*His-0.07918*Trp-0.02426*Lys |
| 0.9331 | 8.58764+0.03434*Tau+0.02170*Gly+0.01653*ABA-0.16519*His-0.07668*Trp-0.02054*Lys |
| 0.9323 | 8.49201+0.03465*Tau+0.02127*Gly+0.00871*Cit-0.16136*His-0.07962*Trp-0.01996*Lys |
| 0.9323 | 8.68083+0.03659*Tau+0.02397*Gly-0.17643*His-0.09933*Trp-0.03018*Lys+0.03320*Arg |
| 0.9315 | 8.56011+0.03478*Tau+0.02120*Gly-0.16079*His-0.07657*Trp-0.01985*Lys |
| 0.9315 | 4.58515+0.02966*Tau+0.02806*Ser+0.01558*Gly-0.16025*His-0.11043*Trp+0.00752*Arg |
| 0.9300 | 4.87624+0.02912*Tau+0.02429*Ser+0.01474*Gly+0.01451*Leu-0.16486*His-0.11276*Trp |
| 0.9292 | 7.19146+0.02894*Tau+0.01828*Gly-0.01148*Cit-0.16416*His-0.10596*Trp+0.01093*Arg |
| 0.9285 | 4.67442+0.02997*Tau+0.02830*Ser+0.01529*Gly-0.15578*His-0.10390*Trp |
| 0.9285 | 6.87575+0.02825*Tau+0.01719*Gly+0.02136*Leu-0.17913*His-0.12253*Trp+0.00965*Arg |
| 0.9277 | 4.54292+0.03079*Tau+0.03013*Ser+0.01471*Gly-0.02298*ABA-0.14957*His-0.10358*Trp |
| 0.9277 | 4.55320+0.02974*Tau+0.02862*Ser+0.01527*Gly+0.00911*Cit-0.15634*His-0.10664*Trp |
| 0.9277 | 4.68403+0.02813*Tau+0.02523*Ser+0.01494*Gly+0.00889*Val-0.16262*His-0.11886*Trp |
| 0.9277 | 5.84923+0.03225*Tau+0.02818*Ser+0.02255*Gly+0.01751*Leu-0.20320*His-0.03460*Lys |
| 0.9277 | 5.17648+0.03372*Tau+0.03552*Ser+0.02232*Gly-0.00566*ABA-0.18754*His-0.03134*Lys |
| 0.9269 | 9.97834+0.03484*Tau-0.00481*Gln+0.02242*Gly-0.16075*His-0.07262*Trp-0.01605*Lys |
| 0.9269 | 7.13060+0.02927*Tau+0.01820*Gly-0.00859*ABA-0.16229*His-0.10794*Trp+0.00909*Arg |
| 0.9269 | 5.20388+0.03371*Tau+0.03513*Ser+0.02250*Gly-0.18908*His-0.03151*Lys |
| 0.9262 | 7.33260+0.03336*Tau+0.03700*Ser-0.00971*Gln+0.01816*Gly-0.15738*His-0.08920*Trp |
| 0.9262 | 6.77026+0.02728*Tau+0.01762*Gly+0.01143*Val-0.17300*His-0.12592*Trp+0.00742*Arg |
| 0.9262 | 7.08083+0.03692*Tau+0.04206*Ser-0.00906*Gln+0.02379*Gly-0.18336*His-0.02475*Lys |
| 0.9254 | 7.30028+0.02929*Tau+0.01809*Gly-0.16093*His-0.09861*Trp |
| 0.9254 | 7.06139+0.02911*Tau+0.01649*Gly-0.01545*ABA+0.02166*Leu-0.17030*His-0.11258*Trp |
| 0.9254 | 7.06139+0.02911*Tau+0.01649*Gly-0.01545*ABA+0.02166*Leu-0.17030*His-0.11258*Trp |
| 0.9254 | 9.55586+0.03034*Tau-0.00697*Gln+0.02085*Gly+0.01944*Cit-0.16185*His-0.09508*Trp |
| 0.9254 | 5.69813+0.03190*Tau+0.03075*Ser+0.02328*Gly+0.00845*Val-0.20061*His-0.03637*Lys |
| 0.9246 | 7.11263+0.02893*Tau+0.01836*Gly-0.16425*His-0.10792*Trp+0.00886*Arg |
| 0.9246 | 6.89182+0.02740*Tau+0.01732*Gly+0.01177*Val-0.16956*His-0.11911*Trp |
| 0.9246 | 6.93216+0.02812*Tau+0.01690*Gly-0.01888*ABA+0.01255*Val-0.16591*His-0.11990*Trp |
| 0.9238 | 9.70727+0.03142*Tau-0.00683*Gln+0.02022*Gly-0.01843*ABA-0.15599*His-0.08667*Trp |
| 0.9238 | 7.31814+0.02955*Tau+0.01796*Gly-0.00630*ABA-0.15942*His-0.09842*Trp |
| 0.9238 | 7.28097+0.02953*Tau+0.01799*Gly+0.00381*Cit-0.00664*ABA-0.15963*His-0.09981*Trp |
| 0.9238 | 9.64704+0.03006*Tau-0.00738*Gln+0.02115*Gly-0.16647*His-0.10168*Trp+0.01429*Arg |
| 0.9238 | 7.28097+0.02953*Tau+0.01799*Gly+0.00381*Cit-0.00664*ABA-0.15963*His-0.09981*Trp |
| 0.9238 | 9.70727+0.03142*Tau-0.00683*Gln+0.02022*Gly-0.01843*ABA-0.15599*His-0.08667*Trp |
| 0.9238 | 6.84063+0.02842*Tau+0.01697*Gly+0.01786*Cit+0.02250*Leu-0.17619*His-0.12040*Trp |
| 0.9238 | 6.92758+0.02794*Tau+0.01692*Gly+0.00476*Val+0.01471*Leu-0.17309*His-0.11677*Trp |
| 0.9238 | 8.27378+0.02895*Tau+0.02392*Gly+0.02874*Leu-0.21132*His-0.03458*Lys |
| 0.9238 | 6.78005+0.02735*Tau+0.01741*Gly+0.01061*Cit+0.01211*Val-0.17069*His-0.12375*Trp |
| 0.9238 | 8.27298+0.02895*Tau+0.02393*Gly+0.00027*ABA+0.02872*Leu-0.21137*His-0.03458*Lys |
| 0.9231 | 9.33288+0.02976*Tau-0.00743*Gln+0.01976*Gly+0.02265*Leu-0.17141*His-0.10152*Trp |
| 0.9231 | 9.84137+0.03065*Tau-0.00697*Gln+0.02580*Gly+0.03121*Leu-0.20417*His-0.03006*Lys |
| 0.9223 | 8.69336+0.02958*Tau+0.02669*Gly+0.03587*Leu-0.24008*His-0.04653*Lys+0.02505*Arg |
| 0.9223 | 8.28359+0.02873*Tau+0.02408*Gly+0.00195*Val+0.02615*Leu-0.21181*His-0.03519*Lys |
| 0.9215 | 8.35192+0.02948*Tau+0.02383*Gly-0.00970*Cit+0.02785*Leu-0.20904*His-0.03394*Lys |
| 0.9208 | 9.15734+0.02858*Tau-0.00694*Gln+0.02020*Gly+0.01202*Val-0.16818*His-0.10859*Trp |
| 0.9208 | 8.25168+0.03050*Tau+0.02365*Gly-0.02081*Cit-0.18371*His-0.02573*Lys |
| 0.9200 | 9.58608+0.03112*Tau-0.00559*Gln+0.02479*Gly-0.01196*Cit-0.18010*His-0.02114*Lys |

# FIG.82

| ROC AUC | FORMULA |
|---|---|
| 0.9192 | 7.96885+0.02905*Tau+0.02557*Gly-0.19609*His-0.03331*Lys+0.01734*Arg |
| 0.9185 | 8.25680+0.02773*Tau+0.02502*Gly+0.01349*Val-0.20523*His-0.03592*Lys |
| 0.9185 | 7.97891+0.02927*Tau+0.02423*Gly+0.01285*ABA-0.18922*His-0.02701*Lys |
| 0.9185 | 9.65833+0.02903*Tau-0.00606*Gln+0.02655*Gly+0.01380*Val-0.19886*His-0.03109*Lys |
| 0.9185 | 8.24679+0.02776*Tau+0.02513*Gly+0.00377*ABA+0.01339*Val-0.20603*His-0.03602*Lys |
| 0.9185 | 9.73506+0.03054*Tau-0.00674*Gln+0.02754*Gly-0.19443*His-0.02974*Lys+0.02297*Arg |
| 0.9169 | 7.18630+0.02967*Tau+0.03754*Ser-0.01286*Gln+0.02021*Gly-0.05760*ABA-0.17344*His |
| 0.9169 | 8.47703+0.02891*Tau+0.02493*Gly-0.02010*Cit+0.01347*Val-0.20307*His-0.03517*Lys |
| 0.9162 | 9.48826+0.03043*Tau-0.00580*Gln+0.02499*Gly-0.18112*His-0.02144*Lys |
| 0.9162 | 9.48826+0.03043*Tau-0.00580*Gln+0.02499*Gly-0.18112*His-0.02144*Lys |
| 0.9154 | 9.49129+0.03043*Tau-0.00580*Gln+0.02497*Gly-0.00055*ABA-0.18098*His-0.02141*Lys |
| 0.9146 | 5.59434+0.02772*Tau+0.04711*Ser+0.02508*Leu-0.12046*His-0.12856*Trp-0.01552*Lys |
| 0.9123 | 5.36445+0.02637*Tau+0.04947*Ser+0.01329*Val-0.11263*His-0.13624*Trp-0.01620*Lys |
| 0.9123 | 4.36992+0.02580*Tau+0.04617*Ser-0.05211*ABA+0.01022*Val-0.10937*His-0.13916*Trp |
| 0.9115 | 4.74526+0.02933*Tau+0.05333*Ser-0.03730*ABA-0.09277*His-0.11333*Trp-0.01157*Lys |
| 0.9100 | 4.33151+0.02093*Tau+0.02005*Ser+0.01875*Gly-0.20183*His |
| 0.9092 | 6.22650+0.02903*Tau+0.05638*Ser-0.00590*Gln-0.06021*ABA-0.09527*His-0.11486*Trp |
| 0.9085 | 4.56848+0.02698*Tau+0.04479*Ser-0.05266*ABA+0.02124*Leu-0.11730*His-0.13445*Trp |
| 0.9069 | 8.72599+0.02368*Tau-0.00867*Gln+0.02298*Gly-0.18626*His |
| 0.9069 | 4.54350+0.02754*Tau+0.05421*Ser+0.03770*Cit-0.10533*His-0.12014*Trp-0.01385*Lys |
| 0.9069 | 8.73205+0.02373*Tau-0.00866*Gln+0.02298*Gly-0.00015*Val-0.18609*His |
| 0.9069 | 4.40962+0.02681*Tau+0.04743*Ser-0.04315*ABA-0.09855*His-0.12186*Trp |
| 0.9069 | 4.40962+0.02681*Tau+0.04743*Ser-0.04315*ABA-0.09855*His-0.12186*Trp |
| 0.9069 | 4.37369+0.02676*Tau+0.04765*Ser-0.04361*ABA-0.10037*His-0.12353*Trp+0.00260*Arg |
| 0.9062 | 9.01527+0.02432*Tau-0.00900*Gln+0.02235*Gly-0.03153*ABA-0.17781*His |
| 0.9062 | 5.81813+0.02552*Tau+0.05443*Ser-0.00552*Gln+0.03747*Cit-0.11012*His-0.12690*Trp |
| 0.9062 | 6.10052+0.01919*Tau+0.01958*Gly-0.01792*ABA+0.00751*Leu-0.20242*His |
| 0.9054 | 6.05663+0.01957*Tau+0.02034*Gly-0.19906*His |
| 0.9054 | 5.06871+0.02773*Tau+0.05084*Ser-0.09877*His-0.11610*Trp-0.01213*Lys |
| 0.9054 | 5.01312+0.02865*Tau+0.05505*Ser-0.10928*His-0.12398*Trp-0.01694*Lys+0.01679*Arg |
| 0.9054 | 6.35498+0.02112*Tau+0.01993*Gly-0.19239*His-0.00910*Arg |
| 0.9038 | 6.40003+0.02549*Tau+0.04777*Ser-0.00490*Gln+0.01684*Leu-0.11900*His-0.13097*Trp |
| 0.9031 | 6.60995+0.02196*Tau+0.02011*Gly-0.03489*Cit-0.19466*His |
| 0.9023 | 8.64957+0.02297*Tau-0.00924*Gln+0.02272*Gly+0.01077*Leu-0.19457*His |
| 0.8985 | 6.22679+0.02604*Tau+0.05086*Ser-0.00487*Gln-0.10383*His-0.12116*Trp |
| 0.8969 | 8.52816+0.02086*Gly+0.02326*Leu-0.18905*His-0.04895*Trp-0.02557*Lys+0.02993*Arg |
| 0.8962 | 4.24726+0.02459*Tau+0.04633*Ser+0.02539*Cit-0.11019*His-0.12883*Trp |
| 0.8962 | 4.64325+0.02482*Tau+0.04471*Ser-0.10684*His-0.12617*Trp+0.00184*Arg |
| 0.8962 | 4.87436+0.02456*Tau+0.04118*Ser+0.01751*Leu-0.12131*His-0.13625*Trp |
| 0.8962 | 4.87631+0.02458*Tau+0.04117*Ser-0.00012*Val+0.01766*Leu-0.12130*His-0.13614*Trp |
| 0.8962 | 1.19125+0.03072*Tau+0.05559*Ser-0.00599*Gln-0.07827*ABA-0.14350*Trp |
| 0.8954 | 0.37436+0.03226*Tau+0.05530*Ser-0.05654*ABA-0.13680*Trp-0.01806*Lys |
| 0.8938 | 8.32980+0.01853*Gly-0.15352*His-0.02851*Trp-0.01201*Lys |
| 0.8908 | 7.90846+0.01973*Gly+0.01594*Leu-0.17437*His-0.01915*Lys |
| 0.8908 | 7.91114+0.01971*Gly-0.00102*ABA+0.01599*Leu-0.17420*His-0.01913*Lys |
| 0.8892 | 7.58532+0.01676*Gly-0.15716*His-0.04358*Trp |
| 0.8854 | 7.98102+0.01984*Gly-0.16326*His-0.01504*Lys |
| 0.8846 | 7.96734+0.01991*Gly+0.00424*ABA-0.16410*His-0.01518*Lys |
| 0.8815 | 6.81693+0.01817*Gly-0.00989*ABA-0.17522*His |
| 0.8808 | 6.75071+0.01833*Gly-0.17751*His |

## FIG.83

# FIG.84

| ROC AUC | FORMULA |
|---|---|
| 0.8658 | 2.64681+0.00056*Tau-0.00013*Urea-0.00749*Glu-0.00154*Gln+0.00794*Cit-0.01301*His |
| 0.8631 | 2.62507-0.00013*Urea-0.00634*Glu-0.00129*Gln-0.00052*Ala+0.00633*Tyr-0.01424*His |
| 0.8631 | 2.63131-0.00014*Urea-0.00670*Glu-0.00146*Gln-0.00067*Pro+0.00643*Tyr-0.01388*His |
| 0.8622 | 2.65230-0.00013*Urea-0.00735*Glu-0.00150*Gln-0.00017*Pro+0.00820*Cit-0.01274*His |
| 0.8622 | 2.63035-0.00013*Urea-0.00638*Glu-0.00138*Gln+0.00642*Tyr-0.01422*His-0.00306*Trp |
| 0.8622 | 2.66594+0.00085*Tau-0.00013*Urea-0.00660*Glu-0.00142*Gln-0.00025*Pro-0.01187*His |
| 0.8613 | 2.64562-0.00013*Urea-0.00742*Glu-0.00151*Gln+0.00822*Cit-0.01290*His |
| 0.8613 | 2.65432-0.00013*Urea-0.00712*Glu-0.00143*Gln-0.00023*Ala+0.00797*Cit-0.01277*His |
| 0.8613 | 2.65830+0.00081*Tau-0.00013*Urea-0.00664*Glu-0.00142*Gln-0.01206*His-0.00024*Trp |
| 0.8613 | 2.66920+0.00093*Tau-0.00013*Urea-0.00631*Glu-0.00131*Gln-0.00035*Ala-0.01197*His |
| 0.8604 | 2.65635+0.00080*Tau-0.00013*Urea-0.00669*Glu-0.00142*Gln-0.01208*His |
| 0.8604 | 2.60468-0.00014*Urea-0.00794*Glu-0.00153*Gln+0.00826*Cit+0.00081*Val-0.01368*His |
| 0.8604 | 2.62571-0.00013*Urea-0.00670*Glu-0.00128*Gln-0.00031*Ala+0.00080*Val-0.01251*His |
| 0.8596 | 2.60233-0.00014*Urea-0.00779*Glu-0.00160*Gln+0.00843*Cit+0.00559*Tyr-0.01521*His |
| 0.8596 | 2.66615-0.00012*Urea-0.00619*Glu-0.00126*Gln-0.00030*Ala-0.01174*His |
| 0.8596 | 2.66419-0.00012*Urea-0.00619*Glu-0.00126*Gln+0.00007*Pro-0.00033*Ala-0.01180*His |
| 0.8587 | 2.65510-0.00013*Urea-0.00654*Glu-0.00136*Gln-0.01187*His |
| 0.8587 | 2.65659-0.00012*Urea-0.00651*Glu-0.00136*Gln-0.01185*His-0.00019*Trp |
| 0.8587 | 2.60587+0.00106*Tau-0.00014*Urea-0.00738*Glu-0.00147*Gln+0.00100*Val-0.01311*His |
| 0.8587 | 2.66229-0.00012*Urea-0.00627*Glu-0.00127*Gln-0.00032*Ala-0.01179*His+0.00056*Trp |
| 0.8578 | 2.61048-0.00014*Urea-0.00747*Glu-0.00152*Gln+0.00825*Cit+0.00145*Phe-0.01330*His |
| 0.8578 | 2.62192-0.00013*Urea-0.00700*Glu-0.00138*Gln-0.00034*Pro+0.00091*Val-0.01243*His |
| 0.8569 | 2.66235-0.00013*Urea-0.00647*Glu-0.00136*Gln-0.00019*Pro-0.01169*His |
| 0.8569 | 2.66267-0.00013*Urea-0.00646*Glu-0.00136*Gln-0.00019*Pro-0.01168*His-0.00004*Trp |
| 0.8551 | 2.63480-0.00013*Urea-0.00687*Glu-0.00145*Gln+0.00574*Tyr-0.00099*Phe-0.01394*His |
| 0.8542 | 2.63251-0.00014*Urea-0.00776*Glu-0.00156*Gln+0.00872*Cit-0.01310*His+0.00155*Trp |
| 0.8542 | 2.61312-0.00013*Urea-0.00688*Glu-0.00145*Gln+0.00545*Tyr-0.01410*His |
| 0.8516 | 2.61518-0.00013*Urea-0.00705*Glu-0.00138*Gln+0.00079*Val-0.01262*His |
| 0.8516 | 2.60851-0.00013*Urea-0.00704*Glu-0.00138*Gln+0.00075*Val+0.00036*Phe-0.01269*His |
| 0.8489 | 2.29860-0.00012*Urea-0.00599*Glu-0.00069*Ala+0.00486*Cit-0.01641*His |
| 0.8489 | 2.31845-0.00012*Urea-0.00550*Glu-0.00070*Ala+0.00052*Phe-0.01565*His |
| 0.8489 | 2.29681-0.00012*Urea-0.00601*Glu+0.00040*Pro-0.00080*Ala+0.00484*Cit-0.01663*His |
| 0.8471 | 2.34618-0.00012*Urea-0.00531*Glu-0.00066*Ala-0.01533*His-0.00117*Trp |
| 0.8462 | 2.46065+0.00067*Tau-0.00012*Urea-0.00114*Gln+0.00003*Pro-0.00065*Ala-0.01294*His |
| 0.8453 | 2.33022-0.00012*Urea-0.00550*Glu+0.00041*Pro-0.00082*Ala-0.01571*His |
| 0.8444 | 2.48307+0.00055*Tau-0.00011*Urea-0.00124*Gln-0.00039*Pro-0.01247*His-0.00345*Trp |
| 0.8444 | 2.46051-0.00012*Urea-0.00110*Gln+0.00005*Pro-0.00062*Ala-0.01279*His |
| 0.8436 | 2.24570-0.00012*Urea-0.00626*Glu+0.00449*Cit-0.01697*His-0.00238*Trp |
| 0.8436 | 2.21288-0.00010*Tau-0.00013*Urea-0.00665*Glu-0.00024*Pro+0.00515*Cit-0.01727*His |
| 0.8427 | 2.23284+0.00005*Tau-0.00012*Urea-0.00624*Glu-0.01663*His |
| 0.8427 | 2.46199-0.00012*Urea-0.00110*Gln-0.00061*Ala-0.01275*His |
| 0.8427 | 2.48926-0.00011*Urea-0.00107*Gln-0.00052*Ala-0.01251*His-0.00231*Trp |
| 0.8418 | 2.21151-0.00013*Urea-0.00666*Glu-0.00025*Pro+0.00508*Cit-0.01730*His |
| 0.8418 | 2.28670-0.00012*Urea-0.00568*Glu-0.01601*His-0.00306*Trp |
| 0.8418 | 2.48304-0.00011*Urea-0.00120*Gln-0.00035*Pro-0.01235*His-0.00340*Trp |
| 0.8418 | 2.43983-0.00012*Urea-0.00117*Gln+0.00002*Pro-0.00060*Ala+0.00402*Cit-0.01336*His |
| 0.8418 | 2.22012-0.00012*Urea-0.00628*Glu+0.00448*Cit+0.00106*Phe-0.01726*His-0.00247*Trp |
| 0.8409 | 2.19984-0.00013*Urea-0.00660*Glu+0.00058*Val-0.01721*His |
| 0.8409 | 2.29548-0.00013*Urea-0.00588*Glu-0.00071*Ala+0.00065*Val-0.01616*His |
| 0.8409 | 2.43797+0.00051*Tau-0.00012*Urea-0.00133*Gln-0.00050*Pro-0.01278*His |

# FIG.85

| ROC AUC | FORMULA |
|---|---|
| 0.8400 | 2.20057-0.00013*Urea-0.00675*Glu+0.00509*Cit-0.01755*His |
| 0.8400 | 2.17869-0.00013*Urea-0.00678*Glu+0.00510*Cit+0.00085*Phe-0.01780*His |
| 0.8400 | 2.47706-0.00011*Urea-0.00120*Gln-0.00012*Val-0.01255*His-0.00364*Trp |
| 0.8400 | 2.24496-0.00012*Urea-0.00614*Glu-0.00025*Pro-0.01635*His |
| 0.8391 | 2.42719-0.00012*Urea-0.00136*Gln-0.00045*Pro+0.00437*Cit-0.00016*Val-0.01315*His |
| 0.8391 | 2.21900+0.00015*Tau-0.00012*Urea-0.00619*Glu-0.00026*Pro+0.00090*Phe-0.01669*His |
| 0.8382 | 2.12118-0.00012*Urea+0.00000196*Pro-0.00114*Ala+0.00182*Cit+0.00571*Tyr-0.01868*His |
| 0.8382 | 2.44466-0.00012*Urea-0.00127*Gln+0.00359*Cit-0.01324*His-0.00327*Trp |
| 0.8373 | 2.24345-0.00011*Urea-0.00078*Ala-0.00005*Val-0.01541*His-0.00340*Trp |
| 0.8373 | 2.43860-0.00012*Urea-0.00129*Gln-0.00046*Pro-0.01267*His |
| 0.8364 | 2.17532-0.00011*Urea+0.00708*Tyr-0.00187*Phe-0.01842*His-0.00884*Trp |
| 0.8364 | 2.26574-0.00012*Urea-0.00566*Glu-0.00016*Pro+0.00107*Phe-0.01616*His-0.00302*Trp |
| 0.8364 | 2.42780-0.00012*Urea-0.00130*Gln-0.00042*Pro-0.00020*Val+0.00093*Phe-0.01275*His |
| 0.8356 | 2.37047+0.00031*Tau-0.00012*Urea-0.00139*Gln+0.00410*Cit+0.00085*Phe-0.01406*His |
| 0.8347 | 2.23422+0.00026*Tau-0.00011*Urea+0.00034*Pro-0.00089*Ala-0.01576*His-0.00343*Trp |
| 0.8329 | 2.22194-0.00011*Urea+0.00034*Pro-0.00088*Ala+0.00138*Cit-0.01595*His-0.00326*Trp |
| 0.8320 | 2.19906-0.00011*Urea-0.00092*Ala-0.00017*Val-0.01578*His |
| 0.8311 | 2.13159-0.00011*Urea+0.00650*Tyr-0.01872*His-0.00875*Trp |
| 0.8311 | 2.23096-0.00011*Urea+0.00035*Pro-0.00087*Ala+0.00026*Phe-0.01573*His-0.00344*Trp |
| 0.8302 | 2.41432-0.00012*Urea-0.00130*Gln-0.01313*His |
| 0.8302 | 2.16470+0.00016*Tau-0.00011*Urea+0.00034*Pro-0.00104*Ala+0.00179*Cit-0.01658*His |
| 0.8302 | 2.18099+0.00024*Tau-0.00011*Urea+0.00035*Pro-0.00104*Ala-0.01626*His |
| 0.8293 | 2.18830-0.00011*Urea+0.00035*Pro-0.00103*Ala-0.00015*Phe-0.01612*His |
| 0.8284 | 2.48198-0.00014*Urea-0.00746*Glu-0.00214*Gln-0.00336*Phe |
| 0.8267 | 2.42141-0.00014*Urea-0.00724*Glu-0.00217*Gln-0.00058*Val |
| 0.8240 | 2.16018-0.00011*Urea-0.00032*Pro+0.00095*Cit-0.01622*His-0.00552*Trp |
| 0.8240 | 2.36231-0.00015*Urea-0.00816*Glu-0.00234*Gln+0.00421*Cit |
| 0.8231 | 2.17133-0.00011*Urea-0.00031*Pro-0.01602*His-0.00560*Trp |
| 0.8231 | 2.17191-0.00003*Tau-0.00011*Urea-0.00031*Pro-0.01600*His-0.00560*Trp |
| 0.8231 | 2.17276-0.00011*Urea-0.00031*Pro-0.00002*Val-0.01600*His-0.00560*Trp |
| 0.8222 | 2.16901-0.00011*Urea-0.00014*Val-0.01615*His-0.00580*Trp |
| 0.8222 | 2.06674-0.00011*Urea-0.00047*Pro-0.00022*Val-0.01686*His |
| 0.8213 | 2.37941-0.00014*Urea-0.00766*Glu-0.00223*Gln |
| 0.8213 | 2.15990-0.00011*Urea-0.01629*His-0.00587*Trp |
| 0.8213 | 2.14956-0.00011*Urea+0.00086*Cit-0.01648*His-0.00580*Trp |
| 0.8213 | 2.04707-0.00012*Urea-0.00051*Pro+0.00018*Phe-0.01711*His |
| 0.8213 | 2.05186-0.00012*Urea-0.00051*Pro-0.01706*His |
| 0.8204 | 2.15230-0.00011*Urea-0.00031*Pro+0.00077*Phe-0.01623*His-0.00568*Trp |
| 0.8204 | 2.15916-0.00011*Urea+0.00092*Cit-0.00015*Val-0.01633*His-0.00571*Trp |
| 0.8204 | 2.39833-0.00014*Urea-0.00717*Glu-0.00208*Gln-0.00041*Ala |
| 0.8196 | 2.14019-0.00011*Urea+0.00080*Phe-0.01651*His-0.00594*Trp |
| 0.8196 | 2.13037-0.00011*Urea+0.00084*Cit+0.00078*Phe-0.01669*His-0.00587*Trp |
| 0.8196 | 2.14858-0.00011*Urea-0.00025*Val+0.00114*Phe-0.01635*His-0.00584*Trp |
| 0.8187 | 2.01274-0.00036*Tau-0.00012*Urea+0.00198*Cit-0.01780*His |
| 0.8178 | 2.02295-0.00012*Urea-0.01760*His |
| 0.8169 | 2.03925-0.00045*Tau-0.00011*Urea+0.00221*Cit-0.00057*Val+0.00072*Phe-0.01736*His |
| 0.8169 | 2.01720-0.00030*Tau+0.00072*Pro-0.00111*Ala-0.00135*Val-0.01578*His |
| 0.8151 | 1.82258-0.00604*Glu+0.00211*Cit+0.00222*Tyr-0.01948*His |
| 0.8142 | 1.85158-0.00570*Glu+0.00048*Pro+0.00293*Tyr-0.01894*His |
| 0.8107 | 1.90366+0.00006*Tau-0.00219*Cit+0.00545*Tyr-0.01865*His-0.01028*Trp |

# FIG.86

# FIG.87

| ROC AUC | FORMULA |
|---|---|
| 0.8667 | 13.05911−0.00096*Urea−0.04677*Glu−0.00985*Gln+0.05012*Cit+0.04204*Tyr−0.09780*His |
| 0.8667 | 13.55778−0.00091*Urea−0.04174*Glu−0.00928*Gln−0.00377*Pro+0.04777*Tyr−0.09294*His |
| 0.8658 | 13.84346−0.00089*Urea−0.03880*Glu−0.00799*Gln−0.00438*Ala+0.04904*Tyr−0.09782*His |
| 0.8649 | 13.82033−0.00091*Urea−0.03731*Glu−0.00857*Gln+0.05159*Tyr−0.09805*His−0.02647*Trp |
| 0.8649 | 14.14839−0.00089*Urea−0.04186*Glu−0.00933*Gln+0.04577*Tyr−0.02171*Phe−0.09171*His |
| 0.8640 | 13.76946+0.01055*Tau−0.00083*Urea−0.04105*Glu−0.00934*Gln−0.07785*His−0.00474*Trp |
| 0.8631 | 13.73874+0.01071*Tau−0.00083*Urea−0.04186*Glu−0.00946*Gln−0.00105*Pro−0.07713*His |
| 0.8613 | 13.07619−0.00084*Urea−0.04160*Glu−0.00807*Gln−0.00238*Ala+0.04497*Cit−0.07646*His |
| 0.8604 | 13.72946+0.01048*Tau−0.00084*Urea−0.04222*Glu−0.00951*Gln−0.07806*His |
| 0.8604 | 12.67900−0.00087*Urea−0.04535*Glu−0.00900*Gln+0.04815*Cit+0.00277*Val−0.07878*His |
| 0.8604 | 13.47271+0.01174*Tau−0.00083*Urea−0.04456*Glu−0.00969*Gln+0.00437*Val−0.08266*His |
| 0.8604 | 14.08217+0.01188*Tau−0.00082*Urea−0.03856*Glu−0.00840*Gln−0.00345*Ala−0.08016*His |
| 0.8596 | 13.48610−0.00089*Urea−0.04246*Glu−0.00927*Gln+0.04091*Tyr−0.09391*His |
| 0.8596 | 13.44971−0.00077*Urea−0.03691*Glu−0.00762*Gln−0.00269*Ala−0.07202*His |
| 0.8596 | 13.30865−0.00079*Urea−0.03771*Glu−0.00766*Gln−0.00265*Ala+0.00177*Val−0.07345*His |
| 0.8596 | 13.51542−0.00078*Urea−0.03748*Glu−0.00749*Gln+0.00265*Pro−0.00351*Ala−0.07468*His |
| 0.8587 | 12.89625−0.00085*Urea−0.04407*Glu−0.00896*Gln+0.04685*Cit−0.07626*His |
| 0.8587 | 12.86128−0.00085*Urea−0.04413*Glu−0.00896*Gln+0.04711*Cit+0.00110*Phe−0.07652*His |
| 0.8587 | 12.85366−0.00085*Urea−0.04530*Glu−0.00913*Gln+0.04826*Cit−0.07665*His+0.00452*Trp |
| 0.8587 | 13.34406+0.00908*Tau−0.00088*Urea−0.04584*Glu−0.00966*Gln+0.03981*Cit−0.08141*His |
| 0.8578 | 13.27600−0.00079*Urea−0.03899*Glu−0.00847*Gln−0.07132*His−0.00265*Trp |
| 0.8578 | 13.43464−0.00078*Urea−0.03789*Glu−0.00773*Gln−0.00287*Ala−0.07234*His+0.00463*Trp |
| 0.8578 | 13.27672−0.00079*Urea−0.03900*Glu−0.00847*Gln+0.00006*Pro−0.07138*His−0.00272*Trp |
| 0.8569 | 13.25600−0.00079*Urea−0.03963*Glu−0.00856*Gln−0.07149*His |
| 0.8569 | 13.10520−0.00081*Urea−0.04057*Glu−0.00858*Gln+0.00204*Val−0.07328*His |
| 0.8569 | 12.89657−0.00085*Urea−0.04418*Glu−0.00897*Gln+0.00023*Pro+0.04691*Cit−0.07650*His |
| 0.8569 | 13.25581−0.00079*Urea−0.03961*Glu−0.00856*Gln−0.00005*Pro−0.07144*His |
| 0.8569 | 13.08633−0.00081*Urea−0.04047*Glu−0.00856*Gln−0.00050*Pro+0.00228*Val−0.07298*His |
| 0.8560 | 13.28532−0.00081*Urea−0.04059*Glu−0.00859*Gln+0.00252*Val−0.00721*Phe−0.07203*His |
| 0.8507 | 11.04802−0.00079*Urea−0.03558*Glu−0.00516*Ala+0.03317*Cit−0.09795*His |
| 0.8507 | 11.44342−0.00074*Urea−0.03040*Glu−0.00497*Ala−0.09282*His−0.00679*Trp |
| 0.8489 | 11.18049−0.00080*Urea−0.03683*Glu+0.00421*Pro−0.00633*Ala+0.03378*Cit−0.10148*His |
| 0.8480 | 11.61162−0.00073*Urea−0.03156*Glu−0.00531*Ala−0.00786*Phe−0.09177*His |
| 0.8480 | 12.39993+0.00954*Tau−0.00077*Urea−0.00677*Gln−0.00049*Pro−0.00485*Ala−0.08436*His |
| 0.8462 | 11.29175−0.00075*Urea−0.03215*Glu−0.00524*Ala+0.00087*Val−0.09428*His |
| 0.8444 | 11.49852−0.00075*Urea−0.03281*Glu+0.00408*Pro−0.00640*Ala−0.09691*His |
| 0.8444 | 12.42326+0.00944*Tau−0.00077*Urea−0.00736*Gln−0.00260*Pro−0.08070*His−0.02745*Trp |
| 0.8436 | 11.48626−0.00081*Urea+0.05825*Tyr−0.02731*Phe−0.12254*His−0.05686*Trp |
| 0.8436 | 11.79054−0.00075*Urea−0.00633*Gln+0.00019*Pro−0.00442*Ala+0.01819*Cit−0.08048*His |
| 0.8427 | 11.98676−0.00073*Urea−0.00615*Gln−0.00448*Ala−0.07798*His |
| 0.8427 | 10.15448−0.00082*Urea−0.03905*Glu+0.03308*Cit−0.00160*Phe−0.10301*His |
| 0.8427 | 10.46581−0.00079*Urea−0.03529*Glu+0.02915*Cit−0.10029*His−0.01629*Trp |
| 0.8427 | 10.47476−0.00079*Urea−0.03528*Glu+0.02912*Cit−0.00030*Phe−0.10022*His−0.01627*Trp |
| 0.8427 | 11.91458−0.00073*Urea−0.00736*Gln−0.00264*Pro−0.00215*Val−0.00645*Phe−0.07233*His |
| 0.8427 | 10.28663+0.00518*Tau−0.00083*Urea−0.03932*Glu−0.00166*Pro+0.02983*Cit−0.10593*His |
| 0.8418 | 11.23604+0.00820*Tau−0.00073*Urea+0.00163*Pro−0.00601*Ala−0.10240*His−0.02645*Trp |
| 0.8418 | 10.10414−0.00082*Urea−0.03910*Glu+0.03329*Cit−0.10341*His |
| 0.8418 | 10.12379−0.00081*Urea−0.03871*Glu−0.00113*Pro+0.03315*Cit−0.10197*His |
| 0.8418 | 10.78888−0.00075*Urea−0.03137*Glu−0.09576*His−0.01955*Trp |
| 0.8418 | 11.96714−0.00072*Urea−0.00673*Gln−0.00175*Pro−0.07497*His−0.02458*Trp |

# FIG.88

| ROC AUC | FORMULA |
|---|---|
| 0.8418 | 10.85356-0.00075*Urea-0.03121*Glu-0.00062*Pro-0.00229*Phe-0.09456*His-0.01895*Trp |
| 0.8418 | 12.23780-0.00072*Urea-0.00588*Gln-0.00376*Ala-0.07699*His-0.01702*Trp |
| 0.8418 | 11.97786-0.00073*Urea-0.00613*Gln+0.00047*Pro-0.00459*Ala-0.07836*His |
| 0.8409 | 10.57636+0.00621*Tau-0.00080*Urea-0.03616*Glu-0.00186*Pro-0.00009*Phe-0.10249*His |
| 0.8409 | 10.53273+0.00576*Tau-0.00080*Urea-0.03667*Glu-0.10439*His |
| 0.8409 | 11.12587-0.00066*Urea-0.00526*Ala-0.00293*Val-0.09135*His-0.02176*Trp |
| 0.8409 | 10.59905+0.00679*Tau-0.00074*Urea+0.00137*Pro-0.00698*Ala+0.00291*Cit-0.10397*His |
| 0.8409 | 10.64247+0.00693*Tau-0.00074*Urea+0.00139*Pro-0.00701*Ala-0.10366*His |
| 0.8409 | 11.87403+0.00875*Tau-0.00079*Urea-0.00803*Gln-0.00415*Pro-0.08001*His |
| 0.8400 | 11.52261-0.00075*Urea-0.00755*Gln-0.00280*Pro+0.02204*Cit-0.00232*Val-0.07652*His |
| 0.8391 | 10.41207-0.00082*Urea+0.00025*Pro-0.00762*Ala+0.00801*Cit+0.04244*Tyr-0.12000*His |
| 0.8391 | 11.51238-0.00075*Urea-0.00734*Gln-0.00308*Pro-0.07519*His |
| 0.8391 | 12.18684-0.00070*Urea-0.00676*Gln-0.00281*Val-0.07403*His-0.02539*Trp |
| 0.8391 | 10.42480-0.00077*Urea-0.03506*Glu-0.00124*Pro-0.09734*His |
| 0.8373 | 10.30252-0.00079*Urea-0.03625*Glu+0.00151*Val-0.10030*His |
| 0.8373 | 11.77444-0.00074*Urea-0.00694*Gln+0.01707*Cit-0.07915*His-0.02506*Trp |
| 0.8373 | 11.60366+0.00679*Tau-0.00081*Urea-0.00808*Gln+0.01518*Cit-0.00311*Phe-0.08430*His |
| 0.8364 | 11.16254-0.00068*Urea+0.00213*Pro-0.00577*Ala-0.00895*Phe-0.09343*His-0.02214*Trp |
| 0.8356 | 10.63044-0.00082*Urea+0.05105*Tyr-0.12440*His-0.05629*Trp |
| 0.8347 | 10.80266-0.00070*Urea+0.00193*Pro-0.00671*Ala-0.01109*Phe-0.09523*His |
| 0.8347 | 10.76218-0.00067*Urea-0.00620*Ala-0.00355*Val-0.09317*His |
| 0.8320 | 11.38835-0.00076*Urea-0.00741*Gln-0.07806*His |
| 0.8311 | 10.79522-0.00070*Urea+0.00201*Pro-0.00568*Ala+0.00604*Cit-0.09612*His-0.02257*Trp |
| 0.8293 | 11.55640-0.00077*Urea-0.03985*Glu-0.01296*Gln-0.00520*Val |
| 0.8284 | 10.30867-0.00069*Urea-0.00199*Pro-0.00464*Phe-0.09416*His-0.03401*Trp |
| 0.8284 | 12.03391-0.00079*Urea-0.04225*Glu-0.01287*Gln-0.02636*Phe |
| 0.8276 | 10.36739-0.00068*Urea-0.00278*Val-0.09467*His-0.03498*Trp |
| 0.8276 | 10.34911-0.00068*Urea-0.00161*Pro-0.00227*Val-0.09351*His-0.03392*Trp |
| 0.8276 | 10.30170-0.00068*Urea+0.00479*Cit-0.00276*Val-0.09559*His-0.03471*Trp |
| 0.8276 | 10.42329+0.00660*Tau-0.00073*Urea-0.00255*Pro-0.10081*His-0.03728*Trp |
| 0.8267 | 10.42439-0.00067*Urea-0.00262*Val-0.00229*Phe-0.09435*His-0.03489*Trp |
| 0.8249 | 10.17077-0.00070*Urea-0.00200*Pro-0.09509*His-0.03430*Trp |
| 0.8249 | 10.28838-0.00070*Urea-0.00491*Phe-0.09612*His-0.03550*Trp |
| 0.8249 | 9.51372-0.00073*Urea-0.00345*Pro-0.00717*Phe-0.09736*His |
| 0.8249 | 9.46404+0.00272*Tau-0.00074*Urea+0.00790*Cit-0.00333*Val-0.00192*Phe-0.10307*His |
| 0.8240 | 11.03044-0.00082*Urea-0.04249*Glu-0.01330*Gln |
| 0.8240 | 10.88922-0.00085*Urea-0.04416*Glu-0.01358*Gln+0.01621*Cit |
| 0.8231 | 10.07465-0.00071*Urea+0.00513*Cit-0.09810*His-0.03553*Trp |
| 0.8231 | 9.33821+0.00138*Tau+0.00306*Pro-0.00774*Ala-0.01040*Val-0.08809*His |
| 0.8222 | 10.09262-0.00070*Urea-0.00208*Pro+0.00620*Cit-0.09619*His-0.03391*Trp |
| 0.8222 | 10.21838-0.00070*Urea+0.00468*Cit-0.00459*Phe-0.09708*His-0.03525*Trp |
| 0.8222 | 11.10861-0.00081*Urea-0.04026*Glu-0.01245*Gln-0.00212*Ala |
| 0.8204 | 10.14151-0.00070*Urea-0.09712*His-0.03582*Trp |
| 0.8196 | 9.12496+0.00335*Tau-0.00078*Urea+0.00835*Cit-0.10757*His |
| 0.8187 | 9.28738-0.00074*Urea-0.00349*Pro-0.09887*His |
| 0.8178 | 9.51542-0.00071*Urea-0.00299*Pro-0.00286*Val-0.09659*His |
| 0.8169 | 9.15790-0.00075*Urea-0.10277*His |
| 0.8160 | 7.30199-0.03011*Glu-0.00283*Pro+0.01789*Tyr-0.10419*His |
| 0.8151 | 7.15027-0.03149*Glu+0.00658*Cit+0.01436*Tyr-0.10623*His |
| 0.8080 | 8.02649+0.00382*Tau-0.01757*Cit+0.03352*Tyr-0.11019*His-0.05786*Trp |

# FIG.89

# FIG.90

# FIG.91

# FIG.92

# FIG.93

# FIG.94

# FIG.95

## FIG.96

FIG.97

| ROC AUC | FORMULA |
|---|---|
| 0.9033 | (Tau)/(Tyr)+(Thr+Arg)/(Cit+His) |
| 0.9024 | (Tau)/(Tyr)+(Thr)/(Cit+ABA+His) |
| 0.9020 | (Tau)/(Tyr)+(Thr)/(Cit+His) |
| 0.9015 | (Tau)/(Tyr)+(Pro+Thr)/(His+Val) |
| 0.9011 | (Tau)/(His)+(Thr+Arg)/(Tyr+Val) |
| 0.9005 | (Tau)/(His)+(Pro+Thr)/(Tyr+Val) |
| 0.9000 | (Tau)/(His)+(Thr)/(Tyr+Val) |
| 0.9000 | (Tau)/(Tyr)+(Thr+Arg)/(His+Leu) |
| 0.8997 | (Tau)/(His)+(Thr)/(ABA+Tyr+Val) |
| 0.8996 | (Tau)/(His)+(Thr)/(Asn+Tyr+Leu) |
| 0.8995 | (Tau)/(His)+(Thr)/(Cit+Tyr+Val) |
| 0.8995 | (Tau)/(Tyr)+(Thr+Arg)/(Asn+His) |
| 0.8993 | (Tau)/(His)+(Thr)/(Cit+Tyr+Leu) |
| 0.8991 | (Tau)/(His)+(Thr)/(ABA+Tyr+Leu) |
| 0.8989 | (Tau)/(Asn+His)+(Thr)/(Tyr+Val) |
| 0.8989 | (Tau)/(Tyr)+(Thr)/(Cit+Met+His) |
| 0.8987 | (Tau)/(His)+(Thr)/(Met+Tyr+Val) |
| 0.8983 | (Tau)/(Tyr)+(Pro+Arg)/(His+Val) |
| 0.8983 | (Tau)/(Tyr)+(Thr)/(ABA+His) |
| 0.8982 | (Tau)/(His)+(Thr)/(Met+Tyr+Leu) |
| 0.8981 | (Tau)/(His)+(Thr)/(Asn+Tyr+Val) |
| 0.8981 | (Tau)/(His)+(Pro+Arg)/(Tyr+Val) |
| 0.8981 | (Tau)/(Tyr)+(Thr)/(Met+ABA+His) |
| 0.8980 | (Tau)/(His)+(Thr)/(Cit+ABA+Val) |
| 0.8980 | (Tau)/(Cit+His)+(Thr)/(Tyr+Val) |
| 0.8980 | (Tau)/(His)+(Pro+Thr)/(Leu+Val) |
| 0.8979 | (Tau)/(His)+(Thr)/(Trp+Tyr+Val) |
| 0.8979 | (Tau)/(His)+(Pro+Thr)/(Ala+Val) |
| 0.8978 | (Tau)/(Asn+Tyr)+(Thr)/(His+Val) |
| 0.8978 | (Tau)/(Tyr)+(Arg)/(Cit+His) |
| 0.8977 | (Tau)/(Tyr)+(Thr)/(Cit+Trp+His) |
| 0.8976 | (Tau)/(His)+(Thr)/(Cit+Asn+Tyr) |
| 0.8976 | (Tau)/(Tyr)+(Pro+Thr)/(His+Leu) |
| 0.8975 | (Tau)/(His)+(Thr)/(Asn+Val) |
| 0.8975 | (Tau)/(His)+(Thr)/(Cit+Val) |
| 0.8975 | (Tau)/(Tyr)+(Pro+Arg)/(His+Leu) |
| 0.8974 | (Tau)/(His)+(Thr)/(Phe+Tyr+Leu) |
| 0.8974 | (Tau)/(His)+(Thr)/(Tyr+Leu) |
| 0.8973 | (Tau)/(Tyr)+(Pro+Thr)/(Leu+Val) |
| 0.8973 | (Tau)/(Tyr)+(Thr)/(Cit+Asn+His) |
| 0.8973 | (Tau)/(His)+(Thr)/(Trp+Tyr+Leu) |
| 0.8972 | (Tau)/(His)+(Thr)/(Cit+Asn+Val) |
| 0.8972 | (Tau)/(Tyr)+(Thr+Arg)/(His+Val) |
| 0.8971 | (Tau)/(Tyr)+(Thr)/(Asn+His) |
| 0.8971 | (Tau)/(His)+(Thr)/(Asn+ABA+Val) |
| 0.8970 | (Tau)/(His)+(Thr)/(Phe+Tyr+Val) |
| 0.8969 | (Tau)/(ABA+His)+(Thr)/(Tyr+Val) |
| 0.8969 | (Tau)/(His)+(Thr)/(Cit+Met+Val) |
| 0.8969 | (Tau)/(His)+(Thr)/(Met+Asn+Tyr) |
| 0.8969 | (Tau)/(His)+(Thr)/(Met+ABA+Val) |

# FIG.98

| ROC AUC | FORMULA |
|---|---|
| 0.8967 | (Tau)/(Met+His)+(Thr)/(Tyr+Val) |
| 0.8967 | (Tau)/(His)+(Thr)/(ABA+Val) |
| 0.8966 | (Tau)/(His)+(Thr)/(Met+Asn+Val) |
| 0.8966 | (Tau)/(Tyr)+(Thr)/(Asn+ABA+His) |
| 0.8966 | (Tau)/(Tyr)+(Pro+Thr)/(Cit+Val) |
| 0.8966 | (Tau)/(His)+(Thr)/(Cit+Trp+Val) |
| 0.8965 | (Tau)/(Tyr)+(Thr)/(His+Leu) |
| 0.8964 | (Tau)/(His)+(Thr)/(Trp+Val) |
| 0.8964 | (Tau)/(Tyr)+(Thr)/(Met+His) |
| 0.8964 | (Tau)/(His)+(Thr+Arg)/(Asn+Val) |
| 0.8964 | (Tau)/(His)+(Thr)/(Asn+ABA+Tyr) |
| 0.8963 | (Tau)/(Tyr)+(Thr+Arg)/(Met+His) |
| 0.8963 | (Tau)/(His)+(Thr)/(Met+Val) |
| 0.8962 | (Tau)/(His)+(Thr)/(Tyr+Leu+Val) |
| 0.8962 | (Tau)/(His)+(Thr+Arg)/(Leu+Val) |
| 0.8962 | (Tau)/(His)+(Pro+Arg)/(Leu+Val) |
| 0.8961 | (Tau)/(His)+(Thr)/(Trp+ABA+Val) |
| 0.8961 | (Tau)/(His)+(Thr)/(Glu+Tyr+Val) |
| 0.8960 | (Tau)/(Asn+Tyr)+(Thr)/(His+Leu) |
| 0.8959 | (Tau)/(His)+(Thr)/(Cit+Phe+Val) |
| 0.8959 | (Tau)/(His)+(Thr)/(Orn+Tyr+Val) |
| 0.8959 | (Tau)/(Tyr)+(Pro+Thr)/(Asn+Val) |
| 0.8959 | (Tau)/(His)+(Thr)/(Asn+Trp+Val) |
| 0.8957 | (Tau)/(Tyr)+(Pro+Ser)/(His+Val) |
| 0.8957 | (Tau)/(His+Tyr)+(Thr)/(Leu+Val) |
| 0.8957 | (Tau)/(His)+(Thr)/(Leu+Val) |
| 0.8957 | (Tau)/(His)+(Thr)/(Phe+Val) |
| 0.8957 | (Tau)/(His)+(Thr)/(ABA+Phe+Val) |
| 0.8957 | (Tau)/(His)+(Thr)/(Orn+Tyr+Leu) |
| 0.8955 | (Tau)/(His)+(Thr)/(Val) |
| 0.8955 | (Tau)/(His)+(Thr)/(Asn+Phe+Val) |
| 0.8955 | (Tau)/(His)+(Thr)/(ABA+Leu+Val) |
| 0.8954 | (Tau)/(Cit+Tyr)+(Thr)/(His+Val) |
| 0.8954 | (Tau)/(His+Tyr)+(Thr)/(Lys+Val) |
| 0.8954 | (Tau)/(Tyr)+(Thr)/(Cit+His+Leu) |
| 0.8952 | (Tau)/(His)+(Thr)/(Met+Trp+Val) |
| 0.8952 | (Tau)/(Tyr)+(Thr)/(Trp+ABA+His) |
| 0.8951 | (Tau)/(Tyr)+(Thr)/(ABA+His+Leu) |
| 0.8951 | (Tau)/(His)+(Thr)/(Cit+Leu+Val) |
| 0.8951 | (Tau)/(His)+(Thr+Arg)/(Cit+Val) |
| 0.8951 | (Tau)/(Tyr)+(Pro+Thr)/(Trp+Val) |
| 0.8951 | (Tau)/(Tyr)+(Pro+Arg)/(Cit+Val) |
| 0.8950 | (Tau)/(His)+(Thr)/(Met+Phe+Val) |
| 0.8949 | (Tau)/(His+Tyr)+(Thr)/(Asn+Val) |
| 0.8949 | (Tau)/(Cit+Tyr)+(Arg)/(His+Leu) |
| 0.8949 | (Tau)/(Cit+Tyr)+(Thr)/(His+Leu) |
| 0.8948 | (Tau)/(His)+(Pro+Thr)/(Asn+Val) |
| 0.8948 | (Tau)/(His)+(Thr)/(Trp+Phe+Val) |
| 0.8948 | (Tau)/(Asn+His)+(Thr)/(Cit+Val) |
| 0.8948 | (Tau)/(His)+(Thr)/(Asn+Leu+Val) |

181

# FIG.99

**FIG.100**

| ROC AUC | FORMULA |
|---|---|
| 0.9331 | (Tau)/(Tyr)+(Pro+Orn+Arg)/(Val) |
| 0.9319 | (Tau)/(Tyr)+(Pro+Thr)/(His+Val) |
| 0.9312 | (Tau)/(Tyr)+(Gly+Pro)/(His+Val) |
| 0.9311 | (Tau)/(Tyr)+(Pro+Orn+Glu)/(Val) |
| 0.9306 | (Tau)/(Tyr)+(Pro+Thr)/(His+Leu) |
| 0.9306 | (Tau)/(Tyr)+(Pro+Thr)/(Cit+Val) |
| 0.9304 | (Tau)/(His)+(Pro+Arg)/(Tyr+Val) |
| 0.9303 | (Tau)/(Tyr)+(Pro+Thr+Glu)/(Val) |
| 0.9302 | (Tau)/(Tyr)+(Pro+Arg)/(His+Leu) |
| 0.9302 | (Tau)/(Tyr)+(Pro+Glu+Arg)/(Val) |
| 0.9299 | (Tau)/(Tyr)+(Pro+Thr)/(Trp+Val) |
| 0.9298 | (Tau)/(Tyr)+(Pro+Orn)/(His+Leu) |
| 0.9298 | (Tau)/(Tyr)+(Pro+Orn)/(His+Val) |
| 0.9298 | (Tau)/(Tyr)+(Pro+Orn)/(Cit+Val) |
| 0.9296 | (Tau)/(Tyr)+(Pro+Orn)/(ABA+Val) |
| 0.9296 | (Tau)/(His)+(Pro+Thr)/(Tyr+Val) |
| 0.9296 | (Tau)/(Tyr)+(Pro+Thr+Orn)/(Val) |
| 0.9293 | (Tau)/(Tyr)+(Pro+Thr)/(ABA+Val) |
| 0.9291 | (Tau)/(Tyr)+(Pro+Thr)/(Asn+Val) |
| 0.9291 | (Tau)/(His)+(Pro+Orn)/(Tyr+Val) |
| 0.9288 | (Tau)/(Tyr)+(Pro+Orn)/(Trp+Val) |
| 0.9287 | (Tau)/(Tyr)+(Pro+Orn)/(Val) |
| 0.9285 | (Tau)/(Tyr)+(Orn+Arg)/(Cit+His) |
| 0.9284 | (Tau)/(Tyr)+(Pro+Arg)/(His+Val) |
| 0.9282 | (Tau)/(Tyr)+(Pro+Arg)/(Cit+Val) |
| 0.9281 | (Tau)/(Tyr)+(Gly+Glu)/(His+Leu) |
| 0.9281 | (Gly)/(His+ABA)+(Tau+Glu)/(Tyr) |
| 0.9280 | (Tau)/(Tyr)+(Pro+Orn)/(Met+Val) |
| 0.9280 | (Tau)/(Tyr)+(Pro+Orn)/(Phe+Val) |
| 0.9279 | (Tau)/(Tyr)+(Pro+Thr)/(Phe+Val) |
| 0.9278 | (Tau)/(Tyr)+(Pro+Orn)/(Asn+Val) |
| 0.9277 | (Tau)/(Tyr)+(Pro+Thr)/(Met+Val) |
| 0.9275 | (Tau)/(Tyr)+(Pro+Thr)/(Leu+Val) |
| 0.9275 | (Tau)/(Tyr)+(Pro+Arg)/(ABA+Val) |
| 0.9274 | (Tau)/(Tyr)+(Pro+Ser)/(His+Val) |
| 0.9273 | (Tau)/(His)+(Gly+Glu)/(Tyr+Val) |
| 0.9272 | (Tau)/(Tyr)+(Pro+Arg)/(Trp+Val) |
| 0.9272 | (Tau)/(Tyr)+(Pro+Arg)/(Val) |
| 0.9270 | (Tau)/(Tyr)+(Pro+Thr)/(Val) |
| 0.9269 | (Tau)/(His)+(Pro+Arg)/(Leu+Val) |
| 0.9269 | (Tau)/(His)+(Gly+Pro)/(Tyr+Val) |
| 0.9269 | (Tau)/(His)+(Pro+Ser)/(Tyr+Val) |
| 0.9268 | (Tau+Glu)/(Tyr)+(Gly+Pro)/(Val) |
| 0.9266 | (Tau)/(Tyr)+(Pro+Orn)/(Leu+Val) |
| 0.9264 | (Tau)/(His)+(Pro+Orn)/(Trp+Val) |
| 0.9262 | (Tau)/(His)+(Pro+Thr)/(Leu+Val) |
| 0.9262 | (Tau)/(Tyr)+(Pro+Ser+Glu)/(Val) |
| 0.9261 | (Tau)/(His)+(Pro+Arg)/(Trp+Val) |
| 0.9259 | (Tau)/(Tyr)+(Pro+Arg)/(Asn+Val) |
| 0.9259 | (Tau)/(Tyr)+(Pro+Thr+Arg)/(Val) |

## FIG.101

| ROC AUC | FORMULA |
|---|---|
| 0.9256 | (Tau)/(His)+(Pro+Thr)/(Trp+Val) |
| 0.9254 | (Tau)/(Tyr)+(Pro+Orn)/(Trp+Leu) |
| 0.9252 | (Tau)/(His)+(Pro+Orn+Arg)/(Val) |
| 0.9251 | (Tau)/(Tyr)+(Pro+Ser+Orn)/(Val) |
| 0.9251 | (Tau)/(Tyr)+(Pro+Arg)/(Met+Val) |
| 0.9251 | (Tau)/(Tyr)+(Gly+Glu)/(His+ABA) |
| 0.9250 | (Tau)/(Tyr)+(Pro+Arg)/(Trp+Leu) |
| 0.9249 | (Tau)/(Tyr)+(Pro+Ser)/(His+Leu) |
| 0.9248 | (Tau)/(Tyr)+(Gly+Glu)/(His+Val) |
| 0.9248 | (Tau)/(Tyr)+(Gly+Glu)/(Phe+His) |
| 0.9245 | (Tau)/(Tyr)+(Pro+Orn)/(Phe+Leu) |
| 0.9245 | (Tau)/(Tyr)+(Pro+Glu)/(His+Leu) |
| 0.9244 | (Tau)/(Tyr)+(Pro+Glu)/(ABA+Val) |
| 0.9244 | (Tau)/(Tyr)+(Gly+Pro)/(His+Leu) |
| 0.9242 | (Tau)/(His)+(Pro+Thr)/(Asn+Val) |
| 0.9242 | (Tau)/(Tyr)+(Pro+Glu)/(His+Val) |
| 0.9241 | (Tau)/(Tyr)+(Pro)/(His+ABA+Leu) |
| 0.9240 | (Tau)/(Tyr)+(Pro+Ser)/(Trp+Val) |
| 0.9240 | (Tau)/(Tyr)+(Pro+Arg)/(Phe+Val) |
| 0.9237 | (Tau)/(ABA+Tyr)+(Pro+Orn)/(Val) |
| 0.9237 | (Tau)/(Tyr)+(Thr+Glu)/(ABA+Leu) |
| 0.9236 | (Tau)/(Tyr)+(Pro+Glu)/(Val) |
| 0.9236 | (Tau)/(Asn)+(Gly+Pro)/(His+Tyr) |
| 0.9235 | (Tau)/(Tyr)+(Pro+Ser)/(ABA+Val) |
| 0.9235 | (Tau)/(Tyr)+(Pro+Glu)/(Trp+Val) |
| 0.9235 | (Tau)/(Tyr)+(Pro+Arg)/(Leu+Val) |
| 0.9234 | (Tau)/(Phe)+(Gly+Glu)/(His+Tyr) |
| 0.9233 | (Tau)/(Tyr)+(Gly+Pro)/(Leu+Val) |
| 0.9233 | (Tau)/(Tyr)+(Thr+Glu)/(His+Leu) |
| 0.9233 | (Tau)/(His)+(Pro+Orn)/(Cit+Val) |
| 0.9232 | (Tau)/(Tyr)+(Pro)/(His+Leu) |
| 0.9231 | (Tau)/(Tyr)+(Gly+Glu)/(Cit+His) |
| 0.9230 | (Gly)/(Cit+His)+(Tau+Glu)/(Tyr) |
| 0.9230 | (Tau)/(Tyr)+(Pro+Orn)/(Asn+Leu) |
| 0.9230 | (Tau)/(His)+(Pro+Thr)/(Phe+Val) |
| 0.9229 | (Tau)/(Tyr)+(Pro+Ser)/(Asn+Val) |
| 0.9229 | (Tau)/(Tyr)+(Pro+Glu)/(Cit+Val) |
| 0.9228 | (Tau)/(His)+(Gly+Pro)/(Asn+Val) |
| 0.9227 | (Arg)/(Val)+(Tau+Orn)/(His+Tyr) |
| 0.9227 | (Tau)/(Tyr)+(Gly+Pro)/(Phe+Val) |
| 0.9227 | (Arg)/(Val)+(Tau+Orn)/(Cit+Tyr) |
| 0.9227 | (Tau)/(Tyr)+(Pro+Ser)/(Cit+Val) |
| 0.9226 | (Tau)/(Tyr)+(Gly+Pro)/(Asn+Val) |
| 0.9226 | (Tau)/(Tyr)+(Thr+Orn+Glu)/(Val) |
| 0.9226 | (Tau)/(Tyr)+(Orn+Arg)/(Trp+His) |
| 0.9226 | (Gly)/(His+Leu)+(Tau+Glu)/(Tyr) |
| 0.9225 | (Tau)/(His)+(Pro+Orn)/(Phe+Val) |
| 0.9225 | (Tau)/(His)+(Pro+Arg)/(Cit+Val) |
| 0.9225 | (Tau)/(His)+(Gly+Orn)/(Tyr+Val) |
| 0.9224 | (Tau)/(Tyr)+(Pro+Ser)/(Phe+Val) |

**FIG.102**

| ROC AUC | FORMULA |
|---|---|
| 0.9224 | (Tau)/(Tyr)+(Glu+Pro)/(Asn+Val) |
| 0.9222 | (Tau)/(Tyr)+(Glu+Pro)/(Leu+Val) |
| 0.922 | (Tau)/(Tyr)+(Glu+Pro)/(Phe+Val) |
| 0.922 | (Tau)/(Tyr)+(Pro)/(Asn+Leu+His) |
| 0.9218 | (Tau)/(Tyr)+(Glu+Pro)/(Met+Val) |
| 0.9199 | (Tau)/(His)+(Glu+Pro+Orn)/(Val) |
| 0.9196 | (Tau)/(Tyr)+(Glu+Pro)/(Phe+Leu) |
| 0.9178 | (Tau)/(Tyr)+(Glu+Pro)/(Asn+Leu) |
| 0.9156 | (Tau)/(His)+(Glu+Pro)/(Trp+Val) |
| 0.9148 | (Tau)/(His)+(Glu+Pro)/(Leu+Val) |
| 0.9104 | (Tau)/(His)+(Glu+Pro)/(Phe+Val) |
| 0.9103 | (Tau)/(His)+(Glu+Pro)/(Asn+Val) |
| 0.9102 | (Tau)/(Tyr+His)+(Glu+Orn)/(Val) |
| 0.9077 | (Tau)/(Tyr)+(Glu+Pro)/(Ser+Leu) |
| 0.9055 | (Gly)/(Ser+His)+(Tau+Glu)/(Tyr) |
| 0.9018 | (Pro)/(Cit+Leu)+(Tau+Glu)/(Tyr) |
| 0.9015 | (Gly)/(Ser+Val)+(Tau+Glu)/(Tyr) |
| 0.9005 | (Tau)/(Tyr)+(Glu+Orn)/(Cit+His) |
| 0.8995 | (Pro)/(Trp+Leu)+(Tau+Glu)/(Tyr) |
| 0.899 | (Pro)/(Phe+Leu)+(Tau+Glu)/(Tyr) |
| 0.8984 | (Pro)/(Asn+Leu)+(Tau+Glu)/(Tyr) |
| 0.8982 | (Tau)/(His)+(Glu+Pro)/(Ser+Val) |
| 0.8969 | (Pro)/(Met+Val)+(Tau+Glu)/(Tyr) |
| 0.8966 | (Gly)/(Ser+Leu)+(Tau+Glu)/(His) |
| 0.8957 | (Pro)/(Phe+Val)+(Tau+Glu)/(Tyr) |
| 0.8953 | (Pro)/(Asn+Val)+(Tau+Glu)/(Tyr) |
| 0.8945 | (Pro)/(Leu+Val)+(Tau+Glu)/(Tyr) |
| 0.8936 | (Gly)/(Ser+Tyr)+(Tau+Glu)/(His) |
| 0.8935 | (Pro)/(Ile+Val)+(Tau+Glu)/(Tyr) |
| 0.8933 | (Tau+Glu)/(Tyr)+(Pro+Ala)/(Val) |
| 0.8932 | (Ala)/(Leu+Val)+(Tau+Glu)/(Tyr) |
| 0.8932 | (Ala)/(Val+His)+(Tau+Glu)/(Tyr) |
| 0.8918 | (Tau)/(Tyr)+(Glu+Pro)/(Asn+His) |
| 0.8911 | (Pro)/(Val)+(Tau+Glu)/(Asn+Tyr) |
| 0.8902 | (Pro)/(Trp+His)+(Tau+Glu)/(Tyr) |
| 0.8899 | (Ala)/(Trp+Val)+(Tau+Glu)/(Tyr) |
| 0.8894 | (Tau+Glu)/(His)+(Pro+Orn)/(Val) |
| 0.8869 | (Ala)/(Phe+Val)+(Tau+Glu)/(Tyr) |
| 0.886 | (Tau)/(Met)+(Pro+Ala)/(Tyr+His) |
| 0.8856 | (Ala)/(Asn+Val)+(Tau+Glu)/(Tyr) |
| 0.8847 | (Pro)/(Asn+His)+(Tau+Glu)/(Tyr) |
| 0.8844 | (Ala)/(Cit+Val)+(Tau+Glu)/(Tyr) |
| 0.8838 | (Ala)/(Leu+His)+(Tau+Glu)/(Tyr) |
| 0.8832 | (Tau+Glu)/(Tyr)+(Pro+Gly)/(Gln) |
| 0.883 | (Gly)/(Gln)+(Tau+Glu)/(Tyr+His) |
| 0.8817 | (Pro)/(Ser+Leu)+(Tau+Glu)/(Tyr) |
| 0.875 | (Ala)/(Ser+Val)+(Tau+Glu)/(Tyr) |
| 0.8747 | (Pro)/(Ser+His)+(Tau+Glu)/(Tyr) |
| 0.8667 | (Tau+Glu)/(Tyr)+(Gly+Ala)/(Gln) |
| 0.8588 | (Ala)/(Tyr+Val)+(Tau+Glu)/(His) |

# FIG.103

# FIG.104

| ROC AUC | FORMULA |
|---|---|
| 0.8936 | (Tau)/(Tyr)+(Thr)/(Cit+His) |
| 0.8935 | (Tau)/(His)+(Thr)/(Cit+Asn+Tyr) |
| 0.8919 | (Tau)/(His)+(Thr)/(Asn+Leu+Tyr) |
| 0.8916 | (Tau)/(His)+(Thr+Pro)/(Ala+Val) |
| 0.8915 | (Tau)/(His)+(Thr)/(Asn+Met+Tyr) |
| 0.8914 | (Tau)/(His)+(Thr)/(Cit+Leu+Tyr) |
| 0.8909 | (Tau)/(His)+(Thr)/(Met+Leu+Tyr) |
| 0.8904 | (Tau)/(Tyr)+(Thr)/(ABA+Cit+His) |
| 0.8903 | (Tau)/(His)+(Thr)/(Val+Tyr) |
| 0.8902 | (Tau)/(His)+(Thr)/(Cit+Val+Tyr) |
| 0.8899 | (Tau)/(His)+(Thr+Pro)/(Ala+Leu) |
| 0.8899 | (Tau)/(His)+(Thr)/(Met+Val+Tyr) |
| 0.8898 | (Tau)/(His)+(Thr)/(Orn+Leu+Tyr) |
| 0.8898 | (Tau)/(His)+(Thr)/(ABA+Val+Tyr) |
| 0.8896 | (Tau)/(His)+(Thr)/(Orn+Asn+Tyr) |
| 0.8894 | (Tau)/(Cit+His)+(Thr)/(Val+Tyr) |
| 0.8893 | (Tau)/(His)+(Thr)/(Asn+Val+Tyr) |
| 0.8892 | (Tau)/(Tyr)+(Thr)/(Cit+Met+His) |
| 0.8889 | (Tau)/(His)+(Thr)/(ABA+Leu+Tyr) |
| 0.8887 | (Tau)/(His)+(Thr)/(Asn+Met+Val) |
| 0.8887 | (Tau)/(His)+(Thr)/(Asn+Val) |
| 0.8886 | (Tau)/(His)+(Thr)/(Cit+Met+Tyr) |
| 0.8885 | (Tau)/(His)+(Thr)/(Cit+Asn+Val) |
| 0.8883 | (Tau)/(His)+(Thr)/(Orn+Val+Tyr) |
| 0.8883 | (Tau)/(His)+(Thr)/(Cit+Met+Val) |
| 0.8882 | (Tau)/(His)+(Thr)/(Val+Leu+Tyr) |
| 0.8882 | (Tau)/(Asn+His)+(Thr)/(Val+Tyr) |
| 0.8881 | (Tau)/(Met+His)+(Thr)/(Val+Tyr) |
| 0.8881 | (Tau)/(His)+(Thr)/(Phe+Leu+Tyr) |
| 0.8880 | (Tau)/(His)+(Thr)/(Cit+Val) |
| 0.8880 | (Tau)/(His)+(Thr)/(Trp+Val+Tyr) |
| 0.8880 | (Tau)/(His+Tyr)+(Thr)/(Ala+Val) |
| 0.8880 | (Tau)/(Tyr)+(Thr)/(Cit+Asn+His) |
| 0.8879 | (Tau)/(His)+(Thr)/(ABA+Cit+Val) |
| 0.8879 | (Tau)/(Met+Tyr)+(Thr)/(Cit+His) |
| 0.8879 | (Tau)/(His)+(Thr)/(Phe+Val+Tyr) |
| 0.8878 | (Tau)/(His)+(Thr)/(Leu+Tyr) |
| 0.8878 | (Tau)/(His)+(Thr)/(Trp+Leu+Tyr) |
| 0.8875 | (Tau)/(His)+(Thr)/(Cit+Asn+Leu) |
| 0.8875 | (Tau)/(His)+(Thr)/(ABA+Asn+Val) |
| 0.8874 | (Tau)/(Asn+Tyr)+(Thr)/(His+Val) |
| 0.8874 | (Tau)/(His)+(Thr)/(Asn+Val+Leu) |
| 0.8873 | (Tau)/(Asn+Met)+(Thr)/(Cit+His) |
| 0.8873 | (Tau)/(His)+(Thr)/(Orn+Cit+Val) |
| 0.8872 | (Tau)/(His)+(Thr)/(ABA+Met+Val) |
| 0.8871 | (Tau)/(Tyr)+(Thr)/(Asn+His) |
| 0.8870 | (Tau)/(His)+(Thr)/(Arg+Leu+Tyr) |
| 0.8870 | (Tau)/(His)+(Thr)/(Cit+Val+Leu) |
| 0.8870 | (Tau)/(His)+(Thr)/(Met+Val+Leu) |
| 0.8869 | (Tau)/(His)+(Thr)/(Val+Leu) |

FIG.105

| ROC AUC | FORMULA |
|---|---|
| 0.8869 | (Tau)/(His)+(Thr)/(Met+Val) |
| 0.8869 | (Tau)/(His)+(Thr)/(Trp+Cit+Val) |
| 0.8868 | (Tau)/(His)+(Thr)/(Cit+Phe+Val) |
| 0.8868 | (Tau)/(His)+(Thr)/(ABA+Val+Leu) |
| 0.8868 | (Tau)/(Tyr)+(Thr)/(Trp+Cit+His) |
| 0.8868 | (Tau)/(His)+(Thr)/(Orn+Met+Val) |
| 0.8867 | (Tau)/(His)+(Thr)/(ABA+Asn+Tyr) |
| 0.8867 | (Tau)/(His)+(Thr)/(Asn+Met+Leu) |
| 0.8866 | (Tau)/(His)+(Thr)/(Asn+Phe+Val) |
| 0.8866 | (Tau)/(Cit+Tyr)+(Thr)/(His+Leu) |
| 0.8866 | (Tau)/(His)+(Thr)/(Glu+Val+Tyr) |
| 0.8866 | (Tau)/(His)+(Thr)/(Arg+Val+Tyr) |
| 0.8865 | (Tau)/(His)+(Thr)/(Trp+Asn+Val) |
| 0.8864 | (Tau)/(Cit+Tyr)+(Thr)/(His+Val) |
| 0.8864 | (Tau)/(Met+Tyr)+(Thr)/(Asn+His) |
| 0.8864 | (Tau)/(His)+(Thr)/(Met+Phe+Val) |
| 0.8863 | (Tau)/(His)+(Thr)/(Ile+Val+Tyr) |
| 0.8863 | (Tau)/(His)+(Thr)/(Asn+Tyr) |
| 0.8863 | (Tau)/(His)+(Thr)/(Orn+Asn+Val) |
| 0.8863 | (Tau)/(Cit+Asn)+(Thr)/(His+Tyr) |
| 0.8863 | (Tau)/(His)+(Thr)/(Orn+Val) |
| 0.8863 | (Tau)/(Asn+Tyr)+(Thr)/(His+Leu) |
| 0.8861 | (Tau)/(Tyr)+(Thr)/(Met+His) |
| 0.8861 | (Tau)/(His)+(Thr)/(Orn+Val+Leu) |
| 0.8860 | (Tau)/(His)+(Thr)/(Trp+Met+Val) |
| 0.8860 | (Tau)/(Met+His)+(Thr)/(Asn+Val) |
| 0.8860 | (Tau)/(His)+(Thr)/(Trp+Val) |
| 0.8860 | (Tau)/(His)+(Thr)/(Trp+Val+Leu) |
| 0.8860 | (Tau)/(His)+(Thr)/(Orn+ABA+Val) |
| 0.8859 | (Tau)/(His)+(Thr)/(Phe+Val+Leu) |
| 0.8859 | (Tau)/(Met+His)+(Thr)/(Val+Leu) |
| 0.8859 | (Tau)/(His)+(Thr)/(Orn+Met+Tyr) |
| 0.8859 | (Tau)/(His)+(Thr)/(Orn+Asn+Leu) |
| 0.8858 | (Tau)/(His+Tyr)+(Thr)/(Ala+Leu) |
| 0.8858 | (Tau)/(Cit+His)+(Thr)/(Asn+Val) |
| 0.8858 | (Tau)/(His)+(Thr)/(ABA+Val) |
| 0.8856 | (Tau)/(His)+(Thr)/(Phe+Val) |
| 0.8856 | (Tau)/(His)+(Thr)/(Glu+Asn+Val) |
| 0.8856 | (Tau)/(His)+(Thr)/(Lys+Leu+Tyr) |
| 0.8856 | (Tau)/(His)+(Thr)/(Glu+Cit+Val) |
| 0.8856 | (Tau)/(Asn+Met)+(Thr)/(His+Tyr) |
| 0.8855 | (Tau)/(His)+(Thr)/(ABA+Phe+Val) |
| 0.8855 | (Tau)/(Cit+Tyr)+(Thr)/(Asn+His) |
| 0.8854 | (Tau)/(Cit+His)+(Thr)/(Val+Leu) |
| 0.8854 | (Tau)/(Met+His)+(Thr)/(Orn+Val) |
| 0.8854 | (Tau)/(His)+(Thr)/(Trp+ABA+Val) |
| 0.8854 | (Tau)/(His)+(Thr+Pro)/(Ala+Lys) |
| 0.8854 | (Tau)/(His)+(Thr+Pro)/(Ala+Tyr) |
| 0.8853 | (Tau)/(Met+His)+(Thr)/(Cit+Val) |
| 0.8852 | (Tau)/(His)+(Thr)/(Orn+Phe+Val) |

# FIG.106

| ROC AUC | FORMULA |
|---|---|
| 0.8701 | (Tau)/(Tyr+His)+(Glu)/(Val+Leu) |
| 0.8651 | (Tau)/(Tyr+His)+(Glu)/(Orn+Val) |
| 0.8645 | (Tau)/(Tyr+His)+(Glu)/(Trp+Val) |
| 0.8641 | (Tau)/(Tyr+His)+(Glu)/(Cit+Val) |
| 0.8631 | (Tau)/(Tyr+His)+(Glu)/(Val+Asn) |
| 0.8629 | (Tau)/(Tyr+His)+(Glu)/(Val+Met) |
| 0.8623 | (Tau)/(His)+(Glu)/(Orn+Leu) |
| 0.8616 | (Tau)/(Tyr+His)+(Glu)/(Val) |
| 0.8578 | (Tau)/(Cit+Tyr)+(Glu)/(Leu+His) |
| 0.8553 | (Tau)/(His)+(Glu)/(Orn+Tyr+Asn) |
| 0.855 | (Tau)/(Met+His)+(Glu)/(Tyr+Leu) |
| 0.8548 | (Tau)/(Tyr)+(Glu)/(Cit+His) |
| 0.8534 | (Tau)/(His)+(Glu)/(Orn+Cit+Tyr) |
| 0.8532 | (Tau)/(His)+(Glu)/(Orn+Tyr+Met) |
| 0.8511 | (Tau)/(Met+His)+(Glu)/(Orn+Leu) |
| 0.8506 | (Tau)/(Cit+Tyr)+(Glu)/(Orn+Leu) |
| 0.8501 | (Tau)/(Tyr+Met)+(Glu)/(Cit+Leu) |
| 0.8491 | (Tau)/(Tyr+His)+(Glu)/(Orn+Leu) |
| 0.8488 | (Tau)/(Tyr+His)+(Glu)/(Ile+Leu) |
| 0.8485 | (Tau)/(Cit+Tyr)+(Glu)/(Leu+Met) |
| 0.8481 | (Tau)/(Cit+Tyr)+(Glu)/(Leu) |
| 0.8461 | (Tau)/(His)+(Glu)/(Orn+Tyr) |
| 0.8461 | (Tau)/(Cit+His)+(Glu)/(Orn+Leu) |
| 0.8456 | (Tau)/(Met+His)+(Glu)/(Cit+Leu) |
| 0.8435 | (Tau)/(Tyr+His)+(Glu)/(Cit+Leu) |
| 0.8434 | (Tau)/(Asn+His)+(Glu)/(Tyr+Leu) |
| 0.8431 | (Tau)/(Tyr+His)+(Glu)/(Asn+Leu) |
| 0.8422 | (Tau)/(Tyr+His)+(Glu)/(Leu+Met) |
| 0.8417 | (Tau)/(Orn+His)+(Glu)/(Tyr+Leu) |
| 0.8408 | (Tau+Glu)/(Tyr+Leu+His) |
| 0.8404 | (Tau)/(Orn+Tyr+His)+(Glu)/(Val) |
| 0.84 | (Tau)/(Tyr+Asn)+(Glu)/(Cit+Leu) |
| 0.8377 | (Tau)/(Asn+His)+(Glu)/(Orn+Leu) |
| 0.8368 | (Tau+Glu)/(Tyr+Leu+Met+His) |
| 0.8368 | (Tau+Glu)/(Cit+Tyr+Leu+His) |
| 0.8364 | (Tau)/(Tyr+Asn)+(Glu)/(Leu) |
| 0.835 | (Tau)/(Tyr+His)+(Glu)/(Leu) |
| 0.8337 | (Tau+Glu)/(Cit+Tyr+Leu) |
| 0.8332 | (Tau)/(Tyr)+(Glu)/(Orn+Cit) |
| 0.8326 | (Tau+Glu)/(Orn+Tyr+Leu+His) |
| 0.8294 | (Tau)/(Orn+His)+(Glu)/(Leu+Met) |
| 0.8282 | (Tau)/(Orn+Tyr)+(Glu)/(Leu) |
| 0.827 | (Tau+Glu)/(Orn+Leu+His) |
| 0.827 | (Tau)/(Orn+His)+(Glu)/(Cit+Leu) |
| 0.825 | (Tau+Glu)/(Orn+Leu+Met+His) |
| 0.8243 | (Tau+Glu)/(Orn+Tyr+Leu) |
| 0.8231 | (Tau+Glu)/(Orn+Cit+Leu+His) |
| 0.823 | (Tau+Glu)/(Orn+Cit+Tyr+Leu) |
| 0.8219 | (Tau+Glu)/(Orn+Tyr+Leu+Met) |
| 0.8176 | (Tau+Glu)/(Orn+Tyr+Asn+Leu) |

## FIG.107

# FIG.108

| ROC AUC | FORMULA |
|---|---|
| 0.7047 | (Met+Ile)/(Val+Trp+ABA) |
| 0.7043 | (Met)/(Trp+ABA)+(Ile+Glu)/(Val) |
| 0.7036 | (Met)/(Trp+ABA)+(Ile)/(Val) |
| 0.7036 | (Met)/(His+Trp+ABA)+(Ile)/(Val) |
| 0.7035 | (Met)/(His+Trp)+(Ile)/(Val+ABA) |
| 0.7033 | (Pro)/(Val)+(Met+Ile)/(Trp+ABA) |
| 0.7001 | (Met+Ile)/(ABA)+(Pro+Orn)/(Trp) |
| 0.6993 | (Met)/(Tyr+Trp+ABA)+(Ile)/(Val) |
| 0.6986 | (Met+Ile)/(Val+Trp) |
| 0.6979 | (Ile)/(Trp+ABA)+(Pro+Orn)/(Val) |
| 0.6977 | (Ile)/(Trp+ABA)+(Met+Pro)/(Val) |
| 0.6975 | (Met)/(Trp+ABA)+(Ile)/(His+Val) |
| 0.6974 | (Met)/(His+ABA)+(Ile)/(Val+Trp) |
| 0.6972 | (Met)/(Trp)+(Pro+Ile)/(Val+ABA) |
| 0.6970 | (Met+Ile)/(His+Val+Trp+ABA) |
| 0.6970 | (Met)/(Trp+ABA)+(Ile)/(Val+Tyr) |
| 0.6967 | (Pro)/(Val)+(Orn+Ile)/(Trp+ABA) |
| 0.6966 | (Met+Ile)/(ABA)+(Pro+Thr)/(Trp) |
| 0.6965 | (Met)/(Trp+ABA)+(Glu+Leu)/(Val) |
| 0.6962 | (Met)/(Trp)+(Pro+Ile+Glu)/(Val) |
| 0.6962 | (Met)/(Trp)+(Pro+Ile)/(His+Val) |
| 0.6960 | (Met)/(His+Trp)+(Ile)/(Val+Tyr) |
| 0.6958 | (Met)/(ABA)+(Pro+Glu)/(Tyr+Trp) |
| 0.6957 | (Met)/(Trp+ABA)+(Ile+Leu)/(Val) |
| 0.6956 | (Met+Ile)/(ABA)+(Pro+Glu)/(Trp) |
| 0.6954 | (Ile)/(Trp+ABA)+(Pro+Glu)/(Val) |
| 0.6949 | (Met)/(Trp+ABA)+(Pro+Ile)/(Val) |
| 0.6949 | (Ile)/(ABA)+(Met+Pro+Orn)/(Trp) |
| 0.6948 | (Met+Pro+Orn+Ile)/(Trp+ABA) |
| 0.6947 | (Met)/(ABA)+(Pro+Glu)/(His+Trp) |
| 0.6945 | (Met)/(ABA)+(Pro+Orn)/(Tyr+Trp) |
| 0.6944 | (Met)/(His)+(Pro+Ile)/(Val+Trp) |
| 0.6942 | (Pro)/(Val+Tyr)+(Ile)/(Trp+ABA) |
| 0.6940 | (Met)/(Tyr)+(Pro+Orn)/(Trp+ABA) |
| 0.6940 | (Ile)/(ABA)+(Pro+Orn+Glu)/(Trp) |
| 0.6940 | (Met)/(His+Tyr+Trp)+(Ile)/(Val) |
| 0.6939 | (Met)/(Trp)+(Pro+Leu)/(His+Val) |
| 0.6938 | (Met+Ile)/(Val+Tyr+Trp+ABA) |
| 0.6938 | (Ile)/(ABA)+(Pro+Orn)/(Trp) |
| 0.6938 | (Met+Pro+Orn+Glu)/(Trp+ABA) |
| 0.6937 | (Pro)/(His+Val)+(Ile)/(Trp+ABA) |
| 0.6936 | (Met)/(Trp)+(Pro+Leu)/(Val+ABA) |
| 0.6932 | (Ile)/(ABA)+(Met+Pro+Thr)/(Trp) |
| 0.6932 | (Pro)/(Val)+(Ile)/(Trp+ABA) |
| 0.6930 | (Ile)/(ABA)+(Pro+Thr)/(Trp) |
| 0.6929 | (Met)/(Trp+ABA)+(Orn+Ile)/(Val) |
| 0.6929 | (Pro)/(Val)+(Met+Orn)/(Trp+ABA) |
| 0.6929 | (Pro+Glu)/(Trp)+(Orn+Ile)/(ABA) |
| 0.6928 | (Met)/(Trp)+(Pro+Ile+Leu)/(Val) |
| 0.6926 | (Met)/(His)+(Pro+Leu)/(Val+Trp) |

# FIG.109

| ROC AUC | FORMULA |
|---------|---------|
| 0.6926 | (Pro+Orn+Ile+Glu)/(Trp+ABA) |
| 0.6926 | (Met)/(Trp)+(Pro+Glu)/(Val+ABA) |
| 0.6925 | (Ile)/(ABA)+(Pro+Thr+Glu)/(Trp) |
| 0.6925 | (Ile)/(Trp+ABA)+(Pro+Thr)/(Val) |
| 0.6925 | (Met+Orn)/(ABA)+(Pro+Glu)/(Trp) |
| 0.6925 | (Met)/(ABA)+(Pro+Orn)/(His+Trp) |
| 0.6924 | (Met+Orn)/(ABA)+(Pro+Ile)/(Trp) |
| 0.6924 | (Met)/(Trp)+(Pro+Ile)/(Val+Tyr) |
| 0.6924 | (Met)/(Trp)+(Pro+Glu+Leu)/(Val) |
| 0.6922 | (Met+Ile)/(ABA)+(Pro+Asn)/(Trp) |
| 0.6922 | (Met)/(Trp+ABA)+(Pro)/(His+Val) |
| 0.6921 | (Met)/(His)+(Pro+Orn)/(Trp+ABA) |
| 0.6920 | (Ile)/(Trp+ABA)+(Pro+Phe)/(Val) |
| 0.6920 | (Ile)/(ABA)+(Met+Pro+Glu)/(Trp) |
| 0.6919 | (Met+Pro+Orn)/(Trp+ABA) |
| 0.6918 | (Pro+Orn+Ile)/(Trp+ABA) |
| 0.6918 | (Orn)/(His)+(Met+Pro)/(Trp+ABA) |
| 0.6918 | (Met)/(ABA)+(Pro+Ile)/(Tyr+Trp) |
| 0.6917 | (Ile)/(ABA)+(Met+Pro)/(Trp) |
| 0.6917 | (Met)/(His+Tyr+ABA)+(Ile)/(Val) |
| 0.6915 | (Met)/(Trp)+(Pro+Ile)/(Val) |
| 0.6915 | (Ile)/(Trp+ABA)+(Pro+Asn)/(Val) |
| 0.6915 | (Ile)/(Tyr)+(Pro+Orn)/(Trp+ABA) |
| 0.6914 | (Orn)/(Tyr)+(Pro+Glu)/(Trp+ABA) |
| 0.6914 | (Glu)/(His)+(Pro+Orn)/(Trp+ABA) |
| 0.6914 | (Met)/(Trp)+(Pro+Orn+Ile)/(Val) |
| 0.6914 | (Orn)/(His)+(Pro+Ile)/(Trp+ABA) |
| 0.6913 | (Pro+Thr)/(Trp)+(Orn+Ile)/(ABA) |
| 0.6913 | (Ile)/(Val)+(Met+Pro)/(Trp+ABA) |
| 0.6912 | (Glu)/(Tyr)+(Pro+Orn)/(Trp+ABA) |
| 0.6912 | (Ile)/(Val)+(Pro+Orn)/(Trp+ABA) |
| 0.6912 | (Met)/(Tyr)+(Pro+Ile)/(Trp+ABA) |
| 0.6911 | (Met)/(His+ABA)+(Ile)/(Val) |
| 0.6911 | (Glu)/(Val)+(Pro+Orn)/(Trp+ABA) |
| 0.6911 | (Thr)/(Val)+(Pro+Ile)/(Trp+ABA) |
| 0.6909 | (Pro)/(ABA)+(Orn+Thr+Glu)/(Trp) |
| 0.6909 | (Ile)/(ABA)+(Pro+Orn+Thr)/(Trp) |
| 0.6909 | (Met+Pro+Ile+Glu)/(Trp+ABA) |
| 0.6908 | (Pro+Thr)/(Trp)+(Ile+Glu)/(ABA) |
| 0.6907 | (Met+Pro+Thr+Ile)/(Trp+ABA) |
| 0.6906 | (Met)/(Trp)+(Pro+Glu)/(His+Val) |
| 0.6906 | (Met)/(His+Tyr)+(Ile)/(Val+Trp) |
| 0.6906 | (Ile)/(ABA)+(Pro+Thr+Cit)/(Trp) |
| 0.6905 | (Met+Ile)/(Val+ABA) |
| 0.6905 | (Ile)/(ABA)+(Pro+Orn+Asn)/(Trp) |
| 0.6905 | (Tau)/(Val)+(Pro+Orn)/(Trp+ABA) |
| 0.6905 | (Orn)/(Val)+(Met+Pro)/(Trp+ABA) |
| 0.6905 | (Ile)/(Tyr)+(Pro+Thr)/(Trp+ABA) |
| 0.6904 | (Ile)/(ABA)+(Pro+Glu)/(Trp) |
| 0.6904 | (Orn)/(Tyr)+(Pro+Ile)/(Trp+ABA) |

# FIG.110

# FIG.111

| ROC AUC | FORMULA |
|---|---|
| 0.8254 | (Thr)/(Val)+(Met+Tyr)/(ABA+Trp) |
| 0.8198 | (Met)/(ABA+Trp)+(Tyr)/(Val+His) |
| 0.8193 | (Tyr)/(ABA)+(Thr+Tau+Orn)/(Trp) |
| 0.8179 | (Met)/(His)+(Thr+Tyr)/(ABA+Trp) |
| 0.8170 | (Orn)/(ABA)+(Thr+Tau+Tyr)/(Trp) |
| 0.8170 | (Thr)/(His+ABA)+(Met+Tyr)/(Trp) |
| 0.8161 | (Orn)/(ABA)+(Thr+Tau+Ile)/(Trp) |
| 0.8156 | (Ile)/(ABA+Trp)+(Thr+Tyr)/(Val) |
| 0.8156 | (Met)/(ABA+Trp)+(Ile+Tyr)/(Val) |
| 0.8142 | (Cit)/(His)+(Thr+Tyr)/(ABA+Trp) |
| 0.8142 | (Ile)/(Val)+(Thr+Tyr)/(ABA+Trp) |
| 0.8142 | (Thr)/(Val)+(Ile+Tyr)/(ABA+Trp) |
| 0.8137 | (Met)/(ABA)+(Thr+Tyr)/(Trp) |
| 0.8123 | (Tau)/(His)+(Thr+Leu)/(ABA+Trp) |
| 0.8119 | (Ile)/(ABA)+(Thr+Tau+Orn)/(Trp) |
| 0.8119 | (Orn)/(ABA)+(Thr+Tau+Ser)/(Trp) |
| 0.8119 | (Met)/(ABA)+(Thr+Tyr+Asn)/(Trp) |
| 0.8119 | (Tyr)/(Trp)+(Met+Thr)/(His+ABA) |
| 0.8119 | (Tyr)/(His)+(Thr+Phe)/(ABA+Trp) |
| 0.8114 | (Met)/(ABA)+(Thr+Tau+Orn)/(Trp) |
| 0.8114 | (Thr+Tau+Orn+Tyr)/(ABA+Trp) |
| 0.8109 | (Tau)/(Val)+(Orn+Tyr)/(ABA+Trp) |
| 0.8100 | (Tau)/(Val)+(Thr+Ile)/(ABA+Trp) |
| 0.8100 | (Ile)/(ABA)+(Thr+Tau+Tyr)/(Trp) |
| 0.8095 | (Orn)/(ABA)+(Thr+Tau+Leu)/(Trp) |
| 0.8095 | (Tyr)/(ABA)+(Thr+Tau+Ile)/(Trp) |
| 0.8095 | (Tau)/(Val)+(Thr+Tyr)/(ABA+Trp) |
| 0.8095 | (Tyr)/(His)+(Thr+Cit)/(ABA+Trp) |
| 0.8095 | (Orn)/(ABA)+(Thr+Tau+Asn)/(Trp) |
| 0.8091 | (Orn)/(ABA)+(Met+Thr+Tau)/(Trp) |
| 0.8091 | (Met+Ile+Tyr)/(Val+ABA+Trp) |
| 0.8086 | (Ile)/(ABA)+(Thr+Tau+Ser)/(Trp) |
| 0.8086 | (Met)/(ABA)+(Thr+Tau+Ile)/(Trp) |
| 0.8086 | (Tau)/(His)+(Thr+Tyr)/(ABA+Trp) |
| 0.8086 | (Met+Thr+Tyr)/(ABA+Trp) |
| 0.8086 | (Met)/(Val)+(Thr+Tyr)/(ABA+Trp) |
| 0.8081 | (Thr)/(Trp)+(Tau+Tyr)/(His+ABA) |
| 0.8081 | (Tyr)/(ABA)+(Met+Thr+Glu)/(Trp) |
| 0.8077 | (Thr)/(Trp)+(Tau+Leu)/(His+ABA) |
| 0.8077 | (Tyr)/(His)+(Thr+Ile)/(ABA+Trp) |
| 0.8072 | (Thr+Tau+Ile+Tyr)/(ABA+Trp) |
| 0.8072 | (Met)/(ABA)+(Thr+Tau+Cit)/(Trp) |
| 0.8072 | (Tyr)/(ABA)+(Met+Thr+Tau)/(Trp) |
| 0.8072 | (Tyr)/(ABA)+(Thr+Orn+Glu)/(Trp) |
| 0.8063 | (Tyr)/(His)+(Met+Thr)/(ABA+Trp) |
| 0.8063 | (Tyr)/(ABA)+(Thr+Tau+Cit)/(Trp) |
| 0.8058 | (Tyr)/(ABA)+(Thr+Tau+Ser)/(Trp) |
| 0.8058 | (Tyr)/(ABA)+(Met+Thr+Ile)/(Trp) |
| 0.8058 | (Orn)/(Val)+(Thr+Tyr)/(ABA+Trp) |
| 0.8053 | (Ile)/(ABA+Trp)+(Thr+Tau)/(Val) |

FIG.112

| ROC AUC | FORMULA |
|---|---|
| 0.8053 | (Met+Thr)/(Trp)+(Tau+Tyr)/(His) |
| 0.8053 | (Met+Thr+Tyr+Cit)/(ABA+Trp) |
| 0.8049 | (Met)/(ABA)+(Thr)/(Trp) |
| 0.8049 | (Asn)/(Val)+(Thr+Tyr)/(ABA+Trp) |
| 0.8049 | (Tyr)/(ABA+Trp)+(Thr+Ile)/(Val) |
| 0.8049 | (Met)/(Trp)+(Ile+Orn+Tyr)/(Val) |
| 0.8044 | (Ile)/(ABA)+(Met+Thr+Tau)/(Trp) |
| 0.8044 | (Thr+Tau+Ile+Orn)/(ABA+Trp) |
| 0.8044 | (Met)/(ABA)+(Thr+Tau+Tyr)/(Trp) |
| 0.8044 | (Tyr)/(ABA)+(Met+Thr+Cit)/(Trp) |
| 0.8044 | (Glu)/(Val)+(Thr+Tyr)/(ABA+Trp) |
| 0.8044 | (Tyr)/(ABA)+(Thr+Glu+Cit)/(Trp) |
| 0.8044 | (Lys)/(Val)+(Thr+Tyr)/(ABA+Trp) |
| 0.8039 | (Tyr)/(His)+(Thr+Tau)/(ABA+Trp) |
| 0.8039 | (Phe)/(His)+(Thr+Tyr)/(ABA+Trp) |
| 0.8035 | (Ile)/(ABA)+(Thr+Tau+Glu)/(Trp) |
| 0.8035 | (Met)/(ABA)+(Thr+Cit)/(Trp) |
| 0.8035 | (Tyr)/(ABA)+(Met+Thr+Orn)/(Trp) |
| 0.8035 | (Met)/(ABA)+(Thr+Tyr+Cit)/(Trp) |
| 0.8035 | (Tyr)/(ABA)+(Thr+Ile+Glu)/(Trp) |
| 0.8030 | (Tau)/(His)+(Thr+Ile)/(ABA+Trp) |
| 0.8030 | (Thr)/(Trp)+(Tau+Ile)/(His+ABA) |
| 0.8030 | (Met+Tyr)/(ABA)+(Thr+Tau)/(Trp) |
| 0.8030 | (Thr)/(Trp)+(Tau+Orn)/(His+ABA) |
| 0.8030 | (Met)/(ABA)+(Thr+Orn+Tyr)/(Trp) |
| 0.8030 | (Leu)/(Val)+(Thr+Tyr)/(ABA+Trp) |
| 0.8030 | (Phe)/(ABA)+(Thr+Tau+Orn)/(Trp) |
| 0.8030 | (Tyr)/(ABA)+(Thr+Glu)/(Trp) |
| 0.8030 | (Met+Thr+Tyr+Asn)/(ABA+Trp) |
| 0.8025 | (Thr+Ile)/(Trp)+(Tau+Tyr)/(His) |
| 0.8025 | (Tyr)/(ABA)+(Thr+Ile)/(Trp) |
| 0.8025 | (Met)/(ABA)+(Thr+Tau+Glu)/(Trp) |
| 0.8025 | (Met+Tyr)/(ABA)+(Thr+Glu)/(Trp) |
| 0.8025 | (Met)/(ABA)+(Thr+Tyr+Glu)/(Trp) |
| 0.8025 | (Orn)/(ABA)+(Thr+Tau+Glu)/(Trp) |
| 0.8021 | (Ile)/(ABA)+(Thr+Tau+Asn)/(Trp) |
| 0.8021 | (Orn)/(ABA)+(Tau+Ser+Ile)/(Trp) |
| 0.8021 | (Tyr)/(His)+(Thr+Leu)/(ABA+Trp) |
| 0.8021 | (Tyr)/(ABA)+(Thr+Orn)/(Trp) |
| 0.8021 | (Cit)/(Val)+(Thr+Tyr)/(ABA+Trp) |
| 0.8021 | (Tyr)/(ABA+Trp)+(Met+Thr)/(Val) |
| 0.8016 | (Met)/(ABA)+(Thr+Tau)/(Trp) |
| 0.8016 | (Orn)/(ABA)+(Thr+Tau)/(Trp) |
| 0.8016 | (Thr)/(Trp)+(Met+Tau)/(His+ABA) |
| 0.8016 | (Cit)/(ABA)+(Thr+Tau+Orn)/(Trp) |
| 0.8016 | (Tau)/(His)+(Met+Thr+Tyr)/(Trp) |
| 0.8016 | (Met+Tyr)/(ABA)+(Thr+Orn)/(Trp) |
| 0.8016 | (Tyr)/(ABA)+(Thr+Tau+Asn)/(Trp) |
| 0.8016 | (Tyr)/(Val)+(Thr+Ile)/(ABA+Trp) |
| 0.8016 | (Tyr)/(ABA)+(Thr+Ile+Cit)/(Trp) |

# FIG.113

| ROC AUC | FORMULA |
|---|---|
| 0.7966 | (Orn+Tau)/(His)+(Thr+Leu)/(Trp) |
| 0.7946 | (Thr)/(Trp)+(Met+Tau+Ile)/(Arg) |
| 0.7931 | (Orn+Leu)/(Trp)+(Thr+Tau)/(His) |
| 0.7922 | (Ile)/(Trp)+(Thr+Tau)/(His+Arg) |
| 0.7912 | (Thr+Tau)/(Arg)+(Met+Ile)/(Trp) |
| 0.7882 | (Thr+Ile)/(Trp)+(Tau+Tyr)/(Arg) |
| 0.7882 | (Thr)/(Trp)+(Orn+Tau+Ile)/(Arg) |
| 0.7877 | (Orn+Thr)/(Trp)+(Tau+Leu)/(Arg) |
| 0.7858 | (Orn+Ile)/(Trp)+(Met+Tau)/(Arg) |
| 0.7853 | (Orn+Ile)/(Trp)+(Thr+Tau)/(Arg) |
| 0.7828 | (Tau)/(Arg)+(Orn+Met+Ile)/(Trp) |
| 0.7824 | (Tau)/(Cit+Arg)+(Orn+Met)/(Trp) |
| 0.7819 | (Ile)/(Trp)+(Thr+Tau)/(Cit+Arg) |
| 0.7814 | (Orn+Tau)/(Arg)+(Met+Ile)/(Trp) |
| 0.7809 | (Tau)/(Arg)+(Orn+Met)/(Trp) |
| 0.7804 | (Thr)/(Arg)+(Orn+Tau+Ile)/(Trp) |
| 0.7794 | (Orn+Met)/(Trp)+(Tau+Ile)/(Arg) |
| 0.7784 | (Orn+Ile)/(Trp)+(Tau+Tyr)/(Arg) |
| 0.7779 | (Orn+Met)/(Trp)+(Tau+Tyr)/(Arg) |
| 0.7779 | (Orn+Leu)/(Trp)+(Thr+Tau)/(Arg) |
| 0.7775 | (Orn+Met)/(Trp)+(Thr+Tau)/(Arg) |
| 0.7760 | (Thr+Tau)/(Arg)+(Ile+Tyr)/(Trp) |
| 0.7755 | (Tau)/(Arg)+(Orn+Ile)/(Trp) |
| 0.7745 | (Orn+Met)/(Trp)+(Tau+Asn)/(Arg) |
| 0.7711 | (Orn+Ile)/(Trp)+(Tau+Ser)/(Arg) |
| 0.7711 | (Ile)/(Trp)+(Thr+Met+Tau)/(Arg) |
| 0.7711 | (Orn+Met)/(Trp)+(Tau+Phe)/(Arg) |
| 0.7706 | (Orn+Ile)/(Trp)+(Tau+Phe)/(Arg) |
| 0.7706 | (Tau)/(Cit+Arg)+(Orn+Ile)/(Trp) |
| 0.7696 | (Orn+Ile)/(Trp)+(Tau+Asn)/(Arg) |
| 0.7691 | (Ile)/(Trp)+(Thr+Tau)/(Arg) |
| 0.7691 | (Orn)/(Trp)+(Met+Tau)/(Cit+Arg) |
| 0.7686 | (Orn+Tau)/(Trp)+(Thr+Ile)/(Arg) |
| 0.7681 | (Orn)/(Trp)+(Tau+Leu)/(His+Arg) |
| 0.7681 | (Ile)/(Trp)+(Orn+Tau)/(His+Arg) |
| 0.7676 | (Orn+Met)/(Trp)+(Tau+Leu)/(Arg) |
| 0.7672 | (Orn+Ile)/(Trp)+(Tau+Glu)/(Arg) |
| 0.7637 | (Ile)/(Trp)+(Orn+Tau)/(Cit+Arg) |
| 0.7608 | (Ile)/(Trp)+(Orn+Met+Tau)/(Arg) |
| 0.7588 | (Orn+Ile)/(Trp)+(Tau+Leu)/(Arg) |
| 0.7588 | (Orn+Ile)/(Trp)+(Pro+Tau)/(Arg) |
| 0.7564 | (Orn)/(Trp)+(Met+Tau)/(Arg) |
| 0.7559 | (Orn)/(Trp)+(Tau+Ile)/(Cit+Arg) |
| 0.7554 | (Ile)/(Trp)+(Orn+Tau)/(Arg) |
| 0.7520 | (Ile)/(Trp)+(Orn+Tau+Tyr)/(Arg) |
| 0.7510 | (Orn)/(Trp)+(Met+Tau+Ile)/(Arg) |
| 0.7485 | (Orn)/(Trp)+(Tau+Ile)/(Arg) |
| 0.7466 | (Ile)/(Trp)+(Orn+Tau+Phe)/(Arg) |
| 0.7402 | (Orn)/(Trp)+(Met+Tau+Leu)/(Arg) |
| 0.7289 | (Orn)/(Trp)+(Tau+Ile+Leu)/(Arg) |

# FIG.114

# FIG.115

| ROC AUC | FORMULA |
|---|---|
| 0.8666 | (Tau)/(Gln)+(Ser+Ile)/(Trp+His) |
| 0.8659 | (Ser+Ile)/(Trp)+(Tau+Met)/(His) |
| 0.8659 | (Tau)/(His)+(Ser+Ile+Met)/(Trp) |
| 0.8659 | (Ser+Ile)/(Trp)+(Gly+Tau)/(His) |
| 0.8651 | (Ile)/(Trp)+(Ser+Tau)/(Glu+His) |
| 0.8641 | (Ser+Met)/(Trp)+(Tau+Ile)/(His) |
| 0.8637 | (Ile)/(Trp)+(Ser+Tau+Tyr)/(Lys) |
| 0.8623 | (Tau)/(His)+(Ser+Ile)/(ABA+Trp) |
| 0.8619 | (Ser+Tau)/(His)+(Ile+Met)/(Trp) |
| 0.8608 | (Ile)/(Trp)+(Ser+Tau+Orn)/(Lys) |
| 0.8605 | (Tau)/(Ala)+(Ser+Ile)/(Trp+His) |
| 0.8601 | (Ile)/(Trp)+(Ser+Tau)/(Asn+His) |
| 0.8601 | (Tau)/(ABA+His)+(Ser+Ile)/(Trp) |
| 0.8598 | (Ile)/(Trp)+(Ser+Tau)/(ABA+His) |
| 0.8583 | (Ile)/(Trp)+(Ser+Tau+Met)/(Lys) |
| 0.8583 | (Ser+Ile)/(Trp)+(Tau+Tyr)/(His) |
| 0.8583 | (Ser+Ile)/(Trp)+(Tau+Orn)/(His) |
| 0.8580 | (Ser+Ile)/(Trp)+(Tau+Thr)/(His) |
| 0.8576 | (Ile)/(Trp)+(Ser+Tau)/(Lys) |
| 0.8576 | (Tau)/(His)+(Ser+Ile)/(Trp) |
| 0.8576 | (Gly+Tau)/(Lys)+(Ile+Tyr)/(Trp) |
| 0.8565 | (Ser)/(Trp)+(Tau+Ile+Met)/(His) |
| 0.8558 | (Tau)/(His)+(Ser+Ile+Tyr)/(Trp) |
| 0.8555 | (Ile)/(Trp)+(Ser+Tau+Thr)/(Lys) |
| 0.8555 | (Tau)/(Cit+His)+(Ser+Ile)/(Trp) |
| 0.8555 | (Tau)/(Asn+His)+(Ser+Ile)/(Trp) |
| 0.8551 | (Ile)/(Trp)+(Ser+Tau)/(Leu+His) |
| 0.8547 | (Ile)/(Trp)+(Ser+Tau)/(Val+His) |
| 0.8547 | (Gly)/(His)+(Ser+Tau+Ile)/(Trp) |
| 0.8544 | (Ile)/(Trp)+(Ser+Tau)/(Glu+Lys) |
| 0.8544 | (Ile)/(Trp)+(Ser+Tau)/(ABA+Lys) |
| 0.8544 | (Ile)/(Trp+His)+(Tyr+Met)/(Val) |
| 0.8544 | (Ser+Gly+Tau+Ile)/(Trp+His) |
| 0.8544 | (Ser+Tau)/(His)+(Ile+Tyr)/(Trp) |
| 0.8540 | (Tau)/(Pro)+(Ser+Ile)/(ABA+Trp) |
| 0.8537 | (Ile)/(ABA+Trp)+(Ser+Tau)/(Lys) |
| 0.8533 | (Ile+Tyr+Met)/(Val+Lys+Trp) |
| 0.8533 | (Ser+Tau)/(Lys)+(Ile+Met)/(Trp) |
| 0.8533 | (Ser)/(Trp)+(Tau+Ile)/(ABA+His) |
| 0.8529 | (Ser+Tau)/(Val)+(Ile+Met)/(Trp) |
| 0.8526 | (Ile)/(Trp)+(Ser+Tau+Tyr)/(Val) |
| 0.8526 | (Ile)/(Trp)+(Tau+Thr+Tyr)/(Lys) |
| 0.8526 | (Met)/(ABA)+(Ser+Tau+Ile)/(Trp) |
| 0.8522 | (Ile)/(Trp)+(Ser+Tau)/(Lys+His) |
| 0.8522 | (Tau)/(His)+(Ser+Tyr+Met)/(Trp) |
| 0.8522 | (Tau)/(Glu+His)+(Ser+Ile)/(Trp) |
| 0.8519 | (Tau)/(Lys)+(Ser+Ile)/(Trp+His) |
| 0.8515 | (Tau)/(Phe+His)+(Ser+Ile)/(Trp) |
| 0.8511 | (Gly+Tau+Ile+Tyr)/(Trp+His) |
| 0.8508 | (Tau)/(His)+(Ile+Tyr+Met)/(Trp) |

# FIG.116

| ROC AUC | FORMULA |
|---|---|
| 0.8508 | (Ile)/(Trp)+(Gly+Tau)/(Lys+His) |
| 0.8504 | (Ile)/(Trp)+(Tau+Tyr)/(Glu+His) |
| 0.8504 | (Ile)/(Trp)+(Ser+Tau)/(Phe+His) |
| 0.8504 | (Tau+Thr)/(Lys)+(Ile+Tyr)/(Trp) |
| 0.8501 | (Ile)/(Val)+(Tyr+Met)/(Lys+His) |
| 0.8501 | (Ser+Orn)/(Trp)+(Tau+Ile)/(His) |
| 0.8497 | (Ser+Met)/(Trp)+(Tau+Tyr)/(His) |
| 0.8494 | (Ile)/(Trp)+(Ser+Tau)/(Asn+Lys) |
| 0.8494 | (Ile)/(Val)+(Tyr+Met)/(Lys+Trp) |
| 0.8494 | (Ser)/(Trp)+(Tau+Ile+Orn)/(His) |
| 0.8490 | (Ile)/(Trp)+(Ser+Tau)/(Phe+Lys) |
| 0.8490 | (Ser+Gly+Tau+Tyr)/(Trp+His) |
| 0.8490 | (Tau)/(Pro)+(Ser+Ile+Met)/(Trp) |
| 0.8486 | (Ile)/(ABA+Trp)+(Ser+Tau)/(Val) |
| 0.8486 | (Met)/(Lys)+(Ile+Tyr)/(Val+His) |
| 0.8486 | (Tau)/(Asn+ABA)+(Ser+Ile)/(Trp) |
| 0.8483 | (Ser+Tyr)/(Trp)+(Tau+Thr)/(His) |
| 0.8483 | (Ser)/(Trp)+(Tau+Ile)/(His) |
| 0.8483 | (Ser+Ile)/(Trp)+(Tau+Met)/(Pro) |
| 0.8483 | (Ser+Tau)/(His)+(Ile+Orn)/(Trp) |
| 0.8483 | (Ile)/(ABA)+(Ser+Tau+Tyr)/(Trp) |
| 0.8479 | (Ile)/(Trp)+(Ser+Tau)/(Asn+Val) |
| 0.8479 | (Tau)/(His)+(Ser+Ile+Orn)/(Trp) |
| 0.8479 | (Ser)/(Trp)+(Tau+Ile)/(Asn+His) |
| 0.8479 | (Ser+Tyr)/(Trp)+(Tau+Ile)/(His) |
| 0.8479 | (Ser+Met)/(Trp)+(Tau+Orn)/(His) |
| 0.8479 | (Tau)/(Asn+Glu)+(Ser+Ile)/(Trp) |
| 0.8479 | (Ser)/(His)+(Tau+Ile+Met)/(Trp) |
| 0.8476 | (Ile)/(Trp)+(Ser+Tau)/(ABA+Val) |
| 0.8476 | (Tau)/(Val)+(Ser+Ile)/(ABA+Trp) |
| 0.8476 | (Tau)/(His)+(Ser+Met)/(Trp) |
| 0.8476 | (Ser+Tau)/(Lys)+(Ile+Tyr)/(Trp) |
| 0.8476 | (Ser+Ile)/(Trp)+(Tau+Tyr)/(Pro) |
| 0.8472 | (Ile+Tyr+Met)/(Val+Lys+His) |
| 0.8472 | (Ile)/(Trp)+(Tau+Tyr+Orn)/(Lys) |
| 0.8472 | (Ser+Ile)/(Trp)+(Tau+Tyr)/(Lys) |
| 0.8468 | (Ile)/(Trp)+(Ser+Tau+Met)/(Val) |
| 0.8468 | (Ile)/(Trp)+(Ser+Tyr)/(ABA+His) |
| 0.8468 | (Thr)/(His)+(Ser+Tau+Ile)/(Trp) |
| 0.8468 | (Tau)/(Arg+His)+(Ser+Ile)/(Trp) |
| 0.8468 | (Tau)/(Pro)+(Ile+Tyr+Met)/(Trp) |
| 0.8468 | (Gly+Tau)/(Lys)+(Ile+Tyr)/(His) |
| 0.8465 | (Ile)/(Trp)+(Tau+Tyr+Met)/(Lys) |
| 0.8465 | (Ser)/(Val+Lys)+(Ile)/(Trp+His) |
| 0.8465 | (Ser)/(Trp)+(Tau+Ile+Tyr)/(His) |
| 0.8465 | (Tau)/(Pro)+(Ser+Ile)/(Trp) |
| 0.8465 | (Tau)/(ABA+Pro)+(Ser+Ile)/(Trp) |
| 0.8461 | (Met)/(Lys)+(Ile+Tyr)/(Val+Trp) |
| 0.8461 | (Tyr)/(Lys)+(Ile+Met)/(Trp+His) |
| 0.8461 | (Tau)/(Val)+(Ile+Tyr+Met)/(Trp) |

# FIG.117

| ROC AUC | FORMULA |
|---|---|
| 0.8461 | (Tau)/(Phe+His)+(Ile+Met)/(Trp) |
| 0.8454 | (Ile)/(Trp)+(Tau+Ser)/(Cit+Val) |
| 0.8451 | (Ile)/(Trp)+(Tau+Ser)/(Val) |
| 0.8447 | (Tau)/(ABA+His)+(Ile+Met)/(Trp) |
| 0.8443 | (Tyr)/(Asn)+(Tau+Ser+Ile)/(Trp) |
| 0.8436 | (Ile)/(Trp)+(Tau+Ser)/(Pro+His) |
| 0.8429 | (Ile)/(Trp)+(Tau+Ser)/(Leu+Arg) |
| 0.8418 | (Tau)/(Cit+His)+(Ile+Met)/(Trp) |
| 0.8415 | (Ile)/(Trp)+(Tau+Tyr)/(Lys) |
| 0.8404 | (Ile)/(Trp)+(Tau+Ser)/(Val+Arg) |
| 0.8397 | (Tau)/(Asn+His)+(Ile+Met)/(Trp) |
| 0.8393 | (Ile)/(Trp)+(Tau+Tyr)/(Glu+Lys) |
| 0.8386 | (Ile)/(Trp)+(Gly+Tau)/(Pro+Lys) |
| 0.8386 | (Ile)/(Trp)+(Tau+Ser)/(Pro+Lys) |
| 0.8386 | (Tau)/(Asn+His)+(Ile+Tyr)/(Trp) |
| 0.8386 | (Ile)/(Trp)+(Tau+Ser)/(Pro+Leu) |
| 0.8382 | (Ile)/(Trp)+(Tau+Thr)/(Arg+His) |
| 0.8379 | (Tau)/(Arg)+(Ile+Tyr+Met)/(Trp) |
| 0.8379 | (Ile)/(Trp)+(Tau+Tyr)/(Leu+His) |
| 0.8375 | (Ile)/(Trp)+(Tau+Tyr)/(Val+His) |
| 0.8375 | (Tau)/(His)+(Ile+Met)/(Trp) |
| 0.8368 | (Ile)/(Trp)+(Tau+Tyr)/(Lys+His) |
| 0.8361 | (Ile)/(Trp)+(Tau+Met)/(Phe+His) |
| 0.8354 | (Tau)/(ABA+His)+(Ile+Tyr)/(Trp) |
| 0.8354 | (Ile)/(Trp)+(Tau+Met)/(Glu+His) |
| 0.835 | (Ile)/(Trp)+(Tau+Tyr)/(Phe+Lys) |
| 0.8346 | (Ile)/(Trp)+(Tau+Tyr)/(Glu+Leu) |
| 0.8346 | (Ile)/(Trp)+(Tau+Tyr)/(Val) |
| 0.8346 | (Ile)/(Trp)+(Tau+Tyr)/(ABA+Val) |
| 0.8346 | (Ile)/(Trp)+(Tau+Met)/(Asn+His) |
| 0.8343 | (Ile)/(Trp)+(Tau+Tyr)/(Arg+His) |
| 0.8339 | (Tau)/(Arg+His)+(Ile+Met)/(Trp) |
| 0.8325 | (Ile)/(Trp)+(Tau+Tyr)/(Asn+Lys) |
| 0.8325 | (Ile)/(Trp)+(Gly+Tau)/(Pro+His) |
| 0.8314 | (Ile)/(Trp)+(Tau+Tyr)/(Phe+His) |
| 0.8314 | (Ile)/(Trp)+(Tau+Ser)/(Pro+Arg) |
| 0.8311 | (Ile)/(Trp)+(Tau+Tyr)/(Asn+His) |
| 0.8307 | (Ile)/(Trp)+(Tau+Tyr)/(Pro+His) |
| 0.8289 | (Ile)/(Trp)+(Tau+Tyr)/(Lys+Arg) |
| 0.8286 | (Ile)/(Trp)+(Tau+Tyr)/(Leu+Arg) |
| 0.8278 | (Ile)/(Trp)+(Tau+Ser)/(Asn+Arg) |
| 0.8271 | (Ile)/(Trp)+(Tau+Tyr)/(Val+Arg) |
| 0.8264 | (Ile)/(Trp)+(Tau+Tyr)/(Asn+Leu) |
| 0.826 | (Ile)/(Trp)+(Tau+Met)/(Arg+His) |
| 0.8257 | (Tau)/(Phe+His)+(Ile)/(Trp) |
| 0.825 | (Ile)/(Trp)+(Tau+Tyr)/(Glu+Arg) |
| 0.8246 | (Tau)/(Asn+His)+(Ile)/(Trp) |
| 0.8246 | (Tau)/(Asn+Arg+His)+(Ile)/(Trp) |
| 0.8189 | (Ile)/(Trp)+(Tau+Orn)/(Arg+His) |
| 0.816 | (Ile)/(Trp)+(Tau+Tyr)/(Asn+Arg) |

# FIG.118

# FIG.119

| ROC AUC | FORMULA |
|---|---|
| 0.6893 | (Gly)/(Trp+His) |
| 0.6864 | (Gly)/(Met+Trp+His) |
| 0.6845 | (Gly)/(His) |
| 0.6836 | (Gly)/(Thr+Trp+His) |
| 0.6830 | (Gly)/(Thr+Met+Trp+His) |
| 0.6817 | (Gly)/(ABA+Thr+Trp+His) |
| 0.6813 | (Gly)/(Met+His) |
| 0.6810 | (Gly)/(ABA+Trp+His) |
| 0.6803 | (Gly)/(ABA+Met+Trp+His) |
| 0.6788 | (Gly)/(Cit+Met+Trp+His) |
| 0.6787 | (Gly)/(Tyr+Trp+His) |
| 0.6781 | (Gly)/(Cit+Trp+His) |
| 0.6779 | (Gly)/(Thr+Tyr+Trp+His) |
| 0.6756 | (Gly)/(Cit+Thr+Trp+His) |
| 0.6750 | (Gly)/(Met+Trp) |
| 0.6741 | (Gly)/(Met+Tyr+Trp+His) |
| 0.6734 | (Gly)/(ABA+His) |
| 0.6728 | (Gly)/(Trp) |
| 0.6718 | (Gly)/(Cit+ABA+Trp+His) |
| 0.6716 | (Gly)/(ABA+Tyr+Trp+His) |
| 0.6713 | (Gly)/(ABA+Thr+His) |
| 0.6712 | (Gly)/(Thr+Met+His) |
| 0.6712 | (Gly)/(Thr+Met+Trp) |
| 0.6709 | (Gly)/(ABA+Met+His) |
| 0.6708 | (Gly)/(ABA+Thr+Met+His) |
| 0.6708 | (Gly)/(Asn+Thr+Trp+His) |
| 0.6706 | (Gly)/(Thr+His) |
| 0.6706 | (Gly)/(Thr+Trp) |
| 0.6702 | (Gly)/(Cit+Tyr+Trp+His) |
| 0.6697 | (Gly)/(ABA+Thr+Trp) |
| 0.6696 | (Gly)/(ABA+Thr+Met+Trp) |
| 0.6693 | (Gly)/(Arg+Thr+Trp+His) |
| 0.6690 | (Gly)/(Thr+Tyr+His) |
| 0.6690 | (Gly)/(ABA+Met+Trp) |
| 0.6689 | (Gly)/(Arg+Trp+His) |
| 0.6686 | (Gly)/(Thr+Tyr+Trp) |
| 0.6684 | (Gly)/(Cit+Met+Trp) |
| 0.6684 | (Gly)/(Arg+Met+Trp+His) |
| 0.6680 | (Gly)/(Thr+Met+Tyr+His) |
| 0.6675 | (Gly)/(Met+Tyr+His) |
| 0.6675 | (Gly)/(Glu+Thr+Trp+His) |
| 0.6671 | (Gly)/(Tyr+His) |
| 0.6670 | (Gly)/(Arg+ABA+Trp+His) |
| 0.6667 | (Gly)/(Thr+Met+Tyr+Trp) |
| 0.6662 | (Gly)/(Asn+Met+Trp+His) |
| 0.6662 | (Gly)/(Glu+Trp+His) |
| 0.6661 | (Gly)/(Pro+Thr+Trp+His) |
| 0.6659 | (Gly)/(Cit+Met+His) |
| 0.6656 | (Gly)/(Asn+Trp+His) |
| 0.6656 | (Gly)/(ABA+Trp) |

# FIG.120

| ROC AUC | FORMULA |
|---|---|
| 0.6654 | (Gly)/(ABA+Thr+Tyr+His) |
| 0.6652 | (Gly)/(Glu+Met+Trp+His) |
| 0.6648 | (Gly)/(Ser+Thr+Trp+His) |
| 0.6645 | (Gly)/(ABA+Thr+Tyr+Trp) |
| 0.6643 | (Gly)/(Cit+His) |
| 0.6642 | (Gly)/(Cit+ABA+Thr+His) |
| 0.6640 | (Gly)/(Cit+Thr+Met+His) |
| 0.6637 | (Gly)/(Ser+Tyr+Trp+His) |
| 0.6636 | (Gly)/(Cit+ABA+Thr+Trp) |
| 0.6636 | (Gly)/(Glu+Cit+Trp+His) |
| 0.6636 | (Gly)/(Cit+Thr+Tyr+Trp) |
| 0.6635 | (Gly)/(Glu+ABA+Trp+His) |
| 0.6633 | (Gly)/(Cit+Trp) |
| 0.6633 | (Gly)/(Cit+Thr+Tyr+His) |
| 0.6632 | (Gly)/(Cit+Thr+His) |
| 0.6632 | (Gly)/(Arg+Tyr+Trp+His) |
| 0.6629 | (Gly)/(Cit+Thr+Met+Trp) |
| 0.6627 | (Gly)/(Cit+Tyr+His) |
| 0.6626 | (Gly)/(Asn+ABA+Trp+His) |
| 0.6626 | (Gly)/(Arg+Thr+Met+His) |
| 0.6624 | (Gly)/(Pro+Lys+Trp+His) |
| 0.6621 | (Gly)/(Cit+Met+Tyr+His) |
| 0.6620 | (Gly)/(Tyr+Trp) |
| 0.6617 | (Gly)/(ABA+Tyr+His) |
| 0.6615 | (Gly)/(Ser+Met+Trp+His) |
| 0.6614 | (Gly)/(Asn+Tyr+Trp+His) |
| 0.6613 | (Gly)/(Cit+ABA+Met+His) |
| 0.6613 | (Gly)/(ABA+Met+Tyr+His) |
| 0.6613 | (Gly)/(Glu+Tyr+Trp+His) |
| 0.6613 | (Gly)/(Cit+ABA+Met+Trp) |
| 0.6611 | (Gly)/(Cit+Arg+Trp+His) |
| 0.6611 | (Gly)/(Cit+Thr+Trp) |
| 0.6611 | (Gly)/(Thr+Lys+Trp+His) |
| 0.6610 | (Gly)/(Arg+Thr+His) |
| 0.6605 | (Gly)/(Cit+ABA+His) |
| 0.6605 | (Gly)/(Arg+ABA+Thr+His) |
| 0.6602 | (Gly)/(Met+Tyr+Trp) |
| 0.6598 | (Gly)/(Arg+Thr+Tyr+His) |
| 0.6598 | (Gly)/(Arg+Thr+Tyr+Trp) |
| 0.6595 | (Gly)/(Met) |
| 0.6594 | (Gly)/(Glu+ABA+Thr+His) |
| 0.6594 | (Gly)/(Ser+ABA+Trp+His) |
| 0.6594 | (Gly)/(Ser+Trp+His) |
| 0.6594 | (Gly)/(Cit+Arg+Thr+His) |
| 0.6591 | (Gly)/(Pro+Tyr+Trp+His) |
| 0.6583 | (Gly)/(Glu+Thr+Tyr+His) |
| 0.6583 | (Gly)/(Arg+Thr+Met+Trp) |
| 0.6583 | (Gly)/(Pro+Met+Trp+His) |
| 0.6582 | (Gly)/(Cit+ABA+Trp) |
| 0.6582 | (Gly)/(Asn+Cit+Trp+His) |

# FIG.121

| ROC AUC | FORMULA |
|---|---|
| 0.7037 | (Gly)/(Met+Trp+His)+(Cit)/(Pro) |
| 0.7029 | (Gly)/(Thr+Trp+His)+(Cit)/(Pro) |
| 0.6889 | (Gly)/(Thr+His)+(Cit)/(Pro) |
| 0.6842 | (Gly)/(Thr+Met+Trp)+(Cit)/(His) |
| 0.6833 | (Gly)/(Thr+Trp+His)+(Cit)/(Tyr) |
| 0.6813 | (Gly)/(Thr+His)+(Cit)/(Met+Trp) |
| 0.674 | (Gly)/(Asn+Thr+Trp)+(Cit)/(His) |
| 0.6715 | (Gly)/(Thr+Met+Trp)+(Cit)/(Tyr) |
| 0.6709 | (Gly)/(Orn+Trp+His)+(Cit)/(Met) |
| 0.6706 | (Gly)/(Thr+Trp)+(Cit)/(Met+Phe) |
| 0.6687 | (Gly)/(Thr+Met+His)+(Cit)/(Trp) |
| 0.6667 | (Gly)/(Thr+Tyr+His)+(Cit)/(Trp) |
| 0.6651 | (Gly)/(Arg+Thr+His)+(Cit)/(Trp) |
| 0.6623 | (Gly)/(Ala+Val+Trp)+(Cit)/(His) |
| 0.662 | (Gly)/(Ala+Thr+Trp)+(Cit)/(His) |
| 0.6604 | (Gly)/(Glu+Thr+His)+(Cit)/(Trp) |
| 0.6601 | (Gly)/(Thr+Trp)+(Cit)/(Phe) |
| 0.6595 | (Gly)/(Thr+Trp+His)+(Cit)/(Phe) |
| 0.6595 | (Gly)/(Orn+Thr+His)+(Cit)/(Trp) |
| 0.6585 | (Gly)/(Asn+Thr+His)+(Cit)/(Trp) |
| 0.6577 | (Gly)/(Val+His)+(Cit)/(Trp) |
| 0.6557 | (Gly)/(Met+Val+His)+(Cit)/(Trp) |
| 0.6554 | (Gly)/(Pro+Thr+His)+(Cit)/(Trp) |
| 0.6551 | (Gly)/(Thr+Met+Trp)+(Cit)/(Phe) |
| 0.6547 | (Gly)/(Thr+Phe+His)+(Cit)/(Trp) |
| 0.6547 | (Gly)/(Arg+Val+His)+(Cit)/(Trp) |
| 0.6544 | (Gly)/(Thr+Leu+His)+(Cit)/(Trp) |
| 0.6541 | (Gly)/(Thr+Val)+(Cit)/(Met+Trp) |
| 0.6529 | (Gly)/(Val+Tyr+His)+(Cit)/(Trp) |
| 0.652 | (Gly)/(Glu+Val+His)+(Cit)/(Trp) |
| 0.6515 | (Gly)/(Orn+Val+His)+(Cit)/(Trp) |
| 0.6497 | (Gly)/(Thr+Val+His)+(Cit)/(Trp) |
| 0.6496 | (Gly)/(Asn+Val+His)+(Cit)/(Trp) |
| 0.6484 | (Gly)/(Leu+Val+His)+(Cit)/(Trp) |
| 0.6475 | (Gly)/(Phe+Val+His)+(Cit)/(Trp) |
| 0.6475 | (Gly)/(Arg+Val+Tyr)+(Cit)/(Trp) |
| 0.6474 | (Gly)/(Pro+Val+His)+(Cit)/(Trp) |
| 0.6463 | (Gly)/(Thr+Val)+(Cit)/(Trp) |
| 0.6446 | (Gly)/(Thr+Met+Val)+(Cit)/(Trp) |
| 0.644 | (Gly)/(Orn+Val+Tyr)+(Cit)/(Trp) |
| 0.6434 | (Gly)/(Thr+Val+Tyr)+(Cit)/(Trp) |
| 0.6421 | (Gly)/(Arg+Thr+Val)+(Cit)/(Trp) |
| 0.6398 | (Gly)/(Pro+Thr+Val)+(Cit)/(Trp) |
| 0.6393 | (Gly)/(Ala+Val+His)+(Cit)/(Trp) |
| 0.6385 | (Gly)/(Asn+Thr+Val)+(Cit)/(Trp) |
| 0.6383 | (Gly)/(Thr+Leu+Val)+(Cit)/(Trp) |
| 0.6382 | (Gly)/(Orn+Thr+Val)+(Cit)/(Trp) |
| 0.6382 | (Gly)/(Glu+Thr+Val)+(Cit)/(Trp) |
| 0.6364 | (Gly)/(Thr+Phe+Val)+(Cit)/(Trp) |
| 0.6434 | (Gly)/(Pro+Val)+(Cit)/(Trp) |

# FIG.122

# FIG.123

| ROC AUC | FORMULA |
|---|---|
| 0.9196 | 0.8206+0.0061*Tau+0.0021*Thr-0.0176*Ile-0.0111*Leu-0.0085*Tyr-0.0031*His |
| 0.9196 | 0.8308+0.0063*Tau+0.0013*Pro-0.0028*Val+0.0197*Ile-0.0094*Leu-0.0077*Tyr |
| 0.9194 | 0.8891+0.0061*Tau+0.0027*Thr-0.0106*Asn-0.0042*Val+0.0213*Ile-0.0091*Leu |
| 0.9190 | 0.8739+0.0062*Tau+0.0013*Pro+0.0172*Ile-0.0113*Leu-0.0085*Tyr-0.0027*His |
| 0.9189 | 0.7773+0.0062*Tau+0.0018*Thr-0.0027*Val+0.0204*Ile-0.0095*Leu-0.0076*Tyr |
| 0.9180 | 0.6809+0.0061*Tau+0.0012*Thr+0.0011*Pro+0.0185*Ile-0.0126*Leu-0.0093*Tyr |
| 0.9178 | 0.7582+0.0062*Tau+0.0020*Thr-0.0037*Cit+0.0194*Ile-0.0125*Leu-0.0087*Tyr |
| 0.9176 | 0.7654+0.0061*Tau+0.0017*Thr-0.0035*Val+0.0202*Ile-0.0095*Leu-0.0031*His |
| 0.9175 | 0.6684+0.0062*Tau+0.0012*Pro+0.0190*Ile-0.0135*Leu-0.0096*Tyr+0.0011*Lys |
| 0.9174 | 0.8274+0.0061*Tau+0.0026*Thr-0.0068*Asn+0.0190*Ile-0.0123*Leu-0.0081*Tyr |
| 0.9173 | 0.8080+0.0063*Tau+0.0013*Pro-0.0031*Cit+0.0187*Ile-0.0125*Leu-0.0088*Tyr |
| 0.9172 | 0.8037+0.0062*Tau+0.0012*Pro-0.0035*Val+0.0198*Ile-0.0096*Leu-0.0029*His |
| 0.9169 | 0.7848+0.0062*Tau+0.0020*Thr-0.0004*Gly+0.0198*Ile-0.0130*Leu-0.0089*Tyr |
| 0.9167 | 0.7833+0.0063*Tau+0.0015*Pro-0.0004*Ala+0.0195*Ile-0.0129*Leu-0.0078*Tyr |
| 0.9161 | 0.7580+0.0063*Tau+0.0013*Pro+0.0189*Ile-0.0126*Leu-0.0087*Tyr-0.0007*Trp |
| 0.9160 | 0.8532+0.0063*Tau-0.0041*Asn+0.0014*Pro+0.0187*Ile-0.0125*Leu-0.0082*Tyr |
| 0.9159 | 0.7154+0.0062*Tau+0.0019*Thr-0.0003*Ala+0.0201*Ile-0.0129*Leu-0.0081*Tyr |
| 0.9159 | 0.7078+0.0062*Tau+0.0018*Thr+0.0199*Ile-0.0129*Leu-0.0086*Tyr-0.0007*Orn |
| 0.9159 | 0.7495+0.0062*Tau+0.0013*Pro+0.0190*Ile-0.0127*Leu-0.0088*Tyr-0.0001*Orn |
| 0.9159 | 0.7917+0.0063*Tau-0.0027*Val+0.0213*Ile-0.0106*Leu-0.0076*Tyr+0.0013*Lys |
| 0.9158 | 0.7473+0.0062*Tau+0.0013*Pro+0.0190*Ile-0.0127*Leu-0.0088*Tyr |
| 0.9158 | 0.6636+0.0062*Tau+0.0014*Thr+0.0198*Ile-0.0133*Leu-0.0091*Tyr+0.0007*Lys |
| 0.9156 | 0.6945+0.0061*Tau+0.0025*Thr-0.0037*Val-0.0124*Met+0.0237*Ile-0.0106*Leu |
| 0.9156 | 0.7112+0.0062*Tau+0.0017*Thr+0.0196*Ile-0.0127*Leu-0.0086*Tyr-0.0009*Trp |
| 0.9156 | 0.6988+0.0062*Tau+0.0017*Thr+0.0197*Ile-0.0128*Leu-0.0087*Tyr |
| 0.9155 | 0.8163+0.0063*Tau+0.0013*Pro-0.0003*Gly+0.0191*Ile-0.0129*Leu-0.0089*Tyr |
| 0.9155 | 0.7702+0.0062*Tau+0.0013*Pro-0.0015*ABA+0.0188*Ile-0.0125*Leu-0.0088*Tyr |
| 0.9155 | 0.7681+0.0062*Tau+0.0013*Pro+0.0190*Ile-0.0126*Leu-0.0086*Tyr-0.0008*Phe |
| 0.9154 | 0.9882+0.0064*Tau-0.0025*Val+0.0199*Ile-0.0088*Leu-0.0064*Tyr-0.0023*His |
| 0.9154 | 0.7274+0.0062*Tau+0.0017*Thr+0.0197*Ile-0.0126*Leu-0.0085*Tyr-0.0013*Phe |
| 0.9153 | 0.7522+0.0062*Tau+0.0015*Pro-0.0007*Ala-0.0033*Val+0.0222*Ile-0.0111*Leu |
| 0.9153 | 0.8305+0.0063*Tau+0.0189*Ile-0.0125*Leu-0.0084*Tyr-0.0027*His+0.0014*Lys |
| 0.9151 | 0.7663+0.0063*Tau+0.0013*Pro-0.0027*Met+0.0194*Ile-0.0127*Leu-0.0085*Tyr |
| 0.9149 | 0.7400+0.0061*Tau+0.0017*Thr-0.0030*ABA+0.0192*Ile-0.0124*Leu-0.0087*Tyr |
| 0.9147 | 0.9071+0.0063*Tau-0.0076*Asn+0.0013*Pro-0.0040*Val+0.0211*Ile-0.0096*Leu |
| 0.9146 | 1.1148+0.0063*Tau+0.0037*Thr-0.0128*Asn-0.0065*Val+0.0131*Ile-0.0039*His |
| 0.9142 | 0.7151+0.0061*Tau+0.0024*Thr-0.0073*Met+0.0206*Ile-0.0128*Leu-0.0082*Tyr |
| 0.9141 | 0.9114+0.0064*Tau-0.0015*Cit-0.0026*Val+0.0212*Ile-0.0098*Leu-0.0067*Tyr |
| 0.9141 | 0.6210+0.0061*Tau+0.0008*Thr+0.0010*Pro-0.0038*Val+0.0216*Ile-0.0109*Leu |
| 0.9139 | 0.7246+0.0062*Tau+0.0011*Pro-0.0029*Cit-0.0036*Val+0.0215*Ile-0.0108*Leu |
| 0.9139 | 0.6926+0.0062*Tau+0.0015*Thr-0.0032*Cit-0.0036*Val+0.0221*Ile-0.0109*Leu |
| 0.9138 | 0.8643+0.0064*Tau-0.0027*Val+0.0210*Ile-0.0098*Leu-0.0069*Tyr+0.0007*Orn |
| 0.9138 | 0.7433+0.0062*Tau+0.0013*Pro-0.0037*Val-0.0076*Met+0.0226*Ile-0.0107*Leu |
| 0.9137 | 0.8885+0.0064*Tau-0.0001*Ala-0.0026*Val+0.0214*Ile-0.0099*Leu-0.0066*Tyr |
| 0.9136 | 0.8980+0.0064*Tau-0.0026*Val+0.0213*Ile-0.0098*Leu-0.0066*Tyr-0.0006*Phe |
| 0.9136 | 0.7413+0.0062*Tau+0.0011*Pro-0.0036*Val+0.0217*Ile-0.0106*Leu-0.0025*Phe |
| 0.9136 | 0.8972+0.0064*Tau-0.0011*ABA-0.0026*Val+0.0211*Ile-0.0098*Leu-0.0067*Tyr |
| 0.9136 | 0.8827+0.0064*Tau-0.0026*Val+0.0213*Ile-0.0099*Leu-0.0067*Tyr |
| 0.9135 | 0.9296+0.0064*Tau-0.0002*Gly-0.0027*Val+0.0215*Ile-0.0099*Leu-0.0067*Tyr |
| 0.9135 | 0.8772+0.0064*Tau-0.0026*Val+0.0213*Ile-0.0099*Leu-0.0068*Tyr+0.0004*Trp |

# FIG.124

| ROC AUC | FORMULA |
|---|---|
| 0.9135 | 0.7715+0.0062*Tau+0.0012*Pro-0.0004*Gly-0.0038*Val+0.0220*Ile-0.0111*Leu |
| 0.9135 | 0.7632+0.0063*Tau-0.0028*Cit+0.0204*Ile-0.0137*Leu-0.0087*Tyr+0.0013*Lys |
| 0.9135 | 0.6220+0.0061*Tau+0.0011*Pro-0.0038*Val+0.0219*Ile-0.0114*Leu+0.0006*Lys |
| 0.9135 | 0.8833+0.0064*Tau-0.0026*Val-0.0001*Met+0.0213*Ile-0.0099*Leu-0.0067*Tyr |
| 0.9133 | 0.6904+0.0062*Tau+0.0017*Thr-0.0005*Ala-0.0034*Val+0.0229*Ile-0.0112*Leu |
| 0.9133 | 0.6984+0.0062*Tau+0.0011*Pro-0.0035*Val+0.0216*Ile-0.0108*Leu-0.0017*Trp |
| 0.9131 | 0.7152+0.0063*Tau+0.0205*Ile-0.0138*Leu-0.0087*Tyr+0.0012*Lys |
| 0.9131 | 0.7168+0.0063*Tau+0.0206*Ile-0.0138*Leu-0.0087*Tyr-0.0001*Orn+0.0012*Lys |
| 0.9130 | 0.6210+0.0060*Tau+0.0010*Thr-0.0009*Pro+0.0186*Ile-0.0141*Leu-0.0034*His |
| 0.9130 | 0.9056+0.0064*Tau-0.0025*Met+0.0185*Ile-0.0117*Leu-0.0078*Tyr-0.0026*His |
| 0.8353 | 1.2802+0.0034*Thr-0.0118*Asn+0.0013*Pro-0.0043*Val+0.0220*Ile-0.0110*Leu |
| 0.8333 | 1.4030+0.0043*Thr-0.0104*Asn-0.0039*Val+0.0210*Ile-0.0099*Leu-0.0033*His |
| 0.8320 | 1.2061+0.0027*Thr+0.0013*Pro+0.0180*Ile-0.0130*Leu-0.0088*Tyr-0.0036*His |
| 0.8315 | 1.3612+0.0041*Thr-0.0091*Asn-0.0033*Val+0.0218*Ile-0.0105*Leu-0.0062*Tyr |
| 0.8311 | 1.2174+0.0032*Thr-0.0079*Asn+0.0013*Pro+0.0197*Ile-0.0143*Leu-0.0083*Tyr |
| 0.8307 | 1.1502+0.0024*Thr+0.0013*Pro-0.0028*Val+0.0212*Ile-0.0116*Leu-0.0080*Tyr |
| 0.8305 | 1.1472+0.0023*Thr+0.0012*Pro-0.0035*Val+0.0208*Ile-0.0114*Leu-0.0036*His |
| 0.8302 | 1.2610+0.0037*Thr-0.0116*Asn-0.0042*Val+0.0235*Ile-0.0119*Leu+0.0009*Lys |
| 0.8302 | 1.1808+0.0016*Pro-0.0028*Val+0.0222*Ile-0.0129*Leu-0.0080*Tyr+0.0016*Lys |
| 0.8300 | 1.2963+0.0034*Thr-0.0025*Val+0.0202*Ile-0.0103*Leu-0.0071*Tyr-0.0034*His |
| 0.8294 | 1.3504+0.0042*Thr-0.0068*Asn+0.0189*Ile-0.0128*Leu-0.0075*Tyr-0.0034*His |
| 0.8289 | 1.4155+0.0017*Pro-0.0025*Val+0.0204*Ile-0.0109*Leu-0.0067*Tyr-0.0027*His |
| 0.8288 | 1.2318+0.0016*Pro+0.0194*Ile-0.0147*Leu-0.0088*Tyr-0.0031*His+0.0017*Lys |
| 0.8286 | 1.2669-0.0084*Asn+0.0016*Pro-0.0041*Val+0.0236*Ile-0.0132*Leu+0.0017*Lys |
| 0.8284 | 1.2714+0.0039*Thr-0.0113*Asn-0.0043*Val+0.0234*Ile-0.0115*Leu+0.0017*Trp |
| 0.8282 | 1.3627+0.0041*Thr-0.0108*Asn-0.0040*Val+0.0232*Ile-0.0109*Leu-0.0028*Phe |
| 0.8281 | 1.3111+0.0042*Thr-0.0108*Asn-0.0040*Val+0.0237*Ile-0.0115*Leu-0.0015*Orn |
| 0.8280 | 1.3077+0.0040*Thr-0.0107*Asn-0.0012*Cit-0.0041*Val+0.0232*Ile-0.0114*Leu |
| 0.8280 | 1.3160+0.0049*Thr-0.0100*Asn-0.0041*Val-0.0114*Met+0.0245*Ile-0.0111*Leu |
| 0.8275 | 1.0286+0.0019*Thr+0.0013*Pro+0.0206*Ile-0.0155*Leu-0.0095*Tyr+0.0009*Lys |
| 0.8275 | 1.2078+0.0033*Thr-0.0093*Asn+0.0012*Pro+0.0191*Ile-0.0151*Leu-0.0036*His |
| 0.8275 | 1.1891+0.0014*Pro-0.0035*Val+0.0220*Ile-0.0128*Leu-0.0031*His+0.0012*Lys |
| 0.8275 | 1.5636+0.0046*Thr-0.0147*Asn+0.0014*Pro-0.0072*Val+0.0121*Ile-0.0046*His |
| 0.8273 | 1.2931+0.0039*Thr-0.0109*Asn-0.0041*Val+0.0233*Ile-0.0114*Leu |
| 0.8271 | 1.1342+0.0025*Thr+0.0013*Pro-0.0039*Cit+0.0201*Ile-0.0146*Leu-0.0091*Tyr |
| 0.8271 | 1.2071-0.0049*Asn+0.0016*Pro+0.0211*Ile-0.0162*Leu-0.0086*Tyr+0.0019*Lys |
| 0.8270 | 1.2993+0.0041*Thr-0.0102*Asn-0.0003*Ala-0.0039*Val+0.0236*Ile-0.0116*Leu |
| 0.8269 | 1.3254+0.0040*Thr-0.0109*Asn-0.0028*ABA-0.0039*Val+0.0228*Ile-0.0112*Leu |
| 0.8268 | 1.3808+0.0042*Thr-0.0106*Asn-0.0005*Gly-0.0043*Val+0.0235*Ile-0.0115*Leu |
| 0.8268 | 1.1934+0.0027*Thr+0.0013*Pro-0.0006*Gly+0.0206*Ile-0.0153*Leu-0.0094*Tyr |
| 0.8265 | 1.1908+0.0030*Thr+0.0196*Ile-0.0138*Leu-0.0085*Tyr-0.0035*His+0.0008*Lys |
| 0.8265 | 1.0979+0.0026*Thr+0.0015*Pro-0.0005*Ala+0.0211*Ile-0.0151*Leu-0.0079*Tyr |
| 0.8264 | 1.1928+0.0034*Thr-0.0078*Asn+0.0213*Ile-0.0153*Leu-0.0081*Tyr+0.0010*Lys |
| 0.8263 | 1.4113-0.0040*Asn+0.0017*Pro-0.0029*Val+0.0220*Ile-0.0115*Leu-0.0063*Tyr |
| 0.8263 | 1.2645+0.0035*Thr-0.0024*Cit+0.0193*Ile-0.0131*Leu-0.0081*Tyr-0.0034*His |
| 0.8262 | 1.0695+0.0033*Thr+0.0013*Pro-0.0038*Val-0.0157*Met+0.0249*Ile-0.0125*Leu |
| 0.8259 | 1.0905+0.0026*Thr+0.0013*Pro+0.0210*Ile-0.0150*Leu-0.0088*Tyr-0.0017*Orn |
| 0.8255 | 1.0498+0.0022*Thr+0.0013*Pro+0.0206*Ile-0.0151*Leu-0.0094*Tyr+0.0013*Trp |
| 0.8255 | 1.3143+0.0036*Thr-0.0004*Gly+0.0196*Ile-0.0136*Leu-0.0083*Tyr-0.0034*His |
| 0.8255 | 1.4326-0.0052*Asn+0.0016*Pro-0.0035*Val+0.0217*Ile-0.0114*Leu-0.0026*His |

# FIG.125

# FIG.126

| ROC AUC | FORMULA |
|---|---|
| 0.9295 | 0.3249+0.1049*Tau+0.0102*Pro-0.0254*Val+0.2065*Ile-0.0994*Leu-0.0612*Tyr |
| 0.9283 | 1.2289+0.1027*Tau+0.0112*Pro+0.1674*Ile-0.1078*Leu-0.0629*Tyr-0.0374*His |
| 0.9278 | 1.1810+0.1045*Tau+0.0098*Pro-0.0310*Val+0.1861*Ile-0.0842*Leu-0.0480*His |
| 0.9277 | -0.1544+0.1018*Tau+0.0162*Thr-0.0257*Val+0.2097*Ile-0.0972*Leu-0.0631*Tyr |
| 0.9276 | 0.8578+0.0990*Tau+0.0220*Thr+0.1630*Ile-0.1010*Leu-0.0662*Tyr-0.0459*His |
| 0.9273 | -0.1405+0.1028*Tau+0.0119*Pro-0.0033*Ala+0.1962*Ile-0.1271*Leu-0.0636*Tyr |
| 0.9272 | 0.2222+0.1042*Tau+0.0107*Pro-0.0355*Cit+0.1911*Ile-0.1238*Leu-0.0704*Tyr |
| 0.9272 | -0.8670+0.1006*Tau+0.0110*Thr+0.0086*Pro+0.1925*Ile-0.1269*Leu-0.0781*Tyr |
| 0.9270 | -0.8268+0.1015*Tau+0.0097*Pro+0.1958*Ile-0.1320*Leu-0.0780*Tyr+0.0074*Lys |
| 0.9269 | 0.7580+0.1006*Tau+0.0279*Thr-0.0982*Asn-0.0376*Val+0.2126*Ile-0.0917*Leu |
| 0.9268 | 0.2764+0.1024*Tau-0.0261*Asn+0.0109*Pro+0.1899*Ile-0.1242*Leu-0.0673*Tyr |
| 0.9268 | 0.9074+0.1009*Tau+0.0190*Thr-0.0322*Val+0.1862*Ile-0.0788*Leu-0.0563*His |
| 0.9266 | -0.2606+0.1017*Tau+0.0207*Thr-0.0494*Cit+0.1920*Ile-0.1204*Leu-0.0746*Tyr |
| 0.9262 | -0.1137+0.1026*Tau+0.0103*Pro+0.1918*Ile-0.1242*Leu-0.0677*Tyr-0.0153*Trp |
| 0.9262 | 0.1640+0.1026*Tau+0.0105*Pro-0.0023*Gly+0.1932*Ile-0.1277*Leu-0.0711*Tyr |
| 0.9259 | -0.4320+0.1020*Tau+0.0098*Pro+0.0091*Met+0.1922*Ile-0.1265*Leu-0.0731*Tyr |
| 0.9259 | -0.0366+0.1036*Tau-0.0272*Val+0.2192*Ile-0.1035*Leu-0.0626*Tyr+0.0114*Lys |
| 0.9258 | -0.3799+0.1020*Tau+0.0100*Pro+0.1926*Ile-0.1262*Leu-0.0715*Tyr |
| 0.9258 | -0.3844+0.1020*Tau+0.0100*Pro+0.1925*Ile-0.1262*Leu-0.0715*Tyr+0.0002*Orn |
| 0.9257 | -0.4497+0.1021*Tau+0.0100*Pro+0.1928*Ile-0.1267*Leu-0.0724*Tyr+0.0029*Phe |
| 0.9256 | 0.4835+0.1042*Tau-0.0519*Asn+0.0099*Pro-0.0334*Val+0.2102*Ile-0.0994*Leu |
| 0.9255 | -0.1967+0.1024*Tau+0.0099*Pro-0.0120*ABA+0.1910*Ile-0.1247*Leu-0.0714*Tyr |
| 0.9255 | -0.7609+0.1010*Tau+0.0223*Thr-0.0346*Val-0.1139*Met+0.2272*Ile-0.0982*Leu |
| 0.9252 | 0.2098+0.0985*Tau+0.0250*Thr-0.0590*Asn+0.1868*Ile-0.1185*Leu-0.0686*Tyr |
| 0.9251 | 0.0375+0.1000*Tau+0.0200*Thr-0.0045*Gly+0.1936*Ile-0.1259*Leu-0.0757*Tyr |
| 0.9251 | 1.6812+0.1044*Tau-0.0253*Val+0.1942*Ile-0.0824*Leu-0.0446*Tyr-0.0268*His |
| 0.9249 | -0.3837+0.1044*Tau+0.0097*Pro-0.0328*Val-0.0691*Met+0.2191*Ile-0.1019*Leu |
| 0.9247 | -0.4815+0.1037*Tau+0.0109*Pro-0.0051*Ala-0.0294*Val+0.2211*Ile-0.1095*Leu |
| 0.9246 | 0.9421+0.1053*Tau-0.0240*Cit-0.0252*Val+0.2106*Ile-0.0940*Leu-0.0504*Tyr |
| 0.9243 | -0.7528+0.0992*Tau+0.0172*Thr-0.0021*Ala+0.1952*Ile-0.1233*Leu-0.0682*Tyr |
| 0.9242 | 3.7093+0.0987*Tau+0.0368*Thr-0.0960*Asn-0.0512*Val+0.1119*Ile-0.0716*His |
| 0.9242 | -0.6279+0.0995*Tau+0.0159*Thr+0.1917*Ile-0.1209*Leu-0.0698*Tyr-0.0138*Trp |
| 0.9241 | -1.0401+0.0991*Tau+0.0137*Thr+0.1947*Ile-0.1263*Leu-0.0768*Tyr+0.0047*Lys |
| 0.9241 | 0.3684+0.1040*Tau-0.0255*Val+0.2077*Ile-0.0953*Leu-0.0545*Tyr+0.0091*Orn |
| 0.9241 | -0.3857+0.1053*Tau+0.0090*Pro-0.0408*Cit-0.0321*Val+0.2166*Ile-0.1056*Leu |
| 0.9240 | 0.9498+0.1045*Tau-0.0017*Gly-0.0258*Val+0.2112*Ile-0.0959*Leu-0.0506*Tyr |
| 0.9239 | 0.5428+0.1039*Tau+0.0000*Ala-0.0252*Val+0.2104*Ile-0.0956*Leu-0.0516*Tyr |
| 0.9239 | -0.7876+0.0990*Tau+0.0165*Thr+0.1938*Ile-0.1228*Leu-0.0724*Tyr-0.0051*Orn |
| 0.9239 | 0.5436+0.1039*Tau-0.0252*Val+0.2104*Ile-0.0957*Leu-0.0516*Tyr |
| 0.9238 | -0.7972+0.0989*Tau+0.0176*Thr-0.0245*Met+0.1930*Ile-0.1218*Leu-0.0699*Tyr |
| 0.9238 | 0.3905+0.1037*Tau-0.0244*Val+0.0229*Met+0.2096*Ile-0.0977*Leu-0.0563*Tyr |
| 0.9237 | 0.5850+0.1039*Tau-0.0253*Val+0.2103*Ile-0.0953*Leu-0.0510*Tyr-0.0016*Phe |
| 0.9237 | 0.5854+0.1040*Tau-0.0250*Val+0.2103*Ile-0.0954*Leu-0.0509*Tyr-0.0029*Trp |
| 0.9236 | -0.9002+0.0992*Tau+0.0154*Thr+0.1927*Ile-0.1231*Leu-0.0740*Tyr+0.0023*Phe |
| 0.9236 | 0.5958+0.1040*Tau-0.0041*ABA-0.0249*Val+0.2095*Ile-0.0954*Leu-0.0516*Tyr |
| 0.9235 | -0.8482+0.0991*Tau+0.0155*Thr+0.1925*Ile-0.1227*Leu-0.0733*Tyr |
| 0.9235 | 0.0707+0.1047*Tau+0.0092*Pro-0.0048*Gly-0.0341*Val+0.2202*Ile-0.1090*Leu |
| 0.9235 | -0.5542+0.0996*Tau+0.0157*Thr-0.0206*ABA+0.1896*Ile-0.1202*Leu-0.0733*Tyr |
| 0.9234 | -0.7018+0.1031*Tau+0.0084*Pro-0.0302*Val+0.2149*Ile-0.1059*Leu-0.0206*Trp |
| 0.9233 | -0.3197+0.1031*Tau+0.0084*Pro-0.0320*Val+0.2160*Ile-0.1027*Leu-0.0248*Phe |

# FIG.127

| ROC AUC | FORMULA |
|---|---|
| 0.9231 | 0.7709+0.1003*Tau+0.1744*Ile-0.1119*Leu-0.0645*Tyr-0.0380*His+0.0127*Lys |
| 0.9228 | -0.2073+0.0987*Tau+0.0132*Thr+0.0081*Pro+0.1603*Ile-0.1137*Leu-0.0637*His |
| 0.9227 | -1.5140+0.1010*Tau+0.0065*Thr+0.0072*Pro-0.0328*Val+0.2181*Ile-0.1083*Leu |
| 0.9227 | 1.1024+0.1008*Tau+0.0831*Met+0.1666*Ile-0.1055*Leu-0.0670*Tyr-0.0402*His |
| 0.9227 | -0.9861+0.1003*Tau+0.0141*Thr-0.0040*Ala-0.0303*Val+0.2244*Ile-0.1072*Leu |
| 0.9225 | -0.2214+0.1019*Tau-0.0336*Cit+0.1984*Ile-0.1276*Leu-0.0715*Tyr+0.0112*Lys |
| 0.9224 | -1.3612+0.1018*Tau-0.0079*Pro-0.0328*Val+0.2198*Ile-0.1101*Leu+0.0030*Lys |
| 0.9221 | -0.7424+0.1028*Tau+0.0159*Thr-0.0515*Cit-0.0330*Val+0.2217*Ile-0.1042*Leu |
| 0.9211 | -0.6958+0.1002*Tau+0.1970*Ile-0.1279*Leu-0.0711*Tyr+0.0013*Orn+0.0090*Lys |
| 0.9210 | -0.6807+0.1002*Tau+0.1974*Ile-0.1281*Leu-0.0709*Tyr+0.0092*Lys |
| 0.8362 | 4.8508+0.0257*Thr-0.0844*Asn+0.0069*Pro-0.0281*Val+0.1589*Ile-0.0759*Leu |
| 0.8343 | 5.0495+0.0199*Thr-0.0076*Pro+0.1185*Ile-0.0796*Leu-0.0491*Tyr-0.0442*His |
| 0.8339 | 6.2030+0.0315*Thr-0.0667*Asn-0.0264*Val+0.1433*Ile-0.0603*Leu-0.0382*His |
| 0.8328 | 5.1770+0.0185*Thr-0.0066*Pro-0.0231*Val+0.1339*Ile-0.0643*Leu-0.0507*His |
| 0.8325 | 5.2576+0.0298*Thr-0.0695*Asn-0.0223*Val+0.1581*Ile-0.0718*Leu-0.0364*Tyr |
| 0.8320 | 4.4315+0.0236*Thr-0.0622*Asn+0.0078*Pro+0.1422*Ile-0.0966*Leu-0.0508*Tyr |
| 0.8314 | 7.5017+0.0330*Thr-0.0849*Asn+0.0080*Pro-0.0411*Val+0.0793*Ile-0.0613*His |
| 0.8314 | 5.5331+0.0240*Thr-0.0183*Val+0.1365*Ile-0.0619*Leu-0.0373*Tyr-0.0412*His |
| 0.8312 | 5.0354+0.0095*Pro+0.1281*Ile-0.0926*Leu-0.0493*Tyr-0.0401*His+0.0140*Lys |
| 0.8310 | 3.8429+0.0157*Thr+0.0071*Pro-0.0184*Val+0.1532*Ile-0.0799*Leu-0.0464*Tyr |
| 0.8310 | 3.9218+0.0086*Pro-0.0190*Val+0.1609*Ile-0.0897*Leu-0.0470*Tyr+0.0125*Lys |
| 0.8309 | 4.5452+0.0261*Thr-0.0885*Asn-0.0295*Val+0.1712*Ile-0.0834*Leu+0.0069*Lys |
| 0.8309 | 6.1103+0.0098*Pro-0.0168*Val+0.1365*Ile-0.0673*Leu-0.0349*Tyr-0.0297*His |
| 0.8308 | 5.5574+0.0296*Thr-0.0472*Asn+0.1282*Ile-0.0811*Leu-0.0429*Tyr-0.0350*His |
| 0.8299 | 5.1945+0.0083*Pro-0.0237*Val+0.1437*Ile-0.0757*Leu-0.0466*His+0.0127*Lys |
| 0.8295 | 4.4737-0.0527*Asn+0.0086*Pro-0.0263*Val+0.1666*Ile-0.0906*Leu+0.0137*Lys |
| 0.8295 | 5.0390+0.0243*Thr-0.0561*Asn+0.0069*Pro+0.1234*Ile-0.0902*Leu-0.0442*His |
| 0.8289 | 5.2036+0.0281*Thr-0.0748*Asn-0.0269*Val+0.1631*Ile-0.0733*Leu-0.0152*Phe |
| 0.8284 | 2.9500+0.0120*Thr+0.0072*Pro+0.1472*Ile-0.1058*Leu-0.0569*Tyr+0.0072*Lys |
| 0.8281 | 4.1079-0.0349*Asn+0.0096*Pro+0.1499*Ile-0.1092*Leu-0.0513*Tyr+0.0139*Lys |
| 0.8281 | 5.0338+0.0290*Thr-0.0746*Asn-0.0176*Cit-0.0271*Val+0.1639*Ile-0.0758*Leu |
| 0.8279 | 4.8869+0.0312*Thr-0.0683*Asn-0.0276*Val-0.0496*Met+0.1657*Ile-0.0727*Leu |
| 0.8278 | 4.6786+0.0280*Thr-0.0799*Asn-0.0278*Val+0.1636*Ile-0.0767*Leu+0.0092*Trp |
| 0.8277 | 4.7127+0.0202*Thr+0.1282*Ile-0.0863*Leu-0.0487*Tyr-0.0452*His+0.0088*Lys |
| 0.8277 | 4.8005+0.0280*Thr-0.0773*Asn-0.0270*Val+0.1634*Ile-0.0762*Leu |
| 0.8277 | 4.8966+0.0294*Thr-0.0760*Asn-0.0266*Val+0.1662*Ile-0.0770*Leu-0.0081*Orn |
| 0.8277 | 4.9508+0.0284*Thr-0.0784*Asn-0.0120*ABA-0.0263*Val+0.1609*Ile-0.0752*Leu |
| 0.8276 | 5.1818+0.0292*Thr-0.0750*Asn-0.0020*Gly-0.0276*Val+0.1641*Ile-0.0769*Leu |
| 0.8276 | 3.7263+0.0170*Thr+0.0073*Pro-0.0282*Cit+0.1432*Ile-0.0978*Leu-0.0541*Tyr |
| 0.8275 | 4.8322+0.0286*Thr-0.0731*Asn-0.0014*Ala-0.0260*Val+0.1651*Ile-0.0774*Leu |
| 0.8274 | 4.0118+0.0242*Thr-0.0642*Asn+0.1531*Ile-0.1047*Leu-0.0506*Tyr+0.0098*Lys |
| 0.8272 | 5.1666+0.0244*Thr-0.0188*Cit+0.1270*Ile-0.0808*Leu-0.0450*Tyr-0.0390*His |
| 0.8270 | 3.4120+0.0169*Thr+0.0083*Pro-0.0031*Ala+0.1470*Ile-0.1004*Leu-0.0467*Tyr |
| 0.8266 | 3.9415+0.0172*Thr+0.0074*Pro-0.0032*Gly+0.1433*Ile-0.1009*Leu-0.0549*Tyr |
| 0.8265 | 5.3462+0.0247*Thr-0.0021*Gly+0.1268*Ile-0.0824*Leu-0.0455*Tyr-0.0398*His |
| 0.8265 | 3.5219+0.0212*Thr-0.0069*Pro-0.0250*Val-0.0967*Met+0.1681*Ile-0.0806*Leu |
| 0.8263 | 5.3918-0.0238*Asn+0.0097*Pro-0.0179*Val+0.1516*Ile-0.0774*Leu-0.0364*Tyr |
| 0.8260 | 3.3945+0.0163*Thr+0.0072*Pro+0.1467*Ile-0.1001*Leu-0.0522*Tyr-0.0089*Orn |
| 0.8257 | 3.1795+0.0145*Thr+0.0070*Pro+0.1433*Ile-0.1001*Leu-0.0545*Tyr+0.0060*Trp |
| 0.8255 | 6.0205-0.0192*Asn+0.0092*Pro-0.0215*Val+0.1403*Ile-0.0689*Leu-0.0313*His |

# FIG.128

## FIG.129

| ROC AUC | FORMULA |
|---|---|
| 0.9446 | 0.9579+0.0040*Tau+0.0022*Pro-0.0046*Val+0.0212*Ile-0.0082*Leu-0.0078*Tyr |
| 0.9437 | 0.8376+0.0038*Tau+0.0020*Pro-0.0051*Val+0.0238*Ile-0.0102*Leu-0.0020*Phe |
| 0.9433 | 0.9080+0.0038*Tau+0.0021*Pro-0.0052*Val+0.0221*Ile-0.0089*Leu-0.0025*His |
| 0.9433 | 0.7241+0.0037*Tau+0.0010*Thr+0.0019*Pro-0.0054*Val+0.0231*Ile-0.0100*Leu |
| 0.9409 | 0.7231+0.0037*Tau+0.0020*Pro-0.0053*Val+0.0236*Ile-0.0108*Leu+0.0008*Lys |
| 0.9405 | 0.7528+0.0038*Tau+0.0020*Pro-0.0054*Val+0.0228*Ile-0.0100*Leu+0.0014*Orn |
| 0.9403 | 0.7254+0.0038*Tau+0.0020*Pro+0.0002*Gly-0.0051*Val+0.0233*Ile-0.0102*Leu |
| 0.9400 | 0.8226+0.0039*Tau+0.0021*Pro-0.0050*Val+0.0232*Ile-0.0099*Leu-0.0025*Trp |
| 0.9395 | 0.7886+0.0038*Tau+0.0020*Pro-0.0053*Val+0.0236*Ile-0.0102*Leu |
| 0.9395 | 0.7890+0.0038*Tau+0.0020*Pro-0.0000*Cit-0.0053*Val+0.0236*Ile-0.0102*Leu |
| 0.9392 | 1.1669+0.0044*Tau+0.0023*Pro-0.0065*Val+0.0145*Ile-0.0095*Tyr-0.0035*His |
| 0.9391 | 0.7985+0.0038*Tau-0.0001*Ser+0.0020*Pro-0.0053*Val+0.0236*Ile-0.0102*Leu |
| 0.9390 | 0.9082+0.0038*Tau+0.0021*Thr-0.0051*Val+0.0223*Ile-0.0086*Leu-0.0027*His |
| 0.9388 | 0.8332+0.0038*Tau+0.0022*Pro-0.0004*Ala-0.0052*Val+0.0239*Ile-0.0102*Leu |
| 0.9388 | 0.7604+0.0039*Tau+0.0012*Thr+0.0020*Pro+0.0185*Ile-0.0133*Leu-0.0098*Tyr |
| 0.9386 | 0.9444+0.0039*Tau-0.0050*Asn+0.0021*Pro-0.0055*Val+0.0230*Ile-0.0091*Leu |
| 0.9377 | 0.6787+0.0040*Tau+0.0020*Pro+0.0006*Gly+0.0186*Ile-0.0130*Leu-0.0094*Tyr |
| 0.9374 | 0.7380+0.0039*Tau+0.0021*Pro+0.0190*Ile-0.0143*Leu-0.0102*Tyr+0.0014*Lys |
| 0.9373 | 0.8452+0.0038*Tau+0.0020*Thr-0.0049*Val+0.0243*Ile-0.0100*Leu-0.0025*Phe |
| 0.9373 | 0.9804+0.0040*Tau+0.0022*Pro+0.0177*Ile-0.0120*Leu-0.0095*Tyr-0.0027*His |
| 0.9369 | 0.8779+0.0037*Tau+0.0019*Pro-0.0065*ABA-0.0048*Val+0.0224*Ile-0.0099*Leu |
| 0.9367 | 0.8862+0.0040*Tau+0.0016*Thr+0.0019*Pro-0.0082*Val+0.0149*Ile-0.0041*His |
| 0.9366 | 0.8766+0.0041*Tau+0.0022*Pro+0.0190*Ile-0.0128*Leu-0.0089*Tyr-0.0034*Trp |
| 0.9363 | 0.8961+0.0040*Tau+0.0022*Pro+0.0198*Ile-0.0132*Leu-0.0091*Tyr-0.0027*Phe |
| 0.9359 | 0.8382+0.0040*Tau+0.0022*Pro-0.0004*Cit+0.0193*Ile-0.0135*Leu-0.0095*Tyr |
| 0.9359 | 0.7931+0.0040*Tau+0.0021*Pro+0.0183*Ile-0.0132*Leu-0.0097*Tyr+0.0016*Orn |
| 0.9358 | 0.8570+0.0040*Tau-0.0011*Asn+0.0022*Pro+0.0192*Ile-0.0134*Leu-0.0093*Tyr |
| 0.9357 | 0.8304+0.0040*Tau+0.0022*Pro+0.0193*Ile-0.0135*Leu-0.0095*Tyr |
| 0.9356 | 0.8176+0.0040*Tau+0.0001*Ser+0.0022*Pro+0.0192*Ile-0.0135*Leu-0.0095*Tyr |
| 0.9353 | 0.9556+0.0039*Tau+0.0025*Thr-0.0065*Asn-0.0055*Val+0.0230*Ile-0.0085*Leu |
| 0.9350 | 0.8214+0.0040*Tau+0.0008*Gly-0.0043*Val+0.0225*Ile-0.0091*Leu-0.0026*His |
| 0.9350 | 0.9376+0.0040*Tau+0.0021*Thr-0.0045*Val+0.0219*Ile-0.0082*Leu-0.0070*Tyr |
| 0.9347 | 0.9638+0.0039*Tau+0.0021*Pro-0.0089*ABA+0.0181*Ile-0.0124*Leu-0.0090*Tyr |
| 0.9346 | 0.8406+0.0040*Tau+0.0022*Pro-0.0001*Ala+0.0195*Ile-0.0135*Leu-0.0092*Tyr |
| 0.9345 | 0.5456+0.0037*Tau+0.0017*Pro+0.0008*Gly+0.0193*Ile-0.0147*Leu-0.0030*His |
| 0.9342 | 0.9208+0.0044*Tau+0.0016*Thr+0.0021*Pro-0.0071*Val+0.0146*Ile-0.0104*Tyr |
| 0.9342 | 0.5312+0.0037*Tau+0.0018*Pro+0.0007*Gly+0.0217*Ile-0.0159*Leu-0.0040*Phe |
| 0.9340 | 0.8588+0.0038*Tau+0.0021*Thr-0.0008*Ser-0.0052*Val+0.0242*Ile-0.0101*Leu |
| 0.9338 | 0.9138+0.0042*Tau+0.0020*Pro-0.0082*Val+0.0145*Ile-0.0039*His+0.0026*Orn |
| 0.9337 | 1.1480+0.0046*Tau-0.0052*Asn+0.0024*Pro-0.0072*Val+0.0155*Ile-0.0088*Tyr |
| 0.9330 | 0.7237+0.0038*Tau+0.0015*Thr+0.0003*Gly-0.0049*Val+0.0238*Ile-0.0101*Leu |
| 0.9328 | 0.9893+0.0042*Tau+0.0024*Thr-0.0113*Asn+0.0020*Pro-0.0087*Val+0.0159*Ile |
| 0.9328 | 0.7963+0.0038*Tau+0.0018*Thr-0.0052*Val+0.0241*Ile-0.0100*Leu |
| 0.9327 | 0.8660+0.0042*Tau+0.0008*Gly-0.0038*Val+0.0222*Ile-0.0087*Leu-0.0064*Tyr |
| 0.9326 | 0.7676+0.0040*Tau+0.0008*Gly-0.0043*Val+0.0243*Ile-0.0104*Leu-0.0020*Phe |
| 0.9325 | 0.7769+0.0039*Tau+0.0015*Thr-0.0052*Val+0.0234*Ile-0.0098*Leu+0.0014*Orn |
| 0.9324 | 0.8209+0.0039*Tau+0.0019*Thr-0.0049*Val+0.0237*Ile-0.0097*Leu-0.0022*Trp |
| 0.9322 | 0.9274+0.0039*Tau-0.0050*Val+0.0237*Ile-0.0102*Leu-0.0024*His+0.0014*Lys |
| 0.9322 | 0.7957+0.0037*Tau+0.0020*Pro+0.0209*Ile-0.0153*Leu-0.0031*Phe-0.0024*His |
| 0.9322 | 1.1247+0.0043*Tau+0.0027*Thr-0.0063*Val+0.0149*Ile-0.0088*Tyr-0.0038*His |

# FIG.130

| ROC AUC | FORMULA |
|---|---|
| 0.9321 | 0.7656+0.0038*Tau+0.0015*Thr-0.0062*Val+0.0242*Ile-0.0105*Leu+0.0006*Lys |
| 0.9320 | 0.7872+0.0038*Tau+0.0018*Thr+0.0006*Cit-0.0052*Val+0.0241*Ile-0.0101*Leu |
| 0.9320 | 0.9550+0.0040*Tau-0.0051*Val+0.0220*Ile-0.0086*Leu-0.0024*His+0.0030*Orn |
| 0.9320 | 0.6550+0.0036*Tau-0.0019*Pro+0.0202*Ile-0.0163*Leu-0.0028*His+0.0009*Lys |
| 0.9320 | 1.0406+0.0045*Tau+0.0023*Pro-0.0070*Val+0.0154*Ile-0.0099*Tyr-0.0012*Phe |
| 0.9319 | 0.6596+0.0035*Tau+0.0011*Thr+0.0018*Pro+0.0196*Ile-0.0154*Leu-0.0030*His |
| 0.9319 | 0.8737+0.0042*Tau-0.0020*Pro+0.0004*Gly-0.0079*Val+0.0150*Ile-0.0040*His |
| 0.9318 | 0.8094+0.0038*Tau+0.0020*Thr-0.0002*Ala-0.0051*Val+0.0242*Ile-0.0100*Leu |
| 0.9317 | 0.9507+0.0042*Tau-0.0044*Val+0.0230*Ile-0.0098*Leu-0.0072*Tyr+0.0017*Lys |
| 0.9317 | 1.1622+0.0042*Tau-0.0034*Thr-0.0092*Asn-0.0078*Val+0.0160*Ile-0.0036*His |
| 0.8989 | 1.1054+0.0020*Thr-0.0021*Pro-0.0050*Val+0.0227*Ile-0.0109*Leu-0.0030*His |
| 0.8975 | 1.1241+0.0022*Pro-0.0049*Val+0.0239*Ile-0.0125*Leu-0.0028*His+0.0014*Lys |
| 0.8951 | 1.1504+0.0025*Thr-0.0067*Asn+0.0021*Pro-0.0055*Val+0.0235*Ile-0.0109*Leu |
| 0.8950 | 1.3748+0.0025*Pro-0.0043*Val+0.0224*Ile-0.0103*Leu-0.0058*Tyr-0.0026*His |
| 0.8946 | 1.1363+0.0023*Pro-0.0043*Val+0.0236*Ile-0.0126*Leu-0.0067*Tyr+0.0017*Lys |
| 0.8945 | 1.0327+0.0019*Thr+0.0020*Pro-0.0048*Val+0.0248*Ile-0.0125*Leu-0.0024*Phe |
| 0.8944 | 1.1245+0.0020*Thr+0.0022*Pro-0.0045*Val+0.0226*Ile-0.0109*Leu-0.0065*Tyr |
| 0.8944 | 1.0494+0.0022*Pro-0.0048*Val+0.0258*Ile-0.0139*Leu-0.0022*Phe+0.0014*Lys |
| 0.8940 | 1.1312+0.0022*Pro+0.0004*Gly-0.0045*Val+0.0235*Ile-0.0116*Leu-0.0027*His |
| 0.8937 | 1.1633+0.0026*Thr-0.0023*Ser+0.0021*Pro-0.0053*Val+0.0249*Ile-0.0125*Leu |
| 0.8935 | 1.1950+0.0022*Pro-0.0049*Val+0.0232*Ile-0.0113*Leu-0.0026*His+0.0016*Orn |
| 0.8932 | 1.2485+0.0023*Pro-0.0048*Val+0.0243*Ile-0.0117*Leu-0.0008*Phe-0.0025*His |
| 0.8929 | 0.9526+0.0015*Thr+0.0020*Pro-0.0051*Val+0.0248*Ile-0.0130*Leu+0.0006*Lys |
| 0.8927 | 1.3124-0.0026*Asn+0.0024*Pro-0.0050*Val+0.0239*Ile-0.0112*Leu-0.0025*His |
| 0.8927 | 1.1391-0.0052*Asn+0.0023*Pro-0.0052*Val+0.0250*Ile-0.0131*Leu+0.0017*Lys |
| 0.8926 | 1.2060+0.0023*Pro+0.0016*Cit-0.0048*Val+0.0241*Ile-0.0117*Leu-0.0026*His |
| 0.8924 | 1.0201+0.0022*Thr+0.0022*Pro-0.0005*Ala-0.0049*Val+0.0249*Ile-0.0124*Leu |
| 0.8923 | 1.2709-0.0004*Ser+0.0023*Pro-0.0049*Val+0.0243*Ile-0.0117*Leu-0.0025*His |
| 0.8922 | 1.2291+0.0023*Pro-0.0049*Val+0.0242*Ile-0.0117*Leu-0.0026*His+0.0002*Trp |
| 0.8919 | 1.2319+0.0023*Pro-0.0048*Val+0.0241*Ile-0.0117*Leu-0.0026*His |
| 0.8867 | 1.1404+0.0023*Pro+0.0200*Ile-0.0160*Leu-0.0085*Tyr-0.0030*His+0.0019*Lys |
| 0.8865 | 1.0867+0.0022*Pro+0.0008*Gly+0.0195*Ile-0.0143*Leu-0.0073*Tyr-0.0030*His |
| 0.8853 | 1.1367+0.0022*Thr+0.0022*Pro+0.0187*Ile-0.0142*Leu-0.0082*Tyr-0.0032*His |
| 0.8850 | 1.4308+0.0036*Thr-0.0103*Asn+0.0022*Pro-0.0085*Val+0.0146*Ile-0.0043*His |
| 0.8821 | 1.0072+0.0021*Pro+0.0229*Ile-0.0186*Leu-0.0033*Phe-0.0027*His+0.0016*Lys |
| 0.8821 | 1.0755+0.0020*Pro-0.0115*ABA+0.0207*Ile-0.0175*Leu-0.0025*His+0.0017*Lys |
| 0.8819 | 1.0728+0.0023*Pro+0.0223*Ile-0.0174*Leu-0.0081*Tyr-0.0032*Phe+0.0019*Lys |
| 0.8818 | 1.0686+0.0021*Thr+0.0021*Pro+0.0212*Ile-0.0157*Leu-0.0077*Tyr-0.0032*Phe |
| 0.8817 | 0.9132+0.0022*Pro-0.0005*Gly+0.0213*Ile-0.0170*Leu-0.0082*Tyr+0.0015*Lys |
| 0.8815 | 0.9563+0.0014*Thr-0.0021*Pro+0.0209*Ile-0.0168*Leu-0.0086*Tyr+0.0011*Lys |
| 0.8815 | 1.1497+0.0021*Pro-0.0116*ABA+0.0200*Ile-0.0163*Leu-0.0080*Tyr+0.0021*Lys |
| 0.8814 | 1.3800+0.0029*Thr-0.0023*Pro-0.0071*Val+0.0134*Ile-0.0089*Tyr-0.0045*His |
| 0.8814 | 0.9318+0.0020*Pro-0.0009*Gly+0.0219*Ile-0.0166*Leu-0.0026*Phe-0.0028*His |
| 0.8812 | 0.9976+0.0021*Thr+0.0020*Pro+0.0216*Ile-0.0169*Leu-0.0034*Phe-0.0029*His |
| 0.8811 | 0.8173+0.0020*Pro+0.0007*Gly+0.0214*Ile-0.0177*Leu-0.0031*His+0.0010*Lys |
| 0.8811 | 1.1462+0.0023*Thr+0.0020*Pro-0.0116*ABA+0.0189*Ile-0.0145*Leu-0.0077*Tyr |
| 0.8811 | 1.0182+0.0019*Pro+0.0008*Gly-0.0099*ABA+0.0201*Ile-0.0157*Leu-0.0027*His |
| 0.8810 | 1.0391+0.0023*Pro+0.0216*Ile-0.0171*Leu-0.0080*Tyr-0.0025*Trp+0.0018*Lys |
| 0.8808 | 0.9567-0.0014*Ser+0.0020*Pro+0.0010*Gly+0.0215*Ile-0.0169*Leu-0.0030*His |
| 0.8807 | 1.3861+0.0034*Thr-0.0024*Ser+0.0021*Pro-0.0089*Val+0.0148*Ile-0.0048*His |

## FIG.131

| ROC AUC | FORMULA |
|---|---|
| 0.9415 | 2.88262+0.03220*Tau+0.01802*Pro-0.03814*Val+0.16549*Ile-0.05089*Leu-0.06949*Tyr |
| 0.9348 | 5.30061+0.02990*Tau+0.01898*Pro-0.03562*Val+0.12332*Ile-0.03021*Leu-0.08670*His |
| 0.9346 | 7.35003+0.03448*Tau+0.02115*Pro-0.03596*Val+0.09766*Ile-0.06924*Tyr-0.09292*His |
| 0.9314 | 5.93142+0.03072*Tau+0.01861*Pro-0.04442*Val+0.08882*Ile-0.11473*His+0.02249*Arg |
| 0.9291 | 6.00625+0.03060*Tau+0.00249*Thr+0.01912*Pro-0.04287*Val+0.09495*Ile-0.10315*His |
| 0.9288 | 5.04236+0.03458*Tau-0.06687*Asn+0.01991*Pro-0.05710*Val+0.13821*Ile-0.06584*Tyr |
| 0.9286 | 4.90685+0.02886*Tau-0.03428*Val+0.13631*Ile-0.03889*Leu-0.07807*His+0.01288*Lys |
| 0.9285 | 7.45825+0.03379*Tau-0.03680*Val+0.10200*Ile-0.06660*Tyr-0.09412*His+0.03125*Arg |
| 0.9282 | 5.52574+0.03043*Tau+0.01987*Pro-0.04527*Val+0.08673*Ile-0.11470*His+0.01363*Lys |
| 0.9279 | 6.13016+0.03062*Tau+0.01906*Pro-0.04256*Val+0.01710*Met+0.09238*Ile-0.10539*His |
| 0.9279 | 6.20835+0.03190*Tau+0.02015*Pro+0.08675*Ile-0.04443*Leu-0.07253*Tyr-0.09470*His |
| 0.9273 | 5.38074+0.02932*Tau-0.03521*Val+0.13098*Ile-0.03328*Leu-0.08431*His+0.02539*Arg |
| 0.9268 | 5.92401+0.03083*Tau+0.01928*Pro+0.01376*Cit-0.04289*Val+0.09580*Ile-0.10464*His |
| 0.9268 | 6.10676+0.03054*Tau+0.01910*Pro+0.00058*Ala-0.04276*Val+0.09507*Ile-0.10327*His |
| 0.9266 | 7.18175+0.03315*Tau+0.00974*Thr-0.03520*Val+0.10729*Ile-0.06188*Tyr-0.08085*His |
| 0.9265 | 6.12204+0.03069*Tau+0.01943*Pro-0.04285*Val+0.09613*Ile-0.10235*His |
| 0.9263 | 7.02689+0.03354*Tau-0.03736*Val+0.10096*Ile-0.07028*Tyr-0.09149*His+0.01828*Lys |
| 0.9262 | 5.41670+0.02915*Tau+0.00480*Thr-0.03378*Val+0.13598*Ile-0.03192*Leu-0.07230*His |
| 0.9254 | 6.11636+0.03033*Tau+0.01954*Pro-0.04386*Val+0.09481*Ile-0.10527*His+0.01120*Trp |
| 0.9252 | 8.56957+0.03146*Tau+0.02123*Pro-0.01012*Val+0.07889*Met-0.07167*Tyr-0.12506*His |
| 0.9250 | 7.68236+0.03328*Tau-0.03387*Val+0.06826*Met+0.09679*Ile-0.06302*Tyr-0.08993*His |
| 0.9250 | 5.67667+0.02920*Tau-0.03338*Val+0.03363*Met+0.13003*Ile-0.03107*Leu-0.07729*His |
| 0.9246 | 5.21944+0.02935*Tau+0.00157*Gly-0.03174*Val+0.13876*Ile-0.03478*Leu-0.07076*His |
| 0.9238 | 7.81436+0.03269*Tau+0.01985*Pro+0.03922*Met-0.08700*Tyr-0.14537*His+0.02551*Arg |
| 0.9237 | 5.95008+0.03052*Tau+0.01951*Pro-0.04411*Val+0.09362*Ile+0.01667*Phe-0.10698*His |
| 0.9236 | 5.61527+0.03074*Tau+0.09960*Ile-0.05375*Leu-0.07201*Tyr-0.08814*His+0.01745*Lys |
| 0.9234 | 6.52362+0.03057*Tau+0.02013*Pro-0.00184*Gly-0.04439*Val+0.09852*Ile-0.10065*His |
| 0.9233 | 8.41478+0.03180*Tau+0.02202*Pro-0.01131*Val-0.07520*Tyr-0.13002*His+0.03256*Arg |
| 0.9231 | 6.77865+0.03241*Tau-0.02899*Val+0.13737*Ile-0.02792*Leu-0.05563*Tyr-0.06358*His |
| 0.9229 | 7.93552+0.03232*Tau-0.07235*Asn+0.02144*Pro-0.04560*Val+0.10982*Ile-0.09276*His |
| 0.9225 | 7.59051+0.03202*Tau+0.02413*Pro-0.01509*Val-0.08466*Tyr-0.13314*His+0.02656*Lys |
| 0.9224 | 5.53396+0.02830*Tau+0.00430*Ala-0.03362*Val+0.12759*Ile-0.03026*Leu-0.07994*His |
| 0.9223 | 6.30314+0.03006*Tau-0.04256*Val+0.03450*Met+0.09351*Ile-0.10751*His+0.02469*Arg |
| 0.9222 | 4.23996+0.02651*Tau-0.02741*Ser+0.01896*Pro-0.04480*Val+0.18620*Ile-0.06764*Leu |
| 0.9220 | 6.99389+0.03358*Tau+0.00279*Gly-0.03294*Val+0.10832*Ile-0.05994*Tyr-0.08048*His |
| 0.9219 | 8.70022+0.03074*Tau-0.01048*Val+0.09511*Met-0.06994*Tyr-0.12954*His+0.03161*Arg |
| 0.9216 | 7.48815+0.03329*Tau+0.02011*Pro+0.01681*Ile-0.08854*Tyr-0.14046*His+0.02544*Arg |
| 0.9215 | 3.87760+0.02690*Tau+0.01838*Pro+0.08380*Ile-0.06329*Leu-0.11076*His+0.01218*Lys |
| 0.9214 | 6.29932+0.02983*Tau+0.00472*Thr-0.04081*Val+0.03824*Met+0.09865*Ile-0.09591*His |
| 0.9204 | 5.85738+0.03033*Tau+0.00191*Gly-0.04163*Val+0.09813*Ile-0.10390*His+0.02607*Arg |
| 0.9203 | 5.95705+0.02998*Tau+0.00703*Thr+0.00101*Gly-0.04061*Val+0.10475*Ile-0.09064*His |
| 0.9202 | 3.68533+0.02692*Tau+0.00304*Gly+0.10040*Ile-0.06195*Leu-0.09463*His+0.01049*Lys |
| 0.9201 | 7.53166+0.03212*Tau+0.00742*Ala-0.03335*Val+0.09654*Ile-0.07036*Tyr-0.09062*His |
| 0.9199 | 6.11260+0.02979*Tau-0.04242*Val+0.03762*Met+0.09499*Ile-0.10234*His+0.00973*Lys |
| 0.9196 | 6.18172+0.03011*Tau+0.00238*Thr-0.04315*Val+0.10014*Ile-0.10199*His+0.02510*Arg |
| 0.9195 | 5.84759+0.02976*Tau+0.00519*Thr-0.04313*Val+0.10076*Ile-0.09736*His+0.00995*Lys |
| 0.9195 | 8.22288+0.03229*Tau+0.02098*Pro-0.02471*Cit-0.08556*Tyr-0.13884*His+0.03270*Arg |
| 0.9194 | 7.25574+0.03295*Tau+0.02143*Pro-0.09426*Tyr-0.14880*His+0.01452*Lys+0.01772*Arg |
| 0.9192 | 7.74045+0.03192*Tau+0.01815*Pro+0.00453*Ala-0.09116*Tyr-0.14595*His+0.02788*Arg |
| 0.9192 | 5.54026+0.03006*Tau+0.00218*Gly-0.04154*Val+0.09919*Ile-0.09925*His+0.01152*Lys |

# FIG.132

| ROC AUC | FORMULA |
|---|---|
| 0.9189 | 6.15098+0.02976*Tau+0.00771*Thr-0.04175*Val+0.10558*Ile-0.09055*His+0.00345*Trp |
| 0.9189 | 7.89712+0.03227*Tau+0.02096*Pro-0.08684*Tyr-0.14308*His+0.01045*Trp+0.02707*Arg |
| 0.9188 | 7.69109+0.03125*Tau+0.01501*Thr-0.07401*Asn-0.04424*Val+0.11733*Ile-0.08128*His |
| 0.9187 | 7.74452+0.03246*Tau+0.02085*Pro-0.08588*Tyr-0.01156*Phe-0.14385*His+0.02707*Arg |
| 0.9182 | 6.12948+0.03009*Tau-0.04375*Val+0.09914*Ile-0.10393*His+0.00422*Lys+0.02379*Arg |
| 0.9180 | 6.28643+0.03019*Tau-0.04320*Val+0.10101*Ile-0.10177*His+0.02646*Arg |
| 0.9177 | 7.20100+0.03281*Tau+0.02168*Pro+0.02255*Met-0.09396*Tyr-0.14633*His+0.01887*Lys |
| 0.9175 | 6.28572+0.03024*Tau-0.04306*Val+0.10115*Ile-0.10142*His-0.00171*Trp+0.02660*Arg |
| 0.9175 | 7.10507+0.03174*Tau+0.02304*Pro-0.01139*Leu-0.08912*Tyr-0.13745*His+0.02349*Lys |
| 0.9174 | 6.10028+0.02898*Tau+0.00497*Ala-0.04265*Val+0.09004*Ile-0.11171*His+0.02603*Arg |
| 0.9171 | 6.47959+0.03004*Tau-0.01499*Cit-0.04335*Val+0.10050*Ile-0.10078*His+0.02928*Arg |
| 0.9168 | 8.99793+0.03279*Tau-0.04635*Asn+0.02200*Pro-0.07654*Tyr-0.13920*His+0.03113*Arg |
| 0.9166 | 7.03919+0.02780*Tau+0.01924*Pro-0.02023*Val+0.04527*Met-0.14200*His+0.02487*Arg |
| 0.9164 | 6.35955+0.02890*Tau+0.00449*Ala-0.04025*Val+0.03696*Met+0.09158*Ile-0.10287*His |
| 0.9164 | 6.93939+0.03281*Tau+0.02195*Pro+0.00119*Gly-0.09307*Tyr-0.14430*His+0.02056*Lys |
| 0.9162 | 6.11608+0.02887*Tau+0.00490*Thr+0.00454*Ala-0.04090*Val+0.09739*Ile-0.09781*His |
| 0.9161 | 6.17199+0.03006*Tau-0.04405*Val+0.09950*Ile+0.01179*Phe-0.10462*His+0.02582*Arg |
| 0.9156 | 5.87283+0.02872*Tau+0.00487*Ala-0.04268*Val+0.09212*Ile-0.10604*His+0.01095*Lys |
| 0.9153 | 8.55811+0.03033*Tau+0.02023*Pro+0.00520*Ala-0.00797*Val-0.07450*Tyr-0.12066*His |
| 0.9151 | 6.99679+0.03319*Tau+0.02179*Pro+0.01038*Ile-0.09500*Tyr-0.14353*His+0.01879*Lys |
| 0.9148 | 7.10737+0.03278*Tau+0.02230*Pro+0.00854*Cit-0.09348*Tyr-0.14507*His+0.02035*Lys |
| 0.9148 | 7.09754+0.03224*Tau+0.02010*Pro+0.00371*Ala-0.09811*Tyr-0.14791*His+0.01949*Lys |
| 0.9148 | 9.03137+0.03226*Tau-0.03772*Asn+0.02144*Pro+0.06606*Met-0.07291*Tyr-0.13207*His |
| 0.9141 | 4.26137+0.02572*Tau+0.00345*Ala+0.09365*Ile-0.06196*Leu-0.09961*His+0.01004*Lys |
| 0.9141 | 5.79992+0.03007*Tau+0.01705*Cit-0.04330*Val+0.10231*Ile-0.09924*His+0.01119*Lys |
| 0.9141 | 7.04019+0.03272*Tau+0.02225*Pro-0.09460*Tyr+0.01131*Phe-0.14759*His+0.01980*Lys |
| 0.9139 | 7.19916+0.03261*Tau+0.02237*Pro-0.09446*Tyr-0.14544*His+0.00540*Trp+0.02002*Lys |
| 0.9133 | 6.51563+0.02753*Tau+0.02090*Pro-0.02185*Val+0.03434*Met-0.14103*His+0.01640*Lys |
| 0.9129 | 8.99399+0.02939*Tau-0.02515*Ser+0.02218*Pro-0.04593*Val+0.10215*Ile-0.10330*His |
| 0.9128 | 9.13245+0.03262*Tau+0.02324*Pro-0.01872*Val+0.02449*Leu-0.07177*Tyr-0.12261*His |
| 0.9127 | 6.15470+0.02900*Tau+0.00083*Gly+0.00493*Ala-0.04009*Val+0.09798*Ile-0.09755*His |
| 0.9124 | 6.56808+0.02763*Tau+0.02092*Pro-0.02183*Val-0.14279*His+0.01242*Lys+0.01916*Arg |
| 0.9119 | 10.73818+0.03072*Tau-0.02424*Ser+0.02221*Pro+0.08910*Met-0.08031*Tyr-0.14288*His |
| 0.9118 | 10.22002+0.03119*Tau-0.02086*Ser+0.02294*Pro-0.08362*Tyr-0.14557*His+0.03053*Arg |
| 0.9117 | 8.30516+0.03324*Tau-0.05507*Asn+0.02383*Pro-0.08634*Tyr-0.14464*His+0.02430*Lys |
| 0.9113 | 6.33468+0.02888*Tau+0.00513*Ala-0.04073*Val+0.09884*Ile-0.09694*His |
| 0.9109 | 6.33523+0.02884*Tau+0.00510*Ala-0.04086*Val+0.09874*Ile-0.09723*His+0.00135*Trp |
| 0.9107 | 7.61791+0.03037*Tau+0.00871*Ala-0.09381*Tyr-0.14559*His+0.00949*Lys+0.02558*Arg |
| 0.9087 | 6.90769+0.02757*Tau+0.02042*Pro-0.02049*Val+0.01859*Phe-0.14144*His+0.02633*Arg |
| 0.9084 | 6.65802+0.02796*Tau+0.02181*Pro-0.02556*Val+0.00940*Leu-0.14075*His+0.01796*Lys |
| 0.9083 | 8.91523+0.02881*Tau-0.06706*Asn+0.02225*Pro-0.01804*Val-0.13400*His+0.03277*Arg |
| 0.9082 | 7.89334+0.02915*Tau+0.00955*Ala-0.01175*Val-0.08528*Tyr-0.12920*His+0.02269*Lys |
| 0.9081 | 6.41203+0.02702*Tau+0.02060*Pro+0.00203*Ala-0.02160*Val-0.14025*His+0.01806*Lys |
| 0.9081 | 8.82965+0.02806*Tau+0.01766*Thr-0.02719*Ser-0.04474*Val+0.10901*Ile-0.09249*His |
| 0.9081 | 9.24903+0.03105*Tau-0.04634*Asn+0.02003*Pro+0.00599*Ala-0.07434*Tyr-0.12794*His |
| 0.9075 | 6.38714+0.02750*Tau+0.02177*Pro+0.00743*Cit-0.02102*Val-0.13937*His+0.01844*Lys |
| 0.9072 | 6.49734+0.02722*Tau+0.02190*Pro-0.02170*Val-0.13958*His+0.00695*Trp+0.01815*Lys |
| 0.9071 | 6.26253+0.02731*Tau+0.02188*Pro-0.02326*Val+0.02015*Phe-0.14285*His+0.01813*Lys |
| 0.9060 | 9.46199+0.03147*Tau-0.02117*Ser+0.02454*Pro-0.09337*Tyr-0.14985*His+0.02223*Lys |
| 0.9040 | 8.28152+0.02871*Tau-0.07877*Asn+0.02450*Pro-0.02233*Val-0.13674*His+0.02592*Lys |

# FIG.133

# FIG.134

| ROC AUC | FORMULA |
|---|---|
| 0.9626 | 2.4178+0.1251*Tau+0.0182*Pro-0.0534*Val+0.2866*Ile-0.1248*Leu-0.0544*His |
| 0.9625 | 1.8145+0.1227*Tau+0.0200*Pro-0.0456*Val+0.3160*Ile-0.1511*Leu-0.0717*Tyr |
| 0.9616 | 1.2119+0.1251*Tau+0.0172*Pro-0.0504*Val+0.3339*Ile-0.1598*Leu-0.0420*Phe |
| 0.9600 | -1.0197+0.1145*Tau+0.0160*Thr+0.0142*Pro-0.0528*Val+0.3248*Ile-0.1594*Leu |
| 0.9600 | -0.9931+0.1178*Tau+0.0155*Pro-0.0521*Val+0.3357*Ile-0.1717*Leu+0.0113*Lys |
| 0.9587 | -0.5353+0.1191*Tau+0.0153*Pro-0.0526*Val+0.3115*Ile-0.1543*Leu+0.0198*Orn |
| 0.9586 | 5.3026+0.1155*Tau+0.0203*Pro-0.0707*Val+0.1582*Ile-0.0693*Tyr-0.0753*His |
| 0.9585 | 0.4927+0.1242*Tau+0.0167*Pro-0.0389*Cit-0.0518*Val+0.3184*Ile-0.1514*Leu |
| 0.9582 | -0.6066+0.1156*Tau+0.0161*Thr+0.0167*Pro+0.2822*Ile-0.1944*Leu-0.0908*Tyr |
| 0.9582 | 0.2309+0.1208*Tau+0.0167*Pro-0.0476*Val+0.3106*Ile-0.1528*Leu-0.0223*Trp |
| 0.9582 | 0.1884+0.1198*Tau+0.0171*Pro-0.0018*Ala-0.0501*Val+0.3181*Ile-0.1557*Leu |
| 0.9579 | 1.3580+0.1150*Tau+0.0285*Thr-0.0530*Val+0.2774*Ile-0.1069*Leu-0.0617*His |
| 0.9575 | 3.1037+0.1094*Tau+0.0255*Thr+0.0147*Pro-0.0777*Val+0.1637*Ile-0.1070*His |
| 0.9575 | 0.4622+0.1206*Tau-0.0058*Ser+0.0164*Pro-0.0509*Val+0.3174*Ile-0.1559*Leu |
| 0.9572 | -0.0944+0.1192*Tau+0.0160*Pro-0.0506*Val+0.3165*Ile-0.1554*Leu |
| 0.9572 | 0.0762+0.1193*Tau-0.0056*Asn+0.0161*Pro-0.0506*Val+0.3147*Ile-0.1541*Leu |
| 0.9572 | -0.0683+0.1192*Tau+0.0159*Pro-0.0019*ABA-0.0504*Val+0.3161*Ile-0.1553*Leu |
| 0.9570 | 0.0497+0.1198*Tau+0.0162*Pro-0.0006*Gly-0.0510*Val+0.3173*Ile-0.1556*Leu |
| 0.9567 | 1.4723+0.1191*Tau+0.0196*Pro+0.2596*Ile-0.1747*Leu-0.0774*Tyr-0.0319*His |
| 0.9564 | -0.8776+0.1156*Tau+0.0171*Pro+0.0054*Gly+0.2795*Ile-0.1882*Leu-0.0859*Tyr |
| 0.9562 | 3.3489+0.1164*Tau+0.0161*Pro-0.0789*Val+0.1519*Ile-0.0980*His+0.0421*Orn |
| 0.9561 | 0.9848+0.1192*Tau+0.0192*Pro+0.2781*Ile-0.1856*Leu-0.0715*Tyr-0.0388*Phe |
| 0.9561 | 0.7478+0.1221*Tau+0.0192*Pro-0.0354*Cit+0.2811*Ile-0.1892*Leu-0.0855*Tyr |
| 0.9561 | 1.0291+0.1187*Tau+0.0189*Pro-0.0457*ABA+0.2760*Ile-0.1866*Leu-0.0868*Tyr |
| 0.9559 | -0.5029+0.1162*Tau+0.0183*Pro+0.2933*Ile-0.2071*Leu-0.0881*Tyr+0.0116*Lys |
| 0.9559 | 0.1446+0.1131*Tau+0.0247*Thr-0.0446*Val+0.3010*Ile-0.1305*Leu-0.0614*Tyr |
| 0.9558 | -0.1673+0.1159*Tau+0.0231*Thr-0.0495*Val+0.3267*Ile-0.1447*Leu-0.0375*Phe |
| 0.9558 | 0.7063+0.1210*Tau+0.0199*Pro+0.2776*Ile-0.1862*Leu-0.0774*Tyr-0.0342*Trp |
| 0.9555 | -0.2086+0.1194*Tau+0.0185*Pro+0.2730*Ile-0.1914*Leu-0.0868*Tyr+0.0173*Orn |
| 0.9554 | -0.1465+0.1185*Tau+0.0134*Asn+0.0185*Pro+0.2840*Ile-0.1946*Leu-0.0853*Tyr |
| 0.9551 | 0.0720+0.1187*Tau+0.0183*Pro+0.0012*Ala+0.2796*Ile-0.1919*Leu-0.0864*Tyr |
| 0.9546 | 0.1765+0.1186*Tau+0.0190*Pro+0.2799*Ile-0.1917*Leu-0.0836*Tyr |
| 0.9545 | 0.2503+0.1188*Tau-0.0009*Ser+0.0190*Pro+0.2799*Ile-0.1918*Leu-0.0835*Tyr |
| 0.9543 | 0.9801+0.1197*Tau-0.0549*Val+0.3035*Ile-0.1266*Leu-0.0602*His+0.0240*Lys |
| 0.9543 | 2.7190+0.1113*Tau+0.0161*Pro+0.0069*Gly-0.0709*Val+0.1537*Ile-0.0989*His |
| 0.9542 | 1.5258+0.1222*Tau-0.0550*Val+0.2656*Ile-0.1023*Leu-0.0494*His+0.0415*Orn |
| 0.9539 | -0.6388+0.1166*Tau+0.0255*Thr-0.0512*Cit-0.0516*Val+0.3165*Ile-0.1394*Leu |
| 0.9536 | 0.1037+0.1129*Tau+0.0279*Thr-0.0166*Ser-0.0511*Val+0.3142*Ile-0.1439*Leu |
| 0.9535 | 0.7026+0.1158*Tau+0.0077*Gly-0.0446*Val+0.2678*Ile-0.1067*Leu-0.0501*His |
| 0.9535 | -1.0694+0.1134*Tau+0.0188*Thr+0.0135*Pro+0.2526*Ile-0.1805*Leu-0.0555*His |
| 0.9533 | -1.3597+0.1127*Tau+0.0177*Thr-0.0511*Val+0.3071*Ile-0.1410*Leu+0.0166*Orn |
| 0.9532 | -1.0721+0.1119*Tau+0.0216*Thr-0.0008*Ala-0.0495*Val+0.3134*Ile-0.1425*Leu |
| 0.9530 | -1.5662+0.1117*Tau+0.0184*Thr-0.0505*Val+0.3217*Ile-0.1507*Leu+0.0067*Lys |
| 0.9530 | -0.0474+0.1174*Tau-0.0452*Val+0.3173*Ile-0.1438*Leu-0.0554*Tyr+0.0173*Lys |
| 0.9530 | -0.9706+0.1128*Tau+0.0215*Thr-0.0475*Val+0.3079*Ile-0.1407*Leu-0.0149*Trp |
| 0.9529 | 3.7820+0.1073*Tau+0.0324*Thr-0.0666*Val+0.1713*Ile-0.0627*Tyr-0.0852*His |
| 0.9528 | -0.1774+0.1106*Tau+0.0285*Thr-0.0445*Asn-0.0517*Val+0.3035*Ile-0.1330*Leu |
| 0.9528 | -1.1610+0.1120*Tau+0.0209*Thr-0.0497*Val+0.3127*Ile-0.1427*Leu |
| 0.9528 | -1.4239+0.1126*Tau+0.0136*Pro+0.0076*Gly+0.2443*Ile-0.1698*Leu-0.0544*His |
| 0.9525 | -0.9731+0.1125*Tau+0.0219*Thr-0.0010*Gly-0.0505*Val+0.3138*Ile-0.1426*Leu |

# FIG.135

| ROC AUC | FORMULA |
|---|---|
| 0.9522 | 1.5062+0.1009*Tau+0.0142*Thr-0.0161*Pro-0.0760*Val+0.1588*Ile-0.0903*Tyr |
| 0.9520 | 2.9289+0.1081*Tau+0.0184*Pro-0.0749*Val+0.1644*Ile-0.0860*Tyr-0.0233*Phe |
| 0.9519 | -0.1796+0.1150*Tau+0.0064*Gly-0.0388*Val+0.2935*Ile-0.1275*Leu-0.0523*Tyr |
| 0.9511 | 0.2547+0.1199*Tau+0.0159*Pro-0.2678*Ile-0.1845*Leu-0.0424*Phe-0.0291*His |
| 0.9509 | -0.0040+0.1178*Tau+0.0051*Gly-0.0436*Val+0.3112*Ile-0.1375*Leu-0.0352*Phe |
| 0.9509 | -1.7977+0.1165*Tau+0.0138*Pro+0.0046*Gly+0.2869*Ile-0.1984*Leu-0.0490*Phe |
| 0.9505 | -1.0062+0.1163*Tau+0.0148*Pro+0.2675*Ile-0.1975*Leu-0.0570*His+0.0160*Lys |
| 0.9504 | 3.8658+0.1049*Tau+0.0406*Thr-0.0645*Asn-0.0735*Val+0.1683*Ile-0.0866*His |
| 0.9504 | 3.2822+0.1063*Tau-0.0356*Asn+0.0193*Pro-0.0739*Val+0.1540*Ile-0.0820*Tyr |
| 0.9474 | 2.0349+0.0945*Tau+0.0300*Thr-0.1129*Asn+0.0142*Pro-0.0838*Val+0.1508*Ile |
| 0.9057 | 5.9335+0.0156*Pro-0.0423*Val+0.2131*Ile-0.0994*Leu-0.0671*His+0.0238*Lys |
| 0.9046 | 5.9514+0.0235*Thr+0.0139*Pro-0.0419*Val+0.1970*Ile-0.0786*Leu-0.0619*His |
| 0.8988 | 7.2847+0.0179*Pro-0.0341*Val+0.1921*Ile-0.0803*Leu-0.0319*Tyr-0.0425*His |
| 0.8982 | 6.4887+0.0164*Pro-0.0394*Val+0.1870*Ile-0.0793*Leu-0.0508*His+0.0197*Orn |
| 0.8978 | 4.6530+0.0273*Thr-0.0196*Ser+0.0132*Pro-0.0429*Val+0.2265*Ile-0.1081*Leu |
| 0.8977 | 5.7454+0.0153*Pro+0.0055*Gly-0.0342*Val+0.1903*Ile-0.0817*Leu-0.0544*His |
| 0.8976 | 4.0062+0.0200*Thr+0.0143*Pro-0.0358*Val+0.2160*Ile-0.1009*Leu-0.0412*Tyr |
| 0.8974 | 6.6496+0.0167*Pro-0.0399*Val+0.1993*Ile-0.0840*Leu-0.0516*His+0.0174*Trp |
| 0.8974 | 4.2822+0.0258*Thr-0.0537*Asn+0.0135*Pro-0.0419*Val+0.2199*Ile-0.1001*Leu |
| 0.8971 | 6.9519+0.0170*Pro-0.0369*Val+0.2020*Ile-0.0860*Leu-0.0124*Phe-0.0435*His |
| 0.8970 | 3.7226+0.0194*Thr+0.0131*Pro-0.0383*Val+0.2307*Ile-0.1097*Leu-0.0224*Phe |
| 0.8967 | 6.5233+0.0168*Pro+0.0182*Cit-0.0375*Val+0.1951*Ile-0.0839*Leu-0.0494*His |
| 0.8961 | 3.9205+0.0158*Pro-0.0336*Val+0.2264*Ile-0.1194*Leu-0.0436*Tyr+0.0181*Lys |
| 0.8960 | 6.4928+0.0120*Asn+0.0165*Pro-0.0374*Val+0.1987*Ile-0.0852*Leu-0.0492*His |
| 0.8958 | 6.7229+0.0168*Pro-0.0376*Val+0.1971*Ile-0.0838*Leu-0.0466*His |
| 0.8957 | 3.7288+0.0145*Pro-0.0363*Val+0.2441*Ile-0.1289*Leu-0.0269*Phe+0.0177*Lys |
| 0.8955 | 6.5339+0.0021*Ser+0.0167*Pro-0.0374*Val+0.1967*Ile-0.0835*Leu-0.0473*His |
| 0.8953 | 2.4804+0.0151*Thr+0.0127*Pro-0.0410*Val+0.2361*Ile-0.1204*Leu+0.0108*Lys |
| 0.8950 | 7.7050+0.0270*Thr+0.0155*Pro-0.0523*Val+0.1177*Ile-0.0477*Tyr-0.0825*His |
| 0.8941 | 3.4158+0.0221*Thr+0.0146*Pro-0.0038*Ala-0.0382*Val+0.2243*Ile-0.1082*Leu |
| 0.8941 | 3.8981-0.0354*Asn+0.0150*Pro-0.0390*Val+0.2303*Ile-0.1206*Leu+0.0188*Lys |
| 0.8928 | 8.3110+0.0336*Thr-0.0186*Ser+0.0143*Pro-0.0615*Val+0.1242*Ile-0.0882*His |
| 0.8926 | 7.9365+0.0322*Thr-0.0574*Asn+0.0146*Pro-0.0598*Val+0.1235*Ile-0.0796*His |
| 0.8913 | 4.9864+0.0162*Pro+0.1761*Ile-0.1297*Leu-0.0542*Tyr-0.0543*His+0.0229*Lys |
| 0.8908 | 4.5672+0.0149*Pro+0.0081*Gly+0.1661*Ile-0.1072*Leu-0.0467*Tyr-0.0472*His |
| 0.8908 | 4.9318+0.0211*Thr+0.0142*Pro+0.1656*Ile-0.1112*Leu-0.0509*Tyr-0.0484*His |
| 0.8886 | 4.5909+0.0136*Pro-0.0551*ABA+0.1732*Ile-0.1344*Leu-0.0567*His+0.0211*Lys |
| 0.8879 | 3.9001+0.0134*Pro+0.0082*Gly+0.1765*Ile-0.1186*Leu-0.0208*Phe-0.0477*His |
| 0.8876 | 3.1264+0.0125*Pro+0.0062*Gly+0.1765*Ile-0.1325*Leu-0.0643*His+0.0167*Lys |
| 0.8869 | 4.0279+0.0130*Pro+0.0080*Gly-0.0406*ABA+0.1625*Ile-0.1116*Leu-0.0509*His |
| 0.8868 | 4.4139+0.0144*Pro+0.1943*Ile-0.1448*Leu-0.0288*Phe-0.0519*His+0.0213*Lys |
| 0.8866 | 3.9408-0.0081*Ser+0.0132*Pro+0.0099*Gly+0.1690*Ile-0.1170*Leu-0.0531*His |
| 0.8850 | 3.5875+0.0146*Pro-0.0534*ABA+0.1915*Ile-0.1430*Leu-0.0552*Tyr+0.0186*Lys |
| 0.8849 | 3.5249+0.0154*Pro+0.2082*Ile-0.1512*Leu-0.0488*Tyr-0.0313*Phe+0.0190*Lys |
| 0.8846 | 3.6995+0.0184*Thr+0.0128*Pro-0.0551*ABA+0.1772*Ile-0.1238*Leu-0.0525*Tyr |
| 0.8842 | 4.3061+0.0198*Thr+0.0127*Pro+0.1797*Ile-0.1253*Leu-0.0231*Phe-0.0478*His |
| 0.8834 | 2.2272+0.0141*Pro+0.0039*Gly+0.2001*Ile-0.1464*Leu-0.0563*Tyr+0.0148*Lys |
| 0.8834 | 3.6029+0.0171*Thr+0.0137*Pro+0.1925*Ile-0.1316*Leu-0.0471*Tyr-0.0258*Phe |
| 0.8833 | 2.4950+0.0115*Thr+0.0137*Pro+0.1988*Ile-0.1467*Leu-0.0585*Tyr+0.0127*Lys |
| 0.8827 | 3.1437+0.0155*Pro+0.2005*Ile-0.1479*Leu-0.0541*Tyr-0.0146*Trp+0.0176*Lys |

# FIG.136

| ROC AUC | FORMULA |
|---|---|
| 0.9584 | 0.13643+0.12053*Tau+0.01261*Pro-0.04926*Val+0.27628*Ile-0.09394*Leu-0.08369*Tyr |
| 0.9544 | 7.69081+0.12719*Tau+0.01721*Pro-0.05314*Val+0.15311*Ile-0.07387*Tyr-0.14981*His |
| 0.9526 | 5.23801+0.12348*Tau+0.01478*Pro-0.04116*Val+0.19477*Ile-0.05773*Leu-0.14474*His |
| 0.9515 | 5.69609+0.12782*Tau+0.01522*Pro-0.06013*Val+0.14065*Ile-0.18827*His+0.03543*Arg |
| 0.9508 | -0.59221+0.12345*Tau-0.05211*Val+0.30903*Ile-0.10785*Leu-0.08974*Tyr+0.01576*Lys |
| 0.9507 | 6.02339+0.11934*Tau+0.01481*Thr+0.01424*Pro-0.05546*Val+0.13989*Ile-0.17828*His |
| 0.9504 | -0.02586+0.12757*Tau-0.05164*Val+0.29533*Ile-0.09527*Leu-0.0857*Tyr+0.01313*Arg |
| 0.9489 | 1.15166+0.11706*Tau-0.09151*Asn+0.01398*Pro-0.06023*Val+0.27334*Ile-0.08809*Leu |
| 0.9486 | 2.26596+0.12411*Tau-0.03873*Ser+0.01620*Pro-0.06202*Val+0.29029*Ile-0.10753*Leu |
| 0.9485 | 6.72537+0.11850*Tau+0.01499*Pro-0.05337*Val+0.07376*Met+0.12503*Ile-0.18366*His |
| 0.9484 | 6.94716+0.12776*Tau+0.01781*Pro+0.16407*Ile-0.08506*Leu-0.07642*Tyr-0.16523*His |
| 0.9484 | 7.10814+0.13118*Tau+0.02031*Thr-0.05880*Val+0.18436*Ile-0.07652*Tyr-0.15274*His |
| 0.9473 | 5.91718+0.12021*Tau+0.01620*Pro+0.02200*Cit-0.05522*Val+0.14041*Ile-0.16745*His |
| 0.9470 | 6.80190+0.11946*Tau-0.04235*Asn+0.01721*Pro-0.05765*Val+0.14591*Ile-0.14496*His |
| 0.9470 | 6.20288+0.11947*Tau+0.01658*Pro-0.05497*Val+0.13844*Ile-0.16183*His |
| 0.9470 | 6.19604+0.11947*Tau+0.01656*Pro+0.00003*Gly-0.05495*Val+0.13843*Ile-0.16187*His |
| 0.9469 | 3.73044+0.12660*Tau-0.04973*Val+0.24803*Ile-0.08001*Leu-0.16158*His+0.03169*Lys |
| 0.9468 | 6.09563+0.11907*Tau+0.01662*Pro-0.05487*Val+0.13601*Ile+0.00755*Phe-0.16409*His |
| 0.9468 | 4.41311+0.13584*Tau-0.04930*Val+0.22542*Ile-0.05737*Leu-0.15494*His+0.03156*Arg |
| 0.9467 | 6.07891+0.11824*Tau+0.01560*Pro+0.00248*Ala-0.05377*Val+0.13281*Ile-0.16684*His |
| 0.9467 | 4.83149+0.12905*Tau+0.01523*Thr-0.04612*Val+0.22069*Ile-0.05621*Leu-0.14952*His |
| 0.9467 | 7.01147+0.13840*Tau-0.06101*Val+0.18327*Ile-0.08080*Tyr-0.15904*His+0.03796*Arg |
| 0.9467 | 5.75627+0.11683*Tau+0.01802*Pro-0.06560*Val+0.13776*Ile-0.20295*His+0.03132*Lys |
| 0.9463 | 8.50392+0.13025*Tau-0.05418*Val+0.16600*Met+0.15827*Ile-0.09101*Tyr-0.17491*His |
| 0.9462 | 6.20274+0.11862*Tau+0.01668*Pro-0.05524*Val+0.13713*Ile-0.16558*His+0.00961*Trp |
| 0.9453 | 6.00371+0.13668*Tau-0.04214*Val+0.24424*Ile-0.06235*Leu-0.06743*Tyr-0.11601*His |
| 0.9452 | 6.80202+0.12910*Tau-0.06540*Val+0.18716*Ile-0.08768*Tyr-0.16717*His+0.03156*Lys |
| 0.9445 | 6.17965+0.13390*Tau+0.00540*Gly-0.05362*Val+0.18398*Ile-0.07396*Tyr-0.14311*His |
| 0.9443 | 7.93293+0.12328*Tau-0.02023*Ser+0.01919*Pro-0.05920*Val+0.14250*Ile-0.15385*His |
| 0.9443 | 5.37123+0.13065*Tau+0.01185*Thr-0.06272*Val+0.16854*Ile-0.18411*His+0.02950*Arg |
| 0.9439 | 6.98060+0.12789*Tau+0.00752*Ala-0.05282*Val+0.16532*Ile-0.07911*Tyr-0.14732*His |
| 0.9437 | 3.25610+0.13545*Tau-0.02495*Ser-0.05201*Val+0.31399*Ile-0.10349*Leu-0.08225*Tyr |
| 0.9435 | 4.27896+0.12956*Tau+0.00237*Gly-0.04161*Val+0.23160*Ile-0.06730*Leu-0.13047*His |
| 0.9431 | 4.59885+0.12703*Tau+0.00363*Ala-0.04201*Val+0.22032*Ile-0.06416*Leu-0.13535*His |
| 0.9430 | 5.45254+0.12803*Tau-0.04190*Val+0.08828*Met+0.21048*Ile-0.06218*Leu-0.15679*His |
| 0.9429 | 7.36258+0.12427*Tau-0.03456*Thr-0.09911*Asn-0.06625*Val+0.18278*Ile-0.15131*His |
| 0.9428 | 4.66721+0.13018*Tau-0.04308*Val+0.23770*Ile-0.07018*Leu+0.00179*Phe-0.12619*His |
| 0.9427 | 4.69650+0.13357*Tau+0.00384*Gly-0.06089*Val+0.16784*Ile-0.18424*His+0.03723*Arg |
| 0.9427 | 4.68449+0.13017*Tau-0.04316*Val+0.23806*Ile-0.07013*Leu-0.12570*His+0.00072*Trp |
| 0.9427 | 4.68673+0.13023*Tau-0.04315*Val+0.23827*Ile-0.07020*Leu-0.12543*His |
| 0.9425 | 5.68259+0.12503*Tau+0.01946*Thr+0.00080*Gly-0.05819*Val+0.16414*Ile-0.17297*His |
| 0.9424 | 6.07860+0.13126*Tau-0.06072*Val+0.08429*Met+0.15177*Ile-0.20137*His+0.03447*Arg |
| 0.9420 | 5.69056+0.13296*Tau-0.02240*Cit-0.06486*Val+0.17263*Ile-0.17461*His+0.04232*Arg |
| 0.9420 | 5.82644+0.12499*Tau+0.02015*Thr-0.05885*Val+0.16503*Ile-0.17224*His |
| 0.9420 | 6.08123+0.13534*Tau+0.18089*Ile-0.08622*Leu-0.08159*Tyr-0.16361*His+0.02482*Arg |
| 0.9420 | 6.28411+0.12426*Tau+0.01590*Thr-0.05660*Val+0.06376*Met+0.15108*Ile-0.18765*His |
| 0.9419 | 5.82651+0.12519*Tau+0.02027*Thr-0.05879*Val+0.16532*Ile-0.17121*His-0.00290*Trp |
| 0.9418 | 5.02976+0.13042*Tau-0.02141*Asn-0.04500*Val+0.24246*Ile-0.06969*Leu-0.11666*His |
| 0.9418 | 5.78085+0.12481*Tau+0.02012*Thr-0.05874*Val+0.16386*Ile+0.00330*Phe-0.17325*His |
| 0.9416 | 5.74039+0.12521*Tau+0.01969*Thr+0.00614*Cit-0.05889*Val+0.16559*Ile-0.17329*His |

# FIG.137

| ROC AUC | FORMULA |
|---|---|
| 0.9415 | 4.18436+0.13319*Tau+0.03749*Cit-0.04312*Val+0.24339*Ile-0.07191*Leu-0.13714*His |
| 0.9414 | 5.39600+0.13337*Tau-0.06395*Val+0.17357*Ile-0.17497*His-0.00565*Trp+0.03837*Arg |
| 0.9414 | 6.19322+0.13706*Tau-0.06046*Asn-0.06861*Val+0.18491*Ile-0.16087*His+0.04521*Arg |
| 0.9413 | 5.09263+0.11654*Tau+0.01655*Pro+0.14990*Ile-0.10078*Leu-0.21511*His+0.02581*Lys |
| 0.9413 | 5.80873+0.13556*Tau-0.01608*Ser-0.04479*Val+0.25521*Ile-0.07935*Leu-0.11539*His |
| 0.9413 | 5.60293+0.12322*Tau+0.01577*Pro+0.13454*Ile-0.08274*Leu-0.20404*His+0.02244*Arg |
| 0.9412 | 5.50397+0.12374*Tau+0.01537*Thr-0.06512*Val+0.16625*Ile-0.19296*His+0.02056*Lys |
| 0.9411 | 7.31237+0.13339*Tau-0.05710*Val+0.18792*Ile-0.07510*Tyr-0.13289*His |
| 0.9411 | 7.30352+0.13246*Tau-0.05729*Val+0.18635*Ile-0.07643*Tyr-0.13634*His+0.01125*Trp |
| 0.9410 | 5.67467+0.12286*Tau+0.01805*Thr+0.00349*Ala-0.05636*Val+0.15383*Ile-0.17826*His |
| 0.9410 | 7.13891+0.13317*Tau+0.01322*Cit-0.05741*Val+0.18836*Ile-0.07395*Tyr-0.13607*His |
| 0.9410 | 5.40080+0.13282*Tau-0.06409*Val+0.17292*Ile-0.17674*His+0.03796*Arg |
| 0.9410 | 6.76810+0.12889*Tau+0.16121*Met+0.17710*Ile-0.08961*Leu-0.09163*Tyr-0.17690*His |
| 0.9410 | 5.19610+0.13029*Tau-0.06850*Val+0.17384*Ile-0.19378*His+0.01715*Lys+0.03068*Arg |
| 0.9410 | 5.36737+0.13263*Tau-0.06400*Val+0.17197*Ile+0.00278*Phe-0.17761*His+0.03791*Arg |
| 0.9409 | 8.38151+0.13661*Tau-0.01168*Ser-0.05886*Val+0.19367*Ile-0.07915*Tyr-0.12848*His |
| 0.9408 | 5.21752+0.12934*Tau+0.00500*Ala-0.06134*Val+0.15902*Ile-0.18820*His+0.03665*Arg |
| 0.9406 | 6.06520+0.12991*Tau+0.01075*Thr+0.18532*Ile-0.08586*Leu-0.07561*Tyr-0.15622*His |
| 0.9406 | 5.32389+0.12732*Tau+0.19958*Ile-0.10537*Leu-0.08471*Tyr-0.16811*His+0.02707*Lys |
| 0.9398 | 7.66149+0.13430*Tau-0.02297*Asn-0.05826*Val+0.19299*Ile-0.07444*Tyr-0.12597*His |
| 0.9396 | 8.92422+0.13116*Tau+0.03600*Thr-0.03477*Ser-0.06457*Val+0.17372*Ile-0.18160*His |
| 0.9393 | 7.10445+0.13488*Tau-0.05433*Val+0.17105*Ile-0.09353*Tyr+0.06112*Phe-0.15605*His |
| 0.9392 | 6.71954+0.12365*Tau-0.05481*Val+0.11508*Met+0.14287*Ile-0.18339*His |
| 0.9390 | 4.55265+0.12452*Tau+0.00486*Gly-0.06273*Val+0.16330*Ile-0.19342*His+0.02755*Lys |
| 0.9389 | 5.82700+0.11947*Tau+0.00529*Thr+0.01528*Pro+0.14418*Ile-0.08427*Leu-0.19091*His |
| 0.9389 | 6.53169+0.13718*Tau-0.01335*Ser-0.06766*Val+0.18143*Ile-0.17560*His+0.04133*Arg |
| 0.9388 | 6.46183+0.12118*Tau+0.00363*Ala-0.05338*Val+0.09466*Met+0.13637*Ile-0.18561*His |
| 0.9385 | 6.33017+0.12518*Tau+0.03237*Cit-0.05483*Val+0.11385*Met+0.14445*Ile-0.19284*His |
| 0.9383 | 6.08202+0.12306*Tau-0.06308*Val+0.08967*Met+0.15165*Ile-0.20553*His+0.02286*Lys |
| 0.9379 | 6.17921+0.11927*Tau+0.01515*Pro+0.03734*Met+0.14099*Ile-0.08506*Leu-0.19547*His |
| 0.9377 | 5.40763+0.12078*Tau+0.01557*Pro+0.02590*Cit+0.15282*Ile-0.08908*Leu-0.18332*His |
| 0.9372 | 8.84707+0.12777*Tau-0.02114*Ser-0.05667*Val+0.16107*Met+0.13958*Ile-0.19044*His |
| 0.9371 | 5.82484+0.12672*Tau+0.00674*Ala+0.17547*Ile-0.08435*Leu-0.08057*Tyr-0.15283*His |
| 0.9370 | 5.86598+0.11973*Tau+0.01610*Pro+0.14992*Ile-0.08833*Leu-0.18323*His |
| 0.9369 | 6.03646+0.13137*Tau+0.20161*Ile-0.09479*Leu-0.07535*Tyr-0.13958*His |
| 0.9365 | 5.52867+0.12353*Tau-0.06598*Val+0.16868*Ile-0.18369*His+0.02689*Lys |
| 0.9359 | 6.07515+0.12518*Tau-0.04884*Asn-0.06976*Val+0.17737*Ile-0.17099*His+0.03126*Lys |
| 0.9359 | 5.69565+0.12160*Tau+0.00574*Ala-0.05515*Val+0.15181*Ile-0.16020*His |
| 0.9355 | 7.97282+0.12711*Tau-0.02545*Ser+0.01895*Pro+0.16578*Ile-0.10210*Leu-0.17580*His |
| 0.9355 | 5.26180+0.12027*Tau+0.00513*Ala-0.06395*Val+0.15505*Ile-0.19312*His+0.02629*Lys |
| 0.9345 | 6.35253+0.12106*Tau-0.04231*Asn+0.00696*Ala-0.05740*Val+0.15620*Ile-0.14637*His |
| 0.9344 | 7.76625+0.11424*Tau+0.02426*Pro-0.02873*Val-0.24581*His+0.02465*Lys+0.02632*Arg |
| 0.9343 | 5.95776+0.12548*Tau-0.05854*Val+0.16990*Ile-0.14696*His |
| 0.9338 | 5.58350+0.13419*Tau+0.19443*Ile-0.09542*Leu-0.09480*Tyr+0.06463*Phe-0.16086*His |
| 0.9327 | 8.16983+0.11542*Tau+0.02275*Pro-0.02077*Val-0.22393*His+0.03759*Arg |
| 0.9311 | 8.11632+0.10863*Tau+0.00609*Thr+0.02375*Pro-0.02732*Val-0.24252*His+0.03060*Lys |
| 0.9293 | 8.19716+0.10897*Tau+0.02446*Pro-0.02762*Val-0.23950*His+0.03256*Lys |
| 0.9292 | 8.21229+0.10958*Tau+0.02414*Pro-0.03227*Val+0.00963*Leu-0.24014*His+0.03213*Lys |
| 0.9282 | 10.03728+0.11120*Tau-0.01796*Ser+0.02591*Pro-0.02956*Val-0.23619*His+0.03260*Lys |
| 0.9270 | 7.97237+0.11770*Tau+0.01030*Ala-0.11637*Tyr+0.07167*Phe-0.24001*His+0.03288*Arg |

FIG.138

# FIG.139

| ROC AUC | FORMULA |
|---|---|
| 0.9100 | 0.7029+0.0097*Tau-0.0004*Ala+0.0207*Ile-0.0126*Leu-0.0054*Tyr-0.0022*His |
| 0.9094 | 0.7133+0.0096*Tau-0.0013*Val+0.0202*Ile-0.0107*Leu-0.0069*Tyr-0.0024*His |
| 0.9092 | 0.6187+0.0096*Tau+0.0199*Ile-0.0128*Leu-0.0071*Tyr-0.0026*His+0.0009*Lys |
| 0.9090 | 0.6460+0.0097*Tau-0.0004*Ala-0.0013*Val+0.0226*Ile-0.0121*Leu-0.0052*Tyr |
| 0.9090 | 0.5439+0.0096*Tau-0.0005*Ala+0.0226*Ile-0.0144*Leu-0.0063*Tyr+0.0010*Lys |
| 0.9089 | 0.7695+0.0097*Tau-0.0056*Asn-0.0018*Val+0.0219*Ile-0.0111*Leu-0.0053*Tyr |
| 0.9089 | 0.6768+0.0096*Tau+0.0198*Ile-0.0123*Leu-0.0064*Tyr-0.0025*His |
| 0.9089 | 0.6854+0.0096*Tau-0.0015*Met+0.0200*Ile-0.0122*Leu-0.0062*Tyr-0.0025*His |
| 0.9089 | 0.6780+0.0096*Tau+0.0004*Glu+0.0197*Ile-0.0123*Leu-0.0065*Tyr-0.0025*His |
| 0.9089 | 0.6227+0.0096*Tau+0.0200*Ile-0.0127*Leu-0.0070*Tyr+0.0023*Phe-0.0026*His |
| 0.9088 | 0.6737+0.0096*Tau+0.0198*Ile-0.0123*Leu-0.0065*Tyr-0.0025*His+0.0003*Trp |
| 0.9088 | 0.7765+0.0097*Tau-0.0010*Ser+0.0199*Ile-0.0124*Leu-0.0064*Tyr-0.0024*His |
| 0.9088 | 0.6976+0.0097*Tau-0.0047*Cit-0.0013*Val+0.0214*Ile-0.0115*Leu-0.0061*Tyr |
| 0.9088 | 0.7172+0.0097*Tau-0.0001*Gln+0.0199*Ile-0.0122*Leu-0.0063*Tyr-0.0025*His |
| 0.9087 | 0.7104+0.0098*Tau-0.0010*Ser-0.0004*Ala+0.0223*Ile-0.0138*Leu-0.0057*Tyr |
| 0.9087 | 0.7037+0.0097*Tau-0.0039*Asn-0.0004*Ala+0.0222*Ile-0.0135*Leu-0.0054*Tyr |
| 0.9087 | 0.6878+0.0096*Tau+0.0200*Ile-0.0123*Leu-0.0063*Tyr-0.0025*His-0.0005*Orn |
| 0.9087 | 0.7518+0.0097*Tau-0.0043*Cit+0.0196*Ile-0.0120*Leu-0.0062*Tyr-0.0023*His |
| 0.9086 | 0.6454+0.0096*Tau+0.0024*ABA+0.0201*Ile-0.0125*Leu-0.0065*Tyr-0.0026*His |
| 0.9086 | 0.7013+0.0098*Tau-0.0004*Ala-0.0048*Cit+0.0219*Ile-0.0133*Leu-0.0055*Tyr |
| 0.9085 | 0.7733+0.0097*Tau-0.0041*Asn+0.0200*Ile-0.0122*Leu-0.0058*Tyr-0.0022*His |
| 0.9085 | 0.5633+0.0097*Tau-0.0004*Ala+0.0225*Ile-0.0141*Leu-0.0062*Tyr+0.0018*Phe |
| 0.9084 | 0.5496+0.0095*Tau-0.0016*Val+0.0221*Ile-0.0122*Leu-0.0070*Tyr+0.0009*Lys |
| 0.9084 | 0.5827+0.0097*Tau-0.0005*Ala+0.0017*ABA+0.0226*Ile-0.0140*Leu-0.0057*Tyr |
| 0.9084 | 0.7358+0.0097*Tau-0.0012*Ser-0.0015*Val+0.0219*Ile-0.0118*Leu-0.0062*Tyr |
| 0.9082 | 0.6025+0.0097*Tau-0.0056*Cit+0.0212*Ile-0.0138*Leu-0.0074*Tyr+0.0010*Lys |
| 0.9082 | 0.6464+0.0096*Tau-0.0063*Asn+0.0218*Ile-0.0141*Leu-0.0069*Tyr+0.0012*Lys |
| 0.9081 | 0.6116+0.0096*Tau+0.0006*Glu-0.0005*Ala+0.0222*Ile-0.0138*Leu-0.0059*Tyr |
| 0.9080 | 0.5756+0.0096*Tau+0.0025*ABA-0.0016*Val+0.0222*Ile-0.0119*Leu-0.0064*Tyr |
| 0.9080 | 0.6384+0.0097*Tau-0.0001*Gln-0.0004*Ala+0.0223*Ile-0.0137*Leu-0.0057*Tyr |
| 0.9080 | 0.6197+0.0097*Tau-0.0004*Ala+0.0224*Ile-0.0138*Leu-0.0056*Tyr-0.0005*Orn |
| 0.9080 | 0.6078+0.0097*Tau-0.0005*Ala+0.0223*Ile-0.0138*Leu-0.0057*Tyr+0.0000*Trp |
| 0.9080 | 0.6080+0.0097*Tau-0.0005*Ala+0.0223*Ile-0.0137*Leu-0.0057*Tyr |
| 0.9079 | 0.6431+0.0097*Tau-0.0052*Asn+0.0218*Ile-0.0138*Leu-0.0068*Tyr+0.0024*Phe |
| 0.9079 | 0.7510+0.0097*Tau-0.0039*Asn-0.0041*Cit+0.0212*Ile-0.0130*Leu-0.0061*Tyr |
| 0.9079 | 0.5652+0.0096*Tau-0.0014*Val+0.0220*Ile-0.0121*Leu-0.0069*Tyr+0.0016*Phe |
| 0.9079 | 0.6079+0.0096*Tau-0.0015*Val+0.0219*Ile-0.0117*Leu-0.0064*Tyr |
| 0.9078 | 0.6159+0.0097*Tau-0.0004*Ala-0.0012*Met+0.0224*Ile-0.0137*Leu-0.0056*Tyr |
| 0.9077 | 0.6354+0.0097*Tau-0.0013*Ser+0.0217*Ile-0.0142*Leu-0.0075*Tyr+0.0009*Lys |
| 0.9077 | 0.6110+0.0096*Tau-0.0015*Val+0.0219*Ile-0.0117*Leu-0.0063*Tyr-0.0002*Trp |
| 0.9077 | 0.6080+0.0097*Tau-0.0052*Cit+0.0213*Ile-0.0136*Leu-0.0073*Tyr+0.0023*Phe |
| 0.9077 | 0.5373+0.0096*Tau-0.0071*Met+0.0221*Ile-0.0141*Leu-0.0067*Tyr+0.0011*Lys |
| 0.9077 | 0.6246+0.0096*Tau-0.0014*Val+0.0220*Ile-0.0118*Leu-0.0062*Tyr-0.0007*Orn |
| 0.9076 | 0.6885+0.0097*Tau-0.0011*Ser+0.0216*Ile-0.0136*Leu-0.0068*Tyr-0.0004*Orn |
| 0.9076 | 0.6946+0.0097*Tau-0.0011*Ser-0.0000*Gln+0.0215*Ile-0.0136*Leu-0.0069*Tyr |
| 0.9076 | 0.6960+0.0097*Tau-0.0049*Asn+0.0216*Ile-0.0133*Leu-0.0062*Tyr |
| 0.9076 | 0.6965+0.0097*Tau-0.0050*Asn-0.0001*Glu+0.0216*Ile-0.0133*Leu-0.0062*Tyr |
| 0.9076 | 0.6134+0.0095*Tau+0.0007*Glu-0.0016*Val+0.0217*Ile-0.0116*Leu-0.0065*Tyr |
| 0.9076 | 0.6550+0.0096*Tau-0.0017*Val-0.0053*Met+0.0222*Ile-0.0113*Leu-0.0054*Tyr |
| 0.9076 | 0.6346+0.0097*Tau-0.0050*Cit+0.0019*ABA+0.0214*Ile-0.0134*Leu-0.0067*Tyr |

# FIG.140

| ROC AUC | FORMULA |
|---|---|
| 0.9075 | 0.7505+0.0098*Tau-0.0009*Ser-0.0046*Cit+0.0211*Ile-0.0132*Leu-0.0066*Tyr |
| 0.9075 | 0.7386+0.0097*Tau-0.0006*Ser-0.0042*Asn+0.0215*Ile-0.0134*Leu-0.0063*Tyr |
| 0.9075 | 0.7003+0.0097*Tau-0.0048*Asn+0.0216*Ile-0.0134*Leu-0.0061*Tyr-0.0003*Orn |
| 0.9075 | 0.6714+0.0097*Tau-0.0049*Asn+0.0016*ABA+0.0218*Ile-0.0135*Leu-0.0062*Tyr |
| 0.9075 | 0.6765+0.0097*Tau-0.0002*Gln-0.0015*Val+0.0219*Ile-0.0116*Leu-0.0062*Tyr |
| 0.9074 | 0.6851+0.0097*Tau-0.0011*Ser+0.0215*Ile-0.0136*Leu-0.0069*Tyr |
| 0.9074 | 0.6942+0.0096*Tau-0.0050*Asn+0.0216*Ile-0.0134*Leu-0.0062*Tyr+0.0002*Trp |
| 0.9074 | 0.6880+0.0097*Tau-0.0011*Ser+0.0215*Ile-0.0136*Leu-0.0068*Tyr-0.0002*Trp |
| 0.9073 | 0.6955+0.0096*Tau-0.0057*Asn+0.0029*Met+0.0214*Ile-0.0134*Leu-0.0065*Tyr |
| 0.9073 | 0.6840+0.0097*Tau-0.0011*Ser+0.0002*Glu+0.0215*Ile-0.0136*Leu-0.0070*Tyr |
| 0.7970 | 1.2517+0.0036*Glu-0.0021*Val+0.0225*Ile-0.0133*Leu-0.0089*Tyr+0.0014*Lys |
| 0.7961 | 1.3163+0.0032*Glu+0.0199*Ile-0.0140*Leu-0.0094*Tyr-0.0029*His+0.0051*Trp |
| 0.7955 | 1.1316+0.0032*Glu+0.0221*Ile-0.0161*Leu-0.0105*Tyr+0.0039*Trp+0.0010*Lys |
| 0.7953 | 1.2584+0.0036*Glu-0.0021*Val+0.0222*Ile-0.0129*Leu-0.0091*Tyr+0.0046*Trp |
| 0.7950 | 1.3061+0.0032*Glu+0.0204*Ile-0.0146*Leu-0.0091*Tyr-0.0026*His+0.0014*Lys |
| 0.7950 | 1.1007+0.0033*Glu+0.0002*Gln+0.0221*Ile-0.0158*Leu-0.0098*Tyr+0.0010*Lys |
| 0.7948 | 1.0718+0.0033*Glu+0.0003*Gln+0.0218*Ile-0.0155*Leu-0.0102*Tyr+0.0041*Trp |
| 0.7944 | 1.2232+0.0034*Glu+0.0004*Gln+0.0201*Ile-0.0138*Leu-0.0086*Tyr-0.0026*His |
| 0.7941 | 1.2544+0.0032*Glu-0.0029*Cit+0.0217*Ile-0.0152*Leu-0.0098*Tyr+0.0047*Trp |
| 0.7939 | 1.2818-0.0032*Asn+0.0029*Glu+0.0220*Ile-0.0153*Leu-0.0094*Tyr+0.0048*Trp |
| 0.7937 | 1.2217+0.0033*Glu+0.0221*Ile-0.0155*Leu-0.0096*Tyr+0.0045*Trp-0.0011*Orn |
| 0.7936 | 1.1681+0.0032*Glu+0.0020*ABA+0.0222*Ile-0.0157*Leu-0.0099*Tyr+0.0044*Trp |
| 0.7935 | 1.1892+0.0038*Glu+0.0004*Gln-0.0019*Val+0.0222*Ile-0.0128*Leu-0.0084*Tyr |
| 0.7935 | 1.2475+0.0032*Glu-0.0028*Cit+0.0220*Ile-0.0157*Leu-0.0096*Tyr+0.0014*Lys |
| 0.7935 | 1.2342-0.0039*Asn+0.0029*Glu+0.0224*Ile-0.0159*Leu-0.0081*Tyr+0.0015*Lys |
| 0.7933 | 1.1936+0.0032*Glu+0.0219*Ile-0.0155*Leu-0.0099*Tyr+0.0002*Phe+0.0043*Trp |
| 0.7930 | 1.2261+0.0034*Glu-0.0004*Ala+0.0228*Ile-0.0161*Leu-0.0088*Tyr+0.0014*Lys |
| 0.7930 | 1.1885+0.0032*Glu+0.0006*ABA+0.0223*Ile-0.0159*Leu-0.0097*Tyr+0.0012*Lys |
| 0.7930 | 1.1984+0.0032*Glu+0.0219*Ile-0.0154*Leu-0.0099*Tyr+0.0043*Trp |
| 0.7929 | 1.1685+0.0003*Ser+0.0033*Glu+0.0219*Ile-0.0154*Leu-0.0099*Tyr+0.0043*Trp |
| 0.7917 | 1.1582-0.0076*Asn+0.0006*Gln+0.0228*Ile-0.0154*Leu-0.0084*Tyr+0.0049*Trp |
| 0.7917 | 1.2666-0.0061*Asn+0.0231*Ile-0.0162*Leu-0.0091*Tyr+0.0045*Trp+0.0015*Lys |
| 0.7912 | 1.1875-0.0077*Asn+0.0005*Gln+0.0232*Ile-0.0158*Leu-0.0081*Tyr+0.0015*Lys |
| 0.7908 | 1.2556+0.0210*Ile-0.0147*Leu-0.0092*Tyr-0.0030*His+0.0047*Trp+0.0012*Lys |
| 0.7906 | 1.2963-0.0058*Asn+0.0006*Gln+0.0214*Ile-0.0138*Leu-0.0070*Tyr-0.0023*His |
| 0.7905 | 1.4002-0.0062*Asn-0.0020*Val+0.0238*Ile-0.0134*Leu-0.0073*Tyr+0.0019*Lys |
| 0.7900 | 1.4102-0.0035*Asn+0.0210*Ile-0.0140*Leu-0.0080*Tyr-0.0027*His+0.0056*Trp |
| 0.7900 | 1.2040-0.0039*Cit+0.0228*Ile-0.0159*Leu-0.0096*Tyr+0.0044*Trp+0.0012*Lys |
| 0.7899 | 1.2061+0.0003*Gln+0.0208*Ile-0.0140*Leu-0.0087*Tyr-0.0031*His+0.0050*Trp |
| 0.7898 | 1.4002-0.0052*Asn-0.0019*Val+0.0233*Ile-0.0129*Leu-0.0075*Tyr+0.0054*Trp |
| 0.7897 | 1.1830-0.0018*Val+0.0235*Ile-0.0140*Leu-0.0090*Tyr+0.0042*Trp+0.0012*Lys |
| 0.7894 | 1.2860-0.0075*Asn+0.0006*Gln-0.0018*Val+0.0233*Ile-0.0127*Leu-0.0065*Tyr |
| 0.7893 | 1.2427-0.0063*Asn+0.0007*Gln-0.0031*Cit+0.0227*Ile-0.0147*Leu-0.0073*Tyr |
| 0.7893 | 1.2996-0.0029*Cit-0.0016*Val+0.0233*Ile-0.0138*Leu-0.0081*Tyr+0.0015*Lys |
| 0.7891 | 1.2817+0.0005*Gln-0.0030*Cit+0.0210*Ile-0.0136*Leu-0.0075*Tyr-0.0025*His |
| 0.7890 | 1.2090-0.0111*Asn+0.0006*Gln-0.0031*Val+0.0248*Ile-0.0139*Leu+0.0012*Lys |
| 0.7890 | 1.3519-0.0015*Val+0.0218*Ile-0.0128*Leu-0.0077*Tyr-0.0025*His+0.0015*Lys |
| 0.7888 | 1.2892+0.0029*ABA+0.0212*Ile-0.0143*Leu-0.0086*Tyr-0.0030*His+0.0054*Trp |
| 0.7887 | 1.3669-0.0014*Val+0.0213*Ile-0.0122*Leu-0.0079*Tyr-0.0028*His+0.0054*Trp |
| 0.7886 | 1.2305+0.0002*Gln+0.0213*Ile-0.0145*Leu-0.0083*Tyr-0.0027*His+0.0012*Lys |

# FIG.141

| ROC AUC | FORMULA |
|---|---|
| 0.9079 | 0.17962+0.06066*Tau+0.00612*Pro+0.11119*Ile-0.08474*Leu-0.07494*Tyr+0.01426*Lys |
| 0.9039 | 3.55187+0.05922*Tau-0.08316*Asn+0.00821*Pro+0.10185*Ile-0.06884*Leu-0.04970*Tyr |
| 0.9025 | 2.31440+0.06517*Tau-0.08183*Cit+0.11075*Ile-0.07813*Leu-0.07402*Tyr+0.01451*Lys |
| 0.9024 | 3.22624+0.06214*Tau-0.09565*Cit+0.09977*Ile-0.06604*Leu-0.06991*Tyr+0.01599*Arg |
| 0.9010 | 2.65268+0.06329*Tau-0.08994*Asn+0.11046*Ile-0.08296*Leu-0.05796*Tyr+0.01938*Lys |
| 0.9006 | 3.86915+0.06212*Tau-0.01294*Gly+0.13491*Ile-0.09882*Leu-0.07679*Tyr+0.01603*Lys |
| 0.9004 | 3.73914+0.06401*Tau+0.06257*Ile-0.05035*Leu-0.05223*Tyr-0.07508*His+0.01732*Lys |
| 0.9003 | -0.04669+0.06101*Tau+0.11606*Ile-0.08899*Leu-0.07971*Tyr+0.03495*Trp+0.01319*Lys |
| 0.9000 | 3.39218+0.06324*Tau-0.07924*Cit-0.00288*Val+0.10998*Ile-0.06425*Leu-0.06107*Tyr |
| 0.8998 | 1.03238+0.06063*Tau-0.13352*Met+0.12370*Ile-0.08608*Leu-0.04654*Tyr+0.02020*Lys |
| 0.8984 | 5.54863+0.06386*Tau-0.06475*Cit+0.07032*Ile-0.04188*Leu-0.04653*Tyr-0.05465*His |
| 0.8982 | 4.24529+0.06094*Tau+0.06149*Ile-0.04452*Leu-0.05186*Tyr-0.07390*His+0.04542*Trp |
| 0.8979 | 4.96534+0.05839*Tau+0.00951*Pro-0.01400*Gly+0.12715*Ile-0.08790*Leu-0.07143*Tyr |
| 0.8976 | 3.65939+0.06215*Tau-0.07562*Cit-0.05586*Met+0.11157*Ile-0.06635*Leu-0.04999*Tyr |
| 0.8974 | 4.49694+0.06285*Tau-0.05626*Asn-0.06041*Cit+0.10575*Ile-0.06540*Leu-0.05097*Tyr |
| 0.8968 | 5.39971+0.06197*Tau-0.01010*Gly-0.04928*Cit+0.12503*Ile-0.07982*Leu-0.06476*Tyr |
| 0.8959 | 7.34840+0.06024*Tau+0.02041*Thr-0.11696*Asn-0.01737*Val+0.03324*Ile-0.08548*His |
| 0.8958 | 3.97664+0.06606*Tau-0.03126*Ser+0.12363*Ile-0.09379*Leu-0.07383*Tyr+0.01654*Lys |
| 0.8957 | 7.36380+0.05953*Tau+0.01762*Thr-0.10176*Asn+0.00898*Pro-0.04273*Tyr-0.09467*His |
| 0.8955 | 5.34079+0.06205*Tau+0.00964*Pro-0.00928*Val-0.05562*Tyr-0.10729*His+0.01658*Lys |
| 0.8953 | 8.37626+0.06281*Tau+0.02777*Thr-0.09262*Asn-0.07546*Cit-0.04525*Tyr-0.08491*His |
| 0.8946 | 6.94400+0.06219*Tau+0.00926*Thr+0.00879*Pro-0.08442*Cit-0.05809*Tyr-0.09200*His |
| 0.8944 | 2.81964+0.06276*Tau-0.08673*Cit+0.10900*Ile-0.07315*Leu-0.07522*Tyr+0.04340*Trp |
| 0.8942 | 6.88603+0.06145*Tau+0.00829*Pro-0.09359*Cit-0.06560*Tyr-0.09254*His+0.02259*Arg |
| 0.8942 | 9.37114+0.06302*Tau+0.02665*Thr-0.02391*Ser-0.07146*Asn-0.04875*Tyr-0.09937*His |
| 0.8940 | 4.28793+0.05938*Tau-0.01226*Gly+0.13197*Ile-0.09136*Leu-0.07416*Tyr+0.03622*Trp |
| 0.8939 | 6.94109+0.06218*Tau+0.00607*Thr-0.09500*Cit-0.06142*Tyr-0.09199*His+0.02314*Arg |
| 0.8939 | 3.18286+0.05989*Tau-0.08510*Asn+0.10855*Ile-0.07568*Leu-0.05725*Tyr+0.04811*Trp |
| 0.8937 | 4.90484+0.06167*Tau+0.00968*Pro-0.02134*Leu-0.05510*Tyr-0.10273*His+0.01937*Lys |
| 0.8936 | 10.17669+0.06663*Tau+0.02706*Thr-0.03204*Ser-0.08418*Cit-0.06590*Tyr-0.09679*His |
| 0.8934 | 7.75426+0.06006*Tau+0.02109*Thr-0.10098*Asn-0.00445*Val-0.03008*Tyr-0.08806*His |
| 0.8932 | 7.16380+0.06037*Tau+0.02238*Thr-0.11282*Asn-0.05378*Tyr-0.10277*His+0.05271*Trp |
| 0.8930 | 8.82102+0.06301*Tau+0.01427*Thr-0.03087*Ser+0.00796*Pro-0.06176*Tyr-0.10745*His |
| 0.8926 | 7.27591+0.05962*Tau+0.01560*Thr-0.09708*Asn-0.04180*Tyr-0.09461*His+0.01411*Arg |
| 0.8923 | 7.74526+0.05770*Tau+0.01785*Thr-0.11653*Asn+0.00970*Pro-0.01041*Val-0.09552*His |
| 0.8922 | 7.44634+0.06155*Tau+0.01879*Thr-0.10817*Asn+0.05168*Met-0.04598*Tyr-0.09441*His |
| 0.8917 | 7.50891+0.05995*Tau+0.02001*Thr-0.09604*Asn-0.00531*Leu-0.03240*Tyr-0.08845*His |
| 0.8915 | 6.32041+0.06456*Tau+0.00981*Pro-0.07539*Cit-0.07020*Tyr-0.10034*His+0.01359*Lys |
| 0.8914 | 9.35587+0.05969*Tau+0.02841*Thr-0.08582*Asn-0.00913*Gly-0.05007*Tyr-0.09767*His |
| 0.8914 | 5.20476+0.05905*Tau+0.00739*Pro-0.06689*Tyr-0.11266*His+0.04136*Trp+0.00960*Arg |
| 0.8914 | 7.02217+0.06107*Tau-0.08880*Cit-0.00946*Leu-0.05353*Tyr-0.08422*His+0.02877*Arg |
| 0.8913 | 7.15014+0.06160*Tau-0.08936*Cit-0.00351*Val-0.05555*Tyr-0.08740*His+0.02762*Arg |
| 0.8913 | 6.86210+0.06318*Tau+0.01811*Thr-0.10402*Asn-0.05154*Tyr-0.10267*His+0.01430*Lys |
| 0.8912 | 6.45663+0.06515*Tau+0.00930*Thr-0.07903*Cit-0.06394*Tyr-0.09890*His+0.01183*Lys |
| 0.8909 | 7.08901+0.06115*Tau+0.01002*Pro-0.06960*Cit-0.00747*Leu-0.04803*Tyr-0.08368*His |
| 0.8908 | 5.47199+0.05835*Tau+0.00910*Pro-0.01513*Leu-0.05404*Tyr-0.10058*His+0.04943*Trp |
| 0.8907 | 8.98658+0.06182*Tau+0.02111*Thr-0.00924*Gly-0.06842*Cit-0.06298*Tyr-0.09580*His |
| 0.8903 | 7.06152+0.06169*Tau-0.08937*Cit-0.00250*Ile-0.05897*Tyr-0.09038*His+0.02684*Arg |
| 0.8902 | 6.66945+0.06143*Tau+0.02317*Thr-0.10604*Asn-0.05695*Tyr+0.05049*Phe-0.10341*His |
| 0.8901 | 4.52199+0.06317*Tau-0.03162*Ser+0.12195*Ile-0.08810*Leu-0.07385*Tyr+0.04566*Trp |

# FIG.142

| ROC AUC | FORMULA |
|---|---|
| 0.8901 | 8.35788+0.06055*Tau+0.02618*Thr-0.10614*Asn-0.06167*Cit-0.00755*Val-0.09168*His |
| 0.8900 | 6.61913+0.06368*Tau-0.08434*Cit-0.06563*Tyr-0.09582*His+0.00814*Lys+0.01933*Arg |
| 0.8898 | 7.08854+0.05871*Tau-0.07844*Asn+0.00898*Pro-0.04695*Tyr-0.09376*His+0.01746*Arg |
| 0.8893 | 5.42761+0.06142*Tau+0.00917*Pro-0.01611*Ile-0.05913*Tyr-0.11449*His+0.01542*Lys |
| 0.8890 | 4.81874+0.06166*Tau+0.00825*Pro-0.07443*Tyr-0.11869*His+0.03751*Trp+0.01110*Lys |
| 0.8888 | 7.69045+0.05903*Tau-0.07261*Asn+0.01109*Pro-0.00638*Val-0.03144*Tyr-0.08340*His |
| 0.8887 | 8.69806+0.06396*Tau+0.01771*Thr-0.03289*Ser-0.07279*Tyr-0.11551*His+0.04597*Trp |
| 0.8885 | 6.61152+0.06217*Tau-0.09966*Cit-0.07853*Tyr-0.09997*His+0.04937*Trp+0.02780*Arg |
| 0.8878 | 8.43035+0.06088*Tau-0.00649*Gly-0.07367*Cit-0.06564*Tyr-0.09220*His+0.02797*Arg |
| 0.8877 | 6.40866+0.06269*Tau-0.08984*Cit-0.07382*Tyr+0.03524*Phe-0.09741*His+0.02579*Arg |
| 0.8875 | 7.92019+0.06139*Tau-0.05778*Asn-0.07692*Cit-0.05346*Tyr-0.08415*His+0.03087*Arg |
| 0.8874 | 6.31816+0.06482*Tau-0.06187*Cit-0.01581*Leu-0.05337*Tyr-0.08985*His+0.01818*Lys |
| 0.8872 | 6.69558+0.06207*Tau+0.00979*Pro-0.08028*Cit-0.07113*Tyr-0.09787*His+0.04775*Trp |
| 0.8870 | 7.60078+0.05819*Tau+0.02272*Thr-0.12962*Asn-0.01218*Val-0.10426*His+0.05095*Trp |
| 0.8869 | 4.75571+0.05783*Tau-0.01339*Gly+0.00528*Ala+0.12177*Ile-0.08479*Leu-0.07772*Tyr |
| 0.8866 | 7.94242+0.06132*Tau-0.05427*Asn+0.01081*Pro-0.05672*Cit-0.04639*Tyr-0.08256*His |
| 0.8865 | 6.59669+0.06275*Tau-0.08174*Asn+0.01060*Pro-0.05838*Tyr-0.10245*His+0.01653*Lys |
| 0.8861 | 7.32313+0.06144*Tau+0.01851*Thr-0.12359*Asn-0.01383*Val-0.10376*His+0.01655*Lys |
| 0.8860 | 5.39438+0.06050*Tau+0.00990*Pro-0.09810*Met-0.04841*Tyr-0.10767*His+0.01746*Lys |
| 0.8856 | 7.56177+0.06118*Tau+0.01078*Pro-0.00937*Gly-0.07375*Tyr-0.11113*His+0.01264*Lys |
| 0.8856 | 9.16744+0.06376*Tau-0.02039*Ser+0.01034*Pro-0.06110*Cit-0.05860*Tyr-0.08956*His |
| 0.8856 | 7.76217+0.06476*Tau-0.02379*Ser+0.00939*Pro-0.07027*Tyr-0.11122*His+0.01310*Lys |
| 0.8851 | 6.58694+0.06492*Tau-0.06501*Cit-0.00422*Ile-0.05923*Tyr-0.09715*His+0.01394*Lys |
| 0.8847 | 7.10751+0.05881*Tau+0.01771*Thr-0.10424*Asn+0.00577*Ala-0.05198*Tyr-0.09113*His |
| 0.8847 | 6.77067+0.06106*Tau+0.00775*Pro+0.00315*Ala-0.07059*Cit-0.06166*Tyr-0.08874*His |
| 0.8845 | 6.98697+0.05952*Tau-0.07952*Asn+0.01038*Pro-0.05673*Tyr-0.09912*His+0.05072*Trp |
| 0.8845 | 4.83982+0.06216*Tau-0.02305*Leu-0.05991*Tyr-0.10546*His+0.04749*Trp+0.01784*Lys |
| 0.8842 | 7.61904+0.05968*Tau+0.02200*Thr-0.10650*Asn+0.00781*Pro-0.06366*Cit-0.10752*His |
| 0.8841 | 7.20180+0.06079*Tau-0.10723*Asn+0.01239*Pro-0.01796*Val-0.10249*His+0.02046*Lys |
| 0.8836 | 7.56851+0.06202*Tau-0.00863*Gly-0.02160*Leu-0.05386*Tyr-0.09542*His+0.01923*Lys |
| 0.8834 | 8.18308+0.06234*Tau-0.02502*Ser+0.00933*Pro-0.07114*Tyr-0.10956*His+0.04603*Trp |
| 0.8833 | 7.80244+0.05874*Tau+0.01057*Pro-0.00902*Gly-0.07196*Tyr-0.10838*His+0.03897*Trp |
| 0.8828 | 6.81698+0.05930*Tau-0.08321*Asn-0.05888*Tyr-0.10146*His+0.05119*Trp+0.02268*Arg |
| 0.8827 | 6.71836+0.06229*Tau-0.07473*Asn-0.05162*Tyr-0.09894*His+0.01260*Lys+0.01210*Arg |
| 0.8826 | 7.18828+0.06388*Tau-0.07838*Asn-0.00991*Val-0.03969*Tyr-0.09147*His+0.02036*Lys |
| 0.8817 | 7.31638+0.06518*Tau-0.05639*Asn-0.04970*Cit-0.05461*Tyr-0.09176*His+0.01570*Lys |
| 0.8815 | 7.48410+0.05707*Tau+0.01833*Thr-0.11836*Asn+0.00474*Ala-0.01027*Val-0.09465*His |
| 0.8814 | 6.65142+0.06344*Tau-0.07321*Asn-0.01977*Leu-0.04084*Tyr-0.08862*His+0.02214*Lys |
| 0.8810 | 8.10937+0.06230*Tau-0.02616*Ser-0.07337*Tyr-0.11185*His+0.04627*Trp+0.01964*Arg |
| 0.8799 | 6.25258+0.05987*Tau-0.09567*Asn+0.01119*Pro-0.02953*Leu-0.10099*His+0.02106*Lys |
| 0.8798 | 4.81644+0.06089*Tau+0.00500*Ala-0.01999*Leu-0.06026*Tyr-0.09803*His+0.01723*Lys |
| 0.8788 | 8.21194+0.06137*Tau-0.02385*Ser+0.00706*Pro+0.00380*Ala-0.06435*Tyr-0.09926*His |
| 0.8786 | 6.52582+0.06330*Tau-0.07808*Asn-0.06282*Tyr-0.10555*His+0.04555*Trp+0.01433*Lys |
| 0.8777 | 7.70276+0.06522*Tau-0.02338*Ser-0.07427*Tyr-0.11384*His+0.04179*Trp+0.01123*Lys |
| 0.8773 | 6.84587+0.05805*Tau-0.08010*Asn+0.00566*Ala-0.05562*Tyr-0.09006*His+0.01687*Arg |
| 0.8731 | 7.97652+0.06186*Tau-0.02414*Ser+0.00457*Ala-0.07458*Tyr-0.10468*His+0.04038*Trp |
| 0.8726 | 7.58800+0.06389*Tau-0.02314*Ser+0.00490*Ala-0.07468*Tyr-0.10610*His+0.01140*Lys |
| 0.8712 | 6.43031+0.06172*Tau-0.08093*Asn+0.00595*Ala-0.06473*Tyr-0.09675*His+0.01447*Lys |
| 0.8712 | 5.25384+0.05955*Tau+0.00554*Ala-0.09799*Met-0.05426*Tyr-0.10226*His+0.01557*Lys |
| 0.8658 | 6.23476+0.05961*Tau-0.07447*Asn+0.00689*Ala-0.07001*Tyr+0.04634*Phe-0.09492*His |

# FIG.143

# FIG.144

| ROC AUC | FORMULA |
|---|---|
| 0.9109 | 1.1801+0.0964*Tau-0.0012*ABA+0.1498*Ile-0.0898*Leu-0.0541*Tyr-0.0309*His |
| 0.9107 | 1.4269+0.0991*Tau-0.0169*Val+0.1624*Ile-0.0733*Leu-0.0485*Tyr-0.0278*His |
| 0.9107 | 1.1214+0.0967*Tau+0.0365*Met+0.1453*Ile-0.0889*Leu-0.0598*Tyr-0.0358*His |
| 0.9106 | 1.1909+0.0968*Tau-0.0020*Ala+0.1554*Ile-0.0920*Leu-0.0491*Tyr-0.0277*His |
| 0.9106 | 1.1667+0.0963*Tau+0.1501*Ile-0.0900*Leu-0.0541*Tyr-0.0308*His-0.0002*Orn |
| 0.9105 | -0.2460+0.0968*Tau-0.0028*Ala+0.1771*Ile-0.1086*Leu-0.0580*Tyr+0.0122*Phe |
| 0.9103 | 1.1921+0.0959*Tau-0.0029*Glu+0.1492*Ile-0.0902*Leu-0.0549*Tyr-0.0309*His |
| 0.9102 | 1.2511+0.0966*Tau-0.0003*Gln+0.1505*Ile-0.0901*Leu-0.0538*Tyr-0.0302*His |
| 0.9102 | 0.6075+0.0965*Tau-0.0228*Asn-0.0023*Ala+0.1747*Ile-0.1055*Leu-0.0506*Tyr |
| 0.9100 | 1.2239+0.0996*Tau-0.0356*Asn-0.0201*Val+0.1812*Ile-0.0822*Leu-0.0468*Tyr |
| 0.9100 | -0.3783+0.0967*Tau-0.0035*Ala+0.1792*Ile-0.1126*Leu-0.0620*Tyr+0.0085*Lys |
| 0.9099 | 1.4424+0.0965*Tau-0.0174*Asn+0.1521*Ile-0.0910*Leu-0.0515*Tyr-0.0271*His |
| 0.9099 | 0.7459+0.0997*Tau-0.0273*Cit-0.0181*Val+0.1809*Ile-0.0840*Leu-0.0536*Tyr |
| 0.9099 | 0.8795+0.0966*Tau+0.1485*Ile-0.0939*Leu-0.0632*Tyr-0.0377*His+0.0100*Lys |
| 0.9099 | 0.7941+0.0969*Tau+0.1510*Ile-0.0923*Leu-0.0589*Tyr+0.0165*Phe-0.0322*His |
| 0.9098 | 0.7692+0.0970*Tau-0.0068*Ser-0.0028*Ala+0.1763*Ile-0.1073*Leu-0.0533*Tyr |
| 0.9098 | 1.1650+0.0963*Tau+0.1500*Ile-0.0900*Leu-0.0541*Tyr-0.0309*His |
| 0.9097 | -0.1732+0.0989*Tau-0.0202*Val+0.1869*Ile-0.0908*Leu-0.0646*Tyr+0.0089*Lys |
| 0.9097 | 1.7003+0.0969*Tau-0.0057*Ser+0.1513*Ile-0.0913*Leu-0.0536*Tyr-0.0295*His |
| 0.9097 | 1.1206+0.0962*Tau+0.1486*Ile-0.0898*Leu-0.0556*Tyr-0.0330*His+0.0076*Trp |
| 0.9097 | 0.4529+0.0990*Tau-0.0023*Ala-0.0172*Val+0.1846*Ile-0.0875*Leu-0.0489*Tyr |
| 0.9094 | 1.3670+0.0971*Tau-0.0211*Cit+0.1514*Ile-0.0899*Leu-0.0538*Tyr-0.0271*His |
| 0.9094 | 0.1278+0.0965*Tau-0.0029*Ala-0.0040*ABA+0.1755*Ile-0.1061*Leu-0.0538*Tyr |
| 0.9094 | 0.0559+0.0963*Tau-0.0029*Ala+0.1760*Ile-0.1066*Leu-0.0542*Tyr+0.0011*Trp |
| 0.9093 | 0.1255+0.0964*Tau-0.0029*Ala+0.1766*Ile-0.1065*Leu-0.0530*Tyr-0.0029*Orn |
| 0.9093 | 0.2169+0.0965*Tau-0.0310*Asn+0.1723*Ile-0.1073*Leu-0.0608*Tyr+0.0158*Phe |
| 0.9093 | 0.6064+0.0963*Tau-0.0071*Ser+0.1720*Ile-0.1065*Leu-0.0609*Tyr-0.0016*Orn |
| 0.9092 | 0.7240+0.0967*Tau-0.0065*Ser-0.0006*Gln+0.1717*Ile-0.1062*Leu-0.0606*Tyr |
| 0.9092 | 0.3455+0.0969*Tau-0.0008*Gln-0.0027*Ala+0.1759*Ile-0.1061*Leu-0.0531*Tyr |
| 0.9091 | 0.2841+0.0987*Tau-0.0184*Val+0.1815*Ile-0.0858*Leu-0.0552*Tyr |
| 0.9091 | 0.2304+0.0985*Tau+0.0046*ABA-0.0186*Val+0.1824*Ile-0.0861*Leu-0.0553*Tyr |
| 0.9091 | 0.7744+0.0998*Tau-0.0014*Gln-0.0185*Val+0.1820*Ile-0.0850*Leu-0.0530*Tyr |
| 0.9091 | 0.1085+0.0958*Tau-0.0036*Glu-0.0030*Ala+0.1751*Ile-0.1067*Leu-0.0548*Tyr |
| 0.9091 | 0.5482+0.0974*Tau-0.0028*Ala-0.0281*Cit+0.1743*Ile-0.1040*Leu-0.0524*Tyr |
| 0.9091 | 0.2683+0.0966*Tau-0.0084*Ser+0.1738*Ile-0.1119*Leu-0.0701*Tyr+0.0077*Lys |
| 0.9090 | 1.0586+0.0994*Tau-0.0074*Ser-0.0185*Val+0.1820*Ile-0.0866*Leu-0.0540*Tyr |
| 0.9090 | 0.6124+0.0968*Tau-0.0461*Asn+0.0564*Met+0.1678*Ile-0.1055*Leu-0.0639*Tyr |
| 0.9090 | -0.0647+0.0971*Tau-0.0353*Cit+0.1724*Ile-0.1092*Leu-0.0702*Tyr+0.0090*Lys |
| 0.9090 | 0.0750+0.0964*Tau-0.0029*Ala+0.1760*Ile-0.1065*Leu-0.0539*Tyr |
| 0.9089 | 0.0450+0.0989*Tau-0.0179*Val+0.1823*Ile-0.0879*Leu-0.0583*Tyr+0.0087*Phe |
| 0.9089 | 0.8092+0.0968*Tau-0.0220*Asn-0.0240*Cit+0.1693*Ile-0.1026*Leu-0.0559*Tyr |
| 0.9088 | 0.2809+0.0962*Tau-0.0405*Asn+0.1733*Ile-0.1110*Leu-0.0649*Tyr+0.0100*Lys |
| 0.9088 | 0.3286+0.0987*Tau-0.0183*Val+0.1822*Ile-0.0859*Leu-0.0543*Tyr-0.0026*Orn |
| 0.9088 | 0.5775+0.0961*Tau-0.0293*Asn-0.0009*Glu+0.1711*Ile-0.1046*Leu-0.0558*Tyr |
| 0.9088 | 0.8496+0.0971*Tau-0.0054*Ser-0.0262*Cit+0.1698*Ile-0.1039*Leu-0.0597*Tyr |
| 0.9088 | 0.4159+0.0987*Tau-0.0190*Val-0.0178*Met+0.1825*Ile-0.0844*Leu-0.0514*Tyr |
| 0.9088 | 0.5833+0.0959*Tau-0.0283*Asn+0.1712*Ile-0.1046*Leu-0.0558*Tyr-0.0012*Orn |
| 0.9087 | 0.3796+0.0979*Tau+0.0075*Glu-0.0197*Val+0.1802*Ile-0.0849*Leu-0.0569*Tyr |
| 0.9087 | 0.8696+0.0963*Tau-0.0041*Ser-0.0240*Asn+0.1711*Ile-0.1052*Leu-0.0567*Tyr |
| 0.9087 | 0.5616+0.0959*Tau-0.0290*Asn+0.1709*Ile-0.1047*Leu-0.0564*Tyr+0.0009*Trp |

# FIG.145

| ROC AUC | FORMULA |
|---|---|
| 0.9086 | 0.4071+0.0970*Tau-0.0293*Cit-0.0049*ABA+0.1689*Ile-0.1026*Leu-0.0602*Tyr |
| 0.9086 | 0.6476+0.0962*Tau-0.0291*Asn-0.0051*ABA+0.1703*Ile-0.1040*Leu-0.0560*Tyr |
| 0.9086 | -0.4862+0.0957*Tau-0.0234*Met+0.1747*Ile-0.1107*Leu-0.0670*Tyr+0.0082*Lys |
| 0.9085 | 0.6025+0.0963*Tau-0.0074*Ser+0.1717*Ile-0.1065*Leu-0.0614*Tyr |
| 0.9084 | 0.6233+0.0964*Tau-0.0073*Ser+0.1716*Ile-0.1064*Leu-0.0610*Tyr-0.0014*Trp |
| 0.9083 | 0.2616+0.0987*Tau-0.0184*Val+0.1816*Ile-0.0859*Leu-0.0556*Tyr+0.0013*Trp |
| 0.9082 | 0.6011+0.0961*Tau-0.0073*Ser+0.0011*Glu+0.1714*Ile-0.1066*Leu-0.0618*Tyr |
| 0.9082 | 0.5729+0.0959*Tau-0.0288*Asn+0.1709*Ile-0.1046*Leu-0.0562*Tyr |
| 0.9082 | -0.0131+0.0973*Tau-0.0295*Cit+0.1710*Ile-0.1056*Leu-0.0646*Tyr+0.0137*Phe |
| 0.9081 | -0.0358+0.0966*Tau-0.0031*Ala+0.0187*Met+0.1754*Ile-0.1071*Leu-0.0570*Tyr |
| 0.7983 | 4.3012+0.0235*Glu-0.0161*Val+0.1437*Ile-0.0801*Leu-0.0497*Tyr+0.0093*Lys |
| 0.7965 | 3.4987+0.0204*Glu+0.1317*Ile-0.0959*Leu-0.0578*Tyr+0.0182*Trp+0.0071*Lys |
| 0.7960 | 3.1092+0.0217*Glu+0.0021*Gln+0.1289*Ile-0.0913*Leu-0.0553*Tyr+0.0187*Trp |
| 0.7959 | 3.2443+0.0214*Glu+0.0016*Gln+0.1335*Ile-0.0953*Leu-0.0548*Tyr+0.0065*Lys |
| 0.7959 | 4.4337+0.0234*Glu-0.0138*Val+0.1353*Ile-0.0752*Leu-0.0484*Tyr+0.0204*Trp |
| 0.7948 | 5.0153+0.0201*Glu+0.1134*Ile-0.0786*Leu-0.0490*Tyr-0.0252*His+0.0264*Trp |
| 0.7945 | 4.1551+0.0199*Glu-0.0178*Cit+0.1344*Ile-0.0950*Leu-0.0534*Tyr+0.0087*Lys |
| 0.7944 | 4.3076+0.0218*Glu+0.0030*Gln+0.1167*Ile-0.0793*Leu-0.0465*Tyr-0.0247*His |
| 0.7942 | 4.8847+0.0198*Glu+0.1210*Ile-0.0854*Leu-0.0491*Tyr-0.0243*His+0.0091*Lys |
| 0.7942 | 4.2433+0.0202*Glu-0.0151*Cit+0.1282*Ile-0.0890*Leu-0.0526*Tyr+0.0218*Trp |
| 0.7940 | 4.2025-0.0179*Asn+0.0185*Glu+0.1352*Ile-0.0955*Leu-0.0510*Tyr+0.0092*Lys |
| 0.7939 | 4.2397-0.0123*Asn+0.0194*Glu+0.1287*Ile-0.0892*Leu-0.0511*Tyr+0.0220*Trp |
| 0.7939 | 3.9005+0.0249*Glu+0.0023*Gln-0.0140*Val+0.1380*Ile-0.0756*Leu-0.0465*Tyr |
| 0.7938 | 3.9305+0.0212*Glu-0.0017*Ala+0.1375*Ile-0.0969*Leu-0.0492*Tyr+0.0086*Lys |
| 0.7938 | 3.7734+0.0206*Glu+0.0102*ABA+0.1300*Ile-0.0913*Leu-0.0534*Tyr+0.0211*Trp |
| 0.7938 | 4.0457+0.0209*Glu+0.1299*Ile-0.0901*Leu-0.0519*Tyr+0.0212*Trp-0.0049*Orn |
| 0.7933 | 3.7442+0.0017*Ser+0.0210*Glu+0.1286*Ile-0.0897*Leu-0.0532*Tyr+0.0201*Trp |
| 0.7932 | 3.9341+0.0207*Glu+0.1286*Ile-0.0899*Leu-0.0530*Tyr+0.0205*Trp |
| 0.7930 | 3.9561+0.0207*Glu+0.1286*Ile-0.0897*Leu-0.0528*Tyr-0.0009*Phe+0.0205*Trp |
| 0.7930 | 3.7971+0.0205*Glu+0.0013*ABA+0.1344*Ile-0.0956*Leu-0.0536*Tyr+0.0078*Lys |
| 0.7923 | 4.2264+0.0025*Gln+0.1128*Ile-0.0740*Leu-0.0440*Tyr-0.0320*His+0.0267*Trp |
| 0.7917 | 4.5203-0.0287*Asn+0.0038*Gln+0.1226*Ile-0.0772*Leu-0.0363*Tyr-0.0214*His |
| 0.7913 | 3.5022-0.0409*Asn+0.0035*Gln+0.1327*Ile-0.0878*Leu-0.0443*Tyr+0.0230*Trp |
| 0.7913 | 3.6289-0.0426*Asn+0.0029*Gln+0.1392*Ile-0.0936*Leu-0.0443*Tyr+0.0089*Lys |
| 0.7913 | 4.6654+0.1168*Ile-0.0801*Leu-0.0477*Tyr-0.0322*His+0.0252*Trp+0.0090*Lys |
| 0.7911 | 4.9470-0.0355*Asn-0.0154*Val+0.1492*Ile-0.0787*Leu-0.0388*Tyr+0.0125*Lys |
| 0.7906 | 4.0781-0.0026*Asn+0.1365*Ile-0.0941*Leu-0.0489*Tyr+0.0217*Trp+0.0098*Lys |
| 0.7900 | 4.5542+0.0032*Gln-0.0197*Cit+0.1208*Ile-0.0759*Leu-0.0387*Tyr-0.0240*His |
| 0.7900 | 3.7978-0.0225*Cit+0.1351*Ile-0.0926*Leu-0.0518*Tyr+0.0198*Trp+0.0084*Lys |
| 0.7900 | 3.9617-0.0337*Asn+0.0041*Gln-0.0202*Cit+0.1351*Ile-0.0861*Leu-0.0393*Tyr |
| 0.7899 | 5.2275-0.0135*Asn+0.1170*Ile-0.0757*Leu-0.0410*Tyr-0.0240*His+0.0283*Trp |
| 0.7899 | 3.7553-0.0125*Val+0.1430*Ile-0.0806*Leu-0.0476*Tyr+0.0178*Trp+0.0086*Lys |
| 0.7898 | 4.2923-0.0411*Asn+0.0037*Gln-0.0121*Val+0.1417*Ile-0.0734*Leu-0.0337*Tyr |
| 0.7893 | 4.9281-0.0262*Asn-0.0119*Val+0.1375*Ile-0.0730*Leu-0.0382*Tyr+0.0246*Trp |
| 0.7893 | 4.7929+0.0155*ABA+0.1172*Ile-0.0768*Leu-0.0426*Tyr-0.0277*His+0.0284*Trp |
| 0.7891 | 4.5033-0.0219*Cit-0.0133*Val+0.1470*Ile-0.0797*Leu-0.0433*Tyr+0.0106*Lys |
| 0.7889 | 5.2636-0.0095*Val+0.1217*Ile-0.0655*Leu-0.0385*Tyr-0.0254*His+0.0270*Trp |
| 0.7888 | 4.3625+0.0017*Gln+0.1214*Ile-0.0805*Leu-0.0436*Tyr-0.0293*His+0.0084*Lys |
| 0.7884 | 3.9355-0.0621*Asn+0.0030*Gln-0.0199*Val+0.1507*Ile-0.0808*Leu+0.0085*Lys |
| 0.7884 | 5.1945-0.0125*Val+0.1324*Ile-0.0703*Leu-0.0393*Tyr-0.0261*His+0.0111*Lys |

# FIG.146

| ROC AUC | FORMULA |
|---|---|
| 0.9079 | 3.26370+0.09797*Tau-0.01204*Gly+0.16013*Ile-0.11546*Leu-0.11300*Tyr+0.02146*Lys |
| 0.9068 | 2.09247+0.10214*Tau-0.07930*Cit+0.14422*Ile-0.09920*Leu-0.11433*Tyr+0.01967*Lys |
| 0.9052 | 3.05591+0.09759*Tau-0.02395*Ser+0.15075*Ile-0.11018*Leu-0.10556*Tyr+0.01967*Lys |
| 0.9052 | 2.39028+0.10109*Tau-0.09539*Asn+0.14297*Ile-0.10592*Leu-0.09163*Tyr+0.02562*Lys |
| 0.9051 | 4.25211+0.10327*Tau-0.08600*Ile-0.06518*Leu-0.09845*Tyr-0.08579*His+0.02431*Lys |
| 0.9029 | 9.52537+0.09525*Tau+0.03840*Thr-0.11144*Asn-0.08626*Cit-0.07947*Tyr-0.09843*His |
| 0.9028 | 6.92663+0.09583*Tau-0.02090*Ser+0.09427*Ile-0.05662*Leu-0.07113*Tyr-0.06584*His |
| 0.9024 | 8.52196+0.09021*Tau+0.02323*Thr-0.11192*Asn+0.00982*Pro-0.07106*Tyr-0.11345*His |
| 0.9006 | 10.79839+0.09548*Tau+0.03502*Thr-0.02592*Ser-0.08218*Asn-0.07725*Tyr-0.12206*His |
| 0.9005 | 6.67596+0.10010*Tau+0.01093*Pro-0.01157*Val-0.10108*Tyr-0.12691*His+0.02284*Lys |
| 0.9005 | 7.85293+0.09733*Tau+0.00948*Pro-0.10238*Cit-0.10751*Tyr-0.10961*His+0.03408*Arg |
| 0.9004 | 7.71585+0.09740*Tau+0.00801*Thr-0.10121*Cit-0.10210*Tyr-0.10762*His+0.03339*Arg |
| 0.8999 | 8.49449+0.09395*Tau+0.02862*Thr-0.12584*Asn-0.07863*Tyr-0.12577*His+0.05233*Trp |
| 0.8994 | 6.37915+0.09933*Tau+0.01115*Pro-0.02597*Leu-0.10450*Tyr-0.12130*His+0.02580*Lys |
| 0.8994 | 8.21626+0.09372*Tau+0.01866*Thr-0.10342*Asn-0.07285*Tyr-0.11541*His+0.02108*Arg |
| 0.8988 | 7.23276+0.09688*Tau+0.03060*Thr-0.12229*Asn-0.09101*Tyr+0.06580*Phe-0.12250*His |
| 0.8987 | 8.35184+0.08439*Tau+0.02336*Thr-0.14118*Asn+0.01102*Pro-0.01323*Val-0.11059*His |
| 0.8980 | 8.64823+0.08959*Tau+0.00923*Pro-0.00815*Gly-0.09642*Tyr-0.12453*His+0.02400*Arg |
| 0.8979 | 7.74337+0.09731*Tau-0.09295*Cit-0.00406*Val-0.09402*Tyr-0.10092*His+0.03947*Arg |
| 0.8979 | 7.12702+0.10125*Tau+0.01216*Thr-0.07601*Cit-0.10520*Tyr-0.11730*His+0.01628*Lys |
| 0.8979 | 7.63467+0.09641*Tau-0.09197*Cit-0.00796*Leu-0.09410*Tyr-0.09909*His+0.03985*Arg |
| 0.8978 | 7.72677+0.09235*Tau-0.07430*Asn+0.00935*Pro-0.07740*Tyr-0.11351*His+0.02546*Arg |
| 0.8977 | 8.00912+0.10324*Tau+0.02298*Thr-0.12078*Asn-0.09054*Tyr-0.12854*His+0.02067*Lys |
| 0.8973 | 8.80749+0.09599*Tau-0.00543*Gly-0.08320*Cit-0.10084*Tyr-0.10854*His+0.04089*Arg |
| 0.8973 | 7.18884+0.10214*Tau-0.08552*Cit-0.10982*Tyr-0.11521*His+0.01176*Lys+0.02907*Arg |
| 0.8972 | 7.59350+0.09750*Tau-0.09334*Cit-0.09854*Tyr-0.10501*His+0.03821*Arg |
| 0.8971 | 7.27824+0.10116*Tau+0.01068*Pro-0.06930*Cit-0.11356*Tyr-0.12015*His+0.01828*Lys |
| 0.8970 | 7.33490+0.09851*Tau-0.09862*Cit-0.11845*Tyr-0.11618*His+0.04819*Trp+0.03939*Arg |
| 0.8969 | 8.16859+0.09780*Tau-0.05164*Asn-0.08306*Cit-0.08943*Tyr-0.09909*His+0.04234*Arg |
| 0.8967 | 9.83905+0.09657*Tau+0.01663*Thr-0.03053*Ser-0.09279*Tyr-0.13120*His+0.01870*Arg |
| 0.8964 | 9.04375+0.09505*Tau-0.02317*Ser+0.00930*Pro-0.09388*Tyr-0.12563*His+0.02341*Arg |
| 0.8963 | 9.04573+0.09842*Tau-0.01606*Ser-0.08318*Cit-0.09848*Tyr-0.10672*His+0.03974*Arg |
| 0.8963 | 6.23626+0.09887*Tau+0.00875*Pro-0.10729*Tyr-0.13461*His+0.01599*Lys+0.00729*Arg |
| 0.8963 | 6.96989+0.09873*Tau-0.08987*Cit-0.10998*Tyr+0.03194*Phe-0.11248*His+0.03738*Arg |
| 0.8952 | 6.28348+0.09891*Tau+0.00932*Pro-0.10623*Tyr-0.13330*His+0.01785*Lys |
| 0.8951 | 8.25642+0.09731*Tau+0.01071*Pro-0.00782*Gly-0.11207*Tyr-0.13517*His+0.01902*Lys |
| 0.8950 | 6.28522+0.09999*Tau-0.02107*Leu-0.09682*Tyr-0.11970*His+0.02102*Lys+0.01498*Arg |
| 0.8949 | 6.86693+0.09843*Tau+0.01067*Pro-0.02104*Ile-0.10507*Tyr-0.13720*His+0.02161*Lys |
| 0.8948 | 8.57271+0.09111*Tau-0.04752*Asn+0.01215*Pro-0.05669*Cit-0.07599*Tyr-0.09482*His |
| 0.8948 | 6.10970+0.09871*Tau+0.00933*Pro-0.11685*Tyr-0.14248*His+0.03661*Trp+0.01663*Lys |
| 0.8946 | 6.28712+0.09903*Tau-0.00116*Thr+0.00943*Pro-0.10599*Tyr-0.13272*His+0.01812*Lys |
| 0.8945 | 7.66171+0.09261*Tau-0.06595*Asn-0.00888*Leu-0.06869*Tyr-0.10404*His+0.03299*Arg |
| 0.8945 | 7.89431+0.09385*Tau-0.07122*Asn-0.00637*Val-0.06594*Tyr-0.10362*His+0.03374*Arg |
| 0.8943 | 8.88405+0.09129*Tau-0.00809*Gly-0.00691*Val-0.08288*Tyr-0.11519*His+0.03296*Arg |
| 0.8941 | 7.46256+0.09636*Tau+0.00363*Ala-0.09004*Cit-0.10906*Tyr-0.10623*His+0.03440*Arg |
| 0.8940 | 8.41704+0.10028*Tau-0.01972*Ser+0.01033*Pro-0.10695*Tyr-0.13366*His+0.01750*Lys |
| 0.8939 | 6.55556+0.09862*Tau+0.01068*Pro-0.08930*Met-0.09214*Tyr-0.13245*His+0.02283*Lys |
| 0.8939 | 7.58961+0.08533*Tau-0.10161*Asn+0.01083*Pro-0.01411*Val-0.11158*His+0.02336*Arg |
| 0.8939 | 7.45912+0.10229*Tau-0.08329*Asn+0.01118*Pro-0.09932*Tyr-0.12501*His+0.02298*Lys |
| 0.8936 | 7.85717+0.09323*Tau+0.02396*Thr-0.15253*Asn-0.01756*Val-0.12201*His+0.01989*Lys |

# FIG.147

| ROC AUC | FORMULA |
|---|---|
| 0.8936 | 6.86230+0.10138*Tau-0.04982*Cit-0.01759*Leu-0.09471*Tyr-0.10645*His+0.02397*Lys |
| 0.8933 | 9.15649+0.09546*Tau-0.02226*Ser-0.00638*Val-0.07930*Tyr-0.11549*His+0.03101*Arg |
| 0.8930 | 8.15103+0.09920*Tau-0.00722*Gly-0.02291*Leu-0.09459*Tyr-0.11698*His+0.02653*Lys |
| 0.8929 | 7.65293+0.09411*Tau-0.06675*Asn-0.07335*Tyr-0.11136*His+0.03093*Arg |
| 0.8928 | 7.73259+0.09587*Tau-0.08133*Asn-0.09073*Tyr-0.12631*His+0.05197*Trp+0.03279*Arg |
| 0.8927 | 8.66519+0.09290*Tau-0.05168*Asn-0.00505*Gly-0.08013*Tyr-0.11453*His+0.03332*Arg |
| 0.8927 | 8.32428+0.08928*Tau+0.02284*Thr-0.11281*Asn+0.00588*Ala-0.07877*Tyr-0.11042*His |
| 0.8926 | 7.85632+0.09056*Tau-0.07304*Asn+0.01127*Pro-0.08207*Tyr-0.11538*His+0.04833*Trp |
| 0.8926 | 6.29563+0.10028*Tau-0.02036*Leu-0.09198*Tyr-0.11634*His+0.02460*Lys |
| 0.8925 | 7.62180+0.09427*Tau-0.12324*Asn+0.01445*Pro-0.02267*Val-0.12354*His+0.02526*Lys |
| 0.8924 | 6.18868+0.09790*Tau+0.00800*Pro+0.00242*Ala-0.11184*Tyr-0.13283*His+0.01707*Lys |
| 0.8924 | 6.71662+0.10208*Tau-0.06085*Cit-0.11311*Tyr-0.12171*His+0.04018*Trp+0.01763*Lys |
| 0.8924 | 8.10923+0.09906*Tau-0.00759*Gly-0.10565*Tyr-0.13395*His+0.01471*Lys+0.02009*Arg |
| 0.8922 | 8.69224+0.09552*Tau-0.01395*Ser-0.04230*Asn-0.07908*Tyr-0.11515*His+0.03113*Arg |
| 0.8921 | 6.05582+0.10064*Tau-0.02582*Leu-0.10691*Tyr-0.12453*His+0.04824*Trp+0.02481*Lys |
| 0.8919 | 8.80281+0.09592*Tau-0.02095*Ser-0.08638*Tyr-0.12187*His+0.02843*Arg |
| 0.8919 | 7.96451+0.10122*Tau-0.00483*Gly-0.04569*Cit-0.10113*Tyr-0.11725*His+0.01947*Lys |
| 0.8918 | 6.89435+0.10186*Tau-0.05683*Cit-0.09956*Tyr-0.11393*His+0.01889*Lys |
| 0.8916 | 8.20837+0.10037*Tau-0.01804*Ser-0.02158*Leu-0.08936*Tyr-0.11524*His+0.02464*Lys |
| 0.8915 | 8.26192+0.09286*Tau-0.00758*Gly-0.10649*Tyr-0.13396*His+0.04267*Trp+0.03069*Arg |
| 0.8914 | 7.73247+0.10521*Tau-0.08035*Asn-0.01136*Val-0.07916*Tyr-0.10997*His+0.02797*Lys |
| 0.8908 | 7.37707+0.10368*Tau-0.07941*Asn-0.08295*Tyr-0.12435*His+0.01847*Lys+0.01997*Arg |
| 0.8907 | 7.31263+0.10316*Tau-0.07283*Asn-0.02146*Leu-0.08357*Tyr-0.10685*His+0.02929*Lys |
| 0.8907 | 8.91093+0.09758*Tau-0.02419*Ser-0.10605*Tyr-0.13733*His+0.04966*Trp+0.03002*Arg |
| 0.8906 | 8.29235+0.10164*Tau-0.01968*Ser-0.10054*Tyr-0.13183*His+0.01337*Lys+0.01913*Arg |
| 0.8905 | 6.73110+0.09768*Tau-0.07140*Asn-0.09329*Tyr+0.04935*Phe-0.12034*His+0.03131*Arg |
| 0.8904 | 8.10144+0.09763*Tau-0.01933*Ser-0.09763*Tyr+0.02846*Phe-0.12798*His+0.02803*Arg |
| 0.8901 | 7.82693+0.09962*Tau-0.00635*Gly-0.09891*Tyr-0.12815*His+0.01943*Lys |
| 0.8899 | 6.34674+0.09919*Tau+0.00505*Ala-0.00937*Val-0.10524*Tyr-0.12167*His+0.02079*Lys |
| 0.8898 | 7.55277+0.10432*Tau-0.05965*Asn-0.03999*Cit-0.09338*Tyr-0.11052*His+0.02258*Lys |
| 0.8885 | 7.27896+0.10395*Tau-0.07564*Asn+0.02083*Met-0.09203*Tyr-0.11890*His+0.02200*Lys |
| 0.8884 | 7.27826+0.10380*Tau-0.07054*Asn-0.08890*Tyr-0.11915*His+0.02282*Lys |
| 0.8882 | 6.09580+0.09818*Tau+0.00545*Ala-0.02348*Leu-0.10871*Tyr-0.11556*His+0.02382*Lys |
| 0.8882 | 8.02233+0.10328*Tau-0.05994*Asn-0.00359*Gly-0.09234*Tyr-0.12107*His+0.02283*Lys |
| 0.8881 | 7.86237+0.10363*Tau-0.00734*Ser-0.05744*Asn-0.09017*Tyr-0.12029*His+0.02198*Lys |
| 0.8878 | 6.58484+0.08864*Tau-0.10707*Asn+0.01316*Pro-0.03366*Leu-0.12196*His+0.02388*Lys |
| 0.8877 | 7.70183+0.09196*Tau-0.07813*Asn+0.00580*Ala-0.08680*Tyr-0.11197*His+0.02610*Arg |
| 0.8875 | 7.47273+0.10346*Tau-0.07052*Asn-0.00806*Ile-0.08744*Tyr-0.11992*His+0.02421*Lys |
| 0.8875 | 8.62925+0.08975*Tau-0.00884*Gly+0.00597*Ala-0.10763*Tyr-0.12421*His+0.02568*Arg |
| 0.8874 | 7.38324+0.10487*Tau-0.08219*Asn-0.10226*Tyr-0.13050*His+0.04513*Trp+0.02203*Lys |
| 0.8874 | 7.87696+0.08790*Tau-0.07017*Asn+0.00847*Pro+0.00514*Ala-0.07908*Tyr-0.10404*His |
| 0.8874 | 6.76840+0.09994*Tau+0.00453*Ala-0.05671*Cit-0.11402*Tyr-0.11494*His+0.01741*Lys |
| 0.8868 | 6.81688+0.10451*Tau-0.07160*Asn-0.09714*Tyr+0.02584*Phe-0.12222*His+0.02150*Lys |
| 0.8867 | 8.94896+0.09484*Tau-0.02361*Ser+0.00549*Ala-0.10283*Tyr-0.12360*His+0.02369*Arg |
| 0.8863 | 6.08733+0.09817*Tau+0.00451*Ala-0.10902*Tyr-0.12800*His+0.01680*Lys |
| 0.8838 | 8.03403+0.09679*Tau-0.00806*Gly+0.00596*Ala-0.11772*Tyr-0.12937*His+0.01758*Lys |
| 0.8838 | 6.49391+0.09747*Tau+0.00528*Ala-0.01717*Ile-0.10896*Tyr-0.12993*His+0.01958*Lys |
| 0.8834 | 7.32784+0.10131*Tau-0.08340*Asn+0.00608*Ala-0.10468*Tyr-0.12037*His+0.02149*Lys |
| 0.8824 | 8.29402+0.09954*Tau-0.02043*Ser+0.00573*Ala-0.11274*Tyr-0.12901*His+0.01641*Lys |
| 0.8763 | 6.59477+0.09271*Tau-0.07146*Asn+0.00798*Ala-0.10550*Tyr+0.05941*Phe-0.11019*His |

# FIG.148

## FIG.149

| ROC AUC | FORMULA |
|---|---|
| 0.7182 | 0.0880+0.0011*Tau+0.0001*Ala+0.0151*Ile-0.0071*Leu-0.0020*Tyr+0.0020*His |
| 0.7173 | 0.1442+0.0012*Tau-0.0028*Val+0.0166*Ile-0.0044*Leu-0.0007*Tyr+0.0031*His |
| 0.7159 | 0.1008+0.0011*Tau+0.0157*Ile-0.0073*Leu-0.0017*Tyr+0.0024*His-0.0002*Lys |
| 0.7154 | 0.2313+0.0012*Tau+0.0003*Ala-0.0027*Val+0.0141*Ile-0.0027*Leu-0.0010*Tyr |
| 0.7149 | 0.1641+0.0011*Tau+0.0002*Ala+0.0137*Ile-0.0060*Leu-0.0019*Tyr-0.0001*Lys |
| 0.7144 | 0.2279+0.0012*Tau+0.0013*Asn-0.0024*Val+0.0146*Ile-0.0031*Leu-0.0007*Tyr |
| 0.7127 | 0.0909+0.0011*Tau+0.0156*Ile-0.0074*Leu-0.0018*Tyr+0.0023*His |
| 0.7126 | 0.1212+0.0010*Tau+0.0161*Met+0.0095*Ile-0.0050*Leu-0.0030*Tyr-0.0013*His |
| 0.7125 | 0.0802+0.0009*Tau+0.0017*Glu+0.0152*Ile-0.0074*Leu-0.0022*Tyr+0.0024*His |
| 0.7124 | 0.0874+0.0011*Tau+0.0155*Ile-0.0074*Leu-0.0018*Tyr+0.0003*Phe+0.0022*His |
| 0.7121 | 0.1198+0.0013*Tau+0.0160*Ile-0.0071*Leu-0.0006*Tyr+0.0039*His-0.0059*Trp |
| 0.7120 | 0.1419+0.0011*Tau-0.0006*Ser+0.0159*Ile-0.0076*Leu-0.0018*Tyr+0.0026*His |
| 0.7119 | 0.2085+0.0012*Tau+0.0029*Cit-0.0027*Val+0.0150*Ile-0.0030*Leu-0.0004*Tyr |
| 0.7115 | 0.0889+0.0011*Tau+0.0000*Gln+0.0156*Ile-0.0074*Leu-0.0018*Tyr+0.0023*His |
| 0.7112 | 0.2100+0.0011*Tau-0.0005*Ser+0.0003*Ala+0.0137*Ile-0.0061*Leu-0.0020*Tyr |
| 0.7109 | 0.1191+0.0011*Tau+0.0017*Asn+0.0002*Ala+0.0137*Ile-0.0060*Leu-0.0021*Tyr |
| 0.7103 | 0.0323+0.0011*Tau+0.0137*Ile-0.0066*Leu-0.0022*Tyr+0.0016*His+0.0028*Orn |
| 0.7102 | 0.0867+0.0011*Tau+0.0003*Cit+0.0156*Ile-0.0074*Leu-0.0018*Tyr+0.0022*His |
| 0.7102 | 0.2251+0.0010*Tau-0.0103*ABA+0.0143*Ile-0.0061*Leu-0.0014*Tyr+0.0025*His |
| 0.7098 | 0.1415+0.0011*Tau-0.0002*Ala-0.0008*Cit+0.0138*Ile-0.0061*Leu-0.0019*Tyr |
| 0.7097 | 0.0574+0.0011*Tau+0.0017*Asn+0.0153*Ile-0.0072*Leu-0.0020*Tyr+0.0020*His |
| 0.7096 | 0.1420+0.0011*Tau-0.0002*Ala+0.0136*Ile-0.0061*Leu-0.0021*Tyr+0.0007*Phe |
| 0.7096 | 0.2607+0.0012*Tau-0.0025*Val+0.0147*Ile-0.0031*Leu-0.0005*Tyr+0.0000*Lys |
| 0.7093 | 0.3024+0.0011*Tau-0.0002*Ala-0.0101*ABA+0.0124*Ile-0.0047*Leu-0.0015*Tyr |
| 0.7093 | 0.3119+0.0012*Tau-0.0004*Ser-0.0025*Val+0.0148*Ile-0.0031*Leu-0.0004*Tyr |
| 0.7091 | 0.1630+0.0011*Tau-0.0012*Cit+0.0143*Ile-0.0062*Leu-0.0015*Tyr-0.0001*Lys |
| 0.7090 | 0.1346+0.0011*Tau-0.0025*Asn+0.0141*Ile-0.0060*Leu-0.0018*Tyr-0.0002*Lys |
| 0.7090 | 0.1521+0.0009*Tau+0.0016*Glu+0.0002*Ala+0.0132*Ile-0.0060*Leu-0.0023*Tyr |
| 0.7089 | 0.3817+0.0012*Tau-0.0093*ABA-0.0021*Val+0.0133*Ile-0.0023*Leu-0.0003*Tyr |
| 0.7089 | 0.1459+0.0011*Tau-0.0000*Gln+0.0002*Ala+0.0137*Ile-0.0060*Leu-0.0019*Tyr |
| 0.7088 | 0.0834+0.0011*Tau+0.0001*Ala+0.0124*Ile-0.0056*Leu-0.0022*Tyr+0.0028*Orn |
| 0.7087 | 0.2267+0.0013*Tau+0.0003*Ala+0.0130*Ile-0.0050*Leu-0.0010*Tyr-0.0052*Trp |
| 0.7087 | 0.1579+0.0011*Tau-0.0002*Ala+0.0137*Ile-0.0060*Leu-0.0019*Tyr |
| 0.7085 | 0.1054+0.0011*Tau-0.0023*Asn+0.0139*Ile-0.0062*Leu-0.0021*Tyr+0.0008*Phe |
| 0.7084 | 0.1146+0.0011*Tau-0.0021*Asn+0.0007*Cit+0.0141*Ile-0.0062*Leu-0.0019*Tyr |
| 0.7083 | 0.2378+0.0012*Tau-0.0026*Val+0.0145*Ile-0.0031*Leu-0.0007*Tyr+0.0013*Phe |
| 0.7083 | 0.2639+0.0012*Tau-0.0025*Val+0.0147*Ile-0.0030*Leu-0.0004*Tyr |
| 0.7083 | 0.0841+0.0010*Tau-0.0001*Ala+0.0152*Met+0.0107*Ile-0.0057*Leu-0.0029*Tyr |
| 0.7082 | 0.2161+0.0011*Tau-0.0003*Ser+0.0143*Ile-0.0062*Leu-0.0015*Tyr+0.0000*Lys |
| 0.7081 | 0.3083+0.0014*Tau-0.0021*Val+0.0143*Ile-0.0029*Leu+0.0002*Tyr-0.0036*Trp |
| 0.7081 | 0.1483+0.0011*Tau-0.0011*Cit+0.0142*Ile-0.0063*Leu-0.0016*Tyr+0.0006*Phe |
| 0.7080 | 0.1423+0.0010*Tau+0.0172*Met+0.0103*Ile-0.0049*Leu-0.0026*Tyr-0.0011*Lys |
| 0.7080 | 0.1696+0.0013*Tau-0.0029*Val+0.0129*Ile-0.0020*Leu-0.0009*Tyr+0.0035*Orn |
| 0.7075 | 0.1830+0.0012*Tau-0.0009*Ser+0.0126*Ile-0.0057*Leu-0.0020*Tyr+0.0033*Orn |
| 0.7075 | 0.1699+0.0011*Tau-0.0006*Ser+0.0002*Gln+0.0143*Ile-0.0063*Leu-0.0016*Tyr |
| 0.7074 | 0.1262+0.0011*Tau+0.0022*Asn+0.0141*Ile-0.0061*Leu-0.0019*Tyr |
| 0.7074 | 0.1045+0.0009*Tau+0.0028*Asn+0.0018*Glu+0.0135*Ile-0.0061*Leu-0.0025*Tyr |
| 0.7072 | 0.2624+0.0010*Tau+0.0021*Glu-0.0027*Val+0.0141*Ile-0.0028*Leu-0.0009*Tyr |
| 0.7072 | 0.1594+0.0011*Tau-0.0027*Val+0.0155*Met+0.0110*Ile-0.0023*Leu-0.0019*Tyr |
| 0.7071 | 0.2792+0.0011*Tau+0.0024*Cit-0.0105*ABA+0.0129*Ile-0.0050*Leu-0.0012*Tyr |

# FIG.150

| ROC AUC | FORMULA |
|---|---|
| 0.7070 | 0.1951+0.0011*Tau-0.0004*Ser+0.0013*Cit+0.0144*Ile-0.0063*Leu-0.0015*Tyr |
| 0.7070 | 0.1814+0.0011*Tau-0.0007*Ser+0.0030*Asn+0.0142*Ile-0.0062*Leu-0.0020*Tyr |
| 0.7069 | 0.0675+0.0011*Tau+0.0012*Asn+0.0126*Ile-0.0057*Leu-0.0022*Tyr+0.0028*Orn |
| 0.7068 | 0.2733+0.0011*Tau+0.0020*Asn-0.0101*ABA+0.0127*Ile-0.0048*Leu-0.0015*Tyr |
| 0.7067 | 0.2246+0.0012*Tau+0.0001*Gln-0.0025*Val+0.0147*Ile-0.0031*Leu-0.0005*Tyr |
| 0.7067 | 0.2179+0.0011*Tau-0.0003*Ser+0.0143*Ile-0.0061*Leu-0.0015*Tyr |
| 0.7067 | 0.1748+0.0013*Tau+0.0036*Asn+0.0135*Ile-0.0051*Leu-0.0010*Tyr-0.0052*Trp |
| 0.7066 | 0.2658+0.0013*Tau-0.0001*Ser+0.0138*Ile-0.0052*Leu-0.0004*Tyr-0.0046*Trp |
| 0.7066 | 0.0774+0.0010*Tau+0.0000*Asn+0.0149*Met+0.0106*Ile-0.0057*Leu-0.0030*Tyr |
| 0.7065 | 0.1990+0.0010*Tau-0.0002*Ser+0.0016*Glu+0.0138*Ile-0.0061*Leu-0.0019*Tyr |
| 0.7063 | 0.3127+0.0027*Glu-0.0026*Val+0.0137*Ile-0.0030*Leu-0.0006*Tyr+0.0001*Lys |
| 0.7062 | 0.1527+0.0023*Glu+0.0154*Ile-0.0074*Leu-0.0008*Tyr+0.0042*His-0.0053*Trp |
| 0.7061 | 0.2866+0.0022*Glu+0.0129*Ile-0.0055*Leu-0.0007*Tyr-0.0041*Trp+0.0004*Lys |
| 0.7059 | 0.3594+0.0026*Glu-0.0023*Val+0.0134*Ile-0.0028*Leu-0.0000*Tyr-0.0027*Trp |
| 0.7058 | 0.1282+0.0022*Glu+0.0152*Ile-0.0076*Leu-0.0018*Tyr+0.0029*His-0.0002*Lys |
| 0.7058 | 0.1604+0.0022*Glu+0.0002*Gln+0.0133*Ile-0.0061*Leu-0.0016*Tyr-0.0001*Lys |
| 0.7056 | 0.1870+0.0023*Glu+0.0003*Gln+0.0128*Ile-0.0054*Leu-0.0007*Tyr-0.0044*Trp |
| 0.7056 | 0.0880+0.0023*Glu+0.0001*Gln+0.0149*Ile-0.0076*Leu-0.0020*Tyr+0.0026*His |
| 0.7054 | 0.2607+0.0022*Glu-0.0024*Cit+0.0132*Ile-0.0055*Leu-0.0006*Tyr-0.0042*Trp |
| 0.7054 | 0.1949+0.0046*Asn-0.0025*Glu+0.0125*Ile-0.0052*Leu-0.0014*Tyr-0.0047*Trp |
| 0.7054 | 0.2230+0.0021*Glu+0.0111*Ile-0.0047*Leu-0.0010*Tyr-0.0046*Trp+0.0033*Orn |
| 0.7054 | 0.4573+0.0027*Glu-0.0110*ABA+0.0113*Ile-0.0039*Leu-0.0004*Tyr-0.0040*Trp |
| 0.7054 | 0.2446+0.0027*Glu+0.0002*Gln-0.0026*Val+0.0137*Ile-0.0030*Leu-0.0007*Tyr |
| 0.7053 | 0.2075+0.0022*Glu+0.0014*Cit+0.0135*Ile-0.0062*Leu-0.0016*Tyr-0.0000*Lys |
| 0.7052 | 0.1540+0.0036*Asn-0.0024*Glu+0.0132*Ile-0.0060*Leu-0.0022*Tyr-0.0002*Lys |
| 0.7052 | 0.2844+0.0022*Glu+0.0128*Ile-0.0055*Leu-0.0008*Tyr+0.0012*Phe-0.0040*Trp |
| 0.7051 | 0.2046+0.0021*Glu+0.0003*Ala+0.0128*Ile-0.0060*Leu-0.0021*Tyr-0.0001*Lys |
| 0.7050 | 0.3487+0.0027*Glu-0.0114*ABA+0.0115*Ile-0.0050*Leu-0.0016*Tyr+0.0005*Lys |
| 0.7049 | 0.3085+0.0022*Glu+0.0130*Ile-0.0054*Leu-0.0006*Tyr-0.0039*Trp |
| 0.7048 | 0.2961+0.0001*Ser+0.0022*Glu+0.0130*Ile-0.0053*Leu-0.0006*Tyr-0.0039*Trp |
| 0.7048 | 0.2039+0.0033*Asn+0.0001*Gln+0.0133*Ile-0.0053*Leu-0.0005*Tyr-0.0046*Trp |
| 0.7047 | 0.2349+0.0038*Asn+0.0133*Ile-0.0053*Leu-0.0005*Tyr-0.0045*Trp+0.0001*Lys |
| 0.7047 | 0.1824+0.0027*Asn+0.0000*Gln+0.0139*Ile-0.0060*Leu-0.0013*Tyr-0.0002*Lys |
| 0.7046 | 0.1756+0.0159*Ile-0.0074*Leu-0.0002*Tyr+0.0041*His-0.0053*Trp+0.0001*Lys |
| 0.7046 | 0.1215+0.0022*Asn-0.0001*Gln+0.0154*Ile-0.0075*Leu-0.0016*Tyr+0.0025*His |
| 0.7046 | 0.2882+0.0019*Asn-0.0022*Val+0.0143*Ile-0.0034*Leu-0.0002*Tyr-0.0001*Lys |
| 0.7046 | 0.1170+0.0031*Asn+0.0155*Ile-0.0071*Leu-0.0006*Tyr+0.0038*His-0.0057*Trp |
| 0.7045 | 0.2699+0.0023*Cit+0.0137*Ile-0.0056*Leu-0.0000*Tyr-0.0043*Trp+0.0002*Lys |
| 0.7045 | 0.1380+0.0001*Gln+0.0158*Ile-0.0073*Leu-0.0001*Tyr+0.0039*His-0.0054*Trp |
| 0.7044 | 0.3011+0.0029*Asn-0.0017*Val+0.0138*Ile-0.0034*Leu+0.0002*Tyr-0.0035*Trp |
| 0.7044 | 0.3597-0.0019*Val+0.0140*Ile-0.0033*Leu+0.0005*Tyr-0.0031*Trp+0.0003*Lys |
| 0.7043 | 0.2644+0.0014*Asn-0.0001*Gln-0.0022*Val+0.0143*Ile-0.0034*Leu-0.0002*Tyr |
| 0.7043 | 0.1680+0.0024*Asn+0.0000*Gln+0.0008*Cit+0.0139*Ile-0.0062*Leu-0.0014*Tyr |
| 0.7043 | 0.2823+0.0031*Cit-0.0026*Val+0.0148*Ile-0.0032*Leu+0.0002*Tyr-0.0001*Lys |
| 0.7043 | 0.1295+0.0000*Gln+0.0003*Cit+0.0155*Ile-0.0075*Leu-0.0013*Tyr+0.0025*His |
| 0.7042 | 0.2643+0.0014*Asn-0.0001*Gln-0.0023*Val+0.0144*Ile-0.0034*Leu-0.0001*Lys |
| 0.7042 | 0.2103-0.0027*Val+0.0168*Ile-0.0047*Leu-0.0001*Tyr+0.0036*His-0.0003*Lys |
| 0.7042 | 0.3164-0.0107*ABA+0.0146*Ile-0.0061*Leu+0.0002*Tyr+0.0044*His-0.0055*Trp |
| 0.7042 | 0.2170-0.0022*Val+0.0167*Ile-0.0051*Leu+0.0005*Tyr+0.0046*His-0.0043*Trp |
| 0.7041 | 0.1367+0.0001*Gln+0.0157*Ile-0.0074*Leu-0.0012*Tyr+0.0027*His-0.0003*Lys |

# FIG.151

# FIG.152

| ROC AUC | FORMULA |
|---|---|
| 0.6782 | -0.1542+0.0121*Glu-0.0536*ABA+0.0505*Ile-0.0171*Leu-0.0015*Tyr-0.0177*Trp |
| 0.6780 | -1.6033+0.0050*Tau-0.0125*Val+0.0786*Met+0.0516*Ile-0.0115*Leu-0.0105*Tyr |
| 0.6720 | -0.4595+0.0054*Tau-0.0464*ABA-0.0098*Val+0.0599*Ile-0.0097*Leu-0.0012*Tyr |
| 0.6719 | -0.7722-0.0509*ABA+0.0656*Ile-0.0271*Leu+0.0010*Tyr+0.0197*His-0.0245*Trp |
| 0.6706 | -0.5982+0.0117*Glu-0.0103*Val+0.0597*Ile-0.0123*Leu-0.0002*Tyr-0.0119*Trp |
| 0.6701 | -0.8154+0.0060*Tau-0.0093*Val+0.0635*Ile-0.0126*Leu+0.0007*Tyr-0.0159*Trp |
| 0.6698 | -0.8480+0.0129*Asn-0.0077*Val+0.0611*Ile-0.0149*Leu+0.0007*Tyr-0.0153*Trp |
| 0.6685 | -0.5957-0.0087*Val+0.0620*Ile-0.0148*Leu+0.0024*Tyr-0.0135*Trp+0.0014*Lys |
| 0.6676 | -1.4384+0.0057*Tau-0.0133*Val+0.0577*Ile-0.0087*Leu-0.0042*Tyr+0.0157*Orn |
| 0.6657 | -0.7869+0.0055*Tau-0.0020*Ser-0.0115*Val+0.0658*Ile-0.0133*Leu-0.0019*Tyr |
| 0.6649 | -1.0164+0.0046*Tau+0.0095*Glu-0.0124*Val+0.0631*Ile-0.0121*Leu-0.0041*Tyr |
| 0.6646 | -0.6280+0.0123*Glu-0.0556*ABA+0.0513*Ile-0.0219*Leu-0.0071*Tyr+0.0024*Lys |
| 0.6629 | -1.1601+0.0053*Tau-0.0056*Asn-0.0110*Val+0.0650*Ile-0.0137*Leu-0.0032*Tyr |
| 0.6626 | -0.8021+0.0120*Glu-0.0120*Val+0.0611*Ile-0.0129*Leu-0.0028*Tyr+0.0005*Lys |
| 0.6625 | -1.0013+0.0054*Tau-0.0114*Val+0.0654*Ile-0.0132*Leu-0.0021*Tyr |
| 0.6622 | -1.0168+0.0054*Tau-0.0114*Val+0.0653*Ile-0.0134*Leu-0.0022*Tyr+0.0002*Lys |
| 0.6618 | -1.1711+0.0054*Tau+0.0004*Gln-0.0114*Val+0.0652*Ile-0.0135*Leu-0.0023*Tyr |
| 0.6617 | -1.1499+0.0052*Tau+0.0014*Ala-0.0122*Val+0.0626*Ile-0.0118*Leu-0.0044*Tyr |
| 0.6603 | -1.1002+0.0123*Glu+0.0007*Gln-0.0119*Val+0.0609*Ile-0.0130*Leu-0.0031*Tyr |
| 0.6602 | -1.1080+0.0055*Tau-0.0118*Val+0.0644*Ile-0.0132*Leu-0.0030*Tyr+0.0054*Phe |
| 0.6597 | -1.2585+0.0054*Tau-0.0133*Cit-0.0126*Val+0.0667*Ile-0.0131*Leu-0.0019*Tyr |
| 0.6597 | -1.2240-0.0099*Val+0.0742*Ile-0.0224*Leu+0.0022*Tyr+0.0203*His-0.0191*Trp |
| 0.6595 | -0.9960+0.0063*Asn+0.0003*Gln-0.0102*Val+0.0636*Ile-0.0149*Leu-0.0005*Lys |
| 0.6594 | -0.8986+0.0081*Asn-0.0100*Val+0.0634*Ile-0.0148*Leu-0.0011*Tyr-0.0003*Lys |
| 0.6592 | -0.9935+0.0063*Asn+0.0003*Gln-0.0101*Val+0.0634*Ile-0.0150*Leu-0.0010*Tyr |
| 0.6576 | -1.1579+0.0047*Tau-0.0502*ABA+0.0638*Ile-0.0270*Leu-0.0065*Tyr+0.0110*His |
| 0.6576 | -1.6770+0.0045*Tau+0.0936*Met+0.0487*Ile-0.0232*Leu-0.0145*Tyr-0.0060*Lys |
| 0.6575 | -0.9413+0.0048*Tau+0.0085*Asn-0.0495*ABA+0.0564*Ile-0.0210*Leu-0.0066*Tyr |
| 0.6572 | -0.8207+0.0048*Tau+0.0008*Ala-0.0493*ABA+0.0553*Ile-0.0207*Leu-0.0067*Tyr |
| 0.6571 | -1.5351+0.0051*Tau-0.0127*Val+0.0740*Ile-0.0192*Leu-0.0032*Tyr+0.0138*His |
| 0.6569 | -1.2112+0.0095*Glu+0.0492*Ile-0.0209*Leu-0.0043*Tyr-0.0206*Trp+0.0144*Orn |
| 0.6562 | -0.9254+0.0049*Tau-0.0106*Cit-0.0514*ABA+0.0577*Ile-0.0217*Leu-0.0053*Tyr |
| 0.6559 | -1.1386+0.0166*Asn+0.0583*Ile-0.0234*Leu-0.0022*Tyr-0.0201*Trp+0.0003*Lys |
| 0.6554 | -1.4145+0.0057*Tau-0.0162*Asn+0.0595*Ile-0.0226*Leu-0.0041*Tyr-0.0234*Trp |
| 0.6547 | -0.9280+0.0141*Cit-0.0117*Val+0.0654*Ile-0.0139*Leu+0.0008*Tyr-0.0004*Lys |
| 0.6545 | -1.3356+0.0206*Asn-0.0113*Glu+0.0552*Ile-0.0231*Leu-0.0060*Tyr-0.0208*Trp |
| 0.6540 | -1.2819+0.0144*Asn-0.0005*Gln+0.0584*Ile-0.0233*Leu-0.0018*Tyr-0.0205*Trp |
| 0.6537 | -1.2477-0.0120*Val+0.0741*Ile-0.0206*Leu-0.0005*Tyr+0.0160*His-0.0011*Lys |
| 0.6521 | -0.9180+0.0097*Glu+0.0566*Ile-0.0244*Leu-0.0032*Tyr-0.0184*Trp+0.0016*Lys |
| 0.6507 | -0.3787+0.0006*Ser+0.0097*Glu+0.0570*Ile-0.0235*Leu-0.0026*Tyr-0.0175*Trp |
| 0.6505 | -1.8020+0.0046*Tau+0.0844*Met+0.0453*Ile-0.0240*Leu-0.0156*Tyr-0.0055*His |
| 0.6504 | -0.8190+0.0096*Glu+0.0571*Ile-0.0236*Leu-0.0026*Tyr-0.0173*Trp |
| 0.6502 | -1.1862+0.0056*Tau+0.0016*Ala+0.0571*Ile-0.0218*Leu-0.0043*Tyr-0.0235*Trp |
| 0.6499 | -1.9430+0.0046*Tau-0.0006*Ala+0.0826*Met+0.0505*Ile-0.0274*Leu-0.0150*Tyr |
| 0.6495 | -0.9844+0.0104*Cit+0.0601*Ile-0.0248*Leu+0.0000*Tyr-0.0193*Trp+0.0010*Lys |
| 0.6485 | -1.3695+0.0101*Glu+0.0013*Gln+0.0564*Ile-0.0238*Leu-0.0029*Tyr-0.0197*Trp |
| 0.6484 | -1.0045+0.0058*Tau-0.0002*Ser+0.0608*Ile-0.0230*Leu-0.0019*Tyr-0.0206*Trp |
| 0.6483 | -1.0290+0.0095*Glu+0.0107*Cit+0.0578*Ile-0.0243*Leu-0.0025*Tyr-0.0188*Trp |
| 0.6483 | -0.9154+0.0097*Glu+0.0561*Ile-0.0239*Leu-0.0035*Tyr+0.0048*Phe-0.0178*Trp |
| 0.6478 | -1.8623+0.0045*Tau-0.0059*Asn+0.0830*Met+0.0500*Ile-0.0273*Leu-0.0153*Tyr |

# FIG.153

| ROC AUC | FORMULA |
|---|---|
| 0.6467 | −1.5151+0.0101*Glu+0.0681*Ile−0.0328*Leu−0.0037*Tyr+0.0188*His−0.0239*Trp |
| 0.6465 | −1.6514+0.0131*Asn+0.0683*Ile−0.0314*Leu−0.0024*Tyr+0.0169*His−0.0253*Trp |
| 0.6464 | −1.6620+0.0056*Tau+0.0710*Ile−0.0315*Leu−0.0027*Tyr+0.0175*His−0.0267*Trp |
| 0.6449 | −1.3717+0.0051*Tau−0.0041*Ser+0.0556*Ile−0.0252*Leu−0.0093*Tyr+0.0147*Orn |
| 0.6441 | −1.5786+0.0005*Gln+0.0697*Ile−0.0322*Leu−0.0006*Tyr+0.0174*His−0.0242*Trp |
| 0.6435 | −1.4056+0.0705*Ile−0.0327*Leu−0.0007*Tyr+0.0183*His−0.0237*Trp+0.0002*Lys |
| 0.6415 | −2.0484+0.0048*Tau+0.0608*Ile−0.0292*Leu−0.0099*Tyr+0.0074*His+0.0121*Orn |
| 0.6414 | −1.8847+0.0050*Tau−0.0051*Asn+0.0557*Ile−0.0251*Leu−0.0099*Tyr+0.0125*Orn |
| 0.6388 | −1.8164+0.0049*Tau+0.0005*Ala+0.0549*Ile−0.0249*Leu−0.0100*Tyr+0.0125*Orn |
| 0.6375 | −1.2567+0.0095*Glu+0.0060*Cit+0.0593*Ile−0.0274*Leu−0.0070*Tyr−0.0002*Lys |
| 0.6368 | −1.3697+0.0048*Tau−0.0031*Ser+0.0134*Asn+0.0622*Ile−0.0273*Leu−0.0089*Tyr |
| 0.6361 | −1.3582+0.0115*Asn+0.0002*Gln+0.0608*Ile−0.0264*Leu−0.0057*Tyr−0.0010*Lys |
| 0.6360 | −1.5064+0.0160*Asn+0.0108*Glu+0.0578*Ile−0.0264*Leu−0.0096*Tyr−0.0011*Lys |
| 0.6354 | −1.4244+0.0103*Asn+0.0001*Gln+0.0037*Cit+0.0610*Ile−0.0273*Leu−0.0060*Tyr |
| 0.6351 | −1.2736+0.0094*Glu+0.0012*Ala+0.0564*Ile−0.0263*Leu−0.0091*Tyr−0.0003*Lys |
| 0.6350 | −1.7281+0.0040*Tau+0.0128*Asn+0.0084*Glu+0.0594*Ile−0.0271*Leu−0.0110*Tyr |
| 0.6346 | −1.5714+0.0048*Tau+0.0109*Asn+0.0620*Ile−0.0263*Leu−0.0081*Tyr−0.0009*Lys |
| 0.6340 | −1.3026+0.0043*Tau−0.0007*Ser+0.0070*Glu+0.0607*Ile−0.0270*Leu−0.0085*Tyr |
| 0.6338 | −1.2418+0.0047*Tau−0.0023*Ser+0.0012*Ala+0.0604*Ile−0.0267*Leu−0.0088*Tyr |
| 0.6338 | −1.6182+0.0099*Glu+0.0671*Ile−0.0335*Leu−0.0082*Tyr+0.0128*His−0.0010*Lys |
| 0.6337 | −1.6110+0.0048*Tau+0.0098*Asn+0.0619*Ile−0.0269*Leu−0.0084*Tyr |
| 0.6329 | −1.6433+0.0047*Tau+0.0074*Asn+0.0008*Ala+0.0603*Ile−0.0266*Leu−0.0095*Tyr |
| 0.6329 | −1.5483+0.0047*Tau−0.0026*Ser+0.0701*Ile−0.0335*Leu−0.0081*Tyr+0.0114*His |
| 0.6328 | −1.8354+0.0040*Tau+0.0076*Glu+0.0671*Ile−0.0329*Leu−0.0100*Tyr+0.0108*His |
| 0.6328 | −1.6652+0.0048*Tau+0.0092*Asn−0.0032*Cit+0.0621*Ile−0.0272*Leu−0.0084*Tyr |
| 0.6326 | −1.4717+0.0099*Glu+0.0008*Gln+0.0587*Ile−0.0270*Leu−0.0072*Tyr−0.0005*Lys |
| 0.6325 | −1.7553+0.0100*Glu+0.0003*Gln+0.0659*Ile−0.0336*Leu−0.0087*Tyr+0.0116*His |
| 0.6323 | −1.4190+0.0048*Tau−0.0026*Ser+0.0008*Gln+0.0628*Ile−0.0275*Leu−0.0070*Tyr |
| 0.6320 | −1.3128+0.0049*Tau−0.0016*Ser+0.0059*Cit+0.0633*Ile−0.0276*Leu−0.0068*Tyr |
| 0.6320 | −1.5093+0.0041*Tau+0.0072*Glu+0.0010*Ala+0.0584*Ile−0.0265*Leu−0.0104*Tyr |
| 0.6314 | −1.2128+0.0050*Tau−0.0013*Ser+0.0628*Ile−0.0271*Leu−0.0067*Tyr+0.0001*Lys |
| 0.6313 | −1.2086+0.0050*Tau−0.0013*Ser+0.0628*Ile−0.0270*Leu−0.0067*Tyr |
| 0.6309 | −1.5676+0.0002*Gln+0.0692*Ile−0.0329*Leu−0.0052*Tyr+0.0120*His−0.0013*Lys |
| 0.6307 | −1.9135+0.0046*Tau+0.0070*Asn+0.0676*Ile−0.0319*Leu−0.0090*Tyr+0.0091*His |
| 0.6307 | −1.6906+0.0048*Tau+0.0101*Asn+0.0612*Ile−0.0273*Leu−0.0092*Tyr+0.0033*Phe |
| 0.6306 | −1.6282+0.0094*Asn−0.0003*Gln+0.0676*Ile−0.0328*Leu−0.0070*Tyr+0.0109*His |
| 0.6306 | −1.4422+0.0047*Tau+0.0010*Ala+0.0604*Ile−0.0261*Leu−0.0084*Tyr−0.0005*Lys |
| 0.6304 | −1.4752+0.0047*Tau+0.0010*Ala+0.0604*Ile−0.0265*Leu−0.0086*Tyr |
| 0.6304 | −1.7959+0.0047*Tau−0.0013*Cit+0.0688*Ile−0.0325*Leu−0.0080*Tyr+0.0100*His |
| 0.6302 | −1.4481+0.0049*Tau+0.0056*Cit+0.0631*Ile−0.0271*Leu−0.0067*Tyr−0.0004*Lys |
| 0.6301 | −1.7830+0.0047*Tau+0.0000*Gln+0.0688*Ile−0.0325*Leu−0.0080*Tyr+0.0102*His |
| 0.6299 | −1.7767+0.0047*Tau+0.0689*Ile−0.0325*Leu−0.0080*Tyr+0.0102*His |
| 0.6299 | −1.5242+0.0047*Tau+0.0001*Gln+0.0009*Ala+0.0604*Ile−0.0266*Leu−0.0086*Tyr |
| 0.6297 | −1.5476+0.0047*Tau+0.0009*Ala+0.0036*Cit+0.0608*Ile−0.0268*Leu−0.0086*Tyr |
| 0.6296 | −1.7879+0.0046*Tau+0.0006*Ala+0.0667*Ile−0.0315*Leu−0.0089*Tyr+0.0087*His |
| 0.6296 | −1.7289+0.0047*Tau+0.0696*Ile−0.0322*Leu−0.0076*Tyr+0.0109*His−0.0011*Lys |
| 0.6294 | −1.6004+0.0001*Gln+0.0014*Cit+0.0685*Ile−0.0333*Leu−0.0057*Tyr+0.0112*His |
| 0.6292 | −1.7882+0.0047*Tau+0.0686*Ile−0.0325*Leu−0.0082*Tyr+0.0008*Phe+0.0100*His |
| 0.6290 | −1.5099+0.0049*Tau+0.0048*Cit+0.0625*Ile−0.0275*Leu−0.0073*Tyr+0.0024*Phe |
| 0.6285 | −1.5360+0.0047*Tau+0.0010*Ala+0.0599*Ile−0.0268*Leu−0.0092*Tyr+0.0028*Phe |

# FIG.154

# FIG.155

| ROC AUC | FORMULA |
|---|---|
| 0.8361 | −0.0123+0.0024*Tau+0.0040*Thr−0.0119*ABA+0.0128*Met+0.0064*Tyr−0.0160*Trp |
| 0.8352 | −0.0336+0.0026*Tau+0.0045*Thr−0.0125*ABA+0.0071*Tyr−0.0183*Trp+0.0057*Orn |
| 0.8347 | 0.2480−0.0106*ABA−0.0007*Val+0.0245*Met+0.0074*Tyr−0.0024*His−0.0087*Trp |
| 0.8343 | −0.0286+0.0028*Tau+0.0280*Met+0.0062*Tyr−0.0088*Phe−0.0193*Trp+0.0080*Orn |
| 0.8343 | −0.1463+0.0024*Tau+0.0049*Thr+0.0029*Ser−0.0131*ABA+0.0075*Tyr−0.0164*Trp |
| 0.8324 | −0.0940+0.0030*Tau−0.0113*ABA+0.0194*Met+0.0055*Tyr−0.0175*Trp+0.0077*Orn |
| 0.8324 | −0.0929+0.0027*Tau+0.0047*Thr−0.0132*ABA+0.0070*Ile+0.0060*Tyr−0.0177*Trp |
| 0.8315 | 0.0206+0.0023*Tau+0.0057*Thr+0.0004*Pro−0.0118*ABA+0.0072*Tyr−0.0164*Trp |
| 0.8310 | 0.1641+0.0027*Asn−0.0109*ABA+0.0238*Met+0.0064*Tyr−0.0026*His−0.0101*Trp |
| 0.8305 | 0.1270+0.0048*Thr−0.0123*ABA+0.0117*Met+0.0072*Tyr−0.0027*His−0.0128*Trp |
| 0.8305 | 0.1163+0.0023*Tau+0.0059*Thr−0.0127*ABA+0.0080*Tyr−0.0023*His−0.0151*Trp |
| 0.8301 | −0.0580+0.0046*Thr+0.0083*Cit−0.0130*ABA+0.0119*Met+0.0068*Tyr−0.0170*Trp |
| 0.8301 | 0.0404+0.0023*Tau+0.0057*Thr+0.0002*Ala−0.0126*ABA+0.0072*Tyr−0.0168*Trp |
| 0.8301 | 0.0618+0.0023*Tau+0.0059*Thr−0.0008*Asn−0.0126*ABA+0.0077*Tyr−0.0162*Trp |
| 0.8301 | −0.1256+0.0028*Tau+0.0041*Thr−0.0028*Val+0.0108*Ile+0.0062*Tyr−0.0152*Trp |
| 0.8301 | 0.1713+0.0042*Thr−0.0106*ABA+0.0182*Met+0.0077*Tyr−0.0059*Phe−0.0144*Trp |
| 0.8296 | −0.0446+0.0030*Tau−0.0032*Val+0.0155*Met+0.0101*Ile+0.0061*Tyr−0.0121*Trp |
| 0.8296 | 0.1859+0.0030*Tau−0.0013*Val+0.0234*Met+0.0098*Ile−0.0065*Phe−0.0139*Trp |
| 0.8291 | 0.1755+0.0025*Tau−0.0311*Met+0.0071*Tyr−0.0074*Phe−0.0023*His−0.0138*Trp |
| 0.8291 | −0.1516+0.0029*Tau−0.0012*Val+0.0208*Met+0.0053*Tyr−0.0168*Trp+0.0078*Orn |
| 0.8291 | 0.1952+0.0025*Tau−0.0093*ABA+0.0297*Met+0.0075*Tyr−0.0067*Phe−0.0141*Trp |
| 0.8291 | 0.0224+0.0028*Tau+0.0021*Thr−0.0094*ABA+0.0172*Met−0.0174*Trp+0.0068*Orn |
| 0.8291 | 0.0172+0.0023*Tau+0.0058*Thr−0.0128*ABA+0.0005*Val+0.0072*Tyr−0.0171*Trp |
| 0.8287 | 0.1487+0.0027*Tau−0.0110*ABA+0.0237*Met+0.0068*Tyr−0.0030*His−0.0120*Trp |
| 0.8287 | 0.0399+0.0023*Tau+0.0058*Thr+0.0006*Glu−0.0129*ABA+0.0075*Tyr−0.0163*Trp |
| 0.8287 | 0.0476+0.0023*Tau+0.0059*Thr−0.0126*ABA+0.0076*Tyr−0.0163*Trp |
| 0.8287 | 0.1299+0.0027*Asn−0.0103*ABA−0.0008*Val+0.0241*Met+0.0065*Tyr−0.0102*Trp |
| 0.8287 | 0.1936−0.0063*Glu−0.0121*ABA+0.0341*Met+0.0075*Tyr−0.0095*Phe−0.0129*Trp |
| 0.8282 | 0.0720+0.0047*Thr−0.0119*ABA−0.0005*Val+0.0117*Met+0.0070*Tyr−0.0135*Trp |
| 0.8282 | −0.0604+0.0024*Tau+0.0058*Thr+0.0087*Cit−0.0135*ABA+0.0077*Tyr−0.0193*Trp |
| 0.8282 | −0.0190+0.0023*Tau+0.0054*Thr+0.0004*Gly−0.0120*ABA+0.0075*Tyr−0.0160*Trp |
| 0.8282 | 0.0341+0.0023*Tau+0.0058*Thr−0.0127*ABA+0.0074*Tyr−0.0005*Phe−0.0163*Trp |
| 0.8277 | 0.0994+0.0070*Glu−0.0136*ABA−0.0021*Val+0.0271*Met+0.0072*Tyr−0.0090*Trp |
| 0.8273 | −0.0165+0.0042*Thr+0.0035*Glu−0.0140*ABA+0.0133*Met+0.0063*Tyr−0.0144*Trp |
| 0.8273 | 0.1936−0.0091*ABA+0.0292*Met+0.0076*Tyr−0.0094*Phe−0.0152*Trp+0.0070*Orn |
| 0.8273 | 0.0543+0.0023*Tau+0.0049*Thr−0.0109*ABA+0.0083*Ile−0.0004*His−0.0168*Trp |
| 0.8268 | 0.0873−0.0105*ABA−0.0014*Val+0.0216*Met+0.0069*Tyr−0.0121*Trp+0.0067*Orn |
| 0.8268 | 0.2293−0.0110*ABA+0.0239*Met+0.0071*Tyr−0.0028*His−0.0094*Trp |
| 0.8268 | 0.1945−0.0103*ABA−0.0010*Val+0.0243*Met+0.0072*Tyr−0.0095*Trp |
| 0.8268 | −0.0083+0.0030*Tau+0.0040*Thr−0.0109*ABA+0.0072*Ile−0.0184*Trp+0.0045*Orn |
| 0.8263 | 0.0440+0.0047*Thr+0.0001*Ala−0.0122*ABA+0.0108*Met+0.0065*Tyr−0.0145*Trp |
| 0.8263 | 0.0748+0.0053*Thr−0.0123*ABA−0.0022*Val+0.0080*Ile+0.0077*Tyr−0.0125*Trp |
| 0.8259 | −0.2859+0.0028*Tau+0.0045*Ser−0.0120*ABA+0.0206*Met+0.0060*Tyr−0.0149*Trp |
| 0.8259 | 0.1832−0.0105*ABA−0.0028*Val+0.0180*Met+0.0075*Ile+0.0076*Tyr−0.0082*Trp |
| 0.8254 | 0.0469+0.0048*Thr−0.0122*ABA+0.0110*Met+0.0067*Tyr−0.0142*Trp |
| 0.8254 | 0.0361+0.0047*Thr+0.0006*Asn−0.0121*ABA+0.0111*Met+0.0066*Tyr−0.0143*Trp |
| 0.8254 | 0.0099+0.0023*Tau+0.0032*Thr+0.0210*Met+0.0065*Tyr−0.0061*Phe−0.0170*Trp |
| 0.8254 | −0.0105+0.0038*Thr−0.0121*ABA+0.0117*Met+0.0063*Tyr−0.0155*Trp+0.0041*Orn |
| 0.8249 | 0.1554+0.0029*Tau−0.0073*ABA+0.0276*Met−0.0055*Phe−0.0172*Trp+0.0085*Orn |
| 0.8249 | 0.0436+0.0028*Tau+0.0249*Met+0.0072*Ile+0.0062*Tyr−0.0099*Phe−0.0169*Trp |

# FIG.156

| ROC AUC | FORMULA |
|---|---|
| 0.8249 | 0.0709–0.0032*Val+0.0183*Met+0.0077*Ile+0.0067*Tyr+0.0005*His–0.0089*Trp |
| 0.8249 | 0.1084+0.0008*Gly–0.0103*ABA+0.0205*Met+0.0071*Tyr–0.0034*His–0.0090*Trp |
| 0.8249 | 0.2136+0.0004*Ala–0.0112*ABA+0.0227*Met+0.0065*Tyr–0.0028*His–0.0104*Trp |
| 0.8245 | –0.1161+0.0026*Tau+0.0024*Thr+0.0036*Ser–0.0099*ABA+0.0178*Met–0.0151*Trp |
| 0.8245 | 0.0970+0.0028*Tau+0.0047*Thr–0.0102*ABA–0.0014*Val+0.0106*Ile–0.0152*Trp |
| 0.8245 | –0.0613+0.0029*Tau+0.0049*Thr+0.0083*Cit–0.0117*ABA+0.0084*Ile–0.0199*Trp |
| 0.8245 | –0.2034+0.0030*Tau+0.0037*Thr+0.0034*Ser–0.0117*ABA+0.0087*Ile–0.0173*Trp |
| 0.8240 | 0.0420+0.0031*Tau–0.0026*Val+0.0169*Met+0.0104*Ile+0.0013*His–0.0126*Trp |
| 0.8240 | –0.1241+0.0027*Tau+0.0047*Thr+0.0085*Ile+0.0058*Tyr–0.0046*Phe–0.0193*Trp |
| 0.8240 | 0.0473+0.0029*Tau+0.0049*Thr–0.0010*Glu–0.0104*ABA+0.0086*Ile–0.0171*Trp |
| 0.8240 | –0.0243+0.0028*Tau+0.0044*Thr+0.0004*Gly–0.0105*ABA+0.0082*Ile–0.0167*Trp |
| 0.8235 | 0.0084+0.0046*Thr+0.0003*Gly–0.0118*ABA+0.0104*Met+0.0067*Tyr–0.0140*Trp |
| 0.8235 | 0.1633+0.0029*Tau+0.0250*Met+0.0078*Ile–0.0077*Phe–0.0003*His–0.0155*Trp |
| 0.8235 | –0.0765+0.0029*Tau+0.0183*Met+0.0050*Ile+0.0048*Tyr–0.0022*His–0.0147*Trp |
| 0.8235 | 0.1399+0.0121*Cit–0.0124*ABA+0.0252*Met+0.0076*Tyr–0.0052*His–0.0123*Trp |
| 0.8235 | 0.1284+0.0028*Tau+0.0016*Glu+0.0263*Met+0.0072*Ile–0.0080*Phe–0.0158*Trp |
| 0.8235 | 0.1874+0.0005*Ala–0.0105*ABA–0.0012*Val+0.0230*Met+0.0067*Tyr–0.0104*Trp |
| 0.8235 | 0.1553+0.0010*Pro–0.0091*ABA+0.0228*Met+0.0062*Tyr–0.0030*His–0.0099*Trp |
| 0.8231 | 0.0722+0.0031*Tau–0.0022*Val+0.0176*Met+0.0097*Ile–0.0123*Trp |
| 0.8231 | 0.0436+0.0029*Tau+0.0019*Glu–0.0025*Val+0.0189*Met+0.0092*Ile–0.0121*Trp |
| 0.8231 | 0.0638+0.0057*Thr–0.0132*ABA+0.0047*Ile+0.0070*Tyr–0.0019*His–0.0143*Trp |
| 0.8226 | 0.0159+0.0046*Thr+0.0004*Pro–0.0113*ABA+0.0109*Met+0.0063*Tyr–0.0144*Trp |
| 0.8226 | –0.1114+0.0024*Tau+0.0037*Thr–0.0004*Val+0.0143*Met+0.0056*Tyr–0.0163*Trp |
| 0.8226 | 0.0792+0.0028*Tau+0.0049*Thr–0.0105*ABA+0.0091*Ile–0.0017*Phe–0.0172*Trp |
| 0.8221 | 0.0876+0.0027*Tau–0.0106*ABA–0.0005*Val+0.0236*Met+0.0067*Tyr–0.0128*Trp |
| 0.8221 | 0.0133+0.0030*Tau+0.0021*Asn–0.0021*Val+0.0178*Met+0.0091*Ile–0.0129*Trp |
| 0.8221 | –0.3399+0.0028*Tau+0.0041*Ser+0.0214*Met+0.0051*Tyr–0.0016*His–0.0151*Trp |
| 0.8221 | –0.0329+0.0006*Gly–0.0026*Val+0.0159*Met+0.0069*Ile+0.0065*Tyr–0.0092*Trp |
| 0.8221 | 0.3669–0.0091*ABA+0.0317*Met+0.0084*Tyr–0.0080*Phe–0.0023*His–0.0105*Trp |
| 0.8221 | 0.0988+0.0065*Thr–0.0099*Cit–0.0140*ABA+0.0087*Tyr–0.0040*His–0.0157*Trp |
| 0.8217 | 0.0827+0.0029*Tau+0.0285*Met–0.0065*Phe–0.0181*Trp+0.0084*Orn |
| 0.8217 | 0.1377–0.0010*Val+0.0249*Met+0.0065*Tyr–0.0020*His–0.0095*Trp |
| 0.8217 | 0.0732+0.0026*Asn–0.0008*Val+0.0247*Met+0.0058*Tyr–0.0019*His–0.0102*Trp |
| 0.8217 | –0.0073–0.0028*Val+0.0183*Met+0.0049*Ile+0.0063*Tyr–0.0115*Trp+0.0058*Orn |
| 0.8217 | –0.2594+0.0031*Tau+0.0045*Ser–0.0128*ABA+0.0074*Tyr–0.0173*Trp+0.0083*Orn |
| 0.8212 | –0.0019+0.0048*Thr–0.0127*ABA+0.0069*Met–0.0035*Ile+0.0062*Tyr–0.0149*Trp |
| 0.8212 | –0.0658+0.0028*Tau+0.0037*Thr+0.0098*Met+0.0059*Ile–0.0012*His–0.0168*Trp |
| 0.8212 | –0.3627+0.0029*Tau+0.0023*Thr+0.0034*Ser+0.0109*Met+0.0061*Ile–0.0178*Trp |
| 0.8212 | 0.0392+0.0027*Tau–0.0004*Val+0.0243*Met+0.0060*Tyr–0.0025*His–0.0126*Trp |
| 0.8212 | 0.0824–0.0031*Val+0.0185*Met+0.0074*Ile+0.0067*Tyr–0.0088*Trp |
| 0.8212 | 0.0914+0.0023*Tau+0.0035*Thr+0.0027*Glu–0.0107*ABA+0.0177*Met–0.0150*Trp |
| 0.8212 | –0.3670+0.0026*Tau+0.0024*Thr+0.0032*Ser+0.0153*Met+0.0049*Tyr–0.0172*Trp |
| 0.8212 | 0.0717+0.0005*Asn–0.0030*Val+0.0186*Met+0.0073*Ile+0.0066*Tyr–0.0090*Trp |
| 0.8212 | 0.1790–0.0114*ABA+0.0206*Met+0.0029*Ile+0.0066*Tyr–0.0025*His–0.0102*Trp |
| 0.8207 | –0.0205+0.0028*Tau+0.0034*Thr–0.0018*Val+0.0092*Met+0.0089*Ile–0.0150*Trp |
| 0.8207 | –0.2584+0.0031*Tau+0.0039*Ser–0.0100*ABA+0.0201*Met–0.0175*Trp+0.0072*Orn |
| 0.8207 | –0.4832+0.0030*Tau+0.0035*Ser+0.0181*Met+0.0041*Tyr–0.0192*Trp+0.0067*Orn |
| 0.8207 | 0.0545+0.0023*Tau+0.0031*Glu+0.0324*Met+0.0064*Tyr–0.0086*Phe–0.0156*Trp |
| 0.8203 | 0.0867+0.0105*Cit–0.0111*ABA–0.0016*Val+0.0256*Met+0.0078*Tyr–0.0122*Trp |
| 0.8198 | –0.2421+0.0030*Tau+0.0030*Ser+0.0234*Met–0.0030*Phe–0.0185*Trp+0.0074*Orn |

# FIG.157

| ROC AUC | FORMULA |
|---|---|
| 0.8273 | 0.25735+0.01480*Tau+0.12364*Met+0.03730*Tyr-0.02666*His-0.08751*Trp-0.00691*Lys |
| 0.8226 | -1.99126+0.01827*Tau+0.02423*Thr+0.07469*Ile+0.03091*Tyr-0.12868*Trp-0.01816*Lys |
| 0.8212 | -2.52197+0.02086*Tau+0.01729*Ser+0.07973*Met+0.07952*Ile-0.11300*Trp-0.01842*Lys |
| 0.8212 | 0.07001+0.01468*Tau+0.12667*Met+0.03626*Tyr-0.01910*Phe-0.02231*His-0.09459*Trp |
| 0.8207 | -0.03388+0.00846*Asn+0.10492*Met+0.03095*Tyr-0.01766*His-0.05777*Trp-0.01227*Arg |
| 0.8198 | -1.46573+0.01926*Tau+0.08698*Met+0.07454*Ile+0.02747*Tyr-0.10644*Trp-0.02166*Lys |
| 0.8189 | 0.23765+0.10349*Met+0.03360*Tyr-0.01909*His-0.05575*Trp-0.01208*Arg |
| 0.8179 | 0.21771+0.01516*Tau+0.11948*Met+0.03236*Tyr-0.02217*His-0.08568*Trp-0.01423*Arg |
| 0.8156 | -1.21976+0.01904*Tau+0.01757*Thr+0.04513*Met+0.07450*Ile-0.12041*Trp-0.01742*Lys |
| 0.8142 | -0.43952+0.01976*Tau+0.10650*Met+0.08056*Ile-0.02436*Phe-0.10444*Trp-0.01749*Lys |
| 0.8133 | -1.11163+0.01897*Tau+0.02167*Thr-0.00407*Val+0.08438*Ile-0.11894*Trp-0.01387*Lys |
| 0.8128 | -2.31189+0.02054*Tau+0.01798*Thr+0.01160*Ser+0.07893*Ile-0.12033*Trp-0.03055*Arg |
| 0.8128 | -1.63673+0.01795*Tau-0.01423*Val+0.05590*Met+0.06817*Ile+0.02860*Tyr-0.10126*Trp |
| 0.8128 | -1.26107+0.01886*Tau+0.02250*Thr+0.07835*Ile-0.12373*Trp-0.01460*Lys |
| 0.8128 | -1.24963+0.01884*Tau+0.02234*Thr+0.07945*Ile-0.00097*Leu-0.12321*Trp-0.01450*Lys |
| 0.8123 | -0.82935+0.01899*Tau+0.02246*Thr+0.07815*Ile-0.01090*His-0.11946*Trp-0.01402*Lys |
| 0.8119 | -2.01127+0.01978*Tau+0.02434*Thr+0.04307*Cit+0.08217*Ile-0.13817*Trp-0.01683*Lys |
| 0.8114 | -1.06448+0.02003*Tau+0.02224*Thr+0.08660*Ile-0.11583*Trp-0.00832*Lys-0.02584*Arg |
| 0.8105 | -1.50626+0.01801*Tau+0.03431*Thr+0.06390*Cit+0.02992*Leu-0.14246*Trp-0.03557*Arg |
| 0.8100 | -1.87021+0.01892*Tau+0.02238*Thr+0.06843*Ile+0.02043*Tyr-0.12265*Trp-0.02893*Arg |
| 0.8086 | -2.79184+0.02254*Tau+0.02534*Thr+0.09523*Cit+0.09054*Ile-0.14355*Trp-0.05033*Arg |
| 0.8086 | -1.24137+0.01962*Tau+0.02151*Thr-0.00172*Asn+0.07570*Ile-0.11768*Trp-0.02999*Arg |
| 0.8086 | -1.30997+0.01963*Tau+0.01735*Thr+0.03594*Met+0.06983*Ile-0.11562*Trp-0.03148*Arg |
| 0.8077 | -1.26616+0.01959*Tau+0.02135*Thr-0.00065*Val+0.07650*Ile-0.11720*Trp-0.02965*Arg |
| 0.8077 | -1.29435+0.01958*Tau+0.02145*Thr+0.07546*Ile+0.00026*Phe-0.11788*Trp-0.02995*Arg |
| 0.8077 | -1.32639+0.01964*Tau+0.02194*Thr+0.07203*Ile+0.00303*Leu-0.11943*Trp-0.03052*Arg |
| 0.8077 | -0.63792+0.01985*Tau-0.00650*Val+0.08039*Met+0.08698*Ile-0.09712*Trp-0.01716*Lys |
| 0.8072 | -1.28965+0.01958*Tau+0.02148*Thr+0.07554*Ile-0.11790*Trp-0.02992*Arg |
| 0.8072 | -1.29125+0.01957*Tau+0.02147*Thr+0.00002*Ala+0.07550*Ile-0.11793*Trp-0.02991*Arg |
| 0.8067 | -1.80051+0.01757*Tau+0.01798*Thr+0.00871*Pro-0.00960*Val+0.06331*Ile-0.12082*Trp |
| 0.8063 | -1.60590+0.01844*Tau+0.02148*Thr+0.00576*Pro+0.07082*Ile-0.12610*Trp-0.01308*Lys |
| 0.8058 | -2.73421+0.02159*Tau+0.01852*Ser+0.06941*Met+0.07496*Ile-0.10865*Trp-0.03371*Arg |
| 0.8053 | -1.65796+0.01922*Tau+0.02038*Thr+0.00732*Pro+0.06933*Ile-0.11998*Trp-0.03007*Arg |
| 0.8053 | -1.39681+0.01955*Tau+0.02048*Thr+0.00080*Gly+0.07471*Ile-0.11731*Trp-0.02929*Arg |
| 0.8053 | -1.13209+0.01789*Tau+0.06903*Met+0.04414*Ile+0.02361*Tyr-0.02332*His-0.10675*Trp |
| 0.8049 | -1.12157+0.01960*Tau+0.02147*Thr+0.07525*Ile-0.00437*His-0.11642*Trp-0.02910*Arg |
| 0.8049 | -1.55435+0.01667*Tau+0.07888*Cit+0.14073*Met+0.03208*Tyr-0.10894*Trp-0.03287*Arg |
| 0.8049 | -1.36186+0.01956*Tau+0.00340*Gly+0.06658*Met+0.07522*Ile-0.10396*Trp-0.01700*Lys |
| 0.8044 | 1.38097+0.01991*Tau+0.13558*Met+0.04421*Leu-0.05961*His-0.09527*Trp-0.01718*Lys |
| 0.8030 | 0.35631+0.01898*Tau+0.03064*Thr+0.04458*Leu-0.04449*His-0.12209*Trp-0.02477*Arg |
| 0.8025 | -0.17891+0.01993*Tau+0.09007*Met+0.07630*Ile-0.01726*His-0.09708*Trp-0.01798*Lys |
| 0.8021 | -2.14836+0.01809*Tau+0.00457*Gly-0.01147*Val+0.07323*Ile+0.02649*Tyr-0.10061*Trp |
| 0.8016 | -0.80367+0.01960*Tau+0.08106*Met+0.08346*Ile-0.00527*Leu-0.10146*Trp-0.01779*Lys |
| 0.8007 | 0.87131+0.02010*Tau+0.11924*Met+0.03965*Leu-0.04839*His-0.09390*Trp-0.02872*Arg |
| 0.8007 | -0.63898+0.02030*Tau+0.09119*Met+0.07466*Ile-0.01881*Phe-0.09985*Trp-0.03130*Arg |
| 0.7983 | -1.38088+0.01983*Tau+0.07241*Met+0.06735*Ile+0.01569*Tyr-0.10174*Trp-0.03189*Arg |
| 0.7983 | -0.65259+0.02095*Tau+0.08629*Met+0.08578*Ile-0.09554*Trp-0.01198*Lys-0.02742*Arg |
| 0.7983 | -2.13554+0.01905*Tau+0.00560*Gly+0.07909*Ile+0.02445*Tyr-0.10444*Trp-0.01465*Lys |
| 0.7969 | -0.85034+0.02033*Tau-0.00331*Val+0.07178*Met+0.07702*Ile-0.09592*Trp-0.03129*Arg |
| 0.7960 | -0.73484+0.01497*Tau+0.00891*Pro+0.10601*Met+0.02012*Tyr-0.09405*Trp-0.01854*Arg |

# FIG.158

| ROC AUC | FORMULA |
|---|---|
| 0.7955 | −0.59528+0.02033*Tau+0.07647*Met+0.07080*Ile−0.00976*His−0.09582*Trp−0.03104*Arg |
| 0.7941 | −0.95767+0.02030*Tau+0.07315*Met+0.07220*Ile−0.09891*Trp−0.03271*Arg |
| 0.7937 | −0.96539+0.02028*Tau+0.00009*Ala+0.07296*Met+0.07205*Ile−0.09907*Trp−0.03268*Arg |
| 0.7932 | −0.94122+0.02025*Tau+0.07242*Met+0.07424*Ile−0.00176*Leu−0.09827*Trp−0.03233*Arg |
| 0.7927 | −1.16118+0.02009*Tau+0.00691*Asn+0.07454*Met+0.07114*Ile−0.10007*Trp−0.03247*Arg |
| 0.7923 | −1.82206+0.01810*Tau+0.00658*Gly+0.05421*Ile+0.02339*Tyr−0.02393*His−0.10279*Trp |
| 0.7908 | −2.51649+0.02351*Tau+0.10006*Cit+0.09826*Met+0.08472*Ile−0.12297*Trp−0.05521*Arg |
| 0.7908 | −2.38045+0.02254*Tau+0.02213*Ser−0.01521*Asn+0.09302*Ile−0.10392*Trp−0.03133*Arg |
| 0.7904 | −2.75857+0.02146*Tau+0.01665*Ser+0.00277*Gly+0.08577*Ile−0.10507*Trp−0.02827*Arg |
| 0.7885 | −2.63503+0.02201*Tau+0.02008*Ser+0.08903*Ile+0.00204*Leu−0.10586*Trp−0.03095*Arg |
| 0.7876 | −2.57525+0.02192*Tau+0.01963*Ser+0.09115*Ile−0.10493*Trp−0.03052*Arg |
| 0.7876 | −2.57667+0.02191*Tau+0.01960*Ser+0.00004*Ala+0.09105*Ile−0.10501*Trp−0.03052*Arg |
| 0.7871 | −2.84788+0.02196*Tau+0.02050*Ser+0.09156*Ile+0.00491*His−0.10706*Trp−0.03150*Arg |
| 0.7866 | −1.34525+0.01990*Tau+0.00749*Pro+0.06762*Met+0.06642*Ile−0.10191*Trp−0.03263*Arg |
| 0.7866 | −2.40150+0.02227*Tau+0.01964*Ser+0.09932*Ile−0.10288*Trp−0.00588*Lys−0.02762*Arg |
| 0.7862 | −1.28494+0.02013*Tau+0.00216*Gly+0.06267*Met+0.07067*Ile−0.09961*Trp−0.03060*Arg |
| 0.7857 | −2.89891+0.02145*Tau+0.01900*Ser+0.08656*Ile+0.01414*Tyr−0.10720*Trp−0.02978*Arg |
| 0.7857 | −2.70947+0.02197*Tau+0.02045*Ser+0.00178*Val+0.08881*Ile−0.10694*Trp−0.03130*Arg |
| 0.7848 | −3.88626+0.02219*Tau+0.02546*Ser+0.07896*Ile+0.03622*Phe−0.10843*Trp−0.03523*Arg |
| 0.7843 | −1.84749+0.01943*Tau+0.00570*Gly+0.07793*Ile+0.01293*Phe−0.10201*Trp−0.01337*Lys |
| 0.7838 | −0.92501+0.01922*Tau+0.00942*Pro−0.01195*Val+0.09241*Ile−0.09420*Trp−0.00805*Lys |
| 0.7834 | −4.22845+0.02511*Tau+0.02329*Ser+0.08891*Cit+0.10782*Ile−0.12690*Trp−0.04970*Arg |
| 0.7824 | −2.58509+0.02137*Tau+0.01634*Ser+0.00600*Pro+0.08515*Ile−0.10585*Trp−0.03046*Arg |
| 0.7815 | −1.88572+0.01966*Tau+0.00451*Gly+0.07569*Ile+0.01622*Tyr−0.10017*Trp−0.02510*Arg |
| 0.7764 | −1.34083+0.02018*Tau+0.00434*Gly−0.00244*Val+0.08451*Ile−0.09494*Trp−0.02504*Arg |
| 0.7754 | −1.12469+0.02015*Tau+0.00505*Gly+0.07920*Ile−0.01070*His−0.09391*Trp−0.02351*Arg |
| 0.7750 | −1.39997+0.02002*Tau+0.00446*Gly+0.08591*Ile−0.00464*Leu−0.09561*Trp−0.02504*Arg |
| 0.7708 | −0.95570+0.02009*Tau+0.00986*Pro−0.00841*Val+0.09129*Ile−0.09139*Trp−0.02609*Arg |
| 0.7694 | −1.63920+0.02334*Tau+0.08200*Cit−0.00765*Val+0.11458*Ile−0.10609*Trp−0.04364*Arg |
| 0.7694 | −1.55186+0.01992*Tau+0.00657*Pro+0.00303*Gly+0.07740*Ile−0.09920*Trp−0.02718*Arg |
| 0.7689 | −1.13315+0.01963*Tau+0.01027*Pro+0.09381*Ile−0.01352*Leu−0.09444*Trp−0.02688*Arg |
| 0.7680 | −2.25578+0.02263*Tau+0.00316*Gly+0.07126*Cit+0.09642*Ile−0.11236*Trp−0.04211*Arg |
| 0.7680 | −1.72678+0.02010*Tau+0.00444*Gly+0.07599*Ile+0.01424*Phe−0.09728*Trp−0.02767*Arg |
| 0.7661 | −1.79119+0.02298*Tau+0.08192*Cit+0.11552*Ile−0.01071*Leu−0.10920*Trp−0.04463*Arg |
| 0.7633 | −1.15007+0.02009*Tau+0.00867*Pro+0.08097*Ile−0.09824*Trp−0.02949*Arg |
| 0.7633 | −2.36613+0.02266*Tau+0.08053*Cit+0.09787*Ile+0.01886*Tyr−0.11593*Trp−0.04550*Arg |
| 0.7633 | −1.65166+0.02346*Tau+0.07783*Cit+0.11102*Ile−0.10994*Trp−0.00548*Lys−0.04316*Arg |
| 0.7628 | −1.02681+0.02032*Tau+0.00830*Pro+0.08615*Ile−0.09686*Trp−0.00348*Lys−0.02777*Arg |
| 0.7628 | −1.81967+0.02315*Tau+0.07820*Cit+0.10348*Ile−0.11194*Trp−0.04594*Arg |
| 0.7619 | −1.04144+0.02020*Tau−0.00411*Asn+0.00883*Pro+0.08125*Ile−0.09767*Trp−0.02967*Arg |
| 0.7619 | −0.79479+0.02010*Tau+0.00941*Pro+0.07960*Ile−0.01029*His−0.09509*Trp−0.02759*Arg |
| 0.7614 | −1.86556+0.02301*Tau+0.00057*Ala+0.07759*Cit+0.10212*Ile−0.11294*Trp−0.04572*Arg |
| 0.7610 | −1.37497+0.01984*Tau+0.00777*Pro+0.07859*Ile+0.01009*Tyr−0.09969*Trp−0.02897*Arg |
| 0.7605 | −0.86155+0.02095*Tau+0.09271*Ile+0.01995*Phe−0.09188*Trp−0.00763*Lys−0.02790*Arg |
| 0.7600 | −1.41525+0.02338*Tau+0.08343*Cit+0.10362*Ile−0.01251*His−0.10889*Trp−0.04472*Arg |
| 0.7586 | −1.08496+0.02030*Tau+0.00971*Pro−0.00126*Ala+0.08276*Ile−0.09617*Trp−0.02971*Arg |
| 0.7577 | −1.57072+0.02359*Tau−0.01062*Asn+0.08201*Cit+0.10530*Ile−0.11114*Trp−0.04721*Arg |
| 0.7572 | −2.23943+0.02264*Tau+0.00820*Pro+0.07630*Cit+0.09540*Ile−0.11518*Trp−0.04574*Arg |
| 0.7530 | −1.54803+0.02000*Tau+0.00899*Pro+0.07416*Ile+0.01843*Phe−0.09856*Trp−0.03170*Arg |
| 0.7521 | −2.48170+0.02330*Tau+0.08581*Cit+0.09545*Ile+0.02670*Phe−0.11391*Trp−0.05075*Arg |

# FIG.159

# FIG.160

| ROC AUC | FORMULA |
|---|---|
| 0.8426 | -3.5145+0.0229*Tau-0.0715*ABA+0.1245*Met+0.0271*Tyr-0.1170*Trp+0.0521*Orn |
| 0.8422 | -4.6698+0.0222*Tau-0.0214*Ser-0.0680*ABA+0.1382*Met-0.1170*Trp+0.0490*Orn |
| 0.8407 | -3.0967+0.0198*Tau+0.1758*Met+0.0380*Tyr-0.0579*Phe-0.1286*Trp+0.0533*Orn |
| 0.8363 | -3.0476+0.0223*Tau-0.0194*Val+0.0936*Met+0.0510*Ile+0.0364*Tyr-0.0690*Trp |
| 0.8363 | -4.7884+0.0206*Tau+0.0273*Ser-0.0707*ABA+0.1214*Met+0.0347*Tyr-0.0933*Trp |
| 0.8358 | -1.8045+0.0204*Tau-0.0083*Val-0.1360*Met+0.0490*Ile-0.0324*Phe-0.0787*Trp |
| 0.8333 | -3.1937+0.0209*Tau+0.0112*Thr-0.0631*ABA+0.1287*Met-0.1160*Trp+0.0463*Orn |
| 0.8333 | -2.9897+0.0152*Tau+0.0341*Thr+0.0588*Cit-0.0813*ABA+0.0436*Tyr-0.1250*Trp |
| 0.8328 | -2.5808+0.0214*Tau+0.0052*Asn-0.0125*Val+0.1078*Met+0.0473*Ile-0.0707*Trp |
| 0.8324 | -1.1575-0.0534*ABA-0.0046*Val+0.1343*Met+0.0409*Tyr-0.0142*His-0.0466*Trp |
| 0.8319 | -1.6785+0.0196*Tau-0.0528*ABA+0.1715*Met+0.0442*Tyr-0.0410*Phe-0.0899*Trp |
| 0.8319 | -2.4940+0.0222*Tau-0.0542*ABA+0.1821*Met-0.0294*Phe-0.1170*Trp+0.0571*Orn |
| 0.8319 | -2.4619+0.0194*Tau+0.1433*Met+0.0380*Ile+0.0398*Tyr-0.0589*Phe-0.1045*Trp |
| 0.8314 | -1.5417-0.0535*ABA-0.0158*Val+0.0991*Met+0.0375*Ile+0.0414*Tyr-0.0427*Trp |
| 0.8314 | -3.9161+0.0185*Tau+0.0221*Thr+0.0200*Ser-0.0670*ABA+0.0465*Ile-0.1049*Trp |
| 0.8309 | -1.7687+0.0134*Asn-0.0504*ABA-0.0060*Val+0.1294*Met+0.0360*Tyr-0.0537*Trp |
| 0.8309 | -2.7201+0.0219*Tau-0.0165*Val+0.1034*Met+0.0555*Ile+0.0104*His-0.0703*Trp |
| 0.8309 | -4.3685+0.0202*Tau+0.0157*Ser+0.1583*Met-0.0225*Phe-0.1200*Trp+0.0480*Orn |
| 0.8304 | -3.2261+0.0177*Tau+0.0273*Thr-0.0718*ABA+0.0374*Ile+0.0346*Tyr-0.1090*Trp |
| 0.8304 | -3.7622+0.0219*Tau-0.0090*Val+0.1317*Met+0.0284*Tyr-0.1039*Trp+0.0477*Orn |
| 0.8304 | -5.8234+0.0206*Tau+0.0214*Ser+0.1118*Met+0.0234*Tyr-0.1238*Trp+0.0416*Orn |
| 0.8299 | -1.4572-0.0506*ABA-0.0065*Val+0.1308*Met+0.0393*Tyr-0.0506*Trp |
| 0.8299 | -2.9650+0.0175*Tau+0.0291*Thr+0.0520*Cit-0.0672*ABA+0.0457*Ile-0.1250*Trp |
| 0.8299 | -2.4684+0.0215*Tau-0.0130*Val+0.1086*Met+0.0490*Ile-0.0690*Trp |
| 0.8289 | -1.6106+0.0125*Asn-0.0550*ABA+0.1275*Met+0.0352*Tyr-0.0158*His-0.0537*Trp |
| 0.8289 | -4.9714+0.0199*Tau+0.0238*Ser+0.1219*Met+0.0291*Tyr-0.0071*His-0.0935*Trp |
| 0.8284 | -2.5614+0.0207*Tau+0.0055*Glu-0.0133*Val+0.1116*Met+0.0475*Ile-0.0687*Trp |
| 0.8284 | -2.0348+0.0022*Asn-0.0163*Val+0.1001*Met+0.0359*Ile+0.0356*Tyr-0.0474*Trp |
| 0.8279 | -2.8785+0.0187*Tau+0.0240*Thr-0.0629*ABA+0.0403*Ile-0.1148*Trp+0.0294*Orn |
| 0.8279 | -2.7688+0.0203*Tau+0.1858*Met-0.0392*Phe-0.1185*Trp+0.0540*Orn |
| 0.8275 | -1.3114-0.0556*ABA+0.1288*Met+0.0383*Tyr-0.0171*His-0.0510*Trp |
| 0.8275 | -1.9874-0.0166*Val+0.0997*Met+0.0366*Ile+0.0362*Tyr-0.0467*Trp |
| 0.8270 | -2.9023+0.0175*Tau+0.0228*Thr-0.0658*ABA+0.0800*Met+0.0328*Tyr-0.0984*Trp |
| 0.8270 | -2.0164-0.0169*Val+0.0988*Met+0.0373*Ile+0.0361*Tyr+0.0012*His-0.0469*Trp |
| 0.8265 | -3.7962+0.0191*Tau+0.0151*Thr+0.0209*Ser-0.0617*ABA+0.1130*Met-0.0946*Trp |
| 0.8265 | -2.0011+0.0186*Tau+0.0061*Glu+0.1466*Met+0.0367*Ile-0.0412*Phe-0.0913*Trp |
| 0.8265 | -4.3628+0.0206*Tau+0.0255*Ser-0.0881*ABA+0.0441*Tyr-0.1121*Trp+0.0579*Orn |
| 0.8265 | -1.9919+0.0592*Cit-0.0553*ABA-0.0108*Val+0.1430*Met+0.0423*Tyr-0.0674*Trp |
| 0.8255 | -5.0879+0.0184*Tau+0.0137*Thr+0.0196*Ser+0.0894*Met+0.0279*Tyr-0.1060*Trp |
| 0.8250 | -2.8846+0.0171*Tau+0.0255*Thr-0.0732*ABA+0.0396*Tyr-0.1175*Trp+0.0377*Orn |
| 0.8250 | -1.9818+0.0372*Glu-0.0725*ABA-0.0117*Val+0.1484*Met+0.0387*Tyr-0.0511*Trp |
| 0.8245 | -3.7618+0.0160*Tau+0.0285*Thr+0.0185*Ser-0.0753*ABA+0.0415*Tyr-0.1022*Trp |
| 0.8240 | -2.1277-0.0550*ABA-0.0087*Val+0.1266*Met+0.0348*Tyr-0.0678*Trp+0.0387*Orn |
| 0.8240 | -2.2563+0.0187*Tau+0.0291*Thr-0.0570*ABA-0.0090*Val+0.0567*Ile-0.0892*Trp |
| 0.8240 | -1.5388+0.0025*Ala-0.0510*ABA-0.0076*Val+0.1250*Met+0.0365*Tyr-0.0556*Trp |
| 0.8235 | -3.5443+0.0197*Tau+0.0262*Thr-0.0173*Val+0.0567*Ile+0.0373*Tyr-0.0904*Trp |
| 0.8235 | -1.7965+0.0187*Tau+0.1770*Met+0.0419*Tyr-0.0433*Phe-0.0133*His-0.0870*Trp |
| 0.8235 | -1.7760-0.0439*ABA+0.1681*Met+0.0427*Tyr-0.0540*Phe-0.0900*Trp+0.0416*Orn |
| 0.8235 | -1.5370-0.0573*ABA+0.1129*Met+0.0122*Ile+0.0364*Tyr-0.0151*His-0.0532*Trp |
| 0.8235 | -2.2922+0.0168*Tau+0.0113*Glu+0.1789*Met+0.0385*Tyr-0.0483*Phe-0.0939*Trp |

EP 2 124 059 A1

# FIG.161

| ROC AUC | FORMULA |
|---|---|
| 0.8230 | -2.9969+0.0276*Thr+0.0523*Cit-0.0715*ABA+0.0669*Met+0.0361*Tyr-0.1042*Trp |
| 0.8230 | -2.0729+0.0205*Tau-0.0611*ABA+0.1357*Met+0.0383*Tyr-0.0180*His-0.0734*Trp |
| 0.8230 | -2.3776+0.0175*Tau+0.0295*Thr-0.0583*ABA+0.0463*Ile-0.0059*Phe-0.1022*Trp |
| 0.8230 | -2.7142+0.0038*Gly-0.0138*Val+0.0832*Met+0.0347*Ile+0.0357*Tyr-0.0509*Trp |
| 0.8225 | -2.2883+0.0209*Tau-0.0570*ABA-0.0066*Val+0.1388*Met+0.0387*Tyr-0.0720*Trp |
| 0.8221 | -1.4224+0.0018*Ala-0.0563*ABA+0.1229*Met+0.0355*Tyr-0.0167*His-0.0559*Trp |
| 0.8221 | -1.7753+0.0650*Cit-0.0650*ABA+0.1403*Met+0.0407*Tyr-0.0297*His-0.0691*Trp |
| 0.8221 | -3.0369+0.0195*Tau+0.0209*Thr-0.0107*Val+0.0645*Met+0.0444*Ile-0.0882*Trp |
| 0.8216 | -1.8707+0.0196*Tau+0.1425*Met+0.0384*Ile-0.0397*Phe-0.0014*His-0.0902*Trp |
| 0.8211 | -2.0670+0.0047*Gly-0.0533*ABA+0.1105*Met+0.0404*Tyr-0.0188*His-0.0512*Trp |
| 0.8206 | -2.6500+0.0166*Tau+0.0184*Thr+0.1237*Met+0.0371*Tyr-0.0377*Phe-0.1055*Trp |
| 0.8201 | -2.0739+0.0154*Tau+0.0349*Thr-0.0675*ABA+0.0434*Tyr-0.0147*His-0.0925*Trp |
| 0.8201 | -2.8442+0.0176*Tau+0.0273*Thr+0.0020*Gly-0.0575*ABA+0.0428*Ile-0.0998*Trp |
| 0.8201 | -0.6073-0.0464*ABA+0.1742*Met+0.0478*Tyr-0.0435*Phe-0.0135*His-0.0617*Trp |
| 0.8191 | -1.5531+0.0344*Glu-0.0658*ABA+0.1860*Met+0.0438*Tyr-0.0521*Phe-0.0756*Trp |
| 0.8191 | -3.3718+0.0165*Tau+0.0283*Thr+0.0445*Ile+0.0363*Tyr-0.0268*Phe-0.1188*Trp |
| 0.8191 | -4.7860+0.0191*Tau+0.0135*Thr+0.0193*Ser+0.0697*Met+0.0291*Ile-0.1069*Trp |
| 0.8186 | -1.6487+0.0238*Thr-0.0547*ABA+0.1039*Met+0.0433*Tyr-0.0347*Phe-0.0858*Trp |
| 0.8186 | -2.4144+0.0153*Tau+0.0334*Thr-0.0679*ABA+0.0404*Tyr-0.0991*Trp |
| 0.8181 | -2.4539+0.0176*Tau+0.0296*Thr-0.0596*ABA+0.0439*Ile-0.0028*His-0.1000*Trp |
| 0.8181 | -1.9413+0.0125*Asn-0.0057*Val+0.1315*Met+0.0323*Tyr-0.0107*His-0.0541*Trp |
| 0.8181 | -2.5677+0.0202*Tau-0.0056*Val+0.1392*Met+0.0355*Tyr-0.0125*His-0.0725*Trp |
| 0.8176 | -2.3725+0.0159*Tau+0.0338*Thr-0.0044*Glu-0.0658*ABA+0.0407*Tyr-0.0995*Trp |
| 0.8176 | -2.4092+0.0153*Tau+0.0334*Thr-0.0679*ABA+0.0405*Tyr-0.0002*Phe-0.0991*Trp |
| 0.8172 | -1.9213+0.0272*Thr-0.0650*ABA+0.0661*Met+0.0379*Tyr-0.0158*His-0.0739*Trp |
| 0.8172 | -2.4421+0.0152*Tau+0.0333*Thr-0.0682*ABA+0.0006*Val+0.0401*Tyr-0.0999*Trp |
| 0.8167 | -2.4747+0.0154*Tau+0.0325*Thr+0.0009*Ala-0.0675*ABA+0.0393*Tyr-0.1010*Trp |
| 0.8167 | -2.8589+0.0155*Tau+0.0303*Thr+0.0027*Gly-0.0655*ABA+0.0408*Tyr-0.0975*Trp |
| 0.8167 | -2.6409+0.0234*Thr+0.0179*Glu-0.0736*ABA+0.0756*Met+0.0320*Tyr-0.0817*Trp |
| 0.8167 | -3.1413+0.0206*Tau+0.1028*Met+0.0241*Ile+0.0279*Tyr-0.0120*His-0.0875*Trp |
| 0.8157 | -2.4413+0.0153*Tau+0.0334*Thr+0.0013*Asn-0.0678*ABA+0.0402*Tyr-0.0994*Trp |
| 0.8157 | -2.4624+0.0190*Tau+0.0300*Thr-0.0098*Glu-0.0555*ABA+0.0455*Ile-0.1025*Trp |
| 0.8152 | -2.6117+0.0150*Tau+0.0324*Thr+0.0029*Pro-0.0624*ABA+0.0386*Tyr-0.1010*Trp |
| 0.8152 | -1.6391-0.0061*Val+0.1325*Met+0.0357*Tyr-0.0115*His-0.0509*Trp |
| 0.8142 | -2.5459-0.0148*Val+0.1074*Met+0.0211*Ile+0.0322*Tyr-0.0644*Trp+0.0328*Orn |
| 0.8137 | -2.1501+0.0264*Thr-0.0619*ABA-0.0033*Val+0.0672*Met+0.0364*Tyr-0.0769*Trp |
| 0.8132 | -2.1508+0.0298*Thr-0.0650*ABA-0.0123*Val+0.0411*Ile+0.0419*Tyr-0.0707*Trp |
| 0.8132 | -1.8533+0.0062*Pro-0.0450*ABA+0.1222*Met+0.0356*Tyr-0.0169*His-0.0564*Trp |
| 0.8127 | -3.2281+0.0183*Tau+0.0219*Thr+0.0653*Met+0.0283*Ile-0.0056*His-0.1008*Trp |
| 0.8118 | -2.7450+0.0213*Thr-0.0642*ABA+0.0720*Met+0.0307*Tyr-0.0914*Trp+0.0253*Orn |
| 0.8113 | -2.4304+0.0266*Thr+0.0046*Asn-0.0631*ABA+0.0621*Met+0.0337*Tyr-0.0824*Trp |
| 0.8108 | -3.3269+0.0178*Tau+0.0210*Thr-0.0050*Val+0.0867*Met+0.0308*Tyr-0.0944*Trp |
| 0.8108 | -2.4746+0.0168*Tau+0.0220*Thr+0.0083*Glu-0.0595*ABA+0.1097*Met-0.0909*Trp |
| 0.8103 | -2.3371+0.0268*Thr-0.0636*ABA+0.0623*Met+0.0345*Tyr-0.0816*Trp |
| 0.8103 | -2.5923+0.0270*Thr-0.0665*ABA+0.0419*Met+0.0167*Ile+0.0333*Tyr-0.0842*Trp |
| 0.8098 | -2.3473+0.0267*Thr+0.0002*Ala-0.0635*ABA+0.0619*Met+0.0343*Tyr-0.0820*Trp |
| 0.8098 | -2.5610+0.0254*Thr+0.0015*Gly-0.0617*ABA+0.0594*Met+0.0348*Tyr-0.0811*Trp |
| 0.8098 | -2.0737+0.0390*Thr+0.0682*Cit-0.0825*ABA+0.0494*Tyr-0.0287*His-0.0989*Trp |
| 0.8088 | -2.1689+0.0321*Thr-0.0692*ABA+0.0232*Ile+0.0392*Tyr-0.0126*His-0.0806*Trp |
| 0.8054 | -2.6203+0.0256*Thr+0.0036*Pro-0.0567*ABA+0.0619*Met+0.0329*Tyr-0.0842*Trp |

244

# FIG.162

| ROC AUC | FORMULA |
|---|---|
| 0.8324 | -3.00357+0.02526*Tau+0.01342*Ser+0.09542*Met+0.07402*Ile-0.10571*Trp-0.01864*Lys |
| 0.8304 | -2.21406+0.02358*Tau+0.01846*Thr+0.06742*Met+0.06853*Ile-0.11475*Trp-0.01795*Lys |
| 0.8260 | -3.15572+0.02574*Tau+0.03473*Thr+0.09243*Cit+0.08350*Ile-0.14543*Trp-0.05729*Arg |
| 0.8250 | -2.58821+0.02163*Tau+0.02600*Thr+0.03538*Cit+0.07637*Ile-0.13242*Trp-0.01492*Lys |
| 0.8230 | -2.81407+0.02095*Tau+0.02557*Thr+0.07440*Ile+0.03728*Tyr-0.12464*Trp-0.01966*Lys |
| 0.8221 | -0.51840+0.02197*Tau+0.14417*Met+0.04186*Tyr-0.02868*His-0.08798*Trp-0.01021*Lys |
| 0.8191 | -3.37191+0.02671*Tau+0.01771*Ser+0.08944*Met+0.06873*Ile-0.09890*Trp-0.03840*Arg |
| 0.8167 | -1.80756+0.02588*Tau+0.10284*Met+0.07343*Ile-0.10055*Trp-0.01901*Lys |
| 0.8162 | -2.37846+0.02138*Tau+0.02376*Thr+0.00552*Pro+0.06687*Ile-0.12219*Trp-0.01216*Lys |
| 0.8162 | -2.22181+0.02231*Tau+0.02496*Thr+0.00120*Ala+0.07199*Ile-0.12143*Trp-0.01405*Lys |
| 0.8152 | -2.41237+0.02428*Tau+0.03026*Thr+0.00724*Pro+0.06693*Ile-0.11564*Trp-0.04128*Arg |
| 0.8152 | -2.12807+0.02233*Tau+0.02571*Thr+0.07328*Ile-0.11963*Trp-0.01381*Lys |
| 0.8147 | -2.45730+0.02646*Tau+0.02704*Thr+0.04847*Met+0.06605*Ile-0.11037*Trp-0.04244*Arg |
| 0.8147 | -1.94585+0.02324*Tau+0.02539*Thr-0.005671*Val+0.07984*Ile-0.11271*Trp-0.01290*Lys |
| 0.8147 | -2.22321+0.02206*Tau+0.02554*Thr+0.00593*Asn+0.07345*Ile-0.12019*Trp-0.01435*Lys |
| 0.8142 | 0.55485+0.02695*Tau+0.14928*Met+0.04227*Leu-0.05712*His-0.09625*Trp-0.01876*Lys |
| 0.8132 | -2.65500+0.02551*Tau+0.02954*Thr+0.00625*Ser+0.07305*Ile-0.11395*Trp-0.04062*Arg |
| 0.8132 | -3.25505+0.02888*Tau+0.11062*Cit+0.12089*Met+0.07832*Ile-0.12586*Trp-0.05794*Arg |
| 0.8127 | -2.49324+0.02247*Tau+0.02060*Thr+0.00861*Pro-0.01038*Val+0.05876*Ile-0.11619*Trp |
| 0.8118 | -2.15550+0.02598*Tau+0.03231*Thr-0.00205*Val+0.07404*Ile-0.11109*Trp-0.03998*Arg |
| 0.8113 | -0.72401+0.02305*Tau+0.13532*Met+0.03488*Tyr-0.02167*His-0.08370*Trp-0.01982*Arg |
| 0.8108 | -2.00590+0.02554*Tau+0.03337*Thr+0.08117*Ile-0.11136*Trp-0.00698*Lys-0.03795*Arg |
| 0.8108 | -1.65628+0.02267*Tau+0.02618*Thr+0.07212*Ile-0.01143*His-0.11562*Trp-0.01331*Lys |
| 0.8103 | -2.07565+0.02570*Tau+0.03261*Thr+0.07097*Ile-0.00367*His-0.11223*Trp-0.04001*Arg |
| 0.8103 | -2.23667+0.02571*Tau+0.03251*Thr+0.00012*Ala+0.07146*Ile-0.11369*Trp-0.04084*Arg |
| 0.8098 | -2.22645+0.02572*Tau+0.03259*Thr+0.07165*Ile-0.11350*Trp-0.04085*Arg |
| 0.8093 | -2.28600+0.02344*Tau+0.02256*Thr-0.00772*Val+0.06263*Ile-0.11731*Trp |
| 0.8088 | -2.40236+0.02592*Tau+0.03217*Thr+0.07048*Ile+0.00593*Phe-0.11278*Trp-0.04153*Arg |
| 0.8083 | -2.23779+0.02573*Tau+0.03274*Thr+0.07016*Ile+0.00137*Leu-0.11416*Trp-0.04119*Arg |
| 0.8083 | -2.11608+0.02599*Tau+0.03450*Thr-0.00112*Gly+0.07306*Ile-0.11493*Trp-0.04206*Arg |
| 0.8078 | -2.74411+0.02494*Tau+0.03124*Thr+0.06609*Ile+0.02350*Tyr-0.11649*Trp-0.04132*Arg |
| 0.8069 | -2.14044+0.02596*Tau+0.03271*Thr-0.00497*Asn+0.07239*Ile-0.11290*Trp-0.04070*Arg |
| 0.8069 | -4.09469+0.02607*Tau+0.02075*Ser+0.08377*Cit+0.10062*Ile-0.12269*Trp-0.04689*Arg |
| 0.8054 | -1.58860+0.02767*Tau+0.11493*Met+0.08189*Ile-0.08817*Trp-0.01338*Lys-0.03129*Arg |
| 0.8049 | -1.47061+0.02723*Tau+0.10962*Met+0.06886*Ile-0.01848*Phe-0.09594*Trp-0.03363*Arg |
| 0.8044 | -3.03294+0.02169*Tau+0.02230*Thr+0.04424*Ile+0.02157*Tyr-0.13086*Trp |
| 0.8044 | -3.06254+0.02236*Tau+0.02023*Thr+0.00663*Ser+0.05258*Ile-0.12850*Trp |
| 0.8039 | -1.59090+0.02727*Tau+0.09362*Met+0.06546*Ile-0.00687*His-0.09176*Trp-0.03348*Arg |
| 0.8039 | -1.75823+0.02758*Tau-0.00337*Val+0.09052*Met+0.07099*Ile-0.08999*Trp-0.03354*Arg |
| 0.8034 | -2.23086+0.02655*Tau+0.08416*Met+0.06374*Ile+0.01892*Tyr-0.09779*Trp-0.03531*Arg |
| 0.8029 | -2.08267+0.02674*Tau+0.00155*Gly+0.08489*Met+0.06600*Ile-0.09320*Trp-0.03396*Arg |
| 0.8025 | -2.20143+0.02566*Tau+0.00926*Pro+0.08803*Met+0.06122*Ile-0.09707*Trp-0.03755*Arg |
| 0.8015 | -2.48718+0.02254*Tau+0.02233*Thr+0.05972*Ile-0.00683*Leu-0.12387*Trp |
| 0.8015 | -1.89154+0.02728*Tau+0.00023*Ala+0.09152*Met+0.06676*Ile-0.09450*Trp-0.03494*Arg |
| 0.8015 | -1.86615+0.02727*Tau+0.09163*Met+0.06841*Ile-0.00119*Leu-0.09353*Trp-0.03466*Arg |
| 0.8010 | -0.41975+0.02490*Tau+0.03926*Thr+0.04235*Leu-0.04264*His-0.11786*Trp-0.03608*Arg |
| 0.8005 | -3.56893+0.02507*Tau+0.01842*Ser+0.08396*Ile+0.02255*Tyr-0.10313*Trp-0.03259*Arg |
| 0.8005 | -1.83961+0.02737*Tau-0.00194*Asn+0.09253*Met+0.06740*Ile-0.09371*Trp-0.03501*Arg |
| 0.8005 | -2.35815+0.02226*Tau+0.02355*Thr+0.05318*Ile-0.00705*Phe-0.12828*Trp |
| 0.8000 | -1.87601+0.02730*Tau+0.09213*Met+0.06709*Ile-0.09401*Trp-0.03496*Arg |

# FIG.163

| ROC AUC | FORMULA |
|---|---|
| 0.7990 | -2.74619+0.02205*Tau+0.02040*Thr+0.00201*Gly+0.04914*Ile-0.12459*Trp |
| 0.7985 | -3.31782+0.02545*Tau+0.02012*Ser+0.08833*Ile+0.00758*His-0.10218*Trp-0.03392*Arg |
| 0.7980 | -3.02088+0.02554*Tau+0.01968*Ser+0.08367*Ile+0.00377*Leu-0.10080*Trp-0.03297*Arg |
| 0.7975 | -2.42409+0.02268*Tau+0.02332*Thr-0.00683*Asn+0.05179*Ile-0.12692*Trp |
| 0.7971 | -2.87280+0.02408*Tau+0.01460*Ser+0.00731*Pro+0.08127*Ile-0.09952*Trp-0.03249*Arg |
| 0.7966 | -2.65621+0.02662*Tau+0.02259*Ser-0.02279*Asn+0.09014*Ile-0.09910*Trp-0.03257*Arg |
| 0.7966 | -2.54656+0.02240*Tau+0.02288*Thr+0.05097*Ile-0.12777*Trp |
| 0.7956 | -2.75556+0.02540*Tau+0.01859*Ser+0.09306*Ile-0.09735*Trp-0.00419*Lys-0.02986*Arg |
| 0.7951 | -4.84708+0.02655*Tau+0.02684*Ser+0.07480*Ile+0.04318*Phe-0.09838*Trp-0.03874*Arg |
| 0.7951 | -3.07975+0.02526*Tau+0.01987*Ser+0.00245*Val+0.08476*Ile-0.10249*Trp-0.03312*Arg |
| 0.7941 | -2.89121+0.02546*Tau+0.01839*Ser+0.00026*Ala+0.08659*Ile-0.09949*Trp-0.03195*Arg |
| 0.7936 | -2.88412+0.02550*Tau+0.01863*Ser+0.08712*Ile-0.09913*Trp-0.03198*Arg |
| 0.7931 | -2.92241+0.02493*Tau+0.01578*Ser+0.00216*Gly+0.08193*Ile-0.09711*Trp-0.03068*Arg |
| 0.7887 | -1.76107+0.02626*Tau-0.00887*Val+0.08989*Ile+0.02807*Tyr-0.08825*Trp-0.02618*Arg |
| 0.7882 | -3.09377+0.02644*Tau+0.08169*Cit+0.09531*Ile+0.02907*Tyr-0.12231*Trp-0.04496*Arg |
| 0.7873 | -2.55722+0.02593*Tau+0.00352*Gly+0.07423*Cit+0.08999*Ile-0.11413*Trp-0.04096*Arg |
| 0.7873 | -2.53967+0.02278*Tau+0.00542*Gly+0.05836*Ile-0.10691*Trp |
| 0.7858 | -1.89097+0.02796*Tau+0.08424*Cit-0.00903*Val+0.10881*Ile-0.10593*Trp-0.04167*Arg |
| 0.7858 | -1.91009+0.02299*Tau+0.00568*Gly+0.05762*Ile-0.01514*His-0.09960*Trp |
| 0.7833 | -1.50153+0.02464*Tau+0.01048*Pro-0.00673*Val+0.08459*Ile-0.08817*Trp-0.02819*Arg |
| 0.7824 | -1.64181+0.02386*Tau+0.01064*Pro+0.08700*Ile-0.00980*Leu-0.09248*Trp-0.02882*Arg |
| 0.7824 | -2.31580+0.02692*Tau+0.00140*Ala+0.07553*Cit+0.09504*Ile-0.11674*Trp-0.04253*Arg |
| 0.7819 | -2.26091+0.02363*Tau+0.00985*Pro+0.07583*Ile+0.02063*Tyr-0.09953*Trp-0.03264*Arg |
| 0.7814 | -2.17980+0.02705*Tau+0.07632*Cit+0.09770*Ile-0.11375*Trp-0.04278*Arg |
| 0.7804 | -2.65930+0.02490*Tau+0.01008*Pro+0.07782*Cit+0.09193*Ile-0.11782*Trp-0.04541*Arg |
| 0.7799 | -1.63772+0.02476*Tau+0.00454*Gly+0.07555*Ile-0.00643*His-0.08986*Trp-0.02561*Arg |
| 0.7799 | -2.09495+0.02690*Tau+0.07552*Cit+0.10154*Ile-0.11169*Trp-0.00276*Lys-0.04145*Arg |
| 0.7789 | -1.82034+0.02727*Tau+0.08024*Cit+0.09707*Ile-0.01046*His-0.11100*Trp-0.04151*Arg |
| 0.7789 | -2.03584+0.02545*Tau+0.09556*Ile-0.01603*Leu+0.03185*Tyr-0.09392*Trp-0.02617*Arg |
| 0.7784 | -1.40419+0.02460*Tau+0.08577*Ile-0.10209*Trp-0.01037*Lys |
| 0.7765 | -1.48646+0.02436*Tau-0.00945*Asn+0.01078*Pro+0.07923*Ile-0.09527*Trp-0.03160*Arg |
| 0.7760 | -1.50958+0.02412*Tau+0.01025*Pro+0.07737*Ile-0.00513*His-0.09426*Trp-0.02994*Arg |
| 0.7750 | -1.69932+0.02411*Tau+0.01017*Pro+0.07832*Ile-0.09619*Trp-0.03115*Arg |
| 0.7745 | -1.62569+0.02409*Tau+0.01140*Pro-0.00134*Ala+0.08029*Ile-0.09413*Trp-0.03147*Arg |
| 0.7740 | -1.91706+0.02570*Tau+0.08176*Ile+0.02348*Tyr-0.09861*Trp-0.02957*Arg |
| 0.7740 | -1.72987+0.02574*Tau+0.08079*Ile+0.02385*Tyr-0.00524*His-0.09676*Trp-0.02829*Arg |
| 0.7730 | -1.72096+0.02594*Tau-0.01105*Asn+0.08242*Ile+0.02704*Tyr-0.09805*Trp-0.02992*Arg |
| 0.7721 | -1.41942+0.02613*Tau+0.00179*Ala+0.08204*Ile-0.09793*Trp-0.02863*Arg |
| 0.7716 | -1.87655+0.02475*Tau+0.00438*Gly+0.07701*Ile-0.09225*Trp-0.02702*Arg |
| 0.7716 | -0.86657+0.02226*Tau+0.00892*Gly+0.03360*Leu-0.04643*His-0.09187*Trp |
| 0.7701 | -1.78392+0.02488*Tau-0.00477*Asn+0.00451*Gly+0.07737*Ile-0.09155*Trp-0.02700*Arg |
| 0.7701 | -1.27390+0.02503*Tau+0.06827*Ile-0.01223*His-0.10539*Trp |
| 0.7701 | -1.80729+0.02476*Tau+0.06794*Ile-0.11043*Trp |
| 0.7696 | -3.06617+0.02777*Tau+0.08577*Cit+0.09033*Ile+0.03003*Phe-0.11450*Trp-0.04911*Arg |
| 0.7676 | -2.39045+0.02436*Tau+0.01131*Pro+0.07078*Ile+0.02399*Phe-0.09445*Trp-0.03526*Arg |
| 0.7657 | -1.29784+0.02618*Tau+0.00237*Asn+0.08522*Ile-0.09439*Trp-0.02866*Arg |
| 0.7657 | -1.73792+0.02659*Tau+0.07997*Ile+0.01816*Phe-0.09227*Trp-0.03117*Arg |
| 0.7647 | -1.10474+0.02610*Tau+0.09096*Ile-0.09213*Trp-0.00395*Lys-0.02689*Arg |
| 0.7642 | -1.10108+0.02630*Tau+0.08498*Ile-0.00330*His-0.09303*Trp-0.02801*Arg |
| 0.7632 | -1.23554+0.02625*Tau+0.08547*Ile-0.09411*Trp-0.02868*Arg |

# FIG.164

# FIG.165

| ROC AUC | FORMULA |
|---|---|
| 0.8971 | 0.7603+0.0026*Tau+0.0042*Ser+0.0120*Ile-0.0104*His-0.0147*Trp-0.0026*Lys |
| 0.8892 | 0.7605+0.0027*Tau+0.0035*Ser-0.0024*Glu+0.0101*Ile-0.0120*His-0.0163*Trp |
| 0.8885 | 1.1877+0.0025*Tau+0.0017*Gly+0.0083*Leu-0.0198*His-0.0114*Trp-0.0026*Lys |
| 0.8885 | 0.9855+0.0024*Tau+0.0012*Gly+0.0122*Ile-0.0131*His-0.0114*Trp-0.0026*Lys |
| 0.8867 | 1.1659+0.0027*Tau-0.0032*Glu+0.0012*Gly+0.0076*Leu-0.0203*His-0.0140*Trp |
| 0.8849 | 0.6757+0.0026*Tau+0.0036*Ser-0.0019*Val+0.0124*Ile-0.0098*His-0.0153*Trp |
| 0.8838 | 0.7235+0.0026*Tau+0.0041*Ser-0.0054*ABA+0.0093*Ile-0.0114*His-0.0165*Trp |
| 0.8834 | 1.0653+0.0025*Tau-0.0010*Pro+0.0016*Gly+0.0073*Leu-0.0192*His-0.0139*Trp |
| 0.8834 | 0.7443+0.0026*Tau+0.0042*Ser-0.0038*Asn+0.0096*Ile-0.0116*His-0.0163*Trp |
| 0.8831 | 0.6359+0.0026*Tau+0.0045*Ser-0.0011*Pro+0.0104*Ile-0.0104*His-0.0171*Trp |
| 0.8831 | 0.6637+0.0026*Tau+0.0031*Ser+0.0005*Gly+0.0091*Ile-0.0127*His-0.0157*Trp |
| 0.8820 | 1.0022+0.0029*Tau+0.0038*Ser-0.0033*Glu+0.0071*Leu-0.0172*His-0.0169*Trp |
| 0.8820 | 0.8858+0.0026*Tau+0.0048*Ser-0.0008*Gln+0.0101*Ile-0.0106*His-0.0160*Trp |
| 0.8813 | 0.9559+0.0027*Tau+0.0047*Ser-0.0044*Asn+0.0064*Leu-0.0160*His-0.0168*Trp |
| 0.8809 | 1.1158+0.0024*Tau+0.0014*Gly-0.0045*ABA+0.0068*Leu-0.0194*His-0.0138*Trp |
| 0.8806 | 0.6943+0.0026*Tau+0.0037*Ser+0.0093*Ile-0.0117*His-0.0169*Trp |
| 0.8802 | 0.6934+0.0026*Tau+0.0037*Ser+0.0001*Cit+0.0093*Ile-0.0117*His-0.0169*Trp |
| 0.8798 | 1.1411+0.0025*Tau-0.0035*Asn+0.0015*Gly+0.0069*Leu-0.0198*His-0.0134*Trp |
| 0.8798 | 0.9973+0.0027*Tau+0.0046*Ser+0.0073*Leu-0.0156*His-0.0156*Trp-0.0021*Lys |
| 0.8795 | 0.9723+0.0026*Tau-0.0023*Glu+0.0009*Gly+0.0102*Ile-0.0141*His-0.0139*Trp |
| 0.8795 | 1.2336+0.0026*Tau-0.0029*Glu+0.0133*Ile-0.0118*His-0.0136*Trp-0.0021*Lys |
| 0.8791 | 0.8247+0.0026*Tau-0.0030*Ser+0.0009*Gly+0.0063*Leu-0.0178*His-0.0157*Trp |
| 0.8791 | 1.2499+0.0025*Tau-0.0006*Gln+0.0017*Gly+0.0069*Leu-0.0194*His-0.0127*Trp |
| 0.8791 | 0.5330+0.0027*Tau+0.0059*Ser-0.0009*Gln+0.0127*Ile-0.0182*Trp-0.0030*Lys |
| 0.8788 | 1.1286+0.0026*Tau+0.0013*Gly-0.0022*Val+0.0093*Leu-0.0189*His-0.0128*Trp |
| 0.8788 | 0.7725+0.0023*Tau+0.0037*Ser-0.0036*Val+0.0165*Ile-0.0103*His-0.0035*Lys |
| 0.8788 | 0.6893+0.0026*Tau+0.0038*Ser+0.0091*Ile+0.0004*Phe-0.0118*His-0.0170*Trp |
| 0.8780 | 1.0650+0.0024*Tau+0.0013*Gly+0.0001*Ala+0.0065*Leu-0.0194*His-0.0144*Trp |
| 0.8780 | 1.0846+0.0024*Tau+0.0014*Gly-0.0015*Cit+0.0066*Leu-0.0192*His-0.0141*Trp |
| 0.8780 | 0.3131+0.0027*Tau+0.0059*Ser-0.0018*Pro+0.0141*Ile-0.0185*Trp-0.0034*Lys |
| 0.8780 | 0.6872+0.0026*Tau+0.0038*Ser-0.0001*Ala+0.0095*Ile-0.0115*His-0.0169*Trp |
| 0.8773 | 0.6988+0.0026*Tau+0.0038*Ser+0.0096*Ile-0.0114*His-0.0167*Trp-0.0006*Arg |
| 0.8763 | 1.0662+0.0025*Tau+0.0014*Gly+0.0066*Leu-0.0193*His-0.0142*Trp |
| 0.8763 | 1.1178+0.0022*Tau-0.0012*Pro+0.0024*Gly+0.0084*Leu-0.0225*His-0.0038*Lys |
| 0.8763 | 0.9280+0.0021*Tau+0.0014*Gly-0.0021*Val+0.0145*Ile-0.0140*His-0.0035*Lys |
| 0.8759 | 1.0402+0.0025*Tau+0.0013*Gly+0.0064*Leu+0.0013*Phe-0.0195*His-0.0144*Trp |
| 0.8759 | 0.4250+0.0027*Tau+0.0043*Ser-0.0037*Val+0.0181*Ile-0.0147*Trp-0.0030*Lys |
| 0.8755 | 1.0679+0.0025*Tau+0.0014*Gly+0.0066*Leu-0.0193*His-0.0142*Trp-0.0001*Arg |
| 0.8755 | 1.1374+0.0025*Tau-0.0029*Glu+0.0002*Ala+0.0107*Ile-0.0131*His-0.0155*Trp |
| 0.8755 | 0.9757+0.0025*Tau+0.0012*Gly+0.0044*Ile+0.0042*Leu-0.0175*His-0.0147*Trp |
| 0.8748 | 1.3979+0.0027*Tau-0.0039*Glu+0.0004*Ala+0.0073*Leu-0.0189*His-0.0162*Trp |
| 0.8748 | 0.7553+0.0027*Tau+0.0038*Ser+0.0059*Ile+0.0029*Leu-0.0138*His-0.0176*Trp |
| 0.8748 | 0.9126+0.0021*Tau-0.0012*Pro+0.0019*Gly+0.0124*Ile-0.0157*His-0.0039*Lys |
| 0.8745 | 0.6024+0.0028*Tau+0.0052*Ser-0.0012*Gln-0.0043*Val+0.0172*Ile-0.0152*Trp |
| 0.8741 | 1.2547+0.0027*Tau-0.0037*Glu+0.0067*Ile+0.0040*Leu-0.0158*His-0.0162*Trp |
| 0.8741 | 1.1698+0.0026*Tau+0.0038*Val+0.0098*Ile+0.0058*Leu-0.0128*His-0.0143*Trp |
| 0.8737 | 0.9259+0.0026*Tau+0.0046*Ser-0.0068*ABA+0.0062*Leu-0.0157*His-0.0170*Trp |
| 0.8737 | 0.8625+0.0027*Tau+0.0048*Ser-0.0010*Pro+0.0066*Leu-0.0151*His-0.0176*Trp |
| 0.8737 | 0.9533+0.0028*Tau+0.0041*Ser-0.0026*Val+0.0092*Leu-0.0155*His-0.0157*Trp |
| 0.8737 | 1.0461+0.0027*Tau+0.0050*Ser-0.0007*Gln+0.0062*Leu-0.0151*His-0.0165*Trp |

# FIG.166

| ROC AUC | FORMULA |
|---|---|
| 0.8734 | 0.9090+0.0024*Tau+0.0009*Gly-0.0014*Val+0.0118*Ile-0.0124*His-0.0133*Trp |
| 0.8734 | 1.1480+0.0024*Tau-0.0020*Val+0.0157*Ile-0.0094*His-0.0127*Trp-0.0021*Lys |
| 0.8730 | 0.7408+0.0021*Tau+0.0023*Ser+0.0014*Gly+0.0107*Ile-0.0160*His-0.0040*Lys |
| 0.8727 | 0.3805+0.0027*Tau+0.0063*Ser-0.0009*Gln-0.0014*Pro+0.0109*Ile-0.0210*Trp |
| 0.8727 | 0.8929+0.0024*Tau-0.0009*Pro+0.0012*Gly+0.0104*Ile-0.0134*His-0.0138*Trp |
| 0.8720 | 1.1809+0.0023*Tau-0.0003*Ala+0.0119*Ile-0.0118*His-0.0143*Trp-0.0022*Lys |
| 0.8720 | 1.2586+0.0024*Tau+0.0094*Ile+0.0027*Leu-0.0134*His-0.0149*Trp-0.0022*Lys |
| 0.8716 | 1.1978+0.0024*Tau-0.0005*Pro+0.0131*Ile-0.0109*His-0.0143*Trp-0.0021*Lys |
| 0.8712 | 0.9452+0.0024*Tau+0.0010*Gly-0.0031*ABA+0.0095*Ile-0.0140*His-0.0138*Trp |
| 0.8712 | 1.5682+0.0027*Tau-0.0041*Glu+0.0086*Leu-0.0182*His-0.0144*Trp-0.0016*Lys |
| 0.8709 | 0.2108+0.0026*Tau+0.0061*Ser-0.0020*Pro-0.0090*ABA+0.0106*Ile-0.0214*Trp |
| 0.8709 | 0.9175+0.0021*Tau+0.0018*Gly+0.0107*Ile-0.0174*His-0.0044*Lys+0.0017*Arg |
| 0.8705 | 1.1539+0.0026*Tau-0.0030*Glu-0.0005*Pro+0.0117*Ile-0.0123*His-0.0155*Trp |
| 0.8705 | 0.9278+0.0023*Tau+0.0010*Gly-0.0016*Cit+0.0095*Ile-0.0138*His-0.0140*Trp |
| 0.8705 | 1.1776+0.0026*Tau-0.0012*Asn-0.0030*Glu+0.0112*Ile-0.0128*His-0.0152*Trp |
| 0.8705 | 1.1514+0.0021*Tau+0.0021*Gly-0.0042*ABA+0.0076*Leu-0.0227*His-0.0036*Lys |
| 0.8705 | 0.3352+0.0027*Tau+0.0051*Ser-0.0022*Asn+0.0122*Ile-0.0191*Trp-0.0033*Lys |
| 0.8702 | 0.3429+0.0027*Tau+0.0047*Ser-0.0015*Glu+0.0127*Ile-0.0191*Trp-0.0035*Lys |
| 0.8698 | 0.9097+0.0024*Tau+0.0010*Gly+0.0094*Ile-0.0140*His-0.0142*Trp |
| 0.8698 | 0.9130+0.0024*Tau+0.0010*Gly+0.0095*Ile-0.0139*His-0.0141*Trp-0.0002*Arg |
| 0.8694 | 0.9078+0.0024*Tau+0.0010*Gly-0.0000*Ala+0.0095*Ile-0.0140*His-0.0141*Trp |
| 0.8694 | 0.9075+0.0024*Tau+0.0010*Gly+0.0093*Ile+0.0002*Phe-0.0141*His-0.0142*Trp |
| 0.8691 | 1.1493+0.0026*Tau-0.0029*Glu-0.0005*ABA+0.0111*Ile-0.0128*His-0.0154*Trp |
| 0.8691 | 1.3167+0.0022*Tau-0.0008*Gln+0.0024*Gly+0.0077*Leu-0.0223*His-0.0033*Lys |
| 0.8691 | 1.1595+0.0026*Tau-0.0030*Glu-0.0011*Cit+0.0111*Ile-0.0126*His-0.0154*Trp |
| 0.8691 | 1.1076+0.0026*Tau-0.0024*Glu-0.0015*Val+0.0134*Ile-0.0112*His-0.0143*Trp |
| 0.8691 | 0.8965+0.0026*Tau-0.0039*Ser+0.0002*Ala+0.0059*Leu-0.0162*His-0.0175*Trp |
| 0.8687 | 1.5012+0.0027*Tau-0.0017*Asn-0.0041*Glu+0.0078*Leu-0.0184*His-0.0156*Trp |
| 0.8687 | 1.5408+0.0027*Tau-0.0041*Glu-0.0002*Gln+0.0077*Leu-0.0181*His-0.0155*Trp |
| 0.8687 | 1.4699+0.0027*Tau-0.0040*Glu-0.0004*Pro+0.0078*Leu-0.0181*His-0.0159*Trp |
| 0.8687 | 1.1440+0.0026*Tau-0.0030*Glu+0.0111*Ile-0.0128*His-0.0155*Trp |
| 0.8687 | 1.1439+0.0026*Tau-0.0030*Glu+0.0111*Ile+0.0000*Phe-0.0128*His-0.0155*Trp |
| 0.8687 | 1.1552+0.0026*Tau-0.0031*Glu+0.0115*Ile-0.0124*His-0.0151*Trp-0.0009*Arg |
| 0.8687 | 1.0815+0.0021*Tau+0.0021*Gly+0.0073*Leu+0.0016*Phe-0.0230*His-0.0039*Lys |
| 0.8687 | 0.3281+0.0026*Tau+0.0048*Ser+0.0125*Ile-0.0008*Phe-0.0192*Trp-0.0034*Lys |
| 0.8684 | 1.4875+0.0028*Tau-0.0036*Glu-0.0021*Val+0.0100*Leu-0.0178*His-0.0145*Trp |
| 0.8684 | 0.1928+0.0027*Tau+0.0058*Ser-0.0037*Asn-0.0018*Pro+0.0106*Ile-0.0218*Trp |
| 0.8684 | 0.5291+0.0026*Tau+0.0064*Ser-0.0012*Gln-0.0088*ABA+0.0098*Ile-0.0201*Trp |
| 0.8684 | 0.3399+0.0026*Tau+0.0051*Ser-0.0049*ABA+0.0120*Ile-0.0190*Trp-0.0033*Lys |
| 0.8684 | 1.0492+0.0024*Tau+0.0002*Ala-0.0022*Val+0.0133*Ile-0.0106*His-0.0144*Trp |
| 0.8680 | 0.9605+0.0024*Tau-0.0024*Asn+0.0011*Gly+0.0097*Ile-0.0142*His-0.0135*Trp |
| 0.8680 | 0.3269+0.0027*Tau+0.0042*Ser-0.0050*ABA-0.0040*Val+0.0154*Ile-0.0171*Trp |
| 0.8680 | 0.3033+0.0026*Tau+0.0048*Ser+0.0001*Gly+0.0122*Ile-0.0192*Trp-0.0035*Lys |
| 0.8680 | 1.3660+0.0024*Tau-0.0006*Ala-0.0032*Val+0.0098*Leu-0.0173*His-0.0147*Trp |
| 0.8676 | 1.0924+0.0022*Tau+0.0022*Gly+0.0073*Leu-0.0235*His-0.0044*Lys+0.0019*Arg |
| 0.8676 | 0.8923+0.0026*Tau+0.0041*Ser+0.0060*Leu-0.0159*His-0.0174*Trp |
| 0.8676 | 0.8549+0.0026*Tau+0.0041*Ser+0.0057*Leu+0.0016*Phe-0.0161*His-0.0176*Trp |
| 0.8676 | 0.8998+0.0026*Tau+0.0041*Ser+0.0061*Leu-0.0157*His-0.0173*Trp-0.0004*Arg |
| 0.8676 | 1.1778+0.0024*Tau+0.0004*Asn+0.0124*Ile-0.0114*His-0.0144*Trp-0.0022*Lys |
| 0.8676 | 1.1834+0.0024*Tau+0.0004*Cit+0.0125*Ile-0.0114*His-0.0143*Trp-0.0021*Lys |

# FIG.167

| ROC AUC | FORMULA |
|---|---|
| 0.8971 | 7.13966+0.02376*Tau-0.02805*Val+0.13728*Met+0.08942*Leu-0.13844*His-0.16655*Trp |
| 0.8949 | 8.69320+0.02528*Tau+0.16064*Met+0.07992*Leu-0.15666*His-0.16319*Trp-0.03051*Lys |
| 0.8888 | 4.60075+0.02370*Tau+0.01503*Ser+0.09782*Met+0.05754*Leu-0.12911*His-0.18483*Trp |
| 0.8881 | 5.67557+0.02240*Tau+0.07851*Met+0.11006*Ile-0.07861*His-0.14408*Trp-0.02630*Lys |
| 0.8877 | 6.42882+0.02295*Tau+0.01209*Gly+0.07835*Leu-0.15401*His-0.12001*Trp-0.02128*Lys |
| 0.8870 | 7.33305+0.02239*Tau+0.02216*Thr+0.07623*Leu-0.12928*His-0.15751*Trp-0.02329*Lys |
| 0.8867 | 2.53306+0.02438*Tau+0.02466*Ser+0.12575*Ile-0.06041*His-0.14914*Trp-0.02465*Lys |
| 0.8867 | 5.83721+0.02325*Tau+0.03232*Cit+0.11295*Met+0.05762*Leu-0.14114*His-0.18557*Trp |
| 0.8845 | 5.40151+0.02270*Tau+0.00563*Gly+0.08436*Met+0.05948*Leu-0.14433*His-0.16815*Trp |
| 0.8838 | 6.36135+0.02267*Tau+0.11926*Met+0.05842*Leu-0.00863*Phe-0.13514*His-0.17925*Trp |
| 0.8827 | 4.65609+0.02117*Tau+0.12399*Ile+0.04252*Tyr-0.07650*His-0.14460*Trp-0.02660*Lys |
| 0.8824 | 4.23157+0.02235*Tau+0.00754*Gly+0.12216*Ile-0.08183*His-0.12382*Trp-0.02526*Lys |
| 0.8820 | 5.83394+0.02138*Tau+0.11223*Met+0.05068*Leu+0.02143*Tyr-0.13389*His-0.18136*Trp |
| 0.8813 | 6.70208+0.02304*Tau-0.00898*Pro+0.12649*Met+0.06516*Leu-0.13722*His-0.18429*Trp |
| 0.8813 | 6.10041+0.02247*Tau+0.00336*Thr+0.10068*Met+0.05763*Leu-0.13467*His-0.18097*Trp |
| 0.8802 | 3.99178+0.02234*Tau+0.01149*Ser+0.00804*Gly+0.06105*Leu-0.13248*His-0.15041*Trp |
| 0.8798 | 0.92794+0.02326*Tau+0.03037*Ser-0.01049*Pro+0.10523*Ile-0.05330*His-0.17370*Trp |
| 0.8798 | 2.34659+0.02196*Tau+0.01741*Ser+0.04018*Met+0.08176*Ile-0.07169*His-0.16965*Trp |
| 0.8795 | 6.64873+0.02302*Tau+0.12092*Met+0.06413*Leu-0.13677*His-0.16935*Trp-0.01992*Arg |
| 0.8795 | 6.28778+0.02257*Tau-0.00020*Ala+0.11038*Met+0.05768*Leu-0.13586*His-0.17850*Trp |
| 0.8788 | 5.22136+0.02535*Tau+0.03160*Ser-0.05816*Asn+0.06723*Leu-0.12392*His-0.16120*Trp |
| 0.8788 | 7.44195+0.02353*Tau-0.03114*Asn+0.10358*Met+0.06227*Leu-0.14341*His-0.17326*Trp |
| 0.8788 | 6.26871+0.02253*Tau+0.10972*Met+0.05753*Leu-0.13594*His-0.17862*Trp |
| 0.8788 | 5.95563+0.02258*Tau+0.10113*Met+0.01234*Ile+0.05145*Leu-0.13010*His-0.17832*Trp |
| 0.8788 | 5.12719+0.02201*Tau-0.01074*Pro+0.01252*Gly+0.07182*Leu-0.14450*His-0.14161*Trp |
| 0.8784 | 4.88688+0.02202*Tau+0.01159*Thr+0.12099*Ile-0.06777*His-0.14565*Trp-0.02572*Lys |
| 0.8784 | -0.89572+0.02572*Tau+0.04575*Ser-0.01692*Pro+0.14708*Ile-0.17007*Trp-0.03013*Lys |
| 0.8777 | 4.91211+0.02150*Tau+0.00673*Thr+0.00794*Gly+0.06110*Leu-0.13638*His-0.15296*Trp |
| 0.8773 | 2.36001+0.01955*Tau-0.03378*Val+0.16626*Ile+0.05399*Tyr-0.13297*Trp-0.02704*Lys |
| 0.8766 | 3.79265+0.01940*Tau+0.06553*Met+0.07068*Ile+0.03630*Tyr-0.08769*His-0.17244*Trp |
| 0.8766 | 5.16558+0.02155*Tau+0.00961*Gly-0.00388*Val+0.06607*Leu-0.13947*His-0.13944*Trp |
| 0.8755 | 1.43698+0.02277*Tau+0.02113*Ser+0.02461*Cit+0.09212*Ile-0.06876*His-0.17108*Trp |
| 0.8755 | 4.80017+0.02182*Tau+0.00958*Gly+0.01823*Cit+0.06167*Leu-0.14230*His-0.14561*Trp |
| 0.8752 | 4.03739+0.02206*Tau+0.00793*Gly+0.04585*Ile+0.03814*Leu-0.11940*His-0.15098*Trp |
| 0.8748 | 6.86680+0.02386*Tau-0.05946*Asn+0.01173*Gly+0.07116*Leu-0.16054*His-0.13395*Trp |
| 0.8748 | 2.44046+0.02344*Tau+0.02170*Ser+0.06297*Ile+0.02704*Leu-0.08856*His-0.17243*Trp |
| 0.8748 | 1.92063+0.02175*Tau+0.01474*Ser+0.00390*Gly+0.08898*Ile-0.07424*His-0.15729*Trp |
| 0.8745 | 2.73148+0.02421*Tau+0.02676*Ser-0.04854*Asn+0.09912*Ile-0.07019*His-0.16251*Trp |
| 0.8741 | 7.44747+0.02264*Tau+0.01687*Thr-0.04843*Asn+0.06599*Leu-0.13250*His-0.16681*Trp |
| 0.8741 | 5.01919+0.02147*Tau+0.00968*Gly+0.06163*Leu-0.13989*His-0.14213*Trp |
| 0.8741 | 4.88571+0.02088*Tau+0.00942*Gly+0.05839*Leu+0.00992*Tyr-0.13819*His-0.14323*Trp |
| 0.8730 | 5.91482+0.02328*Tau+0.09691*Ile+0.02737*Leu-0.09284*His-0.14227*Trp-0.02280*Lys |
| 0.8723 | 1.82065+0.02223*Tau+0.02040*Ser-0.00277*Val+0.09639*Ile-0.06270*His-0.16398*Trp |
| 0.8723 | 1.80490+0.02225*Tau+0.02069*Ser+0.09213*Ile-0.06542*His-0.16628*Trp |
| 0.8720 | 2.83102+0.01961*Tau+0.00555*Gly+0.08383*Ile+0.03010*Tyr-0.08778*His-0.15432*Trp |
| 0.8720 | 5.04209+0.02164*Tau+0.01011*Gly-0.00092*Ala+0.06248*Leu-0.14000*His-0.14048*Trp |
| 0.8720 | 4.29996+0.02050*Tau-0.00481*Val+0.05501*Met+0.08730*Ile-0.07461*His-0.15801*Trp |
| 0.8716 | 1.71661+0.02217*Tau+0.02136*Ser+0.08816*Ile+0.00870*Phe-0.06795*His-0.16726*Trp |
| 0.8716 | 4.37805+0.02061*Tau+0.06306*Met+0.08136*Ile-0.00793*Phe-0.07803*His-0.16222*Trp |
| 0.8716 | 1.84907+0.02215*Tau+0.00105*Thr+0.01996*Ser+0.09138*Ile-0.06560*His-0.16689*Trp |

# FIG.168

| ROC AUC | FORMULA |
|---|---|
| 0.8712 | 3.01799+0.02089*Tau+0.00589*Gly+0.01913*Cit+0.08889*Ile-0.08542*His-0.14960*Trp |
| 0.8712 | 3.59422+0.02050*Tau+0.00462*Gly+0.03379*Met+0.08105*Ile-0.08483*His-0.15230*Trp |
| 0.8712 | 4.18084+0.02220*Tau+0.01060*Thr+0.01628*Ser+0.05860*Leu-0.11371*His-0.17367*Trp |
| 0.8709 | 5.25350+0.02160*Tau+0.00961*Gly+0.06517*Leu-0.13923*His-0.13562*Trp-0.01054*Arg |
| 0.8709 | 3.99466+0.02102*Tau+0.02687*Cit+0.05861*Met+0.07906*Ile-0.08378*His-0.16715*Trp |
| 0.8709 | 4.47051+0.02060*Tau-0.00609*Pro+0.06236*Met+0.08632*Ile-0.07532*His-0.16379*Trp |
| 0.8705 | 4.75594+0.02158*Tau+0.00949*Gly+0.05925*Leu+0.02007*Phe-0.14482*His-0.14698*Trp |
| 0.8698 | 3.24992+0.02049*Tau+0.00064*Thr+0.00585*Gly+0.08838*Ile-0.08259*His-0.14675*Trp |
| 0.8698 | 5.10158+0.02210*Tau-0.00260*Val+0.12904*Ile-0.06655*His-0.13532*Trp-0.02191*Lys |
| 0.8698 | 6.01524+0.02110*Tau+0.01663*Thr+0.06420*Leu-0.11859*His-0.16201*Trp-0.01623*Arg |
| 0.8694 | 3.23671+0.02042*Tau+0.00603*Gly+0.08610*Ile+0.00584*Phe-0.08482*His-0.14626*Trp |
| 0.8694 | 4.25243+0.02044*Tau+0.00164*Thr+0.05102*Met+0.07972*Ile-0.07888*His-0.16280*Trp |
| 0.8691 | 3.25124+0.02051*Tau+0.00600*Gly+0.08874*Ile-0.08291*His-0.14590*Trp |
| 0.8691 | 3.33737+0.01930*Tau-0.01563*Val+0.11131*Ile+0.04066*Tyr-0.06419*His-0.15300*Trp |
| 0.8691 | 1.63275+0.02118*Tau+0.01813*Ser+0.08741*Ile+0.02834*Tyr-0.07163*His-0.17214*Trp |
| 0.8691 | 3.15357+0.02066*Tau-0.00838*Pro+0.00779*Gly+0.09839*Ile-0.07897*His-0.14487*Trp |
| 0.8691 | 5.25535+0.02226*Tau-0.00554*Pro+0.13378*Ile-0.06441*His-0.13769*Trp-0.02274*Lys |
| 0.8687 | 3.24517+0.02051*Tau+0.00609*Gly+0.00104*Val+0.08708*Ile-0.08412*His-0.14657*Trp |
| 0.8687 | 5.12529+0.02211*Tau+0.12532*Ile-0.06918*His-0.13718*Trp-0.02209*Lys |
| 0.8687 | 5.10798+0.02200*Tau+0.00057*Ala+0.12415*Ile-0.06995*His-0.13756*Trp-0.02220*Lys |
| 0.8684 | 5.85055+0.02271*Tau-0.02497*Asn+0.12652*Ile-0.07299*His-0.13506*Trp-0.02007*Lys |
| 0.8684 | 4.32972+0.02049*Tau+0.05532*Met+0.07989*Ile-0.07955*His-0.16175*Trp |
| 0.8680 | 1.56247+0.02299*Tau+0.02371*Ser-0.00227*Ala+0.09699*Ile-0.06128*His-0.16615*Trp |
| 0.8680 | 3.29398+0.01929*Tau+0.00728*Thr+0.08298*Ile+0.03306*Tyr-0.07814*His-0.17020*Trp |
| 0.8680 | 5.03793+0.02207*Tau-0.01637*Val+0.07877*Ile+0.03810*Leu-0.08888*His-0.14980*Trp |
| 0.8676 | 5.11700+0.02203*Tau+0.12297*Ile+0.00531*Phe-0.07085*His-0.13753*Trp-0.02211*Lys |
| 0.8676 | 4.36500+0.02065*Tau-0.00073*Ala+0.05679*Met+0.08118*Ile-0.07874*His-0.16135*Trp |
| 0.8673 | 4.72988+0.02307*Tau+0.03922*Cit+0.12876*Ile-0.07436*His-0.14252*Trp-0.02453*Lys |
| 0.8659 | 4.51895+0.02181*Tau-0.04371*Asn+0.00722*Gly+0.09451*Ile-0.09118*His-0.13766*Trp |
| 0.8655 | 4.43029+0.02081*Tau+0.05870*Met+0.08949*Ile-0.07458*His-0.15232*Trp-0.01774*Arg |
| 0.8655 | 1.89627+0.02254*Tau+0.02058*Ser+0.10185*Ile-0.06046*His-0.15710*Trp-0.01640*Arg |
| 0.8655 | 4.64104+0.02155*Tau+0.01025*Thr+0.05064*Ile+0.03337*Leu-0.10068*His-0.17146*Trp |
| 0.8644 | 5.07079+0.02209*Tau+0.12622*Ile-0.06774*His-0.13545*Trp-0.02037*Lys-0.00555*Arg |
| 0.8637 | 3.21675+0.02096*Tau+0.00685*Gly-0.00212*Ala+0.09278*Ile-0.08148*His-0.14296*Trp |
| 0.8630 | 3.91237+0.02008*Tau+0.07857*Ile+0.01036*Leu+0.02840*Tyr-0.08680*His-0.16402*Trp |
| 0.8630 | 3.38240+0.02077*Tau+0.00556*Gly+0.09777*Ile-0.07772*His-0.13847*Trp-0.01451*Arg |
| 0.8630 | 5.22397+0.02135*Tau-0.02869*Asn+0.04449*Met+0.08738*Ile-0.08220*His-0.15608*Trp |
| 0.8615 | 3.56516+0.01954*Tau+0.08901*Ile+0.03221*Tyr-0.07852*His-0.16286*Trp |
| 0.8615 | 3.28325+0.01997*Tau+0.02170*Cit+0.08906*Ile+0.03217*Tyr-0.08158*His-0.16687*Trp |
| 0.8612 | 3.90184+0.02060*Tau+0.00749*Thr+0.09912*Ile-0.06654*His-0.15209*Trp-0.01760*Arg |
| 0.8608 | 8.15827+0.02164*Tau-0.05486*Asn+0.00509*Ala+0.06300*Leu-0.13813*His-0.14926*Trp |
| 0.8605 | 5.11365+0.02075*Tau-0.05335*Asn+0.09527*Ile+0.04494*Tyr-0.08842*His-0.15822*Trp |
| 0.8601 | 3.57544+0.01943*Tau-0.00844*Pro+0.09896*Ile+0.04100*Tyr-0.07307*His-0.16668*Trp |
| 0.8601 | 3.55377+0.01945*Tau+0.08648*Ile+0.03232*Tyr+0.00562*Phe-0.08032*His-0.16328*Trp |
| 0.8594 | 3.65329+0.01985*Tau+0.09853*Ile+0.03148*Tyr-0.07355*His-0.15385*Trp-0.01586*Arg |
| 0.8583 | 3.58947+0.01978*Tau-0.00142*Ala+0.09193*Ile+0.03418*Tyr-0.07694*His-0.16222*Trp |
| 0.8551 | 7.96476+0.02053*Tau-0.05001*Asn+0.05739*Leu+0.02785*Tyr-0.12893*His-0.14476*Trp |
| 0.8544 | 3.39037+0.02233*Tau+0.06437*Cit+0.11285*Ile-0.07231*His-0.14956*Trp-0.03038*Arg |
| 0.8544 | 6.27324+0.02298*Tau-0.04092*Asn+0.06837*Ile+0.03065*Leu-0.10484*His-0.15480*Trp |
| 0.8504 | 5.40695+0.02196*Tau-0.03767*Asn+0.11147*Ile-0.07225*His-0.13890*Trp-0.01808*Arg |

# FIG.169

EP 2 124 059 A1

# FIG.170

| ROC AUC | FORMULA |
|---|---|
| 0.8978 | 2.1059+0.0194*Tau+0.0394*Ser+0.1120*Ile-0.0992*His-0.1170*Trp-0.0216*Lys |
| 0.8949 | 1.8294+0.0221*Tau+0.0361*Ser-0.0309*Glu+0.0995*Ile-0.1108*His-0.1309*Trp |
| 0.8902 | 1.4577+0.0194*Tau+0.0399*Ser-0.0305*Asn+0.0876*Ile-0.0991*His-0.1301*Trp |
| 0.8895 | 1.0430+0.0187*Tau+0.0378*Ser-0.0341*ABA+0.0860*Ile-0.0962*His-0.1302*Trp |
| 0.8888 | 5.3601+0.0167*Tau+0.0107*Gly+0.0567*Leu-0.1362*His-0.0836*Trp-0.0181*Lys |
| 0.8888 | 4.6386+0.0158*Tau+0.0080*Gly+0.0962*Ile-0.1065*His-0.0797*Trp-0.0216*Lys |
| 0.8877 | 1.1715+0.0195*Tau+0.0349*Ser-0.0187*Val+0.1181*Ile-0.0889*His-0.1164*Trp |
| 0.8863 | 5.3243+0.0190*Tau-0.0281*Glu+0.0076*Gly+0.0527*Leu-0.1421*His-0.0974*Trp |
| 0.8859 | 0.5574+0.0184*Tau+0.0404*Ser-0.0067*Pro+0.0903*Ile-0.0900*His-0.1331*Trp |
| 0.8845 | 0.9889+0.0182*Tau+0.0318*Ser-0.0027*Gly+0.0806*Ile-0.1036*His-0.1239*Trp |
| 0.8841 | 3.7663+0.0214*Tau+0.0340*Ser-0.0322*Glu+0.0546*Leu-0.1346*His-0.1220*Trp |
| 0.8841 | 1.0178+0.0185*Tau+0.0354*Ser+0.0840*Ile-0.0010*Phe-0.0979*His-0.1319*Trp |
| 0.8841 | 0.9919+0.0185*Tau+0.0355*Ser+0.0833*Ile-0.0981*His-0.1324*Trp+0.0005*Arg |
| 0.8838 | 0.7030+0.0188*Tau+0.0363*Ser+0.0139*Cit+0.0834*Ile-0.0994*His-0.1348*Trp |
| 0.8834 | 4.4088+0.0158*Tau-0.0067*Pro+0.0099*Gly+0.0479*Leu-0.1286*His-0.0981*Trp |
| 0.8834 | 1.0041+0.0185*Tau+0.0354*Ser+0.0835*Ile-0.0979*His-0.1320*Trp |
| 0.8834 | 0.9668+0.0186*Tau+0.0358*Ser-0.0002*Ala+0.0839*Ile-0.0972*His-0.1322*Trp |
| 0.8831 | 1.1186+0.0186*Tau+0.0353*Ser+0.0749*Ile+0.0065*Leu-0.1012*His-0.1328*Trp |
| 0.8827 | 2.2422+0.0192*Tau+0.0431*Ser-0.0058*Gln+0.0882*Ile-0.0891*His-0.1302*Trp |
| 0.8824 | 4.2059+0.0181*Tau-0.0256*Glu+0.0054*Gly+0.0830*Ile-0.1097*His-0.1001*Trp |
| 0.8820 | 3.1979+0.0190*Tau+0.0378*Ser-0.0329*Asn+0.0469*Leu-0.1176*His-0.1227*Trp |
| 0.8816 | 3.5120+0.0183*Tau+0.0357*Ser+0.0526*Leu-0.1157*His-0.1158*Trp-0.0135*Lys |
| 0.8813 | 6.1085+0.0184*Tau-0.0293*Glu+0.1101*Ile-0.0958*His-0.0967*Trp-0.0178*Lys |
| 0.8806 | 5.8347+0.0162*Tau-0.0051*Gln+0.0108*Gly+0.0451*Leu-0.1265*His-0.0926*Trp |
| 0.8798 | 4.9587+0.0165*Tau-0.0246*Asn+0.0089*Gly+0.0461*Leu-0.1327*His-0.0955*Trp |
| 0.8795 | 2.3506+0.0178*Tau-0.0262*Ser+0.0053*Gly+0.0435*Leu-0.1252*His-0.1132*Trp |
| 0.8788 | 4.5460+0.0160*Tau-0.0084*Gly-0.0220*ABA+0.0446*Leu-0.1292*His-0.0976*Trp |
| 0.8788 | 4.2272+0.0162*Tau-0.0087*Gly+0.0423*Leu-0.1325*His-0.1033*Trp+0.0057*Arg |
| 0.8788 | 2.3930+0.0178*Tau+0.0377*Ser-0.0064*Pro+0.0477*Leu-0.1093*His-0.1267*Trp |
| 0.8784 | 4.4109+0.0156*Tau-0.0079*Gly+0.0019*Ala+0.0423*Leu-0.1346*His-0.1013*Trp |
| 0.8780 | 5.1479+0.0178*Tau-0.0299*Glu+0.0026*Ala+0.0851*Ile-0.1069*His-0.1128*Trp |
| 0.8780 | 5.7602+0.0158*Tau+0.0811*Ile+0.0130*Leu-0.0950*His-0.1017*Trp-0.0185*Lys |
| 0.8777 | -1.6036+0.0172*Tau+0.0417*Ser-0.0122*Pro+0.1025*Ile-0.1380*Trp-0.0215*Lys |
| 0.8777 | 4.1609+0.0160*Tau-0.0084*Gly+0.0423*Leu+0.0089*Phe-0.1308*His-0.1022*Trp |
| 0.8773 | 2.8127+0.0151*Tau+0.0226*Ser-0.0088*Gly+0.0949*Ile-0.1372*His-0.0326*Lys |
| 0.8770 | 0.2179+0.0182*Tau+0.0425*Ser-0.0070*Gln+0.0972*Ile-0.1395*Trp-0.0195*Lys |
| 0.8770 | 4.3776+0.0159*Tau+0.0085*Gly+0.0434*Leu-0.1302*His-0.0995*Trp |
| 0.8766 | 4.9707+0.0170*Tau+0.0078*Gly-0.0158*Val+0.0632*Leu-0.1309*His-0.0879*Trp |
| 0.8763 | 2.9978+0.0178*Tau+0.0336*Ser-0.0309*Val+0.1557*Ile-0.1030*His-0.0301*Lys |
| 0.8763 | 5.2217+0.0180*Tau-0.0312*Val+0.0854*Ile+0.0390*Leu-0.0965*His-0.0985*Trp |
| 0.8763 | -0.6124+0.0182*Tau+0.0318*Ser-0.0278*Val+0.1391*Ile-0.1117*Trp-0.0206*Lys |
| 0.8763 | -1.0871+0.0179*Tau+0.0473*Ser-0.0066*Gln-0.0106*Pro+0.0849*Ile-0.1532*Trp |
| 0.8759 | 4.6155+0.0151*Tau+0.0088*Gly-0.0198*Val+0.1270*Ile-0.1147*His-0.0273*Lys |
| 0.8759 | 5.6692+0.0193*Tau-0.0328*Glu+0.0637*Ile+0.0210*Leu-0.1137*His-0.1156*Trp |
| 0.8755 | 4.3111+0.0161*Tau+0.0085*Gly+0.0051*Cit+0.0435*Leu-0.1310*His-0.1007*Trp |
| 0.8752 | 4.9760+0.0136*Tau-0.0091*Pro+0.0152*Gly+0.0580*Leu-0.1553*His-0.0264*Lys |
| 0.8752 | 0.5940+0.0201*Tau+0.0401*Ser-0.0081*Gln-0.0310*Val+0.1298*Ile-0.1214*Trp |
| 0.8752 | 3.8752+0.0181*Tau+0.0388*Ser-0.0044*Gln+0.0437*Leu-0.1096*His-0.1205*Trp |
| 0.8752 | 3.7438+0.0160*Tau+0.0052*Gly-0.0150*Val+0.0993*Ile-0.0943*His-0.0904*Trp |
| 0.8748 | 3.7398+0.0161*Tau+0.0073*Gly+0.0455*Ile+0.0201*Leu-0.1186*His-0.1036*Trp |

253

# FIG.171

| ROC AUC | FORMULA |
|---|---|
| 0.8748 | 4.2524+0.0131*Tau−0.0069*Pro+0.0122*Gly+0.0959*Ile−0.1235*His−0.0305*Lys |
| 0.8748 | 5.5962+0.0149*Tau+0.0030*Ala+0.0941*Ile−0.0974*His−0.0980*Trp−0.0194*Lys |
| 0.8745 | 5.5679+0.0163*Tau−0.0191*Val+0.1329*Ile−0.0784*His−0.0840*Trp−0.0179*Lys |
| 0.8741 | 3.2683+0.0190*Tau+0.0324*Ser−0.0172*Val+0.0650*Leu−0.1171*His−0.1112*Trp |
| 0.8741 | −2.5269+0.0171*Tau+0.0442*Ser−0.0137*Pro−0.0551*ABA+0.0800*Ile−0.1513*Trp |
| 0.8737 | 2.6001+0.0183*Tau+0.0372*Ser−0.0450*ABA+0.0468*Leu−0.1138*His−0.1234*Trp |
| 0.8737 | 3.5889+0.0153*Tau−0.0064*Gly−0.0081*ABA+0.0709*Ile−0.1044*His−0.0998*Trp |
| 0.8734 | 5.5293+0.0152*Tau−0.0020*Pro+0.0983*Ile−0.0845*His−0.0987*Trp−0.0183*Lys |
| 0.8734 | 5.1403+0.0180*Tau−0.0288*Glu−0.0013*Pro+0.0884*Ile−0.0966*His−0.1117*Trp |
| 0.8730 | 3.5476+0.0154*Tau−0.0065*Gly+0.0716*Ile−0.0017*Phe−0.1052*His−0.1005*Trp |
| 0.8727 | 4.8310+0.0135*Tau−0.0132*Gly+0.0500*Leu+0.0120*Phe−0.1608*His−0.0273*Lys |
| 0.8723 | 6.2873+0.0141*Tau−0.0057*Gln+0.0150*Gly+0.0515*Leu−0.1511*His−0.0219*Lys |
| 0.8723 | 2.7930+0.0174*Tau+0.0314*Ser+0.0014*Ala+0.0424*Leu−0.1182*His−0.1245*Trp |
| 0.8720 | 6.8301+0.0180*Tau−0.0322*Glu+0.0038*Ala+0.0502*Leu−0.1386*His−0.1108*Trp |
| 0.8720 | 3.5060+0.0154*Tau−0.0065*Gly+0.0010*Cit+0.0708*Ile−0.1054*His−0.1009*Trp |
| 0.8720 | 5.1251+0.0182*Tau−0.0291*Glu+0.0879*Ile−0.0986*His−0.1120*Trp |
| 0.8720 | 5.2077+0.0184*Tau−0.0297*Glu+0.0904*Ile−0.0041*Phe−0.0986*His−0.1119*Trp |
| 0.8720 | 5.1697+0.0181*Tau−0.0294*Glu+0.0890*Ile−0.0977*His−0.1101*Trp−0.0026*Arg |
| 0.8720 | 3.7906+0.0154*Tau−0.0121*Asn+0.0066*Gly+0.0712*Ile−0.1045*His−0.0977*Trp |
| 0.8716 | 3.4855+0.0151*Tau−0.0045*Pro+0.0074*Gly+0.0733*Ile−0.1013*His−0.0983*Trp |
| 0.8716 | 5.3705+0.0182*Tau−0.0087*Asn−0.0290*Glu+0.0883*Ile−0.0986*His−0.1098*Trp |
| 0.8716 | 7.5437+0.0188*Tau−0.0164*Asn−0.0318*Glu+0.0537*Leu−0.1305*His−0.1062*Trp |
| 0.8712 | −1.1486+0.0186*Tau+0.0336*Ser−0.0163*Glu+0.0936*Ile−0.1407*Trp−0.0226*Lys |
| 0.8712 | 3.4451+0.0155*Tau−0.0066*Gly+0.0696*Ile−0.1064*His−0.1026*Trp+0.0029*Arg |
| 0.8709 | 8.0817+0.0188*Tau−0.0332*Glu+0.0615*Leu−0.1313*His−0.0992*Trp−0.0125*Lys |
| 0.8709 | 4.3444+0.0143*Tau−0.0120*Gly+0.0925*Ile−0.1424*His−0.0377*Lys+0.0183*Arg |
| 0.8709 | 5.1944+0.0134*Tau−0.0129*Gly−0.0250*ABA+0.0519*Leu−0.1572*His−0.0249*Lys |
| 0.8709 | 5.0147+0.0181*Tau−0.0228*Glu−0.0135*Val+0.1091*Ile−0.0882*His−0.1019*Trp |
| 0.8705 | 5.0680+0.0187*Tau−0.0330*Glu+0.0198*ABA+0.0893*Ile−0.1013*His−0.1144*Trp |
| 0.8705 | 5.3789+0.0157*Tau−0.0130*Cit+0.0976*Ile−0.0891*His−0.1000*Trp−0.0191*Lys |
| 0.8702 | −1.4055+0.0176*Tau+0.0299*Ser−0.0231*ABA−0.0273*Val+0.1125*Ile−0.1278*Trp |
| 0.8702 | 2.3259+0.0178*Tau+0.0334*Ser+0.0417*Leu+0.0121*Phe−0.1150*His−0.1288*Trp |
| 0.8702 | 5.4518+0.0154*Tau−0.0024*Asn+0.0969*Ile−0.0875*His−0.0990*Trp−0.0185*Lys |
| 0.8694 | 3.5906+0.0151*Tau−0.0062*Gly+0.0015*Ala+0.0693*Ile−0.1098*His−0.1018*Trp |
| 0.8694 | 5.1456+0.0182*Tau−0.0291*Glu−0.0012*Cit+0.0879*Ile−0.0984*His−0.1118*Trp |
| 0.8694 | 2.5664+0.0179*Tau+0.0336*Ser+0.0427*Leu−0.1158*His−0.1274*Trp+0.0035*Arg |
| 0.8687 | 7.3587+0.0189*Tau−0.0269*Glu−0.0148*Val+0.0688*Leu−0.1274*His−0.0983*Trp |
| 0.8684 | −1.2645+0.0176*Tau+0.0362*Ser−0.0157*Asn+0.0873*Ile−0.1419*Trp−0.0208*Lys |
| 0.8684 | 4.5055+0.0154*Tau+0.0029*Ala−0.0206*Val+0.1089*Ile−0.0880*His−0.0982*Trp |
| 0.8680 | 7.6609+0.0184*Tau−0.0312*Glu−0.0016*Gln+0.0518*Leu−0.1263*His−0.1067*Trp |
| 0.8680 | 7.1214+0.0183*Tau−0.0313*Glu−0.0023*Pro+0.0532*Leu−0.1265*His−0.1094*Trp |
| 0.8680 | 6.5169+0.0161*Tau−0.0047*Ala−0.0249*Val+0.0709*Leu−0.1279*His−0.0961*Trp |
| 0.8673 | −2.3754+0.0174*Tau+0.0413*Ser−0.0223*Asn−0.0118*Pro+0.0787*Ile−0.1563*Trp |
| 0.8669 | −1.3803+0.0172*Tau+0.0360*Ser−0.0257*ABA+0.0875*Ile−0.1402*Trp−0.0212*Lys |
| 0.8669 | 3.5206+0.0154*Tau−0.0065*Gly+0.0708*Ile−0.1053*His−0.1006*Trp |
| 0.8666 | 4.9360+0.0145*Tau−0.0145*Gly+0.0523*Leu−0.1721*His−0.0333*Lys+0.0199*Arg |
| 0.8655 | −1.3888+0.0171*Tau+0.0340*Ser+0.0875*Ile−0.0009*Phe−0.1422*Trp−0.0219*Lys |
| 0.8651 | −0.2150+0.0180*Tau+0.0443*Ser−0.0081*Gln−0.0492*ABA+0.0759*Ile−0.1494*Trp |
| 0.8641 | 2.6516+0.0177*Tau+0.0333*Ser+0.0433*Leu−0.1144*His−0.1249*Trp |
| 0.8637 | −1.3548+0.0172*Tau+0.0348*Ser−0.0006*Gly+0.0877*Ile−0.1437*Trp−0.0218*Lys |

# FIG.172

| ROC AUC | FORMULA |
|---|---|
| 0.8999 | 8.58046+0.02373*Tau+0.17974*Met+0.07674*Leu-0.18035*His-0.15150*Trp-0.02895*Lys |
| 0.8999 | 7.64846+0.02732*Tau-0.03872*Val+0.19223*Met+0.10024*Leu-0.17891*His-0.16155*Trp |
| 0.8992 | 1.41168+0.02768*Tau-0.05704*Ser+0.17594*Ile-0.14335*His-0.16189*Trp-0.02587*Lys |
| 0.8895 | 1.76361+0.03010*Tau+0.06105*Ser-0.07473*Asn+0.15560*Ile-0.14876*His-0.18564*Trp |
| 0.8888 | 8.52590+0.02247*Tau+0.03065*Thr+0.07958*Leu-0.16011*His-0.16788*Trp-0.02834*Lys |
| 0.8885 | 0.18376+0.02754*Tau+0.05201*Ser-0.01664*Val+0.16810*Ile-0.12684*His-0.17256*Trp |
| 0.8885 | 6.00788+0.02140*Tau+0.01170*Gly+0.07198*Leu-0.16790*His-0.10557*Trp-0.01656*Lys |
| 0.8877 | 0.86969+0.02621*Tau+0.04499*Ser+0.04462*Met+0.12764*Ile-0.14148*His-0.18833*Trp |
| 0.8877 | 6.26594+0.02003*Tau+0.09909*Met+0.11845*Ile-0.12369*His-0.13579*Trp-0.02344*Lys |
| 0.8877 | 6.76559+0.02342*Tau+0.17904*Met+0.06040*Leu-0.01680*Phe-0.17338*His-0.18018*Trp |
| 0.8870 | 6.69901+0.02274*Tau-0.00865*Pro+0.17527*Met+0.06480*Leu-0.16985*His-0.18303*Trp |
| 0.8870 | 3.73002+0.02605*Tau+0.03045*Ser+0.13384*Met+0.06405*Leu-0.17608*His-0.19137*Trp |
| 0.8867 | -1.02610+0.02716*Tau+0.06258*Ser-0.00961*Pro+0.15110*Ile-0.12673*His-0.18716*Trp |
| 0.8863 | 5.81945+0.02271*Tau+0.00355*Gly+0.12996*Met+0.05896*Leu-0.17261*His-0.16708*Trp |
| 0.8863 | 4.73015+0.02038*Tau+0.00882*Gly+0.13152*Ile-0.13019*His-0.10728*Trp-0.02163*Lys |
| 0.8863 | 5.78151+0.02136*Tau+0.15622*Met+0.05462*Leu+0.01984*Tyr-0.16637*His-0.18317*Trp |
| 0.8856 | 5.98894+0.02355*Tau+0.02796*Cit+0.15406*Met+0.06064*Leu-0.17361*His-0.18860*Trp |
| 0.8841 | 6.44101+0.02006*Tau+0.02060*Thr+0.13426*Ile-0.11765*His-0.14421*Trp-0.02959*Lys |
| 0.8838 | 0.11396+0.02660*Tau+0.05006*Ser+0.13050*Ile+0.00541*Leu-0.13614*His-0.18285*Trp |
| 0.8834 | 0.04065+0.02652*Tau+0.05039*Ser+0.13805*Ile-0.13401*His-0.18286*Trp |
| 0.8834 | -0.24950+0.02540*Tau+0.04763*Ser+0.13421*Ile+0.01334*Tyr-0.13080*His-0.18272*Trp |
| 0.8834 | 0.07526+0.02635*Tau+0.04908*Ser+0.00071*Gly+0.13656*Ile-0.13521*His-0.18005*Trp |
| 0.8831 | -0.54615+0.02726*Tau+0.05103*Ser+0.02723*Cit+0.14059*Ile-0.13521*His-0.19268*Trp |
| 0.8831 | 4.47736+0.01846*Tau+0.13859*Ile+0.04417*Tyr-0.10494*His-0.13272*Trp-0.02618*Lys |
| 0.8827 | 3.58014+0.01906*Tau+0.10321*Met+0.09165*Ile+0.03816*Tyr-0.12576*His-0.17957*Trp |
| 0.8827 | 6.21953+0.02259*Tau+0.00512*Thr+0.13526*Met+0.05921*Leu-0.16730*His-0.18274*Trp |
| 0.8824 | 6.80855+0.02199*Tau+0.15965*Met+0.06158*Leu-0.17092*His-0.16115*Trp-0.01640*Arg |
| 0.8824 | 6.42552+0.02272*Tau+0.15074*Met+0.05907*Leu-0.16985*His-0.17793*Trp |
| 0.8820 | 0.02556+0.02649*Tau+0.05045*Ser+0.13749*Ile+0.00122*Phe-0.13421*His-0.18306*Trp |
| 0.8816 | 5.78470+0.02237*Tau+0.12738*Met+0.03544*Ile+0.04187*Leu-0.15739*His-0.17642*Trp |
| 0.8816 | -0.06942+0.02665*Tau-0.00159*Thr+0.05160*Ser+0.13938*Ile-0.13365*His-0.18156*Trp |
| 0.8816 | 5.17318+0.03145*Tau+0.05692*Ser-0.09254*Asn+0.08527*Leu-0.18830*His-0.16259*Trp |
| 0.8813 | 4.97895+0.02069*Tau-0.00715*Pro+0.01176*Gly+0.06341*Leu-0.15868*His-0.12071*Trp |
| 0.8813 | 5.06958+0.02128*Tau+0.12302*Met+0.10218*Ile-0.02129*Phe-0.12677*His-0.16890*Trp |
| 0.8806 | -0.37814+0.02809*Tau+0.05517*Ser-0.00348*Ala+0.14499*Ile-0.12596*His-0.18563*Trp |
| 0.8806 | 6.54971+0.02357*Tau-0.00184*Ala+0.15643*Met+0.06112*Leu-0.16849*His-0.17979*Trp |
| 0.8802 | 6.77237+0.02384*Tau+0.02501*Thr-0.02537*Val+0.09201*Leu-0.15186*His-0.16540*Trp |
| 0.8795 | 4.69808+0.01998*Tau-0.00299*Pro+0.09584*Met+0.09576*Ile-0.11803*His-0.16325*Trp |
| 0.8791 | 8.57404+0.02470*Tau-0.04924*Asn+0.13853*Met+0.06922*Leu-0.18955*His-0.16958*Trp |
| 0.8784 | 3.56504+0.02517*Tau+0.04625*Ser+0.07748*Leu-0.15559*His-0.14662*Trp-0.01494*Lys |
| 0.8780 | 0.48676+0.01669*Tau-0.04287*Val+0.19979*Ile+0.07728*Tyr-0.13218*Trp-0.02899*Lys |
| 0.8777 | 0.51459+0.02584*Tau+0.04980*Ser+0.14436*Ile-0.13492*His-0.16673*Trp-0.01519*Arg |
| 0.8777 | 4.70146+0.02138*Tau+0.00999*Gly+0.02038*Cit+0.06024*Leu-0.16163*His-0.13170*Trp |
| 0.8777 | 5.05282+0.02144*Tau+0.01379*Thr+0.00639*Gly+0.06002*Leu-0.15707*His-0.15170*Trp |
| 0.8773 | 4.74652+0.02088*Tau-0.01537*Val+0.09042*Met+0.12257*Ile-0.11165*His-0.15339*Trp |
| 0.8766 | 4.96950+0.02088*Tau+0.01000*Gly+0.05930*Leu-0.15964*His-0.12385*Trp |
| 0.8763 | 2.61907+0.02374*Tau+0.03229*Ser+0.00551*Gly+0.06299*Leu-0.15831*His-0.14179*Trp |
| 0.8763 | 3.36774+0.01900*Tau-0.02602*Val+0.15033*Ile+0.04470*Tyr-0.09227*His-0.14178*Trp |
| 0.8759 | -3.44294+0.02297*Tau+0.05295*Ser-0.01798*Pro+0.15322*Ile-0.17547*Trp-0.02457*Lys |
| 0.8755 | 3.99616+0.02043*Tau+0.00469*Gly+0.06308*Met+0.09694*Ile-0.12895*His-0.14972*Trp |

# FIG.173

| ROC AUC | FORMULA |
|---|---|
| 0.8755 | 4.66936+0.02028*Tau+0.08836*Met+0.09545*Ile-0.12151*His-0.16325*Trp |
| 0.8745 | 4.53851+0.02020*Tau+0.00410*Thr+0.07718*Met+0.09543*Ile-0.12033*His-0.16669*Trp |
| 0.8741 | 6.02201+0.02089*Tau+0.11206*Ile+0.01936*Leu-0.11842*His-0.13192*Trp-0.02030*Lys |
| 0.8737 | 1.77284+0.02504*Tau+0.05353*Ser-0.00906*Pro+0.07342*Leu-0.14603*His-0.16099*Trp |
| 0.8734 | 4.28157+0.02079*Tau+0.02094*Cit+0.08867*Met+0.09678*Ile-0.12239*His-0.17016*Trp |
| 0.8730 | 5.75634+0.02046*Tau-0.01333*Val+0.15735*Ile-0.09964*His-0.11972*Trp-0.01874*Lys |
| 0.8730 | 5.16372+0.02044*Tau+0.01015*Gly+0.06104*Leu-0.16024*His-0.11385*Trp-0.00843*Arg |
| 0.8727 | 4.66346+0.02051*Tau-0.00073*Ala+0.09000*Met+0.09641*Ile-0.11958*His-0.16361*Trp |
| 0.8720 | 2.67426+0.02416*Tau+0.04218*Ser+0.06505*Leu-0.14992*His-0.15635*Trp |
| 0.8716 | 4.33318+0.02046*Tau+0.01261*Thr-0.01533*Val+0.13152*Ile-0.10052*His-0.15473*Trp |
| 0.8712 | 8.63679+0.02483*Tau+0.02219*Thr-0.07198*Asn+0.07637*Leu-0.16951*His-0.17395*Trp |
| 0.8712 | 2.62012+0.01919*Tau+0.00707*Gly+0.10440*Ile+0.02856*Tyr-0.12373*His-0.14489*Trp |
| 0.8712 | 3.82076+0.02123*Tau+0.00806*Gly+0.08036*Ile+0.02048*Leu-0.13996*His-0.13732*Trp |
| 0.8705 | 5.08248+0.02089*Tau+0.03142*Cit+0.13742*Ile-0.10822*His-0.13561*Trp-0.02060*Lys |
| 0.8705 | 5.66561+0.02061*Tau-0.03424*Asn+0.07785*Met+0.10189*Ile-0.12248*His-0.15487*Trp |
| 0.8702 | 5.27207+0.01966*Tau+0.09655*Met+0.10288*Ile-0.12560*His-0.14418*Trp-0.01968*Arg |
| 0.8698 | 3.66334+0.02069*Tau+0.00695*Gly-0.00983*Val+0.12341*Ile-0.11849*His-0.12825*Trp |
| 0.8698 | 2.39226+0.02451*Tau+0.04175*Ser+0.02242*Cit+0.06534*Leu-0.15220*His-0.16203*Trp |
| 0.8691 | 3.55252+0.02027*Tau+0.00591*Thr+0.00619*Gly+0.10386*Ile-0.12413*His-0.14396*Trp |
| 0.8691 | 5.59774+0.01993*Tau-0.00150*Pro+0.13515*Ile-0.10346*His-0.12785*Trp-0.01957*Lys |
| 0.8687 | 5.56982+0.01993*Tau+0.00048*Ala+0.13332*Ile-0.10734*His-0.12810*Trp-0.01936*Lys |
| 0.8687 | 5.60390+0.02017*Tau+0.13617*Ile-0.00323*Phe-0.10586*His-0.12825*Trp-0.01929*Lys |
| 0.8684 | 5.56924+0.02006*Tau+0.13400*Ile-0.10600*His-0.12826*Trp-0.01927*Lys |
| 0.8680 | 3.42150+0.01997*Tau-0.00400*Pro+0.00854*Gly+0.10707*Ile-0.12183*His-0.13155*Trp |
| 0.8680 | 5.56170+0.01999*Tau+0.13385*Ile-0.10595*His-0.12715*Trp-0.01842*Lys-0.00209*Arg |
| 0.8680 | 4.16944+0.02001*Tau+0.01198*Thr+0.10486*Ile-0.10921*His-0.16474*Trp |
| 0.8676 | 3.92850+0.01980*Tau+0.00775*Gly+0.11383*Ile-0.12831*His-0.11780*Trp-0.01618*Arg |
| 0.8676 | 7.48723+0.02479*Tau-0.07136*Asn+0.01065*Gly+0.07476*Leu-0.18602*His-0.12108*Trp |
| 0.8676 | 3.51984+0.02044*Tau+0.00756*Gly+0.10763*Ile-0.00267*Phe-0.12551*His-0.13445*Trp |
| 0.8669 | 3.48854+0.02036*Tau+0.00756*Gly+0.10613*Ile-0.12570*His-0.13461*Trp |
| 0.8662 | 4.15555+0.01985*Tau+0.01247*Thr-0.00162*Pro+0.10543*Ile-0.10661*His-0.16474*Trp |
| 0.8655 | 4.94232+0.01936*Tau+0.01698*Thr+0.11475*Ile-0.11703*His-0.14581*Trp-0.02469*Arg |
| 0.8655 | 4.48783+0.02058*Tau+0.00087*Pro-0.01441*Val+0.13582*Ile-0.09816*His-0.14036*Trp |
| 0.8651 | 3.42116+0.02076*Tau+0.00792*Gly-0.00133*Ala+0.10772*Ile-0.12232*His-0.13374*Trp |
| 0.8648 | 4.65090+0.02111*Tau-0.04963*Asn+0.00805*Gly+0.11324*Ile-0.12712*His-0.12592*Trp |
| 0.8644 | 6.41319+0.02049*Tau-0.03230*Asn+0.13396*Ile-0.10909*His-0.12702*Trp-0.01594*Lys |
| 0.8644 | 4.48803+0.02046*Tau-0.01393*Val+0.13529*Ile-0.09692*His-0.14046*Trp |
| 0.8641 | 3.13166+0.02096*Tau+0.00759*Gly+0.02183*Cit+0.10797*Ile-0.12705*His-0.14302*Trp |
| 0.8623 | 5.31097+0.01937*Tau-0.07308*Asn+0.11782*Ile+0.05638*Tyr-0.12050*His-0.15532*Trp |
| 0.8608 | 4.92557+0.02000*Tau-0.01373*Val+0.14135*Ile-0.09884*His-0.12550*Trp-0.01487*Arg |
| 0.8594 | 5.46428+0.02072*Tau+0.01323*Thr-0.04880*Asn+0.11158*Ile-0.11096*His-0.15902*Trp |
| 0.8583 | 5.85967+0.02109*Tau-0.04353*Asn-0.01333*Val+0.13991*Ile-0.10147*His-0.13401*Trp |
| 0.8580 | 4.29968+0.02011*Tau+0.11138*Ile-0.10514*His-0.15002*Trp |
| 0.8569 | 5.86577+0.02019*Tau-0.04702*Asn+0.00203*Ala+0.11410*Ile-0.11679*His-0.14178*Trp |
| 0.8569 | 4.67198+0.01956*Tau-0.00020*Pro+0.11767*Ile-0.10531*His-0.13454*Trp-0.01504*Arg |
| 0.8565 | 4.67191+0.01958*Tau+0.11758*Ile-0.10565*His-0.13456*Trp-0.01506*Arg |
| 0.8558 | 6.05040+0.02016*Tau-0.04295*Asn+0.12268*Ile-0.10948*His-0.12919*Trp-0.01446*Arg |
| 0.8555 | 7.11684+0.02244*Tau-0.05470*Asn+0.08271*Ile+0.03030*Leu-0.13820*His-0.14760*Trp |
| 0.8519 | 5.81880+0.02081*Tau-0.04412*Asn+0.00115*Pro+0.11643*Ile-0.11341*His-0.14241*Trp |
| 0.8504 | 5.80024+0.02067*Tau-0.04328*Asn+0.11674*Ile-0.11130*His-0.14232*Trp |

# FIG.174

# FIG.175

| ROC AUC | FORMULA |
|---|---|
| 0.8118 | 1.7238-0.0031*Val-0.0106*Met+0.0040*Leu-0.0033*Tyr-0.0121*His+0.0015*Arg |
| 0.8114 | 1.7912-0.0029*Val-0.0080*Met+0.0041*Leu-0.0013*Phe-0.0112*His-0.0049*Trp |
| 0.8111 | 1.7458+0.0009*Thr-0.0030*Val-0.0114*Met+0.0041*Leu-0.0117*His-0.0048*Trp |
| 0.8111 | 1.7847-0.0005*Tau-0.0029*Val-0.0091*Met+0.0039*Leu-0.0110*His-0.0048*Trp |
| 0.8111 | 1.7694-0.0006*Tau-0.0029*Val-0.0082*Met+0.0039*Leu-0.0030*Tyr-0.0117*His |
| 0.8105 | 1.7500+0.0000*Gln-0.0029*Val-0.0090*Met+0.0039*Leu-0.0112*His-0.0050*Trp |
| 0.8104 | 1.7656-0.0029*Val-0.0089*Met+0.0039*Leu-0.0112*His-0.0049*Trp |
| 0.8101 | 1.7687-0.0026*Val-0.0060*Met+0.0037*Leu-0.0026*Tyr-0.0110*His-0.0047*Trp |
| 0.8096 | 1.7630+0.0002*Glu-0.0030*Val-0.0088*Met+0.0039*Leu-0.0112*His-0.0050*Trp |
| 0.8096 | 1.7485-0.0031*Val-0.0113*Met+0.0040*Leu-0.0113*His-0.0048*Trp+0.0012*Arg |
| 0.8096 | 1.7374+0.0037*Asn-0.0077*ABA-0.0030*Val-0.0152*Met+0.0042*Leu-0.0120*His |
| 0.8085 | 1.6579+0.0035*Asn-0.0035*Val-0.0168*Met+0.0043*Leu-0.0126*His+0.0010*Arg |
| 0.8092 | 1.6890-0.0007*Tau-0.0009*Val-0.0071*Met-0.0037*Tyr-0.0120*His+0.0014*Arg |
| 0.8091 | 1.6152+0.0046*Asn-0.0082*ABA-0.0100*Met-0.0044*Tyr+0.0018*Phe-0.0123*His |
| 0.8091 | 1.7905-0.0082*ABA-0.0032*Val-0.0148*Met+0.0043*Leu-0.0119*His+0.0016*Arg |
| 0.8089 | 1.7211-0.0000*Ala-0.0035*Val-0.0140*Met+0.0042*Leu-0.0124*His+0.0013*Arg |
| 0.8089 | 1.7482+0.0016*Cit-0.0030*Val-0.0088*Met+0.0040*Leu-0.0114*His-0.0050*Trp |
| 0.8089 | 1.7554-0.0029*Val-0.0076*Met+0.0040*Leu-0.0030*Tyr-0.0006*Phe-0.0120*His |
| 0.8088 | 1.6469+0.0064*Asn-0.0028*Val-0.0147*Met+0.0040*Leu-0.0113*His-0.0058*Trp |
| 0.8088 | 1.7230+0.0010*Thr-0.0030*Val-0.0107*Met+0.0041*Leu-0.0030*Tyr-0.0126*His |
| 0.8088 | 1.6799-0.0012*Thr+0.0064*Asn-0.0085*ABA-0.0038*Tyr-0.0102*His-0.0068*Trp |
| 0.8086 | 1.8130-0.0077*ABA-0.0026*Val-0.0084*Met+0.0039*Leu-0.0028*Tyr-0.0115*His |
| 0.8086 | 1.6794+0.0060*Asn-0.0082*ABA-0.0077*Met-0.0028*Tyr-0.0106*His-0.0062*Trp |
| 0.8085 | 1.7203-0.0036*Val-0.0141*Met+0.0042*Leu-0.0124*His+0.0013*Arg |
| 0.8085 | 1.7204-0.0000*Glu-0.0036*Val-0.0141*Met+0.0042*Leu-0.0124*His+0.0013*Arg |
| 0.8085 | 1.7492-0.0035*Val-0.0130*Met+0.0044*Leu-0.0014*Phe-0.0124*His+0.0013*Arg |
| 0.8085 | 1.6551+0.0047*Asn-0.0028*Val-0.0121*Met+0.0040*Leu-0.0033*Tyr-0.0123*His |
| 0.8083 | 1.7638-0.0001*Gln-0.0029*Val-0.0076*Met+0.0039*Leu-0.0032*Tyr-0.0119*His |
| 0.8082 | 1.7575+0.0002*Ala-0.0031*Val-0.0101*Met+0.0041*Leu-0.0111*His-0.0053*Trp |
| 0.8082 | 1.7264-0.0007*Cit-0.0035*Val-0.0144*Met+0.0042*Leu-0.0123*His+0.0015*Arg |
| 0.8082 | 1.6881-0.0006*Tau+0.0061*Asn-0.0088*ABA-0.0114*Met-0.0107*His-0.0066*Trp |
| 0.8080 | 1.7525-0.0001*Gln-0.0036*Val-0.0139*Met+0.0043*Leu-0.0122*His+0.0014*Arg |
| 0.8080 | 1.7881+0.0022*Cit-0.0082*ABA-0.0031*Val-0.0116*Met+0.0044*Leu-0.0122*His |
| 0.8079 | 1.8480-0.0088*ABA-0.0025*Val-0.0088*Met+0.0039*Leu-0.0105*His-0.0056*Trp |
| 0.8079 | 1.7228+0.0004*Pro-0.0039*Val-0.0149*Met+0.0046*Leu-0.0125*His+0.0011*Arg |
| 0.8079 | 1.7897+0.0009*Thr-0.0077*ABA-0.0031*Val-0.0140*Met+0.0044*Leu-0.0123*His |
| 0.8077 | 1.6511+0.0067*Asn-0.0095*ABA-0.0133*Met+0.0010*Leu-0.0113*His-0.0072*Trp |
| 0.8077 | 1.7079+0.0008*Thr-0.0036*Val-0.0156*Met+0.0044*Leu-0.0128*His+0.0010*Arg |
| 0.8077 | 1.6712+0.0061*Asn-0.0001*Ala-0.0088*ABA-0.0106*Met-0.0110*His-0.0066*Trp |
| 0.8076 | 1.7646+0.0004*Pro-0.0033*Val-0.0102*Met+0.0043*Leu-0.0113*His-0.0048*Trp |
| 0.8076 | 1.8583-0.0001*Gln-0.0081*ABA-0.0030*Val-0.0111*Met+0.0042*Leu-0.0116*His |
| 0.8075 | 1.7930-0.0002*Gln-0.0089*ABA-0.0069*Met-0.0043*Tyr-0.0119*His+0.0017*Arg |
| 0.8075 | 1.7208-0.0006*Glu-0.0085*ABA-0.0079*Met-0.0039*Tyr-0.0122*His+0.0014*Arg |
| 0.8075 | 1.8089-0.0085*ABA-0.0019*Val+0.0031*Leu-0.0033*Tyr-0.0108*His-0.0057*Trp |
| 0.8073 | 1.6607-0.0009*Val-0.0065*Met-0.0038*Tyr-0.0124*His+0.0013*Arg |
| 0.8073 | 1.6605+0.0000*Glu-0.0009*Val-0.0065*Met-0.0038*Tyr-0.0124*His+0.0013*Arg |
| 0.8073 | 1.7120-0.0006*Tau-0.0000*Gln-0.0008*Val-0.0044*Met-0.0035*Tyr-0.0119*His |
| 0.8073 | 1.6817-0.0022*Cit-0.0009*Val-0.0076*Met-0.0039*Tyr-0.0120*His+0.0018*Arg |
| 0.8073 | 1.7442-0.0029*Val-0.0078*Met+0.0039*Leu-0.0031*Tyr-0.0120*His |
| 0.8073 | 1.7317+0.0011*Cit-0.0030*Val-0.0079*Met+0.0040*Leu-0.0031*Tyr-0.0122*His |

# FIG.176

| ROC AUC | FORMULA |
|---|---|
| 0.8073 | 1.8163-0.0078*ABA-0.0030*Val-0.0114*Met+0.0042*Leu-0.0003*Phe-0.0118*His |
| 0.8073 | 1.6867-0.0005*Tau-0.0049*Met-0.0039*Tyr-0.0115*His-0.0050*Trp+0.0010*Arg |
| 0.8072 | 1.6616+0.0041*Asn-0.0003*Glu-0.0077*ABA-0.0086*Met-0.0038*Tyr-0.0123*His |
| 0.8072 | 1.6539-0.0002*Thr+0.0044*Asn-0.0078*ABA-0.0083*Met-0.0039*Tyr-0.0122*His |
| 0.8070 | 1.6990-0.0001*Gln-0.0009*Val-0.0062*Met-0.0039*Tyr-0.0122*His+0.0015*Arg |
| 0.8070 | 1.6499+0.0034*Asn-0.0081*ABA-0.0100*Met-0.0042*Tyr-0.0124*His+0.0011*Arg |
| 0.8070 | 1.7445-0.0004*Tau-0.0023*Val+0.0031*Leu-0.0036*Tyr-0.0113*His-0.0049*Trp |
| 0.8070 | 1.6837-0.0007*Tau+0.0045*Asn-0.0033*Val-0.0160*Met+0.0042*Leu-0.0122*His |
| 0.8070 | 1.7767-0.0006*Tau-0.0096*ABA-0.0089*Met-0.0109*His-0.0060*Trp+0.0012*Arg |
| 0.8069 | 1.7436-0.0007*Glu-0.0085*ABA-0.0062*Met-0.0007*Leu-0.0039*Tyr-0.0123*His |
| 0.8069 | 1.7305-0.0005*Glu+0.0010*Cit-0.0082*ABA-0.0051*Met-0.0036*Tyr-0.0123*His |
| 0.8069 | 1.6739+0.0062*Asn-0.0003*Pro-0.0090*ABA-0.0104*Met-0.0108*His-0.0068*Trp |
| 0.8069 | 1.7401+0.0004*Glu-0.0030*Val-0.0076*Met+0.0039*Leu-0.0032*Tyr-0.0120*His |
| 0.8069 | 1.6550+0.0043*Asn+0.0002*Glu-0.0034*Val-0.0155*Met+0.0043*Leu-0.0126*His |
| 0.8069 | 1.7388+0.0001*Ala-0.0030*Val-0.0085*Met+0.0040*Leu-0.0033*Tyr-0.0120*His |
| 0.8069 | 1.8067+0.0003*Pro-0.0075*ABA-0.0032*Val-0.0124*Met+0.0044*Leu-0.0119*His |
| 0.8069 | 1.6744+0.0059*Asn-0.0086*ABA-0.0001*Val-0.0106*Met-0.0109*His-0.0066*Trp |
| 0.8067 | 1.7047-0.0007*Tau+0.0001*Ala-0.0008*Val-0.0047*Met-0.0036*Tyr-0.0120*His |
| 0.8067 | 1.6991-0.0006*Tau-0.0008*Val-0.0047*Met-0.0036*Tyr+0.0004*Phe-0.0120*His |
| 0.8067 | 1.7040-0.0006*Tau+0.0003*Cit-0.0008*Val-0.0044*Met-0.0035*Tyr-0.0120*His |
| 0.8067 | 1.6542+0.0041*Asn+0.0001*Cit-0.0077*ABA-0.0085*Met-0.0039*Tyr-0.0123*His |
| 0.8067 | 1.7197-0.0012*Cit-0.0083*ABA-0.0081*Met-0.0042*Tyr-0.0121*His+0.0017*Arg |
| 0.8067 | 1.7274-0.0079*ABA-0.0006*Val-0.0074*Met-0.0036*Tyr-0.0119*His+0.0016*Arg |
| 0.8067 | 1.7527-0.0007*Tau+0.0013*Cit-0.0034*Val-0.0116*Met+0.0042*Leu-0.0122*His |
| 0.8067 | 1.6663+0.0060*Asn-0.0088*ABA-0.0110*Met-0.0110*His-0.0068*Trp |
| 0.8067 | 1.6628+0.0059*Asn+0.0006*Cit-0.0089*ABA-0.0108*Met-0.0111*His-0.0068*Trp |
| 0.8066 | 1.6561+0.0042*Asn-0.0001*Pro-0.0078*ABA-0.0084*Met-0.0039*Tyr-0.0123*His |
| 0.8066 | 1.6450-0.0010*Val-0.0071*Met-0.0040*Tyr+0.0009*Phe-0.0123*His+0.0014*Arg |
| 0.8066 | 1.6574+0.0038*Asn-0.0011*Glu-0.0088*ABA-0.0142*Met+0.0005*Leu-0.0130*His |
| 0.8066 | 1.6865+0.0041*Asn-0.0033*Val-0.0144*Met+0.0044*Leu-0.0012*Phe-0.0126*His |
| 0.8066 | 1.7506+0.0066*Asn-0.0004*Gln-0.0081*ABA-0.0089*Met-0.0041*Tyr-0.0116*His |
| 0.8066 | 1.8124-0.0002*Glu-0.0079*ABA-0.0029*Val-0.0117*Met+0.0041*Leu-0.0118*His |
| 0.8066 | 1.8151-0.0077*ABA-0.0030*Val-0.0109*Met+0.0042*Leu-0.0118*His-0.0002*Lys |
| 0.8066 | 1.8141-0.0001*Ala-0.0080*ABA-0.0029*Val-0.0112*Met+0.0041*Leu-0.0118*His |
| 0.8066 | 1.8100-0.0079*ABA-0.0030*Val-0.0116*Met+0.0042*Leu-0.0118*His |
| 0.8066 | 1.8403-0.0007*Tau-0.0080*ABA-0.0030*Val-0.0119*Met+0.0041*Leu-0.0115*His |
| 0.8064 | 1.7843-0.0028*Val-0.0071*Met+0.0041*Leu-0.0111*His-0.0048*Trp-0.0006*Lys |
| 0.8064 | 1.6547+0.0042*Asn-0.0077*ABA-0.0085*Met-0.0039*Tyr-0.0123*His |
| 0.8064 | 1.7499-0.0008*Tau-0.0035*Val-0.0145*Met+0.0042*Leu-0.0120*His+0.0014*Arg |
| 0.8064 | 1.7543-0.0006*Tau-0.0009*Glu-0.0090*ABA-0.0107*Met+0.0004*Leu-0.0125*His |
| 0.8064 | 1.6579+0.0042*Asn-0.0033*Val-0.0155*Met+0.0042*Leu-0.0126*His |
| 0.8064 | 1.6535-0.0006*Glu-0.0021*Cit-0.0080*Met-0.0046*Tyr-0.0127*His+0.0015*Arg |
| 0.8064 | 1.6580+0.0043*Asn-0.0000*Ala-0.0033*Val-0.0154*Met+0.0042*Leu-0.0126*His |
| 0.8063 | 1.7073-0.0006*Tau-0.0008*Val-0.0044*Met-0.0035*Tyr-0.0120*His |
| 0.8063 | 1.6630-0.0000*Ala-0.0009*Val-0.0064*Met-0.0037*Tyr-0.0124*His+0.0014*Arg |
| 0.8063 | 1.6564+0.0042*Asn+0.0003*Cit-0.0033*Val-0.0154*Met+0.0043*Leu-0.0126*His |
| 0.8061 | 1.6563+0.0002*Thr-0.0009*Val-0.0069*Met-0.0038*Tyr-0.0125*His+0.0013*Arg |
| 0.8061 | 1.7798-0.0011*Glu-0.0001*Gln-0.0001*Pro-0.0090*ABA-0.0091*Met-0.0124*His |
| 0.8061 | 1.7199-0.0080*ABA-0.0005*Val-0.0056*Met-0.0037*Tyr+0.0018*Phe-0.0117*His |
| 0.8061 | 1.6369+0.0063*Asn-0.0092*ABA-0.0124*Met+0.0013*Phe-0.0110*His-0.0069*Trp |

# FIG.177

| ROC AUC | FORMULA |
|---|---|
| 0.8151 | 7.43425+0.00396*Gly-0.02068*Val-0.12429*Met+0.06897*Ile-0.03029*His-0.06824*Trp |
| 0.8136 | 9.51651+0.00760*Gly-0.03687*Val-0.15131*Met+0.07475*Leu-0.07143*His-0.06788*Trp |
| 0.8132 | 9.94929+0.05061*Asn-0.03599*Val-0.16506*Met+0.07201*Leu-0.07157*His-0.07550*Trp |
| 0.8120 | 10.04847-0.03805*Val-0.12466*Met+0.02725*Ile+0.05612*Leu-0.05543*His-0.06751*Trp |
| 0.8114 | 8.12009-0.02159*Val-0.09429*Met+0.07198*Ile-0.01463*Tyr-0.02764*His-0.06608*Trp |
| 0.8110 | 7.92400+0.01364*Thr-0.02315*Val-0.14755*Met+0.07481*Ile-0.03483*His-0.07069*Trp |
| 0.8110 | 10.62634+0.01831*Thr-0.03911*Val-0.16577*Met+0.07481*Leu-0.07963*His-0.07027*Trp |
| 0.8108 | 8.47802-0.02164*Val-0.09138*Met+0.07619*Ile-0.02968*Phe-0.02478*His-0.06737*Trp |
| 0.8102 | 10.75049-0.03555*Val-0.08864*Met+0.07058*Leu-0.02422*Tyr-0.06766*His-0.06361*Trp |
| 0.8098 | 10.68521+0.00216*Pro-0.03922*Val-0.12075*Met+0.07191*Leu-0.06848*His-0.06651*Trp |
| 0.8098 | 10.79706-0.00281*Tau-0.03711*Val-0.11493*Met+0.06910*Leu-0.06699*His-0.06627*Trp |
| 0.8083 | 8.22637-0.02202*Val-0.09266*Met+0.07937*Ile-0.02622*His-0.06929*Trp-0.00679*Lys |
| 0.8082 | 10.33515-0.04081*Val-0.14693*Met+0.06919*Leu-0.06979*His-0.06429*Trp+0.02097*Arg |
| 0.8073 | 11.73056-0.03822*Val-0.08468*Met+0.08043*Leu-0.05249*Phe-0.06725*His-0.06567*Trp |
| 0.8072 | 9.96623+0.00725*Ser-0.03654*Val-0.12125*Met+0.07129*Leu-0.06938*His-0.07008*Trp |
| 0.8061 | 11.20759-0.03712*Val-0.08607*Met+0.07808*Leu-0.07109*His-0.06832*Trp-0.01018*Lys |
| 0.8060 | 8.09897-0.00263*Ser-0.02736*Val-0.10345*Met+0.09014*Ile-0.03587*Phe-0.07092*Trp |
| 0.8056 | 11.05828-0.03144*Val+0.07604*Leu-0.03746*Tyr-0.07250*His-0.06566*Trp-0.01450*Lys |
| 0.8051 | 8.03696-0.00374*Tau-0.02625*Val-0.10740*Met+0.08819*Ile-0.03445*Phe-0.07204*Trp |
| 0.8051 | 6.84655-0.00344*Tau+0.00329*Gly-0.02735*Val-0.14553*Met+0.08456*Ile-0.07410*Trp |
| 0.8045 | 6.76667+0.00266*Gly-0.02725*Val-0.13271*Met+0.08595*Ile-0.00857*Tyr-0.07323*Trp |
| 0.8038 | 11.60657-0.00502*Ser-0.03441*Val+0.07034*Leu-0.07228*Phe-0.06732*His-0.06437*Trp |
| 0.8035 | 7.75924-0.02662*Val-0.10390*Met+0.08958*Ile-0.00454*Tyr-0.03138*Phe-0.07194*Trp |
| 0.8035 | 7.80588-0.00081*Pro-0.02657*Val-0.10476*Met+0.08971*Ile-0.03435*Phe-0.07236*Trp |
| 0.8034 | 6.71353-0.00078*Ser+0.00326*Gly-0.02814*Val-0.14422*Met+0.08638*Ile-0.07398*Trp |
| 0.8031 | 7.77674-0.02702*Val-0.10774*Met+0.09013*Ile-0.03329*Phe-0.07241*Trp |
| 0.8031 | 7.49511-0.02593*Val-0.09252*Met+0.09302*Ile-0.01521*Tyr-0.07311*Trp-0.00771*Lys |
| 0.8031 | 7.43034-0.00255*Tau-0.02767*Val-0.11781*Met+0.08622*Ile-0.01296*Tyr-0.07183*Trp |
| 0.8029 | 9.28470+0.00216*Gly-0.02833*Val+0.05695*Leu-0.04110*Tyr-0.06994*His-0.06394*Trp |
| 0.8028 | 11.00274-0.03223*Val+0.07048*Leu-0.02409*Tyr-0.05459*Phe-0.06797*His-0.06420*Trp |
| 0.8023 | 6.77038+0.00343*Gly-0.00207*Ala-0.02720*Val-0.13701*Met+0.08777*Ile-0.07286*Trp |
| 0.8023 | 6.64932+0.00316*Gly-0.02803*Val-0.14453*Met+0.08649*Ile-0.07437*Trp |
| 0.8019 | 7.35550+0.00195*Gly-0.02630*Val-0.11810*Met+0.08846*Ile-0.02939*Phe-0.07303*Trp |
| 0.8018 | 10.12733-0.00231*Ala-0.02818*Val+0.05812*Leu-0.03838*Tyr-0.07071*His-0.06107*Trp |
| 0.8013 | 11.12918-0.00338*Tau-0.03266*Val+0.06858*Leu-0.06998*Phe-0.06721*His-0.06701*Trp |
| 0.8013 | 10.94996-0.03319*Val+0.06932*Leu-0.06879*Phe-0.06845*His-0.06714*Trp |
| 0.8012 | 7.91842-0.02564*Val-0.09116*Met+0.09475*Ile-0.03076*Phe-0.07360*Trp-0.00637*Lys |
| 0.8012 | 10.93855-0.03319*Val+0.00040*Ile+0.06909*Leu-0.06879*Phe-0.06827*His-0.06716*Trp |
| 0.8012 | 10.45372+0.00203*Gly-0.03225*Val+0.06826*Leu-0.06726*Phe-0.06959*His-0.06781*Trp |
| 0.8007 | 10.93465+0.00035*Thr-0.03315*Val+0.06923*Leu-0.06879*Phe-0.06869*His-0.06724*Trp |
| 0.8007 | 7.53179+0.00669*Thr-0.02802*Val-0.13107*Met+0.09258*Ile-0.02948*Phe-0.07468*Trp |
| 0.8007 | 7.03173+0.00849*Thr-0.02913*Val-0.14474*Met+0.09096*Ile-0.01189*Tyr-0.07489*Trp |
| 0.7999 | 11.74612-0.00403*Ala-0.03142*Val+0.07220*Leu-0.07150*Phe-0.07160*His-0.06257*Trp |
| 0.7999 | 10.81770-0.03334*Val+0.06818*Leu-0.06859*Phe-0.06883*His-0.06694*Trp+0.00327*Arg |
| 0.7999 | 11.04778-0.00219*Pro-0.03200*Val+0.06874*Leu-0.06949*Phe-0.06783*His-0.06682*Trp |
| 0.7996 | 11.93656-0.03469*Val+0.08417*Leu-0.05996*Phe-0.07196*His-0.06909*Trp-0.01320*Lys |
| 0.7994 | 11.23215-0.01047*Asn-0.03421*Val+0.07066*Leu-0.06806*Phe-0.06753*His-0.06503*Trp |
| 0.7993 | 10.97331-0.00302*Ala-0.03050*Val+0.07021*Leu-0.07646*His-0.06756*Trp-0.01544*Lys |
| 0.7988 | 7.79706-0.03742*Val-0.12930*Met+0.07289*Ile+0.02601*Leu-0.01666*Tyr-0.07415*Trp |
| 0.7985 | 8.34384-0.00928*Ser-0.02383*Val+0.07529*Ile-0.02251*Tyr-0.04999*Phe-0.06718*Trp |

260

# FIG.178

| ROC AUC | FORMULA |
|---|---|
| 0.7977 | 6.80184+0.09180*Cit-0.02645*Val-0.09528*Met+0.07699*Ile-0.03937*His-0.07714*Trp |
| 0.7968 | 8.70668-0.03934*Val-0.12016*Met+0.07097*Ile+0.03573*Leu-0.04637*Phe-0.07544*Trp |
| 0.7950 | 9.48334+0.09182*Cit-0.04069*Val-0.09675*Met+0.07192*Leu-0.08168*His-0.07552*Trp |
| 0.7917 | 6.44448+0.09257*Cit-0.02013*Val+0.05609*Ile-0.02372*Tyr-0.05013*His-0.07508*Trp |
| 0.7905 | 5.34523+0.03807*Asn+0.06974*Cit-0.03346*Val-0.16872*Met+0.10147*Ile-0.08976*Trp |
| 0.7899 | 9.68200+0.08856*Cit-0.03670*Val+0.07044*Leu-0.05565*Phe-0.08184*His-0.07593*Trp |
| 0.7896 | 5.87232+0.07706*Cit-0.03385*Val-0.12016*Met+0.09674*Ile-0.00561*Tyr-0.08281*Trp |
| 0.7895 | 6.32823+0.07436*Cit-0.03229*Val-0.10777*Met+0.09795*Ile-0.02569*Phe-0.08221*Trp |
| 0.7895 | 6.23870-0.00452*Ser+0.08249*Cit-0.03583*Val-0.12216*Met+0.09779*Ile-0.08176*Trp |
| 0.7890 | 8.66858+0.09143*Cit-0.03367*Val+0.06078*Leu-0.03181*Tyr-0.08236*His-0.07327*Trp |
| 0.7887 | 5.83039+0.07793*Cit-0.03465*Val-0.12729*Met+0.09732*Ile-0.08355*Trp+0.00048*Arg |
| 0.7886 | 6.59579+0.09753*Cit-0.02112*Val+0.07012*Ile-0.04760*His-0.08085*Trp-0.01159*Lys |
| 0.7882 | 5.83176+0.07836*Cit-0.03462*Val-0.12656*Met+0.09749*Ile-0.08364*Trp |
| 0.7879 | 6.13697-0.00615*Tau+0.08350*Cit-0.03384*Val-0.12702*Met+0.09478*Ile-0.08376*Trp |
| 0.7877 | 6.04449+0.07871*Cit-0.03275*Val-0.10452*Met+0.10314*Ile-0.08500*Trp-0.00760*Lys |
| 0.7877 | 9.39569+0.09756*Cit-0.03646*Val+0.07360*Leu-0.08806*His-0.08017*Trp-0.01486*Lys |
| 0.7874 | 5.83076-0.00084*Pro+0.07910*Cit-0.03429*Val-0.12422*Met+0.09709*Ile-0.08372*Trp |
| 0.7873 | 5.86864-0.00030*Gly+0.07932*Cit-0.03484*Val-0.12536*Met+0.09782*Ile-0.08369*Trp |
| 0.7873 | 5.98477-0.00196*Ala+0.07877*Cit-0.03400*Val-0.11847*Met+0.09894*Ile-0.08221*Trp |
| 0.7870 | 6.82169+0.09180*Cit-0.02077*Val+0.06061*Ile-0.03484*Phe-0.04720*His-0.07738*Trp |
| 0.7867 | 7.28370-0.00790*Tau+0.08587*Cit-0.06316*Met+0.01796*Ile-0.06929*His-0.08119*Trp |
| 0.7860 | 6.65250-0.00411*Ala+0.10104*Cit-0.02085*Val+0.05831*Ile-0.05559*His-0.07502*Trp |
| 0.7858 | 6.82822-0.00788*Ser+0.10700*Cit-0.02463*Val+0.05479*Ile-0.05307*His-0.07578*Trp |
| 0.7849 | 5.80827-0.00535*Thr+0.08786*Cit-0.03466*Val-0.10944*Met+0.09646*Ile-0.08315*Trp |
| 0.7849 | 6.26754+0.07537*Cit-0.04161*Val-0.13866*Met+0.08664*Ile+0.01916*Leu-0.08513*Trp |
| 0.7844 | 6.16592+0.10678*Cit-0.02227*Val+0.05887*Ile-0.05168*His-0.08068*Trp-0.00864*Arg |
| 0.7842 | 6.34146-0.00567*Tau+0.10533*Cit-0.02141*Val+0.04961*Ile-0.05448*His-0.07931*Trp |
| 0.7838 | 6.48684-0.01919*Asn+0.10306*Cit-0.02362*Val+0.05559*Ile-0.05144*His-0.07557*Trp |
| 0.7836 | 7.55971-0.00455*Ala+0.08515*Cit+0.03949*Ile-0.06867*His-0.07897*Trp-0.01231*Lys |
| 0.7833 | 8.15139-0.00563*Tau+0.10668*Cit-0.03371*Val+0.05252*Leu-0.08366*His-0.07894*Trp |
| 0.7833 | 8.10591+0.10480*Cit-0.03439*Val+0.05576*Leu-0.08488*His-0.07921*Trp-0.00458*Arg |
| 0.7826 | 8.65714-0.02525*Asn+0.10502*Cit-0.03715*Val+0.05803*Leu-0.08318*His-0.07382*Trp |
| 0.7825 | 8.05074-0.00512*Ala+0.07753*Cit+0.03402*Ile-0.04551*Phe-0.06565*His-0.07416*Trp |
| 0.7822 | 8.46508-0.00491*Ser+0.10556*Cit-0.03566*Val+0.05424*Leu-0.08450*His-0.07637*Trp |
| 0.7819 | 7.67856-0.00813*Tau+0.08385*Cit+0.02093*Ile-0.04344*Phe-0.06535*His-0.07973*Trp |
| 0.7817 | 6.19798-0.01269*Thr+0.11828*Cit-0.02478*Val+0.06161*Ile-0.04446*His-0.07788*Trp |
| 0.7810 | 7.71629+0.10244*Cit-0.03442*Val+0.00834*Ile+0.04929*Leu-0.08128*His-0.07912*Trp |
| 0.7808 | 7.44696-0.01545*Ser+0.08629*Cit-0.03246*Val+0.08565*Ile-0.05878*Phe-0.07769*Trp |
| 0.7804 | 7.95816+0.10186*Cit-0.03446*Val+0.05408*Leu-0.08498*His-0.07864*Trp |
| 0.7800 | 7.91908+0.00020*Gly+0.10138*Cit-0.03436*Val+0.05399*Leu-0.08502*His-0.07866*Trp |
| 0.7792 | 8.26452-0.01012*Thr+0.11524*Cit-0.03621*Val+0.05756*Leu-0.07996*His-0.07712*Trp |
| 0.7791 | 8.58188-0.00348*Ala+0.10160*Cit-0.03280*Val+0.05583*Leu-0.08773*His-0.07473*Trp |
| 0.7789 | 8.21358-0.00850*Tau-0.00215*Ser+0.08833*Cit-0.03042*Phe-0.06817*His-0.07412*Trp |
| 0.7763 | 5.27599-0.01704*Thr+0.10576*Cit-0.03116*Val+0.08167*Ile-0.02130*Tyr-0.08149*Trp |
| 0.7728 | 6.08593-0.01777*Thr+0.10482*Cit-0.03030*Val+0.08713*Ile-0.04334*Phe-0.08166*Trp |
| 0.7711 | 5.91007+0.08671*Cit-0.02818*Val+0.08958*Ile-0.04900*Phe-0.08742*Trp-0.01472*Arg |
| 0.7709 | 5.48791-0.01536*Thr+0.10809*Cit-0.03133*Val+0.09388*Ile-0.08667*Trp-0.01131*Lys |
| 0.7694 | 4.97164-0.01878*Thr+0.11839*Cit-0.03360*Val+0.08341*Ile-0.08601*Trp-0.00464*Arg |
| 0.7692 | 6.31906-0.00442*Ala+0.07379*Cit-0.02730*Val+0.08784*Ile-0.05445*Phe-0.08111*Trp |
| 0.7687 | 5.63243-0.00369*Ala+0.08277*Cit-0.02881*Val+0.09748*Ile-0.08731*Trp-0.01490*Lys |

# FIG.179

# FIG.180

| ROC AUC | FORMULA |
|---|---|
| 0.8126 | 7.8348-0.0197*Val-0.0744*Met+0.0256*Leu-0.0173*Tyr-0.0770*His+0.0088*Arg |
| 0.8108 | 7.4172+0.0217*Asn-0.0211*Val-0.1055*Met+0.0271*Leu-0.0808*His+0.0055*Arg |
| 0.8104 | 8.0292+0.0249*Asn-0.0475*ABA-0.0176*Val-0.0949*Met+0.0261*Leu-0.0812*His |
| 0.8096 | 8.6535-0.0037*Tau-0.0478*ABA-0.0178*Val-0.0715*Met+0.0250*Leu-0.0770*His |
| 0.8095 | 8.1245-0.0033*Tau-0.0181*Val-0.0588*Met+0.0237*Leu-0.0153*Tyr-0.0749*His |
| 0.8092 | 8.0859-0.0163*Val-0.0473*Met+0.0218*Leu-0.0132*Tyr-0.0720*His-0.0202*Trp |
| 0.8091 | 7.7586+0.0032*Thr-0.0219*Val-0.0950*Met+0.0275*Leu-0.0808*His+0.0062*Arg |
| 0.8091 | 7.4415+0.0280*Asn-0.0175*Val-0.0833*Met+0.0248*Leu-0.0168*Tyr-0.0788*His |
| 0.8089 | 7.5403-0.0047*Tau-0.0049*Val-0.0500*Met-0.0200*Tyr-0.0761*His+0.0079*Arg |
| 0.8089 | 7.9502+0.0083*Cit-0.0184*Val-0.0593*Met+0.0235*Leu-0.0749*His-0.0221*Trp |
| 0.8089 | 7.8762+0.0050*Thr-0.0187*Val-0.0720*Met+0.0254*Leu-0.0152*Tyr-0.0794*His |
| 0.8089 | 8.5142-0.0464*ABA-0.0163*Val-0.0567*Met+0.0244*Leu-0.0136*Tyr-0.0771*His |
| 0.8088 | 8.3435-0.0503*ABA-0.0198*Val-0.0909*Met+0.0275*Leu-0.0797*His+0.0098*Arg |
| 0.8088 | 7.8410-0.0035*Cit-0.0219*Val-0.0906*Met+0.0267*Leu-0.0786*His+0.0084*Arg |
| 0.8088 | 7.8659-0.0050*Glu-0.0502*ABA-0.0495*Met-0.0192*Tyr-0.0810*His+0.0079*Arg |
| 0.8088 | 7.7999-0.0039*Cit-0.0500*ABA-0.0480*Met-0.0215*Tyr-0.0810*His+0.0086*Arg |
| 0.8086 | 8.2223-0.0035*Tau-0.0177*Val-0.0599*Met+0.0221*Leu-0.0718*His-0.0219*Trp |
| 0.8086 | 8.5307-0.0470*ABA-0.0181*Val-0.0674*Met+0.0258*Leu-0.0792*His-0.0008*Lys |
| 0.8085 | 7.9243-0.0191*Val-0.0739*Met+0.0239*Leu-0.0740*His-0.0209*Trp+0.0065*Arg |
| 0.8085 | 8.6803-0.0514*ABA-0.0150*Val-0.0536*Met+0.0222*Leu-0.0724*His-0.0259*Trp |
| 0.8085 | 7.7936+0.0020*Pro-0.0232*Val-0.0927*Met+0.0284*Leu-0.0799*His+0.0074*Arg |
| 0.8085 | 7.8827+0.0081*Cit-0.0188*Val-0.0577*Met+0.0252*Leu-0.0158*Tyr-0.0782*His |
| 0.8083 | 7.8400-0.0005*Ala-0.0214*Val-0.0858*Met+0.0264*Leu-0.0794*His+0.0080*Arg |
| 0.8083 | 7.8068-0.0220*Val-0.0892*Met+0.0270*Leu-0.0792*His+0.0077*Arg |
| 0.8083 | 7.8411-0.0002*Gln-0.0507*ABA-0.0454*Met-0.0216*Tyr-0.0811*His+0.0079*Arg |
| 0.8083 | 7.2594-0.0049*Glu-0.0086*Cit-0.0527*Met-0.0238*Tyr-0.0800*His+0.0077*Arg |
| 0.8082 | 8.3296+0.0157*Cit-0.0503*ABA-0.0192*Val-0.0710*Met+0.0277*Leu-0.0821*His |
| 0.8082 | 8.4981+0.0003*Pro-0.0473*ABA-0.0184*Val-0.0712*Met+0.0260*Leu-0.0792*His |
| 0.8080 | 7.9586+0.0042*Thr-0.0183*Val-0.0718*Met+0.0237*Leu-0.0758*His-0.0210*Trp |
| 0.8080 | 7.3932+0.0230*Asn-0.0484*ABA-0.0632*Met-0.0209*Tyr-0.0827*His+0.0057*Arg |
| 0.8080 | 7.7228+0.0339*Asn-0.0012*Gln-0.0482*ABA-0.0576*Met-0.0202*Tyr-0.0798*His |
| 0.8080 | 7.9563-0.0042*Tau-0.0217*Val-0.0923*Met+0.0264*Leu-0.0770*His+0.0085*Arg |
| 0.8079 | 8.0862-0.0180*Val-0.0581*Met+0.0228*Leu-0.0011*Phe-0.0736*His-0.0218*Trp |
| 0.8079 | 7.6689+0.0371*Asn-0.0505*ABA-0.0532*Met-0.0145*Tyr-0.0734*His-0.0330*Trp |
| 0.8079 | 7.8024+0.0013*Glu-0.0225*Val-0.0891*Met+0.0275*Leu-0.0793*His+0.0077*Arg |
| 0.8079 | 8.4480+0.0002*Gln-0.0474*ABA-0.0182*Val-0.0713*Met+0.0257*Leu-0.0795*His |
| 0.8079 | 7.5115-0.0044*Tau-0.0400*Met-0.0215*Tyr-0.0728*His-0.0246*Trp+0.0058*Arg |
| 0.8079 | 8.5070+0.0001*Glu-0.0477*ABA-0.0182*Val-0.0705*Met+0.0258*Leu-0.0791*His |
| 0.8079 | 8.5077-0.0477*ABA-0.0182*Val-0.0705*Met+0.0257*Leu-0.0791*His |
| 0.8077 | 8.0554+0.0020*Pro-0.0193*Val-0.0631*Met+0.0241*Leu-0.0744*His-0.0217*Trp |
| 0.8077 | 7.8685-0.0470*ABA-0.0028*Val-0.0458*Met-0.0186*Tyr-0.0796*His+0.0083*Arg |
| 0.8077 | 7.9926-0.0184*Val-0.0575*Met+0.0243*Leu-0.0156*Tyr-0.0767*His |
| 0.8076 | 7.3805-0.0021*Glu-0.0046*Val-0.0464*Met-0.0197*Tyr-0.0786*His+0.0069*Arg |
| 0.8076 | 7.4128-0.0008*Ala-0.0047*Val-0.0423*Met-0.0191*Tyr-0.0788*His+0.0074*Arg |
| 0.8076 | 7.4910-0.0122*Cit-0.0051*Val-0.0512*Met-0.0203*Tyr-0.0763*His+0.0096*Arg |
| 0.8076 | 8.4683-0.0485*ABA-0.0182*Val-0.0726*Met+0.0254*Leu+0.0031*Phe-0.0793*His |
| 0.8075 | 8.0017-0.0184*Val-0.0572*Met+0.0244*Leu-0.0155*Tyr-0.0006*Phe-0.0767*His |
| 0.8075 | 8.0467+0.0003*Ala-0.0183*Val-0.0610*Met+0.0230*Leu-0.0734*His-0.0225*Trp |
| 0.8075 | 7.4379+0.0267*Asn+0.0012*Cit-0.0468*ABA-0.0563*Met-0.0195*Tyr-0.0826*His |
| 0.8075 | 7.2568-0.0054*Val-0.0500*Met-0.0217*Tyr+0.0072*Phe-0.0785*His+0.0069*Arg |

263

# FIG.181

| ROC AUC | FORMULA |
|---|---|
| 0.8073 | 8.0717-0.0180*Val-0.0587*Met+0.0227*Leu-0.0736*His-0.0220*Trp |
| 0.8073 | 7.3895+0.0355*Asn-0.0166*Val-0.0910*Met+0.0229*Leu-0.0750*His-0.0263*Trp |
| 0.8073 | 8.4205-0.0514*ABA-0.0115*Val+0.0172*Leu-0.0163*Tyr-0.0744*His-0.0280*Trp |
| 0.8073 | 8.0255-0.0055*Glu-0.0512*ABA-0.0414*Met+0.0040*Leu-0.0189*Tyr-0.0815*His |
| 0.8073 | 7.9814+0.0029*Glu-0.0193*Val-0.0566*Met+0.0252*Leu-0.0163*Tyr-0.0768*His |
| 0.8073 | 7.9847+0.0001*Ala-0.0185*Val-0.0581*Met+0.0244*Leu-0.0157*Tyr-0.0767*His |
| 0.8073 | 7.4271+0.0257*Asn+0.0018*Cit-0.0202*Val-0.0975*Met+0.0264*Leu-0.0812*His |
| 0.8073 | 7.7949-0.0053*Tau+0.0400*Asn-0.0536*ABA-0.0723*Met-0.0735*His-0.0357*Trp |
| 0.8072 | 7.9192-0.0218*Val-0.0847*Met+0.0278*Leu-0.0069*Phe-0.0790*His+0.0079*Arg |
| 0.8072 | 7.6185+0.0006*Gln-0.0218*Val-0.0908*Met+0.0269*Leu-0.0806*His+0.0073*Arg |
| 0.8072 | 7.6511-0.0042*Tau-0.0026*Cit-0.0045*Val-0.0352*Met-0.0184*Tyr-0.0767*His |
| 0.8072 | 7.9836-0.0038*Tau+0.0092*Cit-0.0208*Val-0.0743*Met+0.0260*Leu-0.0785*His |
| 0.8072 | 7.4521+0.0267*Asn-0.0073*Glu-0.0535*ABA-0.0854*Met+0.0039*Leu-0.0870*His |
| 0.8072 | 7.6883-0.0041*Tau-0.0045*Val-0.0354*Met-0.0183*Tyr-0.0763*His |
| 0.8072 | 7.1866+0.0297*Asn-0.0508*ABA-0.0703*Met-0.0233*Tyr+0.0151*Phe-0.0830*His |
| 0.8070 | 7.4436-0.0026*Thr+0.0315*Asn-0.0471*ABA-0.0639*Met-0.0198*Tyr-0.0813*His |
| 0.8070 | 8.4000+0.0042*Thr-0.0469*ABA-0.0185*Val-0.0325*Met+0.0267*Leu-0.0812*His |
| 0.8070 | 7.5230+0.0257*Asn-0.0199*Val-0.0944*Met+0.0268*Leu-0.0045*Phe-0.0808*His |
| 0.8070 | 7.4480+0.0270*Asn-0.0466*ABA-0.0567*Met-0.0195*Tyr-0.0823*His |
| 0.8069 | 7.5663-0.0041*Tau+0.0280*Asn-0.0195*Val-0.1008*Met+0.0254*Leu-0.0788*His |
| 0.8067 | 7.3548-0.0049*Val-0.0451*Met-0.0202*Tyr-0.0786*His+0.0069*Arg |
| 0.8067 | 7.3469+0.0004*Thr-0.0048*Val-0.0458*Met-0.0202*Tyr-0.0789*His+0.0066*Arg |
| 0.8067 | 7.4787+0.0410*Asn-0.0568*ABA-0.0783*Met+0.0044*Leu-0.0779*His-0.0365*Trp |
| 0.8066 | 7.7243+0.0011*Gln-0.0177*Val-0.0633*Met+0.0225*Leu-0.0757*His-0.0225*Trp |
| 0.8066 | 7.8894-0.0030*Tau-0.0137*Val+0.0168*Leu-0.0189*Tyr-0.0743*His-0.0238*Trp |
| 0.8066 | 7.8672-0.0489*ABA-0.0034*Val-0.0426*Met-0.0198*Tyr+0.0162*Phe-0.0792*His |
| 0.8066 | 7.7369-0.0096*Thr+0.0425*Asn-0.0544*ABA-0.0194*Tyr-0.0714*His-0.0379*Trp |
| 0.8064 | 8.5905-0.0009*Ala-0.0490*ABA-0.0172*Val-0.0637*Met+0.0246*Leu-0.0793*His |
| 0.8064 | 7.4427+0.0262*Asn-0.0200*Val-0.0979*Met+0.0262*Leu-0.0810*His |
| 0.8064 | 7.9526-0.0068*Glu+0.0003*Gln-0.0017*Pro-0.0530*ABA-0.0516*Met-0.0836*His |
| 0.8063 | 7.4264+0.0266*Asn+0.0021*Glu-0.0208*Val-0.0982*Met+0.0270*Leu-0.0811*His |
| 0.8063 | 8.2610-0.0049*Tau-0.0567*ABA-0.0505*Met-0.0737*His-0.0312*Trp+0.0058*Arg |
| 0.8061 | 8.0653+0.0023*Glu-0.0188*Val-0.0583*Met+0.0234*Leu-0.0737*His-0.0222*Trp |
| 0.8061 | 7.5343+0.0283*Asn-0.0021*Pro-0.0486*ABA-0.0551*Met-0.0185*Tyr-0.0812*His |
| 0.8061 | 7.7594+0.0007*Gln-0.0183*Val-0.0609*Met+0.0243*Leu-0.0152*Tyr-0.0784*His |
| 0.8061 | 7.6659+0.0392*Asn-0.0025*Pro-0.0556*ABA-0.0633*Met-0.0746*His-0.0355*Trp |
| 0.8061 | 8.0936-0.0036*Tau-0.0071*Glu-0.0539*ABA-0.0604*Met+0.0024*Leu-0.0830*His |
| 0.8061 | 7.3177+0.0412*Asn-0.0587*ABA-0.0843*Met+0.0158*Phe-0.0774*His-0.0385*Trp |
| 0.8060 | 7.6033-0.0039*Tau-0.0050*Val-0.0395*Met-0.0195*Tyr+0.0062*Phe-0.0763*His |
| 0.8057 | 7.7046-0.0039*Tau-0.0002*Ala-0.0044*Val-0.0341*Met-0.0179*Tyr-0.0764*His |
| 0.8056 | 8.1972-0.0173*Val-0.0462*Met+0.0232*Leu-0.0741*His-0.0214*Trp-0.0033*Lys |
| 0.8054 | 7.1999+0.0005*Gln-0.0048*Val-0.0467*Met-0.0198*Tyr-0.0799*His+0.0065*Arg |
| 0.8054 | 7.9398-0.0050*Glu+0.0075*Cit-0.0491*ABA-0.0348*Met-0.0175*Tyr-0.0818*His |
| 0.8048 | 7.5415+0.0257*Asn-0.0033*Glu-0.0467*ABA-0.0576*Met-0.0180*Tyr-0.0818*His |
| 0.8048 | 7.4635+0.0274*Asn-0.0006*Ala-0.0193*Val-0.0944*Met+0.0255*Leu-0.0812*His |
| 0.8048 | 7.6949+0.0386*Asn-0.0012*Ala-0.0542*ABA-0.0606*Met-0.0764*His-0.0335*Trp |
| 0.8048 | 7.6672+0.0361*Asn-0.0519*ABA-0.0012*Val-0.0639*Met-0.0754*His-0.0348*Trp |
| 0.8034 | 7.3629-0.0047*Tau+0.0011*Gln-0.0044*Val-0.0408*Met-0.0176*Tyr-0.0785*His |
| 0.8034 | 7.5815+0.0377*Asn-0.0535*ABA-0.0672*Met-0.0763*His-0.0357*Trp |
| 0.8032 | 7.5663+0.0373*Asn+0.0016*Cit-0.0537*ABA-0.0667*Met-0.0766*His-0.0357*Trp |

# FIG.182

| ROC AUC | FORMULA |
|---|---|
| 0.8189 | 9.31725+0.00865*Gly-0.04188*Val-0.16101*Met+0.08818*Leu-0.08139*His-0.06680*Trp |
| 0.8183 | 7.10530-0.01346*Ser+0.07391*Asn-0.03409*Val-0.21655*Met+0.10717*Ile-0.07653*Trp |
| 0.8167 | 6.96178+0.00430*Gly-0.02373*Val-0.12952*Met+0.07968*Ile-0.03178*His-0.06527*Trp |
| 0.8140 | 8.07889-0.02485*Val-0.10503*Met+0.08456*Ile-0.02609*Phe-0.02600*His-0.06140*Trp |
| 0.8137 | 8.43294-0.00775*Ser-0.03216*Val-0.11608*Met+0.10152*Ile-0.03640*Phe-0.06367*Trp |
| 0.8129 | 7.72002-0.02647*Val-0.11549*Met+0.08271*Ile-0.02918*His-0.06440*Trp |
| 0.8127 | 6.56822+0.03968*Asn-0.02953*Val-0.17388*Met+0.10223*Ile-0.02484*Phe-0.07257*Trp |
| 0.8126 | 8.96034-0.00996*Ser-0.02372*Val-0.07092*Ile-0.04375*Phe-0.03610*His-0.06114*Trp |
| 0.8115 | 7.47982-0.02681*Val-0.14115*Met+0.07664*Ile-0.03199*His-0.06180*Trp+0.01529*Arg |
| 0.8114 | 6.09856+0.04564*Asn-0.02976*Val-0.18137*Met+0.10080*Ile-0.01333*Tyr-0.07467*Trp |
| 0.8114 | 10.69991-0.04093*Val-0.10400*Met+0.08077*Leu-0.02116*Tyr-0.07324*His-0.06028*Trp |
| 0.8111 | 10.43623+0.01460*Thr-0.04237*Val-0.16892*Met+0.08290*Leu-0.08385*His-0.06358*Trp |
| 0.8107 | 6.03097+0.04581*Asn-0.03117*Val-0.19694*Met+0.10199*Ile-0.07626*Trp |
| 0.8102 | 11.42261-0.04172*Val-0.10562*Met+0.08593*Leu-0.04479*Phe-0.07192*His-0.05810*Trp |
| 0.8102 | 7.83666-0.00540*Tau-0.03013*Val-0.12768*Met+0.09835*Ile-0.03002*Phe-0.06707*Trp |
| 0.8101 | 10.63033-0.04197*Val-0.11964*Met+0.07923*Leu-0.07536*His-0.06385*Trp |
| 0.8092 | 11.40408-0.00734*Ser-0.03751*Val+0.07243*Leu-0.06057*Phe-0.07407*His-0.05889*Trp |
| 0.8092 | 10.23961-0.04466*Val-0.16476*Met+0.07875*Leu-0.07727*His-0.06059*Trp+0.02278*Arg |
| 0.8085 | 8.56602-0.01462*Ser-0.03019*Val+0.09183*Ile-0.02578*Tyr-0.04786*Phe-0.06180*Trp |
| 0.8079 | 6.39095+0.00275*Gly-0.03065*Val-0.14566*Met+0.09729*Ile-0.00799*Tyr-0.07011*Trp |
| 0.8073 | 7.37106-0.00516*Tau-0.03142*Val-0.13295*Met+0.09758*Ile-0.01214*Tyr-0.06903*Trp |
| 0.8067 | 7.43904-0.00115*Pro-0.02994*Val-0.12389*Met+0.09903*Ile-0.03044*Phe-0.06649*Trp |
| 0.8064 | 7.38583-0.03019*Val-0.12331*Met+0.09926*Ile-0.00409*Tyr-0.02796*Phe-0.06629*Trp |
| 0.8061 | 7.54574-0.02881*Val-0.11139*Met+0.10320*Ile-0.02730*Phe-0.06774*Trp-0.00624*Lys |
| 0.8061 | 6.92781+0.00210*Gly-0.02975*Val-0.13620*Met+0.09852*Ile-0.02592*Phe-0.06790*Trp |
| 0.8060 | 7.32414-0.00541*Tau-0.03260*Val-0.14649*Met+0.09834*Ile-0.07068*Trp |
| 0.8056 | 7.39468-0.03047*Val-0.12715*Met+0.09963*Ile-0.02959*Phe-0.06662*Trp |
| 0.8051 | 6.26041+0.00324*Gly-0.03118*Val-0.15650*Met+0.09753*Ile-0.07141*Trp |
| 0.8050 | 6.95461-0.03150*Val-0.13096*Met+0.09826*Ile-0.01316*Tyr-0.06822*Trp |
| 0.8048 | 7.71520-0.00286*Ala-0.02910*Val-0.10943*Met+0.10142*Ile-0.03543*Phe-0.06398*Trp |
| 0.8044 | 7.17807-0.02929*Val-0.11081*Met+0.10190*Ile-0.01329*Tyr-0.06913*Trp-0.00717*Lys |
| 0.8044 | 10.38849-0.03548*Val+0.07113*Leu-0.05578*Phe-0.07644*His-0.06157*Trp |
| 0.8038 | 6.70224+0.00608*Thr-0.03325*Val-0.16587*Met+0.10118*Ile-0.07122*Trp |
| 0.8037 | 7.10372-0.00189*Ala-0.03102*Val-0.12280*Met+0.09949*Ile-0.01210*Tyr-0.06727*Trp |
| 0.8032 | 7.27608-0.00328*Ser-0.03380*Val-0.14247*Met+0.10009*Ile-0.06927*Trp |
| 0.8032 | 11.26065-0.00439*Ala-0.03476*Val+0.07502*Leu-0.05889*Phe-0.07809*His-0.05620*Trp |
| 0.8029 | 9.66780+0.00317*Gly-0.03508*Val+0.07225*Leu-0.05164*Phe-0.08050*His-0.06348*Trp |
| 0.8026 | 7.04572-0.00200*Ala-0.03218*Val-0.13544*Met+0.10040*Ile-0.06896*Trp |
| 0.8026 | 9.55421-0.03671*Val+0.06330*Leu-0.08551*His-0.07008*Trp |
| 0.8020 | 8.59389-0.00412*Tau-0.01366*Ser-0.03097*Val+0.08946*Ile-0.06117*Phe-0.06635*Trp |
| 0.8016 | 6.64028-0.03369*Val-0.16599*Met+0.09561*Ile-0.06894*Trp+0.01185*Arg |
| 0.8015 | 6.89006-0.03280*Val-0.14577*Met+0.09919*Ile-0.07013*Trp |
| 0.8012 | 6.90109-0.00041*Pro-0.03265*Val-0.14484*Met+0.09904*Ile-0.07013*Trp |
| 0.8009 | 8.40415-0.01459*Ser-0.03138*Val+0.09019*Ile-0.06125*Phe-0.06547*Trp |
| 0.8006 | 11.41482-0.03597*Val+0.08547*Leu-0.04639*Phe-0.07950*His-0.06362*Trp-0.01468*Lys |
| 0.7999 | 7.11421-0.03068*Val-0.12569*Met+0.10293*Ile-0.07108*Trp-0.00714*Lys |
| 0.7993 | 6.60209+0.10063*Cit-0.02558*Val-0.11052*Met+0.07665*Ile-0.04662*His-0.07368*Trp |
| 0.7990 | 7.41996-0.04201*Val-0.16349*Met+0.08494*Ile+0.02552*Leu-0.07338*Trp |
| 0.7968 | 9.32947+0.09978*Cit-0.04127*Val-0.11991*Met+0.07711*Leu-0.09019*His-0.07194*Trp |
| 0.7963 | 5.25087+0.02360*Asn+0.06799*Cit-0.03431*Val-0.17102*Met+0.10184*Ile-0.08117*Trp |

# FIG.183

| ROC AUC | FORMULA |
|---|---|
| 0.7952 | 6.67315-0.01052*Ser+0.08638*Cit-0.03870*Val-0.13891*Met+0.10281*Ile-0.07652*Trp |
| 0.7942 | 6.00416+0.07071*Cit-0.03358*Val-0.13121*Met+0.10146*Ile-0.02190*Phe-0.07570*Trp |
| 0.7940 | 5.81955+0.07437*Cit-0.03328*Val-0.12576*Met+0.10465*Ile-0.07968*Trp-0.00718*Lys |
| 0.7933 | 5.65153+0.07341*Cit-0.03456*Val-0.13768*Met+0.09985*Ile-0.00702*Tyr-0.07772*Trp |
| 0.7931 | 6.12285-0.00797*Tau+0.08134*Cit-0.03479*Val-0.14916*Met+0.09802*Ile-0.07962*Trp |
| 0.7930 | 7.44203-0.01414*Ser+0.11576*Cit-0.02690*Val+0.06066*Ile-0.06178*His-0.07354*Trp |
| 0.7918 | 5.59244+0.07462*Cit-0.03533*Val-0.14577*Met+0.10044*Ile-0.07863*Trp |
| 0.7917 | 8.26083+0.09420*Cit-0.03401*Val+0.06354*Leu-0.03112*Tyr-0.09037*His-0.06940*Trp |
| 0.7912 | 7.49490-0.02086*Ser+0.08604*Cit-0.03636*Val+0.09356*Ile-0.05808*Phe-0.07033*Trp |
| 0.7911 | 5.61163-0.00209*Pro+0.07709*Cit-0.03463*Val-0.14172*Met+0.09983*Ile-0.07884*Trp |
| 0.7909 | 8.85548+0.09075*Cit-0.03487*Val+0.06707*Leu-0.04623*Phe-0.08922*His-0.06858*Trp |
| 0.7899 | 6.32679+0.09659*Cit-0.01994*Val+0.05828*Ile-0.02886*Phe-0.05703*His-0.07224*Trp |
| 0.7896 | 9.33726+0.09706*Cit-0.03588*Val+0.07624*Leu-0.09812*His-0.07552*Trp-0.01646*Lys |
| 0.7895 | 5.73426-0.00222*Ala+0.07616*Cit-0.03464*Val-0.13556*Met+0.10196*Ile-0.07715*Trp |
| 0.7892 | 8.90632-0.00473*Ser-0.04439*Phe-0.06437*His-0.06163*Trp+0.00986*Arg |
| 0.7890 | 5.56189-0.00943*Thr+0.09423*Cit-0.03554*Val-0.11713*Met+0.09885*Ile-0.07870*Trp |
| 0.7886 | 6.33335-0.01675*Ser+0.08304*Cit-0.03641*Val+0.09130*Ile-0.03809*Tyr-0.07303*Trp |
| 0.7883 | 6.43677-0.00432*Ala+0.10048*Cit-0.02125*Val+0.06189*Ile-0.06168*His-0.07195*Trp |
| 0.7880 | 6.03318+0.07027*Cit-0.04199*Val-0.15746*Met+0.08975*Ile+0.01869*Leu-0.08016*Trp |
| 0.7865 | 9.15642-0.01066*Ser+0.10877*Cit-0.03778*Val+0.05844*Leu-0.09526*His-0.07293*Trp |
| 0.7861 | 7.92936+0.09660*Cit-0.03509*Val+0.05821*Leu-0.09506*His-0.07527*Trp |
| 0.7846 | 6.54928-0.03009*Asn+0.11088*Cit-0.02415*Val+0.05927*Ile-0.05956*His-0.07208*Trp |
| 0.7839 | 8.74935-0.03249*Asn+0.10803*Cit-0.03816*Val+0.06218*Leu-0.09354*His-0.07012*Trp |
| 0.7835 | 7.46359-0.00510*Ala+0.09684*Cit+0.03043*Ile-0.04284*Phe-0.07346*His-0.07084*Trp |
| 0.7830 | 5.91424+0.09991*Cit-0.02170*Val+0.05413*Ile-0.06310*His-0.07629*Trp |
| 0.7820 | 6.50293+0.09865*Cit+0.01660*Ile-0.03183*Tyr-0.07772*His-0.07560*Trp |
| 0.7814 | 8.26053-0.01325*Thr+0.12232*Cit-0.03664*Val+0.06083*Leu-0.08883*His-0.07554*Trp |
| 0.7804 | 8.63266-0.00412*Ala+0.09813*Cit-0.03468*Val+0.06242*Leu-0.09725*His-0.07113*Trp |
| 0.7797 | 7.76815-0.00733*Ser+0.10758*Cit-0.02708*Tyr-0.08114*His-0.07153*Trp |
| 0.7787 | 5.28546+0.08313*Cit-0.02960*Val+0.09382*Ile-0.04388*Phe-0.08072*Trp-0.01823*Arg |
| 0.7787 | 7.33622-0.00842*Tau+0.10840*Cit-0.02380*Tyr-0.07856*His-0.07501*Trp |
| 0.7785 | 4.90117-0.02021*Thr+0.11011*Cit-0.03301*Val+0.08745*Ile-0.02214*Tyr-0.07768*Trp |
| 0.7784 | 5.11517+0.06930*Cit-0.02736*Val+0.09345*Ile-0.02902*Tyr-0.07940*Trp-0.01306*Lys |
| 0.7782 | 5.59924-0.02224*Thr+0.11189*Cit-0.03180*Val+0.09041*Ile-0.04176*Phe-0.07503*Trp |
| 0.7770 | 7.37908-0.00276*Ala+0.09976*Cit-0.01721*Tyr-0.07961*His-0.07075*Trp |
| 0.7769 | 6.96913+0.00112*Asn+0.09842*Cit-0.02399*Tyr-0.07962*His-0.07298*Trp |
| 0.7766 | 6.96482+0.09797*Cit-0.02428*Tyr-0.07978*His-0.07276*Trp+0.00092*Arg |
| 0.7765 | 4.75351+0.08157*Cit-0.03052*Val+0.09194*Ile-0.03164*Tyr-0.08092*Trp-0.01577*Arg |
| 0.7762 | 6.99170+0.09882*Cit-0.02381*Tyr-0.07965*His-0.07289*Trp |
| 0.7756 | 6.90232+0.09555*Cit+0.01828*Ile-0.03835*Phe-0.07777*His-0.07658*Trp |
| 0.7749 | 7.42185+0.09854*Cit-0.01804*Tyr-0.07851*His-0.07259*Trp-0.00472*Lys |
| 0.7743 | 8.34087-0.00477*Ala+0.09429*Cit+0.01991*Leu-0.04631*Phe-0.08853*His-0.07061*Trp |
| 0.7741 | 4.37820+0.06715*Cit-0.03056*Val+0.08415*Ile-0.03685*Tyr-0.07804*Trp |
| 0.7721 | 7.22348-0.00351*Ala+0.10102*Cit-0.08472*His-0.07443*Trp |
| 0.7713 | 5.65195-0.00502*Ala+0.06891*Cit-0.02855*Val+0.09136*Ile-0.05184*Phe-0.07208*Trp |
| 0.7700 | 7.24437+0.09658*Cit-0.02492*Phe-0.08047*His-0.07421*Trp |
| 0.7700 | 6.97868+0.10158*Cit-0.00699*Val-0.07861*His-0.07392*Trp |
| 0.7692 | 4.58546-0.02330*Thr+0.11795*Cit-0.03570*Val+0.08699*Ile-0.08152*Trp |
| 0.7667 | 4.26828+0.08793*Cit-0.03388*Val+0.09209*Ile-0.08792*Trp-0.02025*Arg |
| 0.7665 | 6.57120+0.10120*Cit-0.08747*His-0.07986*Trp |

# FIG.184

## FIG.185

| FORMULA |
|---|
| 0.76027+0.00263*Tau+0.00417*Ser+0.01200*Ile-0.01038*His-0.01472*Trp-0.00258*Lys |
| 0.76055+0.00275*Tau+0.00349*Ser-0.00239*Glu+0.01009*Ile-0.01205*His-0.01630*Trp |
| 1.18772+0.00251*Tau+0.00166*Gly+0.00828*Leu-0.01978*His-0.01144*Trp-0.00257*Lys |
| 0.98545+0.00240*Tau+0.00120*Gly+0.01217*Ile-0.01310*His-0.01135*Trp-0.00265*Lys |
| 1.16586+0.00274*Tau-0.00321*Glu+0.00122*Gly+0.00761*Leu-0.02026*His-0.01400*Trp |
| 0.67569+0.00263*Tau+0.00361*Ser-0.00186*Val+0.01240*Ile-0.00985*His-0.01534*Trp |
| 0.72353+0.00256*Tau+0.00412*Ser-0.00541*ABA+0.00929*Ile-0.01144*His-0.01649*Trp |
| 1.06529+0.00247*Tau-0.00097*Pro+0.00158*Gly+0.00725*Leu-0.01922*His-0.01385*Trp |
| 0.74425+0.00264*Tau+0.00422*Ser-0.00380*Asn+0.00962*Ile-0.01163*His-0.01629*Trp |
| 0.63594+0.00261*Tau+0.00448*Ser-0.00114*Pro+0.01043*Ile-0.01044*His-0.01709*Trp |
| 0.66372+0.00255*Tau+0.00307*Ser+0.00055*Gly+0.00906*Ile-0.01273*His-0.01567*Trp |
| 1.00221+0.00291*Tau+0.00379*Ser-0.00334*Glu+0.00712*Leu-0.01716*His-0.01688*Trp |
| 0.86580+0.00265*Tau+0.00484*Ser-0.00085*Gln+0.01014*Ile-0.01064*His-0.01599*Trp |
| 0.95589+0.00272*Tau+0.00467*Ser-0.00437*Asn+0.00635*Leu-0.01602*His-0.01679*Trp |
| 1.11582+0.00245*Tau+0.00139*Gly-0.00446*ABA+0.00675*Leu-0.01936*His-0.01383*Trp |
| 0.69435+0.00258*Tau+0.00374*Ser+0.00929*Ile-0.01171*His-0.01694*Trp |
| 0.69338+0.00258*Tau+0.00375*Ser+0.00006*Cit+0.00929*Ile-0.01172*His-0.01694*Trp |
| 1.14114+0.00251*Tau-0.00348*Asn+0.00148*Gly+0.00693*Leu-0.01980*His-0.01342*Trp |
| 0.99725+0.00268*Tau+0.00457*Ser+0.00733*Leu-0.01565*His-0.01562*Trp-0.00215*Lys |
| 0.97225+0.00255*Tau-0.00231*Glu+0.00086*Gly+0.01025*Ile-0.01411*His-0.01386*Trp |
| 1.23359+0.00259*Tau-0.00293*Glu+0.01331*Ile-0.01177*His-0.01362*Trp-0.00209*Lys |
| 0.82466+0.00263*Tau+0.00296*Ser+0.00091*Gly+0.00629*Leu-0.01784*His-0.01566*Trp |
| 1.24987+0.00247*Tau-0.00065*Gln+0.00165*Gly+0.00687*Leu-0.01935*His-0.01266*Trp |
| 1.12863+0.00257*Tau+0.00130*Gly-0.00224*Val+0.00931*Leu-0.01891*His-0.01282*Trp |
| 0.77249+0.00227*Tau+0.00368*Ser-0.00356*Val+0.01654*Ile-0.01032*His-0.00351*Lys |
| 0.68932+0.00257*Tau+0.00376*Ser+0.00910*Ile+0.00042*Phe-0.01181*His-0.01696*Trp |
| 1.06501+0.00244*Tau+0.00129*Gly+0.00014*Ala+0.00648*Leu-0.01943*His-0.01444*Trp |
| 1.08456+0.00244*Tau+0.00137*Gly-0.00149*Cit+0.00662*Leu-0.01916*His-0.01406*Trp |
| 0.68722+0.00260*Tau+0.00384*Ser-0.00009*Ala+0.00948*Ile-0.01152*His-0.01693*Trp |
| 0.69880+0.00257*Tau+0.00377*Ser+0.00959*Ile-0.01139*His-0.01669*Trp-0.00065*Arg |
| 1.06619+0.00247*Tau+0.00136*Gly+0.00659*Leu-0.01935*His-0.01423*Trp |
| 1.11784+0.00217*Tau-0.00123*Pro+0.00239*Gly+0.00843*Leu-0.02246*His-0.00381*Lys |
| 0.92797+0.00214*Tau+0.00144*Gly-0.00207*Val+0.01450*Ile-0.01397*His-0.00353*Lys |
| 1.04025+0.00245*Tau+0.00135*Gly+0.00637*Leu+0.00130*Phe-0.01952*His-0.01438*Trp |
| 1.06786+0.00246*Tau+0.00136*Gly+0.00661*Leu-0.01932*His-0.01421*Trp-0.00007*Arg |
| 1.13740+0.00255*Tau-0.00294*Glu+0.00016*Ala+0.01068*Ile-0.01309*His-0.01553*Trp |
| 0.97574+0.00248*Tau+0.00120*Gly+0.00439*Ile+0.00424*Leu-0.01749*His-0.01466*Trp |
| 1.39786+0.00265*Tau-0.00388*Glu+0.00045*Ala+0.00726*Leu-0.01891*His-0.01622*Trp |
| 0.75527+0.00265*Tau+0.00385*Ser+0.00592*Ile+0.00290*Leu-0.01381*His-0.01761*Trp |
| 0.91265+0.00206*Tau-0.00120*Pro+0.00191*Gly+0.01237*Ile-0.01566*His-0.00388*Lys |
| 0.60245+0.00278*Tau+0.00522*Ser-0.00116*Gln-0.00429*Val+0.01717*Ile-0.01517*Trp |
| 1.25471+0.00274*Tau-0.00375*Glu+0.00669*Ile+0.00398*Leu-0.01580*His-0.01618*Trp |
| 1.16980+0.00261*Tau-0.00381*Val+0.00978*Ile+0.00576*Leu-0.01278*His-0.01427*Trp |
| 0.92588+0.00263*Tau+0.00458*Ser-0.00682*ABA+0.00621*Leu-0.01569*His-0.01701*Trp |
| 0.86251+0.00267*Tau+0.00482*Ser-0.00102*Pro+0.00660*Leu-0.01513*His-0.01759*Trp |

# FIG.186

| FORMULA |
|---|
| 0.95335+0.00276*Tau+0.00409*Ser-0.00260*Val+0.00917*Leu-0.01549*His-0.01568*Trp |
| 1.04614+0.00267*Tau-0.00502*Ser-0.00067*Gln+0.00616*Leu-0.01509*His-0.01654*Trp |
| 0.90897+0.00241*Tau-0.00088*Gly-0.00136*Val+0.01179*Ile-0.01245*His-0.01326*Trp |
| 1.14800+0.00243*Tau-0.00203*Val+0.01572*Ile-0.00938*His-0.01266*Trp-0.00205*Lys |
| 0.74079+0.00214*Tau+0.00231*Ser+0.00142*Gly+0.01070*Ile-0.01601*His-0.00404*Lys |
| 0.89285+0.00237*Tau-0.00093*Pro+0.00117*Gly+0.01037*Ile-0.01341*His-0.01379*Trp |
| 1.18086+0.00232*Tau+0.00025*Ala+0.01192*Ile-0.01185*His-0.01433*Trp-0.00218*Lys |
| 1.25856+0.00243*Tau+0.00937*Ile+0.00272*Leu-0.01336*His-0.01485*Trp-0.00220*Lys |
| 1.19778+0.00236*Tau-0.00055*Pro+0.01307*Ile-0.01085*His-0.01430*Trp-0.00214*Lys |
| 0.94522+0.00235*Tau+0.00101*Gly-0.00306*ABA+0.00947*Ile-0.01396*His-0.01382*Trp |
| 1.56815+0.00273*Tau-0.00405*Glu+0.00861*Leu-0.01822*His-0.01444*Trp-0.00160*Lys |
| 0.91747+0.00208*Tau+0.00179*Gly+0.01071*Ile-0.01743*His-0.00438*Lys+0.00173*Arg |
| 1.15388+0.00258*Tau-0.00302*Glu-0.00055*Pro+0.01169*Ile-0.01226*His-0.01547*Trp |
| 0.92783+0.00235*Tau+0.00101*Gly-0.00155*Cit+0.00949*Ile-0.01383*His-0.01397*Trp |
| 1.17761+0.00259*Tau-0.00125*Asn-0.00304*Glu+0.01121*Ile-0.01280*His-0.01522*Trp |
| 1.15141+0.00212*Tau+0.00212*Gly-0.00417*ABA+0.00756*Leu-0.02270*His-0.00361*Lys |
| 0.33519+0.00266*Tau+0.00508*Ser-0.00217*Asn+0.01222*Ile-0.01910*Trp-0.00333*Lys |
| 0.90965+0.00237*Tau+0.00099*Gly+0.00944*Ile-0.01404*His-0.01415*Trp |
| 0.91299+0.00237*Tau+0.00099*Gly+0.00955*Ile-0.01393*His-0.01407*Trp-0.00022*Arg |
| 0.90781+0.00238*Tau+0.00101*Gly-0.00005*Ala+0.00953*Ile-0.01399*His-0.01410*Trp |
| 0.90754+0.00237*Tau+0.00099*Gly+0.00933*Ile+0.00024*Phe-0.01411*His-0.01416*Trp |
| 1.14925+0.00257*Tau-0.00295*Glu-0.00048*ABA+0.01105*Ile-0.01277*His-0.01544*Trp |
| 1.31674+0.00218*Tau-0.00075*Gln+0.00237*Gly+0.00767*Leu-0.02234*His-0.00327*Lys |
| 1.15954+0.00257*Tau-0.00302*Glu-0.00113*Cit+0.01111*Ile-0.01263*His-0.01536*Trp |
| 1.10765+0.00259*Tau-0.00244*Glu-0.00152*Val+0.01340*Ile-0.01119*His-0.01433*Trp |
| 0.89653+0.00260*Tau+0.00391*Ser+0.00018*Ala+0.00590*Leu-0.01617*His-0.01752*Trp |
| 1.50118+0.00274*Tau-0.00168*Asn-0.00408*Glu+0.00777*Leu-0.01842*His-0.01557*Trp |
| 1.54085+0.00271*Tau-0.00407*Glu-0.00023*Gln+0.00769*Leu-0.01811*His-0.01549*Trp |
| 1.46990+0.00272*Tau-0.00404*Glu-0.00038*Pro+0.00784*Leu-0.01808*His-0.01587*Trp |
| 1.14403+0.00258*Tau-0.00300*Glu+0.01106*Ile-0.01280*His-0.01547*Trp |
| 1.14389+0.00258*Tau-0.00300*Glu+0.01105*Ile+0.00002*Phe-0.01280*His-0.01548*Trp |
| 1.15523+0.00258*Tau-0.00313*Glu+0.01150*Ile-0.01239*His-0.01511*Trp-0.00085*Arg |
| 1.08152+0.00212*Tau+0.00212*Gly+0.00725*Leu+0.00162*Phe-0.02302*His-0.00387*Lys |
| 1.48749+0.00279*Tau-0.00359*Glu-0.00209*Val+0.00999*Leu-0.01783*His-0.01453*Trp |
| 1.04920+0.00238*Tau+0.00020*Ala-0.00217*Val+0.01326*Ile-0.01061*His-0.01443*Trp |
| 0.96053+0.00240*Tau-0.00244*Asn+0.00106*Gly+0.00966*Ile-0.01417*His-0.01352*Trp |
| 1.36597+0.00245*Tau+0.00062*Ala-0.00325*Val+0.00982*Leu-0.01729*His-0.01472*Trp |
| 1.09237+0.00218*Tau+0.00220*Gly+0.00733*Leu-0.02345*His-0.00440*Lys+0.00193*Arg |
| 0.89231+0.00263*Tau+0.00412*Ser+0.00600*Leu-0.01586*His-0.01744*Trp |
| 0.85494+0.00262*Tau+0.00415*Ser+0.00573*Leu+0.00159*Phe-0.01609*His-0.01761*Trp |
| 0.89981+0.00263*Tau+0.00414*Ser+0.00613*Leu-0.01573*His-0.01728*Trp-0.00044*Arg |
| 1.17777+0.00236*Tau+0.00044*Asn+0.01244*Ile-0.01138*His-0.01436*Trp-0.00217*Lys |
| 1.18345+0.00237*Tau+0.00039*Cit+0.01245*Ile-0.01145*His-0.01433*Trp-0.00215*Lys |
| 0.67471+0.00304*Tau+0.00423*Ser-0.00658*Asn+0.01268*Ile-0.01200*His-0.01629*Trp |
| 0.57698+0.00290*Tau+0.00344*Ser+0.01194*Ile-0.01179*His-0.01746*Trp |

## FIG.187

| FORMULA |
|---|
| 5.22136+0.02535*Tau+0.03160*Ser-0.05816*Asn+0.06723*Leu-0.12392*His-0.16120*Trp |
| 8.69320+0.02528*Tau-0.16064*Met+0.07992*Leu-0.15666*His-0.16319*Trp-0.03051*Lys |
| 6.86680+0.02388*Tau-0.05946*Asn+0.01173*Gly+0.07116*Leu-0.16054*His-0.13395*Trp |
| 7.13966+0.02376*Tau-0.02805*Val+0.13728*Met+0.08942*Leu-0.13844*His-0.16655*Trp |
| 4.60075+0.02370*Tau+0.01503*Ser+0.09782*Met+0.05754*Leu-0.12911*His-0.18483*Trp |
| 2.73148+0.02421*Tau+0.02676*Ser-0.04854*Asn+0.09912*Ile-0.07019*His-0.16251*Trp |
| 4.65609+0.02117*Tau+0.12399*Ile+0.04252*Tyr-0.07650*His-0.14460*Trp-0.02660*Lys |
| 7.44195+0.02353*Tau-0.03114*Asn+0.10358*Met+0.06227*Leu-0.14341*His-0.17326*Trp |
| 7.33305+0.02239*Tau+0.02216*Thr+0.07623*Leu-0.12928*His-0.15751*Trp-0.02329*Lys |
| 6.36135+0.02267*Tau+0.11926*Met+0.05842*Leu-0.00863*Phe-0.13514*His-0.17925*Trp |
| 5.83394+0.02138*Tau+0.11223*Met+0.05068*Leu+0.02143*Tyr-0.13389*His-0.18136*Trp |
| 5.83721+0.02325*Tau+0.03232*Cit+0.11295*Met+0.05762*Leu-0.14114*His-0.18557*Trp |
| 5.67557+0.02240*Tau+0.07851*Met+0.11006*Ile-0.07861*His-0.14408*Trp-0.02630*Lys |
| 7.44747+0.02264*Tau+0.01687*Thr-0.04843*Asn+0.06599*Leu-0.13250*His-0.16681*Trp |
| 5.40151+0.02270*Tau+0.00563*Gly+0.08436*Met+0.05948*Leu-0.14433*His-0.16815*Trp |
| 6.42882+0.02295*Tau+0.01209*Gly+0.07835*Leu-0.15401*His-0.12001*Trp-0.02128*Lys |
| 6.64873+0.02302*Tau+0.12092*Met+0.06413*Leu-0.13677*His-0.16935*Trp-0.01992*Arg |
| 6.70208+0.02304*Tau-0.00898*Pro+0.12649*Met+0.08516*Leu-0.13722*His-0.18429*Trp |
| 6.28778+0.02257*Tau-0.00020*Ala+0.11038*Met+0.05768*Leu-0.13586*His-0.17850*Trp |
| 0.92794+0.02326*Tau+0.03037*Ser-0.01049*Pro+0.10523*Ile-0.05330*His-0.17370*Trp |
| 6.10041+0.02247*Tau+0.00336*Thr+0.10068*Met+0.05763*Leu-0.13467*His-0.18097*Trp |
| 6.26871+0.02253*Tau+0.10972*Met+0.05753*Leu-0.13594*His-0.17862*Trp |
| 3.99178+0.02234*Tau+0.01149*Ser+0.00804*Gly+0.06105*Leu-0.13248*His-0.15041*Trp |
| 5.95563+0.02258*Tau+0.10113*Met+0.01234*Ile+0.05145*Leu-0.13010*His-0.17832*Trp |
| 3.79265+0.01940*Tau+0.06553*Met+0.07068*Ile+0.03630*Tyr-0.08769*His-0.17244*Trp |
| 4.91211+0.02150*Tau+0.00673*Thr+0.00794*Gly+0.06110*Leu-0.13638*His-0.15296*Trp |
| 5.12719+0.02201*Tau-0.01074*Pro+0.01252*Gly+0.07182*Leu-0.14450*His-0.14161*Trp |
| 3.01799+0.02089*Tau+0.00589*Gly+0.01913*Cit+0.08889*Ile-0.08542*His-0.14960*Trp |
| 2.44046+0.02344*Tau+0.02170*Ser+0.06297*Ile+0.02704*Leu-0.08856*His-0.17243*Trp |
| 4.23157+0.02235*Tau+0.00754*Gly+0.12216*Ile-0.08183*His-0.12382*Trp-0.02526*Lys |
| 2.83102+0.01961*Tau+0.00555*Gly+0.08383*Ile+0.03010*Tyr-0.08778*His-0.15432*Trp |
| 5.91482+0.02328*Tau+0.09691*Ile+0.02737*Leu-0.09284*His-0.14227*Trp-0.02280*Lys |
| 4.03739+0.02206*Tau+0.00793*Gly+0.04585*Ile+0.03814*Leu-0.11940*His-0.15098*Trp |
| 4.80017+0.02182*Tau+0.00958*Gly+0.01823*Cit+0.06167*Leu-0.14230*His-0.14561*Trp |
| 5.11365+0.02075*Tau-0.05335*Asn+0.09527*Ile+0.04494*Tyr-0.08842*His-0.15822*Trp |
| 5.01919+0.02147*Tau+0.00968*Gly+0.06163*Leu-0.13989*His-0.14213*Trp |
| 4.86571+0.02088*Tau+0.00942*Gly+0.05839*Leu+0.00892*Tyr-0.13819*His-0.14323*Trp |
| 5.04209+0.02164*Tau+0.01011*Gly-0.00092*Ala+0.06248*Leu-0.14000*His-0.14048*Trp |
| 3.39037+0.02233*Tau+0.06437*Cit+0.11285*Ile-0.07231*His-0.14956*Trp-0.03038*Arg |
| 4.43029+0.02081*Tau+0.05870*Met+0.08949*Ile-0.07458*His-0.15232*Trp-0.01774*Arg |
| 5.16558+0.02155*Tau+0.00961*Gly-0.00388*Val+0.06607*Leu-0.13947*His-0.13944*Trp |
| 5.85055+0.02271*Tau-0.02497*Asn+0.12652*Ile-0.07299*His-0.13506*Trp-0.02007*Lys |
| 4.51895+0.02181*Tau-0.04371*Asn+0.00722*Gly+0.09451*Ile-0.09118*His-0.13766*Trp |
| 3.59422+0.02050*Tau+0.00462*Gly+0.03379*Met+0.08105*Ile-0.08483*His-0.15230*Trp |
| 3.25124+0.02051*Tau+0.00600*Gly+0.08874*Ile-0.08291*His-0.14590*Trp |

# FIG.188

| FORMULA |
|---|
| 3.24992+0.02049*Tau+0.00064*Thr+0.00585*Gly+0.08838*Ile-0.08259*His-0.14675*Trp |
| 4.75594+0.02158*Tau+0.00949*Gly+0.05925*Leu+0.02007*Phe-0.14482*His-0.14698*Trp |
| 5.25350+0.02160*Tau+0.00961*Gly+0.06517*Leu-0.13923*His-0.13562*Trp-0.01054*Arg |
| 3.23671+0.02042*Tau+0.00603*Gly+0.08610*Ile+0.00584*Phe-0.08482*His-0.14626*Trp |
| 3.24517+0.02051*Tau+0.00609*Gly+0.00104*Val+0.08708*Ile-0.08412*His-0.14657*Trp |
| 3.33737+0.01930*Tau-0.01563*Val+0.11131*Ile+0.04066*Tyr-0.06419*His-0.15300*Trp |
| 4.37805+0.02061*Tau+0.06306*Met+0.08136*Ile-0.00793*Phe-0.07803*His-0.16222*Trp |
| 4.88688+0.02202*Tau+0.01159*Thr+0.12099*Ile-0.06777*His-0.14565*Trp-0.02572*Lys |
| 5.40695+0.02196*Tau-0.03767*Asn+0.11147*Ile-0.07225*His-0.13890*Trp-0.01808*Arg |
| 3.99466+0.02102*Tau+0.02687*Cit+0.05861*Met+0.07906*Ile-0.08378*His-0.16715*Trp |
| 3.65329+0.01985*Tau+0.09853*Ile+0.03148*Tyr-0.07355*His-0.15385*Trp-0.01586*Arg |
| 3.38240+0.02077*Tau+0.00556*Gly+0.09777*Ile-0.07772*His-0.13847*Trp-0.01451*Arg |
| 3.29398+0.01929*Tau+0.00728*Thr+0.08298*Ile+0.03306*Tyr-0.07814*His-0.17020*Trp |
| 3.15357+0.02066*Tau-0.00838*Pro+0.00779*Gly+0.09839*Ile-0.07897*His-0.14487*Trp |
| 5.10158+0.02210*Tau-0.00260*Val+0.12904*Ile-0.06655*His-0.13532*Trp-0.02191*Lys |
| 5.25535+0.02226*Tau-0.00554*Pro+0.13378*Ile-0.06441*His-0.13769*Trp-0.02274*Lys |
| 4.29996+0.02050*Tau-0.00481*Val+0.05501*Met+0.08730*Ile-0.07461*His-0.15801*Trp |
| 5.07079+0.02209*Tau+0.12622*Ile-0.06774*His-0.13545*Trp-0.02037*Lys-0.00555*Arg |
| 2.36001+0.01955*Tau-0.03378*Val+0.16626*Ile+0.05399*Tyr-0.13297*Trp-0.02704*Lys |
| 3.57544+0.01943*Tau-0.00844*Pro+0.09896*Ile+0.04100*Tyr-0.07307*His-0.16668*Trp |
| 3.91237+0.02008*Tau+0.07857*Ile+0.01036*Leu+0.02840*Tyr-0.08680*His-0.16402*Trp |
| 4.18084+0.02220*Tau+0.01060*Thr+0.01628*Ser+0.05860*Leu-0.11371*His-0.17367*Trp |
| 5.11700+0.02203*Tau+0.12297*Ile+0.00531*Phe-0.07085*His-0.13753*Trp-0.02211*Lys |
| 5.22397+0.02135*Tau-0.02869*Asn+0.04449*Met+0.08738*Ile-0.08220*His-0.15608*Trp |
| 8.15827+0.02164*Tau-0.05486*Asn+0.00509*Ala+0.06300*Leu-0.13813*His-0.14926*Trp |
| 3.56516+0.01954*Tau+0.08901*Ile+0.03221*Tyr-0.07852*His-0.16286*Trp |
| 3.21675+0.02096*Tau+0.00685*Gly-0.00212*Ala+0.09278*Ile-0.08148*His-0.14296*Trp |
| 5.12529+0.02211*Tau+0.12532*Ile-0.06918*His-0.13718*Trp-0.02209*Lys |
| 4.36500+0.02065*Tau-0.00073*Ala+0.05679*Met+0.08118*Ile-0.07874*His-0.16135*Trp |
| 4.25243+0.02044*Tau+0.00164*Thr+0.05102*Met+0.07972*Ile-0.07888*His-0.16280*Trp |
| 3.58947+0.01978*Tau-0.00142*Ala+0.09193*Ile+0.03418*Tyr-0.07694*His-0.16222*Trp |
| 5.10798+0.02200*Tau+0.00057*Ala+0.12415*Ile-0.06995*His-0.13756*Trp-0.02220*Lys |
| 6.27324+0.02298*Tau-0.04092*Asn+0.06837*Ile+0.03065*Leu-0.10484*His-0.15480*Trp |
| 4.32972+0.02049*Tau+0.05532*Met+0.07989*Ile-0.07955*His-0.16175*Trp |
| 4.47051+0.02060*Tau-0.00609*Pro+0.06236*Met+0.08632*Ile-0.07532*His-0.16379*Trp |
| 3.90184+0.02060*Tau+0.00749*Thr+0.09912*Ile-0.06654*His-0.15209*Trp-0.01760*Arg |
| 4.72988+0.02307*Tau+0.03922*Cit+0.12876*Ile-0.07436*His-0.14252*Trp-0.02453*Lys |
| 3.55377+0.01945*Tau+0.08648*Ile+0.03232*Tyr+0.00562*Phe-0.08032*His-0.16328*Trp |
| 3.28325+0.01997*Tau+0.02170*Cit+0.08906*Ile+0.03217*Tyr-0.08158*His-0.16687*Trp |
| 6.01524+0.02110*Tau+0.01663*Thr+0.06420*Leu-0.11859*His-0.16201*Trp-0.01623*Arg |
| 7.96476+0.02053*Tau-0.05001*Asn+0.05739*Leu+0.02785*Tyr-0.12893*His-0.14476*Trp |
| 4.64104+0.02155*Tau+0.01025*Thr+0.05064*Ile+0.03337*Leu-0.10068*His-0.17146*Trp |
| 5.03793+0.02207*Tau-0.01637*Val+0.07877*Ile+0.03810*Leu-0.08888*His-0.14980*Trp |
| 5.03793+0.02207*Tau-0.01637*Val+0.07877*Ile+0.03810*Leu-0.08888*His-0.14980*Trp |

## FIG.189

# FIG.190

| FORMULA |
|---|
| 0.76027+0.00263*Tau+0.00417*Ser+0.01200*Ile-0.01038*His-0.01472*Trp-0.00258*Lys |
| 0.76055+0.00275*Tau+0.00349*Ser-0.00239*Glu+0.01009*Ile-0.01205*His-0.01630*Trp |
| 1.18772+0.00251*Tau+0.00166*Gly+0.00828*Leu-0.01978*His-0.01144*Trp-0.00257*Lys |
| 0.98545+0.00240*Tau+0.00120*Gly+0.01217*Ile-0.01310*His-0.01135*Trp-0.00265*Lys |
| 1.16586+0.00274*Tau-0.00321*Glu+0.00122*Gly+0.00761*Leu-0.02026*His-0.01400*Trp |
| 0.67569+0.00263*Tau+0.00361*Ser-0.00186*Val+0.01240*Ile-0.00985*His-0.01534*Trp |
| 0.72353+0.00256*Tau+0.00412*Ser-0.00541*ABA+0.00929*Ile-0.01144*His-0.01649*Trp |
| 1.06529+0.00247*Tau-0.00097*Pro+0.00158*Gly+0.00725*Leu-0.01922*His-0.01385*Trp |
| 0.74425+0.00264*Tau+0.00422*Ser-0.00380*Asn+0.00962*Ile-0.01163*His-0.01629*Trp |
| 0.63594+0.00261*Tau+0.00448*Ser-0.00114*Pro+0.01043*Ile-0.01044*His-0.01709*Trp |
| 0.66372+0.00255*Tau+0.00307*Ser+0.00055*Gly+0.00906*Ile-0.01273*His-0.01567*Trp |
| 1.00221+0.00291*Tau+0.00379*Ser-0.00334*Glu+0.00712*Leu-0.01716*His-0.01688*Trp |
| 0.86580+0.00265*Tau+0.00484*Ser-0.00085*Gln+0.01014*Ile-0.01064*His-0.01599*Trp |
| 0.95589+0.00272*Tau+0.00467*Ser-0.00437*Asn+0.00635*Leu-0.01602*His-0.01679*Trp |
| 1.11582+0.00245*Tau+0.00139*Gly-0.00446*ABA+0.00675*Leu-0.01936*His-0.01383*Trp |
| 0.69435+0.00258*Tau+0.00374*Ser+0.00929*Ile-0.01171*His-0.01694*Trp |
| 0.69338+0.00258*Tau+0.00375*Ser+0.00006*Cit+0.00929*Ile-0.01172*His-0.01694*Trp |
| 1.14114+0.00251*Tau-0.00348*Asn+0.00148*Gly+0.00693*Leu-0.01980*His-0.01342*Trp |
| 0.99725+0.00268*Tau+0.00457*Ser+0.00733*Leu-0.01565*His-0.01562*Trp-0.00215*Lys |
| 0.97225+0.00255*Tau-0.00231*Glu+0.00086*Gly+0.01025*Ile-0.01411*His-0.01386*Trp |
| 1.23359+0.00259*Tau-0.00293*Glu+0.01331*Ile-0.01177*His-0.01362*Trp-0.00209*Lys |
| 0.82466+0.00263*Tau+0.00296*Ser+0.00091*Gly+0.00629*Leu-0.01784*His-0.01566*Trp |
| 1.24987+0.00247*Tau-0.00065*Gln+0.00165*Gly+0.00687*Leu-0.01935*His-0.01266*Trp |
| 1.12863+0.00257*Tau+0.00130*Gly-0.00224*Val+0.00931*Leu-0.01891*His-0.01282*Trp |
| 0.77249+0.00227*Tau+0.00368*Ser-0.00356*Val+0.01654*Ile-0.01032*His-0.00351*Lys |
| 0.68932+0.00257*Tau+0.00376*Ser+0.00910*Ile+0.00042*Phe-0.01181*His-0.01696*Trp |
| 1.06501+0.00244*Tau+0.00129*Gly+0.00014*Ala+0.00648*Leu-0.01943*His-0.01444*Trp |
| 1.08456+0.00244*Tau+0.00137*Gly-0.00149*Cit+0.00662*Leu-0.01916*His-0.01406*Trp |
| 0.68722+0.00260*Tau+0.00384*Ser-0.00009*Ala+0.00948*Ile-0.01152*His-0.01693*Trp |
| 0.69880+0.00257*Tau+0.00377*Ser+0.00959*Ile-0.01139*His-0.01669*Trp-0.00065*Arg |
| 1.06619+0.00247*Tau+0.00136*Gly+0.00659*Leu-0.01935*His-0.01423*Trp |
| 1.11784+0.00217*Tau-0.00123*Pro+0.00239*Gly+0.00843*Leu-0.02246*His-0.00381*Lys |
| 0.92797+0.00214*Tau+0.00144*Gly-0.00207*Val+0.01450*Ile-0.01397*His-0.00353*Lys |
| 1.04025+0.00245*Tau+0.00135*Gly+0.00637*Leu+0.00130*Phe-0.01952*His-0.01438*Trp |
| 1.06788+0.00246*Tau+0.00136*Gly+0.00661*Leu-0.01932*His-0.01421*Trp-0.00007*Arg |
| 1.13740+0.00255*Tau-0.00294*Glu+0.00016*Ala+0.01068*Ile-0.01309*His-0.01553*Trp |
| 0.97574+0.00248*Tau+0.00120*Gly+0.00439*Ile+0.00424*Leu-0.01749*His-0.01466*Trp |
| 1.39786+0.00265*Tau-0.00388*Glu+0.00045*Ala+0.00726*Leu-0.01891*His-0.01622*Trp |
| 0.75527+0.00265*Tau+0.00385*Ser+0.00592*Ile+0.00290*Leu-0.01381*His-0.01761*Trp |
| 0.91265+0.00206*Tau-0.00120*Pro+0.00191*Gly+0.01237*Ile-0.01566*His-0.00388*Lys |
| 0.60245+0.00278*Tau+0.00522*Ser-0.00116*Gln-0.00429*Val+0.01717*Ile-0.01517*Trp |
| 1.25471+0.00274*Tau-0.00375*Glu+0.00669*Ile+0.00398*Leu-0.01580*His-0.01618*Trp |
| 1.16980+0.00261*Tau-0.00381*Val+0.00978*Ile+0.00576*Leu-0.01278*His-0.01427*Trp |
| 0.92588+0.00263*Tau+0.00458*Ser-0.00682*ABA+0.00621*Leu-0.01569*His-0.01701*Trp |
| 0.86251+0.00267*Tau+0.00482*Ser-0.00102*Pro+0.00660*Leu-0.01513*His-0.01759*Trp |

## FIG.191

| FORMULA |
|---|
| 0.95335+0.00276*Tau+0.00409*Ser-0.00260*Val+0.00917*Leu-0.01549*His-0.01568*Trp |
| 1.04614+0.00267*Tau+0.00502*Ser-0.00067*Gln+0.00616*Leu-0.01509*His-0.01654*Trp |
| 0.90897+0.00241*Tau+0.00086*Gly-0.00136*Val-0.01179*Ile-0.01245*His-0.01326*Trp |
| 1.14800+0.00243*Tau-0.00203*Val+0.01572*Ile-0.00938*His-0.01266*Trp-0.00205*Lys |
| 0.74079+0.00214*Tau+0.00231*Ser+0.00142*Gly+0.01070*Ile-0.01601*His-0.00404*Lys |
| 0.89285+0.00237*Tau-0.00093*Pro+0.00117*Gly+0.01037*Ile-0.01341*His-0.01379*Trp |
| 1.18086+0.00232*Tau+0.00025*Ala+0.01192*Ile-0.01185*His-0.01433*Trp-0.00218*Lys |
| 1.25856+0.00243*Tau-0.00937*Ile+0.00272*Leu-0.01336*His-0.01485*Trp-0.00220*Lys |
| 1.19778+0.00236*Tau-0.00055*Pro+0.01307*Ile-0.01085*His-0.01430*Trp-0.00214*Lys |
| 0.94522+0.00235*Tau+0.00101*Gly-0.00306*ABA+0.00947*Ile-0.01396*His-0.01382*Trp |
| 1.56815+0.00273*Tau-0.00405*Glu+0.00861*Leu-0.01822*His-0.01444*Trp-0.00160*Lys |
| 0.91747+0.00208*Tau+0.00179*Gly+0.01071*Ile-0.01743*His-0.00438*Lys+0.00173*Arg |
| 1.15388+0.00258*Tau-0.00302*Glu-0.00055*Pro+0.01169*Ile-0.01226*His-0.01547*Trp |
| 0.92783+0.00235*Tau+0.00101*Gly-0.00155*Cit+0.00949*Ile-0.01383*His-0.01397*Trp |
| 1.17761+0.00259*Tau-0.00125*Asn-0.00304*Glu+0.01121*Ile-0.01280*His-0.01522*Trp |
| 1.15141+0.00212*Tau+0.00212*Gly-0.00417*ABA+0.00756*Leu-0.02270*His-0.00361*Lys |
| 0.33519+0.00266*Tau+0.00508*Ser-0.00217*Asn+0.01222*Ile-0.01910*Trp-0.60333*Lys |
| 0.90965+0.00237*Tau+0.00099*Gly+0.00944*Ile-0.01404*His-0.01415*Trp |
| 0.91299+0.00237*Tau+0.00099*Gly+0.00955*Ile-0.01393*His-0.01407*Trp-0.00022*Arg |
| 0.90781+0.00238*Tau+0.00101*Gly-0.00005*Ala+0.00953*Ile-0.01399*His-0.01410*Trp |
| 0.90754+0.00237*Tau+0.00099*Gly+0.00933*Ile+0.00024*Phe-0.01411*His-0.01416*Trp |
| 1.14925+0.00257*Tau-0.00295*Glu-0.00048*ABA+0.01105*Ile-0.01277*His-0.01544*Trp |
| 1.31674+0.00218*Tau-0.00075*Gln+0.00237*Gly+0.00767*Leu-0.02234*His-0.00327*Lys |
| 1.15954+0.00257*Tau-0.00302*Glu-0.00113*Cit+0.01111*Ile-0.01263*His-0.01536*Trp |
| 1.10765+0.00259*Tau-0.00244*Glu-0.00152*Val+0.01340*Ile-0.01119*His-0.01433*Trp |
| 0.89653+0.00260*Tau+0.00391*Ser+0.00018*Ala+0.00590*Leu-0.01617*His-0.01752*Trp |
| 1.50118+0.00274*Tau-0.00168*Asn-0.00408*Glu+0.00777*Leu-0.01842*His-0.01557*Trp |
| 1.54085+0.00271*Tau-0.00407*Glu-0.00023*Gln+0.00769*Leu-0.01811*His-0.01549*Trp |
| 1.46990+0.00272*Tau-0.00404*Glu-0.00038*Pro+0.00784*Leu-0.01808*His-0.01587*Trp |
| 1.14403+0.00258*Tau-0.00300*Glu+0.01106*Ile-0.01280*His-0.01547*Trp |
| 1.14389+0.00258*Tau-0.00300*Glu+0.01105*Ile+0.00002*Phe-0.01280*His-0.01548*Trp |
| 1.15523+0.00258*Tau-0.00313*Glu+0.01150*Ile-0.01239*His-0.01511*Trp-0.00085*Arg |
| 1.08152+0.00212*Tau+0.00212*Gly+0.00725*Leu+0.00162*Phe-0.02302*His-0.00387*Lys |
| 1.48749+0.00279*Tau-0.00359*Glu-0.00209*Val+0.00999*Leu-0.01783*His-0.01453*Trp |
| 1.04920+0.00238*Tau+0.00020*Ala-0.00217*Val+0.01326*Ile-0.01061*His-0.01443*Trp |
| 0.96053+0.00240*Tau-0.00244*Asn-0.00106*Gly+0.00966*Ile-0.01417*His-0.01352*Trp |
| 1.36597+0.00245*Tau-0.00062*Ala-0.00325*Val+0.00982*Leu-0.01729*His-0.01472*Trp |
| 1.09237+0.00218*Tau+0.00220*Gly+0.00733*Leu-0.02345*His-0.00440*Lys+0.00193*Arg |
| 0.89231+0.00263*Tau+0.00412*Ser+0.00600*Leu-0.01586*His-0.01744*Trp |
| 0.85494+0.00262*Tau+0.00415*Ser+0.00573*Leu+0.00159*Phe-0.01609*His-0.01761*Trp |
| 0.89981+0.00263*Tau+0.00414*Ser+0.00613*Leu-0.01573*His-0.01728*Trp-0.00044*Arg |
| 1.17777+0.00236*Tau+0.00044*Asn+0.01244*Ile-0.01138*His-0.01436*Trp-0.00217*Lys |
| 1.18345+0.00237*Tau+0.00039*Cit+0.01245*Ile-0.01145*His-0.01433*Trp-0.00215*Lys |
| 0.67471+0.00304*Tau+0.00423*Ser-0.00658*Asn+0.01268*Ile-0.01200*His-0.01629*Trp |
| 0.57698+0.00290*Tau+0.00344*Ser+0.01194*Ile-0.01179*His-0.01746*Trp |

# FIG.192

| FORMULA |
|---|
| 0.57768+0.00292*Tau+0.00336*Ser-0.00137*Val+0.01404*Ile-0.01042*His-0.01643*Trp |
| 1.43215+0.00272*Tau-0.01927*Met+0.00815*Leu-0.01969*His-0.01579*Trp-0.00331*Lys |
| 0.50045+0.00296*Tau+0.00435*Ser-0.00110*Pro+0.01302*Ile-0.01053*His-0.01774*Trp |
| 0.57862+0.00288*Tau+0.00358*Ser+0.01320*Ile-0.01081*His-0.01594*Trp-0.00256*Arg |
| 1.30484+0.00282*Tau-0.00385*Val+0.01805*Met+0.01024*Leu-0.01864*His-0.01598*Trp |
| 0.65039+0.00284*Tau+0.00291*Ser+0.00493*Met+0.01066*Ile-0.01253*His-0.01766*Trp |
| 1.24976+0.00265*Tau-0.01617*Met+0.00689*Leu-0.01868*His-0.01653*Trp-0.00278*Arg |
| 1.30697+0.00272*Tau-0.00333*Asn+0.01444*Met+0.00650*Leu-0.01936*His-0.01736*Trp |
| 0.78475+0.00263*Tau+0.00103*Gly+0.01272*Ile-0.01316*His-0.01300*Trp-0.00241*Arg |
| 1.11685+0.00263*Tau+0.01130*Met+0.01283*Ile-0.01258*His-0.01494*Trp-0.00294*Lys |
| 1.23363+0.00267*Tau-0.00085*Pro+0.01658*Met+0.00653*Leu-0.01884*His-0.01829*Trp |
| 0.55564+0.00298*Tau+0.00372*Ser-0.00028*Ala+0.01253*Ile-0.01127*His-0.01740*Trp |
| 1.34786+0.00265*Tau+0.00293*Thr+0.00875*Leu-0.01787*His-0.01596*Trp-0.00306*Lys |
| 0.99014+0.00259*Tau+0.00947*Met+0.01124*Ile-0.01263*His-0.01573*Trp-0.00259*Arg |
| 0.57136+0.00289*Tau+0.00347*Ser+0.01169*Ile+0.00053*Phe-0.01193*His-0.01749*Trp |
| 1.20276+0.00267*Tau+0.01470*Met+0.00613*Leu-0.01894*His-0.01804*Trp |
| 1.16145+0.00279*Tau-0.00651*Asn+0.00162*Gly+0.00771*Leu-0.02118*His-0.01188*Trp |
| 0.92146+0.00287*Tau+0.00267*Ser+0.01154*Met+0.00629*Leu-0.01820*His-0.01839*Trp |
| 1.04823+0.00270*Tau+0.00086*Gly+0.01076*Met+0.00634*Leu-0.02002*His-0.01597*Trp |
| 0.87403+0.00269*Tau+0.00122*Gly+0.01468*Ile-0.01312*His-0.01159*Trp-0.00292*Lys |
| 0.77201+0.00266*Tau+0.00101*Gly+0.01153*Ile-0.01402*His-0.01449*Trp |
| 0.55585+0.00292*Tau+0.00343*Ser+0.00147*Cit+0.01193*Ile-0.01196*His-0.01767*Trp |
| 1.07360+0.00269*Tau+0.01140*Met+0.00510*Ile+0.00370*Leu-0.01669*His-0.01798*Trp |
| 0.57995+0.00289*Tau+0.00006*Thr+0.00339*Ser+0.01191*Ile-0.01180*His-0.01748*Trp |
| 0.88512+0.00236*Tau+0.00893*Met+0.00902*Ile+0.00444*Tyr-0.01387*His-0.01815*Trp |
| 1.17539+0.00266*Tau+0.00058*Thr+0.01307*Met+0.00614*Leu-0.01878*His-0.01833*Trp |
| 1.11413+0.00271*Tau-0.00249*Asn+0.01558*Ile-0.01150*His-0.01423*Trp-0.00228*Lys |
| 0.56844+0.00284*Tau+0.00257*Ser+0.00060*Gly+0.01147*Ile-0.01293*His-0.01597*Trp |
| 1.00098+0.00268*Tau-0.00089*Pro+0.00165*Gly+0.00756*Leu-0.01987*His-0.01310*Trp |
| 1.13551+0.00246*Tau+0.01458*Met+0.00526*Leu+0.00301*Tyr-0.01848*His-0.01837*Trp |
| 0.61700+0.00296*Tau+0.00356*Ser+0.00947*Ile+0.00210*Leu-0.01339*His-0.01780*Trp |
| 0.90345+0.00259*Tau+0.00149*Thr+0.01282*Ile-0.01143*His-0.01599*Trp-0.00280*Arg |
| 0.75292+0.00265*Tau-0.00091*Pro+0.00122*Gly+0.01234*Ile-0.01345*His-0.01410*Trp |
| 1.22000+0.00269*Tau+0.01643*Met+0.00626*Leu-0.00148*Phe-0.01886*His-0.01807*Trp |
| 0.89769+0.00273*Tau-0.00550*Asn+0.00117*Gly+0.01211*Ile-0.01457*His-0.01303*Trp |
| 0.82514+0.00263*Tau+0.00080*Gly+0.00501*Met+0.01035*Ile-0.01433*His-0.01529*Trp |
| 1.01538+0.00264*Tau+0.00193*Thr+0.01468*Ile-0.01117*His-0.01533*Trp-0.00314*Lys |
| 1.05398+0.00268*Tau+0.01546*Ile-0.01130*His-0.01459*Trp-0.00246*Lys |
| 0.99628+0.00242*Tau-0.00594*Asn+0.01209*Ile+0.00553*Tyr-0.01308*His-0.01655*Trp |
| 1.17009+0.00270*Tau-0.00212*Cit+0.01476*Met+0.00611*Leu-0.01919*His-0.01836*Trp |
| 1.05921+0.00268*Tau-0.00380*Asn+0.01427*Ile-0.01169*His-0.01451*Trp-0.00227*Arg |
| 1.15547+0.00272*Tau+0.00171*Gly+0.00891*Leu-0.02074*His-0.01065*Trp-0.00270*Lys |
| 0.77419+0.00265*Tau+0.00033*Thr+0.00092*Gly+0.01136*Ile-0.01391*His-0.01491*Trp |
| 1.19662+0.00265*Tau+0.00007*Ala+0.01445*Met+0.00610*Leu-0.01901*His-0.01807*Trp |
| 1.33340+0.00271*Tau+0.00244*Thr-0.00628*Asn+0.00735*Leu-0.01840*His-0.01697*Trp |

# FIG.193

| FORMULA |
|---|
| 1.00478+0.00268*Tau+0.00142*Gly+0.00703*Leu-0.01998*His-0.01352*Trp |
| 0.77739+0.00267*Tau+0.00095*Gly-0.00089*Val+0.01295*Ile-0.01302*His-0.01396*Trp |
| 1.05881+0.00266*Tau-0.00329*Asn+0.00809*Met+0.01078*Ile-0.01358*His-0.01653*Trp |
| 0.76678+0.00266*Tau+0.00100*Gly+0.00043*Cit+0.01153*Ile-0.01406*His-0.01457*Trp |
| 0.96236+0.00264*Tau-0.00163*Val+0.00881*Met+0.01261*Ile-0.01186*His-0.01604*Trp |
| 1.06473+0.00267*Tau-0.00050*Pro+0.01608*Ile-0.01077*His-0.01459*Trp-0.00249*Lys |
| 0.71039+0.00244*Tau+0.00093*Gly+0.01068*Ile+0.00385*Tyr-0.01419*His-0.01543*Trp |
| 0.97025+0.00261*Tau+0.00871*Met+0.01016*Ile-0.01350*His-0.01725*Trp |
| 0.95464+0.00263*Tau+0.01378*Ile-0.01148*His-0.01526*Trp-0.00232*Arg |
| 1.04136+0.00273*Tau-0.00400*Asn-0.00161*Val+0.01561*Ile-0.01092*His-0.01465*Trp |
| 1.04804+0.00270*Tau-0.00395*Asn+0.01315*Ile-0.01255*His-0.01585*Trp |
| 0.95760+0.00311*Tau-0.00502*Ser-0.00730*Asn+0.00779*Leu-0.01773*His-0.01608*Trp |
| 0.82386+0.00272*Tau+0.00112*Gly+0.00815*Ile+0.00280*Leu-0.01637*His-0.01487*Trp |
| 0.62787+0.00232*Tau-0.00491*Val+0.02185*Ile+0.00747*Tyr-0.01506*Trp-0.00330*Lys |
| 0.79137+0.00298*Tau+0.00493*Ser-0.00094*Pro+0.00759*Leu-0.01627*His-0.01746*Trp |
| 0.96882+0.00237*Tau+0.01467*Ile+0.00559*Tyr-0.01152*His-0.01529*Trp-0.00295*Lys |
| 0.95911+0.00299*Tau+0.00464*Ser+0.00866*Leu-0.01707*His-0.01536*Trp-0.00232*Lys |
| 0.98842+0.00260*Tau-0.00074*Pro+0.01012*Met+0.01060*Ile-0.01294*His-0.01739*Trp |
| 1.04593+0.00267*Tau+0.01554*Ile-0.01111*His-0.01432*Trp-0.00216*Lys-0.00085*Arg |
| 0.75536+0.00274*Tau+0.00117*Gly-0.00036*Ala+0.01219*Ile-0.01368*His-0.01398*Trp |
| 1.02747+0.00268*Tau+0.00152*Thr-0.00521*Asn+0.01210*Ile-0.01274*His-0.01663*Trp |
| 0.82029+0.00239*Tau-0.00259*Val+0.01523*Ile+0.00549*Tyr-0.01015*His-0.01584*Trp |
| 0.93921+0.00265*Tau+0.01262*Ile-0.01235*His-0.01666*Trp |
| 1.10995+0.00273*Tau+0.01319*Ile+0.00204*Leu-0.01282*His-0.01482*Trp-0.00255*Lys |
| 0.94708+0.00266*Tau-0.00125*Val+0.01559*Ile-0.01028*His-0.01444*Trp-0.00214*Arg |
| 1.04384+0.00270*Tau-0.00129*Val+0.01732*Ile-0.01003*His-0.01370*Trp-0.00238*Lys |
| 1.04608+0.00270*Tau-0.00365*Asn-0.00034*Pro+0.01350*Ile-0.01219*His-0.01593*Trp |
| 0.94574+0.00261*Tau-0.00053*Thr+0.00724*Met+0.01015*Ile-0.01335*His-0.01751*Trp |
| 0.77124+0.00265*Tau+0.00101*Gly+0.01145*Ile+0.00018*Phe-0.01407*His-0.01450*Trp |
| 0.98241+0.00264*Tau+0.01044*Met+0.01043*Ile-0.00163*Phe-0.01328*His-0.01727*Trp |
| 1.09906+0.00275*Tau-0.00418*Asn+0.01115*Ile+0.00175*Leu-0.01390*His-0.01607*Trp |
| 0.83729+0.00293*Tau+0.00410*Ser+0.00703*Leu-0.01693*His-0.01722*Trp |
| 0.88589+0.00266*Tau+0.00123*Thr-0.00168*Val+0.01417*Ile-0.01076*His-0.01626*Trp |
| 1.05323+0.00267*Tau+0.00003*Ala+0.01540*Ile-0.01134*His-0.01460*Trp-0.00246*Lys |
| 1.05422+0.00267*Tau-0.00435*Asn+0.00017*Ala+0.01281*Ile-0.01286*His-0.01584*Trp |
| 1.00453+0.00268*Tau+0.00142*Gly+0.00002*Cit+0.00703*Leu-0.01998*His-0.01352*Trp |
| 0.96076+0.00263*Tau-0.00037*Pro+0.01417*Ile-0.01113*His-0.01533*Trp-0.00224*Arg |
| 0.89587+0.00262*Tau+0.00119*Thr+0.01166*Ile-0.01245*His-0.01747*Trp |
| 1.03713+0.00266*Tau+0.00147*Gly+0.00769*Leu-0.01977*His-0.01210*Trp-0.00214*Arg |
| 0.99801+0.00267*Tau+0.00088*Thr+0.00117*Gly+0.00689*Leu-0.01955*His-0.01474*Trp |
| 1.05379+0.00268*Tau+0.01544*Ile+0.00005*Phe-0.01131*His-0.01459*Trp-0.00246*Lys |
| 1.22822+0.00270*Tau+0.00231*Thr-0.00246*Val+0.00948*Leu-0.01719*His-0.01645*Trp |
| 0.93124+0.00268*Tau-0.00158*Val+0.01502*Ile-0.01075*His-0.01549*Trp |
| 0.79645+0.00286*Tau+0.00262*Ser+0.00100*Gly+0.00700*Leu-0.01884*His-0.01503*Trp |
| 0.94244+0.00264*Tau+0.00190*Cit+0.00880*Met+0.01011*Ile-0.01373*His-0.01753*Trp |
| 1.01369+0.00272*Tau+0.00313*Cit+0.01557*Ile-0.01161*His-0.01494*Trp-0.00259*Lys |
| 0.90036+0.00262*Tau+0.00138*Thr-0.00063*Pro+0.01223*Ile-0.01182*His-0.01764*Trp |
| 0.97487+0.00264*Tau-0.00012*Ala+0.00899*Met+0.01036*Ile-0.01333*His-0.01721*Trp |
| 0.93850+0.00268*Tau-0.00037*Pro-0.00144*Val+0.01522*Ile-0.01052*His-0.01562*Trp |
| 0.81466+0.00295*Tau+0.00409*Ser+0.00155*Cit+0.00702*Leu-0.01710*His-0.01745*Trp |

# FIG.194

# FIG.195

| FOMULA |
| --- |
| 7.83480-0.01966*Val-0.07441*Met+0.02562*Leu-0.01729*Tyr-0.07701*His+0.00882*Arg |
| 8.08616-0.01797*Val-0.05806*Met+0.02282*Leu-0.00106*Phe-0.07356*His-0.02180*Trp |
| 7.95862+0.00421*Thr-0.01826*Val-0.07180*Met+0.02374*Leu-0.07575*His-0.02098*Trp |
| 8.22228-0.00350*Tau-0.01766*Val-0.05985*Met+0.02206*Leu-0.07177*His-0.02192*Trp |
| 8.12446-0.00332*Tau-0.01808*Val-0.05881*Met+0.02370*Leu-0.01531*Tyr-0.07491*His |
| 7.72425+0.00109*Gln-0.01771*Val-0.06328*Met+0.02252*Leu-0.07567*His-0.02253*Trp |
| 8.07167-0.01799*Val-0.05868*Met+0.02269*Leu-0.07360*His-0.02196*Trp |
| 8.08586-0.01625*Val-0.04730*Met+0.02178*Leu-0.01324*Tyr-0.07201*His-0.02022*Trp |
| 8.06529+0.00231*Glu-0.01879*Val-0.05830*Met+0.02344*Leu-0.07369*His-0.02221*Trp |
| 7.92428-0.01913*Val-0.07389*Met+0.02386*Leu-0.07397*His-0.02086*Trp+0.00655*Arg |
| 8.02916+0.02487*Asn-0.04746*ABA-0.01758*Val-0.09487*Met+0.02614*Leu-0.08119*His |
| 7.41725+0.02172*Asn-0.02108*Val-0.10548*Met+0.02706*Leu-0.08079*His+0.00554*Arg |
| 7.54033-0.00470*Tau-0.00493*Val-0.05001*Met-0.01996*Tyr-0.07607*His+0.00787*Arg |
| 7.18655+0.02974*Asn-0.05077*ABA-0.07034*Met-0.02327*Tyr+0.01508*Phe-0.08296*His |
| 8.34348-0.05029*ABA-0.01982*Val-0.09093*Met+0.02752*Leu-0.07965*His+0.00978*Arg |
| 7.83999-0.00053*Ala-0.02145*Val-0.08584*Met+0.02641*Leu-0.07939*His+0.00805*Arg |
| 7.95022+0.00831*Cit-0.01843*Val-0.05927*Met+0.02352*Leu-0.07492*His-0.02215*Trp |
| 8.00166-0.01836*Val-0.05720*Met+0.02437*Leu-0.01548*Tyr-0.00058*Phe-0.07671*His |
| 7.38947+0.03553*Asn-0.01663*Val-0.09095*Met+0.02286*Leu-0.07503*His-0.02634*Trp |
| 7.87624+0.00503*Thr-0.01866*Val-0.07201*Met+0.02544*Leu-0.01519*Tyr-0.07936*His |
| 7.73693-0.00958*Thr+0.04248*Asn-0.05441*ABA-0.01936*Tyr-0.07140*His-0.03792*Trp |
| 8.51416-0.04637*ABA-0.01628*Val-0.05671*Met+0.02442*Leu-0.01362*Tyr-0.07713*His |
| 7.66888+0.03713*Asn-0.05055*ABA-0.05316*Met-0.01454*Tyr-0.07341*His-0.03301*Trp |
| 7.80680-0.02197*Val-0.08919*Met+0.02700*Leu-0.07924*His+0.00770*Arg |
| 7.80238+0.00135*Glu-0.02245*Val-0.08912*Met+0.02746*Leu-0.07931*His+0.00769*Arg |
| 7.91916-0.02180*Val-0.08467*Met+0.02785*Leu-0.00693*Phe-0.07903*His+0.00791*Arg |
| 7.44145+0.02801*Asn-0.01753*Val-0.08332*Met+0.02476*Leu-0.01676*Tyr-0.07876*His |
| 7.75943+0.00074*Gln-0.01829*Val-0.06089*Met+0.02425*Leu-0.01516*Tyr-0.07842*His |
| 8.04671+0.00033*Ala-0.01827*Val-0.06099*Met+0.02300*Leu-0.07335*His-0.02247*Trp |
| 7.84104-0.00348*Cit-0.02187*Val-0.09060*Met+0.02672*Leu-0.07864*His+0.00844*Arg |
| 7.79488-0.00526*Tau+0.03998*Asn-0.05361*ABA-0.07233*Met-0.07350*His-0.03575*Trp |
| 7.61848+0.00062*Gln-0.02178*Val-0.09077*Met+0.02687*Leu-0.08063*His+0.00726*Arg |
| 8.32959+0.01572*Cit-0.05032*ABA-0.01922*Val-0.07104*Met+0.02770*Leu-0.08206*His |
| 8.68026-0.05142*ABA-0.01502*Val-0.05357*Met+0.02216*Leu-0.07242*His-0.02593*Trp |
| 7.79357+0.00197*Pro-0.02319*Val-0.09270*Met+0.02840*Leu-0.07993*His+0.00743*Arg |
| 8.39999+0.00419*Thr-0.04694*ABA-0.01847*Val-0.08246*Met+0.02671*Leu-0.08123*His |
| 7.47869+0.04098*Asn-0.05679*ABA-0.07826*Met+0.00441*Leu-0.07786*His-0.03649*Trp |
| 7.75860+0.00323*Thr-0.02186*Val-0.09502*Met+0.02746*Leu-0.08078*His+0.00617*Arg |
| 7.69488+0.03861*Asn-0.00117*Ala-0.05421*ABA-0.06064*Met-0.07645*His-0.03354*Trp |
| 8.05537+0.00199*Pro-0.01926*Val-0.06310*Met+0.02414*Leu-0.07439*His-0.02172*Trp |
| 8.44800+0.00018*Gln-0.04742*ABA-0.01818*Val-0.07125*Met+0.02570*Leu-0.07954*His |
| 7.84111-0.00024*Gln-0.05066*ABA-0.04539*Met-0.02158*Tyr-0.08110*His+0.00794*Arg |
| 7.86591-0.00498*Glu-0.05016*ABA-0.04945*Met-0.01923*Tyr-0.08099*His+0.00787*Arg |
| 8.42052-0.05139*ABA-0.01146*Val+0.01721*Leu-0.01678*Tyr-0.07437*His-0.02804*Trp |
| 7.35481-0.00486*Val-0.04509*Met-0.02023*Tyr-0.07862*His+0.00688*Arg |
| 7.38054-0.00205*Glu-0.00460*Val-0.04645*Met-0.01967*Tyr-0.07855*His+0.00690*Arg |
| 7.36293-0.00469*Tau+0.00111*Gln-0.00442*Val-0.04083*Met-0.01759*Tyr-0.07849*His |
| 7.49104-0.01223*Cit-0.00508*Val-0.05116*Met-0.02029*Tyr-0.07633*His+0.00957*Arg |
| 7.99265-0.01836*Val-0.05748*Met+0.02429*Leu-0.01561*Tyr-0.07672*His |
| 7.88269+0.00812*Cit-0.01884*Val-0.05774*Met+0.02516*Leu-0.01582*Tyr-0.07818*His |

## FIG.196

| FOMULA |
|---|
| 8.46827-0.04845*ABA-0.01824*Val-0.07264*Met+0.02535*Leu+0.00305*Phe-0.07930*His |
| 7.51152-0.00436*Tau-0.04001*Met-0.02152*Tyr-0.07283*His-0.02460*Trp+0.00582*Arg |
| 7.54152+0.02565*Asn-0.00335*Glu-0.04666*ABA-0.05760*Met-0.01805*Tyr-0.08180*His |
| 7.44359-0.00262*Thr+0.03146*Asn-0.04712*ABA-0.05395*Met-0.01979*Tyr-0.08128*His |
| 7.19991+0.00052*Gln-0.00480*Val-0.04666*Met-0.01983*Tyr-0.07986*His+0.00648*Arg |
| 7.39315+0.02303*Asn-0.04839*ABA-0.06317*Met-0.02088*Tyr-0.08265*His+0.00574*Arg |
| 7.88942-0.00301*Tau-0.01370*Val+0.01679*Leu-0.01890*Tyr-0.07434*His-0.02375*Trp |
| 7.56630-0.00411*Tau+0.02801*Asn-0.01951*Val-0.10079*Met+0.02538*Leu-0.07884*His |
| 8.26096-0.00489*Tau-0.05670*ABA-0.05051*Met-0.07369*His-0.03115*Trp+0.00581*Arg |
| 8.02551-0.00555*Glu-0.05117*ABA-0.04140*Met+0.00403*Leu-0.01887*Tyr-0.08153*His |
| 7.93978-0.00497*Glu+0.00750*Cit-0.04915*ABA-0.03478*Met-0.01749*Tyr-0.08176*His |
| 7.66585+0.03917*Asn-0.00246*Pro-0.05561*ABA-0.06327*Met-0.07457*His-0.03546*Trp |
| 7.98143+0.00294*Glu-0.01930*Val-0.05665*Met+0.02522*Leu-0.01625*Tyr-0.07680*His |
| 7.42642+0.02655*Asn+0.00215*Glu-0.02078*Val-0.09815*Met+0.02696*Leu-0.08114*His |
| 7.98474+0.00011*Ala-0.01846*Val-0.05815*Met+0.02440*Leu-0.01575*Tyr-0.07667*His |
| 8.49810+0.00035*Pro-0.04728*ABA-0.01844*Val-0.07119*Met+0.02596*Leu-0.07925*His |
| 7.66725+0.03607*Asn-0.05188*ABA-0.00122*Val-0.06391*Met-0.07541*His-0.03482*Trp |
| 7.70457-0.00393*Tau-0.00024*Ala-0.00445*Val-0.03410*Met-0.01792*Tyr-0.07640*His |
| 7.60334-0.00391*Tau-0.00498*Val-0.03954*Met-0.01952*Tyr+0.00621*Phe-0.07632*His |
| 7.65112-0.00418*Tau+0.00265*Cit-0.00449*Val-0.03521*Met-0.01840*Tyr-0.07671*His |
| 7.43785+0.02666*Asn+0.00122*Cit-0.04679*ABA-0.05628*Met-0.01953*Tyr-0.08257*His |
| 7.79989-0.00387*Cit-0.04995*ABA-0.04801*Met-0.02151*Tyr-0.08098*His+0.00857*Arg |
| 7.86852-0.04701*ABA-0.00275*Val-0.04584*Met-0.01863*Tyr-0.07960*His+0.00834*Arg |
| 7.98363-0.00383*Tau+0.00920*Cit-0.02083*Val-0.07429*Met+0.02599*Leu-0.07849*His |
| 7.58148+0.03774*Asn-0.05353*ABA-0.06716*Met-0.07632*His-0.03567*Trp |
| 7.56628+0.03731*Asn+0.00157*Cit-0.05374*ABA-0.06665*Met-0.07657*His-0.03570*Trp |
| 7.53430+0.02833*Asn-0.00207*Pro-0.04864*ABA-0.05507*Met-0.01850*Tyr-0.08122*His |
| 7.25680-0.00538*Val-0.05003*Met-0.02168*Tyr+0.00716*Phe-0.07847*His+0.00685*Arg |
| 7.45209+0.02673*Asn-0.00726*Glu-0.05349*ABA-0.08538*Met+0.00390*Leu-0.08701*His |
| 7.52299+0.02574*Asn-0.01990*Val-0.09445*Met+0.02676*Leu-0.00446*Phe-0.08076*His |
| 7.72279+0.03392*Asn-0.00120*Gln-0.04822*ABA-0.05759*Met-0.02019*Tyr-0.07978*His |
| 8.50705+0.00013*Glu-0.04772*ABA-0.01824*Val-0.07047*Met+0.02575*Leu-0.07915*His |
| 8.53075-0.04698*ABA-0.01806*Val-0.06745*Met+0.02581*Leu-0.07921*His-0.00079*Lys |
| 8.59046-0.00088*Ala-0.04903*ABA-0.01720*Val-0.06367*Met+0.02464*Leu-0.07930*His |
| 8.50769-0.04773*ABA-0.01820*Val-0.07048*Met+0.02571*Leu-0.07914*His |
| 8.65350-0.00370*Tau-0.04781*ABA-0.01785*Val-0.07145*Met+0.02503*Leu-0.07702*His |
| 8.19720-0.01732*Val-0.04624*Met+0.02321*Leu-0.07411*His-0.02144*Trp-0.00326*Lys |
| 7.44804+0.02701*Asn-0.04662*ABA-0.05674*Met-0.01950*Tyr-0.08235*His |
| 7.95627-0.00416*Tau-0.02168*Val-0.09226*Met+0.02637*Leu-0.07697*His+0.00852*Arg |
| 8.09357-0.00356*Tau-0.00713*Glu-0.05394*ABA-0.06037*Met+0.00243*Leu-0.08295*His |
| 7.44266+0.02617*Asn-0.02003*Val-0.09793*Met+0.02622*Leu-0.08098*His |
| 7.25937-0.00485*Glu-0.00860*Cit-0.05273*Met-0.02381*Tyr-0.08000*His+0.00769*Arg |
| 7.46350+0.02737*Asn-0.00060*Ala-0.01935*Val-0.09443*Met+0.02553*Leu-0.08125*His |
| 7.68827-0.00407*Tau-0.00451*Val-0.03536*Met-0.01829*Tyr-0.07627*His |
| 7.41284-0.00079*Ala-0.00467*Val-0.04232*Met-0.01914*Tyr-0.07876*His+0.00740*Arg |
| 7.42707+0.02565*Asn+0.00179*Cit-0.02015*Val-0.09753*Met+0.02641*Leu-0.08125*His |
| 7.34692+0.00045*Thr-0.00482*Val-0.04585*Met-0.02016*Tyr-0.07885*His+0.00665*Arg |
| 7.95258-0.00680*Glu+0.00028*Gln-0.00166*Pro-0.05296*ABA-0.05161*Met-0.08357*His |
| 7.86717-0.04885*ABA-0.00340*Val-0.04261*Met-0.01980*Tyr+0.01618*Phe-0.07924*His |
| 7.31773+0.04123*Asn-0.05869*ABA-0.08427*Met+0.01582*Phe-0.07742*His-0.03851*Trp |

# FIG.197

| FOMULA |
|---|
| 9.48334+0.09182*Cit-0.04069*Val-0.09675*Met+0.07192*Leu-0.08168*His-0.07552*Trp |
| 9.68200+0.08856*Cit-0.03670*Val+0.07044*Leu-0.05565*Phe-0.08164*His-0.07593*Trp |
| 9.51651+0.00760*Gly-0.03687*Val-0.15131*Met+0.07475*Leu-0.07143*His-0.06788*Trp |
| 6.80184+0.09180*Cit-0.02645*Val-0.09528*Met+0.07699*Ile-0.03937*His-0.07714*Trp |
| 11.00274-0.03223*Val+0.07048*Leu-0.02409*Tyr-0.05459*Phe-0.06797*His-0.06420*Trp |
| 6.13697-0.00615*Tau+0.08350*Cit-0.03384*Val-0.12702*Met+0.09478*Ile-0.08376*Trp |
| 8.66858+0.09143*Cit-0.03367*Val+0.06078*Leu-0.03181*Tyr-0.08236*His-0.07327*Trp |
| 5.87232+0.07706*Cit-0.03385*Val-0.12016*Met+0.09674*Ile-0.00561*Tyr-0.08281*Trp |
| 9.39569+0.09756*Cit-0.03646*Val+0.07360*Leu-0.08806*His-0.08017*Trp-0.01486*Lys |
| 5.80827-0.00535*Thr+0.08786*Cit-0.03466*Val-0.10944*Met+0.09646*Ile-0.08315*Trp |
| 6.32823+0.07436*Cit-0.03229*Val-0.10777*Met+0.09795*Ile-0.02569*Phe-0.08221*Trp |
| 11.73056-0.03822*Val-0.08468*Met+0.08043*Leu-0.05249*Phe-0.06725*His-0.06567*Trp |
| 5.83039+0.07793*Cit-0.03465*Val-0.12729*Met+0.09732*Ile-0.08355*Trp+0.00048*Arg |
| 10.62634+0.01831*Thr-0.03911*Val-0.16577*Met+0.07481*Leu-0.07963*His-0.07027*Trp |
| 8.65714-0.02525*Asn+0.10502*Cit-0.03715*Val+0.05803*Leu-0.08318*His-0.07382*Trp |
| 6.26754+0.07537*Cit-0.04161*Val-0.13866*Met+0.08664*Ile+0.01916*Leu-0.08513*Trp |
| 5.83176+0.07836*Cit-0.03462*Val-0.12656*Met+0.09749*Ile-0.08364*Trp |
| 6.23870-0.00452*Ser+0.08249*Cit-0.03583*Val-0.12216*Met+0.09779*Ile-0.08176*Trp |
| 6.82169+0.09180*Cit-0.02077*Val+0.06061*Ile-0.03484*Phe-0.04720*His-0.07738*Trp |
| 10.45372+0.00203*Gly-0.03225*Val+0.06826*Leu-0.06726*Phe-0.06959*His-0.06781*Trp |
| 8.15139-0.00563*Tau+0.10668*Cit-0.03371*Val+0.05252*Leu-0.08366*His-0.07894*Trp |
| 5.86864-0.00030*Gly+0.07932*Cit-0.03484*Val-0.12536*Met+0.09782*Ile-0.08369*Trp |
| 8.26452-0.01012*Thr+0.11524*Cit-0.03621*Val+0.05756*Leu-0.07996*His-0.07712*Trp |
| 11.05828-0.03144*Val+0.07604*Leu-0.03746*Tyr-0.07250*His-0.06566*Trp-0.01450*Lys |
| 5.98477-0.00196*Ala+0.07877*Cit-0.03400*Val-0.11847*Met+0.09894*Ile-0.08221*Trp |
| 8.03696-0.00374*Tau-0.02625*Val-0.10740*Met+0.08819*Ile-0.03445*Phe-0.07204*Trp |
| 8.22637-0.02202*Val-0.09266*Met+0.07937*Ile-0.02622*His-0.06929*Trp-0.00679*Lys |
| 7.44696-0.01545*Ser+0.08629*Cit-0.03246*Val+0.08565*Ile-0.05878*Phe-0.07769*Trp |
| 8.10591+0.10480*Cit-0.03439*Val+0.05576*Leu-0.08488*His-0.07921*Trp-0.00458*Arg |
| 10.75049-0.03555*Val-0.08864*Met+0.07058*Leu-0.02422*Tyr-0.06766*His-0.06361*Trp |
| 7.43425+0.00396*Gly-0.02068*Val-0.12429*Met+0.06897*Ile-0.03029*His-0.06824*Trp |
| 10.93465+0.00035*Thr-0.03315*Val+0.06923*Leu-0.06879*Phe-0.06869*His-0.06724*Trp |
| 11.60657-0.00502*Ser-0.03441*Val+0.07034*Leu-0.07228*Phe-0.06732*His-0.06437*Trp |
| 11.12918-0.00338*Tau-0.03266*Val+0.06858*Leu-0.06998*Phe-0.06721*His-0.06701*Trp |
| 11.23215-0.01047*Asn-0.03421*Val+0.07066*Leu-0.06806*Phe-0.06753*His-0.06503*Trp |
| 5.83076-0.00084*Pro+0.07910*Cit-0.03429*Val-0.12422*Met+0.09709*Ile-0.08372*Trp |
| 6.59579+0.09753*Cit-0.02112*Val+0.07012*Ile-0.04760*His-0.08085*Trp-0.01159*Lys |
| 6.44448+0.09257*Cit-0.02013*Val+0.05609*Ile-0.02372*Tyr-0.05013*His-0.07508*Trp |
| 9.96623+0.00725*Ser-0.03654*Val-0.12125*Met+0.07129*Leu-0.06938*His-0.07008*Trp |
| 7.75924-0.02662*Val-0.10390*Met+0.08958*Ile-0.00454*Tyr-0.03138*Phe-0.07194*Trp |
| 8.09897-0.00263*Ser-0.02736*Val-0.10345*Met+0.09014*Ile-0.03587*Phe-0.07092*Trp |
| 7.91842-0.02564*Val-0.09116*Met+0.09475*Ile-0.03076*Phe-0.07360*Trp-0.00637*Lys |
| 11.93656-0.03469*Val+0.08417*Leu-0.05996*Phe-0.07196*His-0.06909*Trp-0.01320*Lys |
| 10.94996-0.03319*Val+0.06932*Leu-0.06879*Phe-0.06845*His-0.06714*Trp |
| 6.08593-0.01777*Thr+0.10482*Cit-0.03030*Val+0.08713*Ile-0.04334*Phe-0.08166*Trp |
| 6.04449+0.07871*Cit-0.03275*Val-0.10452*Met+0.10314*Ile-0.08500*Trp-0.00760*Lys |
| 10.04847-0.03805*Val-0.12466*Met+0.02725*Ile+0.05612*Leu-0.05543*His-0.06751*Trp |
| 6.65250-0.00411*Ala+0.10104*Cit-0.02085*Val+0.05831*Ile-0.05559*His-0.07502*Trp |
| 5.34523+0.03807*Asn+0.06974*Cit-0.03346*Val-0.16872*Met+0.10147*Ile-0.08976*Trp |
| 10.93855-0.03319*Val+0.00040*Ile+0.06909*Leu-0.06879*Phe-0.06827*His-0.06716*Trp |

# FIG.198

| FOMULA |
|---|
| 7.80588-0.00081*Pro-0.02657*Val-0.10476*Met+0.08971*Ile-0.03435*Phe-0.07236*Trp |
| 10.81770-0.03334*Val+0.06818*Leu-0.06859*Phe-0.06883*His-0.06694*Trp+0.00327*Arg |
| 7.77674-0.02702*Val-0.10774*Met+0.09013*Ile-0.03329*Phe-0.07241*Trp |
| 6.84655-0.00344*Tau+0.00329*Gly-0.02735*Val-0.14553*Met+0.08456*Ile-0.07410*Trp |
| 9.94929+0.05061*Asn-0.03599*Val-0.16506*Met+0.07201*Leu-0.07157*His-0.07550*Trp |
| 5.27599-0.01704*Thr-0.10576*Cit-0.03116*Val+0.08167*Ile-0.02130*Tyr-0.08149*Trp |
| 5.63243-0.00369*Ala+0.08277*Cit-0.02881*Val+0.09748*Ile-0.08731*Trp-0.01490*Lys |
| 11.04778-0.00219*Pro-0.03200*Val+0.06874*Leu-0.06949*Phe-0.06783*His-0.06682*Trp |
| 10.97331-0.00302*Ala-0.03050*Val+0.07021*Leu-0.07646*His-0.06756*Trp-0.01544*Lys |
| 8.70668-0.03934*Val-0.12016*Met+0.07097*Ile+0.03573*Leu-0.04637*Phe-0.07544*Trp |
| 11.74612-0.00403*Ala-0.03142*Val+0.07220*Leu-0.07150*Phe-0.07160*His-0.06257*Trp |
| 8.46508-0.00491*Ser+0.10556*Cit-0.03566*Val+0.05424*Leu-0.08450*His-0.07637*Trp |
| 5.48791-0.01536*Thr+0.10809*Cit-0.03133*Val+0.09388*Ile-0.08667*Trp-0.01131*Lys |
| 6.48684-0.01919*Asn+0.10306*Cit-0.02362*Val+0.05559*Ile-0.05144*His-0.07557*Trp |
| 9.28470+0.00216*Gly-0.02833*Val+0.05695*Leu-0.04110*Tyr-0.06994*His-0.06394*Trp |
| 11.20759-0.03712*Val-0.08607*Met+0.07808*Leu-0.07109*His-0.06832*Trp-0.01018*Lys |
| 6.19798-0.01269*Thr+0.11828*Cit-0.02478*Val+0.06161*Ile-0.04446*His-0.07788*Trp |
| 7.35550+0.00195*Gly-0.02630*Val-0.11810*Met+0.08846*Ile-0.02939*Phe-0.07303*Trp |
| 6.34146-0.00567*Tau+0.10533*Cit-0.02141*Val+0.04961*Ile-0.05448*His-0.07931*Trp |
| 6.31906-0.00442*Ala+0.07379*Cit-0.02730*Val+0.08784*Ile-0.05445*Phe-0.08111*Trp |
| 7.53179+0.00669*Thr-0.02802*Val-0.13107*Met+0.09258*Ile-0.02948*Phe-0.07468*Trp |
| 10.68521+0.00216*Pro-0.03922*Val-0.12075*Met+0.07191*Leu-0.06848*His-0.06651*Trp |
| 10.33515-0.04081*Val-0.14693*Met+0.06919*Leu-0.06979*His-0.06429*Trp+0.02097*Arg |
| 3.47802-0.02164*Val-0.09138*Met+0.07619*Ile-0.02968*Phe-0.02478*His-0.06737*Trp |
| 8.12009-0.02159*Val-0.09429*Met+0.07198*Ile-0.01463*Tyr-0.02764*His-0.06608*Trp |
| 7.49511-0.02593*Val-0.09252*Met+0.09302*Ile-0.01521*Tyr-0.07311*Trp-0.00771*Lys |
| 6.16592+0.10678*Cit-0.02227*Val+0.05887*Ile-0.05168*His-0.08068*Trp-0.00864*Arg |
| 7.03173+0.00849*Thr-0.02913*Val-0.14474*Met+0.09096*Ile-0.01189*Tyr-0.07489*Trp |
| 5.91007+0.08671*Cit-0.02818*Val+0.08958*Ile-0.04900*Phe-0.08742*Trp-0.01472*Arg |
| 7.71629+0.10244*Cit-0.03442*Val+0.00834*Ile+0.04929*Leu-0.08128*His-0.07912*Trp |
| 6.71353-0.00078*Ser+0.00326*Gly-0.02814*Val-0.14422*Met+0.08638*Ile-0.07398*Trp |
| 7.95816+0.10186*Cit-0.03446*Val+0.05408*Leu-0.08498*His-0.07864*Trp |
| 6.77038+0.00343*Gly-0.00207*Ala-0.02720*Val-0.13701*Met+0.08777*Ile-0.07286*Trp |
| 4.97164-0.01878*Thr+0.11839*Cit-0.03360*Val+0.08341*Ile-0.08601*Trp-0.00464*Arg |
| 8.58188-0.00348*Ala+0.10160*Cit-0.03280*Val+0.05583*Leu-0.08773*His-0.07473*Trp |
| 7.91908+0.00020*Gly+0.10138*Cit-0.03436*Val+0.05399*Leu-0.08502*His-0.07866*Trp |
| 7.79706-0.03742*Val-0.12930*Met+0.07289*Ile+0.02601*Leu-0.01666*Tyr-0.07415*Trp |
| 6.64932+0.00316*Gly-0.02803*Val-0.14453*Met+0.08649*Ile-0.07437*Trp |
| 7.43034-0.00255*Tau-0.02767*Val-0.11781*Met+0.08622*Ile-0.01296*Tyr-0.07183*Trp |
| 7.92400+0.01364*Thr-0.02315*Val-0.14755*Met+0.07481*Ile-0.03483*His-0.07069*Trp |
| 7.55971-0.00455*Ala+0.08515*Cit+0.03949*Ile-0.06867*His-0.07897*Trp-0.01231*Lys |
| 8.34384-0.00928*Ser-0.02383*Val+0.07529*Ile-0.02251*Tyr-0.04999*Phe-0.06718*Trp |
| 6.82822-0.00788*Ser+0.10700*Cit-0.02463*Val+0.05479*Ile-0.05307*His-0.07578*Trp |
| 7.28370-0.00790*Tau+0.08587*Cit-0.06316*Met+0.01796*Ile-0.06929*His-0.08119*Trp |
| 10.79706-0.00281*Tau-0.03711*Val-0.11493*Met+0.06910*Leu-0.06699*His-0.06627*Trp |
| 6.76667+0.00266*Gly-0.02725*Val-0.13271*Met+0.08595*Ile-0.00857*Tyr-0.07323*Trp |
| 8.05074-0.00512*Ala+0.07753*Cit+0.03402*Ile-0.04551*Phe-0.06565*His-0.07416*Trp |
| 8.21358-0.00850*Tau-0.00215*Ser+0.08833*Cit-0.03042*Phe-0.06817*His-0.07412*Trp |
| 7.67856-0.00813*Tau+0.08385*Cit+0.02093*Ile-0.04344*Phe-0.06535*His-0.07973*Trp |
| 10.12733-0.00231*Ala-0.02818*Val+0.05812*Leu-0.03838*Tyr-0.07071*His-0.06107*Trp |

# FIG.199

# FIG.200

| FORMULA |
|---|
| 1.72385−0.00311*Val−0.01057*Met+0.00397*Leu−0.00332*Tyr−0.01209*His+0.00146*Arg |
| 1.79117−0.00291*Val−0.00797*Met+0.00406*Leu−0.00127*Phe−0.01118*His−0.00489*Trp |
| 1.74583+0.00090*Thr−0.00300*Val−0.01141*Met+0.00411*Leu−0.01168*His−0.00480*Trp |
| 1.78468−0.00048*Tau−0.00292*Val−0.00910*Met+0.00388*Leu−0.01095*His−0.00478*Trp |
| 1.76937−0.00062*Tau−0.00291*Val−0.00816*Met+0.00386*Leu−0.00302*Tyr−0.01171*His |
| 1.74997+0.00005*Gln−0.00292*Val−0.00901*Met+0.00388*Leu−0.01123*His−0.00497*Trp |
| 1.76565−0.00293*Val−0.00886*Met+0.00389*Leu−0.01116*His−0.00493*Trp |
| 1.76874−0.00258*Val−0.00597*Met+0.00369*Leu−0.00265*Tyr−0.01095*His−0.00468*Trp |
| 1.76300+0.00024*Glu−0.00299*Val−0.00879*Met+0.00393*Leu−0.01116*His−0.00496*Trp |
| 1.74847−0.00311*Val−0.01129*Met+0.00398*Leu−0.01127*His−0.00483*Trp+0.00118*Arg |
| 1.73741+0.00371*Asn−0.00770*ABA−0.00295*Val−0.01519*Met+0.00424*Leu−0.01204*His |
| 1.65792+0.00348*Asn−0.00347*Val−0.01682*Met+0.00429*Leu−0.01259*His+0.00102*Arg |
| 1.68900−0.00072*Tau−0.00090*Val−0.00709*Met−0.00370*Tyr−0.01202*His+0.00145*Arg |
| 1.61515+0.00455*Asn−0.00820*ABA−0.01001*Met−0.00442*Tyr+0.00180*Phe−0.01229*His |
| 1.79051−0.00818*ABA−0.00321*Val−0.01479*Met+0.00428*Leu−0.01191*His+0.00157*Arg |
| 1.72105−0.00002*Ala−0.00355*Val−0.01396*Met+0.00422*Leu−0.01241*His+0.00133*Arg |
| 1.74820+0.00157*Cit−0.00304*Val−0.00883*Met+0.00403*Leu−0.01142*His−0.00500*Trp |
| 1.75542−0.00294*Val−0.00759*Met+0.00397*Leu−0.00298*Tyr−0.00056*Phe−0.01202*His |
| 1.64693+0.00645*Asn−0.00276*Val−0.01466*Met+0.00398*Leu−0.01133*His−0.00577*Trp |
| 1.72299+0.00099*Thr−0.00300*Val−0.01071*Met+0.00413*Leu−0.00299*Tyr−0.01256*His |
| 1.67993−0.00124*Thr+0.00642*Asn−0.00851*ABA−0.00376*Tyr−0.01024*His−0.00676*Trp |
| 1.81299−0.00768*ABA−0.00260*Val−0.00836*Met+0.00393*Leu−0.00285*Tyr−0.01155*His |
| 1.67940+0.00604*Asn−0.00821*ABA−0.00770*Met−0.00283*Tyr−0.01060*His−0.00625*Trp |
| 1.72032−0.00357*Val−0.01406*Met+0.00424*Leu−0.01241*His+0.00131*Arg |
| 1.72035−0.00000*Glu−0.00357*Val−0.01406*Met+0.00424*Leu−0.01241*His+0.00131*Arg |
| 1.74920−0.00353*Val−0.01299*Met+0.00442*Leu−0.00140*Phe−0.01242*His+0.00128*Arg |
| 1.65514+0.00468*Asn−0.00283*Val−0.01210*Met+0.00396*Leu−0.00334*Tyr−0.01225*His |
| 1.76378−0.00006*Gln−0.00293*Val−0.00759*Met+0.00389*Leu−0.00315*Tyr−0.01190*His |
| 1.75751+0.00020*Ala−0.00312*Val−0.01010*Met+0.00410*Leu−0.01111*His−0.00529*Trp |
| 1.72642−0.00074*Cit−0.00354*Val−0.01439*Met+0.00418*Leu−0.01229*His+0.00148*Arg |
| 1.68811−0.00059*Tau+0.00614*Asn−0.00880*ABA−0.01139*Met−0.01074*His−0.00658*Trp |
| 1.75251−0.00010*Gln−0.00360*Val−0.01389*Met+0.00427*Leu−0.01224*His+0.00141*Arg |
| 1.78805+0.00217*Cit−0.00818*ABA−0.00314*Val−0.01163*Met+0.00436*Leu−0.01218*His |
| 1.84803−0.00875*ABA−0.00246*Val−0.00877*Met+0.00388*Leu−0.01045*His−0.00558*Trp |
| 1.72280+0.00045*Pro−0.00387*Val−0.01495*Met+0.00463*Leu−0.01251*His+0.00113*Arg |
| 1.78967+0.00088*Thr−0.00769*ABA−0.00306*Val−0.01402*Met+0.00437*Leu−0.01231*His |
| 1.65113+0.00669*Asn−0.00946*ABA−0.01331*Met+0.00099*Leu−0.01131*His−0.00720*Trp |
| 1.70786+0.00078*Thr−0.00357*Val−0.01562*Met+0.00440*Leu−0.01282*His+0.00102*Arg |
| 1.67117+0.00612*Asn−0.00008*Ala−0.00883*ABA−0.01060*Met−0.01097*His−0.00660*Trp |
| 1.76461+0.00043*Pro−0.00326*Val−0.01015*Met+0.00429*Leu−0.01130*His−0.00477*Trp |
| 1.85832−0.00014*Gln−0.00811*ABA−0.00301*Val−0.01111*Met+0.00419*Leu−0.01156*His |
| 1.79296−0.00025*Gln−0.00890*ABA−0.00691*Met−0.00432*Tyr−0.01185*His+0.00166*Arg |
| 1.72083−0.00060*Glu−0.00846*ABA−0.00791*Met−0.00389*Tyr−0.01223*His+0.00144*Arg |
| 1.80895−0.00849*ABA−0.00187*Val+0.00312*Leu−0.00326*Tyr−0.01076*His−0.00568*Trp |
| 1.66072−0.00090*Val−0.00650*Met−0.00380*Tyr−0.01236*His+0.00134*Arg |
| 1.66046−0.00002*Glu−0.00090*Val−0.00649*Met−0.00381*Tyr−0.01236*His+0.00134*Arg |
| 1.71201−0.00064*Tau−0.00001*Gln−0.00077*Val−0.00436*Met−0.00351*Tyr−0.01194*His |
| 1.68166−0.00221*Cit−0.00090*Val−0.00757*Met−0.00386*Tyr−0.01199*His+0.00184*Arg |
| 1.74419−0.00293*Val−0.00784*Met+0.00388*Leu−0.00312*Tyr−0.01200*His |
| 1.73172+0.00110*Cit−0.00301*Val−0.00785*Met+0.00399*Leu−0.00311*Tyr−0.01220*His |

# FIG.201

| FORMULA |
|---|
| 1.81626-0.00781*ABA-0.00299*Val-0.01136*Met+0.00421*Leu-0.00034*Phe-0.01182*His |
| 1.68667-0.00051*Tau-0.00487*Met-0.00388*Tyr-0.01147*His-0.00502*Trp+0.00103*Arg |
| 1.66162+0.00406*Asn-0.00025*Glu-0.00774*ABA-0.00855*Met-0.00379*Tyr-0.01226*His |
| 1.65393-0.00018*Thr+0.00444*Asn-0.00776*ABA-0.00828*Met-0.00393*Tyr-0.01221*His |
| 1.69899-0.00012*Gln-0.00091*Val-0.00619*Met-0.00389*Tyr-0.01215*His+0.00146*Arg |
| 1.64986+0.00341*Asn-0.00811*ABA-0.01001*Met-0.00415*Tyr-0.01240*His+0.00114*Arg |
| 1.74454-0.00037*Tau-0.00229*Val+0.00310*Leu-0.00357*Tyr-0.01126*His-0.00490*Trp |
| 1.68368-0.00074*Tau+0.00448*Asn-0.00327*Val-0.01597*Met+0.00420*Leu-0.01221*His |
| 1.77674-0.00061*Tau-0.00962*ABA-0.00887*Met-0.01087*His-0.00597*Trp+0.00120*Arg |
| 1.74356-0.00070*Glu-0.00851*ABA-0.00625*Met+0.00072*Leu-0.00387*Tyr-0.01230*His |
| 1.73053-0.00051*Glu+0.00100*Cit-0.00822*ABA-0.00509*Met-0.00361*Tyr-0.01226*His |
| 1.67388+0.00622*Asn-0.00027*Pro-0.00900*ABA-0.01040*Met-0.01079*His-0.00681*Trp |
| 1.74008-0.00038*Glu-0.00301*Val-0.00764*Met+0.00393*Leu-0.00321*Tyr-0.01201*His |
| 1.65503+0.00428*Asn-0.00019*Glu-0.00336*Val-0.01548*Met+0.00426*Leu-0.01257*His |
| 1.73880+0.00011*Ala-0.00303*Val-0.00845*Met+0.00400*Leu-0.00328*Tyr-0.01201*His |
| 1.80669+0.00028*Pro-0.00753*ABA-0.00322*Val-0.01241*Met+0.00442*Leu-0.01191*His |
| 1.67438+0.00586*Asn-0.00860*ABA-0.00015*Val-0.01060*Met-0.01088*His-0.00662*Trp |
| 1.70474-0.00068*Tau+0.00006*Ala-0.00079*Val-0.00472*Met-0.00359*Tyr-0.01196*His |
| 1.69914-0.00063*Tau-0.00080*Val-0.00467*Met-0.00360*Tyr+0.00043*Phe-0.01196*His |
| 1.70397-0.00065*Tau+0.00027*Cit-0.00077*Val-0.00441*Met-0.00350*Tyr-0.01202*His |
| 1.65425+0.00413*Asn+0.00008*Cit-0.00775*ABA-0.00848*Met-0.00390*Tyr-0.01231*His |
| 1.71968-0.00119*Cit-0.00835*ABA-0.00807*Met-0.00419*Tyr-0.01212*His+0.00166*Arg |
| 1.72737-0.00790*ABA-0.00056*Val-0.00741*Met-0.00356*Tyr-0.01191*His+0.00157*Arg |
| 1.75273-0.00070*Tau+0.00131*Cit-0.00343*Val-0.01163*Met+0.00423*Leu-0.01220*His |
| 1.66826+0.00602*Asn-0.00878*ABA-0.01098*Met-0.01099*His-0.00675*Trp |
| 1.66284+0.00586*Asn+0.00055*Cit-0.00888*ABA-0.01080*Met-0.01108*His-0.00678*Trp |
| 1.65612+0.00420*Asn-0.00006*Pro-0.00779*ABA-0.00843*Met-0.00386*Tyr-0.01226*His |
| 1.64501-0.00096*Val-0.00707*Met-0.00400*Tyr+0.00087*Phe-0.01233*His+0.00137*Arg |
| 1.65743+0.00379*Asn-0.00113*Glu-0.00879*ABA-0.01424*Met+0.00054*Leu-0.01302*His |
| 1.68650+0.00406*Asn-0.00329*Val-0.01442*Met+0.00438*Leu-0.00124*Phe-0.01256*His |
| 1.75060+0.00664*Asn-0.00040*Gln-0.00814*ABA-0.00888*Met-0.00413*Tyr-0.01159*His |
| 1.81236-0.00019*Glu-0.00791*ABA-0.00295*Val-0.01166*Met+0.00414*Leu-0.01181*His |
| 1.81514-0.00767*ABA-0.00297*Val-0.01092*Met+0.00422*Leu-0.01181*His-0.00020*Lys |
| 1.81413-0.00007*Ala-0.00798*ABA-0.00292*Val-0.01115*Met+0.00409*Leu-0.01180*His |
| 1.80999-0.00788*ABA-0.00300*Val-0.01161*Met+0.00416*Leu-0.01182*His |
| 1.84032-0.00073*Tau-0.00795*ABA-0.00296*Val-0.01185*Met+0.00413*Leu-0.01146*His |
| 1.78428-0.00285*Val-0.00705*Met+0.00405*Leu-0.01115*His-0.00479*Trp-0.00056*Lys |
| 1.65473+0.00416*Asn-0.00774*ABA-0.00850*Met-0.00390*Tyr-0.01229*His |
| 1.74985-0.00076*Tau-0.00355*Val-0.01454*Met+0.00421*Leu-0.01204*His+0.00143*Arg |
| 1.75430-0.00065*Tau-0.00095*Glu-0.00903*ABA-0.01072*Met+0.00038*Leu-0.01250*His |
| 1.65792+0.00424*Asn-0.00331*Val-0.01548*Met+0.00423*Leu-0.01256*His |
| 1.65354-0.00056*Glu-0.00214*Cit-0.00796*Met-0.00464*Tyr-0.01265*His+0.00154*Arg |
| 1.65802+0.00430*Asn-0.00003*Ala-0.00328*Val-0.01536*Met+0.00420*Leu-0.01256*His |
| 1.70729-0.00064*Tau-0.00077*Val-0.00443*Met-0.00350*Tyr-0.01197*His |
| 1.66303-0.00004*Ala-0.00088*Val-0.00637*Met-0.00374*Tyr-0.01236*His+0.00138*Arg |
| 1.65642+0.00415*Asn+0.00028*Cit-0.00333*Val-0.01540*Met+0.00425*Leu-0.01260*His |
| 1.65629+0.00023*Thr-0.00087*Val-0.00695*Met-0.00377*Tyr-0.01249*His+0.00125*Arg |
| 1.77978-0.00111*Glu-0.00013*Gln-0.00007*Pro-0.00897*ABA-0.00907*Met-0.01235*His |
| 1.71994-0.00800*ABA-0.00053*Val-0.00564*Met-0.00374*Tyr+0.00181*Phe-0.01173*His |
| 1.63695+0.00633*Asn-0.00919*ABA-0.01245*Met+0.00128*Phe-0.01101*His-0.00692*Trp |

# FIG.202

| FORMULA |
| --- |
| 1.92643+0.01412*Cit−0.00618*Val−0.01270*Met+0.01096*Leu−0.01403*His−0.01167*Trp |
| 1.52561+0.01450*Cit−0.00423*Val−0.01341*Met+0.01245*Ile−0.00764*His−0.01173*Trp |
| 1.60226−0.00535*Val−0.02098*Met+0.01614*Ile−0.01245*Trp |
| 1.96307+0.01376*Cit−0.00578*Val+0.01089*Leu−0.00742*Phe−0.01419*His−0.01138*Trp |
| 1.82931+0.01430*Cit−0.00544*Val+0.00968*Leu−0.00357*Tyr−0.01444*His−0.01158*Trp |
| 1.60325−0.00517*Val−0.01942*Met+0.01595*Ile−0.00134*Tyr−0.01225*Trp |
| 2.30415−0.00595*Val−0.01178*Met+0.01242*Leu−0.00740*Phe−0.01215*His−0.01037*Trp |
| 1.34381+0.01239*Cit−0.00593*Val−0.01997*Met+0.01682*Ile−0.01325*Trp |
| 1.83961−0.00255*Asn+0.01572*Cit−0.00590*Val+0.00949*Leu−0.01467*His−0.01187*Trp |
| 1.80653−0.00133*Thr+0.01713*Cit−0.00580*Val+0.00945*Leu−0.01409*His−0.01224*Trp |
| 1.40007−0.00295*Thr+0.01684*Cit−0.00530*Val+0.01515*Ile−0.00674*Phe−0.01299*Trp |
| 1.68721−0.00490*Val−0.01755*Met+0.01624*Ile−0.00545*Phe−0.01177*Trp |
| 1.49746+0.00053*Gly−0.00508*Val−0.02309*Met+0.01578*Ile−0.01237*Trp |
| 1.81759−0.00174*Ser−0.00497*Val+0.01433*Ile−0.00989*Phe−0.01166*Trp |
| 1.68837+0.01363*Cit−0.00319*Tyr−0.01343*His−0.01171*Trp |
| 1.34129−0.00107*Thr+0.01422*Cit−0.00591*Val−0.01630*Met+0.01649*Ile−0.01328*Trp |
| 1.81170−0.00171*Ser−0.00468*Val+0.01425*Ile−0.00314*Tyr−0.00804*Phe−0.01119*Trp |
| 1.73086−0.00044*Ser+0.01389*Cit−0.00336*Tyr−0.01344*His−0.01154*Trp |
| 1.94617+0.00125*Gly−0.00583*Val−0.02126*Met+0.01195*Leu−0.01282*His−0.01079*Trp |
| 1.78046−0.00394*Val−0.01593*Met+0.01259*Ile−0.00603*His−0.01115*Trp |
| 1.27317−0.00289*Thr+0.01721*Cit−0.00555*Val+0.01450*Ile−0.00252*Tyr−0.01360*Trp |
| 1.83592−0.00368*Val−0.01352*Met+0.01298*Ile−0.00451*Phe−0.00553*His−0.01070*Trp |
| 2.18111−0.00597*Val−0.01525*Met+0.01120*Leu−0.01255*His−0.01110*Trp |
| 1.63152−0.00273*Ser+0.01430*Cit−0.00590*Val+0.01536*Ile−0.00917*Phe−0.01206*Trp |
| 1.77853+0.01538*Cit−0.00569*Val+0.00920*Leu−0.01497*His−0.01235*Trp |
| 1.75815−0.00069*Ser−0.00499*Val−0.01662*Met+0.01625*Ile−0.00595*Phe−0.01145*Trp |
| 1.42879−0.00097*Ser+0.01331*Cit−0.00618*Val−0.01888*Met+0.01686*Ile−0.01287*Trp |
| 2.35130−0.00564*Val+0.01332*Leu−0.00792*Phe−0.01281*His−0.01091*Trp−0.00210*Lys |
| 1.24343−0.00327*Thr+0.01843*Cit−0.00591*Val+0.01452*Ile−0.01419*Trp |
| 1.38075+0.01225*Cit−0.00560*Val−0.01639*Met+0.01754*Ile−0.01334*Trp−0.00121*Lys |
| 1.63993−0.00481*Val−0.01536*Met+0.01673*Ile−0.00149*Tyr−0.01234*Trp−0.00131*Lys |
| 1.63068+0.01433*Cit−0.01455*His−0.01280*Trp |
| 1.95358+0.01432*Cit−0.00582*Val+0.01167*Leu−0.01481*His−0.01233*Trp−0.00215*Lys |
| 1.86058−0.00056*Ala+0.01537*Cit−0.00542*Val+0.00946*Leu−0.01512*His−0.01174*Trp |
| 1.25043+0.00680*Asn+0.01079*Cit−0.00572*Val−0.02755*Met+0.01754*Ile−0.01412*Trp |
| 1.56265+0.00118*Thr−0.00547*Val−0.02489*Met+0.01661*Ile−0.01255*Trp |
| 2.22520−0.00550*Val+0.01120*Leu−0.00924*Phe−0.01275*His−0.01074*Trp |
| 1.24345+0.01430*Cit−0.00534*Val+0.01537*Ile−0.00380*Tyr−0.01425*Trp−0.00235*Arg |
| 2.32662−0.00080*Ser−0.00569*Val+0.01134*Leu−0.00976*Phe−0.01254*His−0.01029*Trp |
| 1.49635−0.00185*Asn+0.01595*Cit−0.00390*Val+0.00966*Ile−0.00965*His−0.01203*Trp |
| 1.88000−0.00102*Ser+0.01621*Cit−0.00593*Val+0.00923*Leu−0.01484*His−0.01189*Trp |
| 1.21190+0.01215*Cit−0.00534*Val+0.01395*Ile−0.00476*Tyr−0.01387*Trp |
| 1.74818−0.00402*Val−0.01888*Met+0.01158*Ile−0.00655*His−0.01071*Trp+0.00220*Arg |
| 1.63829−0.00501*Val−0.01715*Met+0.01692*Ile−0.01256*Trp−0.00129*Lys |
| 1.46066+0.01570*Cit−0.00378*Val+0.00939*Ile−0.01001*His−0.01237*Trp |
| 1.82229−0.00018*Tau−0.00170*Ser−0.00493*Val+0.01423*Ile−0.00986*Phe−0.01166*Trp |
| 1.50247+0.00050*Gly−0.00504*Val−0.02253*Met+0.01575*Ile−0.00040*Tyr−0.01231*Trp |
| 1.41595+0.00999*Asn−0.00516*Val−0.03192*Met+0.01732*Ile−0.01388*Trp |
| 1.63140−0.00041*Ala−0.00523*Val−0.01904*Met+0.01646*Ile−0.01218*Trp |
| 1.40005+0.01193*Cit−0.00683*Val−0.02136*Met+0.01515*Ile+0.00262*Leu−0.01350*Trp |

# FIG.203

| FORMULA |
| --- |
| 1.37179-0.00044*Ala+0.01257*Cit-0.00580*Val-0.01785*Met+0.01718*Ile-0.01296*Trp |
| 1.44182-0.00084*Ala+0.01210*Cit-0.00498*Val+0.01603*Ile-0.00860*Phe-0.01260*Trp |
| 1.54640+0.01467*Cit-0.00357*Val+0.01037*Ile-0.00485*Phe-0.00895*His-0.01173*Trp |
| 1.66476-0.00659*Val-0.02279*Met+0.01394*Ile+0.00352*Leu-0.01283*Trp |
| 1.58950+0.00012*Ser-0.00533*Val-0.02110*Met+0.01614*Ile-0.01250*Trp |
| 1.34170-0.00019*Pro+0.01258*Cit-0.00585*Val-0.01940*Met+0.01675*Ile-0.01329*Trp |
| 1.71076-0.00464*Val-0.01456*Met+0.01690*Ile-0.00497*Phe-0.01193*Trp-0.00111*Lys |
| 1.52748-0.00153*Ser+0.01284*Asn-0.00545*Val-0.03345*Met+0.01764*Ile-0.01360*Trp |
| 1.59634-0.00148*Ser+0.01693*Cit-0.00424*Val+0.00986*Ile-0.00965*His-0.01173*Trp |
| 1.68894-0.00493*Val-0.01783*Met+0.01629*Ile+0.00031*Tyr-0.00558*Phe-0.01181*Trp |
| 1.38034-0.00100*Tau+0.01338*Cit-0.00582*Val-0.01991*Met+0.01641*Ile-0.01311*Trp |
| 1.50395+0.00903*Asn-0.00481*Val-0.02804*Met+0.01729*Ile-0.00450*Phe-0.01319*Trp |
| 1.60194+0.00003*Pro-0.00537*Val-0.02107*Met+0.01615*Ile-0.01245*Trp |
| 2.10763-0.00630*Val-0.02028*Met+0.01090*Leu-0.01268*His-0.01051*Trp+0.00310*Arg |
| 1.43006-0.00216*Ser+0.01403*Cit-0.00587*Val+0.01441*Ile-0.00494*Tyr-0.01274*Trp |
| 2.14799+0.00269*Thr-0.00615*Val-0.02331*Met+0.01184*Leu-0.01415*His-0.01102*Trp |
| 1.41774+0.00994*Asn-0.00500*Val-0.03048*Met+0.01715*Ile-0.00119*Tyr-0.01369*Trp |
| 1.56849-0.00550*Val-0.02337*Met+0.01563*Ile-0.01223*Trp+0.00155*Arg |
| 1.76982-0.00085*Ala+0.01293*Cit+0.00554*Ile-0.00702*Phe-0.01213*His-0.01143*Trp |
| 2.33191-0.00065*Ala-0.00520*Val+0.01163*Leu-0.00980*Phe-0.01287*His-0.00997*Trp |
| 1.67694+0.01293*Cit+0.00345*Ile-0.00623*Phe-0.01292*His-0.01243*Trp |
| 1.74569-0.00048*Ala+0.01414*Cit-0.00184*Tyr-0.01354*His-0.01133*Trp |
| 1.73525+0.01370*Cit-0.00397*Phe-0.01343*His-0.01188*Trp |
| 1.72911-0.00058*Ala+0.01459*Cit-0.01411*His-0.01180*Trp |
| 1.37316+0.01434*Cit-0.00518*Val+0.01628*Ile-0.00750*Phe-0.01387*Trp-0.00272*Arg |
| 1.32257+0.01191*Cit-0.00491*Val+0.01613*Ile-0.00352*Tyr-0.01377*Trp-0.00213*Lys |
| 1.53103-0.00069*Ala+0.01575*Cit-0.00362*Val+0.01057*Ile-0.00973*His-0.01168*Trp |
| 1.98152-0.00039*Ser-0.00601*Phe-0.01215*His-0.01082*Trp+0.00168*Arg |
| 1.19262+0.01579*Cit-0.00587*Val+0.01568*Ile-0.01533*Trp-0.00302*Arg |
| 1.67766+0.01511*Cit-0.00119*Val-0.01302*His-0.01175*Trp |
| 1.34498+0.01234*Cit-0.00589*Val-0.01969*Met+0.01678*Ile-0.00024*Tyr-0.01321*Trp |
| 1.74492-0.00056*Ala-0.00463*Val-0.01417*Met+0.01671*Ile-0.00659*Phe-0.01126*Trp |
| 1.62634-0.00042*Tau-0.00529*Val-0.02098*Met+0.01594*Ile-0.01237*Trp |
| 1.42611+0.01180*Cit-0.00553*Val-0.01719*Met+0.01687*Ile-0.00449*Phe-0.01265*Trp |
| 1.66174-0.00065*Gly-0.00353*Val-0.01824*Met+0.01196*Ile-0.00637*His-0.01097*Trp |
| 1.69054-0.00013*Pro-0.00483*Val-0.01708*Met+0.01619*Ile-0.00559*Phe-0.01178*Trp |
| 1.64421+0.00235*Asn+0.01329*Cit-0.00357*Tyr-0.01356*His-0.01197*Trp |
| 1.63083-0.00039*Ala-0.00512*Val-0.01814*Met+0.01633*Ile-0.00084*Tyr-0.01206*Trp |
| 1.67352+0.01302*Cit-0.00359*Tyr-0.01356*His-0.01161*Trp+0.00067*Arg |
| 1.62375-0.00036*Tau-0.00514*Val-0.01965*Met+0.01581*Ile-0.00114*Tyr-0.01220*Trp |
| 2.12000+0.00041*Gly-0.00537*Val+0.01109*Leu-0.00888*Phe-0.01300*His-0.01078*Trp |
| 2.17750-0.00570*Val-0.01256*Met+0.01117*Leu-0.00251*Tyr-0.01243*His-0.01072*Trp |
| 1.60656+0.01295*Cit+0.00316*Ile-0.00462*Tyr-0.01305*His-0.01220*Trp |
| 1.93911-0.00102*Ser-0.00338*Val+0.01074*Ile-0.00731*Phe-0.00694*His-0.01048*Trp |
| 1.93760-0.00079*Ala+0.01241*Cit+0.00357*Leu-0.00769*Phe-0.01479*His-0.01142*Trp |
| 1.71017-0.00041*Tau-0.00484*Val-0.01756*Met+0.01605*Ile-0.00544*Phe-0.01169*Trp |
| 1.61479+0.00032*Gly-0.00478*Val-0.01919*Met+0.01602*Ile-0.00487*Phe-0.01179*Trp |
| 1.70665-0.00100*Tau+0.01468*Cit-0.00284*Tyr-0.01334*His-0.01175*Trp |
| 1.72761+0.01366*Cit-0.00253*Tyr-0.01311*His-0.01163*Trp-0.00058*Lys |
| 2.02818-0.00534*Val+0.00907*Leu-0.01354*His-0.01187*Trp |

## INTERNATIONAL SEARCH REPORT

| | |
|---|---|
| | International application No. |
| | PCT/JP2008/050942 |

A. CLASSIFICATION OF SUBJECT MATTER
*G01N33/68*(2006.01)i, *A61B10/00*(2006.01)n

According to International Patent Classification (IPC) or to both national classification and IPC

B. FIELDS SEARCHED

Minimum documentation searched (classification system followed by classification symbols)
G01N33/68, A61B10/00

Documentation searched other than minimum documentation to the extent that such documents are included in the fields searched
Jitsuyo Shinan Koho          1922-1996    Jitsuyo Shinan Toroku Koho    1996-2008
Kokai Jitsuyo Shinan Koho    1971-2008    Toroku Jitsuyo Shinan Koho    1994-2008

Electronic data base consulted during the international search (name of data base and, where practicable, search terms used)
JMEDPlus(JDream2), JST7580(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| X | WO 2006/098192 A1  (Ajinomoto Co., Inc.), 21 September, 2006 (21.09.06), Claims; Fig. (Family: none) | 1-57 |
| X | VILLANUEVA V. R., Chromatography, flow injection analysis and electrophoresis in computer-assisted comparative biochemistry: its application and possibilities in clinical research. Preliminary studies on crohn's disease, J. Chromatogr., 1988, Vol.440, Page.261-273 Summary | 1,2,5-8, 15-18 |
| Y | | 3,4,9-14, 19-57 |

☒ Further documents are listed in the continuation of Box C.          ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered   to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 13 February, 2008 (13.02.08) | 26 February, 2008 (26.02.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**INTERNATIONAL SEARCH REPORT**

| International application No. |
|---|
| PCT/JP2008/050942 |

C (Continuation). DOCUMENTS CONSIDERED TO BE RELEVANT

| Category* | Citation of document, with indication, where appropriate, of the relevant passages | Relevant to claim No. |
|---|---|---|
| X | Nobuo AOYAMA, "Kaiyosei Daichoen ni Okeru Kessho Amino acid Bunpu", JJPEN, 1994, | 1,2,5-8, 15-18 |
| Y | Vol.16, No.7, pages 719 to 721, Kekka | 3,4,9-14, 19-57 |
| X | Norikazu MORITA, "56 Kaiyosei Daichoen Katsudoki Kanja no Kecchu Amino acid no | 1,2,5-8, 15-18 |
| Y | Dotai to Byotai ni Sokushita Eiyo Ryohozai no Kaihatsu", Journal of Japanese Society of Chemical Nutrition, 1999, Vol.21, No.2, page 98, full text | 3,4,9-14, 19-57 |
| X | Kunihiko AOYAGI, "Crohn-byo Kanja ni Okeru Kessho Amino acid Chi no Kento", JJPEN, 1987, | 1,2,5-8, 15-18 |
| Y | Vol.9, No.4, pages 593 to 599, table 3 | 3,4,9-14, 19-57 |
| X | Tadashi KOSAKA, "Crohn-byo Kanja ni Okeru Kecchu Taurine Nodo no Kento", Digestion & | 1,2,5-8, 15-18 |
| Y | Absorption, 1993, Vol.16, No.2, pages 82 to 86, Kekka | 3,4,9-14, 19-57 |
| X | Hiroyuki HIRAKAWA, "<Tokushu> Kecchu Amino acid Hyoka to Chiryo Crohn-byo Kanja no Seibun | 1,2,5-8, 15-18 |
| Y | Eiyo Ryoho Keikachu ni Okeru Eiyo Shihyo no Kento Kessho Yuri Amino acid · Repid Turnover Protein · Rinsho Eiyo Shihyo no Hendo", Japanese Journal of Nutritional Assessment, 1991, Vol.8, No.4, pages 301 to 306, II. Kekka | 3,4,9-14, 19-57 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004135546 A
- JP 2004321179 A
- JP 11190734 A
- JP 2003232788 A
- WO 2004052191 A
- WO 2006098192 A

**Non-patent literature cited in the description**

- **Munakata ; Akihiro.** Research Report in 1997 by the Division for Investigation and Research on Intractable Inflammatory Bowel Disorders as Diseases. *Diagnostic Criteria for Ulcerative Colitis,* 1998, 96
- **Hiwatashi ; Nobuo.** Investigation and Research on Intractable Inflammatory Bowel Disorders. *Diagnostic Criteria for Crohn disease,* 2001, 76-77
- Simple mucosal biopsy criteria differentiating among Crohn disease, ulcerative colitis, and other forms of colitis: measurement of validity. *Scand J Gastroenterol.,* March 2000, vol. 35 (3), 281-6
- Rederived values of the eight coefficients of the Crohn's Disease Activity Index (CDAI). *Gastroenterology,* October 1979, vol. 77, 843-6
- **Harvey RF ; Bradshaw JM.** A simple index of Crohn's-disease activity. *Lancet,* 1980, vol. 1, 514
- Faecal calprotectin: a new marker for Crohn's disease?. *Ann Clin Biochem.,* May 2004, vol. 41, 230-2
- The Montreal classification of inflammatory bowel disease: controversies, consensus, and implications. *Gut.,* June 2006, vol. 55 (6), 749-53